(19) Europäisches Patentamt
European Patent Office
Office européen des brevets

(11) EP 4 610 260 B1

(12) EUROPEAN PATENT SPECIFICATION

(45) Date of publication and mention of the grant of the patent:
**01.07.2026 Bulletin 2026/27**

(21) Application number: **23881919.7**

(22) Date of filing: **26.10.2023**

(51) International Patent Classification (IPC):
***C07D 231/54*** (2006.01) ***C07D 487/04*** (2006.01)
***C07D 519/00*** (2006.01) ***A61K 31/415*** (2006.01)
***A61K 31/4164*** (2006.01) ***A61K 31/4196*** (2006.01)
***A61P 1/02*** (2006.01) ***A61P 1/04*** (2006.01)
***A61P 3/04*** (2006.01) ***A61P 3/10*** (2006.01)
***A61P 9/10*** (2006.01) ***A61P 11/06*** (2006.01)
***A61P 13/12*** (2006.01) ***A61P 17/06*** (2006.01)
***A61P 19/00*** (2006.01) ***A61P 19/02*** (2006.01)
***A61P 25/28*** (2006.01) ***A61P 29/00*** (2006.01)
***A61P 35/00*** (2006.01) ***A61P 37/02*** (2006.01)

(52) Cooperative Patent Classification (CPC):
**A61P 1/00; A61P 1/02; A61P 1/04; A61P 3/04; A61P 3/10; A61P 9/10; A61P 11/06; A61P 13/12; A61P 17/06; A61P 19/00; A61P 19/02; A61P 25/28; A61P 29/00; A61P 35/00; A61P 37/02;** (Cont.)

(86) International application number:
**PCT/CN2023/126822**

(87) International publication number:
**WO 2024/088343 (02.05.2024 Gazette 2024/18)**

# (54) ARYL HETEROCYCLIC KV1.3 INHIBITOR, PREPARATION METHOD THEREFOR, AND USE THEREOF

ARYLHETEROCYCLISCHER KV1.3-INHIBITOR, HERSTELLUNGSVERFAHREN DAFÜR UND VERWENDUNG DAVON

INHIBITEUR DE KV1.3 HÉTÉROCYCLIQUE ARYLE, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.10.2022 CN 202211338806**

(43) Date of publication of application:
**03.09.2025 Bulletin 2025/36**

(73) Proprietor: **Shanghai Shenshi Wise Technology Co., Ltd.**
**Shanghai 201203 (CN)**

(72) Inventors:
- **HUANG, Dandan**
  **Shanghai 201203 (CN)**
- **WANG, Dongdong**
  **Shanghai 201203 (CN)**
- **ZHANG, Xiaomin**
  **Shanghai 201203 (CN)**
- **SUN, Weijie**
  **Shanghai 201203 (CN)**

(74) Representative: **Huang, Liwei**
**Cäcilienstraße 12**
**40597 Düsseldorf (DE)**

(56) References cited:
**WO-A1-2021/071806** **WO-A1-2022/076285**
**CN-A- 114 728 177** **CN-A- 114 746 151**
**CN-A- 114 828 963**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**C07D 231/54; C07D 487/04; C07D 519/00**

## Description

### TECHNICAL FIELD

The present invention relates to a Kv1.3 inhibitor, and specially relates to a compound, a pharmaceutical composition, and the product for use as a medicament.

### BACKGROUND

Kv1.3 channel (or Kv1.3) is a voltage-gated potassium channel (Kv) that is highly expressed on macrophages, microglia, and effector memory T (TEM) cells. The Kv1.3 channel can regulate cellular membrane potential and thus indirectly affect calcium signaling in TEM cells. TEM cells mediate a variety of immune and inflammatory diseases, such as multiple sclerosis, inflammatory bowel disease, rheumatoid arthritis, type 1 diabetes, psoriasis, asthma, etc.

Autoimmune diseases are a large group of diseases caused by the body's own immune system attacking normal cells and tissues. Current therapeutic strategies, primarily relying on anti-inflammatory or immunosuppressive drugs, can only relieve symptoms and are associated with severe toxic and side effects. Studies have demonstrated a strong etiological link between TEM cells and autoimmune diseases (Proceedings of the National Academy of Sciences, 103, 46 (2006): 17414-17419). Selective inhibition of TEM cell functions offers a therapeutic strategy for autoimmune diseases while avoiding impairment of protective immune responses and reducing treatment-induced side effects. TEM cells abundantly express Kv1.3 and rely on it to fulfill their cellular functions. Kv1.3 inhibitors/blockers have been disclosed in WO 2022/076285, WO 2021/071806, CN114746151A, CN114828963A and CN114728177A. Therefore, selective Kv1.3 inhibitors hold promise as low-toxicity, high-efficiency therapeutic drugs for autoimmune diseases.

Dalazatide (ShK-186), a derivative obtained by derivatization with unnatural amino acids of Kv1.3-inhibitory peptide toxins isolated from scorpion and sea anemone, shows a certain therapeutic effect in Phase Ib clinical trials for psoriasis treatment. However, the application of such molecules is limited due to the poor subtype selectivity and the incapability of oral administration of peptidic molecules. Therefore, the development of highly selective, orally administerable small-molecule Kv1.3 inhibitors has great social and market value.

### SUMMARY

The scope of the invention and of protection is solely defined by the appended claims. In particular, the scope of protection does not include any method of therapeutic treatment of the human or animal body, even if such subject-matter is disclosed or implied herein. The references to methods of treatment in the subsequent paragraphs of this description are to be interpreted as references to the product disclosed therein for use in those methods.

The present invention provides a novel Kv1.3 channel (or Kv1.3) inhibitor which can be used for preventing and/or treating Kv1.3 channel (or Kv1.3)-associated diseases.

According to one aspect of the present invention, provided is a compound represented by Formula (I) or a pharmaceutically acceptable salt or a deuterated compound thereof:

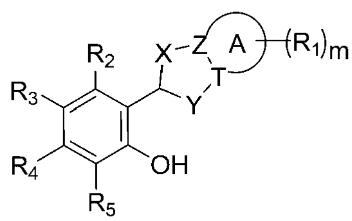

(I)

wherein

A is selected from 5-membered heteroaryl containing 1-3 ring heteroatoms independently selected from N, O, and S;
Z and T are independently selected from C and N;
X and Y are independently selected from CRaRb, NRc, and O;
Ra and Rb are independently selected from H, OH, $NH_2$, $C_{1-4}$ linear or branched alkyl, $C_{2-4}$ linear or branched alkenyl, $C_{2-4}$ linear or branched alkynyl, and $C_{1-4}$ linear or branched alkyloxy, or Ra and Rb, together with the carbon atom to which they are attached, form $C_{3-7}$ cycloalkyl or 3- to 7-membered heterocycloalkyl containing 1-3 ring heteroatoms independently selected from N, O, and S;
Rc is selected from H, $C_{1-4}$ linear or branched alkyl, $C_{2-4}$ linear or branched alkenyl, and $C_{3-7}$ cycloalkyl;

$R_2$, $R_3$, $R_4$, and $R_5$ are independently selected from H, F, Cl, Br, CN, $C_{1-4}$ linear or branched alkyl, $C_{2-4}$ linear or branched alkenyl, $C_{2-4}$ linear or branched alkynyl, $C_{1-4}$ linear or branched alkyloxy, $C_{2-4}$ linear or branched alkenyloxy, $C_{2-4}$ linear or branched alkynyloxy, and $C_{3-7}$ cycloalkyl;

each $R_1$ is independently selected from F, Cl, Br, CN, =0, $C_{1-4}$ linear or branched alkyl, $C_{2-4}$ linear or branched alkenyl, $C_{1-4}$ linear or branched alkynyl, $C_{3-7}$ cycloalkyl, 3- to 7-membered heterocycloalkyl attached via C or N and containing 1-3 ring heteroatoms independently selected from N, O, and S, aryl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, $-C_{1-4}$ linear or branched alkylene-$R_7$, $-OR_7$, $-O-C_{1-4}$ linear or branched alkylene-$R_7$, $-SR_7$, $-S-C_{1-4}$ linear or branched alkylene-$R_7$, $-N(R_6)R_7$, $-N(R_6)-C_{1-4}$ linear or branched alkylene-$R_7$, $-C(O)R_7$, $-C(O)-C_{1-4}$ linear or branched alkylene-$R_7$, $-C(O)R_7-OR_7$, $-C(O)R_7-N(R_6)R_7$, $-S(O)_2R_7$, $-N(R_6)C(O)R_7$, $-N(R_6)C(O)-C_{1-4}$ linear or branched alkylene-$R_7$, $-C(O)N(R_6)R_7$, $-C(O)N(R_6)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, and $-S(O)_2N(R_6)-C_{1-4}$ linear or branched alkylene-$R_7$, wherein:

the alkylene is optionally substituted with OH;

the alkyl, alkenyl, alkynyl, cycloalkyl, heterocycloalkyl, aryl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $-C(O)OC_{1-4}$ linear or branched alkyl, $C(O)OC_{2-4}$ linear or branched alkenyl, $C(O)OC_{2-4}$ linear or branched alkynyl, $-C(O)R_7$, $-S(O)_2R_7$, $-N(R_6)R_7$, $-N(R_6)-C_{1-4}$ linear or branched alkylene-$R_7$, $-N(R_6)C(O)R_7$, $-N(R_6)C(O)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, $-S(O)_2N(R_6)-C_{1-4}$ linear or branched alkylene-$R_7$, $-C(O)N(R_6)R_7$, $-C(O)N(R_6)-C_{1-4}$ linear or branched alkylene-$R_7$, $-P(O)(R_6)_2$, substituted or unsubstituted $C_{3-7}$ cycloalkyl, substituted or unsubstituted aryl, substituted or unsubstituted 3- to 7-membered heterocycloalkyl attached via C or N and containing 1-3 ring heteroatoms independently selected from N, O, and S, and substituted or unsubstituted 5- to 6-membered heteroaryl attached via C or N and containing 1-3 ring heteroatoms independently selected from N, O, and S, wherein substituent groups for the substituted $C_{3-7}$ cycloalkyl, substituted aryl, substituted 3- to 7-membered heterocycloalkyl, and substituted 5- to 6-membered heteroaryl are selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, and $C_{1-4}$ linear or branched alkyloxy; the two substituents on the same ring carbon atom in the cycloalkyl, heterocycloalkyl, and heteroaryl, together with the carbon atom to which they are attached, may optionally form $C_{3-6}$ cycloalkyl or 3- to 6-membered heterocycloalkyl containing 1 ring heteroatom selected from N and O, the formed cycloalkyl or heterocycloalkyl being optionally substituted with one or more groups independently selected from OH, CN, $NH_2$, =O, F, Cl, Br, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $C_{3-6}$ cycloalkyl, and 3- to 6-membered heterocycloalkyl containing 1-3 ring heteroatoms independently selected from N, O, and S;

$R_6$ is selected from H, $C_{1-4}$ linear or branched alkyl, $C_{2-4}$ linear or branched alkenyl, and $C_{2-4}$ linear or branched alkynyl;

$R_7$ is independently selected from H, OH, $NH_2$, $C_{1-4}$ linear or branched alkyl, $C_{2-4}$ linear or branched alkenyl, $C_{2-4}$

linear or branched alkynyl, $C_{1-4}$ linear or branched alkyloxy, $C_{1-4}$ linear or branched alkylene-OH, $C_{3-7}$ cycloalkyl, 3- to 7-membered heterocycloalkyl attached via C or N and containing 1-3 ring heteroatoms independently selected from N, O, and S, aryl, and 5- to 11-membered heteroaryl containing 1-3 ring heteroatoms independently selected from N, O, and S. the alkyl, alkenyl, alkynyl, cycloalkyl, heterocycloalkyl, aryl, and heteroaryl being optionally substituted with one or more groups independently selected from F, Cl, Br, OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;
m is selected from 1 and 2.

In some preferred embodiments, provided is the compound of Formula (I):

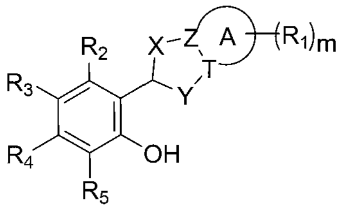
(I)

wherein

A is selected from 5-membered heteroaryl containing 1-3 ring heteroatoms independently selected from N, O, and S;
Z and T are independently selected from C and N;
X and Y are independently selected from CRaRb, NRc, and O;
Ra and Rb are independently selected from H, OH, $NH_2$, $C_{1-4}$ linear or branched alkyl, $C_{2-4}$ linear or branched alkenyl, $C_{2-4}$ linear or branched alkynyl, and $C_{1-4}$ linear or branched alkyloxy, or Ra and Rb, together with the carbon atom to which they are attached, form $C_{3-7}$ cycloalkyl or 3- to 7-membered heterocycloalkyl containing 1-3 ring heteroatoms independently selected from N, O, and S;
Rc is selected from H, $C_{1-4}$ linear or branched alkyl, $C_{2-4}$ linear or branched alkenyl, and $C_{3-7}$ cycloalkyl;
$R_2$, $R_3$, $R_4$, and $R_5$ are independently selected from H, F, Cl, Br, CN, $C_{1-4}$ linear or branched alkyl, $C_{2-4}$ linear or branched alkenyl, $C_{2-4}$ linear or branched alkynyl, and $C_{3-7}$ cycloalkyl;
each $R_1$ is independently selected from F, Cl, Br, CN, $C_{1-4}$ linear or branched alkyl, $C_{2-4}$ linear or branched alkenyl, $C_{2-4}$ linear or branched alkynyl, $C_{3-7}$ cycloalkyl, 3- to 7-membered heterocycloalkyl attached via C or N and containing 1-3 ring heteroatoms independently selected from N, O, and S, aryl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -O$R_7$, -O-$C_{1-4}$ linear or branched alkylene-$R_7$, -S$R_7$, -S-$C_{1-4}$ linear or branched alkylene-$R_7$, -N($R_6$)$R_7$, -N($R_6$)-$C_{1-4}$ linear or branched alkylene-$R_7$, -C(O)$R_7$, -C(O)-$C_{1-4}$ linear or branched alkylene-$R_7$, -C(O)$R_7$-O$R_7$, -C(O)$R_7$-N($R_6$)$R_7$, -S$(O)_2$$R_7$, -N($R_6$)C(O)$R_7$, -N($R_6$)C(O)-$C_{1-4}$ linear or branched alkylene-$R_7$, -C(O)N($R_6$)$R_7$, -C(O)N($R_6$)-$C_{1-4}$ linear or branched alkylene-$R_7$, -N($R_6$)S$(O)_2$$R_7$, -N($R_6$)S$(O)_2$-$C_{1-4}$ linear or branched alkylene-$R_7$, -S$(O)_2$N($R_6$)$R_7$, and -S$(O)_2$N($R_6$)-$C_{1-4}$ linear or branched alkylene-$R_7$, wherein:

the alkylene is optionally substituted with OH;
the alkyl, alkenyl, alkynyl, cycloalkyl, heterocycloalkyl, aryl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or

more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, -C(O)O$C_{1-4}$ linear or branched alkyl, C(O)O$C_{2-4}$ linear or branched alkenyl, C(O)O$C_{2-4}$ linear or branched alkynyl, -C(O)$R_7$, -S(O)$_2R_7$, -N($R_6$)$R_7$, -N($R_6$)-$C_{1-4}$ linear or branched alkylene-$R_7$, -N($R_6$)C(O)$R_7$, -N($R_6$)C(O)-$C_{1-4}$ linear or branched alkylene-$R_7$, -N($R_6$)S(O)$_2R_7$, -N($R_6$)S(O)$_2$-$C_{1-4}$ linear or branched alkylene-$R_7$, -S(O)$_2$N($R_6$)$R_7$, -S(O)$_2$N($R_6$)-$C_{1-4}$ linear or branched alkylene-$R_7$, -C(O)N($R_6$)$R_7$, -C(O)N($R_6$)-$C_{1-4}$ linear or branched alkylene-$R_7$, -P(O)($R_6$)$_2$, $C_{3-7}$ cycloalkyl, and 3- to 7-membered heterocycloalkyl attached via C or N and containing 1-3 ring heteroatoms independently selected from N, O, and S; the two substituents on the same ring carbon atom in the cycloalkyl, heterocycloalkyl, and heteroaryl, together with the carbon atom to which they are attached, may optionally form $C_{3-6}$ cycloalkyl or 3-to 6-membered heterocycloalkyl containing 1 ring heteroatom selected from N and O, the formed cycloalkyl or heterocycloalkyl being optionally substituted with one or more groups independently selected from OH, CN, $NH_2$, =O, F, Cl, Br, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $C_{3-6}$ cycloalkyl, and 3- to 6-membered heterocycloalkyl containing 1-3 ring heteroatoms independently selected from N, O, and S;

$R_6$ is selected from H, $C_{1-4}$ linear or branched alkyl, $C_{2-4}$ linear or branched alkenyl, and $C_{2-4}$ linear or branched alkynyl;

$R_7$ is independently selected from H, OH, $NH_2$, $C_{1-4}$ linear or branched alkyl, $C_{2-4}$ linear or branched alkenyl, $C_{2-4}$ linear or branched alkynyl, $C_{1-4}$ linear or branched alkyloxy, $C_{1-4}$ linear or branched alkylene-OH, $C_{3-7}$ cycloalkyl, 3- to 7-membered heterocycloalkyl attached via C or N and containing 1-3 ring heteroatoms independently selected from N, O, and S, aryl, and 5- to 6-membered heteroaryl containing 1-3 ring heteroatoms independently selected from N, O, and S. the alkyl, alkenyl, alkynyl, cycloalkyl, heterocycloalkyl, aryl, and heteroaryl being optionally substituted with one or more groups independently selected from F, Cl, Br, OH, CN, $NH_2$, =O, $C_{1-4}$ linear or branched alkyl. $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;

m is selected from 1 and 2.

In a preferred embodiment,

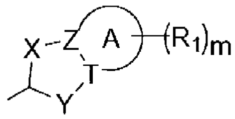

is selected from

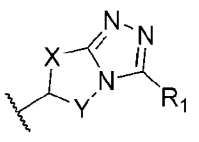 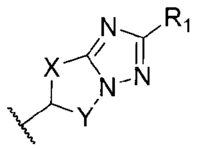 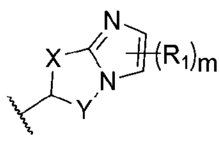 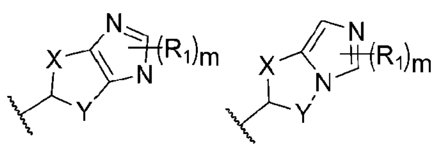

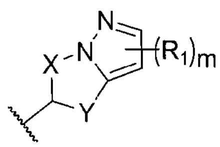 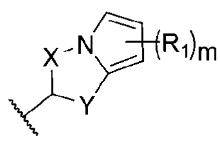 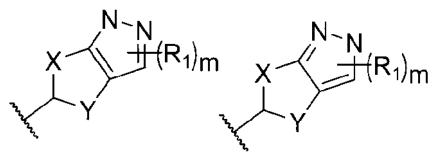

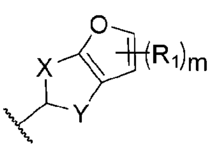 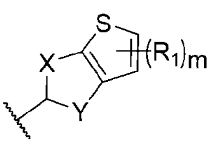 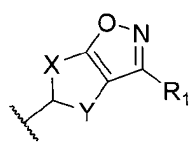 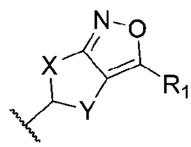 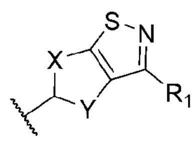 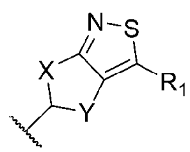

, wherein X, Y, $R_1$, m, $R_2$, $R_3$, $R_4$, and $R_5$ are as defined for the aforementioned compound of Formula (I).

In a further preferred embodiment, X and Y are CRaRb, and $R_1$, m, $R_2$, $R_3$, $R_4$, $R_5$, Ra, and Rb are as defined for the aforementioned compound of Formula (I).

In one preferred embodiment, the present invention provides a compound represented by Formula (II) or a pharmaceutically acceptable salt or a deuterated compound thereof:

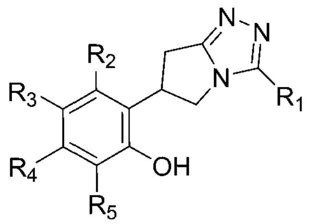

(II)

wherein:

$R_1$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, $-C_{1-4}$ linear or branched alkylene-$R_7$, $-OR_7$, $-O-C_{1-4}$ linear or branched alkylene-$R_7$, $-SR_7$, $-S-C_{1-4}$ linear or branched alkylene-$R_7$, $-N(R_6)R_7$, $-N(R_6)-C_{1-4}$ linear or branched alkylene-$R_7$, $-C(O)R_7$, $-C(O)-C_{1-4}$ linear or branched alkylene-$R_7$, $-N(R_6)C(O)R_7$, $-N(R_6)C(O)-C_{1-4}$ linear or branched alkylene-$R_7$, $-C(O)N(R_6)R_7$, $-C(O)N(R_6)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, and $-S(O)_2N(R_6)-C_{1-4}$ linear or branched alkylene-$R_7$, wherein:

the alkylene is optionally substituted with OH;

the alkyl, alkenyl, alkynyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkyloxy, $-C(O)OC_{1-4}$ linear or branched alkyl, $C(O)OC_{2-4}$ linear or branched alkenyl, $C(O)OC_{2-4}$ linear or branched alkynyl, $C(O)NHC_{1-4}$ linear or branched alkyl, $C(O)NHC_{2-4}$ linear or branched alkenyl, $C(O)NHC_{2-4}$ linear or branched alkynyl, $-C(O)R_7$, substituted or unsubstituted $C_{3-7}$ cycloalkyl, substituted or unsubstituted aryl, substituted or unsubstituted 3- to 7-membered heterocycloalkyl attached via C or N and containing 1-3 ring heteroatoms independently selected from N, O, and S, and substituted or unsubstituted 5-to 6-membered heteroaryl attached via C or N and containing 1-3 ring heteroatoms independently selected from N, O, and S, wherein substituent groups for the substituted $C_{3-7}$ cycloalkyl, substituted aryl, substituted 3-to 7-membered heterocycloalkyl, and substituted 5- to 6-membered heteroaryl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, $C_{2-4}$ linear or branched alkenylene-$NH_2$, $C_{2-4}$ linear or branched alkynylene-$NH_2$, and $C_{1-4}$ linear or branched alkyloxy;

$R_6$ is selected from H, $C_{1-4}$ linear or branched alkyl, $C_{2-4}$ linear or branched alkenyl, and $C_{2-4}$ linear or branched alkynyl;

$R_7$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently

selected from N, O, and S; the alkyl, alkenyl, alkynyl, cycloalkyl, heterocycloalkyl, phenyl, and heteroaryl are optionally substituted with one or more groups independently selected from OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;
$R_2$ and $R_3$ are independently selected from F, Cl, and Br;
$R_4$ and $R_5$ are H.

In some preferred embodiments of the present invention, provided is the compound of Formula (II):

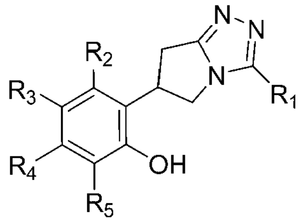

(II)

wherein:

$R_1$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, -$C_{1-4}$ linear or branched alkylene-$R_7$, -O$R_7$, -O-$C_{1-4}$ linear or branched alkylene-$R_7$, -S$R_7$, -S-$C_{1-4}$ linear or branched alkylene-$R_7$, -N($R_6$)$R_7$, -N($R_6$)-$C_{1-4}$ linear or branched alkylene-$R_7$, -C(O)$R_7$, -C(O)-$C_{1-4}$ linear or branched alkylene-$R_7$, -N($R_6$)C(O)$R_7$, -N($R_6$)C(O)-$C_{1-4}$ linear or branched alkylene-$R_7$, -C(O)N($R_6$)$R_7$, -C(O)N($R_6$)-$C_{1-4}$ linear or branched alkylene-$R_7$, -N($R_6$)S$(O)_2R_7$, -N($R_6$)S$(O)_2$-$C_{1-4}$ linear or branched alkylene-$R_7$, -S$(O)_2$N($R_6$)$R_7$, and -S$(O)_2$N($R_6$)-$C_{1-4}$ linear or branched alkylene-$R_7$; the alkylene is optionally substituted with OH; the alkyl, alkenyl, alkynyl, cycloalkyl, heterocycloalkyl, phenyl, and heteroaryl are optionally substituted with one or more groups independently selected from OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;
$R_6$ is selected from H, $C_{1-4}$ linear or branched alkyl, $C_{2-4}$ linear or branched alkenyl, and $C_{2-4}$ linear or branched alkynyl;
$R_7$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, alkenyl, alkynyl, cycloalkyl, heterocycloalkyl, phenyl, and heteroaryl are optionally substituted with one or more groups independently selected from OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;
$R_2$ and $R_3$ are independently selected from F, Cl, and Br;
$R_4$ and $R_5$ are H.

In a further preferred embodiment, in Formula (II):

$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S,

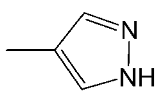,

pyridinyl,

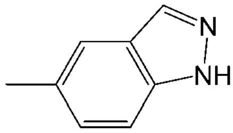
,

$-C_{1-4}$ linear or branched alkylene-$R_7$, $-OR_7$, $-SR_7$, $-S-C_{1-4}$ linear or branched alkylene-$R_7$, $-N(R_6)R_7$, $-C(O)R_7$, $-N(R_6)C(O)R_7$, $-C(O)N(R_6)R_7$, $-N(R_6)S(O)_2R_7$, and $-S(O)_2N(R_6)R_7$; the alkylene is optionally substituted with OH; the alkyl, cycloalkyl, heterocycloalkyl,

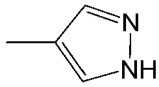
,

pyridinyl, and

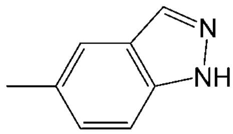

are optionally substituted with one or more groups independently selected from OH, CN, $NH_2$, =O, $C_{1-4}$ linear or branched alkyl, $C_{1-4}$ linear or branched alkylene-OH, $-C(O)OC_{1-4}$ linear or branched alkyl, and $C(O)NHC_{1-4}$ linear or branched alkyl;

$R_6$ is selected from H and $C_{1-4}$ linear or branched alkyl;

$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S,

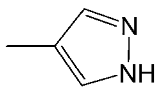
,

pyridinyl, and

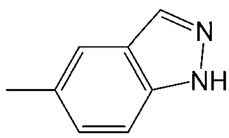
;

the alkyl, cycloalkyl, heterocycloalkyl,

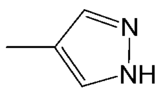
,

pyridinyl, and

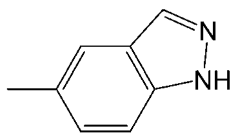

are optionally substituted with one or more groups independently selected from OH, CN, $NH_2$, =O, $C_{1-4}$ linear or branched alkyl, $C_{1-4}$ linear or branched alkylene-OH, $-C(O)OC_{1-4}$ linear or branched alkyl, and $C(O)NHC_{1-4}$ linear or branched alkyl;

$R_2$ and $R_3$ are independently selected from F, Cl, and Br;
$R_4$ and $R_5$ are H.

In a more preferred embodiment, in Formula (II):

$R_1$ is $C_{1-4}$ linear or branched alkyl substituted with one or more OHs;
$R_2$ and $R_3$ are independently selected from F, Cl, and Br;
$R_4$ and $R_5$ are H.

In a more preferred embodiment, in Formula (II):

$R_1$ is $C_{1-4}$ linear or branched alkyl substituted with one OH;
$R_2$ and $R_3$ are Cl;
$R_4$ and $R_5$ are H.

In some other preferred embodiments of the present invention, in the compound represented by Formula (II):

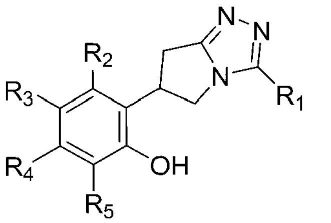

(II):

$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, $-C_{1-4}$ linear or branched alkylene-$R_7$, $-OR_7$, $-O-C_{1-4}$ linear or branched alkylene-$R_7$, $-C(O)R_7$, and $-C(O)-C_{1-4}$ linear or branched alkylene-$R_7$, wherein:

the alkylene is optionally substituted with OH;
the alkyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkyloxy, $-C(O)OC_{1-4}$ linear or branched alkyl, $-C(O)R_7$, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, and $C_{1-4}$ linear or branched alkyloxy;
$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, cycloalkyl, heterocycloalkyl, phenyl, and heteroaryl are optionally substituted with one or more groups independently selected from OH, $C_{1-4}$ linear or branched alkyl, and $C_{1-4}$ linear or branched alkylene-OH;
$R_2$ and $R_3$ are independently selected from F, Cl, and Br;
$R_4$ and $R_5$ are H.

In a further preferred embodiment, in Formula (II):

$R_1$ is selected from $C_{1-4}$ linear or branched alkyl, $C_{3-6}$ cycloalkyl, 3- to 6-membered heterocycloalkyl attached via C or N and containing 1-2 ring heteroatoms independently selected from N and O, phenyl,

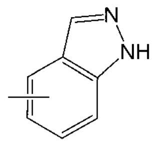

,

pyridinyl,

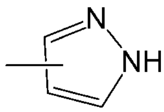

,

5- to 7-membered bridged heterocycloalkyl containing 1 ring heteroatom of O, a fused-ring structure consisting of a 3- to 7-membered heteroalkane ring containing 1 ring heteroatom of O and a 3- to 7-membered alkane ring, -$C_{1-4}$ linear or branched alkylene-$R_7$, -$OR_7$, and -$C(O)R_7$, wherein: the alkyl, cycloalkyl, heterocycloalkyl, phenyl,

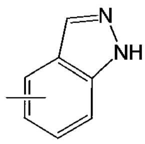

,

pyridinyl,

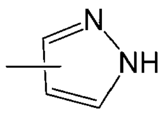

,

bridged heterocycloalkyl, and fused-ring structure are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -$C(O)OC_{1-4}$ linear or branched alkyl, -$C(O)R_7$, substituted or unsubstituted $C_{3-7}$ cycloalkyl, and substituted or unsubstituted 3- to 7-membered heterocycloalkyl attached via C or N and containing 1-2 ring heteroatoms independently selected from N and O, wherein substituent groups for the substituted $C_{3-7}$ cycloalkyl and substituted 3- to 7-membered heterocycloalkyl are selected from $C_{1-4}$ linear or branched alkyl optionally substituted with one or more groups independently selected from F, Cl, and Br;

$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;

$R_2$ and $R_3$ are independently selected from F, Cl, and Br;

$R_4$ and $R_5$ are H.

In a more preferred embodiment, in Formula (II):

$R_1$ is selected from $C_{3-6}$ cycloalkyl and 3- to 6-membered heterocycloalkyl attached via C or N and containing 1-2 ring heteroatoms independently selected from N and O, wherein: the cycloalkyl and heterocycloalkyl are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -$C(O)OC_{1-4}$ linear or branched alkyl, -$C(O)R_7$, substituted or unsubstituted $C_{3-6}$ cycloalkyl, and substituted or unsubstituted 3- to 6-membered heterocycloalkyl attached via C or N and containing 1-2 ring heteroatoms independently selected from N and O, wherein substituent groups for the substituted $C_{3-6}$ cycloalkyl and substituted 3- to 6-membered heterocycloalkyl are selected from $C_{1-4}$ linear or branched alkyl optionally substituted with one or more groups independently selected from F, Cl,

and Br;

$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;

$R_2$ and $R_3$ are independently selected from F, Cl, and Br;

$R_4$ and $R_5$ are H.

In one preferred embodiment of the present invention, further provided is a compound represented by Formula (III) or a pharmaceutically acceptable salt or a deuterated compound thereof:

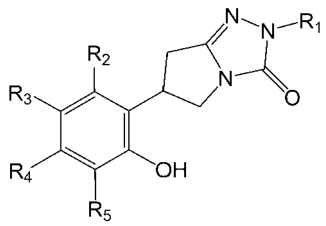

Formula (III),

wherein:

$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, $C_{5-7}$ bridged cycloalkyl, and 5- to 7-membered bridged heterocycloalkyl containing 1-3 ring heteroatoms independently selected from N, O, and S, wherein: the alkyl, cycloalkyl, heterocycloalkyl, bridged cycloalkyl, and bridged heterocycloalkyl are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from $C_{1-4}$ linear or branched alkyl optionally substituted with one or more groups independently selected from F, Cl, and Br;

$R_2$ and $R_3$ are independently selected from F, Cl, and Br;

$R_4$ and $R_5$ are H.

In a further preferred embodiment, in Formula (III):

$R_1$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O, wherein: the alkyl, cycloalkyl, and heterocycloalkyl are optionally substituted with one or more groups independently selected from F, Cl, Br, $C_{1-4}$ linear or branched alkyl, $C_{1-4}$ linear or branched alkylene-OH, and $C_{1-4}$ linear or branched alkyloxy;

$R_2$ and $R_3$ are independently selected from F, Cl, and Br;

$R_4$ and $R_5$ are H.

In one preferred embodiment of the present invention, further provided is a compound represented by Formula (IV) or a pharmaceutically acceptable salt or a deuterated compound thereof:

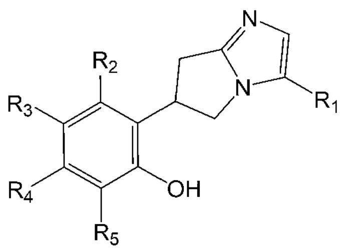

Formula (IV),

wherein:

$R_1$ is selected from $C_{1-4}$ linear or branched alkyl, $C_{3-6}$ cycloalkyl, 3- to 6-membered heterocycloalkyl attached via C or N

and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -$OR_7$, -O-$C_{1-4}$ linear or branched alkylene-$R_7$, -C(O)$R_7$, and -C(O)-$C_{1-4}$ linear or branched alkylene-$R_7$, wherein:

the alkylene is optionally substituted with OH;
the alkyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, and $C_{1-4}$ linear or branched alkyloxy;
$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, cycloalkyl, heterocycloalkyl, phenyl, and heteroaryl are optionally substituted with one or more groups independently selected from OH, $C_{1-4}$ linear or branched alkyl, and $C_{1-4}$ linear or branched alkylene-OH;
$R_2$ and $R_3$ are independently selected from F, Cl, and Br;
$R_4$ and $R_5$ are H.

In a further preferred embodiment, in Formula (IV):

$R_1$ is selected from $C_{1-4}$ linear or branched alkyl, $C_{3-6}$ cycloalkyl, 3- to 6-membered heterocycloalkyl attached via C or N and containing 1-2 ring heteroatoms independently selected from N and O, phenyl,

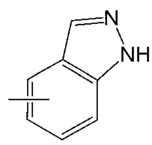

,

pyridinyl,

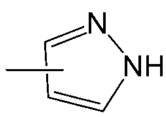

,

5- to 7-membered bridged heterocycloalkyl containing 1 ring heteroatom of O, a fused-ring structure consisting of a 3- to 7-membered heteroalkane ring containing 1 ring heteroatom of O and a 3- to 7-membered alkane ring, -$C_{1-4}$ linear or branched alkylene-$R_7$, -C(O)$R_7$, and -$OR_7$, wherein: the alkyl, cycloalkyl, heterocycloalkyl, phenyl,

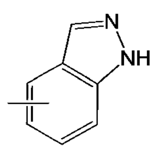

,

pyridinyl,

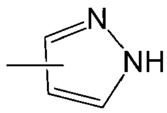

,

bridged heterocycloalkyl, and fused-ring structure are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-2 ring heteroatoms independently selected from N and O, wherein substituent groups for the substituted $C_{3-7}$ cycloalkyl and substituted 3- to 7-membered heterocycloalkyl are selected from $C_{1-4}$ linear or branched alkyl optionally substituted with one or more groups independently selected from F, Cl, and Br;

$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;

$R_2$ and $R_3$ are independently selected from F, Cl, and Br;

$R_4$ and $R_5$ are H.

In a more preferred embodiment, in Formula (IV):

$R_1$ is selected from $C_{1-4}$ linear or branched alkyl, $C_{3-6}$ cycloalkyl, 3- to 6-membered heterocycloalkyl attached via C or N and containing 1-2 ring heteroatoms independently selected from N and O, phenyl,

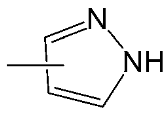

,

-$C_{1-4}$ linear or branched alkylene-$R_7$, -C(O)$R_7$, and -O$R_7$, wherein: the alkyl, cycloalkyl, heterocycloalkyl, phenyl, and

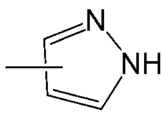

are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, and -C(O)O$C_{1-4}$ linear or branched alkyl;

$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;

$R_2$ and $R_3$ are independently selected from F, Cl, and Br;

$R_4$ and $R_5$ are H.

In some other preferred embodiments, the present invention further provides compounds of Formula (V) and Formula (V'), or pharmaceutically acceptable salts or deuterated compounds thereof:

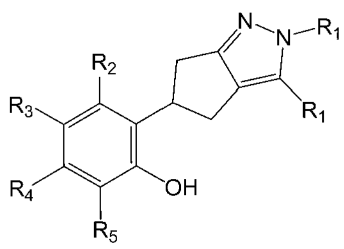 Formula (V) and 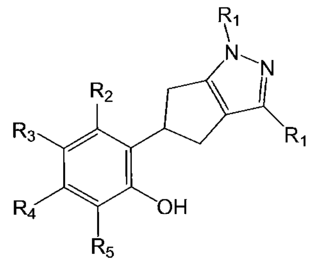 Formula (V'),

wherein:

in Formula (V) and Formula (V'), each $R_1$ is identical or different and is independently selected from H, $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -$OR_7$, -O-$C_{1-4}$ linear or branched alkylene-$R_7$, -C(O)$R_7$, and -C(O)-$C_{1-4}$ linear or branched alkylene-$R_7$, wherein:

the alkylene is optionally substituted with OH;
the alkyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, and $C_{1-4}$ linear or branched alkyloxy;
$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, cycloalkyl, heterocycloalkyl, phenyl, and heteroaryl are optionally substituted with one or more groups independently selected from OH, $C_{1-4}$ linear or branched alkyl, and $C_{1-4}$ linear or branched alkylene-OH;
$R_2$ and $R_3$ are independently selected from F, Cl, and Br;
$R_4$ and $R_5$ are H.

In a further preferred embodiment, in Formula (V) and Formula (V'):

each $R_1$ is identical or different and is independently selected from H, $C_{1-4}$ linear or branched alkyl, $C_{3-6}$ cycloalkyl, 3- to 6-membered heterocycloalkyl attached via C or N and containing 1-2 ring heteroatoms independently selected from N and O, and phenyl, wherein: the alkyl, cycloalkyl, heterocycloalkyl, and phenyl are optionally substituted with one or more groups independently selected from OH, F, Cl, Br, $C_{1-4}$ linear or branched alkyl, $C_{1-4}$ linear or branched alkylene-OH, and $C_{1-4}$ linear or branched alkyloxy;
$R_2$ and $R_3$ are independently selected from F, Cl, and Br;
$R_4$ and $R_5$ are H.

In a more preferred embodiment, in Formula (V) and Formula (V'):

each $R_1$ is identical or different and is independently selected from H and $C_{1-4}$ linear or branched alkyl, wherein: the alkyl is optionally substituted with one or more groups independently selected from OH, F, Cl, Br, $C_{1-4}$ linear or

branched alkyl, $C_{1-4}$ linear or branched alkylene-OH, and $C_{1-4}$ linear or branched alkyloxy;
$R_2$ and $R_3$ are independently selected from F, Cl, and Br;
$R_4$ and $R_5$ are H.

In a preferred embodiment, the compound of the present invention is selected from the following compounds or pharmaceutically acceptable salts or deuterated compounds thereof:

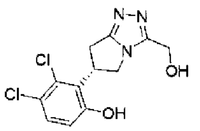
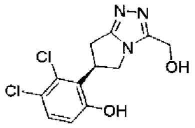
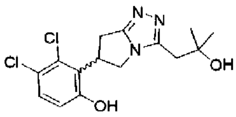
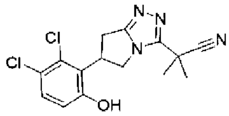
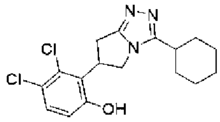
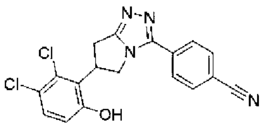
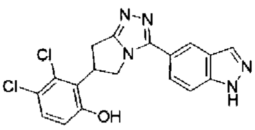
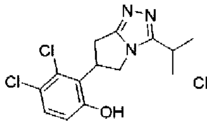
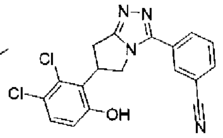
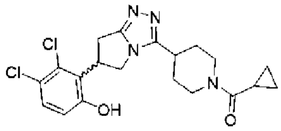
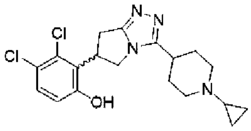
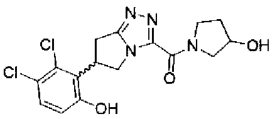
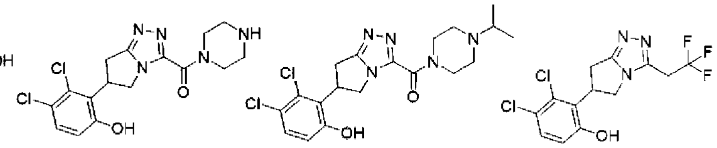
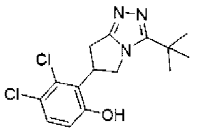
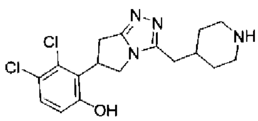
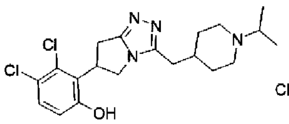
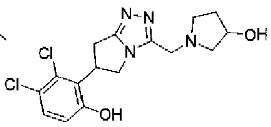
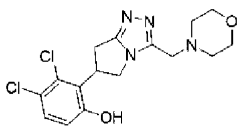
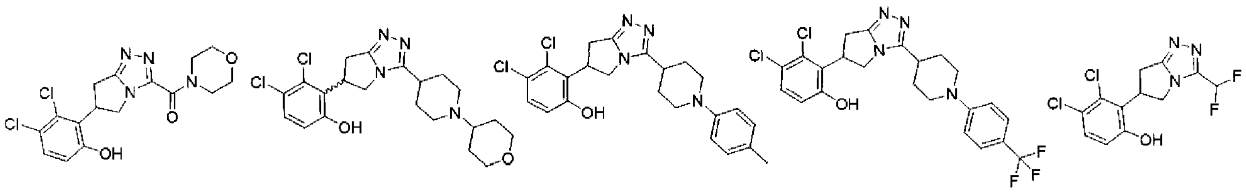
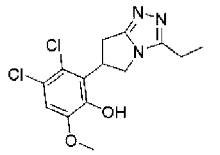
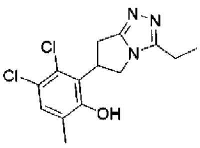
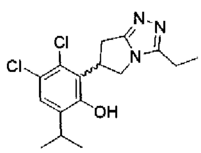
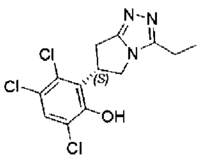
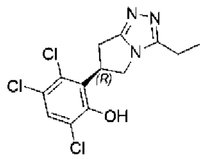
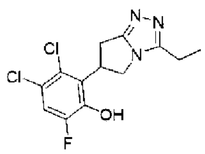
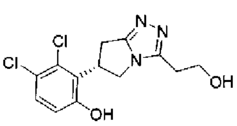
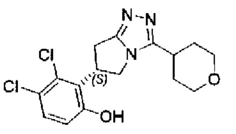
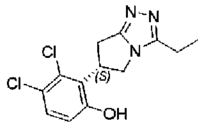
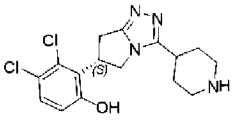
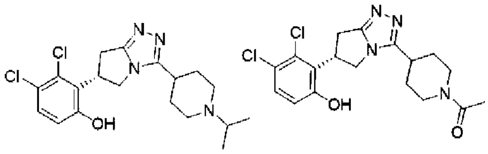
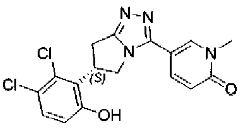
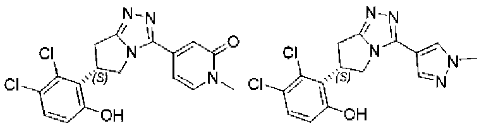

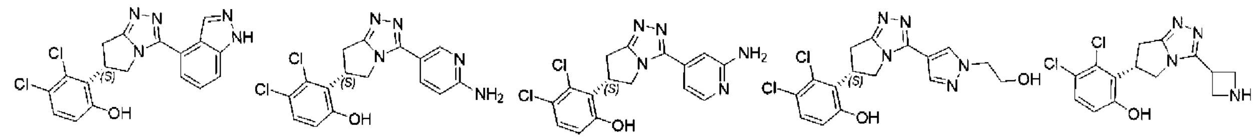

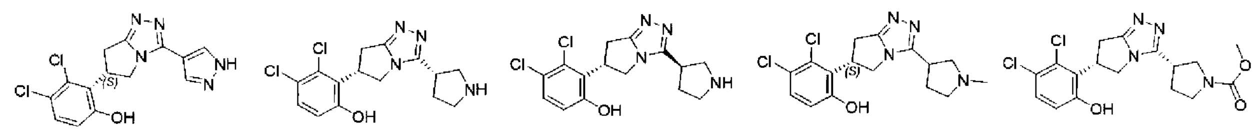

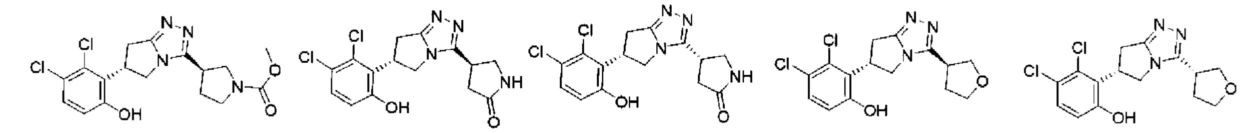

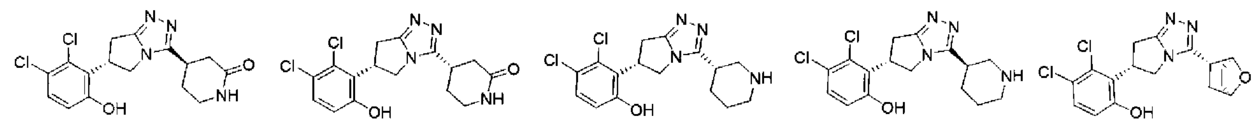

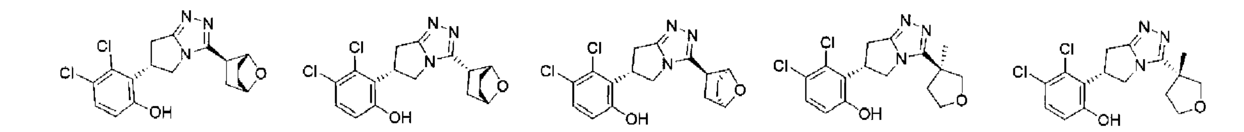

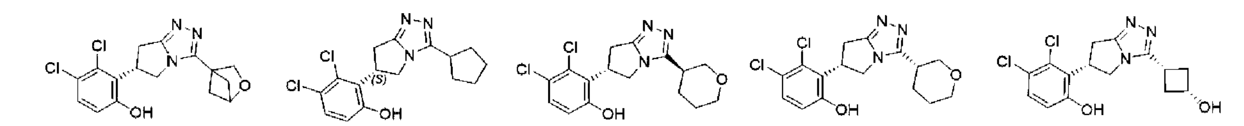

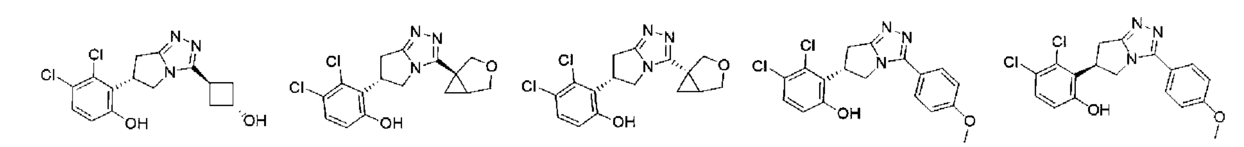

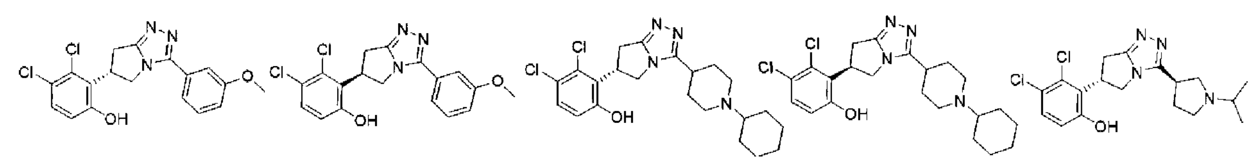

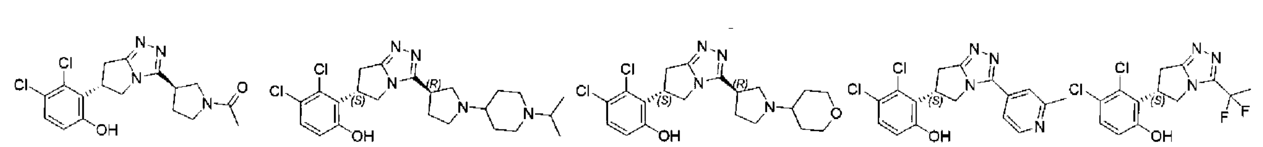

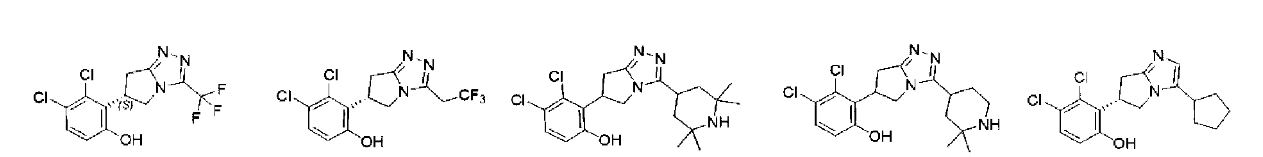

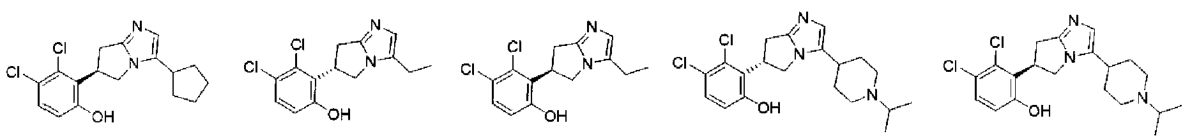

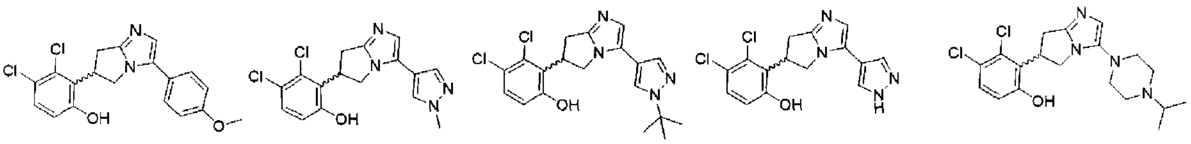

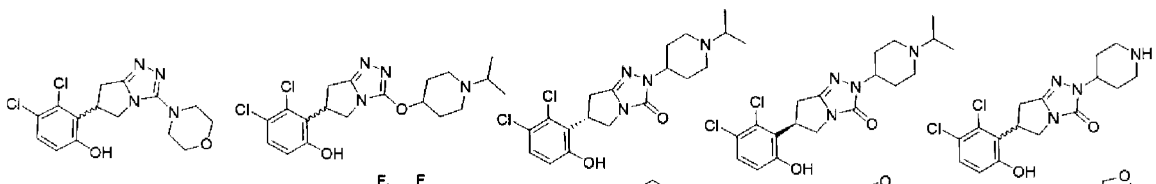

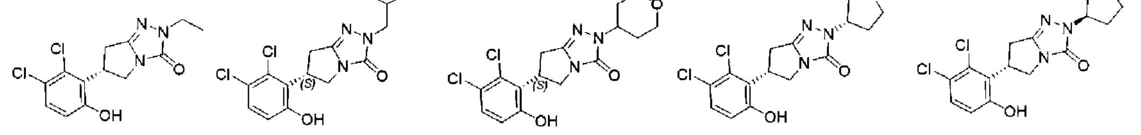

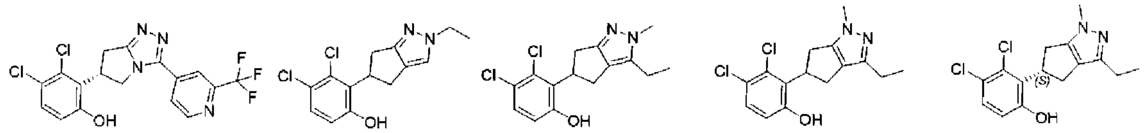

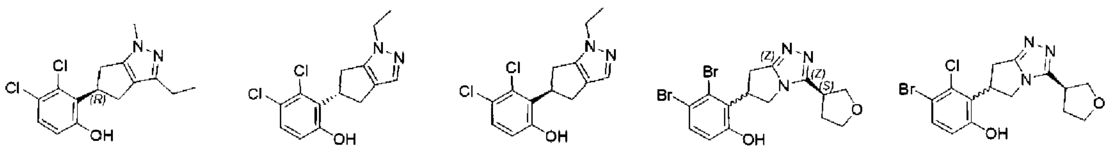

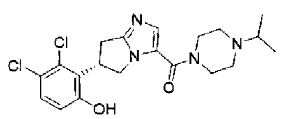

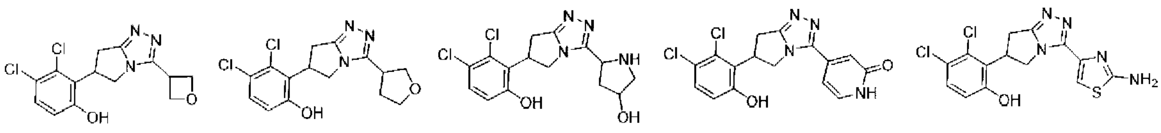

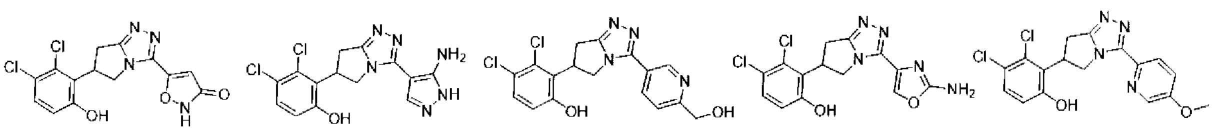

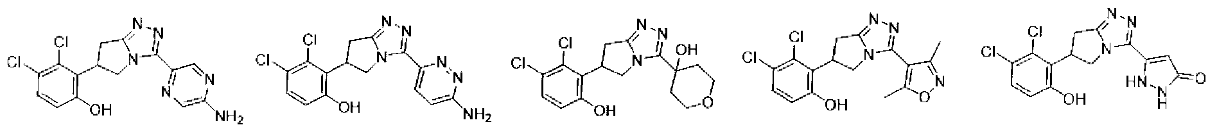

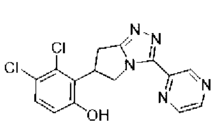 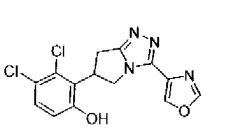 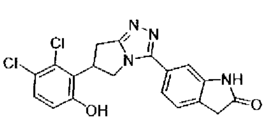 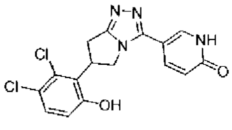 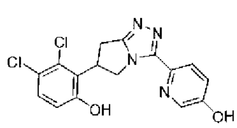

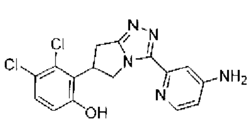 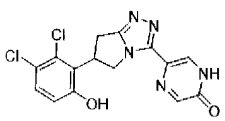 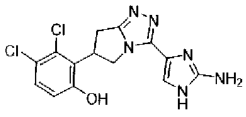 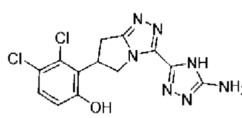 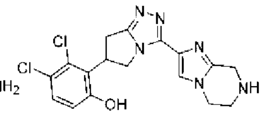

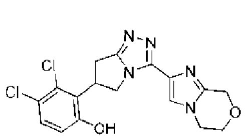 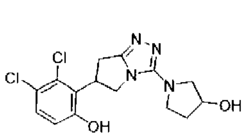 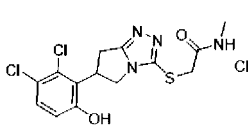 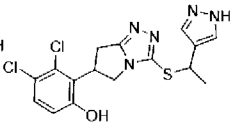 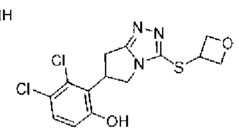

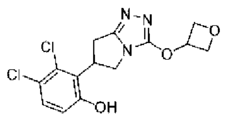 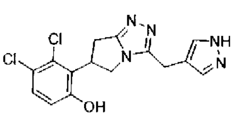 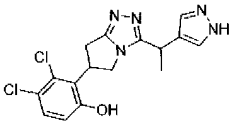 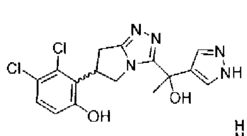 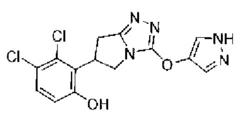

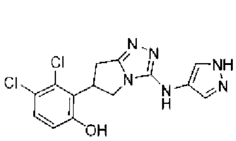 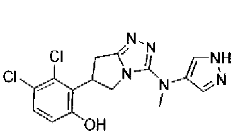 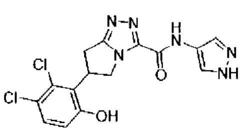 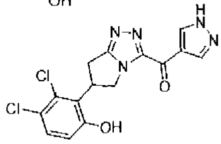 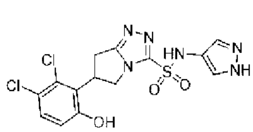

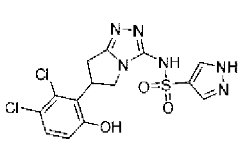 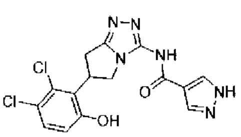 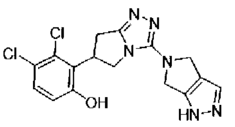 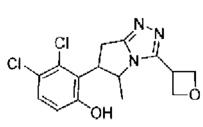 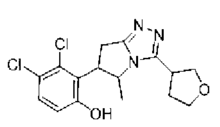

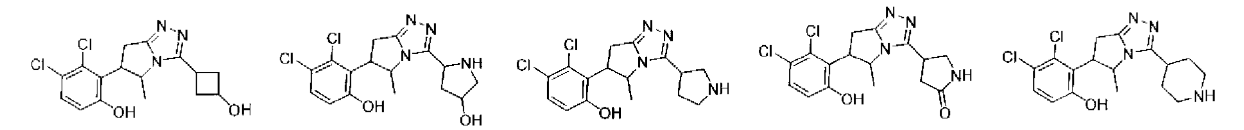

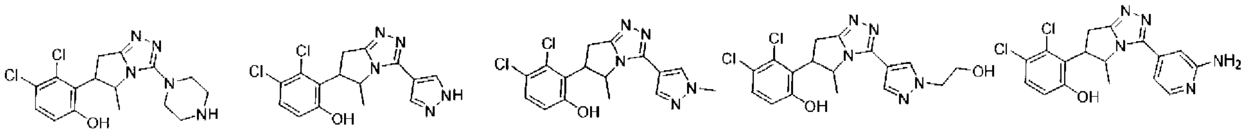

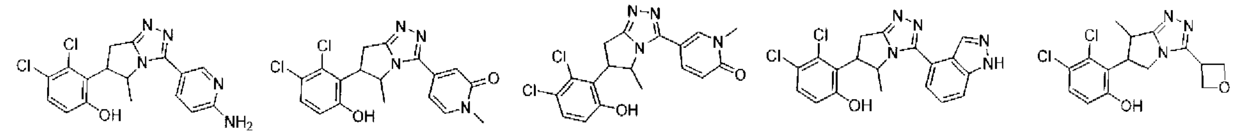

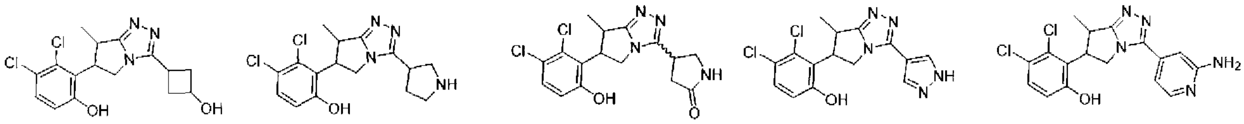

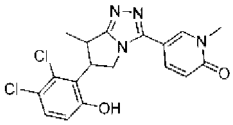

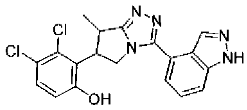

In the present invention, although substituents are disclosed as groups or ranges, each group or range disclosed herein is intended to specifically cover every individual substituent encompassed therein. For example, the term "C1-4 alkyl" refers specifically to independently disclosed methyl (i.e., C1 alkyl), ethyl (i.e., C2 alkyl), propyl (i.e., C3 alkyl), and butyl (i.e., C4 alkyl).

In the present invention, examples of the "$C_{3-6}$ cycloalkyl" include cyclopropyl, cyclobutyl, cyclopentyl, and cyclohexyl.

In the present invention, examples of the "$C_{3-7}$ cycloalkyl" also include cycloheptyl, in addition to those mentioned above.

In the present invention, examples of the "3- to 6-membered heterocycloalkyl containing 1-3 ring heteroatoms independently selected from N, O, and S" include, but are not limited to, oxiranyl, thiiranyl, aziridinyl, oxetanyl, azetidinyl, α-lactamyl, β-lactamyl, β-lactonyl, tetrahydrofuranyl, thiacyclopentanyl, pyrrolidinyl, pyrrolinyl, dioxolanyl, oxazolidinyl, oxazolinyl, isoxazolidinyl, thiazolidinyl, isothiazolidinyl, thiazolinyl, imidazolidinyl, imidazolinyl, pyrazolidinyl, pyrazolinyl, tetrahydropyranyl, dihydropyranyl, pyranyl, piperidinyl, 1,4-dioxanyl, morpholinyl, piperazinyl, 1.4-oxazepanyl, 1,4-thiazepanyl, and the like.

In the present invention, "3- to 6-membered heterocycloalkyl attached via C or N and containing 1-3 ring heteroatoms independently selected from N, O, and S" means that the heterocycloalkyl is attached via its ring C or ring N to other groups.

In the present invention, examples of the "3- to 7-membered heterocycloalkyl containing 1-3 ring heteroatoms independently selected from N, O, and S" also include, but are not limited to, azepane, oxepane, thiepane, and the like, in addition to those mentioned above.

In the present invention, examples of the "aryl" include, but are not limited to, phenyl, naphthyl, tetrahydronaphthyl, phenanthryl, benzonaphthyl, fluorenyl, and the like.

In the present invention, examples of the "5-membered heteroaryl containing 1-3 ring heteroatoms independently selected from N, O, and S" include, but are not limited to, furanyl, thiophenyl, pyrrolyl, oxazolyl, isoxazolyl, thiazolyl, isothiazolyl, pyrazolyl, imidazolyl, 1,2,3-triazolyl, 1,2,4-triazolyl, oxadiazolyl, thiadiazolyl, and the like.

In the present invetion, examples of "5- to 11-membered heteroaryl containing 1-3 ring heteroatoms independently selected from N, O, and S" include, but are not limited to, pyridinyl, pyrimidinyl, pyridazinyl, pyrazinyl, triazinyl, benzazetidinyl, benzo-β-lactamyl, benzo-β-lactonyl, benzodioxolyl, benzofuranyl, benzothiopheneyl, indolyl, indazolyl, isoindazolyl, benzimidazolyl, benzothiazolyl, benzisothiazolyl, benzoxazolyl, benzisoxazolyl, benzotriazolyl, benzoxadiazolyl, benzothiadiazolyl, benzodioxanyl, benzomorpholinyl, benzopiperidinyl, benzopyranyl, benzopyridinyl, benzopyrimidinyl, benzotriazinyl, benzazacyclohexanonyl, benzazepinonyl, pyridoazetidinyl, pyrido-β-lactamyl, pyrido-β-lactonyl, pyridodioxolyl, pyridofuranyl, pyridothiophenyl, pyridopyrrolyl, pyridopyrazolyl, pyridoimidazolyl, pyridothiazolyl, pyridoisothiazolyl, pyridooxazolyl, pyridoisoxazolyl, pyridotriazolyl, pyridooxadiazolyl, pyridothiadiazolyl, pyridodioxane, pyridomorpholine, pyridopiperidinyl, pyridopyranyl, pyridopyridinyl, pyridopyrimidinyl, pyridotriazinyl, pyridoazacyclohexanonyl, furopyrrolyl, thiazolopyrimidinyl, and the like, in addition to those mentioned above.

In the present invention, examples of the "$C_{5-7}$ bridged cycloalkyl" include, but are not limited to, bicyclo[1.1.1]pentanyl, bicyclo[2.2.1]heptanyl, bicyclo[2.2.2]octanyl, bicyclo[2.1.1]hexanyl, bicyclo[3.1.1]heptanyl, and the like.

In the present invention, examples of the "5- to 7-membered bridged heterocycloalkyl containing 1-3 ring heteroatoms independently selected from N, O, and S" include, but are not limited to, 2-oxabicyclo[2.2.2]octanyl, 2-oxabicyclo[2.1.1]hexanyl, 1-azabicyclo[2.2.1]heptanyl, 2-azabicyclo[2.2.1]heptanyl, 7-azabicyclo[2.2.1]heptanyl, 3,6-diazabicyclo[3.2.2]nonanyl, 3,6-diazabicyclo[3.1.1]heptanyl, 3-aza-6-oxabicyclo[3.1.1]heptanyl, 3-oxa-6-azabicyclo[3.1.1]heptanyl, 3,8-diazabicyclo[3.2.1]octanyl, 3-oxa-8-azabicyclo[3.2.1]octanyl, 3-aza-8-oxabicyclo[3.2.1]octanyl, 2-oxa-5-azabicyclo[2.2.1]heptanyl, 2,5-diazabicyclo[2.2.1]heptanyl, 2-oxa-5-azabicyclo[2.2.2]octanyl, 2,5-diazabicyclo[2.2.2]octanyl, and the like.

In the present invention, examples of the "fused-ring structure consisting of a 3- to 7-membered heteroalkane ring containing 1-3 ring heteroatoms independently selected from N, O, and S and a 3- to 7-membered alkane ring" include, but are not limited to, pyrrolidine-fused cyclopropanyl, pyrrolidine-fused cyclobutanyl, pyrrolidine-fused cyclopentanyl, tetrahydrofuran-fused cyclopentanyl, piperidine-fused cyclopropanyl, piperazine-fused cyclopropanyl, piperazine-fused cyclobutanyl, tetrahydropyran-fused cyclopropanyl, tetrahydropyran-fused cyclohexanyl, morpholine-fused cyclopropanyl, morpholine-fused cyclobutanyl, and the like.

In the present invention, examples of the "fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S" include, but are not limited to, pyrrolidine-fused

oxetanyl, pyrrolidine-fused azetidinyl, pyrrolidine-fused pyrrolidinyl, pyrrolidine-fused tetrahydrofuranyl, tetrahydrofuran-fused tetrahydrofuranyl, pyrrolidine-fused tetrahydropyranyl, piperidine-fused oxetanyl, piperidine-fused azetidinyl, piperidine-fused pyrrolidinyl, piperidine-fused piperidinyl, piperazine-fused piperidinyl, piperazine-fused pyrrolidinyl, morpholine-fused oxetanyl, morpholine-fused azetidinyl , morpholine-fused pyrrolidinyl, and the like.

In the present invention, examples of the "fused-ring structures consisting of a benzene ring and a 3- to 7-membered heteroalkane ring containing 1-3 ring heteroatoms independently selected from N, O, and S" include, but are not limited to, benzoxetanyl, benzotetrahydrofuranyl, benzopyrrolidinyl, isatinyl, 1,3-benzodioxolyl, 1,4-benzodioxanyl, benzopiperidinyl, benzopiperazinyl, 3,4-dihydro-1H-quinazolin-2-onyl, 3,4-dihydro-2H-1,4-benzothiazinyl, 2,3-dihydro-1,4-benzodithiinyl, and the like.

In the present invention, examples of the "fused-ring structures consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S" include, but are not limited to, pyrrolidine-fused thiophenyl, pyrrolidine-fused furanyl, pyrrolidine-fused imidazolyl, pyrrolidine-fused pyrazolyl, pyrrolidine-fused isoxazolyl, pyrrolidine-fused thiazolyl, pyrrolidine-fused triazolyl, piperazine-fused imidazolyl, piperazine-fused pyrazolyl, piperazine-fused triazolyl, piperazine-fused tetrazolyl, piperidine-fused oxazolyl, piperidine-fused thiazolyl, piperidine-fused imidazolyl, piperidine-fused isoxazolyl, piperidine-fused pyrazolyl, piperidine-fused triazolyl, pyrazole-fused tetrahydrofuranyl, pyrazole-fused tetrahydropyranyl, morpholine-fused imidazolyl, morpholine-fused pyrazolyl, morpholine-fused thiophenyl, morpholine-fused thiazolyl, morpholine-fused oxazolyl, imidazole-fused dihydrothiazinyl, pyrazole-fused dihydrothiazinyl, pyridine-fused pyrrolidinyl, pyridine-fused piperidinyl, pyridine-fused piperazinyl, pyridine-fused dioxanyl, pyridine-fused tetrahydropyranyl, pyrimidine-fused pyrrolidinyl, pyridine-fused tetrahydrofuranyl, pyrimidine-fused piperazinyl, pyrimidine-fused piperidinyl, tetrahydrobenzazepinyl, dihydrobenzodioxepanyl, tetrahydrobenzodiazepinyl, tetrahydrobenzoxazepinyl, tetrahydrobenzothiazepinyl, tetrahydropyridoazepinyl, pentylenetetrazolyl, 3,4-propylenedioxythiophenyl, tetrahydroimidazoazepinyl, tetrahydropyrazoloazepinyl, and the like.

The compound of the present invention may be asymmetric, for example, having one or more stereocenters. All stereoisomers, such as enantiomers and diastereomers, are included within the scope of the present invention unless otherwise indicated. In the present invention, compounds containing asymmetrically substituted carbon atoms may be separated in any optically active form or racemic form. Various methods for preparing optically active forms are known in the art, such as resolution of racemic mixtures or stereoselective synthesis.

The present invention also includes a pharmaceutically acceptable salt of the compound. The compound of the present invention may be prepared as a pharmaceutically acceptable salt by a reaction with a non-toxic inorganic or organic acid. Examples of inorganic acids include hydrochloric acid, hydrobromic acid, hydroiodic acid, phosphoric acid, sulfuric acid, nitric acid, hydrogen sulfate, boric acid, hemisulfuric acid, and the like. Examples of organic acids include formic acid, acetic acid, propionic acid, butyric acid, valeric acid, hexanoic acid, heptanoic acid, undecanoic acid, palmitic acid, stearic acid, oleic acid, oxalic acid, malonic acid, adipic acid, lactic acid, malic acid, maleic acid, hippuric acid, tartaric acid, citric acid, succinic acid, ascorbic acid, methanesulfonic acid, ethanesulfonic acid, benzenesulfonic acid, p-toluenesulfonic acid, benzoic acid, camphoric acid, camphorsulfonic acid, citric acid, fumaric acid, gluconic acid, galacturonic acid, dodecyl sulfuric acid, various amino acids, and the like.

The present invention also includes a hydrate and a solvate of the compound.

The present invention also includes all forms of the compound whose atoms are replaced by various isotopes. Isotopes include all atoms having the same atomic number but different mass numbers. For example, isotopes of hydrogen include deuterium.

The present disclosure includes a prodrug of the compound. The "prodrug" refers to a compound obtained through structural modification of the compound, which has no or minimal activity outside the patient's body, but releases the compound through enzymatic or non-enzymatic conversion in the patient's body to exert its therapeutic effect.

It will be appreciated by those skilled in the art that the compound of the present invention can be prepared by various methods already disclosed in the literature. The compound of the present invention can be prepared by a reaction in a suitable solvent, and those skilled in the art of organic synthesis can readily select a suitable solvent that is essentially nonreactive with the reactants, intermediates, or products. The reaction may be conducted in one solvent or a mixture of more than one solvent. The reaction for preparing the compound of the present invention may be conducted at a suitable temperature, for example, a temperature between the freezing point and the boiling point of the solvent. The methods for preparing the compound of the present invention involve the protection and deprotection of various chemical groups. Those skilled in the art of organic synthesis can readily determine whether protection and deprotection of chemical groups are required and select appropriate protecting groups. The reaction for preparing the compound of the present invention may be monitored using any method known in the art, such as nuclear magnetic resonance spectroscopy, infrared spectroscopy, mass spectrometry, chromatography, and the like. Schemes 1-8 below describe synthetic routes that can be used to synthesize the compound of the present invention, such as a compound having a structure of Formula (I).

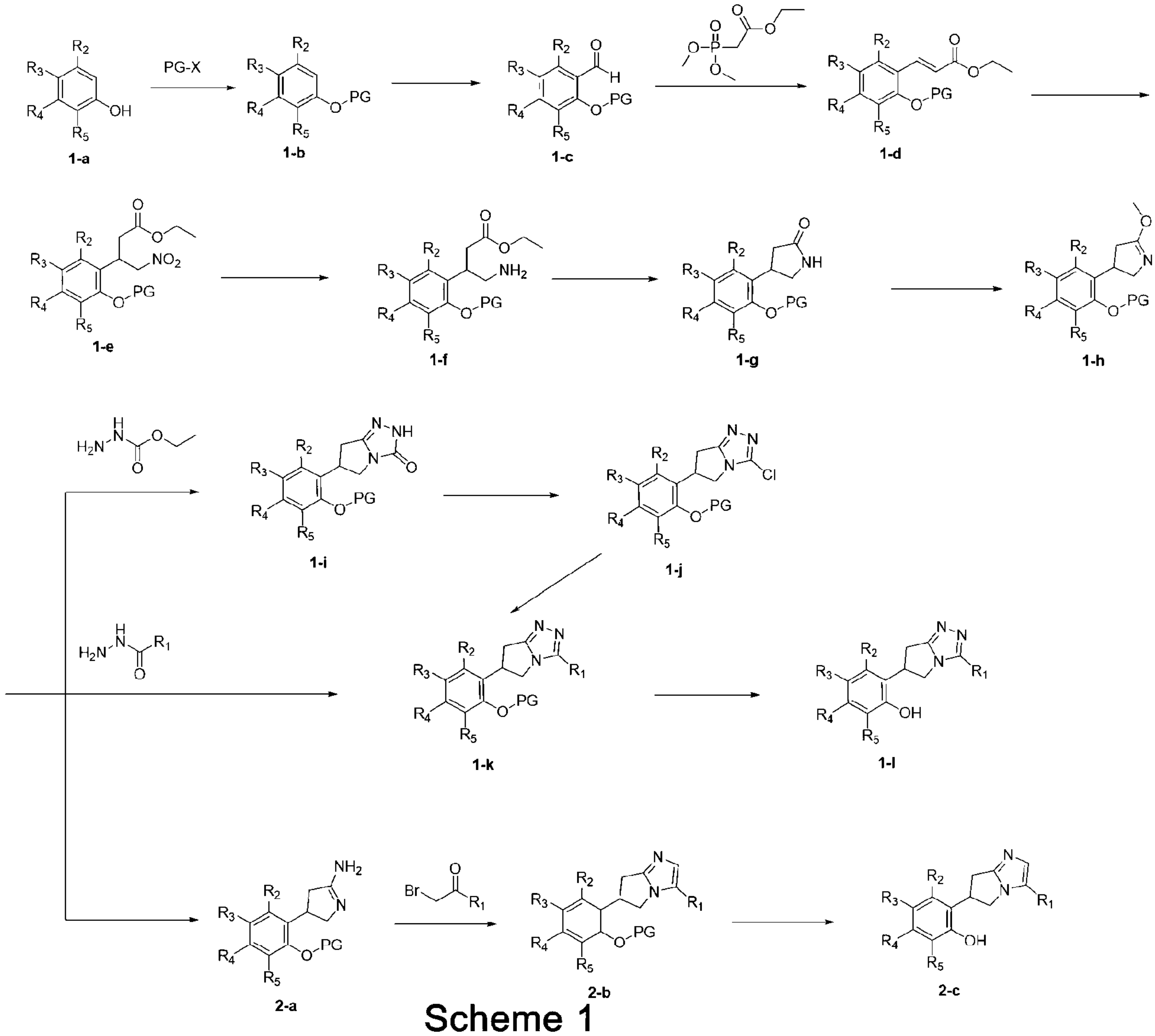


Scheme 1

Compounds 1-a, 1-b, and 1-c, as shown in Scheme 1, can be prepared by any method known in the art and/or purchased commercially. As shown in Scheme 1, PG refers to a protecting group. Non-limiting examples of protecting groups include methyl, allyl, acetyl, tert-butoxycarbonyl, benzyl, or other protecting groups known in the art that are suitable as OH-protecting groups. Other substituents are defined herein. As shown in Scheme 1: 1-a can undergo a two-step reaction involving phenolic hydroxyl protection followed by formylation to give 1-c. 1-c is subjected to a Wittig reaction, nitromethane substitution, nitro reduction, and intramolecular cyclization to give 1-g, which is then reacted with methyloxonium tetrafluoroborate to give Intermediate 1-h. Intermediate 1-h is reacted with ethyl carbazate to give 1-i, which is then subjected to chlorination using a chlorinating reagent (e.g., phosphoryl chloride, oxalyl chloride, etc.) to give 1-j; 1-j can be converted to 1-k via a Suzuki reaction or a Stille reaction, or through substitution with a hydroxyl- or amino-containing compound. Alternatively, Intermediate 1-h can undergo cyclization with a substituted acyl hydrazide to give 1-k. Finally, 1-k is subjected to deprotection to give 1-i.

1-h can be subjected to amino substitution to give 2-a, which is then reacted with a bromoacetyl compound to undergo cyclization, thus giving 2-b. Finally, 2-b is subjected to deprotection to give 2-c.

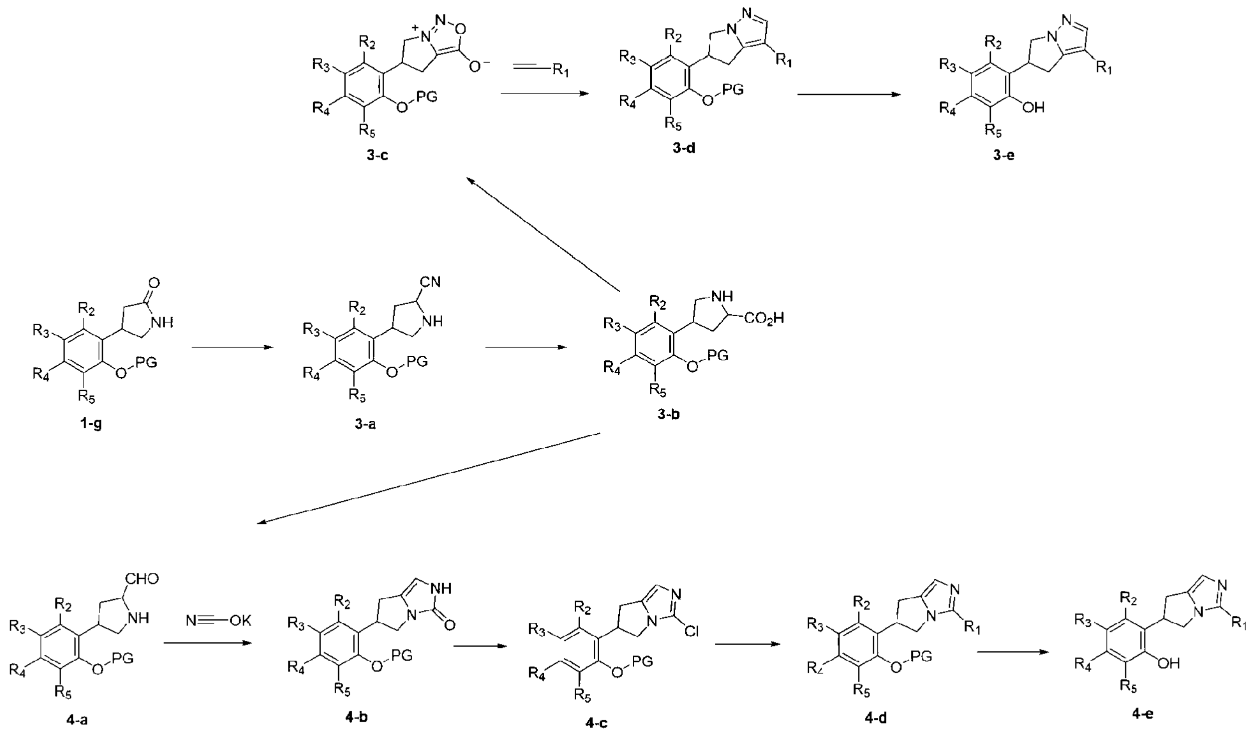

3-c 3-d 3-e

1-g 3-a 3-b

4-a 4-b 4-c 4-d 4-e

Scheme 2

As shown in Scheme 2, 1-g is reacted with trimethylsilyl cyanide to produce 3-a, which is then hydrolyzed to give 3-b. 3-b is subjected to diazotization with nitrous acid, followed by cyclization with trifluoroacetic anhydride to give 3-c. Subsequently, 3-c is reacted with a substituted alkyne to give 3-d, which is finally subjected to deprotection to give 3-e. Alternatively, 3-b can be subjected to a reduction reaction to give 4-a, which is then reacted with potassium cyanate to undergo cyclization, followed by chlorination to give 4-c. 4-c can be converted to 4-d via a Suzuki reaction or a Stille reaction, or through substitution with a hydroxyl- or amino-containing compound. Finally, 4-d is subjected to deprotection to give 4-e.

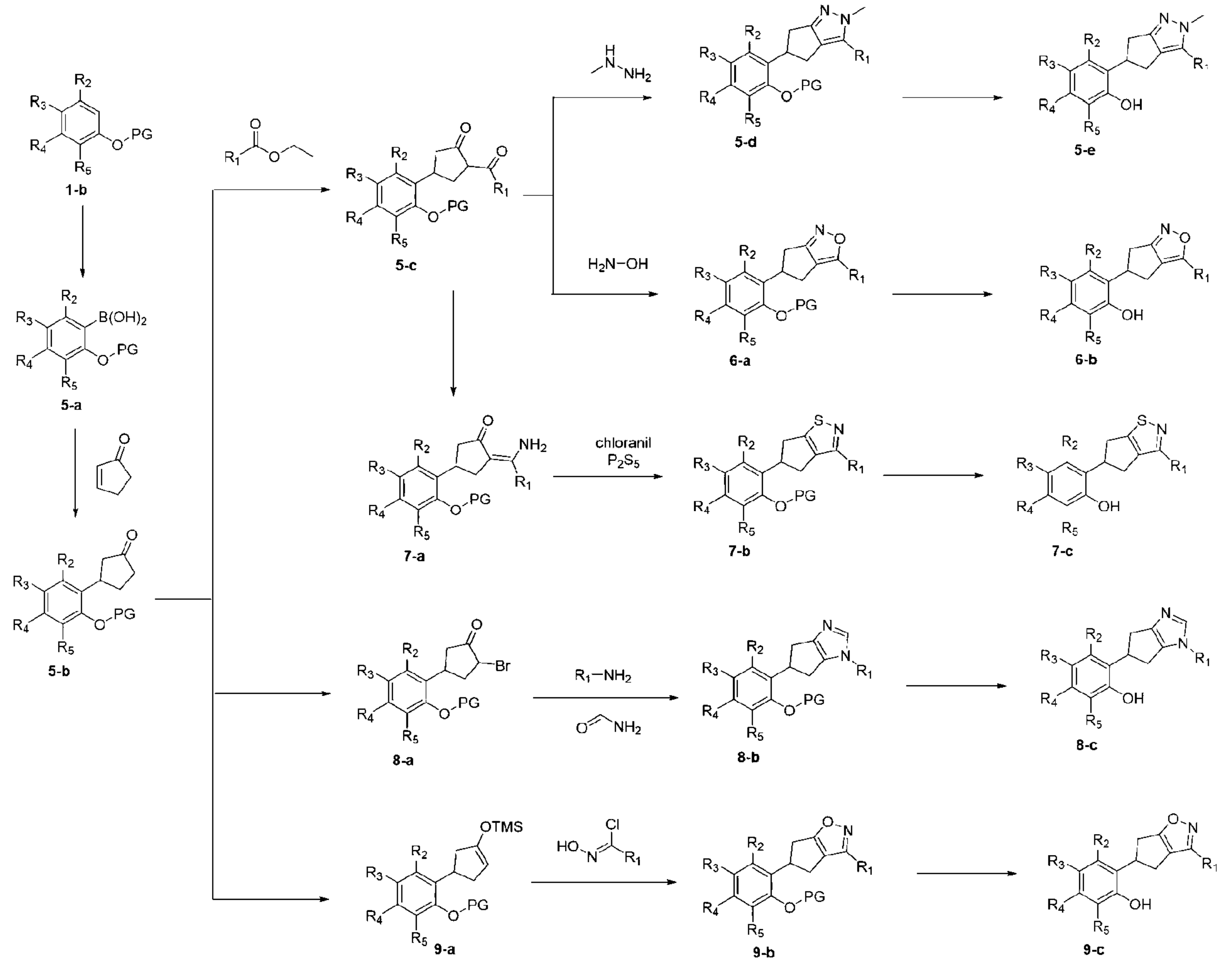


Scheme 3

Compounds 1-b and 5-a, as shown in Scheme 3, can be prepared by any method known in the art and/or purchased commercially. As shown in scheme 3, 5-a is reacted with cyclopentenone to give a common intermediate 5-b. 5-b can be reacted with a substituted ester to give 5-c, which is then reacted with methylhydrazine to undergo cyclization, followed by deprotection to give 5-e. 5-c can undergo cyclization with hydroxylamine hydrochloride to give 6-a, which is then subjected to deprotection to give 6-b. Alternatively, 5-c can be reacted with aqueous ammonia to give 7-a, which is then subjected to a cyclization reaction with phosphorus pentasulfide/chloranil to give 7-b. Subsequently, 7-b is subjected to deprotection to give 7-c.

5-b can undergo a bromination reaction to give 8-a, which is then reacted with a substituted amine/formamide to undergo cyclization, thus giving 8-b. Subsequently, 8-b is subjected to deprotection to give 8-c. Alternatively, 5-b can be reacted with a suitable silicon reagent to give a silyl enol ether 9-a, which is then reacted with substituted chlorinated formaldoxime to undergo cyclization, thus giving 9-b. Subsequently, 9-b is subjected to deprotection to give 9-c.

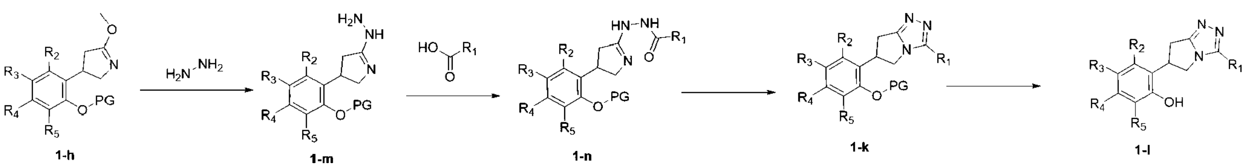


Scheme 4

As shown in Scheme 4, Intermediate 1-h can also be reacted with a hydrazine to give 1-m, which is then reacted with a carboxylic acid to give 1-n. 1-n undergoes cyclization to give 1-k, which is finally subjected to deprotection to give the final product 1-l.

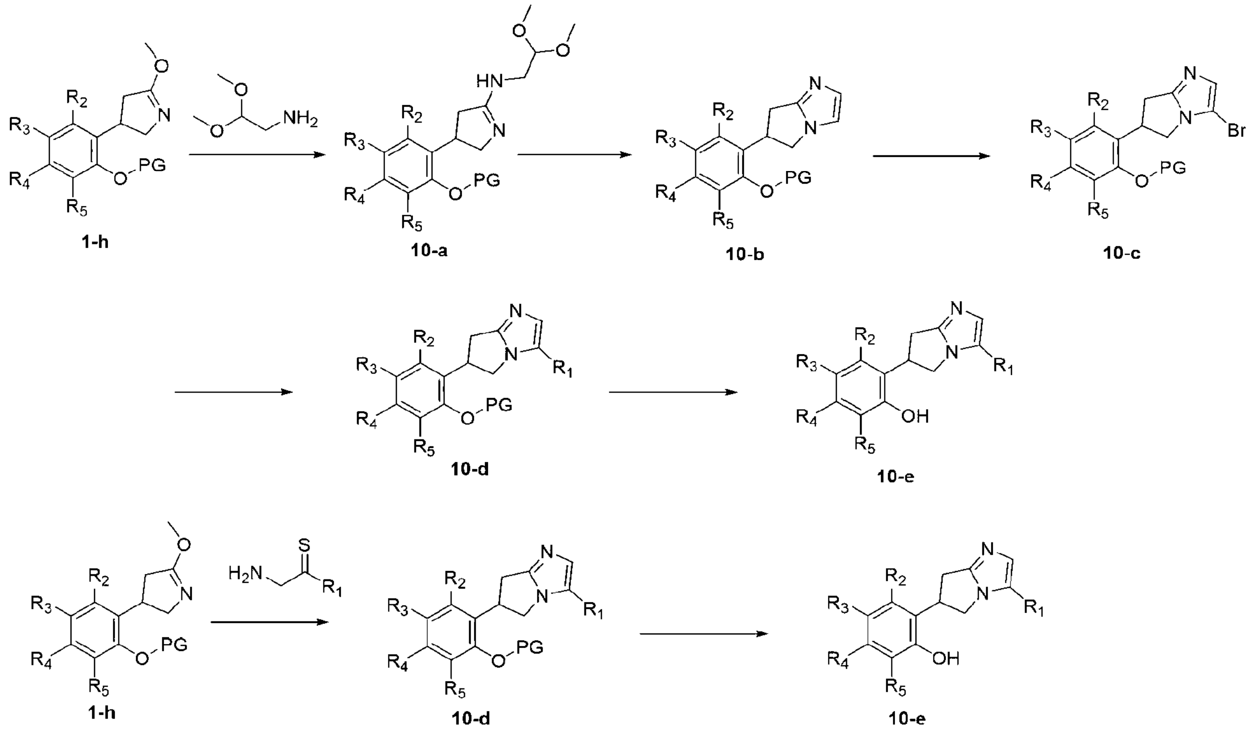

1-h 10-a 10-b 10-c

10-d 10-e

1-h 10-d 10-e

Scheme 5

As shown in Scheme 5, 1-h undergoes a two-step reaction involving substitution with aminoacetaldehyde dimethyl acetal followed by cyclization to give 10-b, which is then subjected to a bromination reaction to give 10-c. 10-c can be converted to 10-d via a Suzuki reaction or a Stille reaction, or through substitution with a hydroxyl- or amino-containing compound; subsequently, 10-d is subjected to deprotection to give the final product 10-e. Alternatively, 1-h can be reacted with a thioamide acetyl compound to give 10-d, which is then subjected to deprotection to give the final product 10-e.

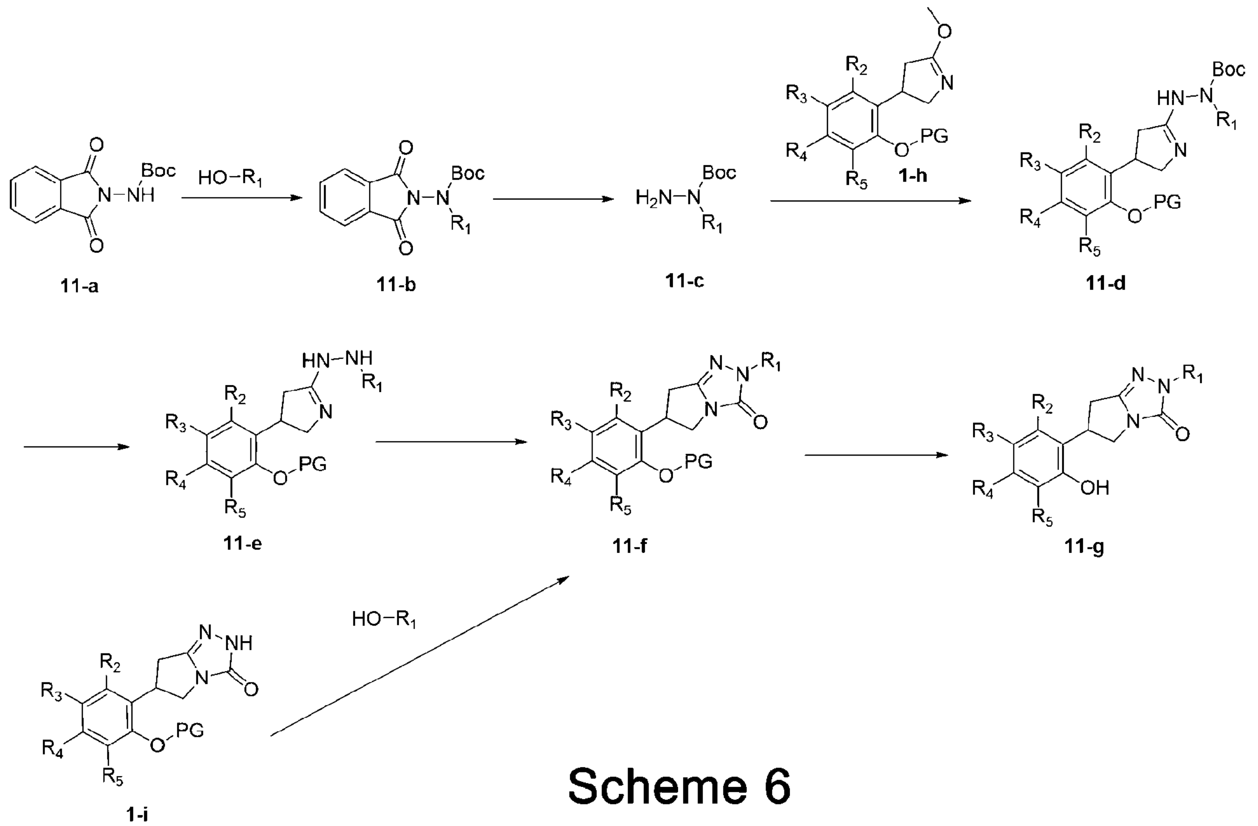


11-a 11-b 11-c 1-h 11-d

11-e 11-f 11-g

1-i

Scheme 6

As shown in Scheme 6, 11-a undergoes a Mitsunobu reaction with a hydroxyl compound to give 11-b, which is then subjected to deprotection to give intermediate 11-c. 11-c undergoes a substitution reaction with 1-h, followed by cyclization with triphosgene to give 11-f, which is then deprotected to give the final product 11-g. Alternatively, 1-i can undergo a Mitsunobu reaction with a hydroxyl compound to give 11-f, which is then deprotected to give the final product 11-g.

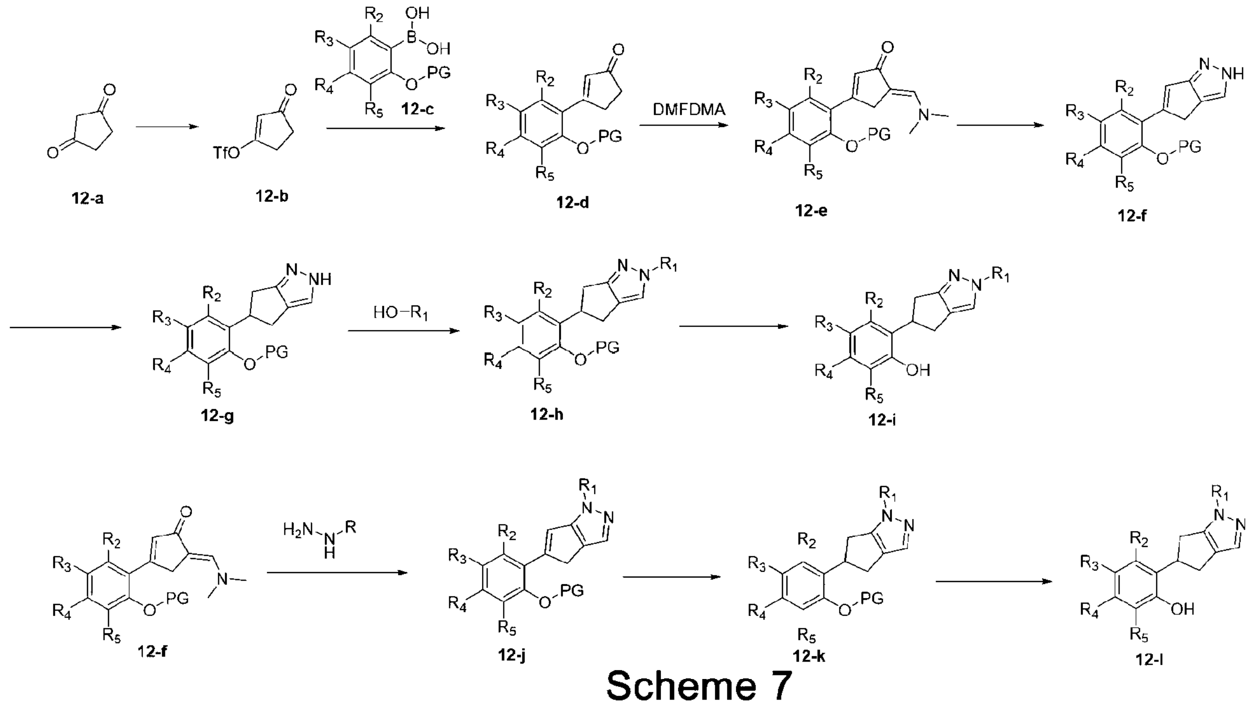


12-a 12-b 12-c 12-d 12-e 12-f 12-g 12-h 12-i 12-f 12-j 12-k 12-l

Scheme 7

Compound 12-c, as shown in Scheme 7, can be prepared by any method known in the art and/or purchased commercially. As shown in Scheme 7, 12-a is reacted with trifluoromethanesulfonate to give 12-b, which then undergoes a Suzuki reaction with 12-c to give 12-d. 12-d is reacted with DMFDMA to give 12-e, which then undergoes a two-step reaction involving cyclization with hydrazine hydrate followed by reduction to give 12-g. 12-g undergoes a Mitsunobu reaction with a hydroxyl compound to give 12-h, which is then deprotected to give the final product 12-i. Alternatively, Intermediate 12-f can undergo cyclization with a substituted hydrazine to give 12-j, which then undergoes a two-step reaction involving reduction followed by deprotection to give the final product 12-l.

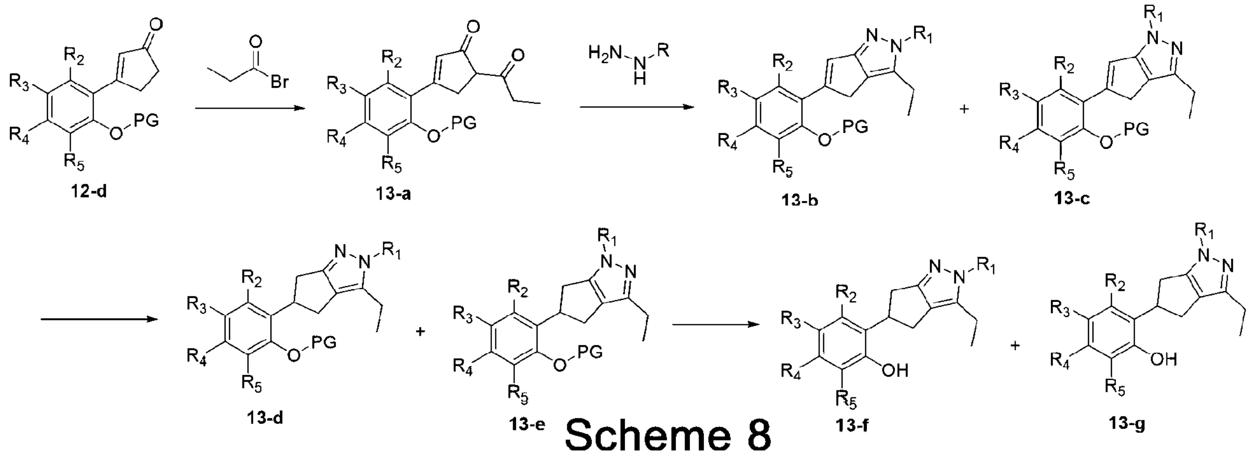

12-d 13-a 13-b + 13-c

13-d + 13-e 13-f + 13-g

Scheme 8

As shown in Scheme 8, 12-d is reacted with propionyl bromide to give 13-a, which is then reacted with a substituted hydrazine to undergo cyclization, thus giving 13-b and 13-c. Subsequently, 13-b and 13-c undergo a two-step reaction involving reduction followed by deprotection to give 13-f and 13-g.

The compound of the present invention is capable of inhibiting Kv1.3. Therefore, according to another aspect of the present invention, provided is a method for inhibiting Kv1.3 by using the compound of the present invention.

According to another aspect of the present disclosure, provided is a method for preventing and/or treating a Kv1.3-associated disease, wherein a therapeutically and/or prophylactically effective amount of the compound of the present invention or a pharmaceutical composition comprising the compound of the present invention is administered to an individual in need thereof. The Kv1.3-associated disease includes any disease directly and/or indirectly associated with the expression and/or activity of Kv1.3, for example, a disease that can be prevented and/or treated by inhibiting Kv1.3.

The Kv1.3-associated disease includes any of a group of immune and inflammatory diseases, such as multiple sclerosis, inflammatory bowel disease, ulcerative colitis, Crohn's disease, rheumatoid arthritis, type 1 diabetes, psoriasis and asthma, spondylitis, and periodontitis, as well as obesity, type 2 diabetes, renal fibrosis, Alzheimer's disease, ischemic stroke, etc.

According to another aspect of the present invention, provided is use of the compound of the present invention in the preparation of a Kv1.3 inhibitor.

According to another aspect of the present invention, provided is use of the compound of the present invention in the preparation of a medicament for preventing and/or treating a Kv1.3-associated disease.

When used to prevent and/or treat a Kv1.3-associated disease, the compound of the present invention may be administered in the form of a pharmaceutical composition. Therefore, according to another aspect of the present invention, provided is a pharmaceutical composition comprising the compound of the present invention and a pharmaceutically acceptable carrier.

It will be appreciated by those skilled in the art that the pharmaceutical composition of the present invention can be prepared by various methods already disclosed in the literature. The compounds or the pharmaceutical compositions of the present invention may be administered via various routes, depending on whether local or systemic treatment is required and the area in need of treatment. For example, administration may be carried out orally, parenterally (e.g., by intravenous, intraarterial, subcutaneous, intraperitoneal or intramuscular injection or infusion), intracranially (e.g., intrathecally or intracerebroventricularly), transdermally, ocularly, nasally, vaginally, rectally, or pulmonarily (e.g., by inhalation or insufflation of powders or aerosols).

For oral administration, the pharmaceutical composition of the present invention is typically provided in the form of a tablet, a capsule, or a solution. The tablet may contain the compound or the pharmaceutically acceptable salt thereof of the present invention and a pharmaceutically acceptable carrier. The carrier includes, but is not limited to, a diluent, a disintegrant, a binder, a lubricant, a colorant, or a preservative. The capsule includes a hard capsule and a soft capsule. For parenteral administration, the pharmaceutical composition of the present invention may be administered by intravenous, intramuscular, or subcutaneous injection. It is usually provided as a sterile aqueous solution or suspension or lyophilized powder, with appropriately adjusted pH and isotonicity.

The effective amount of the compound of the present invention may be determined based on the specific therapeutic use, the mode of administration, and the condition of the individual in need thereof (e.g., a patient). Those skilled in the art are capable of determining the effective amount of the compound of the present invention. A typical dose range is, for example, from 1 μg/kg/day to 1000 mg/kg/day.

When preventing and/or treating a Kv1.3-associated disease, the compound of the present invention may be used in combination with one or more additional drugs. The additional drugs include, but are not limited to, prednisone, methylprednisolone, ocrelizumab, natalizumab, alemtuzumab, β-interferon, glatiramer, fingolimod, dimethyl fumarate, diroximel, teriflunomide, siponimod, cladribine, mesalamine, balsalazide, olsalazine, azathioprine, mercaptopurine, methotrexate, infliximab, adalimumab, golimumab, certolizumab, vedolizumab, ustekinumab, leflunomide, hydroxychloroquine, sulfasalazine, abatacept, anakinra, etanercept, rituximab, sarilumab, tocilizumab, baricitinib, tofacitinib, upadacitinib, insulin glulisine, insulin lispro, insulin aspart, insulin glargine, insulin detemir, insulin degludec, fluticasone, budesonide, mometasone furoate, beclomethasone, ciclesonide, montelukast, zafirlukast, zileuton, salmeterol, omalizumab, mepolizumab, benralizumab, reslizumab, etc.

## BRIEF DESCRIPTION OF THE DRAWINGS

FIG. 1 shows an ellipsoid plot of the three-dimensional molecular structure of (*S*)-4-(6-(allyloxy)-2,3-dichlorophenyl) pyrrolidin-2-one.

FIG. 2 shows an ellipsoid plot of the three-dimensional molecular structure of (*R*)-4-(6-(allyloxy)-2,3-dichlorophenyl) pyrrolidin-2-one.

## DETAILED DESCRIPTION

The present invention will be further illustrated with reference to the following specific examples. It should be understood that these examples are merely intended to illustrate the present invention rather than limit the scope of the

present invention.

**Intermediate 1. 3-(6-(Allyloxy)-2,3-dichlorophenyl)-5-hydrazinyl-3,4-dihydro-2*H*-pyrrole**

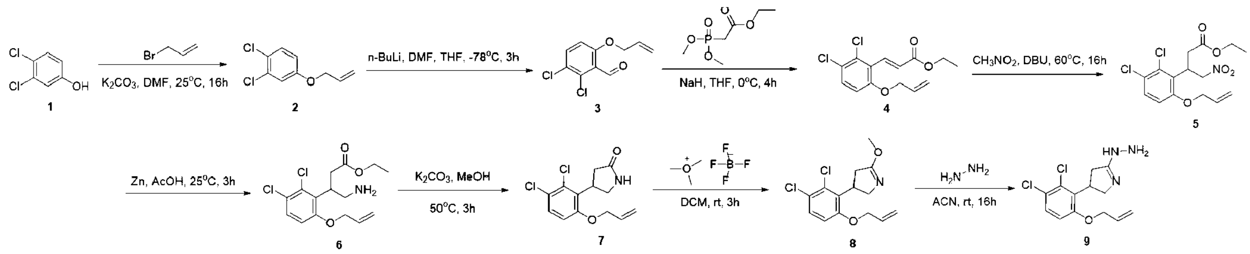


**Synthesis of 4-(allyloxy)-1,2-dichlorobenzene (2):**

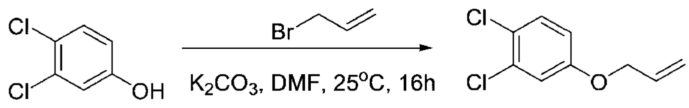


1 2

3,4-Dichlorophenol (120.0 g, 0.74 mol) and potassium carbonate (253.9 g, 1.84 mol) were mixed in *N,N*-dimethylformamide (1.5 L), and allyl bromide (106.9 g, 0.88 mol) was added in portions at 0 °C. The resulting mixture was stirred at room temperature for 16 h. After completion of the reaction, the mixture was diluted with water (1500 mL) and extracted twice with ethyl acetate (1500 mL). The organic phases were combined, washed with saturated brine (1500 mL), and dried over anhydrous sodium sulfate. After filtration, the filtrate was concentrated under reduced pressure, and the resulting residue was purified by silica gel column chromatography (petroleum ether:ethyl acetate = 20:1) to give a yellow solid (120.0 g, yield: 68%).

**Synthesis of 6-(allyloxy)-2,3-dichlorobenzaldehyde (3):**

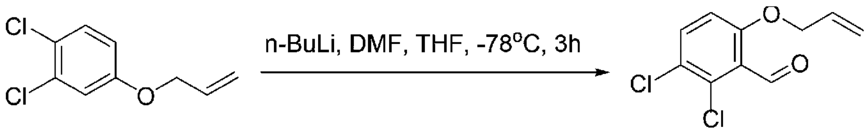


2 3

At -78 °C, 4-(allyloxy)-1.2-dichlorobenzene (120.0 g, 0.59 mol) was dissolved in tetrahydrofuran (1200 mL), and *n*-butyllithium (260.0 mL, 0.65 mol, 2.5 M in *n*-hexane) was added dropwise over a period of 30 min or longer. Then, *N,N*-dimethylformamide (86.4 g, 1.18 mol) was added at the same temperature. The resulting mixture was then stirred at the same temperature for 3 h. After completion of the reaction, the reaction mixture was quenched with a saturated aqueous ammonium chloride solution (1000 mL) and extracted twice with ethyl acetate (3000 mL). The organic phases were combined, washed with saturated brine (1500 mL), dried over anhydrous sodium sulfate, and then concentrated under reduced pressure. The residue was purified by silica gel column chromatography and eluted (petroleum ether: ethyl acetate = 15:1) to give a pale yellow solid (139.5 g, yield: 86%).

$^1$H NMR (400 MHz, DMSO-$d_6$) δ 10.36 (s, 1H), 7.83 (d, *J* = 9.1 Hz, 1H), 7.25 (d, *J* = 9.1 Hz, 1H), 6.13 - 5.96 (m, 1H), 5.46 (m, *J* = 17.3, 1.7 Hz, 1H), 5.31 (m, *J* = 10.6, 3.0, 1.4 Hz, 1H), 4.74 (dt, *J* = 4.9, 1.5 Hz, 2H).

**Synthesis of ethyl (*E*)-3-(6-(allyloxy)-2,3-dichlorophenyl)acrylate (4):**

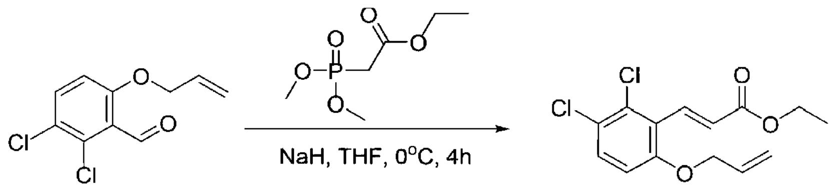


3 4

At 0 °C, sodium hydride (29.1 g, 0.73 mol, 60% in mineral oil) was added to a solution of ethyl 2-(dimethoxyphosphoryl)acetate (142.6 g, 0.73 mol) in tetrahydrofuran (2000 mL). The mixture was stirred for 30 min, and then 6-(allyloxy)-2,3-dichlorobenzaldehyde (139.5 g, 0.61 mol) was added. The resulting mixture was stirred at 0 °C for 4 h. After completion of the reaction, the reaction mixture was diluted with water (2000 mL) and extracted with ethyl acetate (4000 mL). The organic phase was washed with saturated brine (2000 mL), dried over anhydrous sodium sulfate, and concentrated under reduced pressure. The resulting residue was purified by silica gel column chromatography (petroleum ether:ethyl acetate = 5:1) to give a yellow solid (170.0 g, yield: 79%).

$^1$H NMR (400 MHz, DMSO-$d_6$) δ 7.84 (d, *J* = 16.2 Hz, 1H), 7.64 (d, *J* = 9.1 Hz, 1H), 7.17 (d, *J* = 9.2 Hz, 1H), 6.81 (d, *J* = 16.2 Hz, 1H), 6.13 - 6.00 (m, 1H), 5.47 - 5.25 (m, 2H), 4.74 (dt, *J* = 5.2, 1.5 Hz, 2H), 4.21 (q, *J* = 7.1 Hz, 2H), 1.26 (t, *J* = 7.1 Hz, 3H).

**Synthesis of ethyl 3-(6-(allyloxy)-2,3-dichlorophenyl)-4-nitrobutanoate (5):**

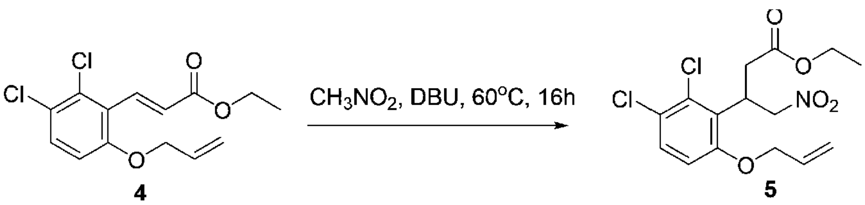


4 5

At room temperature, ethyl (*E*)-3-(6-(allyloxy)-2,3-dichlorophenyl)acrylate (170.0 g, 0.56 mol) and 1,8-diazabicyclo[5.4.0]undec-7-ene (103.1 g, 0.68 mol) were added to a reaction flask containing nitromethane (2000 mL). The reaction mixture was stirred at 60 °C for 16 h. After completion of the reaction, the mixture was diluted with water (1000 mL) and extracted with ethyl acetate (3000 mL). The organic phase was washed with saturated brine (1000 mL), dried over anhydrous sodium sulfate, and then concentrated under reduced pressure. The residue was purified by silica gel column chromatography (petroleum ether:ethyl acetate = 5:1) to give a pale yellow oily substance (174.0 g, yield: 72%).

$^1$H NMR (400 MHz, DMSO-$d_6$) δ 7.53 (d, *J* = 9.0 Hz, 1H), 7.06 (d, *J* = 9.1 Hz, 1H), 6.07 (ddd, *J* = 15.9, 10.6, 5.2 Hz, 1H), 5.41 (d, *J* = 17.2 Hz, 1H), 5.33 (dd, *J* = 10.6, 1.1 Hz, 1H), 5.00 (ddd, *J* = 19.0, 13.0, 7.5 Hz, 2H), 4.69 (d, *J* = 4.4 Hz, 3H), 4.04 - 3.91 (m, 2H), 2.89 (dq, *J* = 17.6, 8.7 Hz, 2H), 1.06 (t, *J* = 7.1 Hz, 3H).

**Synthesis of ethyl 3-(6-(allyloxy)-2,3-dichlorophenyl)-4-aminobutanoate (6):**

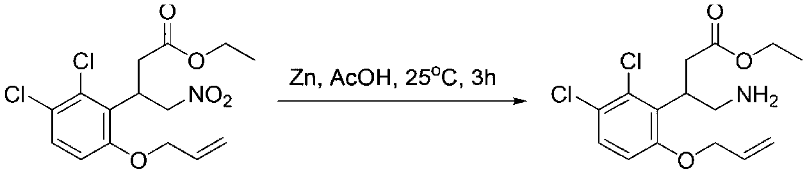


5 6

At room temperature, ethyl 3-(6-(allyloxy)-2,3-dichlorophenyl)-4-nitrobutanoate (174.0 g, 0.48 mol) and zinc powder (314.2 g, 4.80 mol) were added to a reaction flask containing glacial acetic acid (2000 mL). The mixture was stirred at 25 °C for 3 h. After completion of the reaction, the reaction mixture was filtered, and the filter cake was washed twice with water and ethyl acetate. The filtrate was collected and concentrated under reduced pressure to give a crude product (200.0 g), which was directly used in the next step without purification.

LCMS $[M+H]^+$: 332.1.

**Synthesis of 4-(6-(allyloxy)-2,3-dichlorophenyl)pyrrolidin-2-one (7):**

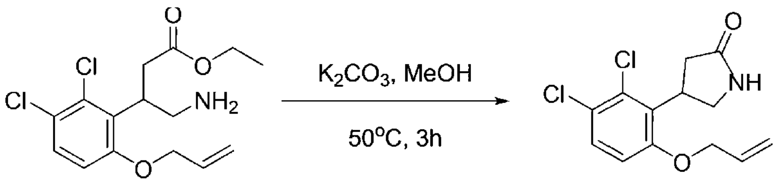


6 7

The crude product of ethyl 3-(6-(allyloxy)-2,3-dichlorophenyl)-4-aminobutanoate (200.0 g, 0.45 mol) and potassium carbonate (311.5 g, 2.26 mol) were added to methanol (2000 mL), and the mixture was stirred at 50 °C for 3 h. After completion of the reaction, the reaction mixture was diluted with water (1000 mL) and extracted three times with ethyl acetate (3000 mL). The organic phases were combined and then dried over anhydrous sodium sulfate. After filtration, the filtrate was concentrated under reduced pressure. The residue was purified by silica gel column chromatography (petroleum ether:ethyl acetate = 5:1) to give a white solid (105.0 g, yield: 69%).

LCMS $[M+H]^+$: 286.0.

$^1$H NMR (400 MHz, DMSO-$d_6$) δ 7.75 (s, 1H), 7.51 (d, *J* = 9.0 Hz, 1H), 7.08 (d, *J* = 9.0 Hz, 1H), 6.01 (ddt, *J* = 17.2, 10.4, 5.0 Hz, 1H), 5.44 - 5.35 (m, 1H), 5.26 (dd, *J* = 10.6, 1.5 Hz, 1H), 4.64 (dd, *J* = 3.6, 1.4 Hz, 2H), 4.33 (dd, *J* = 18.4, 9.2 Hz, 1H), 3.43 (dt, *J* = 16.7, 8.9 Hz, 2H), 2.54 (dd, *J* = 16.7, 9.0 Hz, 1H), 2.36 (dd, *J* = 16.5, 10.7 Hz, 1H).

**Synthesis of 3-(6-(allyloxy)-2,3-dichlorophenyl)-5-methoxy-3,4-dihydro-2*H*-pyrrole (8):**

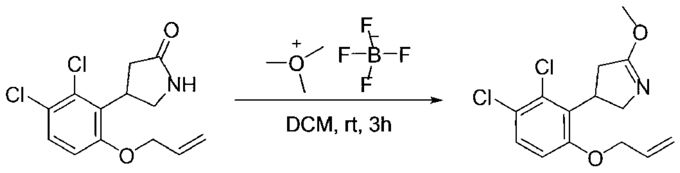


7 8

4-(6-(Allyloxy)-2,3-dichlorophenyl)pyrrolidin-2-one (500.0 mg, 1.75 mmol) was dissolved in dichloromethane (5 mL), and trimethyloxonium tetrafluoroborate (311.6 mg, 2.11 mmol) was added. The mixture was stirred at room temperature for 3 h under a nitrogen atmosphere. After completion of the reaction, the reaction mixture was directly rotary evaporated to dryness, and the resulting crude product was directly used in the next step without purification.

**Synthesis of 3-(6-(allyloxy)-2,3-dichlorophenyl)-5-hydrazinyl-3,4-dihydro-2*H*-pyrrole (9):**

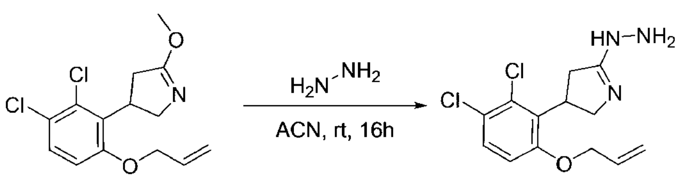


8 9

A solution of 3-(6-(allyloxy)-2,3-dichlorophenyl)-5-methoxy-3,4-dihydro-2*H*-pyrrole (500 mg, 1.67 mmol) and hydrazine hydrate (267.0 mg, 6.67 mmol, 80% in water) in acetonitrile (8.0 mL) was stirred at room temperature for 16 h. After completion of the reaction, the acetonitrile was removed by distillation under reduced pressure. The resulting crude product was purified by silica gel chromatography (methanol:dichloromethane = 1:20) to give a white solid (440.0 mg,

yield: 88%).

LCMS $[M+H]^+$: 299.9.

**Intermediate 2. (*S*)-3-(6-(Allyloxy)-2,3-dichlorophenyl)-5-methoxy-3,4-dihydro-2*H*-pyrrole**

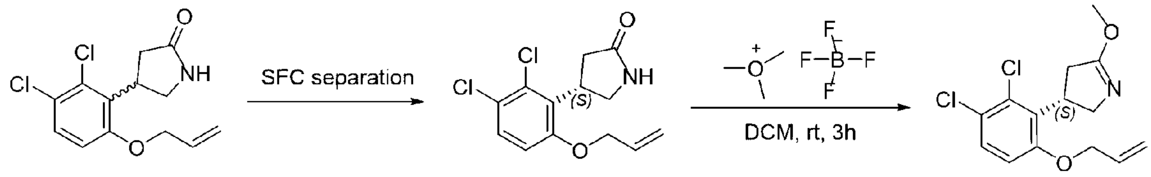


1 2 4

**Preparation of (*S*)-4-(6-(allyloxy)-2,3-dichlorophenyl)pyrrolidin-2-one (2):**

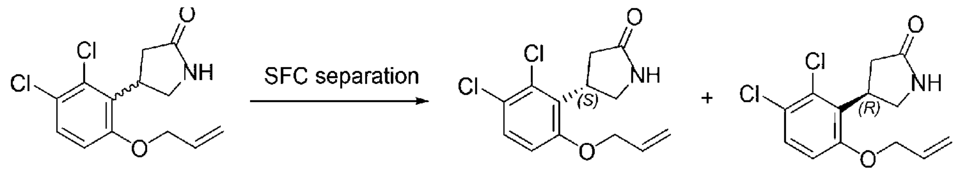


1 2 3

4-(6-(Allyloxy)-2,3-dichlorophenyl)pyrrolidin-2-one (10 g) was taken and purified by preparative chiral chromatography (gradient column: CHIRALPAK IC 250 mm × 4.6 mm, 5 μm, flow rate:3 mL/min, gradient: 40% IPA ($NH_4OH$ 0.2%): 60% $CO_2$).

The first compound obtained from the SFC resolution was Compound 2 (4.5 g, yield: 45%), with its RT of 4.51 min analyzed under the analytical chromatographic conditions of SFC.

LC-MS: $[M+H]^+$: 286.0.

The second compound obtained from the SFC resolution was Compound 3 (4.3 g, yield: 43%),with its RT of 6.5 min analyzed under the analytical chromatographic conditions of SFC.

LC-MS: $[M+H]^+$: 286.0.

**Single crystal X-ray diffraction detection analysis of Compound 2 and Compound 3**

Preparation of single crystals: Single crystals of Compound 2 and Compound 3 were obtained through solvent diffusion crystallization under ethyl acetate/*n*-hexane conditions, with crystal growth proceeding at room temperature for 2 days.

Single crystal detection instrument model: Bruker D8 Venture

Instrument parameters:

| | |
|---|---|
| Light source: Cu target | X-ray: Cu-Kα (λ= 1.54178 Å) |
| Detector: CMOS area detector | Resolution: 0.86 Å |

Current and voltage:

| | | | |
|---|---|---|---|
| 50 kV, 1.2 mA | | Exposure time: | 5 s |
| Area detector-to-sample distance: | 40 mm | Test temperature: | 100(2) K |
| Structure solution and refinement process | | | |

The diffraction data were integrated and reduced using the SAINT program, followed by empirical absorption correction using the SADABS program. The single crystal structure was solved by the direct method using SHELXT2014

and refined by the least square method. The hydrogen atom refinement process was conducted by isotropic calculation. The hydrogen atoms on C-Hs were positioned by computational hydrogen addition and refined using a riding model. The Flack parameter for the single crystal of Compound 2 was 0.087 (13), confirming the *S*-configuration at C9. The Flack parameter for the single crystal of compound 3 was 0.073 (13), confirming the *R*-configuration at C3. The ellipsoid plots of the three-dimensional molecular structures of Compounds 2 and 3 are shown in FIG. 1 and FIG. 2, respectively. The crystal structure data for Compound 2 are shown in Table 1, and the crystal structure data for Compound 3 are shown in Table 2.

Table 1. Crystal structure data for Compound 2

| | |
|---|---|
| $C_{13}H_{13}Cl_2NO_2$ | Z = 4 |
| $M_r$ = 286.14 | *F*(000) = 592 |
| Triclinic, *P*1 | $D_x$ = 1.458 Mg $m^{-3}$ |
| *a* = 8.1337 (3) Å | Cu *K*a radiation, 1 = 1.54178 Å |
| *b* = 12.0320 (4) Å | Cell parameters from 9892 reflections |
| *c* = 14.0408 (5) Å | q = 3.9-63.7° |
| *a* = 71.569 (1)° | m = 4.43 $mm^{-1}$ |
| *b* = 89.604 (1)° | *T* = 100 K |
| *g* = 89.392 (1)° | Block, colourless |
| *V* = 1303.53 (8) $Å^3$ | 0.15 × 0.08 × 0.05 mm |

Table 2. Crystal structure data for Compound 3

| | |
|---|---|
| $C_{13}H_{13}Cl_2NO_2$ | Z = 4 |
| $M_r$ = 286.14 | *F*(000) = 592 |
| Triclinic, *P*1 | $D_x$ = 1.458 Mg $m^{-3}$ |
| *a* = 8.1370 (2) Å | Cu *K*a radiation, l = 1.54178 Å |
| *b* = 12.0239 (3) Å | Cell parameters from 9846 reflections |
| *c* = 14.0409 (3) Å | q = 3.3-63.7° |
| *a* = 71.591 (1)° | m = 4.43 $mm^{-1}$ |
| *b* = 89.548 (1)° | *T* = 100 K |
| *g* = 89.353 (1)° | Block, colourless |
| *V* = 1303.34 (5) $Å^3$ | 0.15 × 0.08 × 0.05 mm |

**Synthesis of (S)-3-(6-(allyloxy)-2,3-dichlorophenyl)-5-methoxy-3,4-dihydro-2*H*-pyrrole (4):**

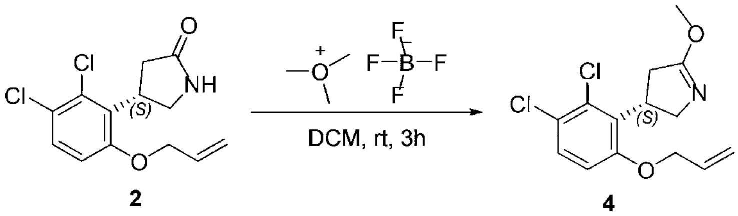


(*S*)-4-(6-(Allyloxy)-2,3-dichlorophenyl)pyrrolidin-2-one (1.0 g, 3.5 mmol) was dissolved in dichloromethane (10 mL), and trimethyloxonium tetrafluoroborate (623.4 mg, 4.23 mmol) was added. The mixture was stirred at room temperature for 3 h under a nitrogen atmosphere. After completion of the reaction, the reaction mixture was directly rotary evaporated to dryness, and the resulting crude product was directly used in the next step without purification.

**Intermediate 3. 1-(2-Hydroxyethyl)-1*H*-pyrazole-4-carbohydrazide**

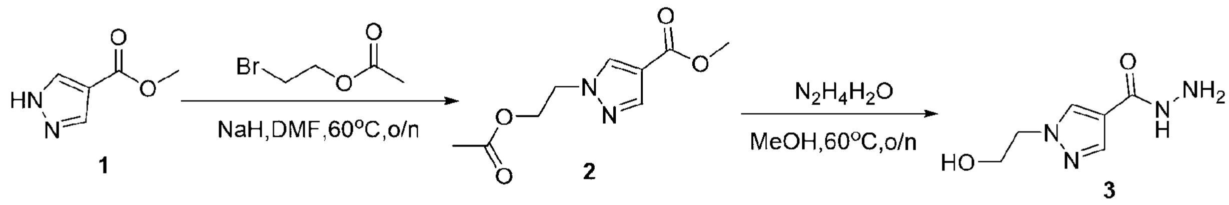

1 2 3

**Synthesis of methyl 1-(2-acetoxyethyl)-1*H*-pyrazole-4-carboxylate (2):**

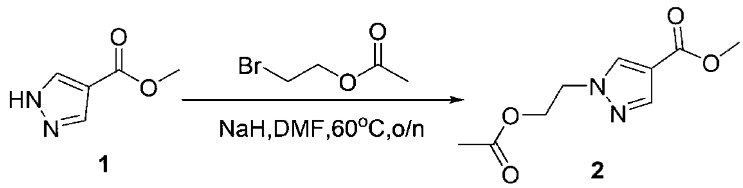

1 2

Methyl 1*H*-pyrazole-4-carboxylate (600.0 mg, 4.75 mmol) was dissolved in *N,N*-dimethylformamide (5.0 mL), and sodium hydride (666.6 mg, 16.65 mmol, 60% in mineral oil) was added in portions. The reaction mixture was stirred at room temperature for 20 min, and further stirred at 60 °C overnight after 2-bromoethyl acetate (2.8 g, 16.65 mmol) was added. After completion of the reaction, the *N,N*-dimethylformamide was removed by lyophilization under reduced pressure, and the resulting crude product was purified by silica gel chromatography (methanol:dichloromethane = 1:10) to give a white solid (500.0 mg, yield: 49%).
LC-MS: $[M+H]^+$: 213.1.

**Synthesis of 1-(2-hydroxyethyl)-1*H*-pyrazole-4-carbohydrazide (3):**

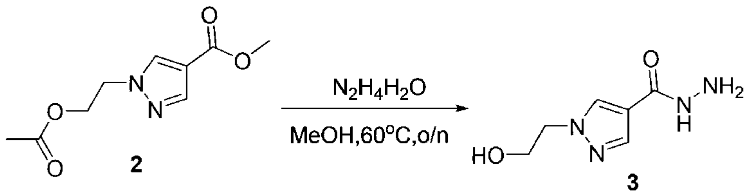

2 3

A solution of methyl 1-(2-acetoxyethyl)-1*H*-pyrazole-4-carboxylate (500 mg, 2.35 mmol) and hydrazine hydrate (353.8 g, 8.84 mmol, 80% in water) in methanol (10.0 mL) was stirred at 60 °C for 12 h. The methanol was then removed by distillation under reduced pressure. The resulting crude product was purified by silica gel chromatography (methanol:dichloromethane = 1:10) to give a white solid (200.0 mg, yield: 40%).
LC-MS: $[M+H]^+$: 171.1.

**Examples 1-2. (*R*)-3,4-Dichloro-2-(3-(hydroxymethyl)-6,7-dihydro-5*H*-pyrrolo[2,1-*c*][1,2,4]triazol-6-yl)phenol and (*S*)-3,4-dichloro-2-(3-(hydroxymethyl)-6,7-dihydro-5*H*-pyrrolo[2,1-*c*][1,2,4]triazol-6-yl)phenol**

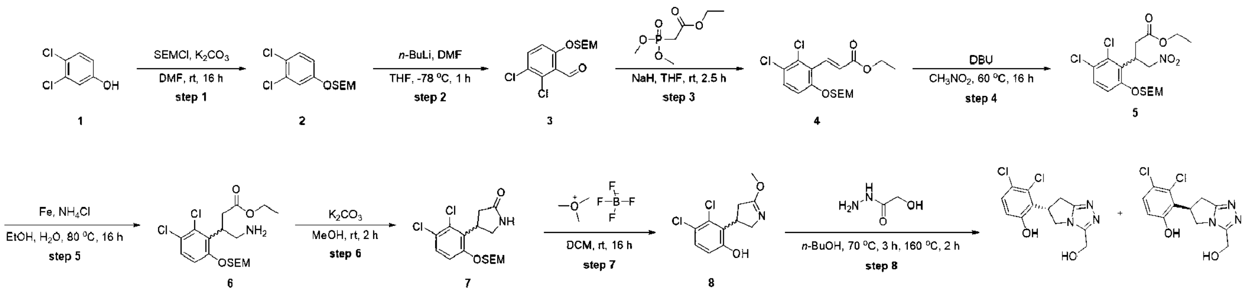


**Synthesis of (2-((3,4-dichlorophenoxy)methoxy)ethyl)trimethylsilane (2):**

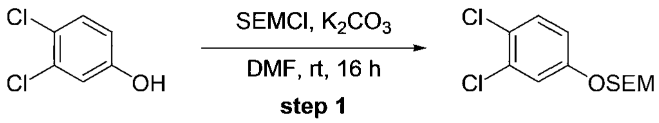


1 2

3,4-Dichlorophenol (44.0 g, 0.27 mol) and potassium carbonate (74.5 g, 0.54 mol) were mixed in *N,N*-dimethylformamide (1 L), and 2-(trimethylsilyl)ethoxymethyl chloride (54.0 g, 0.33 mol) was added in portions with stirring at 0 °C. The resulting mixture was stirred at room temperature for 16 h. After completion of the reaction, the reaction mixture was diluted with water (500 mL) and extracted twice with ethyl acetate (500 mL). The organic phases were combined, washed with saturated brine (600 mL), and dried over anhydrous sodium sulfate. After filtration, the filtrate was concentrated under reduced pressure, and the resulting residue was purified by silica gel column chromatography and eluted with petroleum ether/ethyl acetate (15/1) to give a colorless oily subtance (65.1 g, yield: 82%).

$^1H$ NMR (400 MHz, $CDCl_3$) δ 7.31 (d, *J* = 8.9 Hz, 1H), 7.16 (d, *J* = 2.8 Hz, 1H), 6.88 (dd, *J* = 8.9, 2.8 Hz, 1H), 5.17 (s, 2H), 3.72 (dd, *J* = 8.9, 7.8 Hz, 2H), 0.94 (dd, *J* = 8.9, 7.8 Hz, 2H), -0.01 (d, *J* = 3.3 Hz, 9H).

**Synthesis of 2,3-dichloro-6-((2-(trimethylsilyl)ethoxy)methoxy)benzaldehyde (3):**

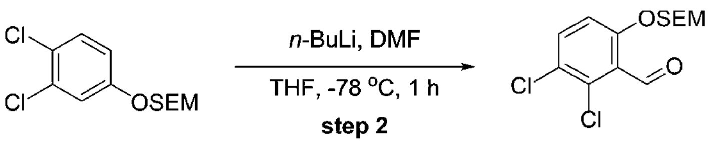


2 3

At -78 °C, (2-((3,4-dichlorophenoxy)methoxy)ethyl)trimethylsilane (10.0 g, 34.10 mmol) was dissolved in tetrahydrofuran (100 mL), and *n*-butyllithium (15.0 mL, 36.00 mmol, 2.4 M in *n*-hexane) was added dropwise over a period of 30 min or longer. Then, *N,N*-dimethylformamide (5.0 g, 68.50 mmol) was added at the same temperature. The mixture was then stirred for 1 h. After completion of the reaction, the reaction mixture was quenched with a saturated aqueous ammonium chloride solution (100 mL) and extracted twice with ethyl acetate (300 mL). The organic phases were combined, washed with saturated brine (300 mL), dried over anhydrous sodium sulfate, and then concentrated under reduced pressure. The residue was purified by silica gel column chromatography and eluted with petroleum ether/ethyl acetate (10/1) to give a pale yellow solid (10.2 g, yield: 91%).

$^1H$ NMR (400 MHz, $CDCl_3$) δ 10.45 (s, 1H), 7.54 (d, *J* = 9.1 Hz, 1H), 7.17 (d, *J* = 9.1 Hz, 1H), 5.30 (s, 2H), 3.76 (dd, *J* = 11.3, 5.4 Hz, 2H), 0.94 (dd, *J* = 11.0, 5.7 Hz, 2H), -0.00 (s, 9H).

**Synthesis of ethyl (E)-3-(2,3-dichloro-6-((2-(trimethylsilyl)ethoxy)methoxy)phenyl)acrylate (4):**

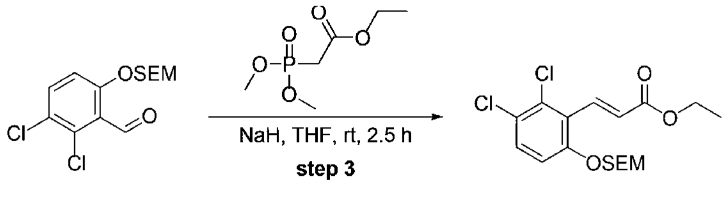


3 4

At 0 °C, sodium hydride (1.4 g, 35.00 mmol, 60% in mineral oil) was added to a solution of ethyl 2-(dimethoxyphosphoryl)acetate (6.7 g, 34.16 mmol) in tetrahydrofuran (100 mL). The mixture was stirred for 30 min, and then 2,3-dichloro-6-((2-(trimethylsilyl)ethoxy)methoxy)benzaldehyde (9.2 g, 28.63 mmol) was added. The resulting mixture was stirred at room temperature for 2.5 h. After completion of the reaction, the reaction mixture was diluted with water (100 mL) and extracted with ethyl acetate (200 mL). The organic phase was washed with saturated brine (200 mL), dried over anhydrous sodium sulfate, and concentrated under reduced pressure. The resulting residue was purified by silica gel column chromatography and eluted (petroleum ether:ethyl acetate = 5:1) to give a white solid (10.0 g, yield: 89%).

$^1$H NMR (400 MHz, $CDCl_3$) δ 7.93 (d, *J* = 16.2 Hz, 1H), 7.34 (d, *J* = 9.1 Hz, 1H), 7.08 (d, *J* = 9.1 Hz, 1H), 6.76 (d, *J* = 16.2 Hz, 1H), 5.26 (s, 2H), 4.26 (q, *J* = 7.1 Hz, 2H), 3.76 - 3.68 (m, 2H), 1.33 (t, *J* = 7.1 Hz, 3H), 0.95 - 0.88 (m, 2H), -0.02 - -0.04 (m, 9H).

**Synthesis of ethyl 3-(2,3-dichloro-6-((2-(trimethylsilyl)ethoxy)methoxy)phenyl)-4-nitrobutanoate (5):**

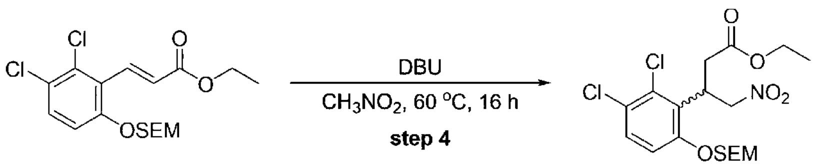


4 5

At room temperature, ethyl (*E*)-3-(2,3-dichloro-6-((2-(trimethylsilyl)ethoxy)methoxy)phenyl)acrylate (3.0 g, 7.67 mmol) and 1,8-diazabicyclo[5.4.0]undec-7-ene (1.4 g, 9.52 mmol) were added to a reaction flask containing nitromethane (50 mL). The reaction mixture was stirred at 60 °C for 16 h. After completion of the reaction, the mixture was diluted with water (50 mL) and extracted with ethyl acetate (150 mL). The organic phase was washed with saturated brine (100 mL), dried over anhydrous sodium sulfate, and then concentrated under reduced pressure. The residue was purified by silica gel column chromatography and eluted (petroleum ether:ethyl acetate = 5:1) to give a pale yellow oily substance (2.5 g, yield: 73%).

$^1$H NMR (400 MHz, $CDCl_3$) δ 7.30 (d, *J* = 9.1 Hz, 1H), 7.04 (d, *J* = 9.0 Hz, 1H), 5.25 (s, 2H), 4.94 - 4.75 (m, 3H), 4.07 (q, *J* = 7.1 Hz, 2H), 3.80 - 3.71 (m, 2H), 2.86 (d, *J* = 3.8 Hz, 2H), 1.16 (dd, *J* = 7.8, 6.5 Hz, 3H), 0.97 = 0.92 (m, 2H), 0.00 - -0.01 (m, 9H).

**Synthesis of ethyl 3-(2,3-dichloro-6-((2-(trimethylsilyl)ethoxy)methoxy)phenyl)-4-aminobutanoate (6):**

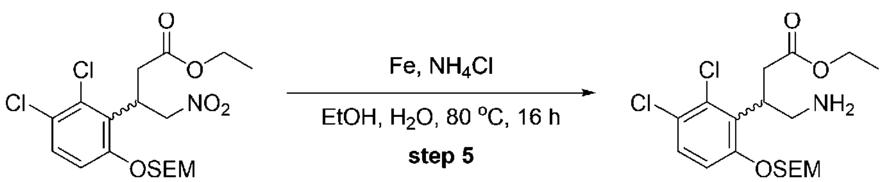


5 6

At room temperature, ethyl 3-(2,3-dichloro-6-((2-(trimethylsilyl)ethoxy)methoxy)phenyl)-4-nitrobutanoate (5.1 g, 11.27 mmol), iron powder (3.1 g, 56.36 mmol), and ammonium chloride (5.9 g, 0.11 mol) were added to a reaction flask containing ethanol and water (50 mL + 10 mL). The mixture was stirred at 80 °C for 16 h. After completion of the reaction, the reaction mixture was filtered, and the filtrate was diluted with water (50 mL) and extracted with ethyl acetate (50 mL).

The organic phase was washed with saturated brine (50 mL), dried over anhydrous sodium sulfate, and then concentrated under reduced pressure to give a crude product (4.0 g), which was directly used in the next step without purification.

LCMS $[M+H]^+$: 422.0.

**Synthesis of 4-(2,3-dichloro-6-((2-(trimethylsilyl)ethoxy)methoxy)phenyl)pyrrolidin-2-one (7):**

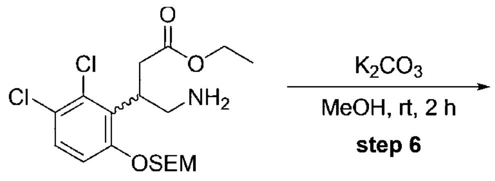


**6** **7**

Ethyl 3-(2,3-dichloro-6-((2-(trimethylsilyl)ethoxy)methoxy)phenyl)-4-aminobutanoate (4.0 g, 9.46 mmol) and potassium carbonate (3.2 g, 23.19 mmol) were added to methanol (30 mL), and the mixture was stirred at room temperature for 2 h. After completion of the reaction, the reaction mixture was diluted with water (50 mL) and extracted three times with ethyl acetate (50 mL). The organic phases were combined and then dried over anhydrous sodium sulfate. After filtration, the filtrate was concentrated under reduced pressure. The residue was purified by silica gel column chromatography and eluted (petroleum ether:ethyl acetate = 5:1) to give a pale yellow oily substance (2.4 g, two-step yield: 57%).

LCMS $[M+H]^+$: 398.0.

$^1H$ NMR (400 MHz, $CDCl_3$) δ 7.34 (d, *J*= 9.0 Hz, 1H), 7.11 (d, *J*= 9.0 Hz, 1H), 6.61 (s, 1H), 5.27 (s, 2H),4.64-4.50 (m, 1H), 3.78-3.71 (m, 2H), 3.63 (dt, *J*= 29.9, 8.8 Hz, 2H), 2.78 (dd, *J*= 17.0, 8.3Hz, 1H), 2.61 (dd, *J*= 17.0, 10.8 Hz, 1H), 0.98-0.92 (m, 2H), 0.02 (s, 9H).

**Synthesis of 3,4-dichloro-2-(5-methoxy-3,4-dihydro-2*H*-pyrrol-3-yl)phenol (8):**

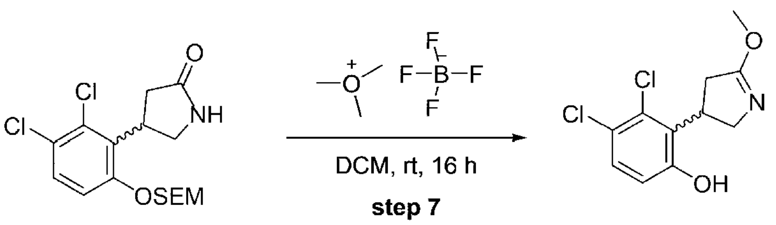


**7** **8**

4-(2,3-Dichloro-6-((2-(trimethylsilyl)ethoxy)methoxy)phenyl)pyrrolidin-2-one (200.0 mg, 0.53 mmol) and trimethyloxonium tetrafluoroborate (141.9 mg, 0.96 mmol) were dissolved in dichloromethane (6.6 mL), and the mixture was stirred at room temperature for 16 h. The resulting mixture was directly rotary evaporated to dryness to give a crude product (330.0 mg) as a colorless oily substance, which was directly used in the next step without further purification.

LC-MS: $[M+H]^+$: 260.0.

**Synthesis of (*R*)-3,4-Dichloro-2-(3-(hydroxymethyl)-6,7-dihydro-5*H*-pyrrolo[2,1-c][1,2,4]triazol-6-yl)phenol and (*S*)-3,4-dichloro-2-(3-(hydroxymethyl)-6,7-dihydro-5*H*-pyrrolo[2,1-c][1,2,4]triazol-6-yl)phenol:**

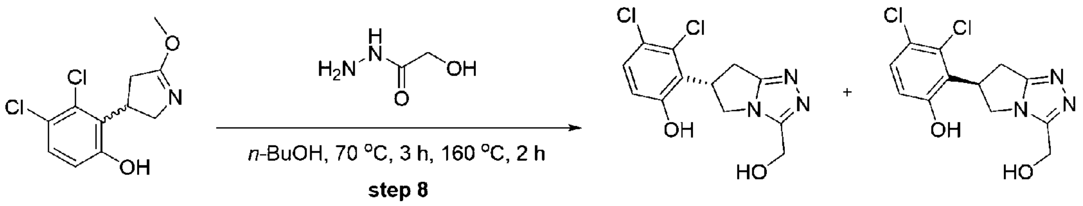


8

The crude product of 3,4-dichloro-2-(5-methoxy-3,4-dihydro-2*H*-pyrrol-3-yl)phenol (330.0 mg, about 0.53 mmol) and hydroxyacetohydrazide (143.0 mg, 1.59 mmol) were dissolved in *n*-butanol (3.0 mL), and the mixture was stirred at 70 °C for 3 h. The mixture was then directly transferred to a microwave tube and allowed to react under microwave heating conditions at 160 °C for 2 h. After the reaction system was cooled to room temperature, the mixture was rotary evaporated to dryness. The residue was then separated by preparative chromatography to give a racemic compound (31.0 mg, yield: 19%) as a white solid, which was further subjected to SFC resolution to give a pair of enantiomers.

SFC chiral resolution conditions:

Instrument: Thar Prep SFC 80
Chiral chromatographic column: CHIRALPAK® AD-H, 250 mm × 20 mm, 5μm
Mobile phase: $CO_2$-EtOH (DEA), gradient: 0-40%
Total flow rate: 40 g/min

SFC chiral analysis conditions:

Instrument: Thar SFC X-5
Chiral chromatographic column: CHIRALPAK® AD-H, 250 mm × 4.6 mm, 5μm
Mobile phase: $CO_2$-EtOH (DEA), gradient: 0-40%
Total flow rate: 2.5 mL/min

The first compound obtained from the SFC resolution was designated as Example 1, with its RT of 1.69 min analyzed under the analytical chromatographic conditions of SFC.

LC-MS: $[M+H]^+$: 299.9.

$^1$H NMR: (400 MHz, DMSO-*d6*) δ 10.59 (s, 1H), 7.40 (d, *J* = 8.8 Hz, 1H), 6.87 (d, *J* = 8.8 Hz, 1H), 5.50 (t, *J* = 5.0 Hz, 1H), 5.03 - 4.89 (m, 1H), 4.54 (d, *J* = 4.7 Hz, 2H), 4.26 (t, *J* = 10.0 Hz, 1H), 4.11 (dd, *J* = 10.5, 7.9 Hz, 1H), 3.15 - 3.06 (m, 2H).

The second compound obtained from the SFC resolution was designated as Example 2, with its RT of 2.16 min analyzed under the analytical chromatographic conditions of SFC.

LC-MS: $[M+H]^+$: 299.9.

$^1$H NMR: (400 MHz, DMSO-*d6*) δ 10.65 (s, 1H), 7.39 (d, *J* = 8.8 Hz, 1H), 6.86 (d, *J* = 8.8 Hz, 1H), 5.50 (s, 1H), 5.01 - 4.91 (m, 1H), 4.54 (s, 2H), 4.26 (t, *J* = 10.0 Hz, 1H), 4.11 (dd, *J* = 10.5, 7.9 Hz, 1H), 3.11 (dd,*J* = 9.0, 4.0 Hz, 2H).

**Example 3. 3,4-Dichloro-2-(3-(2-hydroxy-2-methylpropyl)-6,7-dihydro-5*H*-pyrrolo[2,1-*c*][1,2,4]triazol-6-yl)phenol**

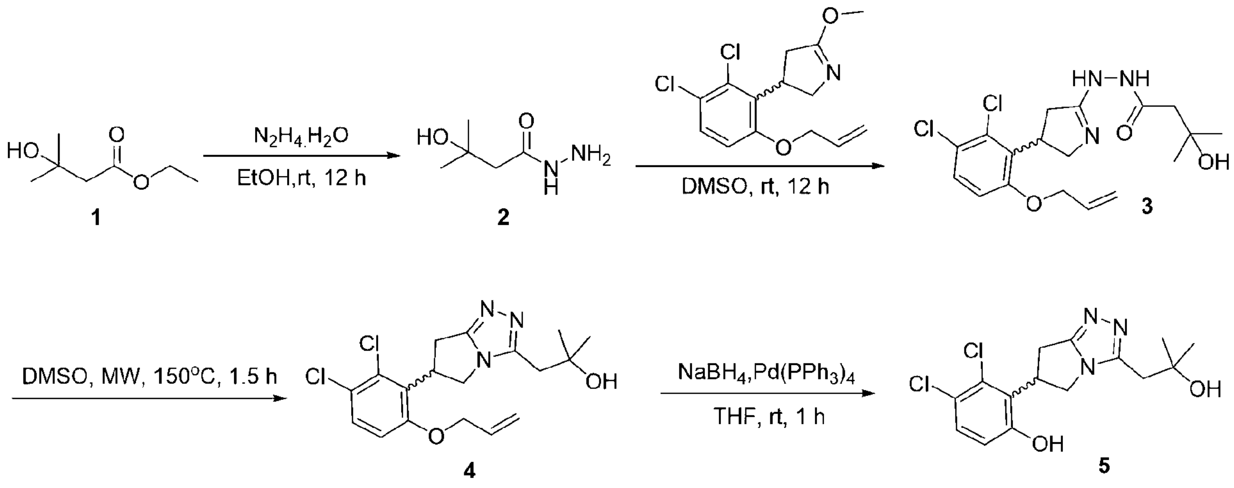


1 2 3 4 5

**Synthesis of 3-hydroxy-3-methylbutanehydrazide (2):**

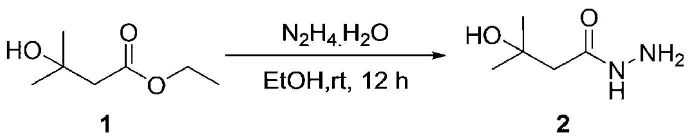


1 2

A solution of ethyl 3-hydroxy-3-methylbutanoate (2000.0 mg, 13.70 mmol) and hydrazine hydrate (1041.1 mg, 26.03 mmol, 80% in water) in ethanol (15.0 mL) was stirred at room temperature for 12 h. After completion of the reaction, the resulting mixture was diluted by adding dichloromethane (20.0 mL), resulting in the precipitation of a product. The mixture was filtered to give a white solid (620.0 mg, yield: 34%).

LCMS $[M+H]^+$: 133.1.

**Synthesis of *N'*-(3-(6-(allyloxy)-2,3-dichlorophenyl)-3,4-dihydro-2*H*-pyrrol-5-yl)-3-hydroxy-3-methylbutanehydrazide (3):**

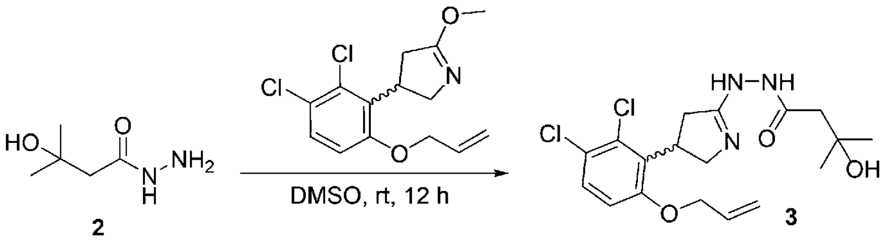


2 3

A solution of 3-hydroxy-3-methylbutanehydrazide (66.2 mg, 0.50 mmol) and 3-(6-(allyloxy)-2,3-dichlorophenyl)-5-methoxy-3,4-dihydro-2H-pyrrole (50.0 mg, 0.17 mmol) in dimethylsulfoxide (2.0 mL) was stirred at room temperature for 12 h. After completion of the reaction, the reaction mixture was directly used in the next step without purification.

LCMS $[M+H]^+$: 400.1.

**Synthesis of 1-(6-(6-(allyloxy)-2,3-dichlorophenyl)-6,7-dihydro-5*H*-pyrrolo[2,1-*c*][1,2,4]triazol-3-yl)-2-methylpropan-2-ol (4):**

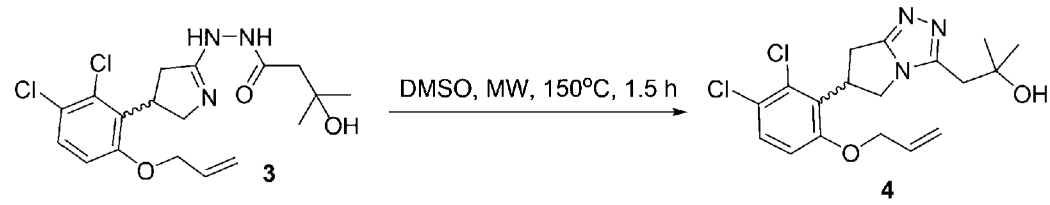

3 4

A solution of *N'*-(3-(6-(allyloxy)-2,3-dichlorophenyl)-3,4-dihydro-2*H*-pyrrol-5-yl)-3-hydroxy-3-methylbutanehydrazide in dimethylsulfoxide was transferred to a microwave tube and then stirred under microwave at 150 °C for 1.5 h. After completion of the reaction, the dimethylsulfoxide was removed by lyophilization under reduced pressure. The resulting crude product was purified by silica gel chromatography (methanol:dichloromethane = 1:10) to give a white solid (30.0 mg, yield: 47%).

LCMS $[M+H]^+$: 382.0.

**Synthesis of 3,4-dichloro-2-(3-(2-hydroxy-2-methylpropyl)-6,7-dihydro-5*H*-pyrrolo[2,1-c][1,2,4]triazol-6-yl)phenol (5):**

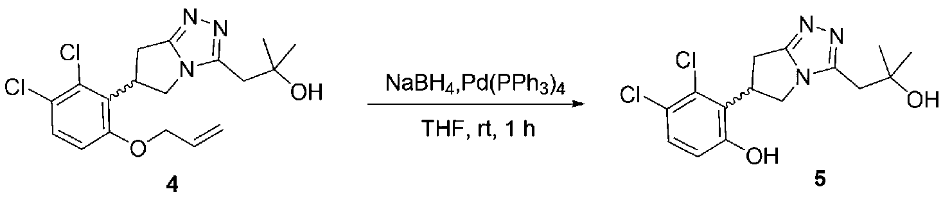

4 5

At room temperature, tetrakis(triphenylphosphine)palladium(0) (7.6 mg, 0.01 mmol) and sodium borohydride (3.7 mg, 0.10 mmol) were added to a solution of 1-(6-(6-(allyloxy)-2,3-dichlorophenyl)-6,7-dihydro-5*H*-pyrrolo[2,1-*c*][1,2,4]triazol-3-yl)-2-methylpropan-2-ol (25.0 mg, 0.07 mmol) in tetrahydrofuran (1.0 mL). The mixture was stirred at room temperature for 1 h. After completion of the reaction, the reaction mixture was filtered through an organic-phase filter membrane, and the filtrate was rotary evaporated to dryness. The crude product was purified by preparative chromatography to give a white solid (3.0 mg, yield: 13%).

LCMS $[M+H]^+$: 342.1.

$^1$H NMR (400 MHz, DMSO-$d_6$) δ 10.73 (s, 1H), 8.36 (s, 1H), 7.39 (d, *J* = 8.8 Hz, 1H), 6.86 (d, *J* = 8.9 Hz, 1H), 4.95 - 4.88 (m, 1H), 4.22 (t, *J* = 10.0 Hz, 1H), 4.13 - 4.06 (m, 1H), 3.10 (d, *J* = 9.0 Hz, 2H), 2.73 (s, 2H), 1.23 (s, 3H), 1.14 (d, *J* = 10.3 Hz, 3H).

**Example 4. 2-(6-(2,3-Dichloro-6-hydroxyphenyl)-6,7-dihydro-5*H*-pyrrolo[2,1-*c*][1,2,4]triazol-3-yl)-2-methylpropanenitrile**

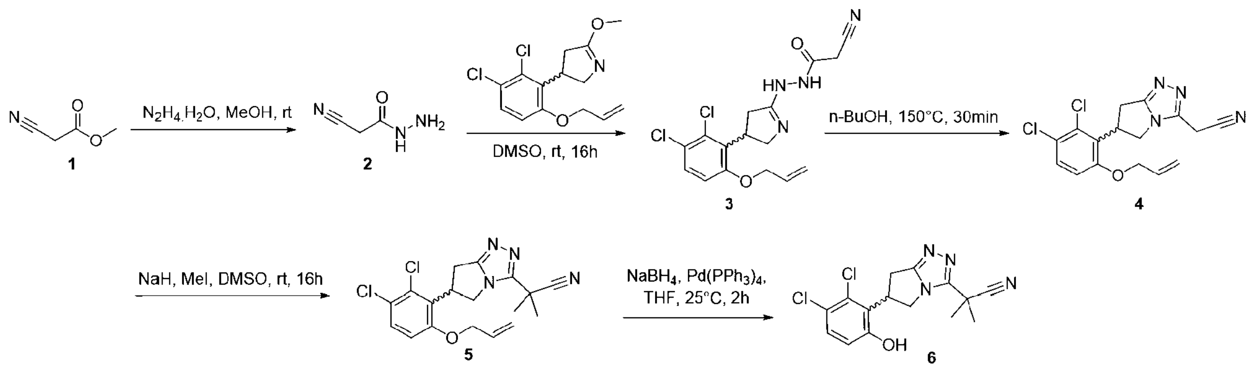

1 2 3 4 5 6

**Synthesis of cyanoacetohydrazide (2):**

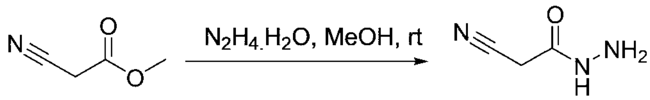


1 2

A solution of methyl cyanoacetate (5.0 g, 50.51 mmol) and hydrazine hydrate (4.85 g, 121.25 mmol, 80% in water) in methanol (50.0 mL) was stirred at room temperature for 12 h. After completion of the reaction, the resulting mixture was concentrated under reduced pressure and then diluted by adding dichloromethane (20.0 mL), resulting in the precipitation of a product. The mixture was filtered to give a white solid (4.4 g, yield: 88%).
LCMS $[M+H]^+$: 100.1.

**Synthesis of *N*'-(3-(6-(allyloxy)-2,3-dichlorophenyl)-3,4-dihydro-2*H*-pyrrol-5-yl)-2-cyanoacetohydrazide** (3):

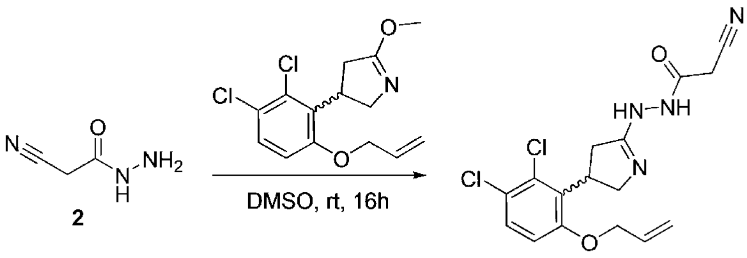


2 3

A solution of cyanoacetohydrazide (297.0 mg, 3.00 mmol) and 3-(6-(allyloxy)-2,3-dichlorophenyl)-5-methoxy-3,4-dihydro-2H-pyrrole (300.0 mg, 1.00 mmol) in dimethylsulfoxide (2.0 mL) was stirred at room temperature for 16 h. After completion of the reaction, the product was obtained as a solution in dimethylsulfoxide, which was directly used in the next step without purification.
LCMS $[M+H]^+$: 367.0.

**Synthesis** of **2-(6-(6-(allyloxy)-2,3-dichlorophenyl)-6,7-dihydro-5*H*-pyrrolo[2,1-*c*][1,2,4]triazol-3-yl)-acetonitrile** (4):

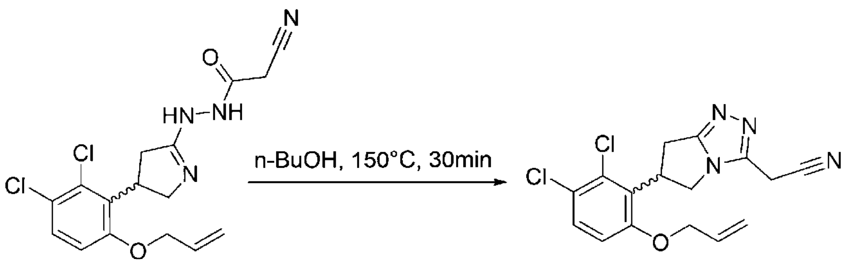


3 4

A solution of *N*'-(3-(6-(allyloxy)-2,3-dichlorophenyl)-3,4-dihydro-2*H*-pyrrol-5-yl)-2-cyanoacetohydrazide in butanol was transferred to a microwave tube and then stirred under microwave at 150 °C for 0.5 h. After completion of the reaction, the dimethylsulfoxide was removed by lyophilization under reduced pressure. The resulting crude product was purified by silica gel chromatography (methanol:dichloromethane = 1:10) to give a white solid (139.7 mg, yield: 40%).
LCMS $[M+H]^+$: 349.0.

**Synthesis of 2-(6-(6-(allyloxy)-2,3-dichlorophenyl)-6,7-dihydro-5*H*-pyrrolo[2,1-*c*][1,2,4]triazol-3-yl)-2-methylpropanenitrile (5):**

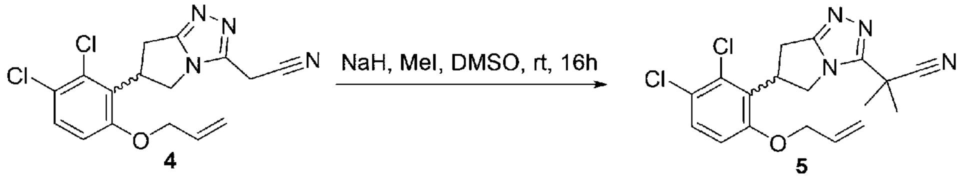


4

5

At 0 °C, sodium hydride (40 mg, 60% by mass, 1.0 mmol) was added to a solution of 2-(6-(6-(allyloxy)-2,3-dichlorophenyl)-6,7-dihydro-5*H*-pyrrolo[2,1-c][1,2,4]triazol-3-yl)-acetonitrile (70.0 mg, 0.20 mmol) in dimethylsulfoxide (0.5 mL). The mixture was stirred at room temperature for 1 h and then cooled again to 0 °C, followed by the addition of iodomethane (142.0 mg, 1.0 mmol). The mixture was stirred at room temperature for 16 h. After completion of the reaction, the reaction mixture was diluted with ethyl acetate (10 mL), quenched with ice water (10 mL), and extracted twice with ethyl acetate (10 mL). The organic phases were combined, washed with saturated brine (30 mL), dried over anhydrous sodium sulfate, filtered, and concentrated under reduced pressure. The resulting crude product was purified by silica gel chromatography (methanol:dichloromethane = 1:10) to give a white solid (13.6 mg, yield: 18%).
LCMS $[M+H]^+$: 377.0.

**Synthesis of 2-(6-(2,3-dichloro-6-hydroxyphenyl)-6,7-dihydro-5*H*-pyrrolo[2,1-*c*][1,2,4]triazol-3-yl)-2-methylpropanenitrile (6):**

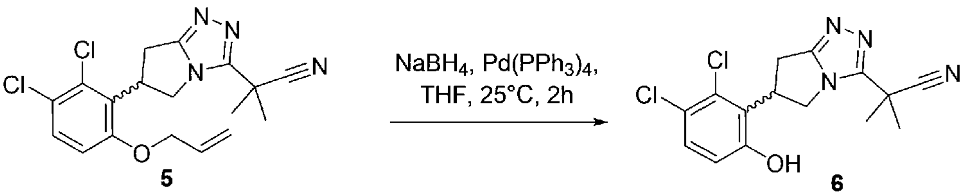


5

6

The compound 2-(6-(6-(allyloxy)-2,3-dichlorophenyl)-6,7-dihydro-5*H*-pyrrolo[2,1-*c*][1,2,4]triazol-3-yl)-2-methylpropanenitrile (18.0 mg, 0.048 mmol) was dissolved in a tetrahydrofuran solution (2.0 mL), and sodium borohydride (2.7 mg, 0.072 mmol) and tetrakis(triphenylphosphine)palladium(0) (27.6 mg, 0.024 mmol) were added to the reaction mixture. The resulting mixture was allowed to react at 25 °C for 2 h. The reaction mixture was filtered and concentrated under reduced pressure, and the residue was separated by preparative chromatography to give a white solid (2.0 mg, yield: 12%).
LCMS $[M+H]^+$: 337.0.
$^1H$ NMR (400 MHz, $CD_3OD$) δ 7.31 (d, *J* = 8.8 Hz, 1H), 6.80 (d, *J* = 8.8 Hz, 1H), 5.24 (s, 1H), 4.54 (t, *J* = 10.1 Hz, 1H), 4.38 (dd, *J* = 10.5, 6.8 Hz, 1H), 3.34 (d, *J* = 10.4 Hz, 1H), 3.20 (d, *J* = 7.0 Hz, 1H), 1.82 (s, 6H).

**Example 5. 3,4-Dichloro-2-(3-cyclohexyl-6,7-dihydro-5*H*-pyrrolo[2,1-*c*][1,2,4]triazol-6-yl)phenol**

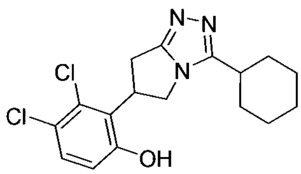

This example was prepared by reference to the preparation method for Example 3, except that the ethyl 3-hydroxy-3-methylbutanoate was replaced by methyl cyclohexanecarboxylate.
LCMS $[M+H]^+$: 352.0.

$^1$H NMR (400 MHz, DMSO-$d_6$) δ 10.57 (s, 1H), 7.40 (d, *J* = 8.8 Hz, 1H), 6.86 (d, *J* = 8.8 Hz, 1H), 4.97 (t, *J* = 8.4 Hz, 1H), 4.30 (t, *J* = 9.9 Hz, 1H), 4.08 (dd, *J* = 10.2, 7.4 Hz, 1H), 3.09 (dt, *J* = 15.9, 7.0 Hz, 2H), 2.80 - 2.74 (m, 1H), 1.89 (t, *J* = 12.2 Hz, 2H), 1.75 (s, 2H), 1.66 (d, *J* = 12.3 Hz, 1H), 1.54 - 1.43 (m, 2H), 1.30 (dt, *J* = 18.1, 7.9 Hz, 3H).

**Example 6. 4-(6-(2,3-dichloro-6-hydroxyphenyl)-6,7-dihydro-5*H*-pyrrolo[2,1-*c*][1,2,4]triazol-3-yl)benzonitrile**

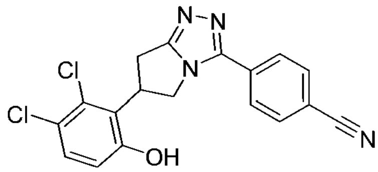

This example was prepared by reference to the preparation method for Example 3, except that the ethyl 3-hydroxy-3-methylbutanoate was replaced by methyl 4-cyanobenzoate.
LCMS [M+H]$^+$: 371.0.
$^1$H NMR (400 MHz, DMSO-$d_6$) δ 10.69 (s, 1H), 8.08 (d, *J* = 8.5 Hz, 2H), 7.97 (d, *J* = 8.5 Hz, 2H), 7.40 (d, *J* = 8.8 Hz, 1H), 6.85 (d, *J* = 8.8 Hz, 1H), 5.08 (ddd, *J* = 17.2, 9.8, 7.4 Hz, 1H), 4.68 (t, *J* = 10.0 Hz, 1H), 4.40 (dd, *J* = 10.3, 7.1 Hz, 1H), 3.32 - 3.25 (m, 1H), 3.15 (dd, *J* = 16.0, 7.4 Hz, 1H).

**Example 7. 2-(3-(1*H*-Indazol-5-yl)-6,7-dihydro-5*H*-pyrrolo[2,1-*c*][1,2,4]triazol-6-yl)-3,4-dichlorophenol**

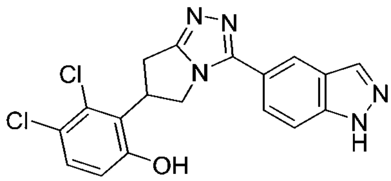

This example was prepared by reference to the preparation method for Example 3, except that the ethyl 3-hydroxy-3-methylbutanoate was replaced by methyl 1*H*-indazole-5-carboxylate.
LCMS [M+H]$^+$: 368.1.
$^1$H NMR (400 MHz, DMSO-$d_6$) δ 13.30 (s, 1H), 11.00 (s, 1H), 8.39 (s, 1H), 8.24 (s, 1H), 8.13 (s, 1H), 7.96 (dd, J=8.8, 1.4, 1H), 7.65 (d, *J*=8.8, 1H), 7.38 (d, *J*=8.8, 1H), 6.87 (d, *J*=8.8, 1H), 5.12 - 5.05 (m, 1H), 4.65 (t, *J*=9.9, 1H), 4.41 (dd, *J*=10.0, 7.5, 1H), 3.24 - 3.14 (m, 2H).

**Example 8. 3,4-Dichloro-2-(3-isopropyl-6,7-dihydro-5*H*-pyrrolo[2,1-*c*][1,2,4]triazol-6-yl)phenol**

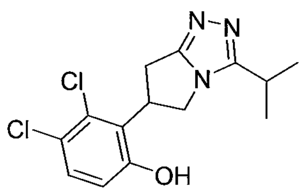

This example was prepared by reference to the preparation method for Example 3, except that the ethyl 3-hydroxy-3-methylbutanoate was replaced by methyl isobutyrate.
LCMS [M+H]$^+$: 312.0.
$^1$H NMR (400 MHz, DMSO-$d_6$) δ 10.59 (s, 1H), 7.40 (d, *J* = 8.8 Hz, 1H), 6.86 (d, *J* = 8.8 Hz, 1H), 5.05 - 4.88 (m, 1H), 4.27 (t, *J* = 9.9 Hz, 1H), 4.05 (dd, *J* = 10.1, 7.5 Hz, 1H), 3.15 - 2.98 (m, 3H), 1.24 (t, *J* = 6.7 Hz, 6H).

**Example 9. 3-(6-(2,3-dichloro-6-hydroxyphenyl)-6,7-dihydro-5*H*-pyrrolo[2,1-*c*][1,2,4]triazol-3-yl)benzonitrile**

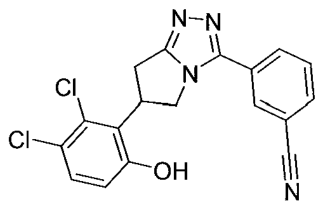

This example was prepared by reference to the preparation method for Example 3, except that the ethyl 3-hydroxy-3-methylbutanoate was replaced by methyl 3-cyanobenzoate.

LCMS $[M+H]^+$: 371.5.

$^1H$ NMR (400 MHz, DMSO-$d_6$) δ 10.66 (s, 1H), 8.28 - 8.21 (m, 2H), 7.94 (d, *J* = 7.8 Hz, 1H), 7.72 (t, *J* = 7.8 Hz, 1H), 7.64 - 7.54 (m, 1H), 7.40 (d, *J* = 8.8 Hz, 1H), 6.86 (d, *J* = 8.8 Hz, 1H), 5.12 - 5.03 (m, 1H), 4.69 (t, *J* = 10.0 Hz, 1H), 4.43 (dd, *J* = 10.3, 7.3 Hz, 1H), 3.29 - 3.13 (m, 2H).

**Example 10. Cyclopropyl(4-(6-(2,3-dichloro-6-hydroxyphenyl)-6,7-dihydro-5*H*-pyrrolo[2,1-*c*][1,2,4]triazol-3-yl) piperidin-1-yl)methanone**

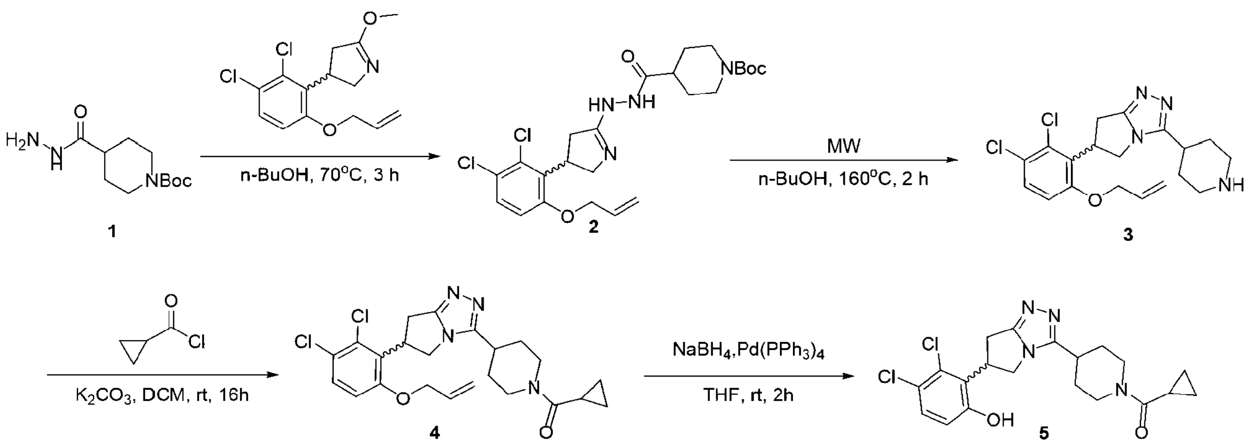


**Synthesis of *tert*-butyl 4-(2-(3-(6-(allyloxy)-2,3-dichlorophenyl)-3,4-dihydro-2*H*-pyrrol-5-yl)hydrazine-1-carbonyl)piperidine-1-carboxylate (2):**

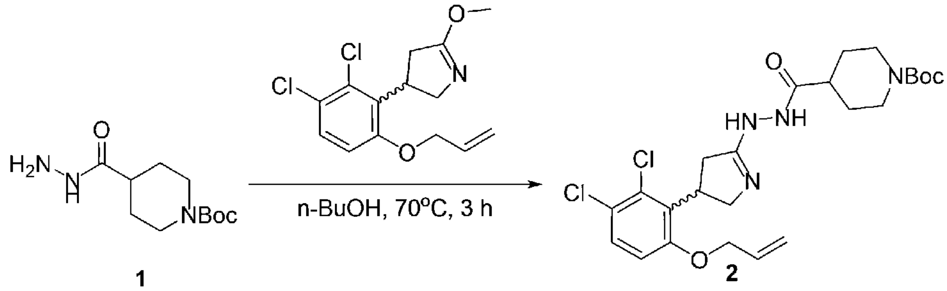


A solution of 3-(6-(allyloxy)-2,3-dichlorophenyl)-5-methoxy-3,4-dihydro-2*H*-pyrrole (600.0 mg, 2.00 mmol) and *tert*-butyl 4-(hydrazinecarbonyl)piperidine-1-carboxylate (1.458 g, 6.00 mmol) in *n*-butanol (10.0 mL) was stirred at 70 °C for 3 h. After completion of the reaction, the resulting mixture was directly used in the next step without further processing.

LCMS $[M+H]^+$: 511.0.

**Synthesis of 6-(6-(allyloxy)-2,3-dichlorophenyl)-3-(piperidin-4-yl)-6,7-dihydro-5*H*-pyrrolo[2,1-*c*][1,2,4]triazole (3):**

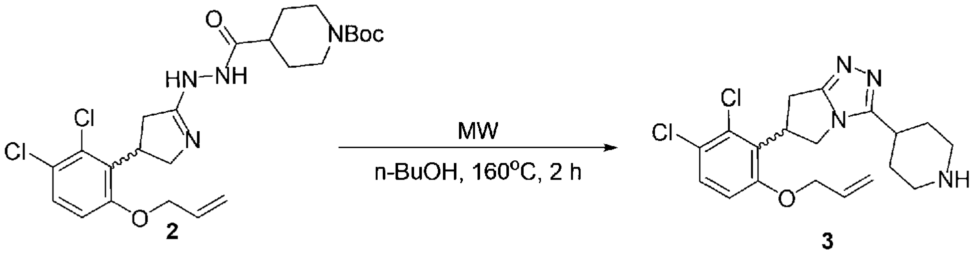


2 3

A solution of *tert*-butyl 4-(2-(3-(6-(allyloxy)-2,3-dichlorophenyl)-3,4-dihydro-2*H*-pyrrol-5-yl)hydrazine-1-carbonyl)piperidine-1-carboxylate in n-butanol (1.0 g, 1.96 mmol, 15 mL of n-butanol solution) was transferred to a microwave tube and then stirred under microwave at 160 °C for 2 h. After completion of the reaction, the n-butanol was removed by distillation under reduced pressure. The resulting crude product was purified by silica gel chromatography (ethyl acetate:petroleum ether = 1:1) to give a white solid (250.0 mg, yield: 33%).

LCMS $[M+H]^+$: 392.9.

**Synthesis of (4-(6-(6-(allyloxy)-2,3-dichlorophenyl)-6,7-dihydro-5*H*-pyrrolo[2,1-*c*][1,2,4]triazol-3-yl)piperidin-1-yl)(cyclopropyl)methanone (4):**

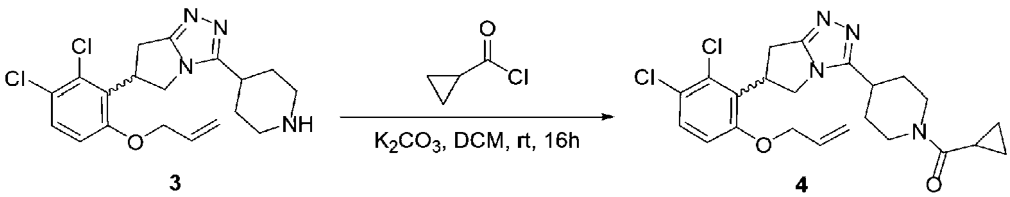


3 4

Potassium carbonate (88.0 mg, 0.64 mmol) and cyclopropanecarbonyl chloride (50.0 mg, 0.48 mmol) were added to a solution of 6-(6-(allyloxy)-2,3-dichlorophenyl)-3-(piperidin-4-yl)-6,7-dihydro-5H-pyrrolo[2,1-c][1,2,4]triazole in dichloromethane (125.0 mg, 0.32 mmol, 3 mL of dichloromethane solution). The mixture was stirred at room temperature for 16 h. After completion of the reaction, the reaction mixture was diluted with water (20 mL) and extracted three times with ethyl acetate (10 mL). The organic phase was washed with saturated brine (20 mL) and dried over anhydrous sodium sulfate, and the solvent was removed by distillation under reduced pressure. The resulting crude product was purified by silica gel chromatography (ethyl acetate:petroleum ether = 1:1) to give the target product (45.0 mg, yield: 31%).

LCMS $[M+H]^+$: 460.8.

**Synthesis of cyclopropyl(4-(6-(2,3-dichloro-6-hydroxyphenyl)-6,7-dihydro-5*H*-pyrrolo[2,1-*c*][1,2,4]triazol-3-yl) piperidin-1-yl)methanone (5):**

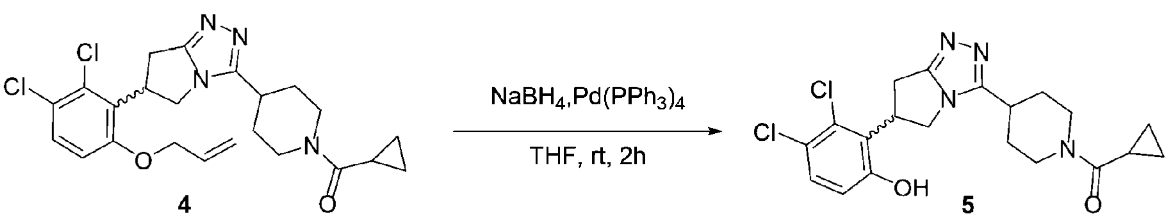


4 5

At room temperature, sodium borohydride (8.0 mg, 0.20 mmol) and tetrakis(triphenylphosphine)palladium(0) (5 mg, 0.004 mmol) were added to a solution of (4-(6-(6-(allyloxy)-2,3-dichlorophenyl)-6,7-dihydro-5*H*-pyrrolo[2,1-*c*][1,2,4] triazol-3-yl)piperidin-1-yl)(cyclopropyl)methanone (45.0 mg, 0.10 mmol) in tetrahydrofuran (2.0 mL). The flask was

purged using an argon balloon, and the mixture was stirred at room temperature for 2 h. After completion of the reaction, the mixture was filtered through an organic-phase filter membrane, and the filtrate was rotary evaporated to dryness. The crude product was purified by preparative chromatography to give a white solid (5.0 mg, yield: 11%).

LCMS $[M+H]^+$: 420.8.

$^1H$ NMR (400 MHz, $CD_3OD$) $\delta$ 7.30 (d, $J$ = 8.7 Hz, 1H), 6.78 (d, $J$ = 8.8 Hz, 1H), 5.16 (dd, $J$ = 16.6, 8.8 Hz, 1H), 4.54 (t, $J$ = 11.7 Hz, 1H), 4.42 (d, $J$ = 12.0 Hz, 1H), 4.35 (d, $J$ = 9.8 Hz, 1H), 4.25 (dd, $J$ = 10.3, 7.2 Hz, 1H), 3.37 - 3.30 (m, 1H), 3.22 (dd, $J$ = 8.8, 5.1 Hz, 2H), 3.16 (td, $J$ = 7.8, 3.9 Hz, 1H), 2.84 (t, $J$ = 12.4 Hz, 1H), 2.10 (t, $J$ = 12.7 Hz, 1H), 1.99 (ddd, $J$ = 13.2, 7.9, 3.7 Hz, 2H), 1.83 (dd, $J$ = 25.1, 12.5 Hz, 1H), 1.67 (dd, $J$ = 16.1, 10.7 Hz, 1H), 0.86 (t, $J$ = 3.7 Hz, 2H), 0.82 - 0.78 (m, 2H).

**Example 11. 3,4-Dichloro-2-(3-(1-cyclopropylpiperidin-4-yl)-6,7-dihydro-5*H*-pyrrolo[2,1-*c*][1,2,4]triazol-6-yl) phenol**

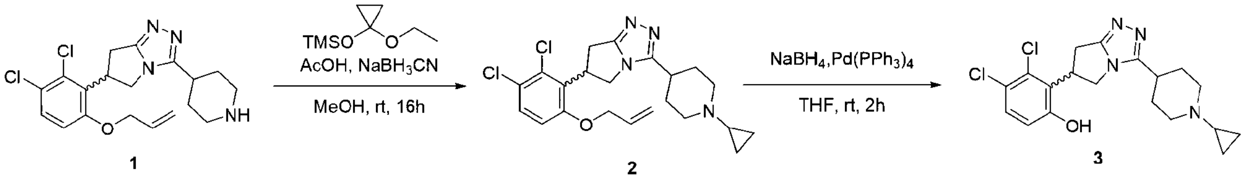


1 2 3

**Synthesis of 6-(6-(allyloxy)-2,3-dichlorophenyl)-3-(1-cyclopropylpiperidin-4-yl)-6,7-dihydro-5*H*-pyrrolo[2,1-c][1,2,4]triazole (2):**

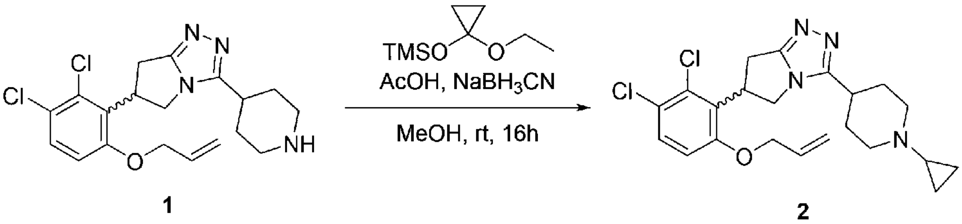


1 2

Acetic acid (16.0 mg, 0.26 mmol) and (1-ethoxycyclopropoxy)trimethylsilane (70.0 mg, 0.40 mmol) were added to a solution of 6-(6-(allyloxy)-2,3-dichlorophenyl)-3-(piperidin-4-yl)-6,7-dihydro-5*H*-pyrrolo[2,1-*c*][1,2,4]triazole in methanol (105.0 mg, 0.26 mmol, 3 mL of methanol solution). The mixture was stirred at room temperature for 0.5 h. The mixture was cooled to 0 °C, and then sodium cyanoborohydride (34.0 mg, 0.54 mmol) was added. The resulting mixture was stirred at room temperature for 16 h. After completion of the reaction, the reaction mixture was diluted with water (20 mL) and extracted with ethyl acetate (20 mL). The organic phase was washed with saturated brine (20 mL) and dried over anhydrous sodium sulfate, and the solvent was removed by distillation under reduced pressure. The resulting crude product was purified by silica gel chromatography (ethyl acetate:petroleum ether = 1:1) to give the target product (65.0 mg, yield: 54%).

LCMS $[M+H]^+$: 432.8.

**Synthesis of 3,4-dichloro-2-(3-(1-cyclopropylpiperidin-4-yl)-6,7-dihydro-5*H*-pyrrolo[2,1-c][1,2,4]triazol-6-yl) phenol (3):**

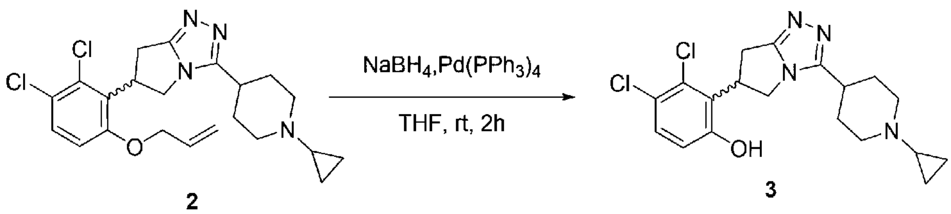


2 3

At room temperature, sodium borohydride (11.0 mg, 0.28 mmol) and tetrakis(triphenylphosphine)palladium(0) (8.0 mg, 0.007 mmol) were added to a solution of 6-(6-(allyloxy)-2,3-dichlorophenyl)-3-(1-cyclopropylpiperidin-4-yl)-6,7-dihydro-5*H*-pyrrolo[2,1-c][1,2,4]triazole (60.0 mg, 0.14 mmol) in tetrahydrofuran (2.0 mL). The flask was purged using an argon balloon, and the mixture was stirred at room temperature for 2 h. After completion of the reaction, the reaction mixture was filtered through an organic-phase filter membrane, and the filtrate was rotary evaporated to dryness. The crude product was purified by preparative chromatography to give a white solid (5.5 mg, yield: 10%).

LCMS $[M+H]^+$: 392.9.

$^1$H NMR (400 MHz, DMSO-*d6*) δ 10.59 (s, 1H), 7.40 (d, *J* = 8.7 Hz, 1H), 6.86 (d, *J* = 8.6 Hz, 1H), 5.00 - 4.91 (m, 1H), 4.28 (t, *J* = 9.6 Hz, 1H), 4.09 - 4.02 (m, 1H), 3.07 (dt, *J* = 15.9, 7.0 Hz, 4H), 2.80 (s, 1H), 2.53 (d, *J* = 8.7 Hz, 1H), 2.47 - 2.35 (m, 1H), 2.09 - 1.80 (m, 3H), 1.69 (s, 2H), 1.18 (s, 1H), 0.48 (s, 3H).

**Example 12. (6-(2,3-Dichloro-6-hydroxyphenyl)-6,7-dihydro-5*H*-pyrrolo[2,1-*c*][1,2,4]triazol-3-yl)(3-hydroxypyrrolidin-1-yl)methanone**

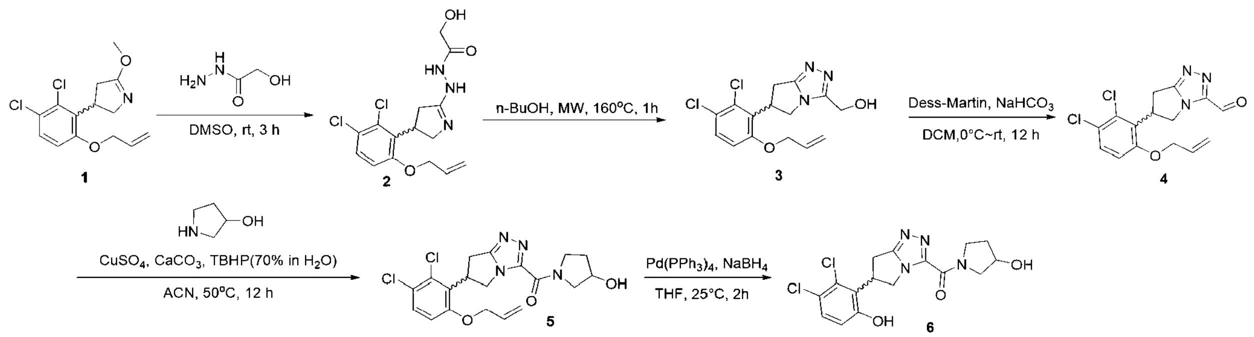


**Synthesis of *N*'-(3-(6-(allyloxy)-2,3-dichlorophenyl)-3,4-dihydro-2*H*-pyrrol-5-yl)-2-hydroxyacetohydrazide (2):**

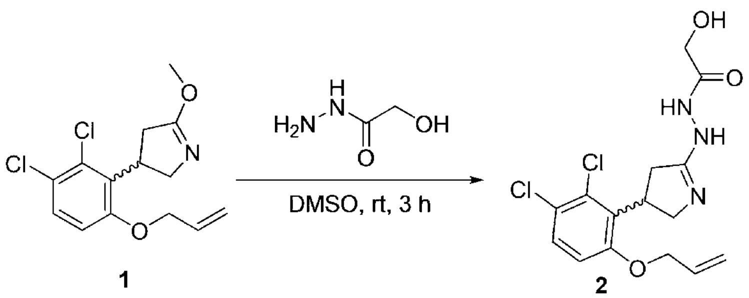


A solution of 3-(6-(allyloxy)-2,3-dichlorophenyl)-5-methoxy-3,4-dihydro-2*H*-pyrrole (3.0 g, 0.01 mol) and 2-hydroxyacetohydrazide (1.4 g, 0.02 mol) in dimethylsulfoxide (30.0 mL) was stirred at room temperature for 3 h. After completion of the reaction, the solvent was removed by lyophilization under reduced pressure. The resulting crude product was purified by silica gel chromatography (methanol:dichloromethane = 1:10) to give a colorless oily liquid (3.0 g, yield: 84%).

LCMS $[M+H]^+$: 358.0.

**Synthesis of (6-(6-(allyloxy)-2,3-dichlorophenyl)-6,7-dihydro-5*H*-pyrrolo[2,1-*c*][1,2,4]triazol-3-yl)methanol (3):**

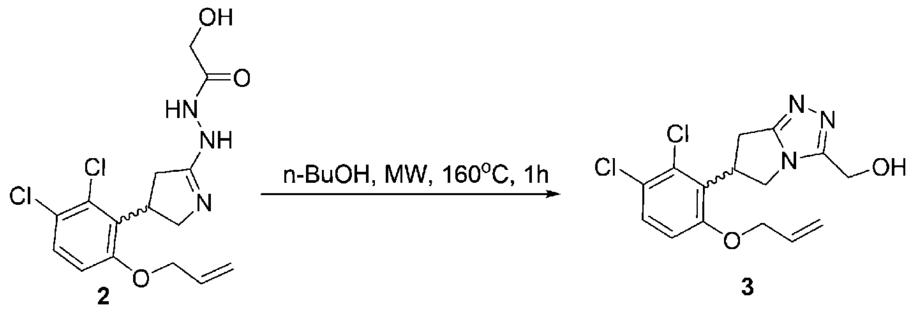


A solution of *N'*-(3-(6-(allyloxy)-2,3-dichlorophenyl)-3,4-dihydro-2*H*-pyrrol-5-yl)-2-hydroxyacetohydrazide (3.0 g, 8.40 mmol) in *n*-butanol (30.0 mL) was stirred under microwave conditions at 160 °C for 1 h. After completion of the reaction, the reaction mixture was cooled to room temperature and then rotary evaporated to dryness by concentration under reduced pressure to remove the solvent. The resulting crude product was purified by silica gel chromatography (methanol:dichloromethane = 1:10) to give a white solid (1.0 g, yield: 35%).

LCMS $[M+H]^+$: 340.0.

**Synthesis of 6-(6-(allyloxy)-2,3-dichlorophenyl)-6,7-dihydro-5*H*-pyrrolo[2,1-*c*][1,2,4]triazole-3-carbaldehyde (4):**

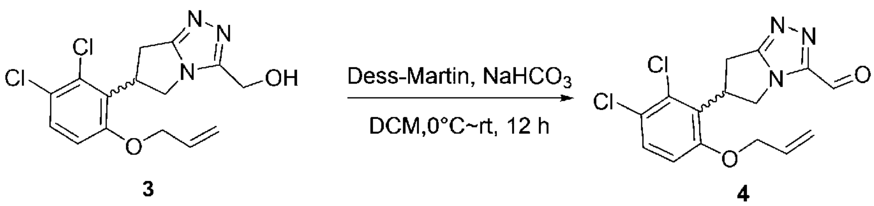


A solution of (6-(6-(allyloxy)-2,3-dichlorophenyl)-6,7-dihydro-5*H*-pyrrolo[2,1-*c*][1,2,4]triazol-3-yl)methanol (1.0 g, 2.94 mmol) and sodium bicarbonate (2.46 g, 29.29 mmol) in dichloromethane (25.0 mL) was cooled to 0 °C. At 0 °C, Dess-Martin periodinane (1.87 g, 4.41 mmol) was slowly added to the reaction mixture. The reaction mixture was then stirred at room temperature for 12 h. After completion of the reaction, the reaction mixture was quenched by dropwise adding a saturated aqueous sodium thiosulfate solution at 0 °C, then diluted by adding water (40.0 mL), and extracted with ethyl acetate (50.0 mL × 2). The organic phases were combined, washed with brine (100.0 mL), dried over anhydrous sodium sulfate, and rotary evaporated to dryness by concentration under reduced pressure to remove the solvent. The resulting crude product was purified by silica gel chromatography (ethyl acetate:petroleum ether = 1:1) to give a yellow solid (900.0 mg, yield: 91%).

LCMS $[M+H]^+$: 338.0.

**Synthesis of (6-(6-(allyloxy)-2,3-dichlorophenyl)-6,7-dihydro-5*H*-pyrrolo[2,1-*c*][1,2,4]triazol-3-yl)(3-hydroxypyrrolidin-1-yl)methanone (5):**

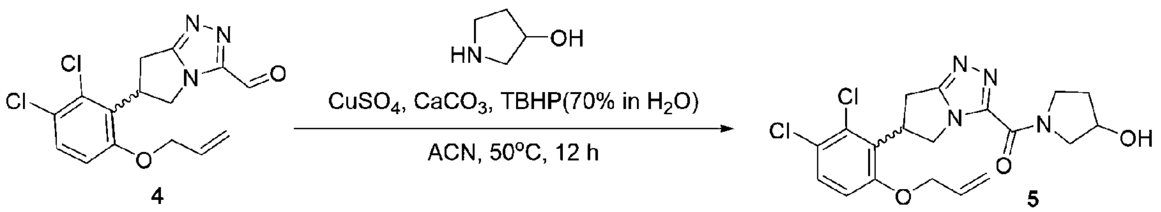


At room temperature, *tert*-butyl hydroperoxide (41.0 mg, 0.46 mmol, 70% in water) was added to a solution of 6-(6-(allyloxy)-2,3-dichlorophenyl)-6,7-dihydro-5*H*-pyrrolo[2,1-*c*][1,2,4]triazole-3-carbaldehyde (70.0 mg, 0.21 mmol), copper sulfate (3.3 mg, 0.02 mmol), pyrrolidin-3-ol (43.3 mg, 0.50 mmol), and calcium carbonate (45.6 mg, 0.46 mmol) in acetonitrile (5.0 mL). The mixture was stirred at 50 °C for 12 h. After completion of the reaction, the reaction mixture was

diluted by adding water (8.0 mL) and extracted with ethyl acetate (8.0 mL × 2). The organic phases were combined, washed with brine (10.0 mL), dried over anhydrous sodium sulfate, and rotary evaporated to dryness by concentration under reduced pressure to remove the solvent. The resulting crude product was purified by silica gel chromatography (methanol:dichloromethane = 1:12.5) to give the target product (40.0 mg, yield: 46%).

LCMS $[M+H]^+$: 423.0.

**Synthesis of (6-(2,3-dichloro-6-hydroxyphenyl)-6,7-dihydro-5*H*-pyrrolo[2,1-*c*][1,2,4]triazol-3-yl)(3-hydroxypyrrolidin-1-yl)methanone (6):**

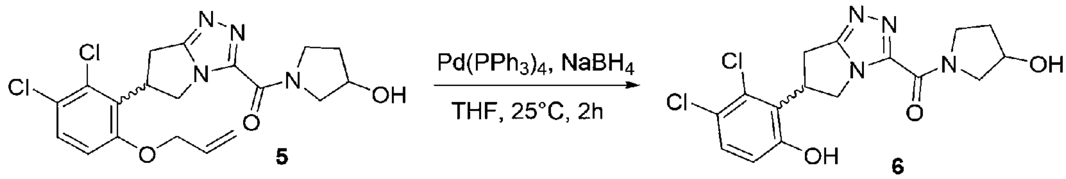


5 6

At room temperature, tetrakis(triphenylphosphine)palladium(0) (10.9 mg, 0.01 mmol) was added to a solution of (6-(6-(allyloxy)-2,3-dichlorophenyl)-6,7-dihydro-5*H*-pyrrolo[2,1-*c*][1,2,4]triazol-3-yl)(3-hydroxypyrrolidin-1-yl)methanone (40.0 mg, 0.09 mmol) and sodium borohydride (5.4 mg, 0.14 mmol) in tetrahydrofuran (3.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was purified by preparative chromatography to give a white solid (4.2 mg, yield: 12%).

$^1$H NMR (400 MHz, DMSO-$d_6$) δ 10.64 (s, 1H), 7.38 (d, *J* = 8.8 Hz, 1H), 6.83 (d, *J* = 8.8 Hz, 1H), 5.09 - 4.94 (m, 2H), 4.46 (dd, *J* = 20.1, 10.2 Hz, 1H), 4.34 (d, *J* = 25.2 Hz, 1H), 4.24 - 4.18 (m, 1H), 4.13 - 3.86 (m, 2H), 3.62 - 3.44 (m, 2H), 3.29 - 3.21 (m, 1H), 3.11 - 3.02 (m, 1H), 2.01 - 1.80 (m, 2H).

**Example 13. (6-(2,3-Dichloro-6-hydroxyphenyl)-6,7-dihydro-5*H*-pyrrolo[2,1-*c*][1,2,4]triazol-3-yl)(piperazin-1-yl) methanone**

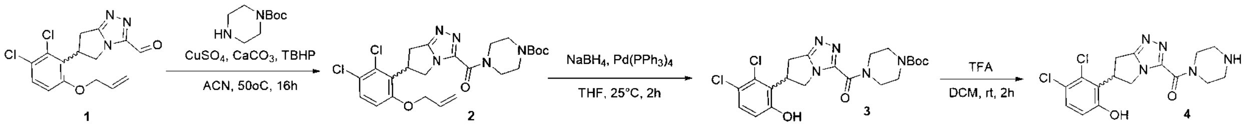


1 2 3 4

**Synthesis of *tert*-butyl 4-(6-(6-(allyloxy)-2,3-dichlorophenyl)-6,7-dihydro-5*H*-pyrrolo[2,1-*c*][1,2,4]triazole-3-carbonyl)piperazine-1-carboxylate (2):**

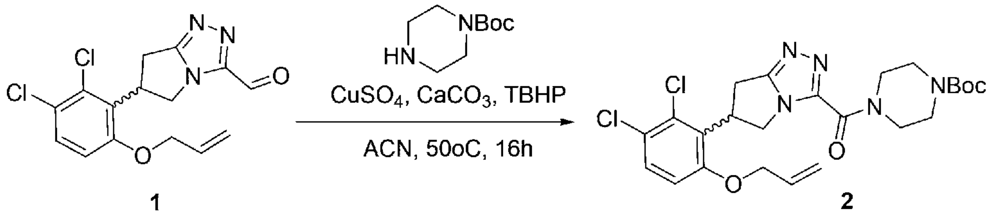


1 2

*tert*-Butyl piperazine-1-carboxylate (110.0 mg, 0.60 mmol), calcium carbonate (33.0 mg, 0.33 mmol), and anhydrous copper sulfate (4.0 mg, 0.02 mmol) were added to a solution of 6-(6-(allyloxy)-2,3-dichlorophenyl)-6,7-dihydro-5*H*-pyrrolo[2,1-c][1,2,4]triazole-3-carbaldehyde in acetonitrile (100.0 mg, 0.30 mmol, 3 mL of acetonitrile solution). The mixture was added to *tert-butyl* hydroperoxide (29.0 mg, 0.33 mmol) at room temperature, and the resulting mixture was stirred at 50 °C for 16 h under a nitrogen atmosphere. After completion of the reaction, the mixture was extracted with ethyl acetate (10 mL). The organic phase was washed with saturated brine (10 mL) and extracted three times. The resulting organic phase was dried over anhydrous sodium sulfate, and the solvent was removed by distillation

under reduced pressure. The resulting crude product was purified by silica gel chromatography (methanol:dichloromethane = 20:1) to give the target product (50.0 mg, yield: 32%).

LCMS $[M+H]^+$: 522.1.

**Synthesis of *tert-butyl* 4-(6-(2,3-dichloro-6-hydroxyphenyl)-6,7-dihydro-5*H*-pyrrolo[2,1-*c*][1,2,4]triazole-3-carbonyl)piperazine-1-carboxylate (3):**

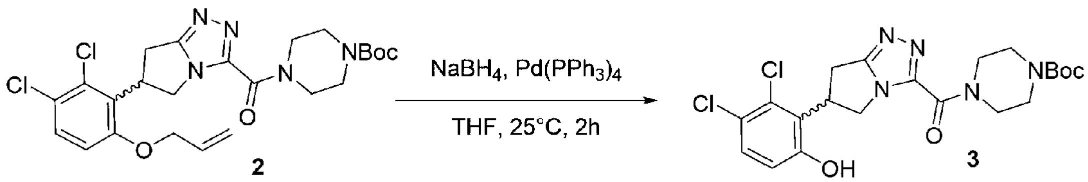


At room temperature, sodium borohydride (8.0 mg, 0.20 mmol) and tetrakis(triphenylphosphine)palladium(0) (2.0 mg, 0.0017 mmol) were added to a solution of *tert-butyl* 4-(6-(6-(allyloxy)-2,3-dichlorophenyl)-6,7-dihydro-5*H*-pyrrolo [2,1-*c*][1,2,4]triazole-3-carbonyl)piperazine-1-carboxylate (50.0 mg, 0.10 mmol) in tetrahydrofuran (2.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was purified by silica gel chromatography (methanol:dichloromethane = 20:1) to give the target product (26.0 mg, yield: 54%).

LCMS $[M+H]^+$: 481.8.

**Synthesis of (6-(2,3-dichloro-6-hydroxyphenyl)-6,7-dihydro-5*H*-pyrrolo[2,1-*c*][1,2,4]triazol-3- yl)(piperazin-1-yl) methanone (4):**

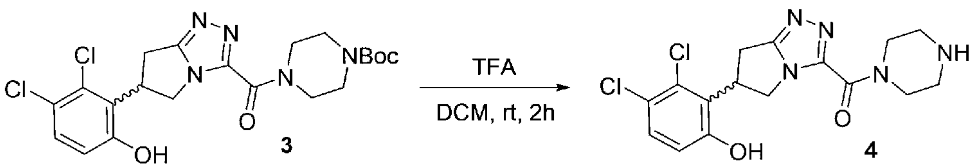


At room temperature, *tert*-butyl 4-(6-(2,3-dichloro-6-hydroxyphenyl)-6,7-dihydro-5*H*-pyrrolo[2,1-c][1,2,4]triazole-3-carbonyl)piperazine-1-carboxylate (26.0 mg, 0.05 mmol) was added to a mixed solvent of dichloromethane and trifluoroacetic acid (2.0 mL) in a ratio of 5:1. The mixture was stirred at room temperature for 2 h. After completion of the reaction, the reaction mixture was directly rotary evaporated to dryness, and the resulting crude product was purified by preparative chromatography to give a white solid (2.1 mg, yield: 11%).

LCMS $[M+H]^+$: 381.8.

$^1$H NMR (400 MHz, $CD_3OD$) δ 7.33 (d, *J* = 8.8 Hz, 1H), 6.81 (d, *J* = 8.8 Hz, 1H), 5.24 - 5.17 (m, 1H), 4.67 (d, *J* = 20.0 Hz, 2H), 4.59 - 4.53 (m, 1H), 4.44 (dd, *J* = 11.7, 7.6 Hz, 1H), 3.98 (d, *J* = 5.2 Hz, 2H), 3.37 (d, *J* = 19.3 Hz, 6H).

**Example 14. (6-(2,3-Dichloro-6-hydroxyphenyl)-6,7-dihydro-5*H*-pyrrolo[2,1-*c*][1,2,4]triazol-3-yl)(4-isopropylpiperazin-1-yl)methanone**

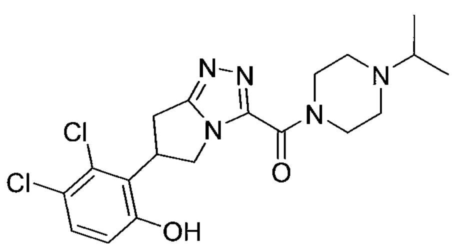

At room temperature, (6-(2,3-dichloro-6-hydroxyphenyl)-6,7-dihydro-5*H*-pyrrolo[2,1-*c*][1,2,4]triazol-3-yl)(piperazin-1-yl)methanone (15.0 mg, 0.04 mmol) was added to dichloroethane (2.0 mL). Acetone (9.0 mg, 0.16 mmol) was then added at room temperature, and the mixture was stirred at room temperature for 2 h and then cooled to 0 °C, followed by the addition of sodium triacetoxyborohydride (25.0 mg, 0.12 mmol). The resulting mixture was stirred at room temperature for 16 h. After completion of the reaction, the reaction mixture was extracted with ethyl acetate (5 mL). The organic phase was washed with saturated brine (5 mL) and extracted three times. The resulting organic phase was dried over anhydrous sodium sulfate, and the solvent was removed by distillation under reduced pressure. The crude product was purified by preparative chromatography to give a white solid (2.6 mg, yield: 20%).

LCMS $[M+H]^+$: 423.8.

$^1H$ NMR (400 MHz, $CD_3OD$) δ 7.32 (d, *J* = 8.8 Hz, 1H), 6.81 (d, *J* = 8.8 Hz, 1H), 5.86 (s, 1H), 5.24 - 5.16 (m, 1H), 4.86 - 4.72 (m, 1H), 4.56 (t, *J* = 10.6 Hz, 1H), 4.48 - 4.41 (m, 1H), 3.61 (d, *J* = 6.6 Hz, 4H), 3.34 (s, 2H), 3.23 (s, 2H), 1.41 (d, *J* = 6.6 Hz, 6H), 1.31 (d, *J* = 18.1 Hz, 1H).

**Example 15. 3,4-Dichloro-2-(3-(2,2,2-trifluoroethyl)-6,7-dihydro-5*H*-pyrrolo[2,1-*c*][1,2,4]triazol-6-yl)phenol**

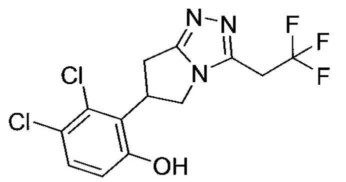

This example was prepared by reference to the preparation method for Example 3, except that the ethyl 3-hydroxy-3-methylbutanoate was replaced by ethyl 3,3,3-trifluoropropanoate.

LCMS $[M+H]^+$: 352.1.

$^1H$ NMR (400 MHz, DMSO-d6) δ 10.59 (s, 1H), 7.40 (d, *J* = 8.8, 1H), 6.86 (d, *J* = 8.8, 1H), 4.99 (t, *J* = 8.7, 1H), 4.30 (t, *J* = 10.1, 1H), 4.09 (dd, *J* = 10.4, 7.6, 1H), 4.05 - 3.95 (m, 2H), 3.24 - 3.17 (m, 1H), 3.12 - 3.04 (m, 1H).

**Example 16. 2-(3-(*tert*-Butyl)-6,7-dihydro-5*H*-pyrrolo[2,1-*c*][1,2,4]triazol-6-yl)-3,4-dichlorophenol**

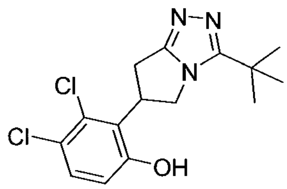

This example was prepared by reference to the preparation method for Example 3, except that the ethyl 3-hydroxy-3-methylbutanoate was replaced by ethyl pivalate.

$^1H$ NMR (400 MHz, DMSO-$d_6$) δ 10.57 (s, 1H), 7.39 (d, J = 8.8 Hz, 1H), 6.86 (d, J = 8.8 Hz, 1H), 5.07 - 4.89 (m, 1H), 4.38 (t, J = 9.9 Hz, 1H), 4.14 (dd, J = 10.2, 7.4 Hz, 1H), 3.04 (ddd, J = 23.5, 15.8, 9.0 Hz, 2H), 1.30 (s, 9H).

**Example 17. 3,4-Dichloro-2-(3-(piperidin-4-ylmethyl)-6,7-dihydro-5*H*-pyrrolo[2,1-*c*][1,2,4]triazol-6-yl)phenol**

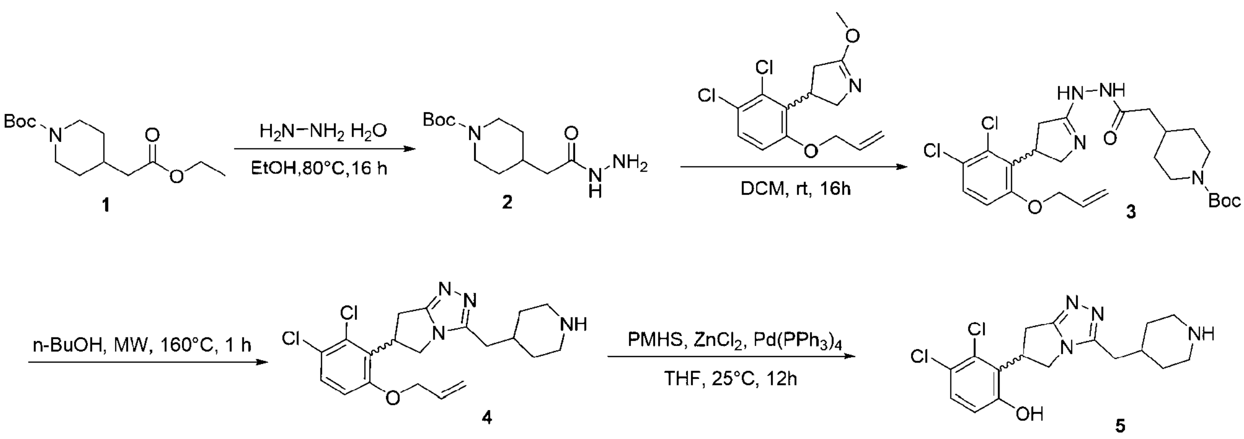


**Synthesis of *tert*-butyl 4-(2-hydrazinyl-2-oxoethyl)piperidine-1-carboxylate (2):**

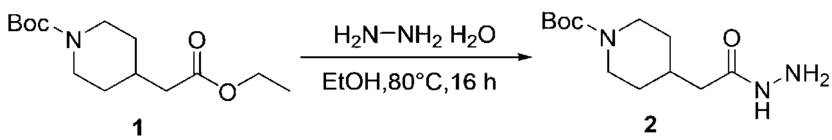


A solution of *tert*-butyl 4-(2-ethoxy-2-oxoethyl)piperidine-1-carboxylate (2.0 g, 0.01 mol) and hydrazine hydrate (0.7 g, 0.02 mol, 80% in water) in ethanol (20.0 mL) was stirred at 80 °C for 16 h. After completion of the reaction, the reaction mixture was cooled to room temperature and filtered to give a solid. The solid was then washed with dichloromethane to give a crude product. The resulting crude product was purified by silica gel chromatography (methanol:dichloromethane = 1:10) to give a white solid (1.6 g, yield: 83%).

LCMS $[M+H-56]^+$: 202.2.

**Synthesis of *tert*-butyl 4-(2-(2-(3-(6-(allyloxy)-2,3-dichlorophenyl)-3,4-dihydro-2*H*-pyrrol-5-yl)hydrazinyl)-2-oxoethyl)piperidine-1-carboxylate (3):**

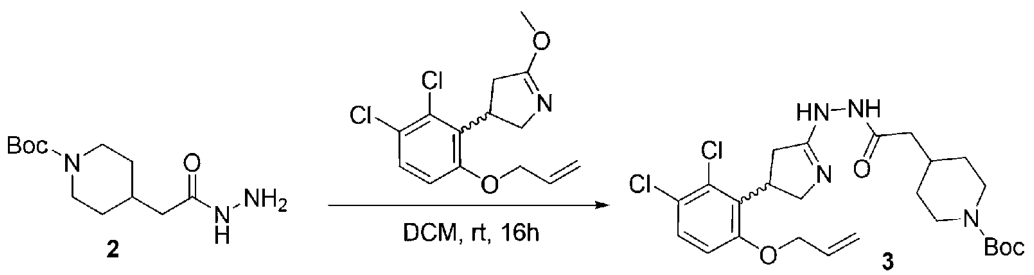


A solution of 3-(6-(allyloxy)-2,3-dichlorophenyl)-5-methoxy-3,4-dihydro-2*H*-pyrrole (600.0 mg, 1.99 mmol) and *tert*-butyl 4-(2-hydrazinyl-2-oxoethyl)piperidine-1-carboxylate (771.5 mg, 2.99 mmol) in dichloromethane (2.0 mL) was stirred at 25 °C for 16 h. After completion of the reaction, the dichloromethane was removed by distillation under reduced pressure. The resulting crude product was purified by silica gel chromatography (methanol:dichloromethane = 1:20) to give a white solid (1.0 g, yield: 95%).

LCMS $[M+H]^+$: 525.1.

**Synthesis of 6-(6-(allyloxy)-2,3-dichlorophenyl)-3-(piperidin-4-ylmethyl)-6,7-dihydro-5*H*-pyrrolo[2,1-*c*][1,2,4] triazole (4):**

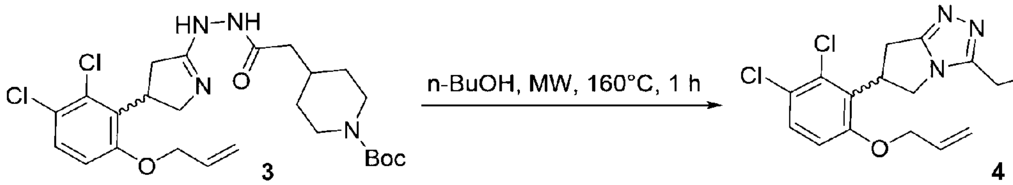

3 4

A solution of *tert*-butyl 4-(2-(2-(3-(6-(allyloxy)-2,3-dichlorophenyl)-3,4-dihydro-2*H*-pyrrol-5-yl)hydrazinyl)-2-oxoethyl)piperidine-1-carboxylate (1.0 g, 1.89 mmol) in n-butanol (10 mL) was stirred under microwave conditions at 160 °C for 1 h. After completion of the reaction, the mixture was rotary evaporated to dryness by concentration under reduced pressure to remove the solvent. The resulting crude product was purified by silica gel chromatography (methanol:dichloromethane = 1:15) to give a white solid (400.0 mg, yield: 52%).

LCMS $[M+H]^+$: 407.1.

**Synthesis of 3,4-dichloro-2-(3-(piperidin-4-ylmethyl)-6,7-dihydro-5*H*-pyrrolo[2,1-*c*][1,2,4]triazol-6-yl)phenol (5):**

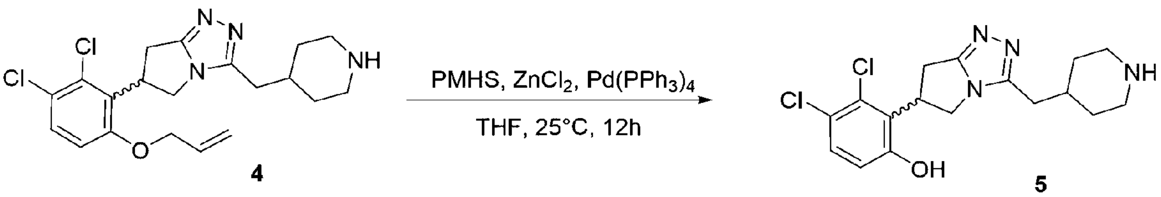

4 5

At room temperature, tetrakis(triphenylphosphine)palladium(0) (4.5 mg, 0.004 mmol) was added to a solution of 6-(6-(allyloxy)-2,3-dichlorophenyl)-3-(piperidin-4-ylmethyl)-6,7-dihydro-5*H*-pyrrolo[2,1-*c*][1,2,4]triazole (40.0 mg, 0.09 mmol), polymethylhydrosiloxane (43.7 mg, 0.19 mmol), and zinc chloride (1.2 mg, 0.008 mmol) in tetrahydrofuran (3.0 mL). The flask was purged using an argon balloon, and the mixture was stirred at 25 °C for 12 h. After completion of the reaction, the reaction mixture was filtered through an organic-phase filter membrane, and the filtrate was rotary evaporated to dryness. The crude product was subjected to preparative chromatography to give a white solid (5.1 mg, yield: 13%).

LCMS $[M+H]^+$: 407.1.

$^1H$ NMR (400 MHz, DMSO-$d_6$) δ 10.75 (s, 1H), 7.42 (d, J = 8.8 Hz, 1H), 6.89 (d, J = 8.8 Hz, 1H), 5.04 -5.00 (m, 1H), 4.38 - 4.37 (m, 1H), 4.16-4.15 (m, 1H), 3.44 - 3.09 (m, 5H), 2.93 - 2.73 (m, 4H), 2.06 - 1.97 (m, 1H), 1.81-1.78 (m, 2H), 1.38-1.36 (m, 2H).

**Example 18. 3,4-Dichloro-2-(3-((1-isopropylpiperidin-4-yl)methyl)-6,7-dihydro-5*H*-pyrrolo[2,1-*c*][1,2,4]triazol-6-yl)phenol**

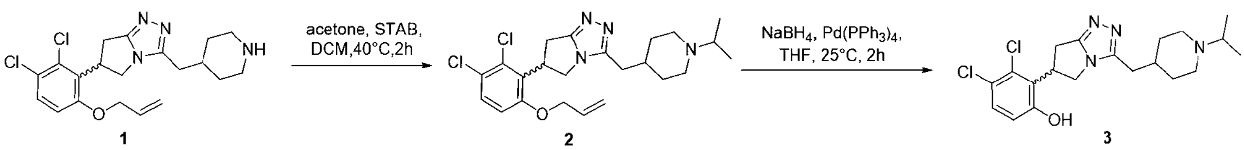

1 2 3

**Synthesis of 6-(6-(allyloxy)-2,3-dichlorophenyl)-3-((1-isopropylpiperidin-4-yl)methyl)-6,7-dihydro-5*H*-pyrrolo[2,1-*c*][1,2,4]triazole (2):**

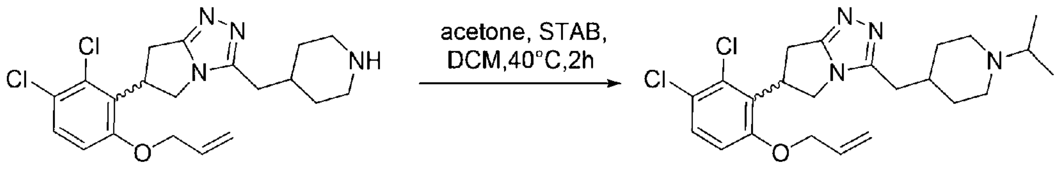


1 2

At 0 °C, sodium triacetoxyborohydride (234.1 mg, 1.10 mmol) was added in portions to a solution of 6-(6-(allyloxy)-2,3-dichlorophenyl)-3-(piperidin-4-ylmethyl)-6,7-dihydro-5*H*-pyrrolo[2,1-*c*][1,2,4]triazole (150.0 mg, 0.36 mmol) and acetone (64.2 mg, 1.10 mmol) in dichloromethane (2.0 mL). The mixture was stirred at 40 °C for 2 h. After completion of the reaction, the resulting solution was directly loaded onto a column via wet loading and purified by silica gel chromatography (methanol:dichloromethane = 1:10) to give a white solid (100.0 mg, yield: 60%).

LCMS $[M+H]^+$: 449.1.

**Synthesis of 3,4-dichloro-2-(3-((1-isopropylpiperidin-4-yl)methyl)-6,7-dihydro-5*H*-pyrrolo[2,1-*c*][1,2,4]triazol-6-yl)phenol (3):**

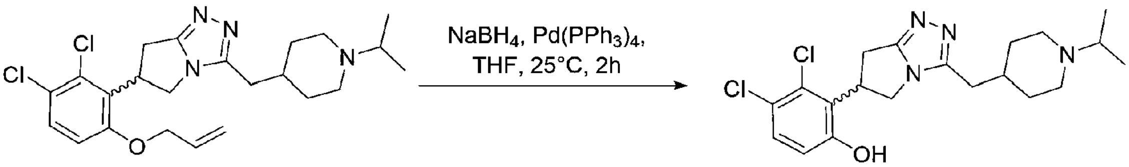


2 3

At room temperature, tetrakis(triphenylphosphine)palladium(0) (25.7 mg, 0.02 mmol) was added to a solution of 6-(6-(allyloxy)-2,3-dichlorophenyl)-3-((1-isopropylpiperidin-4-yl)methyl)-6,7-dihydro-5*H*-pyrrolo[2,1-*c*][1,2,4]triazole (100.0 mg, 0.22 mmol) and sodium borohydride (12.6 mg, 0.33 mmol) in tetrahydrofuran (5.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was subjected to preparative chromatography to give a white solid (5.8 mg, yield: 6.0%).

$^1$H NMR (400 MHz, DMSO-$d_6$) δ 8.19 (s, 1H), 7.39 (d, J = 8.8 Hz, 1H), 6.87 (d, J = 8.8 Hz, 1H), 4.99-4.95 (m, 1H), 4.21 (t, J = 9.9 Hz, 1H), 4.01 (dd, J = 10.1, 7.6 Hz, 1H), 3.17 - 2.98 (m, 6H), 2.62 (d, J = 6.3 Hz, 2H), 2.45-2.44 (m, 1H), 1.73 (d, J = 13.5 Hz, 2H), 1.37 - 1.27 (m, 2H), 1.07 (d, J = 6.6 Hz, 6H), 1.00-0.99 (m, 1H).

**Example 19. 1-((6-(2,3-Dichloro-6-hydroxyphenyl)-6,7-dihydro-5*H*-pyrrolo[2,1-*c*][1,2,4]triazol-3-yl)methyl)pyrrolidin-3-ol**

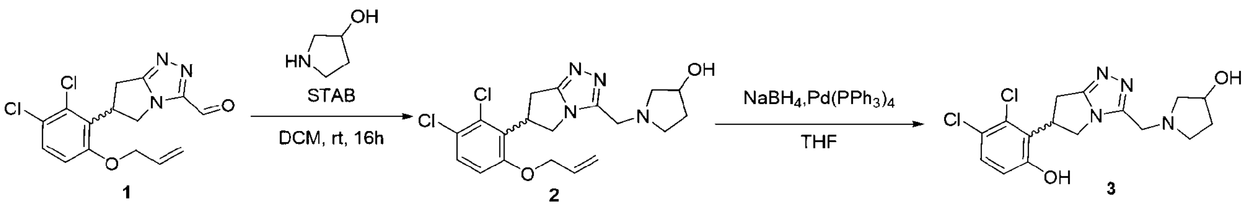


1 2 3

**Synthesis of 1-((6-(6-(allyloxy)-2,3-dichlorophenyl)-6,7-dihydro-5*H*-pyrrolo[2,1-*c*][1,2,4]triazol-3-yl)methyl)pyrrolidin-3-ol (2):**

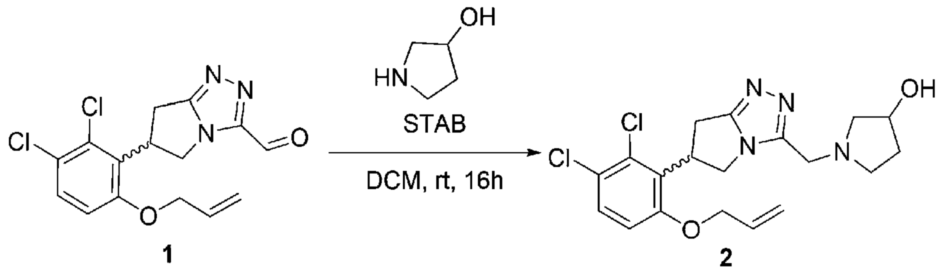


The compound 6-(6-(allyloxy)-2,3-dichlorophenyl)-6,7-dihydro-5*H*-pyrrolo[2,1-c][1,2,4]triazole-3-carbaldehyde (192.1 mg, 0.57 mmol) and pyrrolidin-3-ol (45.0 mg, 0.52 mmol) were dissolved in dichloromethane (6 mL). At 0 °C, sodium triacetoxyborohydride (164.2 mg, 0.77 mmol) was added to the mixture. The resulting mixture was stirred at room temperature for 16 h. The reaction mixture was diluted with water (30 mL) and extracted three times with dichloromethane (30 mL). The organic phases were combined and washed twice with brine (80 mL). The resulting organic phase was dried over anhydrous sodium sulfate and concentrated *in vacuo.* The resulting crude product was purified by silica gel chromatography (dichloromethane:methanol = 50:1) to give a pale yellow solid (120.0 mg, yield: 57%).

LCMS $[M+H]^+$: 409.0.

**Synthesis of 1-((6-(2,3-dichloro-6-hydroxyphenyl)-6,7-dihydro-5*H*-pyrrolo[2,1-*c*][1,2,4]triazol-3-yl)methyl)pyrrolidin-3-ol (3):**

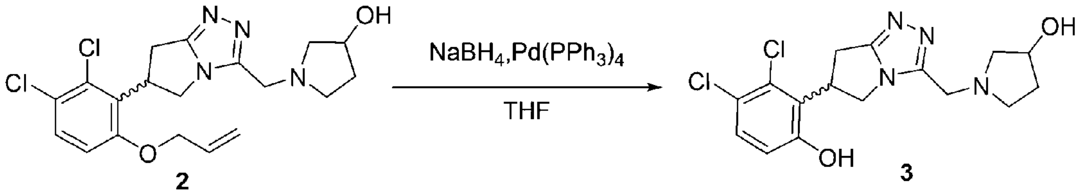


The compound 1-((6-(6-(allyloxy)-2,3-dichlorophenyl)-6,7-dihydro-5*H*-pyrrolo[2,1-*c*][1,2,4]triazol-3-yl)methyl) pyrrolidin-3-ol (100.0 mg, 0.24 mmol) was dissolved in a tetrahydrofuran solution (10 mL), and sodium borohydride (13.9 mg, 0.37 mmol) and tetrakis(triphenylphosphine)palladium(0) (56.5 mg, 0.049 mmol) were added to the reaction mixture. The resulting mixture was allowed to react at room temperature for 2 h. The reaction mixture was rotary evaporated to dryness, and the residue was separated by preparative chromatography to give a white solid (15.6 mg, yield: 17%).

LCMS $[M+H]^+$: 369.0.

$^1$H NMR (400 MHz, DMSO-$d_6$) δ 10.60 (s, 1H), 8.15 (s, 1H), 7.39 (dd, *J* = 8.8, 0.8 Hz, 1H), 6.87 (d, *J* = 8.7 Hz, 1H), 4.95 (dd, *J* = 17.2, 9.2 Hz, 1H), 4.73 (s, 1H), 4.28 - 4.15 (m, 2H), 4.08 - 4.01 (m, 1H), 3.70 (ddd, *J* = 17.0, 13.9, 4.7 Hz, 2H), 3.18 - 3.01 (m, 2H), 2.73 - 2.58 (m, 2H), 2.46 - 2.32 (m, 2H), 1.98 (dt, *J* = 14.2, 7.3 Hz, 1H), 1.54 (dd, *J* = 8.2, 4.4 Hz, 1H).

**Example 20. 3,4-Dichloro-2-(3-(morpholinomethyl)-6,7-dihydro-5*H*-pyrrolo[2,1-*c*][1,2,4]triazol-6-yl)phenol**

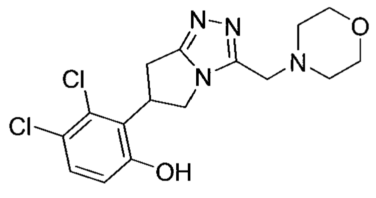

This example was prepared by reference to the preparation method for Example 19, except that the pyrrolidin-3-ol was replaced by morpholine.

LCMS $[M+H]^+$: 369.0.

$^1$H NMR (400 MHz, DMSO-*d6*) δ 10.67 (s, 1H), 7.39 (d, *J* = 8.8 Hz, 1H), 6.86 (d, *J* = 8.8 Hz, 1H), 5.02 - 4.93 (m, 1H), 4.27 (t, *J* = 10.2 Hz, 1H), 4.08 (dd, *J* = 10.7, 7.3 Hz, 1H), 3.66 - 3.50 (m, 6H), 3.17 (dd, *J* = 15.9, 10.2 Hz, 1H), 3.01 (dd, *J* =

15.9, 7.4 Hz, 1H), 2.36 (d, *J* = 4.5 Hz, 4H).

**Example 21. (6-(2,3-Dichloro-6-hydroxyphenyl)-6,7-dihydro-5*H*-pyrrolo[2,1-*c*][1,2,4]triazol-3-yl)(morpholino) methanone**

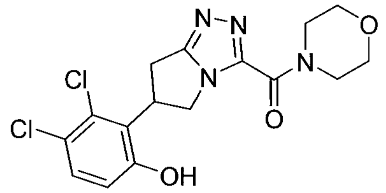

This example was prepared by reference to the preparation method for Example 12, except that the pyrrolidin-3-ol was replaced by morpholine.

LCMS $[M+H]^+$: 383.0.

$^1$H NMR (400 MHz, DMSO-d6) δ 10.59 (s, 1H), 7.39 (d, *J* = 8.8 Hz, 1H), 6.85 (d, *J* = 8.8 Hz, 1H), 5.05 - 4.95 (m, 1H), 4.45 (dd, *J* = 11.3, 9.9 Hz, 1H), 4.39 - 4.27 (m, 2H), 4.22 (dd, *J* = 11.6, 7.1 Hz, 1H), 3.70 - 3.61 (m, 6H), 3.28 - 3.22 (m, 1H), 3.07 (dd, *J* = 16.2, 7.2 Hz, 1H).

**Example 22. 3,4-Dichloro-2-(3-(1-(tetrahydro-2*H*-pyran-4-yl)piperidin-4-yl)-6,7-dihydro-5*H*-pyrrolo[2,1-*c*][1,2,4] triazol-6-yl)phenol**

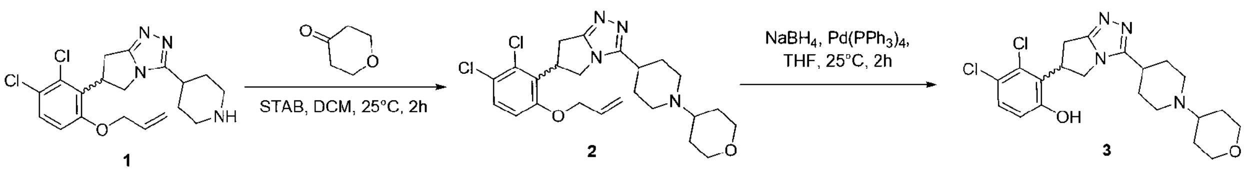


**Synthesis of 6-(6-(allyloxy)-2,3-dichlorophenyl)-3-(1-(tetrahydro-2*H*-pyran-4-yl)piperidin-4-yl)-6,7-dihydro-5*H*-pyrrolo[2,1-*c*][1,2,4]triazole (2):**

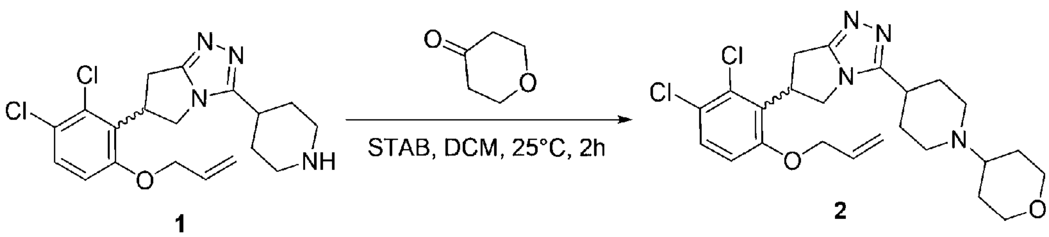


At 0 °C, sodium triacetoxyborohydride (242.5 mg, 1.14 mmol) was added to a solution of 6-(6-(allyloxy)-2,3-dichlorophenyl)-3-(piperidin-4-yl)-6,7-dihydro-5*H*-pyrrolo[2,1-*c*][1,2,4]triazole (150.0 mg, 0.38 mmol) and tetrahydro-4*H*-pyran-4-one (114.0 mg, 1.14 mmol) in dichloromethane (2.0 mL). The mixture was stirred at 25 °C for 2 h. After completion of the reaction, the reaction mixture was filtered through an organic-phase filter membrane, and the filtrate was rotary evaporated to dryness. The resulting crude product was purified by silica gel chromatography (methanol:dichloromethane = 1:20) to give a white solid (150.0 mg, yield: 82%).

LCMS $[M+H]^+$: 476.8.

**Synthesis of 3,4-dichloro-2-(3-(1-(tetrahydro-2*H*-pyran-4-yl)piperidin-4-yl)-6,7-dihydro-5*H*-pyrrolo[2,1-*c*][1,2,4] triazol-6-yl)phenol (3):**

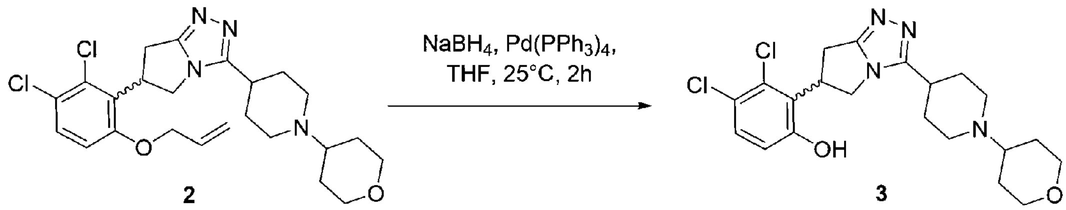


At room temperature, tetrakis(triphenylphosphine)palladium(0) (60.5 mg, 0.05 mmol) was added to a solution of 6-(6-(allyloxy)-2,3-dichlorophenyl)-3-(1-(tetrahydro-2*H*-pyran-4-yl)piperidin-4-yl)-6,7-dihydro-5*H*-pyrrolo[2,1-*c*][1,2,4]triazole (150.0 mg, 0.31 mmol) and sodium borohydride (17.9 mg, 0.47 mmol) in tetrahydrofuran (2.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was subjected to preparative chromatography to give a white solid (53.0 mg, yield: 39%).

$^1H$ NMR (400 MHz, DMSO-$d_6$) δ 10.62 (s, 1H), 7.42 (d, *J* = 8.8 Hz, 1H), 6.88 (d, *J* = 8.9 Hz, 1H), 5.04-4.99 (m, 1H), 4.36 (t, *J* = 9.9 Hz, 1H), 4.13-4.08 (m, 1H), 3.99-3.97 (m, 2H), 3.60 (d, *J* = 12.4 Hz, 2H), 3.43-3.41 (m, 1H), 3.32 (t, *J* = 11.3 Hz, 2H), 3.14-3.01 (m, 5H), 2.22 (t, *J* = 12.9 Hz, 2H), 1.97 (d, *J* = 12.4 Hz, 4H), 1.73-1.61 (m, 2H).

**Example 23. 3,4-Dichloro-2-(3-(1-(p-tolyl)piperidin-4-yl)-6,7-dihydro-5*H*-pyrrolo[2,1-*c*][1,2,4]triazol-6-yl)phenol**

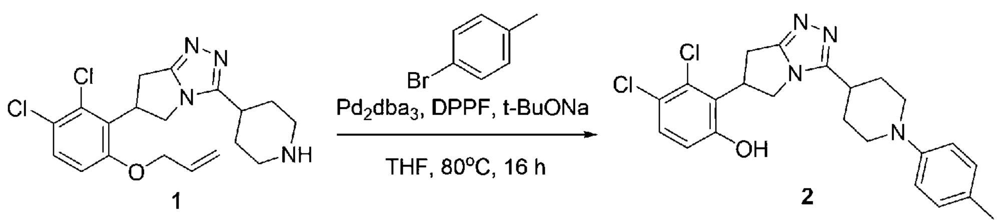


At room temperature, tris(dibenzylideneacetone)dipalladium(0) (46.5 mg, 0.05 mmol), 1,1'-bis(diphenylphosphino)ferrocene (28.1 mg, 0.05 mmol), and sodium *tert*-butoxide (97.7 mg, 1.02 mmol) were added to a solution of 6-(6-(allyloxy)-2,3-dichlorophenyl)-3-(piperidin-4-yl)-6,7-dihydro-5*H*-pyrrolo[2,1-*c*][1,2,4]triazole (200.0 mg, 0.50 mmol) and 1-bromo-4-methylbenzene (86.9 mg, 0.50 mmol) in tetrahydrofuran (5.0 mL). The mixture was stirred at 80 °C for 16 h under an argon atmosphere. After completion of the reaction, the mixture was diluted by adding water (10 mL) and extracted three times with ethyl acetate (10 mL). The organic phases were combined, washed with saturated brine (20 mL), dried over anhydrous sodium sulfate, and then concentrated under reduced pressure. The residue was purified by silica gel column chromatography and eluted with dichloromethane:methanol = 10:1 to give a crude product, which was then subjected to preparative chromatography to give a white solid (1.2 mg, yield: 0.53%).

LCMS $[M+H]^+$: 443.2.

$^1H$ NMR (400 MHz, DMSO-$d_6$) δ 10.57 (s, 1H), 7.40 (d, *J*=8.8, 1H), 7.02 (d, *J*=8.2, 2H), 6.86 (d, *J*=8.6, 2H), 5.00 - 4.95 (m, 1H), 4.31 (s, 1H), 4.09 (s, 1H), 3.65 (s, 2H), 3.14 (d, *J*=5.5, 1H), 3.05 (d, *J*=7.4, 1H), 2.92 (s, 1H), 2.74 (t, *J*=11.1, 2H), 2.19 (s, 3H), 2.02 - 1.96 (m, 2H), 1.84 - 1.78 (m, 2H).

Example 24. 3,4-Dichloro-2-(3-(1-(4-trifluoromethylphenyl)piperidin-4-yl)-6,7-dihydro-5H-pyrrolo[2,1-*c*][1,2,4]triazol-6-yl)phenol

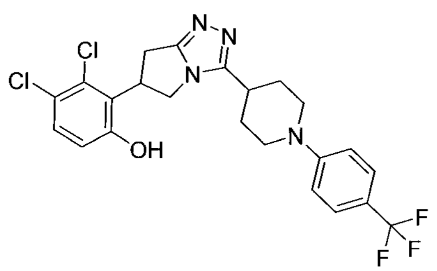

This example was prepared by reference to the preparation method for Example 23, except that the 1-bromo-4-methylbenzene was replaced by 1-bromo-4-trifluoromethylbenzene.
LCMS $[M+H]^+$: 497.2.
$^1$H NMR (400 MHz, DMSO-d6) δ 8.48 (s, 2H), 7.47 (d, J=8.8, 2H), 7.31 (d, J=8.7, 1H), 7.07 (d, J=8.7, 2H), 6.80 (d, J=8.8, 1H), 5.19 (s, 1H), 4.39 (t, J=9.9, 1H), 4.27 (dd, J=10.3, 7.2, 1H), 4.01 - 3.91 (m, 2H), 3.23 (dd, J=8.8, 4.2, 2H), 3.21 - 2.99 (m, 2H), 2.20 - 1.94 (m, 5H).

**Example 25. 3,4-Dichloro-2-(3-(difluoromethyl)-6,7-dihydro-5*H*-pyrrolo[2,1-*c*][1,2,4]triazol-6-yl)phenol**

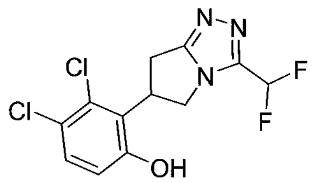

This example was prepared by reference to the preparation method for Example 3, except that the ethyl 3-hydroxy-3-methylbutanoate was replaced by methyl 2,2-difluoroacetate.
LCMS $[M+H]^+$: 320.1.
$^1$H NMR (400 MHz, DMSO-d6) δ 10.59 (s, 1H), 7.46 - 7.20 (m, 2H), 6.85 (d, *J* = 8.8 Hz, 1H), 5.09 - 5.03 (m, 1H), 4.43 (t, *J* = 10.2 Hz, 1H), 4.15 (dd, *J* = 10.7, 6.8 Hz, 1H), 3.30 - 3.25 (m, 1H), 3.07 - 3.01 (m, 1H).

**Example 26. 3,4-Dichloro-2-(3-ethyl-6,7-dihydro-5*H*-pyrrolo[2,1-*c*][1,2,4]triazol-6-yl)-6-methoxyphenol**

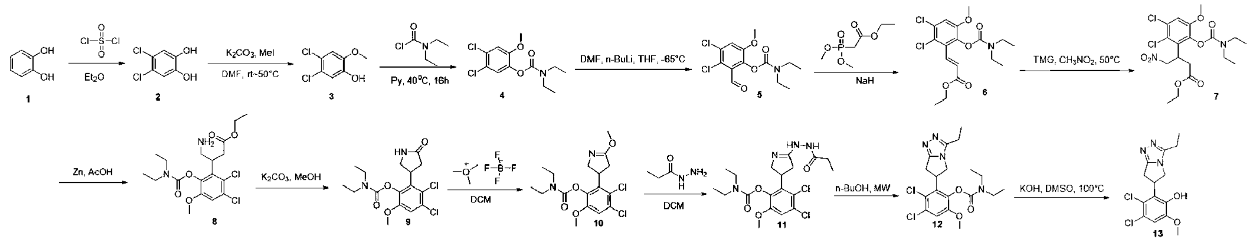


**Synthesis of 4,5-dichlorobenzene-1,2-diol (2):**

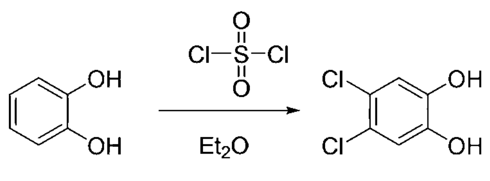


1 2

The compound pyrocatechol (19.5 g, 177.1 mmol) was dissolved in an ethyl ether solution (80.0 mL). At 0 °C, the compound sulfonyl chloride (21.9 g, 162.3 mmol) was slowly added to the mixture. The resulting mixture was stirred at room temperature for 1 h under a nitrogen atmosphere. The reaction mixture was concentrated *in vacuo,* and the concentrate was slurried with petroleum ether and then filtered to give a gray solid (23.0 g, yield: 73%).
LCMS $[M+H]^+$: 176.9.

**Synthesis of 4,5-dichloro-2-methoxyphenol (3):**

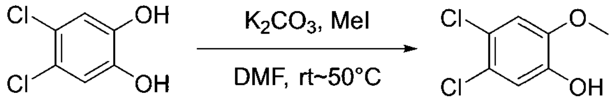


**2** **3**

The compound 4,5-dichlorobenzene-1,2-diol (100 mg, 0.56 mmol) and potassium carbonate (77.22 mg, 0.56 mmol) were dissolved in an *N*,*N*-dimethylformamide solution (2.0 mL). At 0 °C, iodomethane (21.9 mg, 0.15 mmol) was added to the mixture. The resulting mixture was stirred at 50 °C for 2 h. The reaction mixture was diluted with ethyl acetate and washed twice with brine. The resulting organic phase was dried over anhydrous sodium sulfate and concentrated *in vacuo.* The resulting crude product was purified by silica gel chromatography (petroleum ether:ethyl acetate = 3:2) to give a pale yellow solid (60.0 mg, yield: 56%).
LCMS $[M+H]^+$: 190.9.

**Synthesis of 4,5-dichloro-2-methoxyphenyl diethylcarbamate (4):**

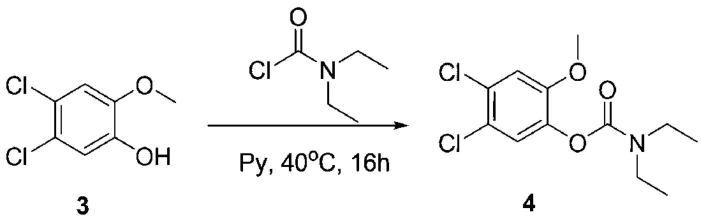


**3** **4**

The compound 4,5-dichloro-2-methoxyphenol (4.8 g, 24.87 mmol) was dissolved in pyridine (24.0 mL). At room temperature, the compound diethylcarbamic chloride (6.75 g, 49.78 mmol) was added to the mixture. The resulting mixture was stirred at 40 °C for 16 h. The reaction mixture was concentrated *in vacuo* to remove the pyridine, and the residue was diluted with ethyl acetate and washed twice with brine. The resulting organic phase was dried over anhydrous sodium sulfate and concentrated *in vacuo.* The resulting crude product was purified by silica gel chromatography (petroleum ether:ethyl acetate = 10:1) to give a pale yellow solid (5.4 g, yield: 74%).
LCMS $[M+H]^+$: 291.9.

**Synthesis of 3,4-dichloro-2-formyl-6-methoxyphenyl diethylcarbamate (5):**

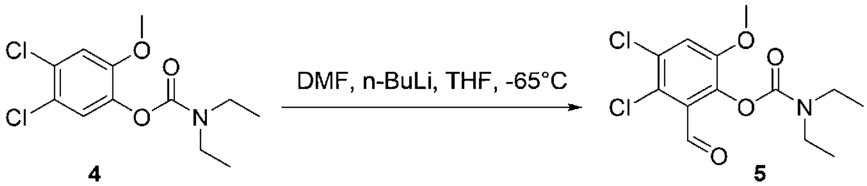


**4** **5**

The compound 4,5-dichloro-2-methoxyphenyl diethylcarbamate (5.40 g, 18.50 mmol) was dissolved in tetrahydrofuran (108.0 mL). At -65 °C, n-butyllithium (1.42 g, 22.20 mmol) was added to the mixture. The resulting mixture was stirred at -65 °C for 30 min under a nitrogen atmosphere. At -65 °C, a solution of N,N-dimethylformamide (2.03 g, 27.75 mmol) was added to the mixture. The resulting mixture was stirred at -65 °C for another 1 h under a nitrogen atmosphere. The reaction mixture was quenched with a saturated ammonium chloride solution, extracted three times with ethyl acetate, and washed twice with brine. The resulting organic phase was dried over anhydrous sodium sulfate and concentrated *in vacuo.* The resulting crude product was purified by silica gel chromatography (petroleum ether:ethyl acetate = 4:1) to give a pale yellow solid (2.2 g, yield: 37%).
LCMS $[M+H]^+$: 319.8.

**Synthesis of ethyl (*E*)-3-(2,3-dichloro-6-((diethylcarbamoyl)oxy)-5-methoxyphenyl)acrylate (6):**

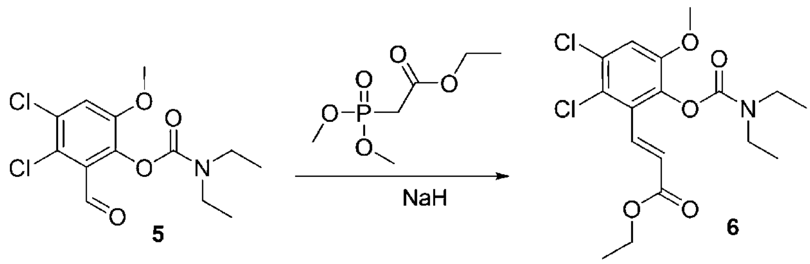


5

6

The compound ethyl 2-(dimethoxyphosphoryl)acetate (2.03 g, 10.35 mmol) was dissolved in tetrahydrofuran (44.0 mL). At 0 °C, sodium hydride (0.25 g, 6.25 mmol, 60% in mineral oil) was added to the mixture. The resulting mixture was stirred at 0 °C for 30 min. At 0 °C, the compound 3,4-dichloro-2-formyl-6-methoxyphenyl diethylcarbamate (2.2 g, 6.90 mmol) was added to the mixture. The resulting mixture was stirred at room temperature for 1 h. The reaction mixture was quenched with a saturated ammonium chloride solution, extracted three times with ethyl acetate, and washed twice with brine. The resulting organic phase was dried over anhydrous sodium sulfate and concentrated *in vacuo.* The resulting crude product was purified by silica gel chromatography (petroleum ether:ethyl acetate = 4:1) to give a white solid (2.2 g, yield: 81%).

LCMS $[M+H]^+$: 389.9.

**Synthesis of ethyl 3-(2,3-dichloro-6-((diethylcarbamoyl)oxy)-5-methoxyphenyl)-4-nitrobutanoate (7):**

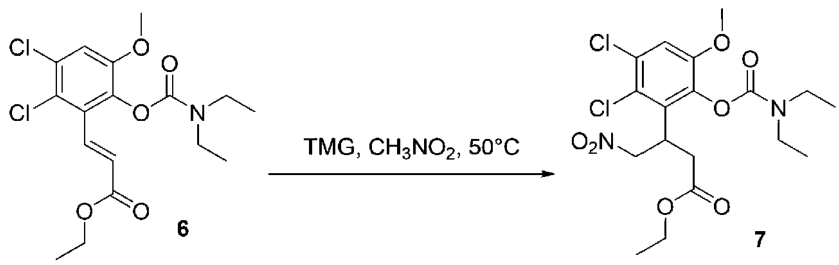


6

7

The compound ethyl (*E*)-3-(2,3-dichloro-6-((diethylcarbamoyl)oxy)-5-methoxyphenyl)acrylate (1.7 g, 4.40 mmol) was dissolved in nitromethane (17.0 mL). At room temperature, the compound tetramethylguanidine (100.0 mg, 0.88 mmol) was added to the mixture. The resulting mixture was stirred at 50 °C for 3 h. The reaction mixture was diluted with ethyl acetate and washed twice with brine. The resulting organic phase was dried over anhydrous sodium sulfate and concentrated *in vacuo.* The resulting crude product was purified by silica gel chromatography (petroleum ether:ethyl acetate = 3:1) to give a pale yellow solid (0.8 g, yield: 41%). LCMS $[M+H]^+$: 450.8.

**Synthesis of ethyl 4-amino-3-(2,3-dichloro-6-((diethylcarbamoyl)oxy)-5-methoxyphenyl)butanoate (8):**

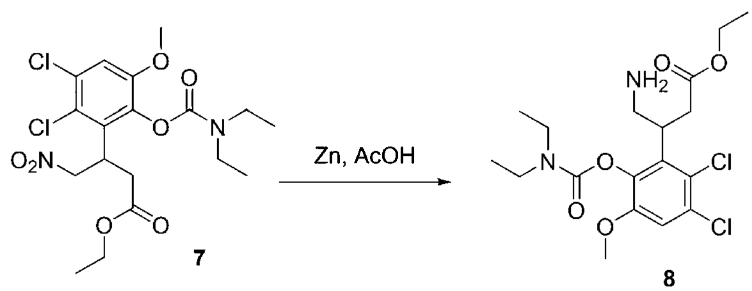


7

8

The compound ethyl 3-(2,3-dichloro-6-((diethylcarbamoyl)oxy)-5-methoxyphenyl)-4-nitrobutanoate (240.0 mg, 0.53 mmol) was dissolved in glacial acetic acid (5.0 mL). At room temperature, zinc powder (347.9 mg, 5.32 mmol) was added to the mixture. The resulting mixture was stirred at room temperature for 16 h. The reaction mixture was filtered, and the filter cake was washed with methanol. The resulting filtrate was concentrated *in vacuo,* and the pH was adjusted to 8

with a saturated aqueous sodium carbonate solution. The resulting mixture was extracted three times with ethyl acetate and washed twice with brine. The resulting organic phase was dried over anhydrous sodium sulfate and concentrated *in vacuo.* The resulting crude product was purified by silica gel chromatography (dichloromethane:methanol = 10:1) to give a colorless oily liquid (200.0 mg, yield: 89%).

LCMS $[M+H]^+$: 421.1.

**Synthesis of 3,4-dichloro-6-methoxy-2-(5-oxopyrrolidin-3-yl)phenyl diethylcarbamate (9):**

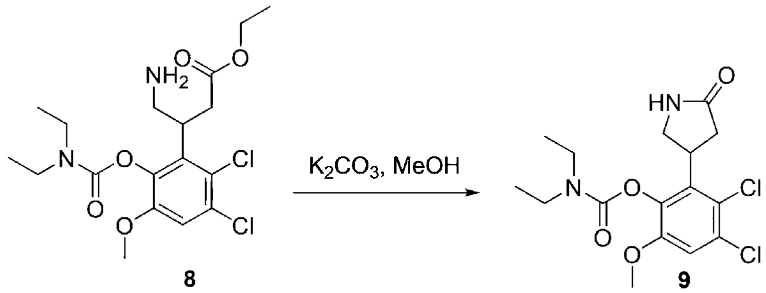


8

9

The compound ethyl 4-amino-3-(2,3-dichloro-6-((diethylcarbamoyl)oxy)-5-methoxyphenyl)butanoate (600 mg, 1.42 mmol) was dissolved in methanol (12.0 mL). At room temperature, potassium carbonate (590.5 mg, 4.27 mmol) was added to the mixture. The resulting mixture was stirred at room temperature for 16 h. The reaction mixture was diluted with ethyl acetate and washed twice with brine. The resulting organic phase was dried over anhydrous sodium sulfate and concentrated *in vacuo.* The resulting crude product was purified by silica gel chromatography (dichloromethane:methanol = 10:1) to give a white solid (450.0 mg, yield: 84%).

LCMS $[M+H]^+$: 375.0.

**Synthesis of 3,4-dichloro-6-methoxy-2-(5-methoxy-3,4-dihydro-2*H*-pyrrol-3-yl)phenyl diethylcarbamate (10):**

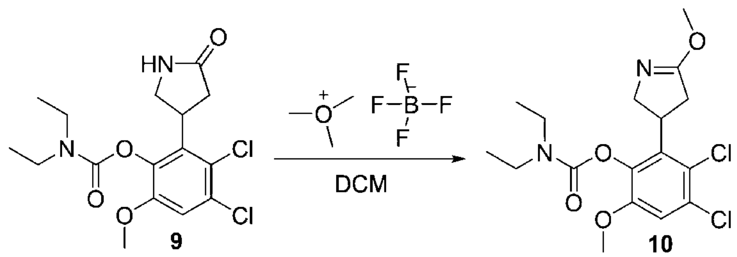


9

10

The compound 3,4-dichloro-6-methoxy-2-(5-oxopyrrolidin-3-yl)phenyl diethylcarbamate (600.0 mg, 1.60 mmol) was dissolved in dichloromethane (12.0 mL). At room temperature, the compound trimethyloxonium tetrafluoroborate (283.8 mg, 1.92 mmol) was added to the mixture. The resulting mixture was stirred at 30 °C for 3 h. The reaction mixture was concentrated *in vacuo* to give a white solid (600.0 mg, yield: 96%).

LCMS $[M+H]^+$: 389.0.

**Synthesis of 3,4-dichloro-6-methoxy-2-(5-(2-propionylhydrazino)-3,4-dihydro-2*H*-pyrrol-3-yl)phenyl diethylcarbamate (11):**

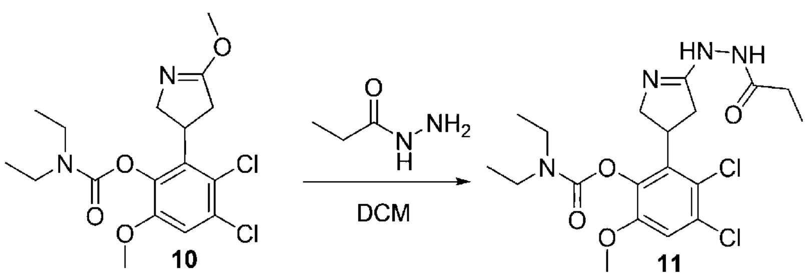


The compound 3,4-dichloro-6-methoxy-2-(5-methoxy-3,4-dihydro-2*H*-pyrrol-3-yl)phenyl diethylcarbamate (600.0 mg, 1.54 mmol) was dissolved in a dichloromethane solution (12.0 mL). At room temperature, propionohydrazide (407.4 mg, 4.62 mmol) was added to the mixture. The resulting mixture was stirred at room temperature for 16 h. The reaction mixture was concentrated *in vacuo.* The resulting crude product was purified by silica gel chromatography (dichloromethane:methanol = 10:1) to give a colorless oily liquid (650.0 mg, yield: 95%).

LCMS $[M+H]^+$: 444.9.

**Synthesis of 3,4-dichloro-2-(3-ethyl-6,7-dihydro-5*H*-pyrrolo[2,1-*c*][1,2,4]triazol-6-yl)-6-methoxyphenyl diethylcarbamate (12):**

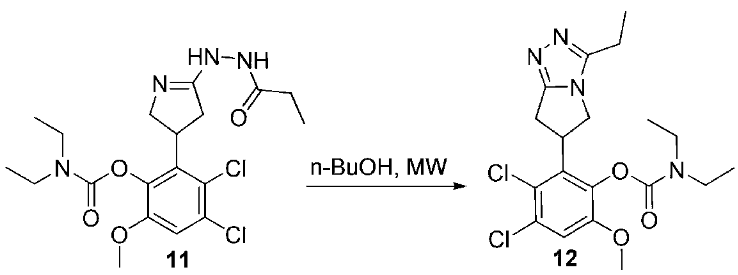


The compound 3,4-dichloro-6-methoxy-2-(5-(2-propionylhydrazino)-3,4-dihydro-2*H*-pyrrol-3-yl)phenyl diethylcarbamate (650.0 mg, 1.46 mmol) was dissolved in an n-butanol solution (13.0 mL), and the resulting mixture was stirred under microwave radiation at 150 °C for 1.5 h. The reaction mixture was concentrated *in vacuo.* The resulting crude product was purified by silica gel chromatography (dichloromethane:methanol = 5:1) to give a white solid (460.0 mg, yield: 74%).

LCMS $[M+H]^+$: 427.0.

**Synthesis of 3,4-dichloro-2-(3-ethyl-6,7-dihydro-5*H*-pyrrolo[2,1-*c*][1,2,4]triazol-6-yl)-6-methoxyphenol (13):**

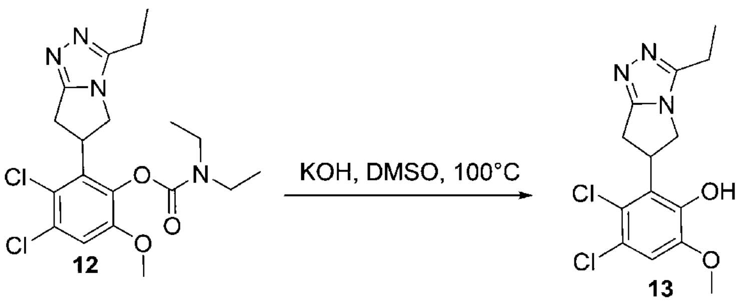


The compound 3,4-dichloro-2-(3-ethyl-6,7-dihydro-5*H*-pyrrolo[2,1-*c*][1,2,4]triazol-6-yl)-6-methoxyphenyl diethylcarbamate (25.0 mg, 0.059 mmol) was dissolved in a dimethylsulfoxide solution (2.5 mL), and potassium hydroxide (16.4 mg, 0.29 mmol) was added to the reaction mixture. The resulting mixture was allowed to react at 100 °C for 4 h. The reaction mixture was diluted with ethyl acetate and washed twice with brine. The resulting organic phase was dried over anhydrous sodium sulfate and concentrated *in vacuo.* The resulting crude product was separated by preparative

chromatography to give a white solid (4.1 mg, yield: 21%).

LCMS [M+H]+: 328.0.

$^1$H NMR (400 MHz, DMSO-$d_6$) δ 9.80 (s, 1H), 7.22 (s, 1H), 4.97 - 4.90 (m, 1H), 4.21 (t, *J* = 9.8 Hz, 1H), 4.05 - 3.99 (m, 1H), 3.82 (s, 3H), 3.12 - 3.05 (m, 2H), 2.67 (d, *J*= 7.6 Hz, 2H), 1.20 (t, *J* = 7.6 Hz, 3H).

**Example 27. 3,4-Dichloro-2-(3-ethyl-6,7-dihydro-5*H*-pyrrolo[2,1-*c*][1,2,4]triazol-6-yl)-6-methylphenol**

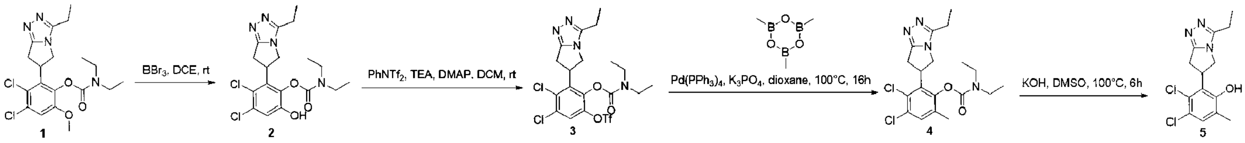


**Synthesis of 3,4-dichloro-2-(3-ethyl-6,7-dihydro-5*H*-pyrrolo[2,1-*c*][1,2,4]triazol-6-yl)-6-hydroxyphenyl diethylcarbamate (2):**

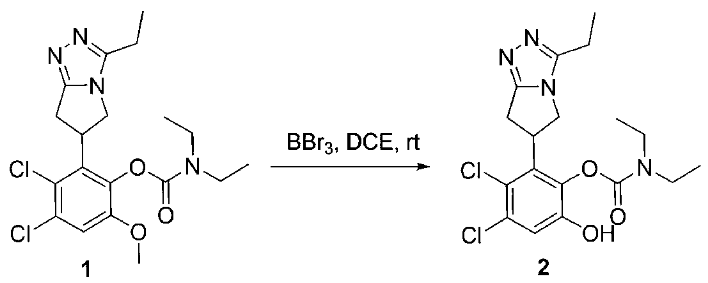


The compound 3,4-dichloro-2-(3-ethyl-6,7-dihydro-5*H*-pyrrolo[2,1-c][1,2,4]triazol-6-yl)-6-methoxyphenyl diethylcarbamate (480.0 mg, 1.12 mmol) was dissolved in a dichloroethane solution (10.0 mL). At room temperature, boron tribromide (562.8 mg, 2.25 mmol) was added to the mixture. The resulting mixture was stirred at room temperature for 1 h. The reaction mixture was quenched with ice water, and the pH was adjusted to 8 with a saturated aqueous sodium bicarbonate solution. The resulting mixture was extracted three times with ethyl acetate and washed twice with brine. The resulting organic phase was dried over anhydrous sodium sulfate and concentrated *in vacuo.* The resulting crude product was purified by silica gel chromatography (dichloromethane:methanol = 7:1) to give a gray solid (300.0 mg, yield: 65%).

LCMS [M+H]+: 413.0.

**Synthesis of 4,5-dichloro-2-((diethylcarbamoyl)oxy)-3-(3-ethyl-6,7-dihydro-5H-pyrrolo[2,1-*c*][1,2,4]triazol-6-yl) phenyl trifluoromethanesulfonate (3):**

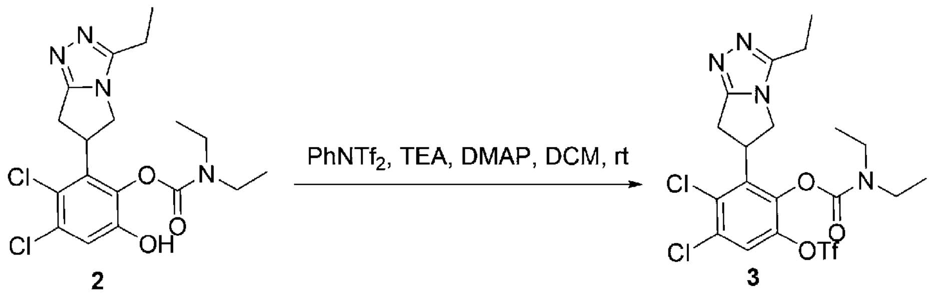


The compound 3,4-dichloro-2-(3-ethyl-6,7-dihydro-5*H*-pyrrolo[2,1-*c*][1,2,4]triazol-6-yl)-6-hydroxyphenyl diethylcarbamate (100.0 mg, 0.24 mmol) and 4-dimethylaminopyridine (14.8 mg, 0.12 mmol) were dissolved in dichloromethane (5.0 mL). At 0 °C, *N*-phenyl-bis(trifluoromethanesulfonimide) (172.9 mg, 0.48 mmol) and triethylamine (10.1 mg, 0.1 mmol) were added to the mixture. The resulting mixture was stirred at room temperature for 3 h. The reaction

mixture was diluted with ethyl acetate and washed twice with brine. The resulting organic phase was dried over anhydrous sodium sulfate and concentrated *in vacuo.* The resulting crude product was purified by silica gel chromatography (dichloromethane:methanol = 7:1) to give a colorless oily liquid (60.0 mg, yield: 45%).

LCMS $[M+H]^+$: 544.7.

**Synthesis of 3,4-dichloro-2-(3-ethyl-6,7-dihydro-5*H*-pyrrolo[2,1-*c*][1,2,4]triazol-6-yl)-6-methylphenyl diethylcarbamate (4):**

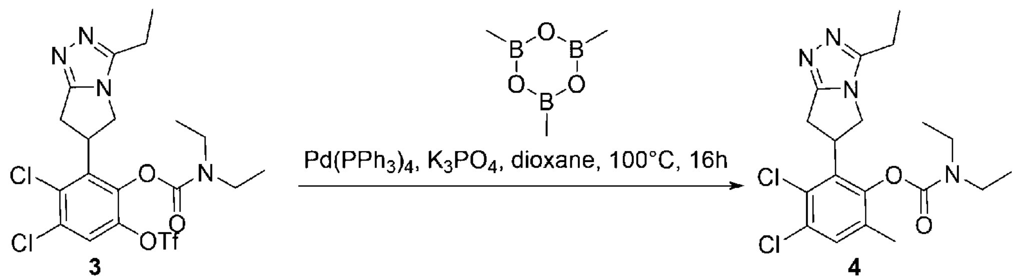


3 4

The compound 4,5-dichloro-2-((diethylcarbamoyl)oxy)-3-(3-ethyl-6,7-dihydro-5*H*-pyrrolo[2,1-*c*][1,2,4]triazol-6-yl)phenyl trifluoromethanesulfonate (50.0 mg, 0.092 mmol) and trimethylboroxine (13.8 mg, 0.11 mmol) were dissolved in dioxane (5.0 mL). At room temperature, the compounds tetrakis(triphenylphosphine)palladium(0) (5.30 mg, 0.0046 mmol) and potassium phosphate (29.2 mg, 0.14 mmol) were added to the mixture. The resulting mixture was stirred at 100 °C for 16 h under a nitrogen atmosphere. The reaction mixture was diluted with ethyl acetate and washed twice with brine. The resulting organic phase was dried over anhydrous sodium sulfate and concentrated *in vacuo.* The resulting crude product was purified by silica gel chromatography (dichloromethane:methanol = 14:1) to give a pale yellow solid (30.0 mg, yield: 80%).

LCMS $[M+H]^+$: 410.9.

**Synthesis of 3,4-dichloro-2-(3-ethyl-6,7-dihydro-5H-pyrrolo[2,1-*c*][1,2,4]triazol-6-yl)-6-methylphenol (5):**

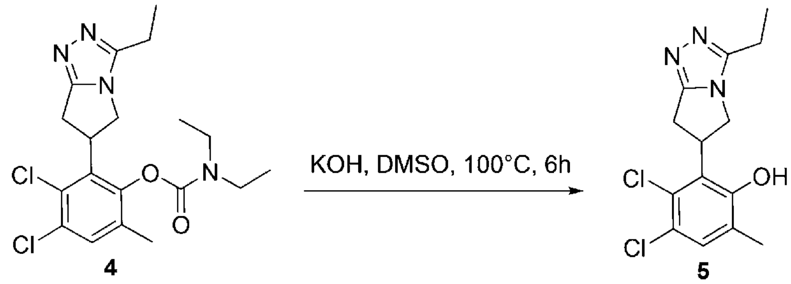


4 5

The compound 3,4-dichloro-2-(3-ethyl-6,7-dihydro-5*H*-pyrrolo[2,1-*c*][1,2,4]triazol-6-yl)-6-methylphenyl diethylcarbamate (30.0 mg, 0.073 mmol) was dissolved in a dimethylsulfoxide solution (3.0 mL), and potassium hydroxide (20.5 mg, 0.37 mmol) was added to the reaction mixture. The resulting mixture was allowed to react at 100 °C for 6 h. The reaction mixture was diluted with ethyl acetate and washed twice with brine. The resulting organic phase was dried over anhydrous sodium sulfate and concentrated *in vacuo.* The resulting crude product was separated by preparative chromatography to give a white solid (0.9 mg, yield: 4%).

LCMS $[M+H]^+$: 312.0.

$^1H$ NMR (400 MHz, DMSO-$d_6$) δ 7.35 (s, 1H), 5.03 - 4.94 (m, 1H), 4.23 (t, *J* = 9.9 Hz, 1H), 4.00 (dd, *J* = 10.1, 7.4 Hz, 1H), 3.08 (dt, *J* = 15.8, 6.9 Hz, 2H), 2.67 (d, *J* = 7.5 Hz, 2H), 2.16 (s, 3H), 1.21 (t, *J* = 7.6 Hz, 3H).

**Example 28. 3,4-Dichloro-2-(3-ethyl-6,7-dihydro-5*H*-pyrrolo[2,1-*c*][1,2,4]triazol-6-yl)-6-isopropylphenol**

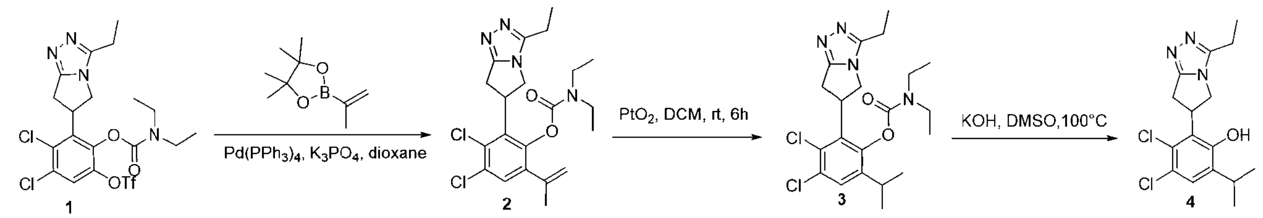

1 2 3 4

**Synthesis of 3,4-dichloro-2-(3-ethyl-6,7-dihydro-5*H*-pyrrolo[2,1-*c*][1,2,4]triazol-6-yl)-6-(prop-1-en-2-yl)phenyl diethylcarbamate (2):**

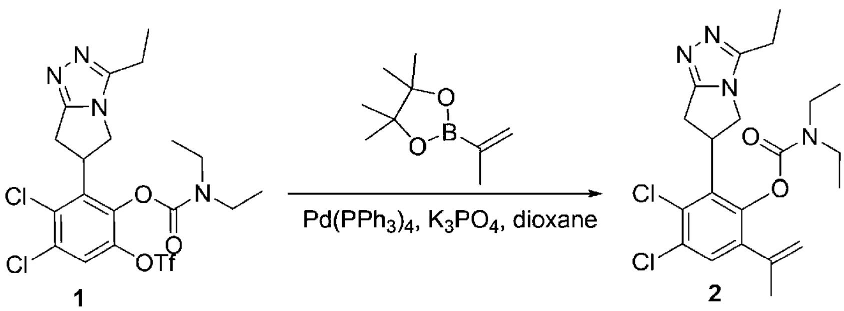

1 2

The compound 4,5-dichloro-2-((diethylcarbamoyl)oxy)-3-(3-ethyl-6,7-dihydro-5*H*-pyrrolo[2,1-*c*][1,2,4]triazol-6-yl)phenyl trifluoromethanesulfonate (80.0 mg, 0.15 mmol) and 4,4,5,5-tetramethyl-2-(prop-1-en-2-yl)-1,3,2-dioxaborolane (123.3 mg, 0.73 mmol) were dissolved in dioxane (8.0 mL). At room temperature, the compounds tetrakis(triphenylphosphine)palladium(0) (33.9 mg, 0.029 mmol) and potassium phosphate (155.7 mg, 0.73 mmol) were added to the mixture. The resulting mixture was heated and stirred in a microwave reactor at 100 °C for 30 min under a nitrogen atmosphere. The reaction mixture was diluted with water, extracted three times with ethyl acetate, and washed twice with brine. The resulting organic phase was dried over anhydrous sodium sulfate and concentrated *in vacuo*. The resulting crude product was purified by silica gel chromatography (dichloromethane:methanol = 14:1) to give a yellow oily subtance (40.0 mg, yield: 62%).

LCMS $[M+H]^+$: 436.8.

**Synthesis of 3,4-dichloro-2-(3-ethyl-6,7-dihydro-5*H*-pyrrolo[2,1-*c*][1,2,4]triazol-6-yl)-6-isopropylphenyl diethylcarbamate (3):**

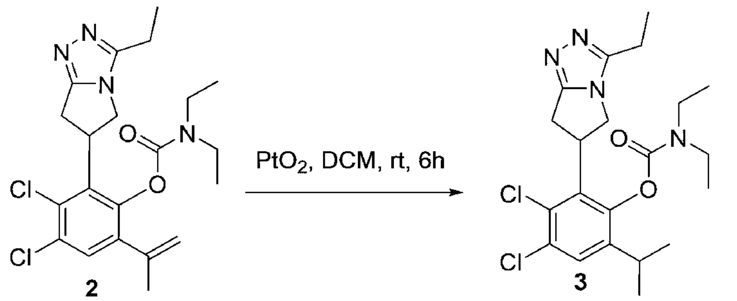

2 3

The compound 3,4-dichloro-2-(3-ethyl-6,7-dihydro-5*H*-pyrrolo[2,1-*c*][1,2,4]triazol-6-yl)-6-(prop-1-en-2-yl)phenyl diethylcarbamate (30.0 mg, 0.073 mmol) was dissolved in a dichloromethane solution (6.0 mL), and platinum dioxide (20.5 mg, 0.37 mmol) was added to the reaction mixture. The resulting mixture was stirred at 25 °C for 6 h. The reaction mixture was filtered through celite. The filter cake was washed with methanol, and the filtrate was collected and concentrated *in vacuo* to give a yellow oily subtance (25.0 mg, yield: 83%).

LCMS $[M+H]^+$: 438.9.

**Synthesis of 3,4-dichloro-2-(3-ethyl-6,7-dihydro-5*H*-pyrrolo[2,1-*c*][1,2,4]triazol-6-yl)-6-isopropylphenol (4):**

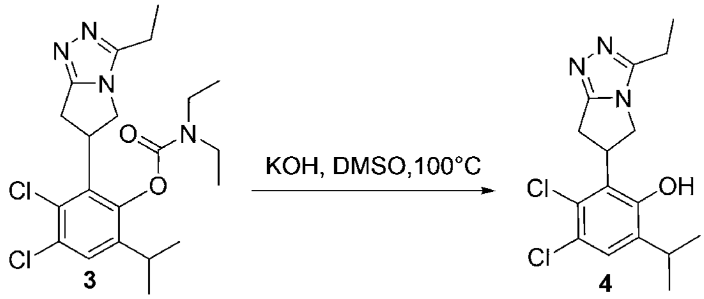


3 4

The compound 3,4-dichloro-2-(3-ethyl-6,7-dihydro-5*H*-pyrrolo[2,1-*c*][1,2,4]triazol-6-yl)-6-isopropylphenyl diethylcarbamate (25.0 mg, 0.057 mmol) was dissolved in a dimethylsulfoxide solution (2.5 mL), and potassium hydroxide (16.0 mg, 0.28 mmol) was added to the reaction mixture. The resulting mixture was allowed to react at 100 °C for 1 h. The reaction mixture was diluted with water, extracted three times with ethyl acetate, and washed twice with brine. The resulting organic phase was dried over anhydrous sodium sulfate and concentrated *in vacuo.* The resulting crude product was separated by preparative chromatography to give a white solid (4.0 mg, yield: 4%).

LCMS $[M+H]^+$: 339.8.

$^1H$ NMR (400 MHz, DMSO-$d_6$) δ 7.28 (s, 1H), 5.02 - 4.92 (m, 1H), 4.19 (t, *J* = 10.0 Hz, 1H), 3.98 (dd, *J* = 10.0, 7.4 Hz, 1H), 3.20 (dd, *J* = 13.7, 6.8 Hz, 1H), 3.13 - 2.97 (m, 2H), 2.63 (q, *J* = 7.6 Hz, 2H), 1.17 (t, *J* = 7.6 Hz, 3H), 1.08 (d, *J* = 6.7 Hz, 6H).

**Examples 29/30. (*S*)-3,4,6-Trichloro-2-(3-ethyl-6,7-dihydro-5*H*-pyrrolo[2,1-*c*][1,2,4]triazol-6-yl)phenol and (*R*)-3,4,6-trichloro-2-(3-ethyl-6,7-dihydro-5*H*-pyrrolo[2,1-*c*][1,2,4]triazol-6-yl)phenol**

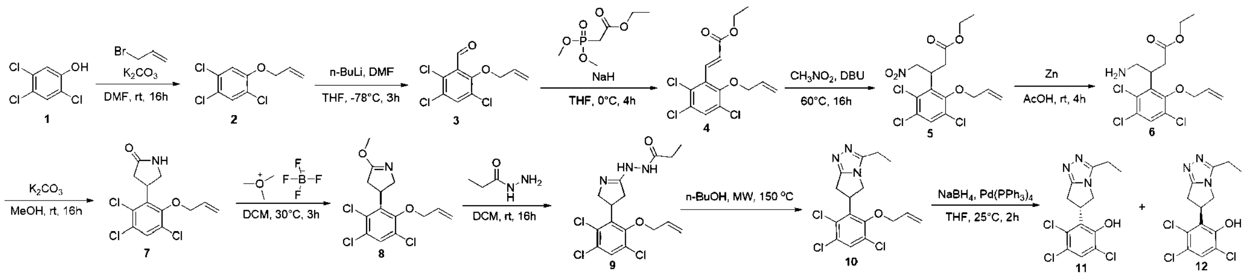


**Synthesis of 1-(allyloxy)-2,4,5-trichlorobenzene (2):**

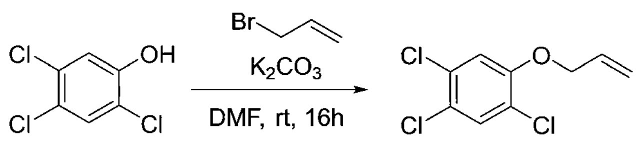


1 2

2,4,5-Trichlorophenol (10.0 g, 50.65 mmol) and potassium carbonate (14.1 g, 102.02 mmol) were mixed in *N*,*N*-dimethylformamide (150.0 mL), and allyl bromide (7.4 g, 61.16 mmol) was added in portions at 0 °C. The resulting mixture was stirred at room temperature for 16 h. After completion of the reaction, the mixture was diluted with water (150.0 mL) and extracted twice with ethyl acetate (150.0 mL). The organic phases were combined, washed with saturated brine (150.0 mL), and dried over anhydrous sodium sulfate. After filtration, the filtrate was concentrated under reduced pressure, and the resulting residue was purified by silica gel column chromatography (petroleum ether:ethyl acetate = 20:1) to give a yellow solid (10.0 g, yield: 83%).

**Synthesis of 2-(allyloxy)-3,5,6-trichlorobenzaldehyde (3):**

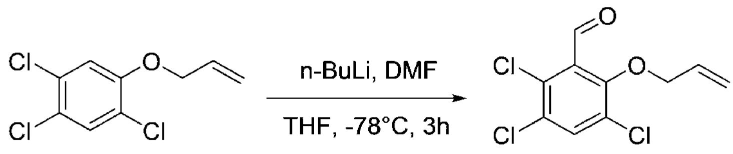


2 3

At -78 °C, 1-(allyloxy)-2,4,5-trichlorobenzene (500.0 mg, 2.12 mmol) was dissolved in tetrahydrofuran (10.0 mL), and n-butyllithium (1.0 mL, 2.54 mmol, 2.5 M in n-hexane) was added dropwise over a period of 30 min or longer. After completion of the dropwise addition, the mixture was stirred for 30 min, and then N,N-dimethylformamide (198.0 mg, 2.54 mmol) was added at the same temperature. The resulting mixture was then stirred at the same temperature for another 2 h. After completion of the reaction, the reaction mixture was quenched with a saturated aqueous ammonium chloride solution (10.0 mL) and extracted twice with ethyl acetate (30.0 mL). The organic phases were combined, washed with saturated brine (15.0 mL), dried over anhydrous sodium sulfate, and then concentrated under reduced pressure. The residue was purified by silica gel column chromatography and eluted (petroleum ether:ethyl acetate = 15:1) to give the target product (370.0 mg, yield: 47%).

**Synthesis of ethyl (*E*)-3-(2-(allyloxy)-3,5,6-trichlorophenyl)acrylate (4):**

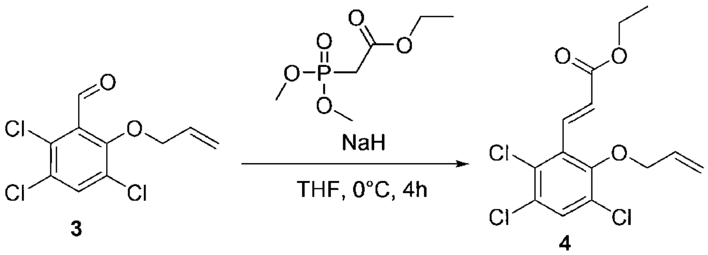


3 4

At 0 °C, sodium hydride (112.0 mg, 2.80 mmol, 60% in mineral oil) was added to a solution of ethyl 2-(dimethoxyphosphoryl)acetate (412.0 mg, 2.10 mmol) in tetrahydrofuran (10.0 mL). The mixture was stirred at 0 °C for 30 min, and then 2-(allyloxy)-3,5,6-trichlorobenzaldehyde (370 mg, 1.40 mmol) was added. The resulting mixture was stirred at room temperature for 4 h. After completion of the reaction, the reaction mixture was diluted with water (10.0 mL) and extracted with ethyl acetate (20.0 mL). The organic phase was washed with saturated brine (20.0 mL), dried over anhydrous sodium sulfate, and concentrated under reduced pressure. The resulting residue was purified by silica gel column chromatography (petroleum ether:ethyl acetate = 5:1) to give a yellow solid (370.0 mg, yield: 79%).

**Synthesis of ethyl 3-(2-(allyloxy)-3,5,6-trichlorophenyl)-4-nitrobutanoate (5):**

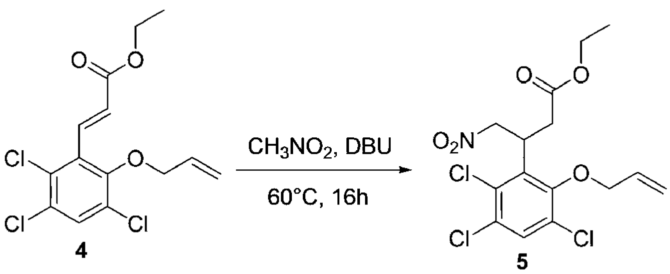


4 5

At room temperature, ethyl (*E*)-3-(2-(allyloxy)-3,5,6-trichlorophenyl)acrylate (370.0 mg, 1.11 mmol) and 1,8-diazabicyclo[5.4.0]undec-7-ene (201.0 mg, 1.33 mmol) were added to a reaction flask containing nitromethane (5.0 mL).

The reaction mixture was stirred at 60 °C for 16 h. After completion of the reaction, the reaction mixture was diluted with water (10.0 mL) and extracted with ethyl acetate (20.0 mL). The organic phase was washed with saturated brine (10.0 mL), dried over anhydrous sodium sulfate, and then concentrated under reduced pressure. The residue was purified by silica gel column chromatography (petroleum ether:ethyl acetate = 5:1) to give a pale yellow oily subtance (270.0 mg, yield: 62%).

**Synthesis of ethyl 3-(2-(allyloxy)-3,5,6-trichlorophenyl)-4-aminobutanoate (6):**

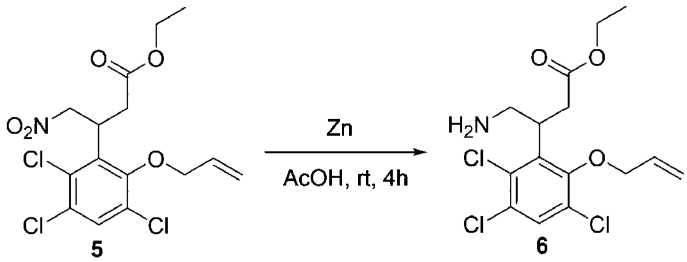


5 6

At room temperature, ethyl 3-(2-(allyloxy)-3,5,6-trichlorophenyl)-4-nitrobutanoate (270.0 mg, 0.68 mmol) and zinc powder (445.0 mg, 6.80 mmol) were added to a reaction flask containing glacial acetic acid (5.0 mL). The mixture was stirred at room temperature for 4 h. After completion of the reaction, the reaction mixture was filtered, and the filter cake was washed twice with ethyl acetate. The filtrate was collected and concentrated under reduced pressure to give a crude product (300 mg), which was directly used in the next step without purification.
LCMS $[M+H]^+$: 365.9.

**Synthesis of 4-(2-(allyloxy)-3,5,6-trichlorophenyl)pyrrolidin-2-one (7):**

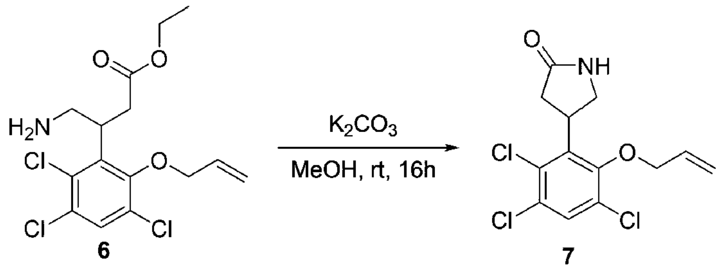


6 7

The crude product of ethyl 3-(2-(allyloxy)-3,5,6-trichlorophenyl)-4-aminobutanoate (300.0 mg, 0.82 mmol) and potassium carbonate (565.0 mg, 4.09 mmol) were added to methanol (7.0 mL), and the mixture was stirred at room temperature for 16 h. After completion of the reaction, the reaction mixture was diluted with water (10 mL) and extracted three times with ethyl acetate (20 mL). The organic phases were combined and then dried over anhydrous sodium sulfate. After filtration, the filtrate was concentrated under reduced pressure. The residue was purified by silica gel column chromatography (petroleum ether: ethyl acetate = 5:1) to give a white solid (150.0 mg, yield: 86%).
LCMS $[M+H]^+$: 320.0.

**Synthesis of 3-(2-(allyloxy)-3,5,6-trichlorophenyl)-5-methoxy-3,4-dihydro-2*H*-pyrrole (8):**

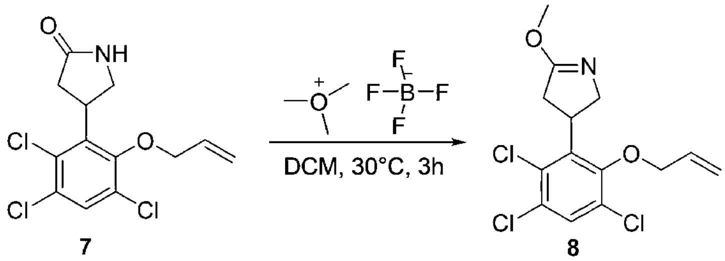


7 8

4-(2-(Allyloxy)-3,5,6-trichlorophenyl)pyrrolidin-2-one (150.0 mg, 0.47 mmol) and trimethyloxonium tetrafluoroborate (84.0 mg, 0.56 mmol) were added to dichloromethane (3.0 mL), and the mixture was stirred at 30 °C for 3 h under a nitrogen atmosphere. After completion of the reaction, the reaction mixture was directly concentrated under reduced pressure to give a crude product (170 mg), which was directly used in the next step without purification.

LCMS $[M+H]^+$: 333.8.

**Synthesis of *N'*-(3-(2-(allyloxy)-3,5,6-trichlorophenyl)-3,4-dihydro-2*H*-pyrrol-5-yl)propionohydrazide (9):**

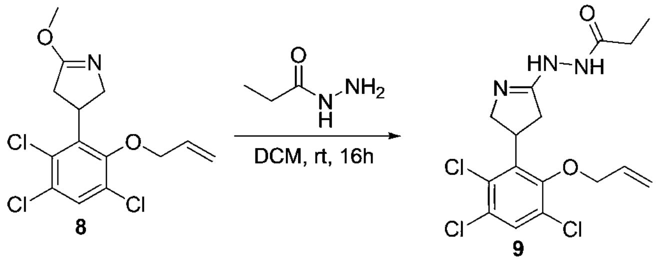


8 9

3-(2-(Allyloxy)-3,5,6-trichlorophenyl)-5-methoxy-3,4-dihydro-2*H*-pyrrole (170.0 mg, 0.51 mmol) and propionohydrazide (135.0 mg, 1.53 mmol) were added to dichloromethane (3.0 mL), and the mixture was stirred at room temperature for 16 h. After completion of the reaction, the reaction mixture was directly concentrated under reduced pressure. The residue was purified by silica gel column chromatography (dichloromethane:methanol = 20:1) to give a white solid (150.0 mg, yield: 90%).

LCMS $[M+H]^+$: 389.8.

**Synthesis of 6-(2-(allyloxy)-3,5,6-trichlorophenyl)-3-ethyl-6,7-dihydro-5*H*-pyrrolo[2,1-*c*][1,2,4]triazole (10):**

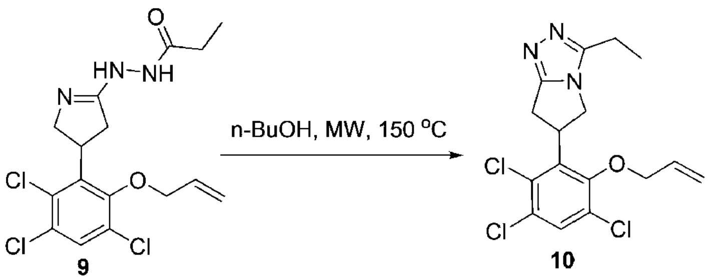


9 10

The compound *N'*-(3-(2-(allyloxy)-3,5,6-trichlorophenyl)-3,4-dihydro-2*H*-pyrrol-5-yl)propionohydrazide (150.0 mg, 0.40 mmol) was dissolved in *n*-butanol (3.0 mL), and the mixture was directly added to a microwave tube and allowed to react at 150 °C for 20 min. After completion of the reaction, the reaction mixture was cooled to room temperature. The mixture was rotary evaporated to dryness, and then the resulting crude product was purified by silica gel chromatography (dichloromethane:methanol = 10:1) to give a white solid (80.0 mg, yield: 95%).

LCMS $[M+H]^+$: 286.0.

**Synthesis of (*S*)-3,4,6-trichloro-2-(3-ethyl-6,7-dihydro-5*H*-pyrrolo[2,1-*c*][1,2,4]triazol-6-yl)phenol and (R)-3,4,6-trichloro-2-(3-ethyl-6,7-dihydro-5*H*-pyrrolo[2,1-*c*][1,2,4]triazol-6-yl)phenol (11/12):**

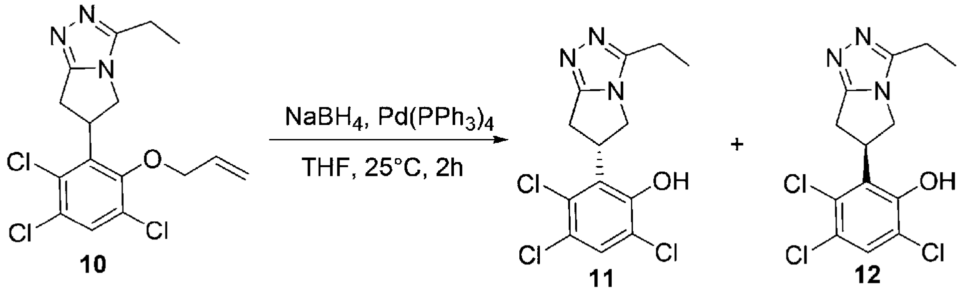


10 11 12

At room temperature, sodium borohydride (16.0 mg, 0.43 mmol) and tetrakis(triphenylphosphine)palladium(0) (12.0 mg, 0.01 mmol) were added to a solution of 6-(2-(allyloxy)-3,5,6-trichlorophenyl)-3-ethyl-6,7-dihydro-5*H*-pyrrolo [2,1-c][1,2,4]triazole (80.0 mg, 0.21 mmol) in tetrahydrofuran (2.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was subjected to preparative chromatography to give a white solid (20.0 mg, yield: 23%), which was further subjected to SFC resolution (chromatographic column: CHIRALPAK AD-H 250 mm × 20 mm, 5 μm; mobile phase: 40% EtOH ($NH_4OH$ 0.2%): 60% $CO_2$) to give a pair of enantiomers. The first compound obtained from the SFC resolution was designated as Example 29, with its RT of 2.19 min analyzed under the analytical chromatographic conditions of SFC.

LC-MS: $[M+H]^+$: 331.9.

$^1H$ NMR (400 MHz, DMSO-*d6*) δ 11.23 (s, 1H), 7.17 (s, 1H), 5.35 - 5.28 (m, 1H), 4.34 (t, *J* = 10.4 Hz, 1H), 4.08 (dd, *J* = 10.8, 7.3 Hz, 1H), 3.25 (s, 1H), 3.07 (dd, *J* = 16.3, 7.5 Hz, 1H), 2.68 (q, *J* = 7.6 Hz, 2H), 1.20 (t, *J* = 7.6 Hz, 3H).

The second compound obtained from the SFC resolution was designated as Example 30, with its RT of 2.9 min analyzed under the analytical chromatographic conditions of SFC.

LC-MS: $[M+H]^+$: 331.9.

$^1H$ NMR (400 MHz, DMSO-d6) δ 7.16 (s, 1H), 5.34 - 5.28 (m, 1H), 4.34 (t, *J* = 10.5 Hz, 1H), 4.08 (dd, *J* = 10.8, 7.3 Hz, 1H), 3.23 (s, 1H), 3.08 (d, *J* = 8.8 Hz, 1H), 2.68 (d, *J* = 7.6 Hz, 2H), 1.20 (t, *J* = 7.6 Hz, 3H).

**Example 31. 3,4-Dichloro-2-(3-ethyl-6,7-dihydro-5*H*-pyrrolo[2,1-*c*][1,2,4]triazol-6-yl)-6-fluorophenol**

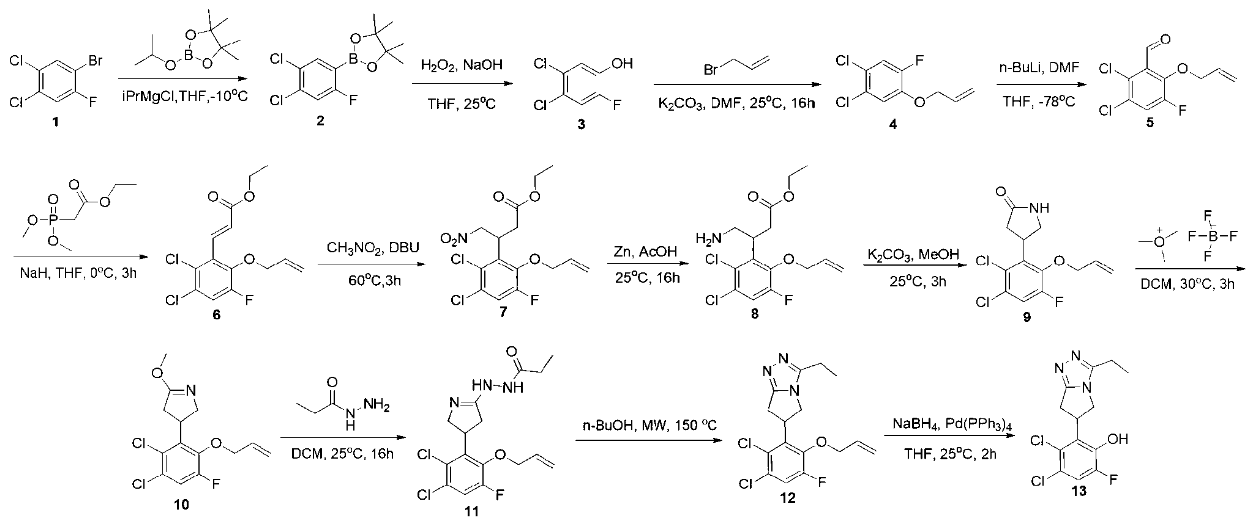


1 2 3 4 5 6 7 8 9 10 11 12 13

**Synthesis of 2-(4,5-dichloro-2-fluorophenyl)-4,4,5,5-tetramethyl-1,3,2-dioxaborolane (2):**

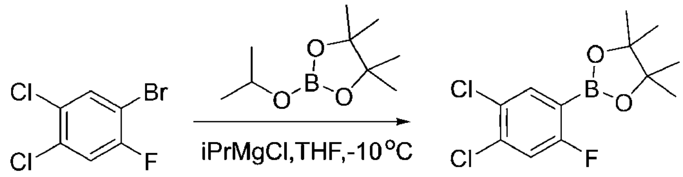


1 2

A solution of 1-bromo-4,5-dichloro-2-fluorobenzene (200.0 mg, 0.82 mmol) in tetrahydrofuran (5 mL) was stirred at -10 °C for 5 min, and then isopropylmagnesium chloride (0.5 mL, 2 M in tetrahydrofuran) was added dropwise to the mixture. The resulting mixture was stirred at -10 °C for 1 h, and then a solution of isopropoxyboronic acid pinacol ester (181.1 mg, 0.98 mmol) in tetrahydrofuran (3 mL) was added dropwise. After completion of the dropwise addition, the mixture was allowed to warm to room temperature naturally and stirred for another 1 h. After completion of the reaction, the mixture was diluted with water (20.0 mL) and extracted twice with ethyl acetate (30.0 mL). The organic phases were combined, washed with saturated brine (50.0 mL), and dried over anhydrous sodium sulfate. After filtration, the filtrate was concentrated under reduced pressure, and the resulting residue was purified by silica gel column chromatography and eluted (dichloromethane:methanol = 20:1) to give a white solid (100.0 mg, yield: 41%).
$^1$H NMR (400 MHz, $CDCl_3$) δ 7.80 (d, *J* = 5.6 Hz, 1H), 7.18 (d, *J* = 8.3 Hz, 1H), 1.36 (s, 12H).

**Synthesis of 4,5-dichloro-2-fluorophenol (3):**

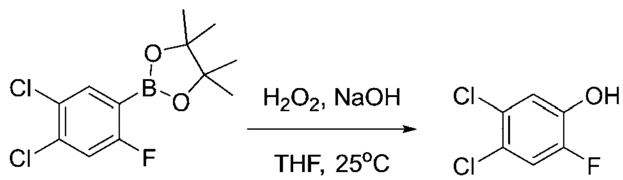


2 3

A 1 M aqueous solution of sodium hydroxide (0.4 g, 10.20 mmol) was added to a solution of 2-(4,5-dichloro-2-fluorophenyl)-4,4,5,5-tetramethyl-1,3,2-dioxaborolane (1.0 g, 3.40 mmol) in tetrahydrofuran (10 mL). The mixture was cooled to 0 °C, and then hydrogen peroxide (0.3 g, 10.20 mmol) was added to the mixture. The resulting mixture was warmed to 25 °C and stirred for 4 h. After completion of the reaction, the reaction mixture was quenched with sodium hydrogen sulfite (10.0 mL) and extracted twice with ethyl acetate (50.0 mL). The organic phases were combined, washed with saturated brine (50.0 mL), and dried over anhydrous sodium sulfate. After filtration, the filtrate was concentrated under reduced pressure, and the resulting residue was directly used in the next step without purification.
LC-MS: $[M+H]^+$: 179.0.

**Synthesis of 1-(allyloxy)-4,5-dichloro-2-fluorobenzene (4):**

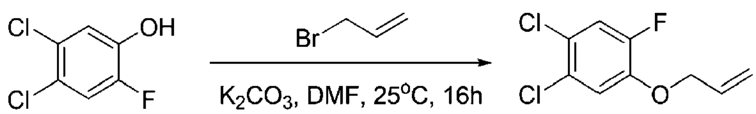


3 4

4,5-Dichloro-2-fluorophenol (1.0 g, 5.50 mmol) and potassium carbonate (1.9 g, 13.75 mmol) were mixed in N,N-dimethylformamide (10.0 mL), and allyl bromide (1.0 g, 8.25 mmol) was added in portions at 0 °C. The mixture was stirred at 25 °C for 16 h. After completion of the reaction, the reaction mixture was diluted with water (20.0 mL) and extracted twice with ethyl acetate (100.0 mL). The organic phases were combined, washed with saturated brine (100.0 mL), and dried over anhydrous sodium sulfate. After filtration, the filtrate was concentrated under reduced pressure, and the resulting residue was purified by silica gel column chromatography and eluted (petroleum ether: ethyl acetate = 20:1) to give a yellow solid (0.8 g, yield: 65%).
$^1$H NMR (400 MHz, DMSO-$d_6$) δ 7.69 (d, *J* = 10.9 Hz, 1H), 7.49 (d, *J* = 8.3 Hz, 1H), 6.04 (ddd, *J* = 22.5, 10.6, 5.4 Hz, 1H), 5.42 (dd, *J* = 17.3, 1.6 Hz, 1H), 5.31 (dd, *J* = 10.5, 1.3 Hz, 1H), 4.70 (d, *J* = 5.4 Hz, 2H).

**Synthesis of 2-(allyloxy)-5,6-dichloro-3-fluorobenzaldehyde (5):**

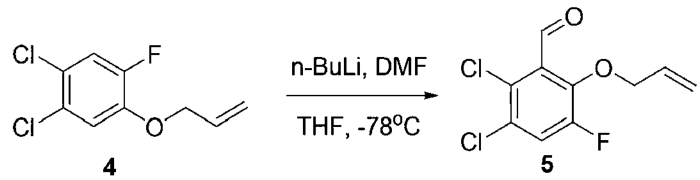


4 5

At -78 °C, 1-(allyloxy)-4,5-dichloro-2-fluorobenzene (550.0 mg, 2.49 mmol) was dissolved in tetrahydrofuran (10.0 mL), and *n*-butyllithium (1.0 mL, 2.61 mmol, 2.5 M in *n*-hexane) was added dropwise over a period of 3 min or longer. After completion of the dropwise addition, the mixture was stirred for 30 min, and then *N*,*N*-dimethylformamide (363.7 mg, 4.98 mmol) was added at the same temperature. The resulting mixture was then stirred at the same temperature for another 2 h. After completion of the reaction, the reaction mixture was quenched with a saturated aqueous ammonium chloride solution (10.0 mL) and extracted twice with ethyl acetate (30.0 mL). The organic phases were combined, washed with saturated brine (15.0 mL), dried over anhydrous sodium sulfate, and then concentrated under reduced pressure. The residue was purified by silica gel column chromatography and eluted with petroleum ether:ethyl acetate (15:1) to give the target product (500.0 mg, yield: 80%).

$^1$H NMR (400 MHz, DMSO-$d_6$) δ 10.25 (s, 1H), 7.81 (d, *J* = 8.1 Hz, 1H), 6.18 - 5.96 (m, 1H), 5.43 (dd, *J* = 17.3, 1.6 Hz, 1H), 5.33 (dd, *J* = 10.5, 1.3 Hz, 1H), 4.76 (d, *J* = 5.3 Hz, 2H).

**Synthesis of ethyl (E)-3-(2-(allyloxy)-5,6-dichloro-3-fluorophenyl)acrylate (6):**

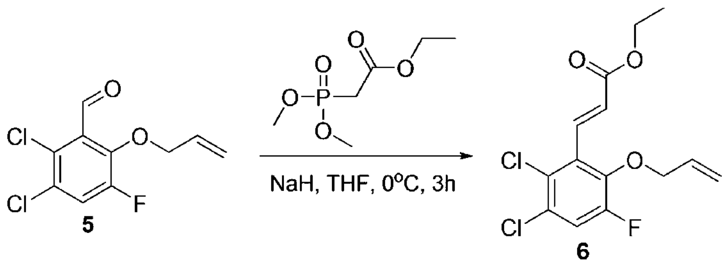


5 6

At 0 °C, sodium hydride (82.9 mg, 2.07 mmol, 60% in mineral oil) was added to a solution of ethyl 2-(dimethoxyphosphoryl)acetate (406.4 mg, 2.07 mmol) in tetrahydrofuran (10.0 mL). The mixture was stirred at 0 °C for 30 min, and then 2-(allyloxy)-5,6-dichloro-3-fluorobenzaldehyde (430.0 mg, 1.73 mmol) was added. The resulting mixture was stirred for another 3 h. After completion of the reaction, the mixture was diluted with water (30.0 mL) and extracted twice with ethyl acetate (30.0 mL). The organic phases were combined, washed with saturated brine (50.0 mL), dried over anhydrous sodium sulfate, and concentrated under reduced pressure. The resulting residue was purified by silica gel column chromatography and eluted (petroleum ether:ethyl acetate = 5:1) to give a yellow solid (550.0 mg, yield: 94%).

$^1$H NMR (400 MHz, DMSO-$d_6$) δ 7.58 (d, *J* = 16.3 Hz, 1H), 7.53 (d, *J* = 8.2 Hz, 1H), 6.53 (dd, *J* = 16.4, 0.7 Hz, 1H), 6.07 - 5.94 (m, 1H), 5.39 (dd, *J* = 17.3, 1.6 Hz, 1H), 5.32 - 5.24 (m, 1H), 4.68 (dd, *J* = 4.1, 1.2 Hz, 2H), 4.19 (q, *J* = 7.1 Hz, 2H), 1.23 (t, *J* = 7.1 Hz, 3H).

**Synthesis of ethyl 3-(2-(allyloxy)-5,6-dichloro-3-fluorophenyl)-4-nitrobutanoate (7):**

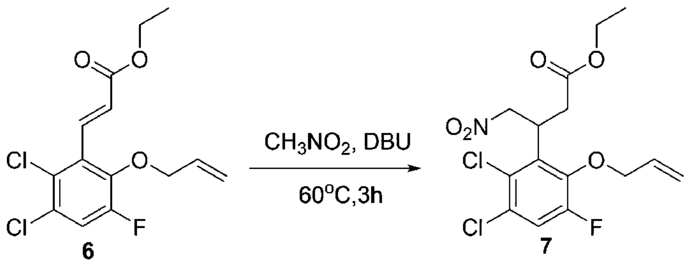


At room temperature, ethyl (*E*)-3-(2-(allyloxy)-5,6-dichloro-3-fluorophenyl)acrylate (550.0 mg, 1.72 mmol) and 1,8-diazabicyclo[5.4.0]undec-7-ene (314.8 mg, 2.07 mmol) were added to a reaction flask containing nitromethane (10.0 mL). The reaction mixture was stirred at 60 °C for 3 h. After completion of the reaction, the mixture was concentrated under reduced pressure, and the resulting crude product was diluted with water (10.0 mL) and extracted with ethyl acetate (20.0 mL). The organic phase was washed with saturated brine (10.0 mL), dried over anhydrous sodium sulfate, and then concentrated under reduced pressure. The residue was purified by silica gel column chromatography (petroleum ether:ethyl acetate = 5:1) to give a pale yellow oily subtance (500.0 mg, yield: 64%).

LC-MS: $[M+Na]^+$: 401.8.

**Synthesis of ethyl 3-(2-(allyloxy)-5,6-dichloro-3-fluorophenyl)-4-aminobutanoate (8):**

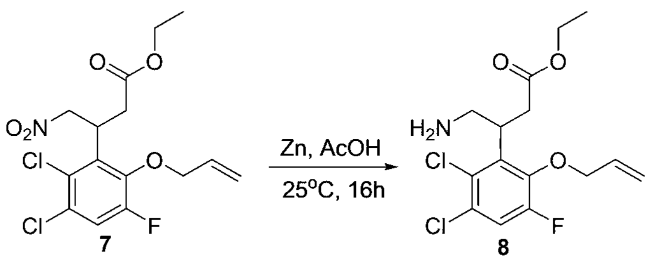


At room temperature, ethyl 3-(2-(allyloxy)-5,6-dichloro-3-fluorophenyl)-4-nitrobutanoate (250.0 mg, 0.66 mmol) and zinc powder (430.1 mg, 6.58 mmol) were added to a reaction flask containing glacial acetic acid (5.0 mL). The mixture was stirred at 25 °C for 16 h. After completion of the reaction, the reaction mixture was filtered, and the filter cake was washed twice with ethyl acetate (10 mL).The filtrate was collected and concentrated under reduced pressure to give a crude product (200 mg), which was directly used in the next step without purification.

LC-MS: $[M+H]^+$: 350.0.

**Synthesis of 4-(2-(allyloxy)-5,6-dichloro-3-fluorophenyl)pyrrolidin-2-one (9):**

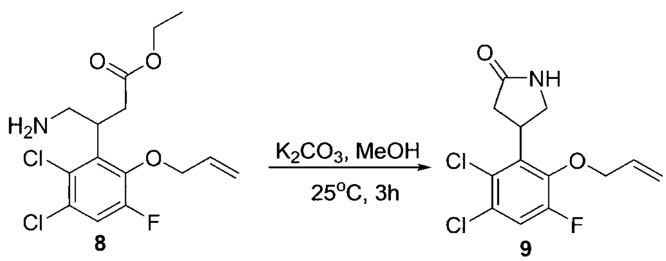


The crude product of ethyl 3-(2-(allyloxy)-5,6-dichloro-3-fluorophenyl)-4-aminobutanoate (400.0 mg, 1.14 mmol) and potassium carbonate (472.9 mg, 3.43 mmol) were added to methanol (10.0 mL), and the mixture was stirred at 25 °C for 3 h. After completion of the reaction, the reaction mixture was diluted with water (10 mL) and extracted three times with ethyl acetate (20 mL). The organic phases were combined, washed with saturated brine (50 mL), and then dried over anhydrous sodium sulfate. After filtration, the filtrate was concentrated under reduced pressure. The residue was purified by silica gel column chromatography and eluted (petroleum ether:ethyl acetate = 5:1) to give a yellow solid (260.0

mg, yield: 63%).

LC-MS: $[M+H]^+$: 304.0.

**Synthesis of 3-(2-(allyloxy)-5,6-dichloro-3-fluorophenyl)-5-methoxy-3,4-dihydro-2*H*-pyrrole (10):**

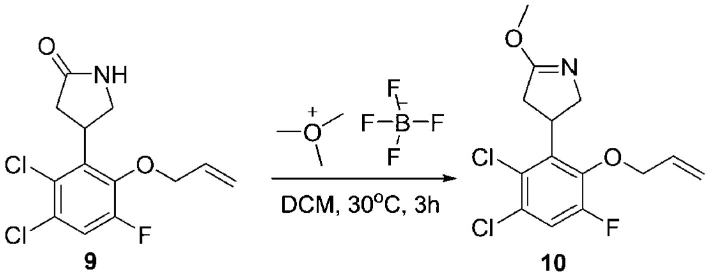


4-(2-(Allyloxy)-5,6-dichloro-3-fluorophenyl)pyrrolidin-2-one (260.0 mg, 0.85 mmol) and trimethyloxonium tetrafluoroborate (139.1 mg, 0.94 mmol) were added to dichloromethane (3.0 mL), and the mixture was stirred at 30 °C for 3 h under a nitrogen atmosphere. After completion of the reaction, the reaction mixture was directly concentrated under reduced pressure to give a crude product, which was directly used in the next step without purification.

LC-MS: $[M+H]^+$: 318.0.

**Synthesis of *N'*-(3-(2-(allyloxy)-5,6-dichloro-3-fluorophenyl)-3,4-dihydro-2*H*-pyrrol-5-yl)propionohydrazide** (11):

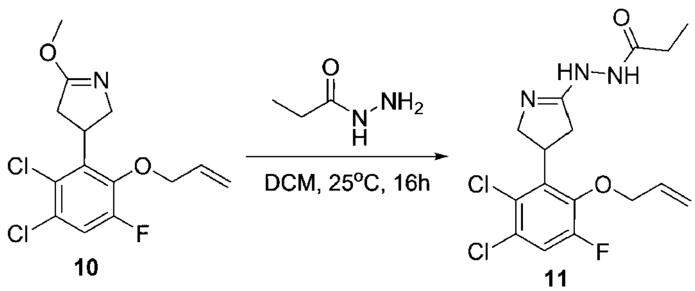


3-(2-(Allyloxy)-5,6-dichloro-3-fluorophenyl)-5-methoxy-3,4-dihydro-2*H*-pyrrole (260.0 mg, 0.82 mmol) and propionohydrazide (216.0 mg, 2.45 mmol) were added to dichloromethane (3.0 mL), and the mixture was stirred at 25 °C for 16 h. After completion of the reaction, the reaction mixture was directly concentrated under reduced pressure. The residue was purified by silica gel column chromatography and eluted (dichloromethane:methanol = 20:1) to give a white solid (140.0 mg, yield: 38%).

LC-MS: $[M+H]^+$: 374.0.

**Synthesis of 6-(2-(allyloxy)-5,6-dichloro-3-fluorophenyl)-3-ethyl-6,7-dihydro-5*H*-pyrrolo[2,1-c][1,2,4]triazole** (12):

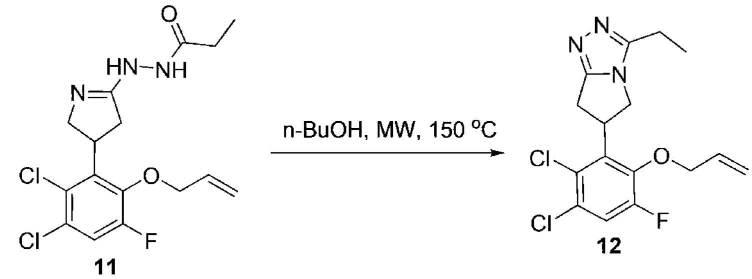


11 12

The compound *N'*-(3-(2-(allyloxy)-5,6-dichloro-3-fluorophenyl)-3,4-dihydro-2*H*-pyrrol-5-yl)propionohydrazide (120.0 mg) was dissolved in n-butanol (3.0 mL), and the mixture was directly added to a microwave tube and allowed to react at 150 °C for 30 min. After completion of the reaction, the reaction mixture was cooled to room temperature. The mixture was rotary evaporated to dryness, and then the resulting crude product was purified by silica gel chromatography (dichloromethane:methanol = 10:1) to give a white solid (80.0 mg, yield: 70%).

LC-MS: $[M+H]^+$: 356.1.

**Synthesis of 3,4-dichloro-2-(3-ethyl-6,7-dihydro-5*H*-pyrrolo[2,1-*c*][1,2,4]triazol-6-yl)-6-fluorophenol (13):**

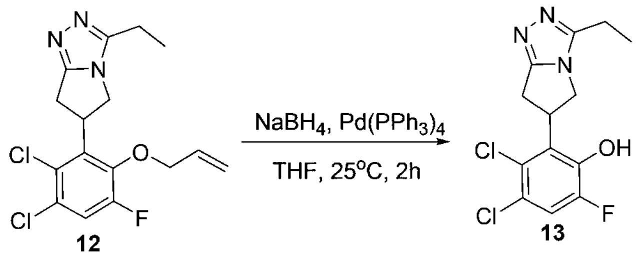


12 13

At room temperature, sodium borohydride (12.7 mg, 0.34 mmol) and tetrakis(triphenylphosphine)palladium(0) (25.9 mg, 0.02 mmol) were added to a solution of 6-(2-(allyloxy)-5,6-dichloro-3-fluorophenyl)-3-ethyl-6,7-dihydro-5H-pyrrolo[2,1-c][1,2,4]triazole (80.0 mg, 0.22 mmol) in tetrahydrofuran (2.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was subjected to preparative chromatography to give a white solid (5.1 mg, yield: 7%).

LC-MS: $[M+H]^+$: 316.1.

$^1$H NMR (400 MHz, DMSO-$d_6$) δ 10.82 (s, 1H), 7.21 (d, *J* = 8.5 Hz, 1H), 4.91 (dd, *J* = 12.2, 4.7 Hz, 1H), 4.37 (t, *J* = 10.1 Hz, 1H), 4.00 (dd, *J* = 10.8, 7.0 Hz, 1H), 3.30 (d, *J* = 11.2 Hz, 1H), 2.95 (dd, *J* = 16.5, 7.4 Hz, 1H), 2.69 (q, *J* = 7.5 Hz, 2H), 1.21 (t, *J* = 7.6 Hz, 3H).

**Example 32. (*S*)-3,4-Dichloro-2-(3-(2-hydroxyethyl)-6,7-dihydro-5*H*-pyrrolo[2,1-*c*][1,2,4]triazol-6-yl)phenol**

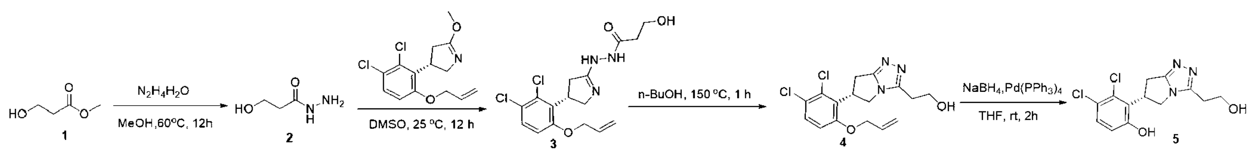


1 2 3 4 5

**Synthesis of 3-hydroxypropanehydrazide (2):**

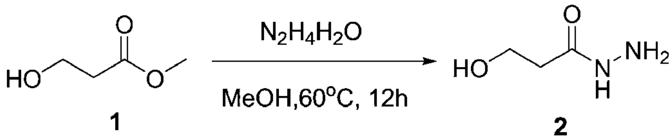

1 2

A solution of methyl 3-hydroxypropanoate (5.0 g, 0.048 mol) and hydrazine hydrate (6.0 g, 0.14 mol, 80% in water) in methanol (50.0 mL) was stirred at 60 °C for 12 h. After completion of the reaction, the resulting mixture was diluted by adding dichloromethane (20.0 mL), resulting in the precipitation of a product. The mixture was filtered to give a white solid (2.5 g, yield: 50%).
LC-MS: $[M+H]^+$: 105.2.

**Synthesis of (*S*)-*N'*-(3-(6-(allyloxy)-2,3-dichlorophenyl)-3,4-dihydro-2*H*-pyrrol-5-yl)-3-hydroxypropanehydrazide (3):**

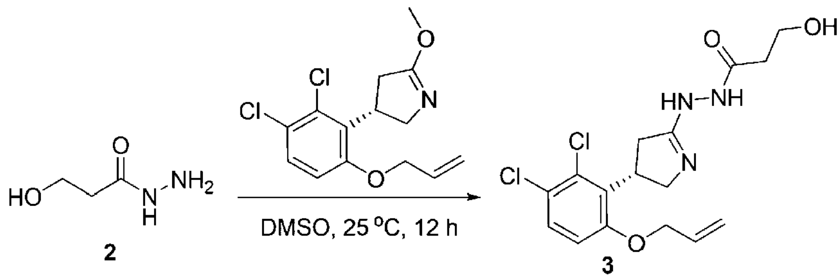

2 3

A solution of 3-hydroxypropanehydrazide (208.0 mg, 1.99 mmol) and (*S*)-3-(6-(allyloxy)-2,3-dichlorophenyl)-5-methoxy-3,4-dihydro-2*H*-pyrrole (200.0 mg, 0.66 mmol) in dimethylsulfoxide (2.0 mL) was stirred at 25 °C for 12 h. After completion of the reaction, the dimethylsulfoxide was removed by lyophilization under reduced pressure. The resulting crude product was purified by silica gel chromatography (methanol:dichloromethane = 1:10) to give a white solid (150.0 mg, yield: 60%).
LC-MS: $[M+H]^+$: 372.1.

**Synthesis of (*S*)-2-(6-(6-(allyloxy)-2,3-dichlorophenyl)-6,7-dihydro-5*H*-pyrrolo[2,1-*c*][1,2,4]triazol-3-yl)ethan-1-ol (4):**

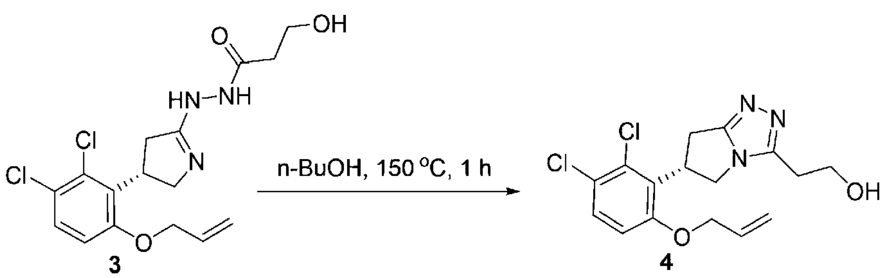

3 4

(*S*)-*N'*-(3-(6-(Allyloxy)-2,3-dichlorophenyl)-3,4-dihydro-2*H*-pyrrol-5-yl)-3-hydroxypropanehydrazide(150.0 mg, 0.41 mmol) was transferred to a microwave tube, and an n-butanol solution (3.0 mL) was added. The mixture was stirred under microwave at 150 °C for 1 h. After completion of the reaction, the n-butanol was removed by distillation under reduced pressure. The resulting crude product was purified by silica gel chromatography (methanol:dichloromethane = 1:10) to give a white solid (80.0 mg, yield: 56%).
LC-MS: $[M+H]^+$: 354.2.

**Synthesis of (*S*)-3,4-dichloro-2-(3-(2-hydroxyethyl)-6,7-dihydro-5*H*-pyrrolo[2,1-c][1,2,4]triazol-6-yl)phenol (5):**

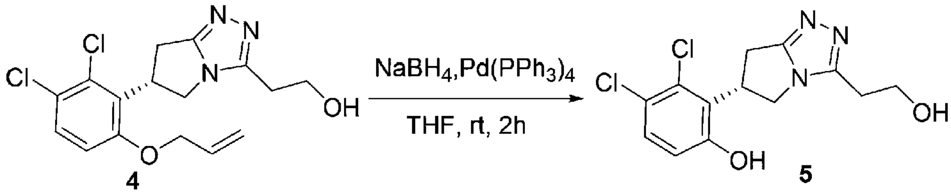


4

5

At room temperature, tetrakis(triphenylphosphine)palladium(0) (130.4 mg, 0.11 mmol) and sodium borohydride (13.8 mg, 0.36 mmol) were added to a solution of (S)-2-(6-(6-(allyloxy)-2,3-dichlorophenyl)-6,7-dihydro-5*H*-pyrrolo[2,1-*c*][1,2,4]triazol-3-yl)ethan-1-ol (80.0 mg, 0.22 mmol) in tetrahydrofuran (1.0 mL). The mixture was stirred at room temperature for 2 h. After completion of the reaction, the reaction mixture was filtered through an organic-phase filter membrane, and the filtrate was rotary evaporated to dryness. The crude product was subjected to preparative chromatography to give a white solid (10.5 mg, yield: 14%).

LC-MS: $[M+H]^+$: 314.1.

$^1H$ NMR (400 MHz, DMSO-$d_6$) δ 10.58 (s, 1H), 7.40 (d, *J*=8.8, 1H), 6.87 (d, *J*=8.8, 1H), 4.98 - 4.88 (m, 1H), 4.83 (t, *J*=5.3, 1H), 4.23 (t, *J*=9.9, 1H), 4.08 (dd, *J*=10.2, 8.0, 1H), 3.68 (dd, *J*=11.9, 6.4, 2H), 3.10 (d, *J*=9.0, 2H), 2.81 (t, *J*=6.6, 2H).

**Example 33. (S)-3,4-Dichloro-2-(3-(tetrahydro-2*H*-pyran-4-yl)-6,7-dihydro-5*H*-pyrrolo[2,1-*c*][1,2,4]triazol-6-yl)phenol**

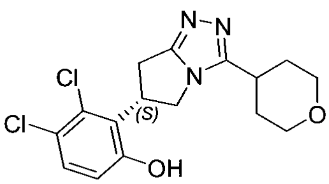

This example was prepared by reference to the preparation method for Example 32, except that the 3-hydroxypropanehydrazide was replaced by tetrahydro-2*H*-pyran-4-carbohydrazide.

LCMS $[M+H]^+$: 354.0.

$^1H$ NMR (400 MHz, DMSO-*d6*) δ 7.39 (d, *J* = 8.8 Hz, 1H), 6.87 (d, *J* = 8.8 Hz, 1H), 5.02 - 4.90 (m, 1H), 4.28 (t, *J* = 9.8 Hz, 1H), 4.06 (dd, *J* = 10.1, 7.5 Hz, 1H), 3.91 (dd, *J* = 8.3, 3.6 Hz, 2H), 3.45 - 3.39 (m, 2H), 3.12 (dd, *J* = 15.8, 10.0 Hz, 1H), 3.06 - 2.98 (m, 2H), 1.87 - 1.67 (m, 4H).

**Example 34. (*S*)-3,4-Dichloro-2-(3-ethyl-6,7-dihydro-5*H*-pyrrolo[2,1-*c*][1,2,4]triazol-6-yl)phenol**

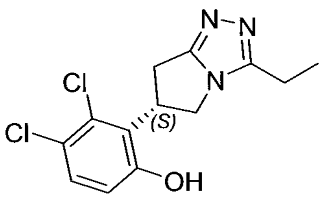

This example was prepared by reference to the preparation method for Example 32, except that the 3-hydroxypropanehydrazide was replaced by propionohydrazide.

LCMS $[M+H]^+$: 298.0.

$^1H$ NMR (400 MHz, DMSO-*d6*) δ 10.77 (s, 1H), 7.39 (d, *J* = 8.8 Hz, 1H), 6.87 (d, *J* = 8.8 Hz, 1H), 4.97 (dd, *J* = 17.5, 8.5 Hz, 1H), 4.21 (t, *J* = 9.8 Hz, 1H), 4.03 (dd, *J* = 10.1, 7.8 Hz, 1H), 3.14 - 3.03 (m, 2H), 2.67 (q, *J* = 7.6 Hz, 2H), 1.20 (t, *J* = 7.6 Hz, 3H).

**Example 35. (S)-3,4-Dichloro-2-(3-(piperidin-4-yl)-6,7-dihydro-5*H*-pyrrolo[2,1-*c*][1,2,4]triazol-6-yl)phenol**

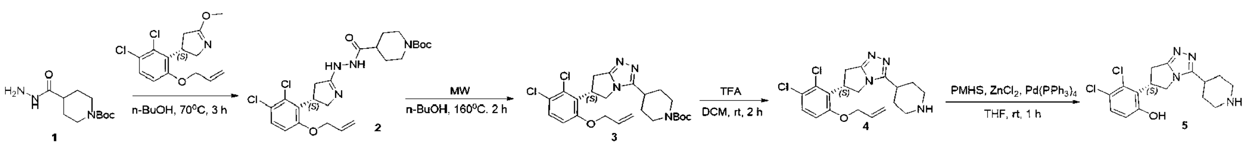


1 2 3 4 5

**Synthesis of *tert*-butyl (*S*)-4-(2-(3-(6-(allyloxy)-2,3-dichlorophenyl)-3,4-dihydro-2*H*-pyrrol-5-yl)hydrazine-1-carbonyl)piperidine-1-carboxylate (2):**

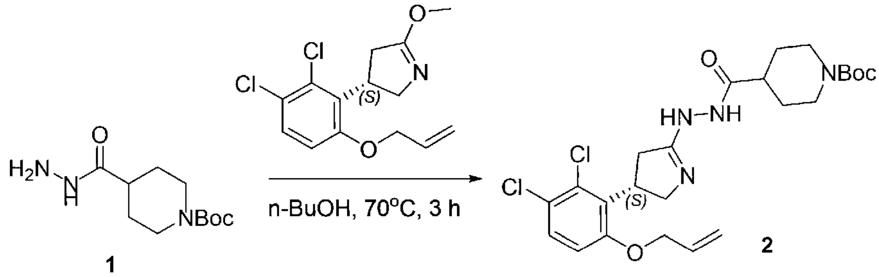


1 2

A solution of (*S*)-3-(6-(allyloxy)-2,3-dichlorophenyl)-5-methoxy-3,4-dihydro-2*H*-pyrrole (125.0 mg, 0.42 mmol) and *tert*-butyl 4-(hydrazinecarbonyl)piperidine-1-carboxylate (305.2 mg, 1.25 mmol) in n-butanol (2.0 mL) was stirred at 70 °C for 3 h. After completion of the reaction, a solution of the target product in n-butanol (about 200 mg, yield: 94%) was obtained and directly used in the next step.

LC-MS: $[M+H]^+$: 511.1.

**Synthesis of *tert*-butyl (*S*)-4-(6-(6-(allyloxy)-2,3-dichlorophenyl)-6,7-dihydro-5*H*-pyrrolo[2,1-*c*][1,2,4]triazol-3-yl) piperidine-1-carboxylate (3):**

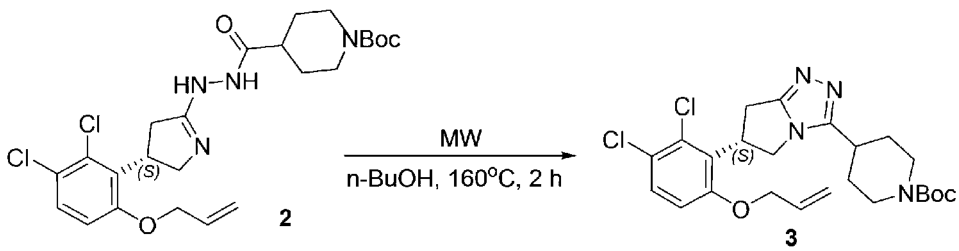


2 3

A solution of *tert*-butyl (*S*)-4-(2-(3-(6-(allyloxy)-2,3-dichlorophenyl)-3,4-dihydro-2H-pyrrol-5-yl)hydrazine-1-carbonyl)piperidine-1-carboxylate in *n*-butanol (200.0 mg, 0.39 mmol, 2.0 mL of *n*-butanol solution) was transferred to a microwave tube and then stirred under microwave at 160 °C for 2 h. After completion of the reaction, the n-butanol was removed by distillation under reduced pressure. The resulting crude product was purified by silica gel chromatography (ethyl acetate:petroleum ether = 1:1) to give a white solid (140 mg, yield: 73%).

LC-MS: $[M\text{-}Boc +H]^+$: 393.1.

**Synthesis of (S)-6-(6-(allyloxy)-2,3-dichlorophenyl)-3-(piperidin-4-yl)-6,7-dihydro-5*H*-pyrrolo[2,1-*c*][1,2,4]triazole (4):**

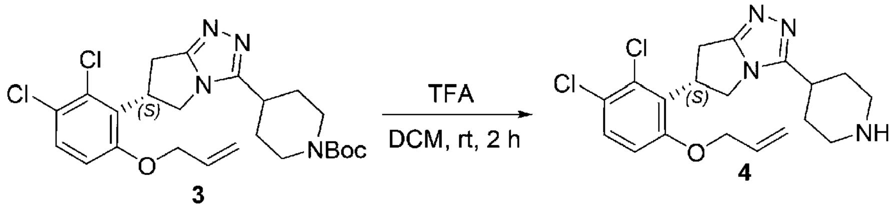


A solution of *tert*-butyl (*S*)-4-(6-(6-(allyloxy)-2,3-dichlorophenyl)-6,7-dihydro-5*H*-pyrrolo[2,1-*c*][1,2,4]triazol-3-yl)piperidine-1-carboxylate (120.0 mg, 0.24 mmol) in trifluoroacetic acid (1.0 mL) and dichloromethane (2.0 mL) was stirred at room temperature for 2 h. After completion of the reaction, the solvent was removed by distillation under reduced pressure. The resulting crude product was purified by silica gel chromatography (methanol:dichloromethane = 1:10) to give a white solid (30.0 mg, yield: 31%).

LC-MS: $[M+H]^+$: 393.1.

**Synthesis of (*S*)-3,4-dichloro-2-(3-(piperidin-4-yl)-6,7-dihydro-5*H*-pyrrolo[2,1-*c*][1,2,4]triazol-6-yl)phenol (5):**

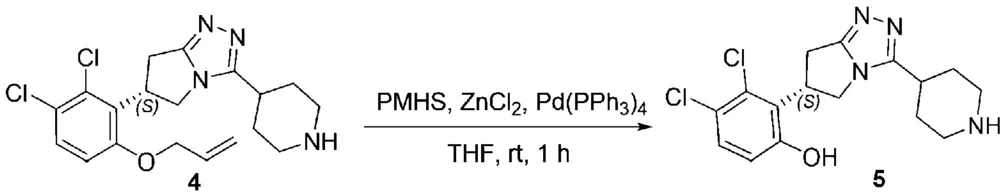


At room temperature, tetrakis(triphenylphosphine)palladium(0) (2.8 mg, 0.002 mmol) was added to a solution of (*S*)-6-(6-(allyloxy)-2,3-dichlorophenyl)-3-(piperidin-4-yl)-6,7-dihydro-5*H*-pyrrolo[2,1-*c*][1,2,4]triazole (24.0 mg, 0.06 mmol), polymethylhydrosiloxane (27.1 mg, 0.12 mmol), and zinc chloride (0.8 mg, 0.005 mmol) in tetrahydrofuran (2.0 mL). The flask was purged using an argon balloon, and the mixture was stirred at room temperature for 1 h. After completion of the reaction, the reaction mixture was filtered through an organic-phase filter membrane, and the filtrate was rotary evaporated to dryness. The crude product was purified by preparative chromatography to give a white solid (2.1 mg, yield: 10%).

LC-MS: $[M+H]^+$: 353.1.

$^1H$ NMR (400 MHz, DMSO-$d_6$) δ 8.34 (s, 1H), 7.37 (d, *J* = 8.8 Hz, 1H), 6.89 (d, *J* = 8.8 Hz, 1H), 5.00 - 4.92 (m, 1H), 4.26 (t, *J* = 9.8 Hz, 1H), 4.07 (dd, *J* = 9.9, 7.7 Hz, 1H), 3.19 (d, *J* = 9.4 Hz, 2H), 3.12 - 3.04 (m, 2H), 3.01 (d, *J* = 4.4 Hz, 1H), 2.81 (t, *J* = 11.1 Hz, 2H), 1.95 (t, *J* = 12.1 Hz, 2H), 1.82 - 1.71 (m, 2H).

**Example 36. (*S*)-3,4-Dichloro-2-(3-(1-isopropylpiperidin-4-yl)-6,7-dihydro-5*H*-pyrrolo[2,1-*c*][1,2,4]triazol-6-yl) phenol**

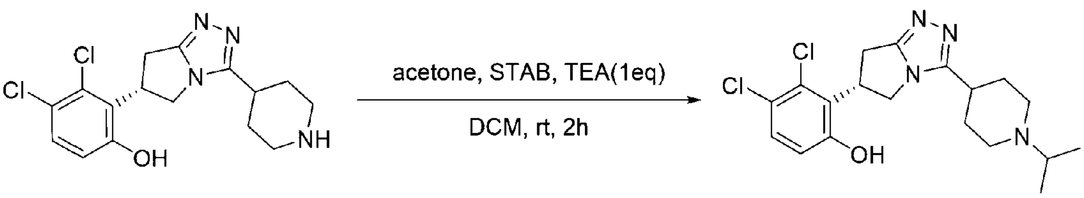


At room temperature, triethylamine (6.0 mg, 0.06 mmol) was added to a mixed solution of (*S*)-3,4-dichloro-2-(3-(piperidin-4-yl)-6,7-dihydro-5*H*-pyrrolo[2,1-*c*][1,2,4]triazol-6-yl)phenol (21.2 mg, 0.06 mmol) in methanol and dichloromethane (volume ratio: 1:1, 3.0 mL). The mixture was stirred for 5 min, and then acetone (7.0 mg, 0.12 mmol) was added. The resulting mixture was stirred at room temperature for 1 h and then cooled to 0 °C. Sodium triacetoxyborohydride (39.0 mg, 0.18 mmol) was added, and the resulting mixture was allowed to react at room temperature for 2 h. After completion of the reaction, as monitored by liquid chromatography-mass spectrometry, the reaction mixture was extracted with ethyl acetate (10 mL). The organic phase was washed with saturated brine (10 mL) and extracted three times. The resulting organic phase was dried over anhydrous sodium sulfate and concentrated under reduced pressure.

The resulting residue was purified by preparative chromatography to give a white solid (6.9 mg, yield: 29%).

LC-MS: $[M+H]^+$: 395.1.

$^1H$ NMR (400 MHz, DMSO-$d_6$) δ 8.25 (s, 1H), 7.38 (d, *J* = 8.8 Hz, 1H), 6.88 (d, *J* = 8.8 Hz, 1H), 4.97 (t, *J* = 8.6 Hz, 1H), 4.27 (t, *J* = 9.8 Hz, 1H), 4.07 (dd, *J* = 10.0, 7.6 Hz, 1H), 3.13 - 2.96 (m, 5H), 2.89 - 2.81 (m, 1H), 2.54 (d, *J* = 3.7 Hz, 1H), 2.48 (s, 1H), 1.98 (t, *J* = 12.5 Hz, 2H), 1.85 - 1.74 (m, 2H), 1.07 (d, *J* = 6.6 Hz, 6H).

**Example 37. (*S*)-1-(4-(6-(2,3-Dichloro-6-hydroxyphenyl)-6,7-dihydro-5H-pyrrolo[2,1-c][1,2,4]triazol-3-yl)piperidin-1-yl)ethan-1-one**

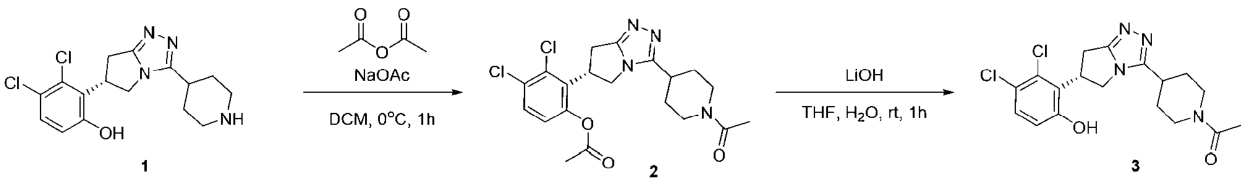


**Synthesis of (*S*)-2-(3-(1-acetylpiperidin-4-yl)-6,7-dihydro-5*H*-pyrrolo[2,1-*c*][1,2,4]triazol-6-yl)-3,4-dichlorophenyl acetate (2):**

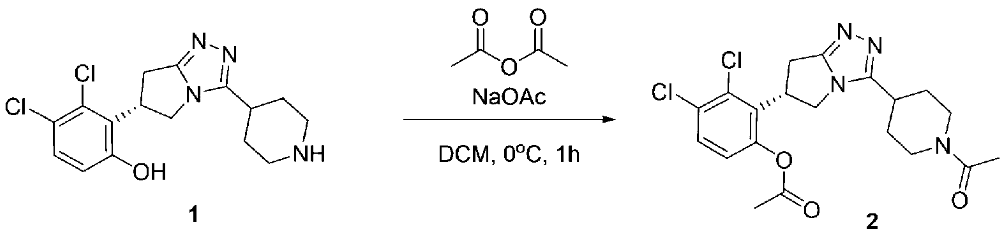


At room temperature, acetic anhydride (12.0 mg, 0.12 mmol) was added to a solution of (S)-3,4-dichloro-2-(3-(piperidin-4-yl)-6,7-dihydro-5H-pyrrolo[2,1-c][1,2,4]triazol-6-yl)phenol (21.2 mg, 0.06 mmol) in dichloromethane (3.0 mL). The mixture was stirred at room temperature for 10 min and then cooled to 0 °C. Sodium acetate (15 mg, 0.18 mmol) was added, and the resulting mixture was allowed to react at 0 °C for 1 h. After completion of the reaction, as monitored by liquid chromatography-mass spectrometry, the mixture was directly rotary evaporated to dryness to give a white solid, which was directly used in the next step.

LC-MS: $[M+H]^+$: 436.1.

Synthesis of (S)-1-(4-(6-(2,3-dichloro-6-hydroxyphenyl)-6,7-dihydro-5H-pyrrolo[2,1-c][1,2,4]triazol-3-yl)piperidin-1-yl) ethan-1-one (3):

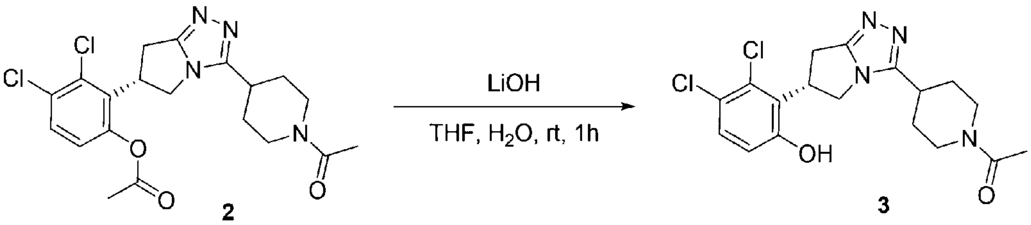


At room temperature, lithium hydroxide (6.0 mg, 0.24 mmol) was added to a mixed solution of the crude product of (S)-2-(3-(1-acetylpiperidin-4-yl)-6,7-dihydro-5H-pyrrolo[2,1-c][1,2,4]triazol-6-yl)-3,4- dichlorophenyl acetate (25.0 mg, 0.06 mmol) in tetrahydrofuran and water (5:1, 3.0 mL). The mixture was stirred at room temperature for 1 h. After completion of the reaction, as monitored by liquid chromatography-mass spectrometry, the mixture was directly rotary evaporated to dryness, and then the residue was purified by preparative chromatography to give a white solid (1.7 mg,

yield: 7%).

LC-MS: $[M+H]^+$: 395.0.

$^1H$ NMR (400 MHz, DMSO-$d_6$) δ 10.49 (s, 1H), 7.39 (d, *J* = 8.8 Hz, 1H), 6.87 (d, *J* = 8.8 Hz, 1H), 5.01 - 4.92 (m, 1H), 4.30 (dd, *J* = 20.4, 10.9 Hz, 2H), 4.07 (s, 1H), 3.85 (d, *J* = 10.3 Hz, 1H), 3.08 (ddd, *J* = 23.3, 21.4, 9.9 Hz, 4H), 2.72 (t, *J* = 11.4 Hz, 1H), 2.01 (s, 3H), 1.95 - 1.83 (m, 2H), 1.72 - 1.62 (m, 1H), 1.54 - 1.45 (m, 1H).

**Example 38. (*S*)-5-(6-(2,3-Dichloro-6-hydroxyphenyl)-6,7-dihydro-5*H*-pyrrolo[2,1-*c*][1,2,4]triazol-3-yl)-1-methylpyridin-2(1*H*)-one**

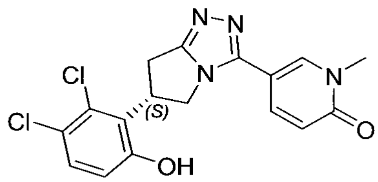

This example was prepared by reference to the preparation method for Example 32, except that the 3-hydroxypropanehydrazide was replaced by 1-methyl-6-oxo-1,6-dihydropyridine-3-carbohydrazide.

LCMS $[M+H]^+$: 377.0.

$^1H$ NMR (400 MHz, DMSO-d6) δ 8.19 (s, 1H), 8.01 (d, *J* = 9.3 Hz, 1H), 7.33 (d, *J* = 8.8 Hz, 1H), 6.82 (d, *J* = 8.8 Hz, 1H), 6.67 (d, *J* = 9.5 Hz, 1H), 5.26 (dd, *J* = 16.2, 8.0 Hz, 1H), 4.62 (t, *J* = 9.6 Hz, 1H), 4.53 - 4.47 (m, 1H), 3.64 (s, 3H), 3.36 (d, *J* = 8.9 Hz, 2H).

**Example 39. (*S*)-4-(6-(2,3-Dichloro-6-hydroxyphenyl)-6,7-dihydro-5*H*-pyrrolo[2,1-*c*][1,2,4]triazol-3-yl)-1-methylpyridin-2(1*H*)-one**

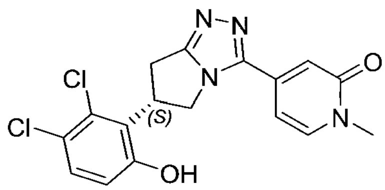

This example was prepared by reference to the preparation method for Example 32, except that the methyl 3-hydroxypropanoate was replaced by methyl 1-methyl-2-oxo-1,2-dihydropyridine-4-carboxylate.

LCMS $[M+H]^+$: 377.0.

$^1H$ NMR (400 MHz, DMSO-d6) δ 10.79 (s, 0H), 7.81 (d, *J* = 7.1 Hz, 1H), 7.38 (d, *J* = 8.8 Hz, 1H), 6.83 (d, *J* = 8.7 Hz, 1H), 6.75 (dd, *J* = 7.1, 2.0 Hz, 1H), 6.71 (d, *J* = 1.8 Hz, 1H), 5.10 - 5.00 (m, 1H), 4.63 (t, *J* = 10.0 Hz, 1H), 4.33 (dd, *J* = 10.3, 7.0 Hz, 1H), 3.45 (s, 3H), 3.25 (d, *J* = 10.2 Hz, 1H), 3.11 (dd, *J* = 16.2, 7.3 Hz, 1H).

**Example 40. (*S*)-3,4-Dichloro-2-(3-(1-methyl-1*H*-pyrazol-4-yl)-6,7-dihydro-5H-pyrrolo[2,1-*c*][1,2,4]triazol-6-yl) phenol**

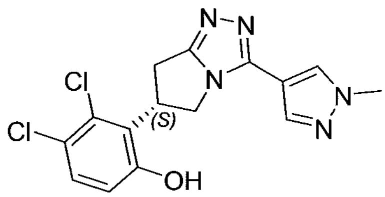

This example was prepared by reference to the preparation method for Example 32, except that the 3-hydroxypropanehydrazide was replaced by 1-methylpyrazole-4-carbohydrazide.

LCMS $[M+H]^+$: 350.0.

$^1$H NMR (400 MHz, DMSO-d6) δ 10.94 (s, 1H), 8.26 (s, 1H), 7.87 (s, 1H), 7.38 (d, *J*=8.8, 1H), 6.85 (d, *J*=8.8, 1H), 5.10 - 5.01 (m, 1H), 4.43 (t, *J*=9.9, 1H), 4.19 (dd, *J*=10.1, 7.4, 1H), 3.88 (s, 3H), 3.14 (dt, *J*=15.8, 6.9, 2H).

**Example 41. (*S*)-2-(3-(1*H*-Indazol-4-yl)-6,7-dihydro-5*H*-pyrrolo[2,1-*c*][1,2,4]triazol-6-yl)-3,4-dichlorophenol**

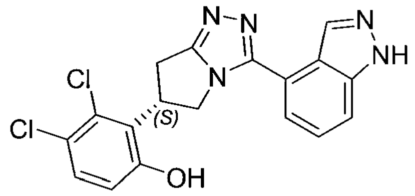

This example was prepared by reference to the preparation method for Example 32, except that the methyl 3-hydroxypropanoate was replaced by methyl 1*H*-indazole-4-carboxylate.

LCMS $[M+H]^+$: 386.0.

$^1$H NMR (400 MHz, DMSO-*d6)* δ 13.33 (s, 1H), 10.76 (s, 1H), 8.63 (s, 1H), 7.64 (d, *J* = 8.1 Hz, 1H), 7.48 (d, *J* = 6.9 Hz, 1H), 7.41 (dd, *J* = 15.9, 8.4 Hz, 2H), 6.85 (d, *J* = 8.8 Hz, 1H), 5.12 (t, *J* = 8.7 Hz, 1H), 4.68 (t, *J* = 9.9 Hz, 1H), 4.41 (dd, *J* = 10.2, 7.0 Hz, 1H), 3.28 (d, *J* = 10.2 Hz, 1H), 3.18 (d, *J* = 7.4 Hz, 1H).

**Example 42. (*S*)-2-(3-(6-Aminopyridin-3-yl)-6,7-dihydro-5*H*-pyrrolo[2,1-*c*][1,2,4]triazol-6-yl)-3,4-dichlorophenol**

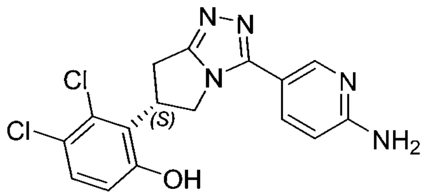

This example was prepared by reference to the preparation method for Example 32, except that the 3-hydroxypropanehydrazide was replaced by 6-aminonicotinohydrazide.

LCMS $[M+H]^+$: 362.0.

$^1$H NMR (400 MHz, DMSO-d6) δ 10.70 (s, 1H), 8.41 (s, 1H), 8.22 (s, 1H), 7.94 (s, 2H), 7.43 (d, *J* = 8.8 Hz, 1H), 6.90 (t, *J* = 9.5 Hz, 2H), 5.12 - 5.03 (m, 1H), 4.66 (t, *J* = 10.0 Hz, 1H), 4.44 - 4.32 (m, 1H), 3.35 (dd, *J* = 16.1, 10.0 Hz, 1H), 3.23 (dd, *J* = 16.3, 7.4 Hz, 1H).

**Example 43. (*S*)-2-(3-(2-Aminopyridin-4-yl)-6,7-dihydro-5*H*-pyrrolo[2,1-*c*][1,2,4]triazol-6-yl)-3,4-dichlorophenol**

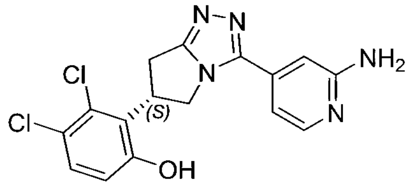

This example was prepared by reference to the preparation method for Example 32, except that the 3-hydroxypropanehydrazide was replaced by 2-aminoisonicotinohydrazide.

LCMS $[M+H]^+$: 362.0.

$^1$H NMR (400 MHz, DMSO-d6) δ 10.62 (s, 1H), 8.13 (s, 1H), 7.99 (d, *J* = 5.3 Hz, 1H), 7.41 (d, *J* = 8.8 Hz, 1H), 6.95 (dd, *J* = 7.6, 2.2 Hz, 2H), 6.87 (d, *J* = 8.8 Hz, 1H), 6.19 (s, 2H), 5.13 - 5.04 (m, 1H), 4.59 (t, *J* = 10.0 Hz, 1H), 4.30 (dd, *J* = 10.2, 7.0 Hz, 1H), 3.26 (dd, *J* = 16.0, 10.2 Hz, 1H), 3.12 (dd, *J* = 16.0, 7.4 Hz, 1H).

**Example 44. (*S*)-3,4-Dichloro-2-(3-(1-(2-hydroxyethyl)-1*H*-pyrazol-4-yl)-6,7-dihydro-5*H*-pyrrolo[2,1-*c*][1,2,4]triazol-6-yl)phenol**

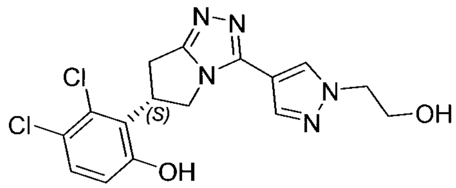

This example was prepared by reference to the preparation method for Example 32, except that the 3-hydroxypropanehydrazide was replaced by 1-(2-hydroxyethyl)-1*H*-pyrazole-4-carbohydrazide.

LCMS $[M+H]^+$: 380.1.

$^1$H NMR (400 MHz, DMSO-*d6*) δ 10.59 (s, 1H), 8.24 (s, 1H), 7.89 (d, *J*=0.4, 1H), 7.41 (d, *J*=8.8, 1H), 6.87 (d, *J*=8.8, 1H), 5.06 (s, 1H), 4.93 (t, *J*=5.3, 1H), 4.45 (t, *J*=10.0, 1H), 4.21 - 4.14 (m, 3H), 3.76 (q, *J*=5.5, 2H), 3.24 - 3.16 (m, 1H), 3.10 (d, *J*=7.6, 1H).

**Example 45. (*S*)-2-(3-(Azetidin-3-yl)-6,7-dihydro-5*H*-pyrrolo[2,1-*c*][1,2,4]triazol-6-yl)-3,4- dichlorophenol**

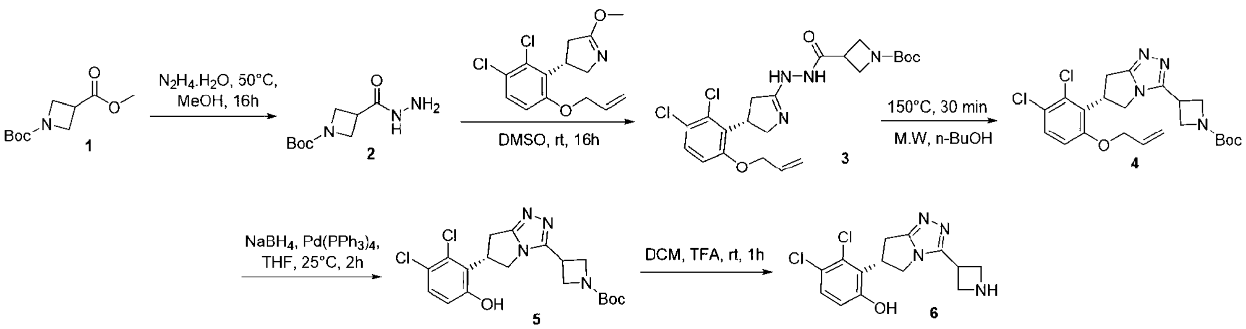


**Synthesis of *tert*-butyl 3-(hydrazinecarbonyl)azetidine-1-carboxylate (2):**

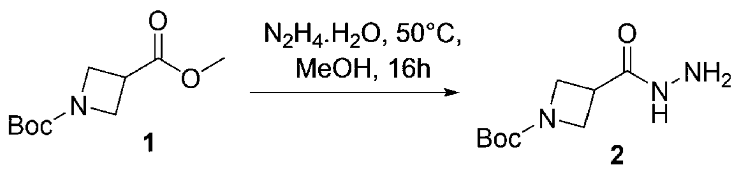


A solution of 1-(*tert*-butyl) 3-methyl azetidine-1,3-dicarboxylate (3.0 g, 0.014 mol) and hydrazine hydrate (2.5 g, 0.062 mol, 80% in water) in methanol (30.0 mL) was stirred at 50 °C for 16 h. The methanol was then removed by distillation under reduced pressure. The resulting crude product was purified by silica gel chromatography (methanol:dichloromethane = 1:10) to give a white solid (2 g, yield: 67%).

**Synthesis of *tert*-butyl (*S*)-3-(2-(3-(6-(allyloxy)-2,3-dichlorophenyl)-3,4-dihydro-2*H*-pyrrol-5-yl)hydrazine-1-carbonyl)azetidine-1-carboxylate (3):**

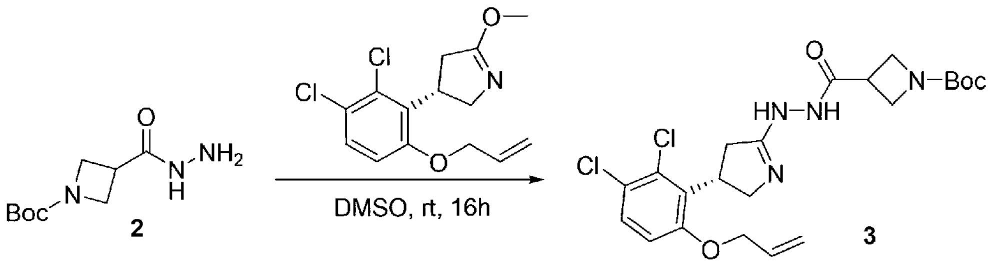


A solution of *tert*-butyl 3-(hydrazinecarbonyl)azetidine-1-carboxylate (648.3 mg, 2.99 mmol) and (*S*)-3-(6-(allyloxy)-2,3-dichlorophenyl)-5-methoxy-3,4-dihydro-2*H*-pyrrole (300.0 mg, 0.99 mmol) in dimethylsulfoxide (10.0 mL) was stirred at room temperature for 16 h. After completion of the reaction, the dimethylsulfoxide was removed by lyophilization under reduced pressure. The resulting crude product was purified by silica gel chromatography (methanol:dichloromethane = 1:10) to give a white solid (250.0 mg, yield: 51%).

LC-MS: $[M+H]^+$: 483.1.

**Synthesis of *tert*-butyl (*S*)-3-(6-(6-(allyloxy)-2,3-dichlorophenyl)-6,7-dihydro-5*H*-pyrrolo[2,1-*c*][1,2,4]triazol-3-yl) azetidine-1-carboxylate (4):**

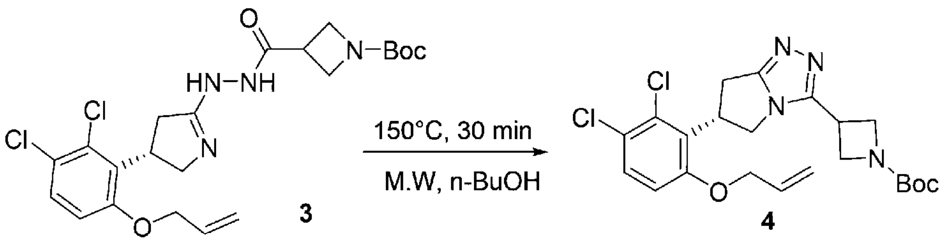


*tert*-Butyl (*S*)-3-(2-(3-(6-(allyloxy)-2,3-dichlorophenyl)-3,4-dihydro-2*H*-pyrrol-5-yl)hydrazine-1-carbonyl)azetidine-1-carboxylate (250.0 mg, 0.51 mmol) was transferred to a microwave tube, and an n-butanol solution (3.0 mL) was added. The mixture was stirred under microwave at 150 °C for 30 min. After completion of the reaction, the n-butanol was removed by distillation under reduced pressure. The resulting crude product was purified by silica gel chromatography (methanol:dichloromethane = 1:10) to give a white solid (100.0 mg, yield: 41%).

LC-MS: $[M+H]^+$: 365.2.

**Synthesis of *tert*-butyl (*S*)-3-(6-(2,3-dichloro-6-hydroxyphenyl)-6,7-dihydro-5*H*-pyrrolo[2,1-*c*][1,2,4]triazol-3-yl) azetidine-1-carboxylate (5):**

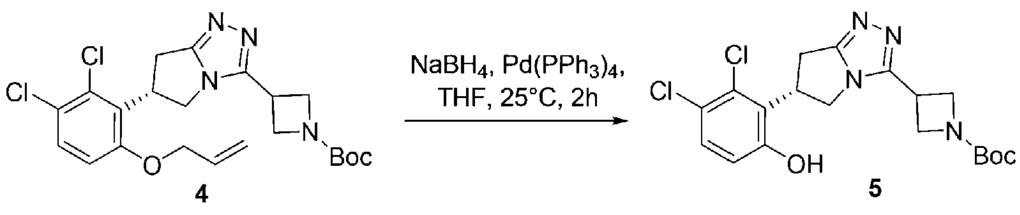


At room temperature, tetrakis(triphenylphosphine)palladium(0) (123.8 mg, 0.10 mmol) and sodium borohydride (12.1 mg, 0.32 mmol) were added to a solution of *tert*-butyl (*S*)-3-(6-(6-(allyloxy)-2,3-dichlorophenyl)-6,7-dihydro-5*H*-pyrrolo[2,1-c][1,2,4]triazol-3-yl)azetidine-1-carboxylate (100.0 mg, 0.20 mmol) in tetrahydrofuran (3.0 mL). The mixture was stirred at 25 °C for 2 h. After completion of the reaction, the solvent was removed by distillation under reduced pressure. The resulting crude product was purified by silica gel chromatography (methanol:dichloromethane = 1:5) to give the target product (80.0 mg, yield: 87%).

LC-MS: $[M+H]^+$: 425.2.

**Synthesis of (*S*)-2-(3-(azetidin-3-yl)-6,7-dihydro-5*H*-pyrrolo[2,1-*c*][1,2,4]triazol-6-yl)-3,4-dichlorophenol** (6):

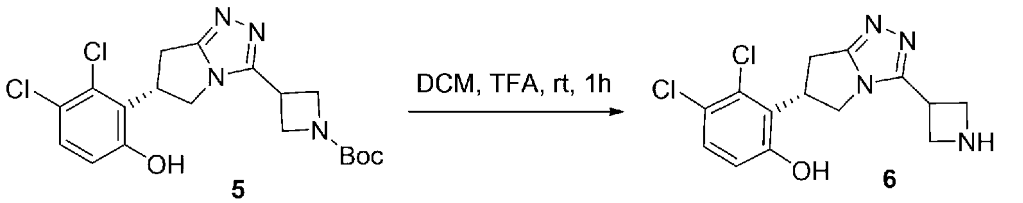


5 6

At room temperature, *tert*-butyl (S)-3-(6-(2,3-dichloro-6-hydroxyphenyl)-6,7-dihydro-5*H*-pyrrolo[2,1-*c*][1,2,4]triazol-3-yl)azetidine-1-carboxylate (60.0 mg, 0.14 mmol) was dissolved in a mixed solvent of dichloromethane/trifluoroacetic acid (3:1, 3.0 mL). The mixture was stirred at room temperature for 1 h. After completion of the reaction, the reaction mixture was filtered through an organic-phase filter membrane, and the filtrate was rotary evaporated to dryness. The crude product was subjected to preparative chromatography to give a white solid (5.0 mg, yield: 10%).

LC-MS: $[M+H]^+$: 325.1.

$^1$H NMR (400 MHz, DMSO-*d*$_6$) δ 8.31 (s, 0.33H), 7.39 (d, *J*=8.8, 1H), 6.87 (d, *J*=8.9, 1H), 5.00 - 4.94 (m, 1H), 4.26 (q, *J*=9.8, 1H), 4.03 (dd, *J*=15.8, 8.1, 2H), 3.91 - 3.77 (m, 4H), 3.10 (t, *J*=8.9, 3H).

**Example 46. (*S*)-2-(3-(1*H*-Pyrazol-4-yl)-6,7-dihydro-5*H*-pyrrolo[2,1-*c*][1,2,4]triazol-6-yl)-3,4-dichlorophenol**

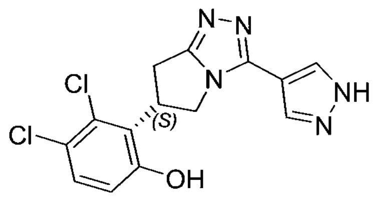

This example was prepared by reference to the preparation method for Example 32, except that the methyl 3-hydroxypropanoate was replaced by methyl 1*H*-pyrazole-4-carboxylate.

LCMS $[M+H]^+$: 336.1.

$^1$H NMR (400 MHz, DMSO-d6) δ 13.26 (s, 1H), 10.82 - 10.40 (m, 1H), 8.17 (ddd, J=103.5, 75.2, 55.7, 2H), 7.35 (d, J=8.8, 1H), 6.81 (d, J=8.7, 1H), 5.01 (s, 1H), 4.42 (t, J=10.0, 1H), 4.16 (dd, J=10.1, 7.4, 1H), 3.11 (dd, J=24.1, 8.9, 2H).

**Examples 47-48. 3,4-Dichloro-2-((*S*)-3-((*S*)-pyrrolidin-3-yl)-6,7-dihydro-5*H*-pyrrolo[2,1-*c*][1,2,4]triazol-6-yl)phenol and 3,4-dichloro-2-((*S*)-3-((*R*)-pyrrolidin-3-yl)-6,7-dihydro-5*H*-pyrrolo[2,1-*c*][1,2,4]triazol-6-yl)phenol**

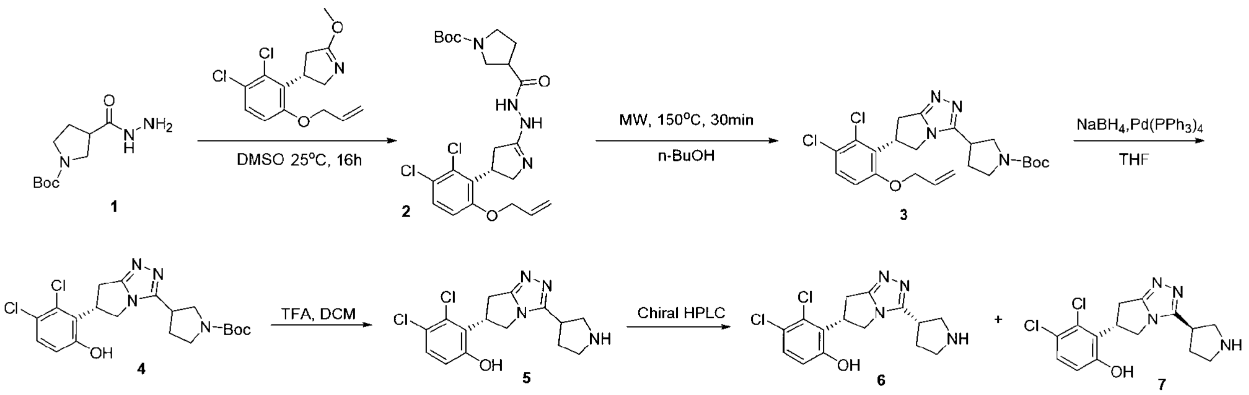


1 2 3 4 5 6 7

**Synthesis of *tert-butyl* 3-(2-((S)-3-(6-(allyloxy)-2,3-dichlorophenyl)-3,4-dihydro-2*H*-pyrrol-5-yl)hydrazine-1-carbonyl)pyrrolidine-1-carboxylate (2):**

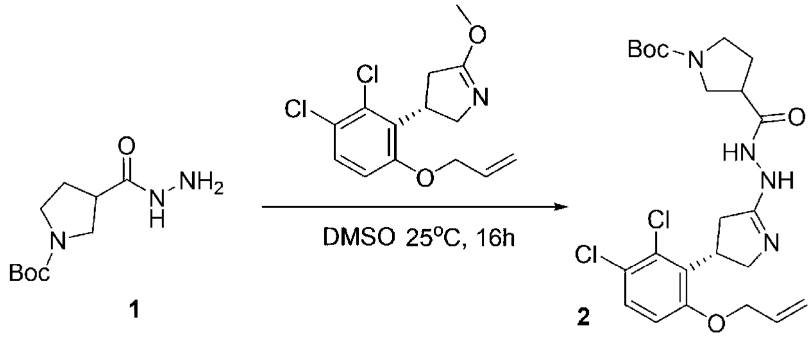


1

2

The compound (*S*)-3-(6-(allyloxy)-2,3-dichlorophenyl)-5-methoxy-3,4-dihydro-2*H*-pyrrole (600.0 mg, 2.00 mmol) and *tert*-butyl 3-(hydrazinecarbonyl)pyrrolidine-1-carboxylate (920.6 mg, 4.00 mmol) were dissolved in a dimethylsulfoxide solution (6 mL), and the mixture was allowed to react at 25 °C for 16 h. After completion of the reaction, the reaction mixture was lyophilized *in vacuo.* The resulting crude product was purified by silica gel chromatography (dichloromethane:methanol = 10:1) to give a white solid (800.0 mg, yield: 80%).

LC-MS: $[M+H]^+$: 497.0.

**Synthesis of *tert*-butyl 3-((*S*)-6-(6-(allyloxy)-2,3-dichlorophenyl)-6,7-dihydro-5*H*-pyrrolo[2,1-*c*][1,2,4]triazol-3-yl) pyrrolidine-1-carboxylate (3) and (*S*)-6-(6-(allyloxy)-2,3-dichlorophenyl)-3-(pyrrolidin-3-yl)-6,7-dihydro-5*H*-pyrrolo[2,1-*c*][1,2,4]triazole** (3'):

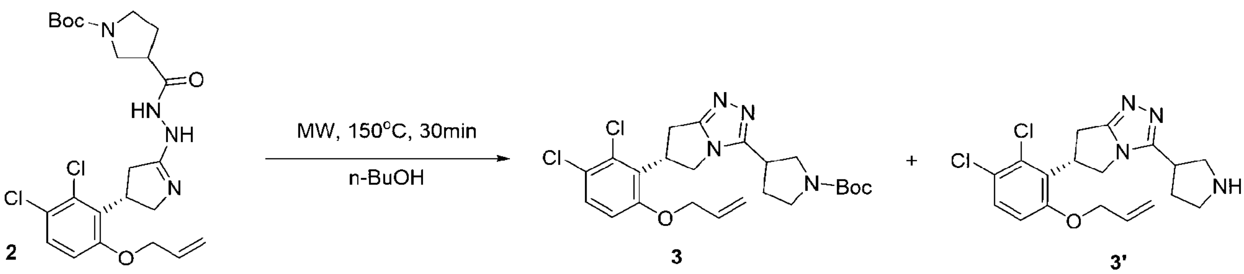


2

3

+

3'

The compound *tert*-butyl 3-(2-((*S*)-3-(6-(allyloxy)-2,3-dichlorophenyl)-3,4-dihydro-2H-pyrrol-5-yl)hydrazine-1-carbonyl)pyrrolidine-1-carboxylate (600.0 mg, 1.20 mmol) was dissolved in *n*-butanol (6 mL), and the mixture was stirred under microwave at 150 °C for 30 min. After completion of the reaction, the reaction mixture was concentrated *in vacuo.* The resulting crude product was purified by silica gel chromatography (dichloromethane:methanol = 10:1) to give a white solid as the target product (3) (200.0 mg, yield: 35%). LC-MS: $[M+H]^+$: 479.0.

In addition, in the column chromatography process, a white solid as the target product (3') (300.0 mg, yield: 66%) was simultaneously obtained. LC-MS: $[M+H]^+$: 379.0.

**Synthesis of *tert*-butyl 3-((*S*)-6-(2,3-dichloro-6-hydroxyphenyl)-6,7-dihydro-5H-pyrrolo[2,1-*c*][1,2,4]triazol-3-yl) pyrrolidine-1-carboxylate (4):**

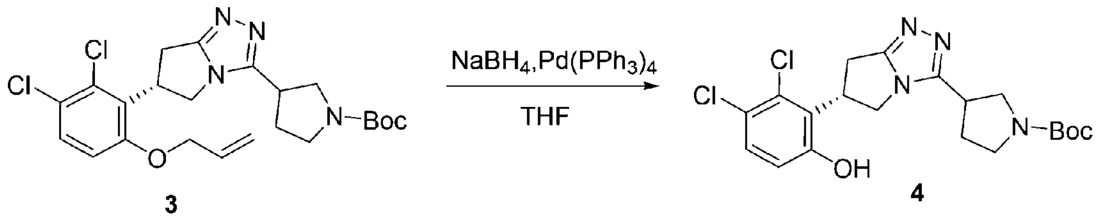


The compound tert-butyl 3-((S)-6-(6-(allyloxy)-2,3-dichlorophenyl)-6,7-dihydro-5*H*-pyrrolo[2,1-*c*][1,2,4]triazol-3-yl)pyrrolidine-1-carboxylate (200.0 mg, 0.42 mmol) was dissolved in a tetrahydrofuran solution (20 mL), and sodium borohydride (23.6 mg, 0.62 mmol) and tetrakis(triphenylphosphine)palladium(0) (96.2 mg, 0.083 mmol) were added to the reaction mixture. The resulting mixture was allowed to react at room temperature for 2 h. After completion of the reaction, the reaction mixture was concentrated *in vacuo*. The resulting crude product was purified by silica gel chromatography (dichloromethane:methanol = 10:1) to give a white solid (120.0 mg, yield: 65%).

LC-MS: $[M+H]^+$: 439.0.

**Synthesis of 3,4-dichloro-2-((6*S*)-3-(pyrrolidin-3-yl)-6,7-dihydro-5*H*-pyrrolo[2,1-*c*][1,2,4]triazol-6-yl)phenol (5):**

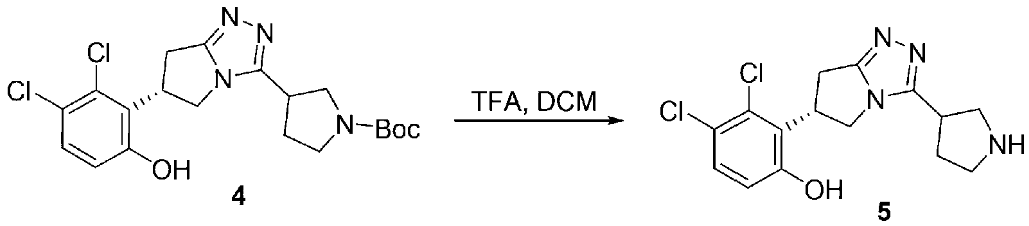


The compound *tert*-*butyl* 3-((*S*)-6-(2,3-dichloro-6-hydroxyphenyl)-6,7-dihydro-5*H*-pyrrolo[2,1-*c*][1,2,4]triazol-3-yl)pyrrolidine-1-carboxylate (100.0 mg, 0.23 mmol) was dissolved in a mixed solution of trifluoroacetic acid and dichloromethane (3:1, 4 mL), and the mixture was allowed to react at room temperature for 2 h. After completion of the reaction, the reaction mixture was evaporated to dryness, and the residue was separated by preparative chromatography to give a white solid (50.0 mg, yield: 65%).

LC-MS: $[M+H]^+$: 339.0.

**Preparation of 3,4-dichloro-2-((6*S*)-3-((*S*)-pyrrolidin-3-yl)-6,7-dihydro-5*H*-pyrrolo[2,1-*c*][1,2,4]triazol-6-yl)phenol and 3,4-dichloro-2-((6*S*)-3-((*R*)-pyrrolidin-3-yl)-6,7-dihydro-5*H*-pyrrolo[2,1-*c*][1,2,4]triazol-6-yl)phenol:**

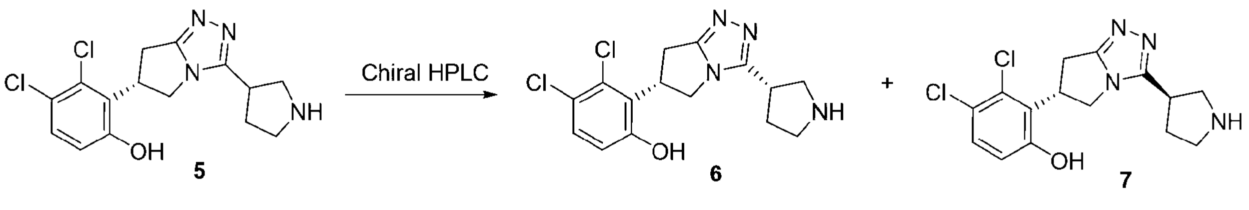


*3,4-Dichloro-2-((6S)-3-(pyrrolidin-3-yl)-6,7-dihydro-5H-pyrrolo[2,1-c][1,2,4]triazol-6-yl)phenol* (50.0 mg, 0.15 mmol) was subjected to preparative chiral chromatography (chromatographic column: CHIRALPAK IC 250 mm 20 mm, 5 μm; mobile phase: 40% IPA ($NH_4OH$ 0.2%)) to give a pair of enantiomers.

The first compound obtained from the SFC resolution was designated as Example 47 (10.1 mg, yield: 20%), with its RT of 6.75 min analyzed under the analytical chromatographic conditions of SFC.

$^1$H NMR (400 MHz, DMSO-*d6*) δ 7.32 (d, *J* = 8.8 Hz, 1H), 6.77 (d, *J* = 8.8 Hz, 1H), 4.99 - 4.90 (m, 1H), 4.22 (t, *J* = 9.8 Hz, 1H), 4.11 - 4.03 (m, 1H), 3.67 - 3.37 (m, 1H), 3.34 - 3.25 (m, 1H), 3.20 - 3.12 (m, 1H), 3.08 (d, *J* = 8.9 Hz, 2H), 2.93 - 2.85 (m, 2H), 2.08 (dt, *J* = 13.8, 8.2 Hz, 1H), 1.93 (dt, *J* = 19.8, 7.2 Hz, 1H).

The second compound obtained from the SFC resolution was designated as Example 48 (11.9 mg, yield: 24%), with its RT of 9.0 min analyzed under the analytical chromatographic conditions of SFC.

$^1$H NMR (400 MHz, DMSO-*d6*) δ 9.54 (s, 1H), 7.38 (d, *J* = 8.8 Hz, 1H), 6.89 (d, *J* = 8.8 Hz, 1H), 4.95 (dd, *J* = 17.3,

8.9 Hz, 1H), 4.27 (t, *J* = 9.9 Hz, 1H), 4.07 (dd, *J* = 10.0, 7.7 Hz, 1H), 3.55 - 3.48 (m, 1H), 3.44 - 3.37 (m, 1H), 3.23 (dd, *J* = 11.1, 7.2 Hz, 1H), 3.11 (dtt, *J* = 15.7, 10.5, 5.2 Hz, 4H), 2.23 (td, *J* = 13.3, 7.7 Hz, 1H), 1.97 (td, *J* = 15.0, 7.5 Hz, 1H).

**Example 49. 3,4-Dichloro-2-((6*S*)-3-(1-methylpyrrolidin-3-yl)-6,7-dihydro-5*H*-pyrrolo[2,1-*c*][1,2,4]triazol-6-yl) phenol**

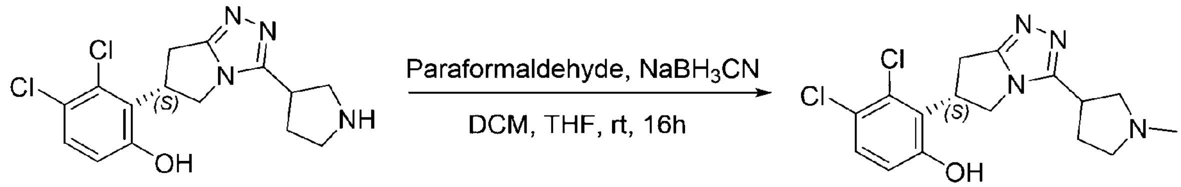


At room temperature, paraformaldehyde (12.0 mg, 0.40 mmol) was added to a solution of 3,4-dichloro-2-((6*S*)-3-(pyrrolidin-3-yl)-6,7-dihydro-5*H*-pyrrolo[2,1-*c*][1,2,4]triazol-6-yl)phenol (15.0 mg, 0.04 mmol) in dichloromethane (2.0 mL). The mixture was stirred at room temperature for 0.5 h and then cooled to 0 °C. Sodium triacetoxyborohydride (28.0 mg, 0.13 mmol) was added, and the resulting mixture was stirred at room temperature for 16 h. After completion of the reaction, the reaction mixture was directly rotary evaporated to dryness. The crude product was purified by preparative chromatography to give a white solid (3.0 mg, yield: 20%).

LC-MS: $[M+H]^+$: 353.0.

$^1H$ NMR (400 MHz, DMSO-d6) δ 10.70 (s, 1H), 7.42 (d, *J* = 8.8 Hz, 1H), 6.90 (d, *J* = 8.9 Hz, 1H), 5.00 (dd, *J* = 15.6, 7.6 Hz, 1H), 4.32 (t, *J* = 9.9 Hz, 1H), 4.16 - 4.09 (m, 1H), 3.93 (d, *J* = 7.1 Hz, 1H), 3.70 (d, *J* = 29.7 Hz, 1H), 3.48 - 3.36 (m, 1H), 3.18 (t, *J* = 8.8 Hz, 2H), 2.91 (d, *J* = 17.5 Hz, 3H), 2.51 (s, 1H), 2.35 (dd, *J* = 27.3, 9.3 Hz, 2H).

**Examples 50-51. Methyl (*S*)-3-((*S*)-6-(2,3-dichloro-6-hydroxyphenyl)-6,7-dihydro-5*H*-pyrrolo[2,1-*c*][1,2,4]triazol-3-yl)pyrrolidine-1-carboxylate and methyl (*R*)-3-((*S*)-6-(2,3-dichloro-6-hydroxyphenyl)-6,7-dihydro-5*H*-pyrrolo[2,1-*c*][1,2,4]triazol-3-yl)pyrrolidine-1-carboxylate**

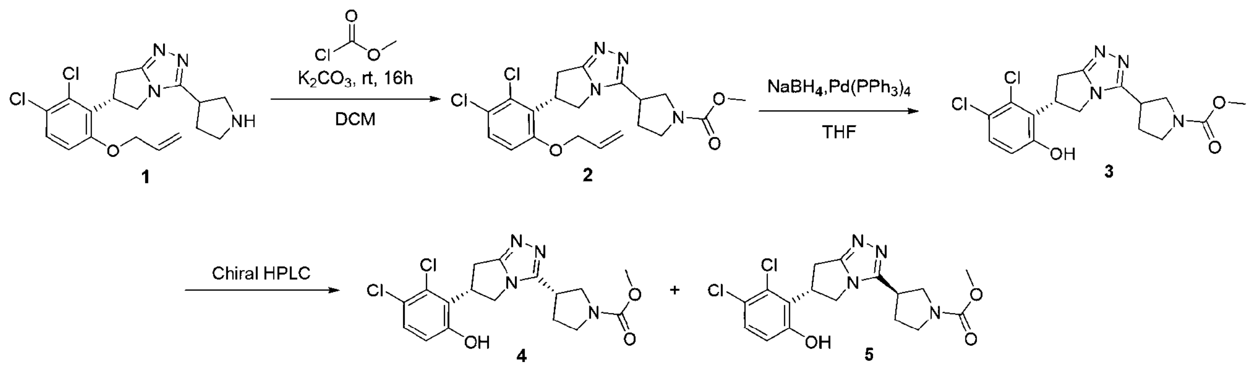


**Synthesis of methyl 3-((*S*)-6-(6-(allyloxy)-2,3-dichlorophenyl)-6,7-dihydro-5*H*-pyrrolo[2,1-*c*][1,2,4]triazol-3-yl) pyrrolidine-1-carboxylate** (2):

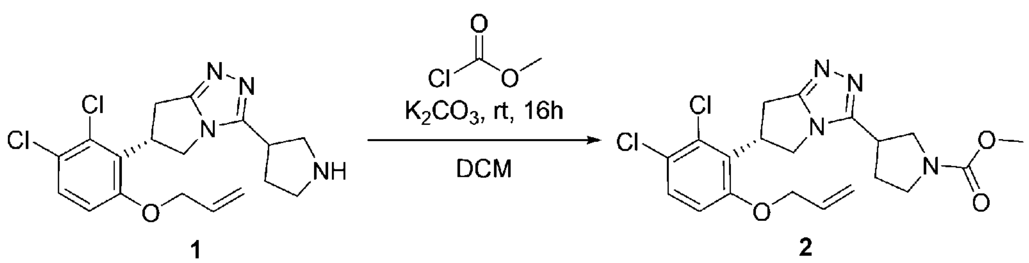

The compound (6*S*)-6-(6-(allyloxy)-2,3-dichlorophenyl)-3-(pyrrolidin-3-yl)-6,7-dihydro-5*H*-pyrrolo[2,1-*c*][1,2,4]triazole (380.0 mg, 1.00 mmol) and potassium carbonate (415.4 mg, 3.00 mmol) were dissolved in dichloromethane (15 mL), and methyl chloroformate (303.0 mg, 3.21 mmol) was slowly added dropwise to the mixture in an ice bath. The resulting mixture was stirred at room temperature for 16 h. The reaction mixture was diluted with water (50 mL) and extracted three times with dichloromethane (50 mL). The organic phases were combined and washed with brine (100 mL). The resulting organic phase was dried over anhydrous sodium sulfate and concentrated *in vacuo.* The resulting crude product was purified by silica gel chromatography (dichloromethane:methanol = 50:1) to give a pale yellow solid (200.0 mg, yield: 46%).

LC-MS: $[M+H]^+$: 437.0.

**Synthesis of methyl 3-((*S*)-6-(2,3-dichloro-6-hydroxyphenyl)-6,7-dihydro-5*H*-pyrrolo[2,1-*c*][1,2,4]triazol-3-yl)pyrrolidine-1-carboxylate (3):**

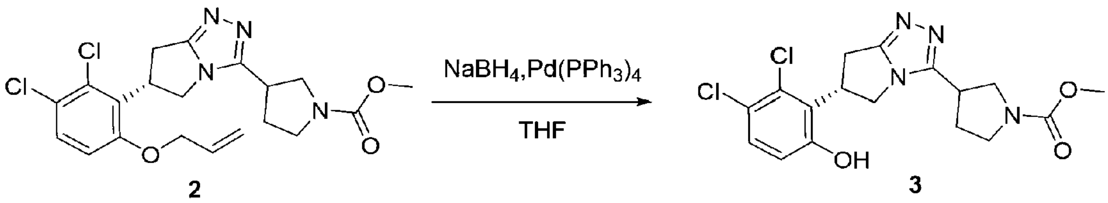


2 3

The compound methyl 3-((*S*)-6-(6-(allyloxy)-2,3-dichlorophenyl)-6,7-dihydro-5*H*-pyrrolo[2,1-*c*][1,2,4]triazol-3-yl)pyrrolidine-1-carboxylate (200.0 mg, 0.46 mmol) was dissolved in a tetrahydrofuran solution (10 mL), and sodium borohydride (26.0 mg, 0.69 mmol) and tetrakis(triphenylphosphine)palladium(0) (105.7 mg, 0.091 mmol) were added to the reaction mixture. The resulting mixture was allowed to react at room temperature for 2 h. The reaction mixture was evaporated to dryness, and the residue was separated by preparative chromatography to give a white solid (30.0 mg, yield: 17%).

LC-MS: $[M+H]^+$: 397.0.

**Preparation of methyl (*S*)-3-((*S*)-6-(2,3-dichloro-6-hydroxyphenyl)-6,7-dihydro-5*H*-pyrrolo[2,1-*c*][1,2,4]triazol-3-yl)pyrrolidine-1-carboxylate and methyl (*R*)-3-((*S*)-6-(2,3-dichloro-6-hydroxyphenyl)-6,7-dihydro-5*H*-pyrrolo[2,1-*c*][1,2,4]triazol-3-yl)pyrrolidine-1-carboxylate:**

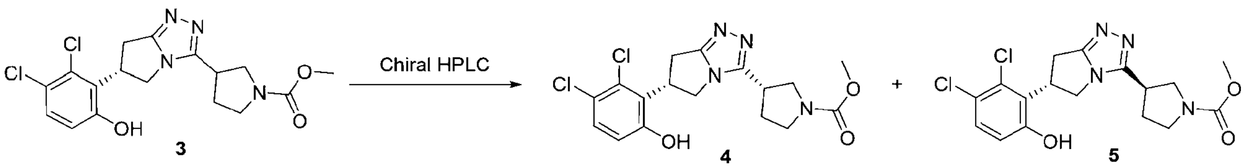


3 4 + 5

Methyl 3-((S)-6-(2,3-dichloro-6-hydroxyphenyl)-6,7-dihydro-5*H*-pyrrolo[2,1-*c*][1,2,4]triazol-3-yl)pyrrolidine-1-carboxylate (30.0 mg, 0.076 mmol) was subjected to preparative chiral chromatography (chromatographic column: CHIRALPAK IC 250 mm 20 mm, 5 μm; mobile phase: 40% EtOH ($NH_4OH$ 0.2%)) to give a pair of enantiomers.

The first compound obtained from the SFC resolution was designated as Example 50 (11.7 mg, yield: 39%), with its RT of 6.24 min analyzed under the analytical chromatographic conditions of SFC.

$^1$H NMR (400 MHz, DMSO-*d6*) δ 10.69 (s, 1H), 7.39 (d, *J* = 8.8 Hz, 1H), 6.85 (d, *J* = 8.8 Hz, 1H), 4.99 (dd, *J* = 17.4, 7.9 Hz, 1H), 4.27 (t, *J* = 9.9 Hz, 1H), 4.06 (t, *J* = 8.5 Hz, 1H), 3.69 (dd, *J* = 13.0, 8.6 Hz, 1H), 3.59 (s, 3H), 3.57 - 3.42 (m, 3H), 3.37 (d, *J* = 8.4 Hz, 1H), 3.17 - 3.01 (m, 2H), 2.25 (s, 1H), 2.16 - 2.07 (m, 1H).

The second compound obtained from the SFC resolution was designated as Example 51 (11.4 mg, yield: 38%), with its RT of 8.08 min analyzed under the analytical chromatographic conditions of SFC.

$^1$H NMR (400 MHz, DMSO-d6) δ 10.75 (s, 1H), 7.38 (d, *J* = 8.8 Hz, 1H), 6.83 (d, *J* = 8.8 Hz, 1H), 5.02 - 4.93 (m, 1H), 4.27 (t, *J* = 9.9 Hz, 1H), 4.06 (dd, *J* = 10.1, 7.5 Hz, 1H), 3.70 (d, *J* = 6.1 Hz, 1H), 3.59 (s, 3H), 3.50 (td, *J* = 13.8, 6.0 Hz, 3H), 3.40 - 3.35 (m, 1H), 3.09 (dt, *J* = 15.7, 6.9 Hz, 2H), 2.25 (d, *J* = 5.5 Hz, 1H), 2.11 - 2.01 (m, 1H).

**Examples 52-53. (*R*)-4-((*S*)-6-(2,3-Dichloro-6-hydroxyphenyl)-6,7-dihydro-5*H*-pyrrolo[2,1-*c*][1,2,4]triazol-3-yl) pyrrolidin-2-one and (*S*)-4-((*S*)-6-(2,3-dichloro-6-hydroxyphenyl)-6,7-dihydro-5*H*-pyrrolo[2,1-*c*][1,2,4]triazol-3-yl)pyrrolidin-2-one**

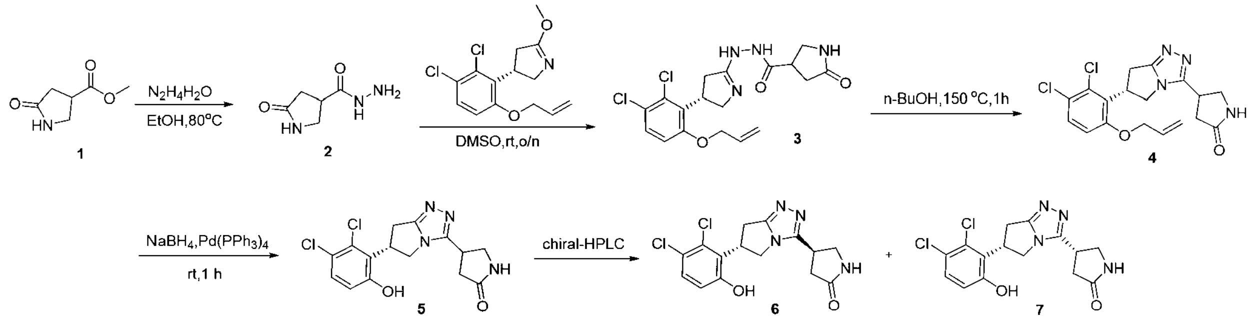


**Synthesis of 5-oxopyrrolidine-3-carbohydrazide (2):**

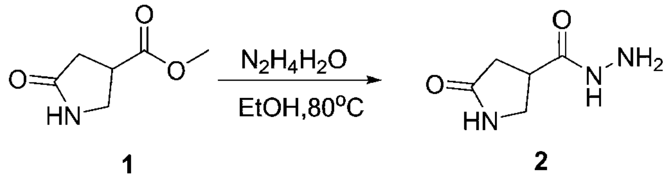


A solution of methyl 5-oxopyrrolidine-3-carboxylate (1.0 g, 7.00 mmol) and hydrazine hydrate (1.05 g, 41.02 mmol, 80% in water) in ethanol (10.0 mL) was stirred at 80 °C for 12 h. The ethanol was then removed by distillation under reduced pressure. The resulting crude product was purified by silica gel chromatography (methanol:dichloromethane = 1:10) to give a white solid (500.0 mg, yield: 50%).

LC-MS: $[M+H]^+$: 144.2.

**Synthesis of *N*'-((*S*)-3-(6-(allyloxy)-2,3-dichlorophenyl)-3,4-dihydro-2*H*-pyrrol-5-yl)-5-oxopyrrolidine-3-carbohydrazide (3):**

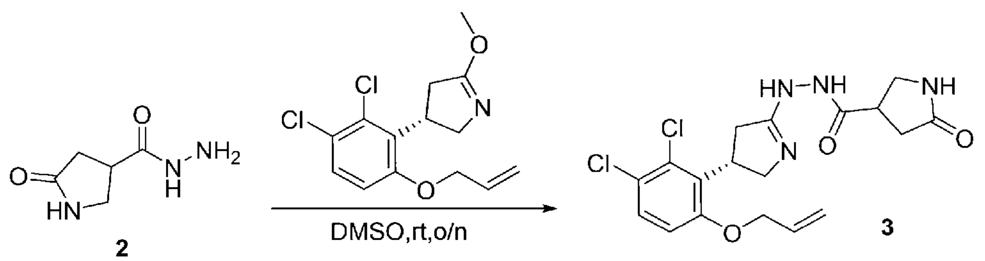


A solution of 5-oxopyrrolidine-3-carbohydrazide (178.8 mg, 1.24 mmol) and (S)-3-(6-(allyloxy)-2,3-dichlorophenyl)-5-methoxy-3,4-dihydro-2H-pyrrole (125.0 mg, 0.41 mmol) in dimethylsulfoxide (2.0 mL) was stirred at room temperature for 12 h. After completion of the reaction, the dimethylsulfoxide was removed by lyophilization under reduced pressure. The resulting crude product was purified by silica gel chromatography (methanol:dichloromethane = 1:10) to give a white solid (80.0 mg, yield: 46%).

LC-MS: $[M+H]^+$: 411.1.

**Synthesis of 4-((*S*)-6-(6-(allyloxy)-2,3-dichlorophenyl)-6,7-dihydro-5*H*-pyrrolo[2,1-*c*][1,2,4]triazol-3-yl)pyrrolidin-2-one (4):**

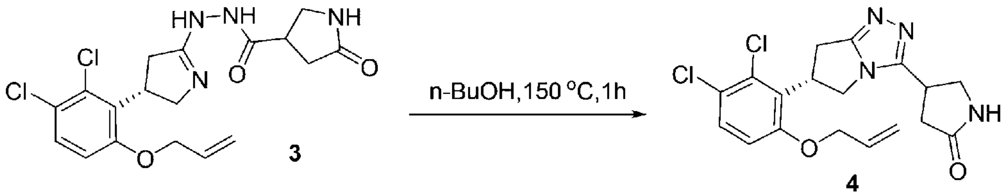

3 4

*N'*-((*S*)-3-(6-(Allyloxy)-2,3-dichlorophenyl)-3,4-dihydro-2*H*-pyrrol-5-yl)-5-oxopyrrolidine-3-carbohydrazide (80.0 mg, 0.19 mmol) was added to a microwave tube, and *n*-butanol (3.0 mL) was added. The mixture was stirred under microwave at 150 °C for 1 h. After completion of the reaction, the n-butanol was removed by distillation under reduced pressure. The resulting crude product was purified by silica gel chromatography to give a white solid (50.0 mg, yield: 65%).
LC-MS: $[M+H]^+$: 393.1.

**Synthesis of 4-((*S*)-6-(2,3-dichloro-6-hydroxyphenyl)-6,7-dihydro-5*H*-pyrrolo[2,1-*c*][1,2,4]triazol-3-yl)pyrrolidin-2-one (5):**

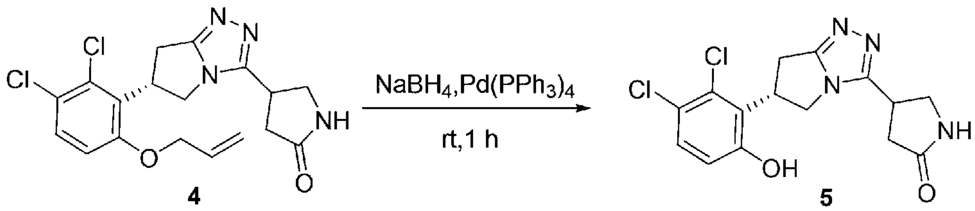

4 5

At room temperature, tetrakis(triphenylphosphine)palladium(0) (73.4 mg, 0.06 mmol) and sodium borohydride (7.2 mg, 0.19 mmol) were added to a solution of 4-((*S*)-6-(6-(allyloxy)-2,3-dichlorophenyl)-6,7-dihydro-5*H*-pyrrolo[2,1-*c*][1,2,4]triazol-3-yl)pyrrolidin-2-one (50.0 mg, 0.12 mmol) in tetrahydrofuran (1.0 mL). The mixture was stirred at room temperature for 1 h. After completion of the reaction, the reaction mixture was filtered through an organic-phase filter membrane, and the filtrate was rotary evaporated to dryness. The crude product was subjected to preparative chromatography to give a white solid (10.5 mg, yield: 12%).
LC-MS: $[M+H]^+$: 353.1.

**Preparation of (*R*)-4-((*S*)-6-(2,3-dichloro-6-hydroxyphenyl)-6,7-dihydro-5*H*-pyrrolo[2,1-*c*][1,2,4]triazol-3-yl)pyrrolidin-2-one and (*S*)-4-((*S*)-6-(2,3-dichloro-6-hydroxyphenyl)-6,7-dihydro-5*H*-pyrrolo[2,1-*c*][1,2,4]triazol-3-yl)pyrrolidin-2-one:**

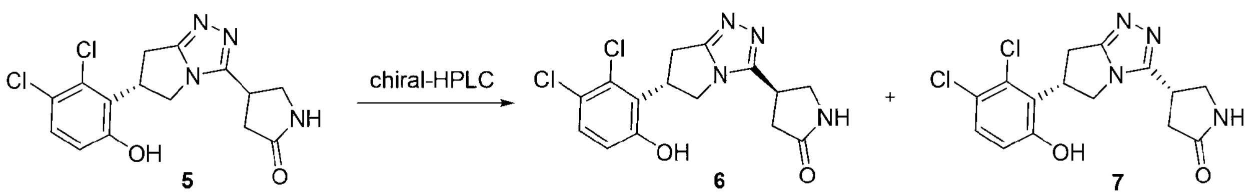

5 6 7

4-(*S*)-6-(2,3-Dichloro-6-hydroxyphenyl)-6,7-dihydro-5*H*-pyrrolo[2,1-*c*][1,2,4]triazol-3-yl)pyrrolidin-2-one (45.0 mg, 0.12 mmol) was subjected to preparative chiral chromatography (chromatographic column: CHIRALPAK IC 250 mm 20 mm, 5 μm; mobile phase: $CO_2$-MeOH ($NH_4OH$ 0.2%)) to give a pair of enantiomers.
The first compound obtained from the SFC resolution was designated as Example 52 (10.5 mg, yield: 23%), with its RT of 2.22 min analyzed under the analytical chromatographic conditions of SFC.
LC-MS: $[M+H]^+$: 353.1.

$^1$H NMR (400 MHz, DMSO-*d6*) δ 10.69 (s, 1H), 7.77 (s, 1H), 7.39 (d, *J* = 8.8 Hz, 1H), 6.85 (d, *J* = 8.8 Hz, 1H), 4.97 (m, 1H), 4.24 (t, *J* = 9.9 Hz, 1H), 4.04 (dd, *J* = 10.1, 7.5 Hz, 1H), 3.77 (dd, *J* = 15.6, 7.9 Hz, 1H), 3.63 (t, *J* = 8.9 Hz, 1H), 3.43 (dd, *J* = 9.5, 6.5 Hz, 1H), 3.10 (m, 2H), 2.56 (dd, *J* = 16.4, 9.2 Hz, 1H), 2.44 (dd, *J* = 16.4, 7.7 Hz, 1H).

The second compound obtained from the SFC resolution was designated as Example 53 (10.2 mg, yield: 23%), with its RT of 3.08 min analyzed under the analytical chromatographic conditions of SFC.

LC-MS: $[M+H]^+$: 353.1.

$^1$H NMR (400 MHz, DMSO-d6) δ 10.58 (s, 1H), 7.77 (s, 1H), 7.40 (d, *J* = 8.8 Hz, 1H), 6.86 (d, *J* = 8.8 Hz, 1H), 4.98 (t, *J* = 8.6 Hz, 1H), 4.24 (d, *J* = 9.9 Hz, 1H), 4.03 (dd, *J* = 10.1, 7.5 Hz, 1H), 3.78 (m, 1H), 3.61 (d, *J* = 8.6 Hz, 1H), 3.42 (dd, *J* = 9.5, 6.6 Hz, 1H), 3.07 (m, 2H), 2.56 (m, 1H), 2.45 (m, 1H).

**Examples 54-55. 3,4-Dichloro-2-((*S*)-3-((*S*)-tetrahydrofuran-3-yl)-6,7-dihydro-5*H*-pyrrolo[2,1-*c*][1,2,4]triazol-6-yl)phenol and 3,4-dichloro-2-((*S*)-3-((*R*)-tetrahydrofuran-3-yl)-6,7-dihydro-5*H*-pyrrolo[2,1-*c*][1,2,4]triazol-6-yl)phenol**

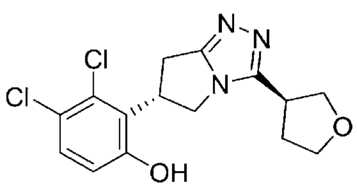

+

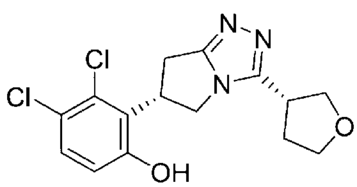

This example was prepared by reference to the preparation method for Examples 52-53, except that the methyl 5-oxopyrrolidine-3-carboxylate was replaced by methyl tetrahydrofuran-3-carboxylate.

The first compound obtained from the SFC resolution was designated as Example 54, with its RT of 2.89 min analyzed under the analytical chromatographic conditions of SFC.

LC-MS: $[M+H]^+$: 340.0.

$^1$H NMR (400 MHz, DMSO-d6) δ 10.66 (s, 1H), 7.40 (d, *J* = 8.8 Hz, 1H), 6.86 (d, *J* = 8.8 Hz, 1H), 4.97 (m, 1H), 4.26 (m, 1H), 4.06 - 3.98 (m, 2H), 3.86 (m, 1H), 3.80 - 3.73 (m, 2H), 3.57 - 3.50 (m, 1H), 3.09 (m, 2H), 2.30 - 2.22 (m, 1H), 2.17 - 2.09 (m, 1H).

The second compound obtained from the SFC resolution was designated as Example 55, with its RT of 3.70 min analyzed under the analytical chromatographic conditions of SFC.

LC-MS: $[M+H]^+$: 340.0.

$^1$H NMR (400 MHz, DMSO-d6) δ 10.73 (s, 1H), 7.39 (d, *J* = 8.8 Hz, 1H), 6.86 (d, *J* = 8.8 Hz, 1H), 4.96 (m, 1H), 4.25 (m, 1H), 4.07 - 3.98 (m, 2H), 3.87 (m, 1H), 3.77 (m, 2H), 3.53 (m, 1H), 3.16 - 3.01 (m, 2H), 2.31 - 2.22 (m, 1H), 2.10 (m, 1H).

**Examples 56-57. (*R*)-4-((*S*)-6-(2,3-Dichloro-6-hydroxyphenyl)-6,7-dihydro-5*H*-pyrrolo[2,1-*c*][1,2,4]triazol-3-yl)piperidin-2-one and (*S*)-4-((*S*)-6-(2,3-dichloro-6-hydroxyphenyl)-6,7-dihydro-5*H*-pyrrolo[2,1-*c*][1,2,4]triazol-3-yl)piperidin-2-one**

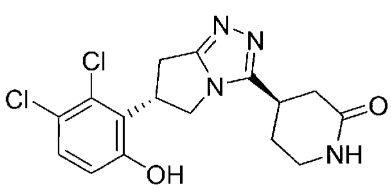

+

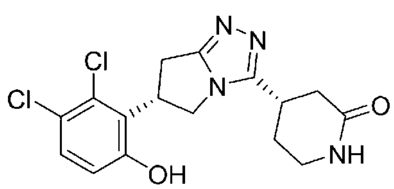

This example was prepared by reference to the preparation method for Examples 52-53, except that the methyl 5-oxopyrrolidine-3-carboxylate was replaced by methyl 2-oxopiperidine-4-carboxylate.

The first compound obtained from the SFC resolution was designated as Example 56, with its RT of 2.40 min analyzed under the analytical chromatographic conditions of SFC.

LC-MS: $[M+H]^+$: 407.0.

$^1$H NMR (400 MHz, $CD_3OD$) δ 7.21 (d, *J* = 8.8 Hz, 1H), 6.70 (d, *J* = 8.8 Hz, 1H), 5.12 - 5.03 (m, 1H), 4.23 (t, *J* = 10.0 Hz, 1H), 4.14 (dd, *J* = 10.4, 7.5 Hz, 1H), 3.26 - 3.22 (m, 2H), 3.15 (d, *J* = 9.3 Hz, 2H), 3.08 (d, *J* = 10.2 Hz, 1H), 2.83 - 2.74 (m, 2H), 2.30 - 2.20 (m, 1H), 1.88 - 1.78 (m, 1H).

The second compound obtained from the SFC resolution was designated as Example 57, with its RT of 3.01 min analyzed under the analytical chromatographic conditions of SFC.

LC-MS: $[M+H]^+$: 407.0.

$^1H$ NMR (400 MHz, $CD_3OD$) δ 7.33 (d, *J* = 8.8 Hz, 1H), 6.81 (d, *J* = 8.8 Hz, 1H), 5.19 (dd, *J* = 9.1, 7.5 Hz, 1H), 4.38 (t, *J* = 10.0 Hz, 1H), 4.25 (dd, *J* = 10.4, 7.3 Hz, 1H), 3.38 - 3.34 (m, 2H), 3.28 (t, *J* = 8.4 Hz, 2H), 3.20 (d, *J* = 10.7 Hz, 1H), 2.95 - 2.84 (m, 2H), 2.41 - 2.30 (m, 1H), 2.06 - 1.94 (m, 1H).

**Examples 58-59. 3,4-Dichloro-2-((*S*)-3-((*S*)-piperidin-3-yl)-6,7-dihydro-5*H*-pyrrolo[2,1-*c*][1,2,4]triazol-6-yl)phenol and 3,4-dichloro-2-((*S*)-3-((*R*)-piperidin-3-yl)-6,7-dihydro-5*H*-pyrrolo[2,1-*c*][1,2,4]triazol-6-yl)phenol**

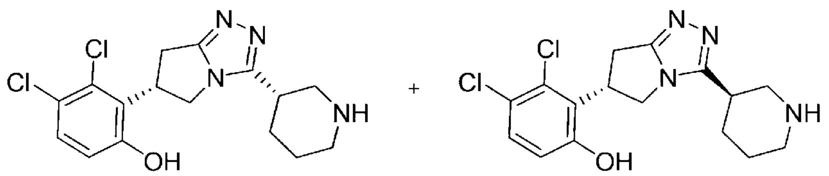

This example was prepared by reference to the preparation method for Examples 47-48, except that the *tert*-butyl 3-(hydrazinecarbonyl)pyrrolidine-1-carboxylate was replaced by *tert*-butyl 3-(hydrazinecarbonyl)piperidine-1-carboxylate.

The first compound obtained from the SFC resolution was designated as Example 58, with its RT of 3.95 min analyzed under the analytical chromatographic conditions of SFC.

LC-MS: $[M+H]^+$: 353.0.

$^1H$ NMR (400 MHz, DMSO-$d_6$) δ 8.28 (s, 1H), 7.38 (d, *J* = 8.8 Hz, 1H), 6.88 (d, *J* = 8.9 Hz, 1H), 4.97 (s, 1H), 4.29 (s, 1H), 4.14 - 4.03 (m, 1H), 3.28 (s, 1H), 3.08 (s, 4H), 2.91 (s, 1H), 2.77 - 2.61 (m, 1H), 1.98 (s, 1H), 1.65 (d, *J* = 9.7 Hz, 2H), 1.54 (s, 1H).

The second compound obtained from the SFC resolution was designated as Example 59, with its RT of 3.99 min analyzed under the analytical chromatographic conditions of SFC.

LC-MS: $[M+H]^+$: 353.0.

$^1H$ NMR (400 MHz, DMSO-*d6*) δ 7.36 (d, J = 8.7 Hz, 1H), 6.84 (d, J = 8.8 Hz, 1H), 4.94 (dd, J = 16.9, 8.9 Hz, 1H), 4.26 (t, J = 9.8 Hz, 1H), 4.11 - 4.02 (m, 1H), 3.30 (s, 1H), 3.06 (dt, J = 15.6, 11.4 Hz, 3H), 2.93 - 2.78 (m, 2H), 2.67 (t, J = 11.0 Hz, 1H), 1.95 (d, J = 8.4 Hz, 1H), 1.62 (d, J = 10.7 Hz, 2H), 1.43 (d, J = 11.3 Hz, 1H).

**Examples 60-61. 2-((*S*)-3-((1*S*,2*R*,4*R*)-7-Oxabicyclo[2.2.1]heptan-2-yl)-6,7-dihydro-5*H*-pyrrolo[2,1-*c*][1,2,4]triazol-6-yl)-3,4-dichlorophenol and 2-((*S*)-3-((1*R*,2*S*,4*S*)-7-oxabicyclo[2.2.1]heptan-2-yl)-6,7-dihydro-5*H*-pyrrolo[2,1-*c*][1,2,4]triazol-6-yl)-3,4-dichlorophenol**

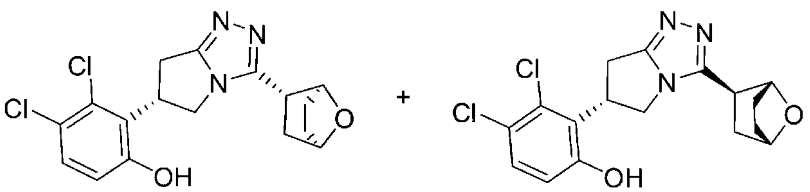

This example was prepared by reference to the preparation method for Examples 52-53, except that the methyl 5-oxopyrrolidine-3-carboxylate was replaced by methyl *endo*-7-oxabicyclo[2.2.1]heptane-2-carboxylate.

The first compound obtained from the SFC resolution was designated as Example 60, with its RT of 2.38 min analyzed under the analytical chromatographic conditions of SFC.

LC-MS: $[M+H]^+$: 366.1.

$^1H$ NMR (400 MHz, DMSO-$d_6$) δ 10.61 (s, 1H), 7.39 (d, *J* = 8.8 Hz, 1H), 6.86 (d, *J* = 8.8 Hz, 1H), 4.94 (t, *J* = 8.6 Hz, 1H), 4.65 (t, *J* = 4.0 Hz, 1H), 4.57 (s, 1H), 4.28 (t, *J* = 10.0 Hz, 1H), 4.03 (dd, *J* = 10.4, 7.4 Hz, 1H), 3.17 (dd, *J* = 8.8, 5.1 Hz, 1H), 3.13 - 3.00 (m, 2H), 1.97 (dd, *J* = 11.2, 5.7 Hz, 1H), 1.90 (dd, *J* = 11.9, 9.0 Hz, 1H), 1.64 - 1.49 (m, 4H).

The second compound obtained from the SFC resolution was designated as Example 61, with its RT of 2.92 min analyzed under the analytical chromatographic conditions of SFC.

LC-MS: $[M+H]^+$: 353.0.

$^1$H NMR (400 MHz, DMSO-d6) δ 10.54 (s, 1H), 7.39 (d, *J* = 8.8 Hz, 1H), 6.86 (d, *J* = 8.8 Hz, 1H), 5.01 - 4.89 (m, 1H), 4.64 (dd, *J* = 12.9, 8.9 Hz, 2H), 4.24 (t, *J* = 10.0 Hz, 1H), 4.05 (dd, J = 10.4, 7.5 Hz, 1H), 3.19 - 3.14 (m, 1H), 3.13 - 2.98 (m, 2H), 2.06 - 1.97 (m, 1H), 1.89 (dd, *J* = 11.9, 9.1 Hz, 1H), 1.64 - 1.48 (m, 4H).

**Examples 62-63. 2-((*S*)-3-((1*R*,2*R*,4*S*)-7-Oxabicyclo[2.2.1]heptan-2-yl)-6,7-dihydro-5*H*-pyrrolo[2,1-*c*][1,2,4]triazol-6-yl)-3,4-dichlorophenol and 2-((*S*)-3-((1*S*,2*S*,4*R*)-7-oxabicyclo[2.2.1]heptan-2-yl)-6,7-dihydro-5*H*-pyrrolo[2,1-*c*][1,2,4]triazol-6-yl)-3,4-dichlorophenol**

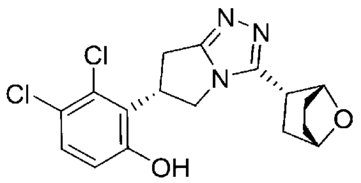
+
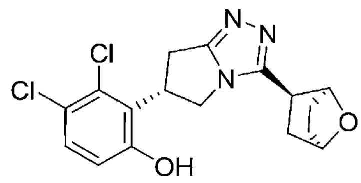

This example was prepared by reference to the preparation method for Examples 52-53, except that the methyl 5-oxopyrrolidine-3-carboxylate was replaced by methyl *exo*-7-oxabicyclo[2.2.1]heptane-2-carboxylate.

The first compound obtained from the SFC resolution was designated as Example 62, with its RT of 3.073 min analyzed under the analytical chromatographic conditions of SFC.

LC-MS: $[M+H]^+$: 366.1.

$^1$H NMR (400 MHz, DMSO-$d_6$) δ 10.60 (s, 1H), 7.38 (d, *J* = 8.8 Hz, 1H), 6.85 (d, *J* = 8.8 Hz, 1H), 4.94 (t, *J* = 8.6 Hz, 1H), 4.65 (t, *J* = 4.0 Hz, 1H), 4.57 (s, 1H), 4.27 (t, *J* = 10.0 Hz, 1H), 4.03 (dd, *J* = 10.4, 7.4 Hz, 1H), 3.17 (dd, *J* = 8.8, 5.1 Hz, 1H), 3.12 - 3.00 (m, 2H), 1.93 (ddd, *J* = 20.9, 12.0, 7.8 Hz, 2H), 1.63 - 1.48 (m, 4H). The second compound obtained from the SFC resolution was designated as Example 63, with its RT of 3.074 min analyzed under the analytical chromatographic conditions of SFC.

LC-MS: $[M+H]^+$: 353.0.

$^1$H NMR (400 MHz, DMSO-*d*6) δ 10.54 (s, 1H), 7.39 (d, *J* = 8.8 Hz, 1H), 6.86 (d, *J* = 8.8 Hz, 1H), 5.02 - 4.88 (m, 1H), 4.64 (dd, *J* = 13.0, 9.0 Hz, 2H), 4.24 (t, *J* = 10.0 Hz, 1H), 4.05 (dd, *J* = 10.4, 7.6 Hz, 1H), 3.19 - 3.14 (m, 1H), 3.13 - 2.98 (m, 2H), 2.04 - 1.97 (m, 1H), 1.89 (dd, *J* = 11.9, 9.1 Hz, 1H), 1.64 - 1.47 (m, 4H).

**Examples 64-65. 3,4-Dichloro-2-((*S*)-3-((*S*)-3-methyltetrahydrofuran-3-yl)-6,7-dihydro-5*H*-pyrrolo[2,1-*c*][1,2,4]triazol-6-yl)phenol and 3,4-dichloro-2-((*S*)-3-((*R*)-3-methyltetrahydrofuran-3-yl)-6,7-dihydro-5*H*-pyrrolo[2,1-*c*][1,2,4]triazol-6-yl)phenol**

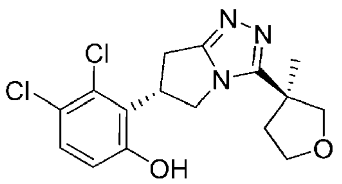
+
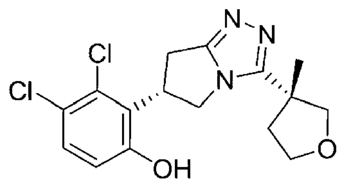

This example was prepared by reference to the preparation method for Examples 52-53, except that the methyl 5-oxopyrrolidine-3-carboxylate was replaced by methyl 3-methyltetrahydrofuran-3-carboxylate.

The first compound obtained from the SFC resolution was designated as Example 64, with its RT of 3.28 min analyzed under the analytical chromatographic conditions of SFC.

LC-MS: $[M+H]^+$: 354.0.

$^1$H NMR (400 MHz, DMSO-$d_6$) δ 7.31 (d, *J* = 8.8 Hz, 1H), 6.79 (d, *J* = 8.8 Hz, 1H), 5.19 (s, 1H), 4.43 (d, *J* = 9.9 Hz, 1H), 4.34 - 4.24 (m, 1H), 4.13 (d, *J* = 8.7 Hz, 1H), 3.98 (t, *J* = 7.2 Hz, 2H), 3.76 (d, *J* = 8.7 Hz, 1H), 3.25 (d, *J* = 10.3 Hz, 1H), 3.17 (d, *J* = 7.1 Hz, 1H), 2.50 (d, *J* = 12.5 Hz, 1H), 2.14 - 2.06 (m, 1H), 1.49 (s, 3H).

The second compound obtained from the SFC resolution was designated as Example 65, with its RT of 4.11 min analyzed under the analytical chromatographic conditions of SFC.

LC-MS: $[M+H]^+$: 354.0.

$^1$H NMR (400 MHz, DMSO-d6) δ 7.30 (d, *J* = 8.8 Hz, 1H), 6.79 (d, *J* = 8.8 Hz, 1H), 5.17 (d, *J* = 9.8 Hz, 1H), 4.42 (t, *J*

= 10.0 Hz, 1H), 4.30 (dd, *J* = 10.4, 7.0 Hz, 1H), 4.13 (d, *J* = 8.6 Hz, 1H), 3.98 (dd, *J* = 7.6, 6.7 Hz, 2H), 3.76 (d, *J* = 8.6 Hz, 1H), 3.28 - 3.22 (m, 1H), 3.16 (dd, *J* = 16.2, 7.4 Hz, 1H), 2.56 - 2.45 (m, 1H), 2.09 (dt, *J* = 12.9, 6.6 Hz, 1H), 1.49 (s, 3H).

**Example 66. (*S*)-2-(3-(2-Oxabicyclo[2.1.1]hexan-4-yl)-6,7-dihydro-5*H*-pyrrolo[2,1-*c*][1,2,4]triazol-6-yl)-3,4-dichlorophenol**

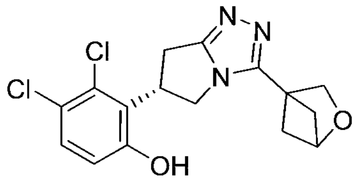

This example was prepared by reference to the preparation method for Example 32, except that the methyl 3-hydroxypropanoate was replaced by methyl 2-oxabicyclo[2.1.1]hexane-4-carboxylate.

LCMS $[M+H]^+$: 352.1.

$^1$H NMR (400 MHz, DMSO-*d*6) δ 10.75 (s, 1H), 7.39 (d, *J*=8.8, 1H), 6.86 (d, *J*=8.8, 1H), 5.04 - 4.97 (m, 1H), 4.58 (s, 1H), 4.36 (t, *J* =10.0, 1H), 4.13 - 4.08 (m, 1H), 3.88 (dd, *J*=11.8, 5.7, 2H), 3.16 - 3.06 (m, 2H), 2.24 (dd, *J*=12.9, 5.1, 2H), 1.77 (dd, *J*=9.4, 6.6, 2H).

**Example 67. (*S*)-3,4-Dichloro-2-(3-cyclopentyl-6,7-dihydro-5*H*-pyrrolo[2,1-*c*][1,2,4]triazol-6-yl)phenol**

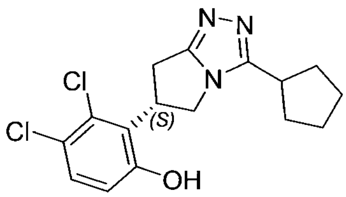

This example was prepared by reference to the preparation method for Example 32, except that the methyl 3-hydroxypropanoate was replaced by methyl cyclopentanecarboxylate.

LCMS $[M+H]^+$: 338.0.

$^1$H NMR (400 MHz, DMSO-d6) δ 10.65 (s, 1H), 7.39 (d, *J* = 8.8 Hz, 1H), 6.85 (d, *J* = 8.8 Hz, 1H), 4.97 (t, *J* = 8.6 Hz, 1H), 4.23 (t, *J* = 9.9 Hz, 1H), 4.02 (dd, *J* = 10.1, 7.4 Hz, 1H), 3.12 (ddd, *J* = 15.8, 8.9, 3.5 Hz, 2H), 3.01 (dd, *J* = 15.7, 7.8 Hz, 1H), 2.02 - 1.93 (m, 2H), 1.80 - 1.56 (m, 6H).

**Examples 68-69. 3,4-Dichloro-2-((*S*)-3-((*S*)-tetrahydro-2*H*-pyran-3-yl)-6,7-dihydro-5*H*-pyrrolo[2,1-*c*][1,2,4]triazol-6-yl)phenol and 3,4-dichloro-2-((*S*)-3-((*R*)-tetrahydro-2*H*-pyran-3-yl)-6,7-dihydro-5*H*-pyrrolo[2,1-*c*][1,2,4]triazol-6-yl)phenol**

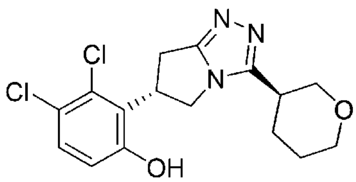

+

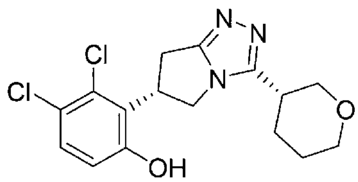

This example was prepared by reference to the preparation method for Examples 52-53, except that the 5-oxopyrrolidine-3-carbohydrazide was replaced by tetrahydro-2*H*-pyran-3-carbohydrazide.

The first compound obtained from the SFC resolution was designated as Example 68, with its RT of 2.69 min analyzed under the analytical chromatographic conditions.

LC-MS: $[M+H]^+$: 354.0.

$^1$H NMR (400 MHz, DMSO-d6) δ 10.77 (s, 1H), 7.38 (d, *J* = 8.8 Hz, 1H), 6.84 (d, *J* = 8.8 Hz, 1H), 4.95 (t, *J* = 8.6 Hz, 1H), 4.29 (t, *J* = 9.9 Hz, 1H), 4.09 (dd, *J* = 10.1, 7.6 Hz, 1H), 3.93 (dd, *J* = 11.0, 2.8 Hz, 1H), 3.83 (d, *J* = 11.3 Hz, 1H), 3.46 (t, *J* = 10.7 Hz, 1H), 3.38 (dd, *J* = 11.1, 8.3 Hz, 1H), 3.14 - 3.03 (m, 2H), 3.01 - 2.95 (m, 1H), 2.02 (d, *J* = 13.1 Hz, 1H), 1.82 - 1.74 (m, 1H), 1.68 - 1.56 (m, 2H).

The second compound obtained from the SFC resolution was designated as Example 69, with its RT of 3.54 min analyzed under the analytical chromatographic conditions of SFC.

LC-MS: [M+H]$^+$: 354.1.

$^1$H NMR (400 MHz, DMSO-d6) δ 10.74 (s, 1H), 7.38 (d, *J* = 8.8 Hz, 1H), 6.84 (d, *J* = 8.9 Hz, 1H), 4.95 (t, *J* = 8.7 Hz, 1H), 4.30 (t, *J* = 9.9 Hz, 1H), 4.07 (dd, *J* = 10.2, 7.5 Hz, 1H), 4.00 (dd, *J* = 11.2, 2.7 Hz, 1H), 3.84 (d, *J* = 11.1 Hz, 1H), 3.45 (t, *J* = 10.8 Hz, 1H), 3.39 (dd, *J* = 11.0, 3.2 Hz, 1H), 3.14 - 3.02 (m, 2H), 2.98 (dd, *J* = 7.9, 3.9 Hz, 1H), 2.01 (s, 1H), 1.80 - 1.72 (m, 1H), 1.68 - 1.58 (m, 2H).

**Examples 70-71. 3,4-Dichloro-2-((*S*)-3-((1*s*,3*R*)-3-hydroxycyclobutyl)-6,7-dihydro-5*H*-pyrrolo[2,1-*c*][1,2,4]triazol-6-yl)phenol and 3,4-dichloro-2-((*S*)-3-((1*r*,3*S*)-3-hydroxycyclobutyl)-6,7-dihydro-5*H*-pyrrolo[2,1-*c*][1,2,4]triazol-6-yl)phenol**

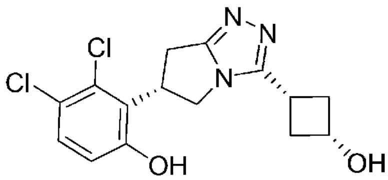
+
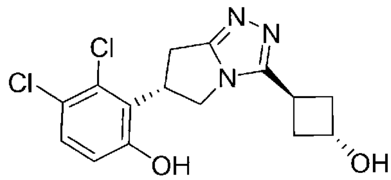

This example was prepared by reference to the preparation method for Examples 52-53, except that the methyl 5-oxopyrrolidine-3-carboxylate was replaced by methyl 3-hydroxycyclobutane-1-carboxylate.

The first compound obtained from the SFC resolution was designated as Example 70,with its RT of 4.31 min analyzed under the analytical chromatographic conditions of SFC.

LC-MS: [M+H]$^+$: 340.0.

$^1$H NMR (400 MHz, $CD_3OD$) δ 7.30 (d, *J* = 8.8 Hz, 1H), 6.78 (d, *J* = 8.8 Hz, 1H), 5.17 (t, *J* = 8.4 Hz, 1H), 4.35 (d, *J* = 10.5 Hz, 1H), 4.29 - 4.19 (m, 2H), 3.26 - 3.06 (m, 3H), 2.73 (ddd, *J* = 11.4, 7.0, 3.7 Hz, 2H), 2.32 - 2.12 (m, 2H).

The second compound obtained from the SFC resolution was designated as Example 71, with its RT of 4.56 min analyzed under the analytical chromatographic conditions of SFC.

LC-MS: [M+H]$^+$: 340.0.

**Examples 72-73. 2-((6*S*)-3-((1*S*)-3-Oxabicyclo[3.1.0]hexan-1-yl)-6,7-dihydro-5*H*-pyrrolo[2,1-*c*][1,2,4]triazol-6-yl)-3,4-dichlorophenol and 2-((6*S*)-3-((1*R*)-3-oxabicyclo[3.1.0]hexan-1-yl)-6,7-dihydro-5*H*-pyrrolo[2,1-*c*][1,2,4]triazol-6-yl)-3,4-dichlorophenol**

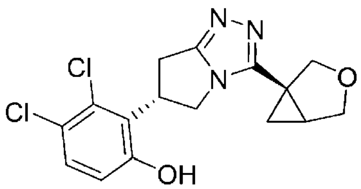
+
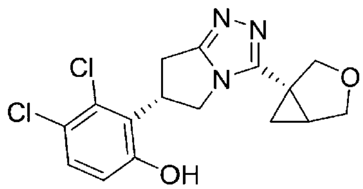

This example was prepared by reference to the preparation method for Examples 52-53, except that the methyl 5-oxopyrrolidine-3-carboxylate was replaced by methyl 3-oxabicyclo[3.1.0]hexane-1-carboxylate.

The first compound obtained from the SFC resolution was designated as Example 72, with its RT of 4.35 min analyzed under the analytical chromatographic conditions of SFC.

LC-MS: [M+H]$^+$: 351.9.

$^1$H NMR (400 MHz, DMSO-$d_6$) δ 10.57 (s, 1H), 7.40 (d, *J* = 8.8 Hz, 1H), 6.87 (d, *J* = 8.8 Hz, 1H), 4.96 (dd, *J* = 13.3, 5.5 Hz, 1H), 4.26 (t, *J* = 9.8 Hz, 1H), 4.06 *(dd, J* = 10.0, 7.5 Hz, 1H), 4.00 (d, *J* = 8.2 Hz, 1H), 3.87 (d, *J* = 8.2 Hz, 1H), 3.78 (dt, *J* = 8.5, 5.5 Hz, 2H), 3.08 (dt, *J* = 15.8, 6.9 Hz, 2H), 2.18 (ddd, *J* = 7.8, 4.8, 2.6 Hz, 1H), 1.25 (d, *J* = 4.8 Hz, 1H), 0.91 (t, *J* = 4.8 Hz, 1H).

The second compound obtained from the SFC resolution was designated as Example 73, with its RT of 5.04 min analyzed under the analytical chromatographic conditions of SFC.

LC-MS: $[M+H]^+$: 351.9.

$^1H$ NMR (400 MHz, DMSO-$d_6$) δ 7.39 (d, *J* = 8.8 Hz, 1H), 6.84 (d, *J* = 8.8 Hz, 1H), 4.95 (td, *J* = 9.5, 4.7 Hz, 1H), 4.27 (t, *J* = 9.8 Hz, 1H), 4.05 (dd, *J* = 10.0, 7.5 Hz, 1H), 3.99 (d, *J* = 8.2 Hz, 1H), 3.92 (d, *J* = 8.3 Hz, 1H), 3.81 (d, *J* = 8.4 Hz, 1H), 3.76 (dd, *J* = 8.5, 2.7 Hz, 1H), 3.08 (dt, *J* = 15.8, 6.9 Hz, 2H), 2.19 (ddd, *J* = 7.9, 4.9, 2.7 Hz, 1H), 1.22 - 1.19 (m, 1H), 0.91 (t, *J* = 4.9 Hz, 1H).

**Examples 74-75. (*S*)-3,4-Dichloro-2-(3-(4-methoxyphenyl)-6,7-dihydro-5*H*-pyrrolo[2,1-*c*][1,2,4]triazol-6-yl)phenol and (*R*)-3,4-dichloro-2-(3-(4-methoxyphenyl)-6,7-dihydro-5*H*-pyrrolo[2,1-*c*][1,2,4]triazol-6-yl)phenol**

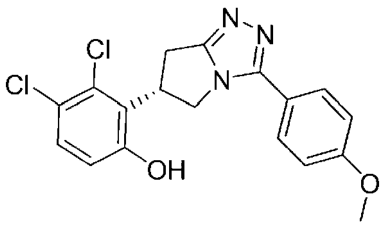
+
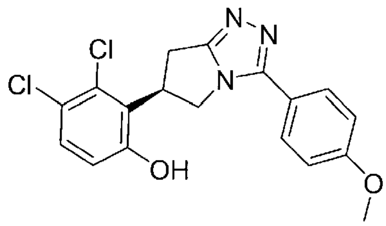

This example was prepared by reference to the preparation method for Examples 52-53, except that the 5-oxopyrrolidine-3-carbohydrazide was replaced by 4-methoxybenzohydrazide, and the (*S*)-3-(6-(allyloxy)-2,3-dichlorophenyl)-5-methoxy-3,4-dihydro-2*H*-pyrrole was replaced by 3-(6-(allyloxy)-2,3-dichlorophenyl)-5-methoxy-3,4-dihydro-2*H*-pyrrole.

The first compound obtained from the SFC resolution was designated as Example 74, with its RT of 2.49 min analyzed under the analytical SFC chromatographic conditions of SFC.

LC-MS: $[M+H]^+$: 375.8.

$^1H$ NMR (400 MHz, DMSO-$d_6$) δ 10.58 (s, 1H), 7.81 (d, *J* = 8.8 Hz, 2H), 7.40 (d, *J* = 8.8 Hz, 1H), 7.05 (d, *J* = 8.8 Hz, 2H), 6.86 (d, *J* = 8.8 Hz, 1H), 5.09 - 5.03 (m, 1H), 4.58 (t, *J* = 9.8 Hz, 1H), 4.30 (dd, *J* = 9.9, 7.4 Hz, 1H), 3.81 (s, 3H), 3.26 - 3.19 (m, 1H), 3.10 (dd, *J* = 15.9, 7.8 Hz, 1H).

The second compound obtained from the SFC resolution was designated as Example 75, with its RT of 4.62 min analyzed under the analytical chromatographic conditions of SFC.

LC-MS: $[M+H]^+$: 375.8.

$^1H$ NMR (400 MHz, DMSO-$d_6$) δ 7.81 (d, *J* = 8.9 Hz, 2H), 7.36 (d, *J* = 8.7 Hz, 1H), 7.04 (d, *J* = 8.9 Hz, 2H), 6.81 (d, *J* = 8.8 Hz, 1H), 5.09 - 5.03 (m, 1H), 4.56 (t, *J* = 9.8 Hz, 1H), 4.31 (dd, *J* = 10.1, 7.3 Hz, 1H), 3.81 (s, 3H), 3.23 - 3.18 (m, 1H), 3.14 - 3.08 (m, 1H).

**Examples 76-77. (*S*)-3,4-Dichloro-2-(3-(3-methoxyphenyl)-6,7-dihydro-5*H*-pyrrolo[2,1-*c*][1,2,4]triazol-6-yl)phenol and (*R*)-3,4-dichloro-2-(3-(3-methoxyphenyl)-6,7-dihydro-5*H*-pyrrolo[2,1-*c*][1,2,4]triazol-6-yl)phenol**

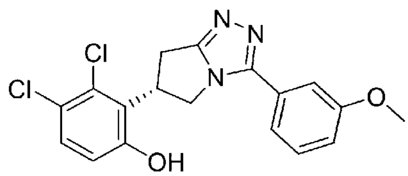
+
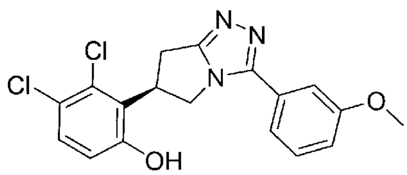

This example was prepared by reference to the preparation method for Examples 52-53, except that the 5-oxopyrrolidine-3-carbohydrazide was replaced by 3-methoxybenzohydrazide, and the (*S*)-3-(6-(allyloxy)-2,3-dichlorophenyl)-5-methoxy-3,4-dihydro-2*H*-pyrrole was replaced by 3-(6-(allyloxy)-2,3-dichlorophenyl)-5-methoxy-3,4-dihydro-2*H*-pyrrole.

The first compound obtained from the SFC resolution was designated as Example 76, with its RT of 2.67 min analyzed under the analytical chromatographic conditions of SFC.

LC-MS: $[M+H]^+$: 375.8.

$^1H$ NMR (400 MHz, DMSO-$d_6$) δ 10.92 (s, 1H), 7.45 - 7.38 (m, 3H), 7.33 (d, *J* = 8.7 Hz, 1H), 7.03 (dt, *J* = 7.1, 2.2 Hz,

1H), 6.79 (d, *J* = 8.7 Hz, 1H), 5.10 - 5.01 (m, 1H), 4.62 (t, *J* = 9.9 Hz, 1H), 4.33 (dd, *J* = 10.1, 7.1 Hz, 1H), 3.82 (s, 3H), 3.24 (dd, *J* = 15.9, 10.2 Hz, 1H), 3.12 (dd, *J* = 15.9, 7.5 Hz, 1H).

The second compound obtained from the SFC resolution was designated as Example 77, with its RT of 3.47 min analyzed under the analytical chromatographic conditions of SFC.

LC-MS: $[M+H]^+$: 375.8.

$^1H$ NMR (400 MHz, DMSO-$d_6$) δ 10.11 (s, 1H), 7.40 (dd, *J* = 12.1, 5.2 Hz, 4H), 7.03 (dt, *J* = 7.1, 2.2 Hz, 1H), 6.85 (d, *J* = 8.8 Hz, 1H), 5.15 - 4.92 (m, 1H), 4.64 (t, *J* = 9.9 Hz, 1H), 4.33 (dd, *J* = 10.2, 7.2 Hz, 1H), 3.82 (s, 3H), 3.18 (ddd, *J* = 23.4, 15.9, 8.9 Hz, 2H).

**Examples 78-79. (*S*)-3,4-Dichloro-2-(3-(1-cyclohexylpiperidin-4-yl)-6,7-dihydro-5*H*-pyrrolo[2,1-*c*][1,2,4]triazol-6-yl)phenol and (*R*)-3,4-dichloro-2-(3-(1-cyclohexylpiperidin-4-yl)-6,7-dihydro-5*H*-pyrrolo[2,1-*c*][1,2,4]triazol-6-yl)phenol**

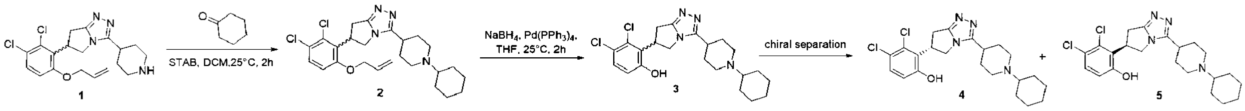


**Synthesis of 6-(6-(allyloxy)-2,3-dichlorophenyl)-3-(1-cyclohexylpiperidin-4-yl)-6,7-dihydro-5*H*-pyrrolo[2,1-*c*][1,2,4]triazole (2):**

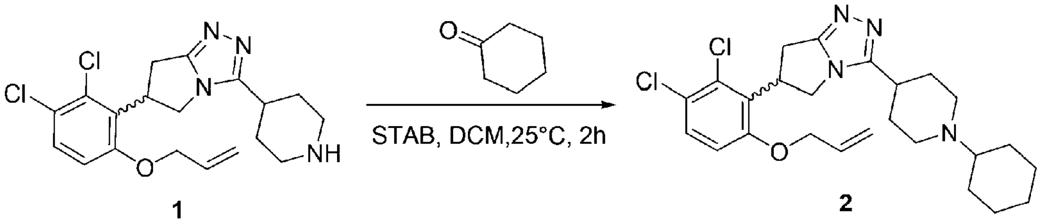


At 0 °C, sodium triacetoxyborohydride (242.5 mg, 1.14 mmol) was added to a solution of 6-(6-(allyloxy)-2,3-dichlorophenyl)-3-(piperidin-4-yl)-6,7-dihydro-5*H*-pyrrolo[2,1-*c*][1,2,4]triazole (150.0 mg, 0.38 mmol) and cyclohexanone (112.5 mg, 1.14 mmol) in dichloromethane (2.0 mL). The mixture was stirred at 25 °C for 2 h. After completion of the reaction, the reaction mixture was filtered through an organic-phase filter membrane, and the filtrate was rotary evaporated to dryness. The resulting crude product was purified by silica gel chromatography (methanol:dichloromethane = 1:20) to give a white solid (150.0 mg, yield: 83%).

LC-MS: $[M+H]^+$: 475.2.

**Synthesis of 3,4-dichloro-2-(3-(1-cyclohexylpiperidin-4-yl)-6,7-dihydro-5*H*-pyrrolo[2,1-*c*][1,2,4]triazol-6-yl)phenol (3):**

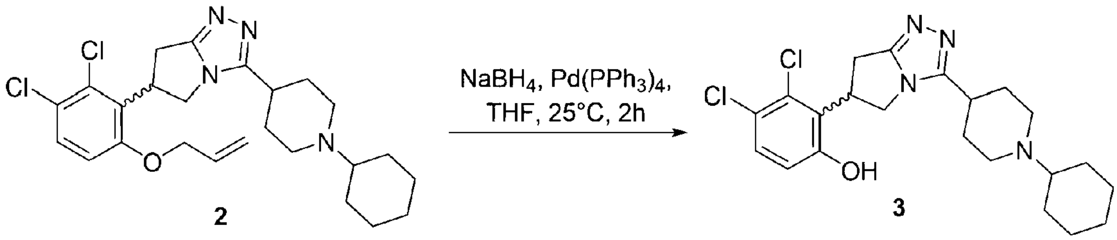


At room temperature, tetrakis(triphenylphosphine)palladium(0) (60.5 mg, 0.05 mmol) was added to a solution of 6-(6-(allyloxy)-2,3-dichlorophenyl)-3-(1-cyclohexylpiperidin-4-yl)-6,7-dihydro-5*H*-pyrrolo[2, 1-*c*][1,2,4]triazole (150.0 mg, 0.32 mmol) and sodium borohydride (18.2 mg, 0.48 mmol) in tetrahydrofuran (2.0 mL). The flask was purged using

an argon balloon, and the mixture was stirred at 25 °C for 2 h. After completion of the reaction, the reaction mixture was filtered through an organic-phase filter membrane, and the filtrate was rotary evaporated to drynes. The crude product was subjected to preparative chromatography to give a white solid (56.0 mg, yield: 41%).

LC-MS: $[M+H]^+$: 435.1.

**Preparation of (*S*)-3,4-dichloro-2-(3-(1-cyclohexylpiperidin-4-yl)-6,7-dihydro-5*H*-pyrrolo[2,1-*c*][1,2,4]triazol-6-yl)phenol and (*R*)-3,4-dichloro-2-(3-(1-cyclohexylpiperidin-4-yl)-6,7-dihydro-5*H*-pyrrolo[2,1-*c*][1,2,4]triazol-6-yl) phenol:**

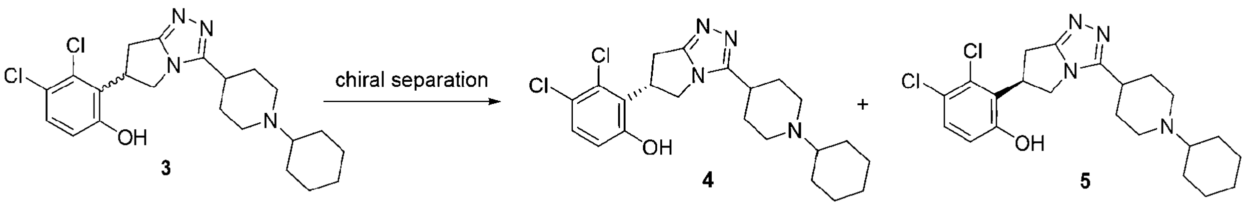

3 4 + 5

3,4-Dichloro-2-(3-(1-cyclohexylpiperidin-4-yl)-6,7-dihydro-5*H*-pyrrolo[2,1-*c*][1,2,4]triazol-6-yl)phenol (30.0 mg, 0.07 mmol) was subjected to preparative chiral chromatography (chromatographic column: CHIRALPAK IC 250 mm 20 mm, 5 μm; mobile phase: 40% EtOH ($NH_4OH$ 0.2%)) to give a pair of enantiomers.

The first compound obtained from the SFC resolution was designated as Example 78 (2.3 mg, yield: 7.6%), with its RT of 2.69 min analyzed under the analytical chromatographic conditions of SFC.

LC-MS: $[M+H]^+$: 435.1.

$^1$H NMR (400 MHz, DMSO-*d6*) δ 10.62 (s, 1H), 7.40 (d, *J* = 8.8 Hz, 1H), 6.87 (d, *J* = 8.8 Hz, 1H), 4.95-4.92 (m, 1H), 4.27 (t, *J* = 9.8 Hz, 1H), 4.05 (dd, *J* = 10.0, 7.6 Hz, 1H), 3.16-3.00 (m, 5H), 2.55-2.52 (m, 3H), 1.99-1.93 (m, 2H), 1.86-1.72 (m, 6H), 1.58 (d, *J* = 12.5 Hz, 1H), 1.33-1.24 (m, 4H), 1.07-1.05 (m, 1H).

The second compound obtained from the SFC resolution was designated as Example 79 (7.3 mg, yield: 14%), with its RT of 3.22 min analyzed under the analytical chromatographic conditions of SFC.

LC-MS: $[M+H]^+$: 435.1.

$^1$H NMR (400 MHz, DMSO-*d6*) δ 10.59 (s, 1H), 7.41 (d, *J* = 8.8 Hz, 1H), 6.88 (d, *J* = 8.8 Hz, 1H), 4.99-4.95 (m, 1H), 4.28-4.26 (m, 1H), 4.07 (dd, *J* = 10.1, 7.4 Hz, 1H), 3.49-3.45 (m, 3H), 3.19-3.07 (m, 5H), 2.17-2.10 (m, 2H), 2.00-1.99 (m, 4H), 1.83 *(d, J* = 13.0 Hz, 2H), 1.62 (d, *J* = 12.7 Hz, 1H), 1.47-1.27 (m, 4H), 1.16-1.09 (m, 1H).

**Example 80. 3,4-Dichloro-2-((*S*)-3-((*R*)-1-isopropylpyrrolidin-3-yl)-6,7-dihydro-5*H*-pyrrolo[2,1-*c*][1,2,4]triazol-6-yl)phenol**

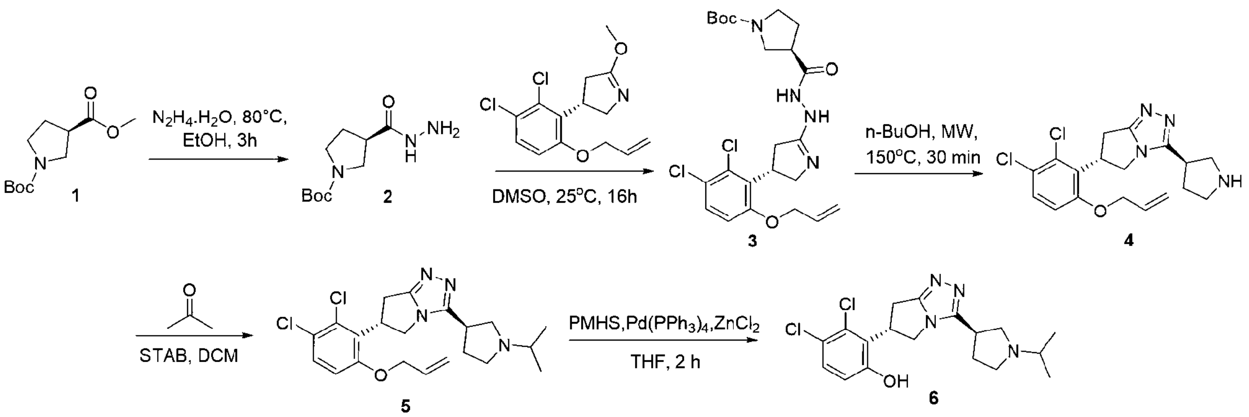

1 2 3 4 5 6

**Synthesis of *tert-butyl* (*R*)-3-(hydrazinecarbonyl)pyrrolidine-1-carboxylate (2):**

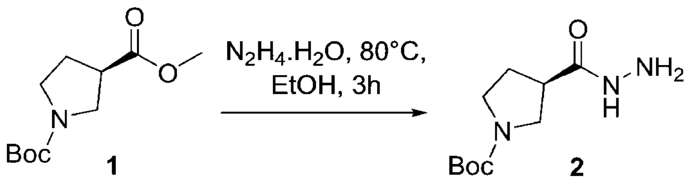


1 2

A solution of (*R*)-1-(*tert*-butyl)-3-methylpyrrolidine-1,3-dicarboxylate (5.0 g, 0.02 mol) and hydrazine hydrate (3.2 g, 0.08 mol, 80% in water) in ethanol (100.0 mL) was stirred at 80 °C for 3 h. The ethanol was then removed by distillation under reduced pressure. The resulting crude product was purified by silica gel chromatography (methanol:dichloromethane = 1:10) to give a white solid (2.2 g, yield: 44%).
LC-MS: $[M+H]^+$: 174.2.

**Synthesis of *tert*-butyl (*R*)-3-(2-((*S*)-3-(6-(allyloxy)-2,3-dichlorophenyl)-3,4-dihydro-2*H*-pyrrol-5-yl)hydrazine-1-carbonyl)pyrrolidine-1-carboxylate (3):**

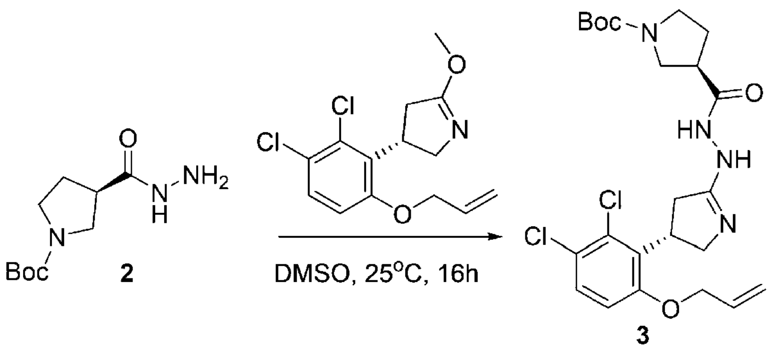


2 3

The compound (*S*)-3-(6-(allyloxy)-2,3-dichlorophenyl)-5-methoxy-3,4-dihydro-2*H*-pyrrole (800.0 mg, 2.66 mmol) and *tert*-butyl (R)-3-(hydrazinecarbonyl)pyrrolidine-1-carboxylate (1841.4 mg, 7.99 mmol) were dissolved in a dimethylsulfoxide solution (10 mL), and the mixture was allowed to react at 25 °C for 16 h. After completion of the reaction, the reaction mixture was lyophilized *in vacuo.* The resulting crude product was purified by silica gel chromatography (dichloromethane:methanol = 10:1) to give a white solid (1200.0 mg, yield: 90%).
LC-MS: $[M+H]^+$: 497.2.

**Synthesis of (*S*)-6-(6-(allyloxy)-2,3-dichlorophenyl)-3-((*R*)-pyrrolidin-3-yl)-6,7-dihydro-5*H*-pyrrolo[2,1-*c*][1,2,4]triazole (4):**

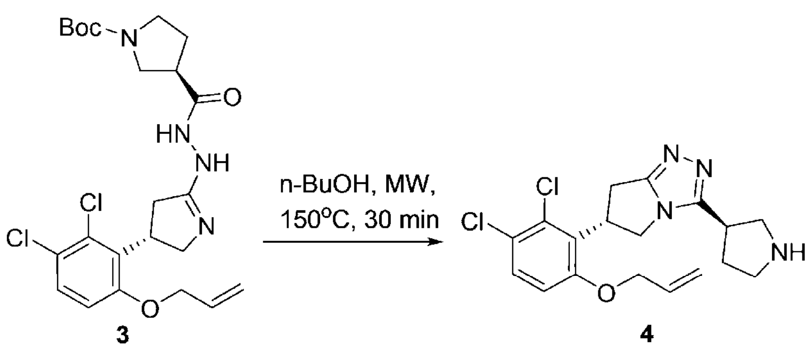


3 4

The compound *tert*-butyl (*R*)-3-(2-((*S*)-3 -(6-(allyloxy)-2,3-dichlorophenyl)-3,4-dihydro-2*H*-pyrrol-5-yl)hydrazine-1-carbonyl)pyrrolidine-1-carboxylate (600.0 mg, 1.20 mmol) was dissolved in n-butanol (6 mL), and the mixture was stirred under microwave at 150 °C for 30 min. After completion of the reaction, the reaction mixture was concentrated *in vacuo.* The resulting crude product was purified by silica gel chromatography (dichloromethane:methanol = 10:1) to give

a white solid (370.0 mg, yield: 40%).

LC-MS: $[M+H]^+$: 379.2.

**Synthesis of (*S*)-6-(6-(allyloxy)-2,3-dichlorophenyl)-3-((*R*)-1-isopropylpyrrolidin-3-yl)-6,7-dihydro-5*H*-pyrrolo[2,1-*c*][1,2,4]triazole (5):**

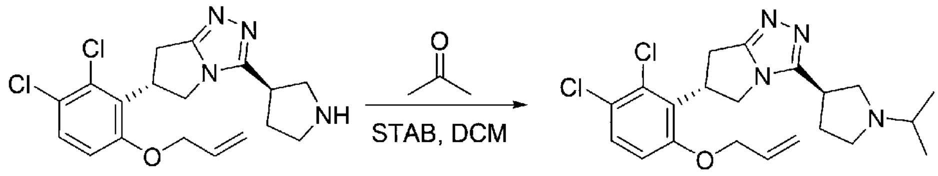


4 5

The compound (*S*)-6-(6-(allyloxy)-2,3-dichlorophenyl)-3-((*R*)-pyrrolidin-3-yl)-6,7-dihydro-5*H*-pyrrolo[2,1-*c*][1,2,4]triazole (400.0 mg, 1.05 mmol) was dissolved in a dichloromethane solution (5 mL), and acetone (1.2 g, 20.66 mmol) was added to the reaction mixture. The resulting mixture was stirred at room temperature for 2 h. Then, the reaction mixture was cooled to 0 °C, and sodium triacetoxyborohydride (447.0 mg, 2.1 mmol) was added in portions. The resulting mixture was stirred at room temperature overnight. After completion of the reaction, the reaction mixture was concentrated *in vacuo.* The resulting crude product was subjected to silica gel chromatography (dichloromethane:methanol = 10:1) to give a white solid (300.0 mg, yield: 67%).

LC-MS: $[M+H]^+$: 421.2.

**Synthesis of 3,4-dichloro-2-((*S*)-3-((*R*)-1-isopropylpyrrolidin-3-yl)-6,7-dihydro-3*H*-pyrrolo[2,1-*c*][1,2,4]triazol-6-yl)phenol (6):**

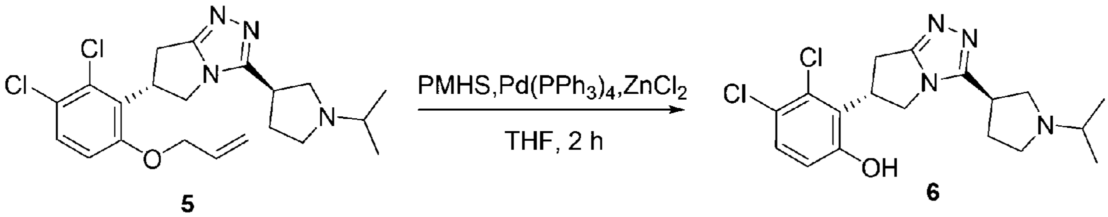


5 6

The compound (*S*)-6-(6-(allyloxy)-2,3-dichlorophenyl)-3-((*R*)-1-isopropylpyrrolidin-3-yl)-6,7-dihydro-5*H*-pyrrolo[2,1-*c*][1,2,4]triazole (140.0 mg, 0.33 mmol) was dissolved in a tetrahydrofuran solution (20 mL), and polymethylhydrosiloxane (147.8 mg, 0.66 mmol), zinc chloride (4.1 mg, 0.029 mmol), and tetrakis(triphenylphosphine)palladium(0) (15.3 mg, 0.013 mmol) were added to the reaction mixture. The resulting mixture was allowed to react at room temperature for 2 h. After completion of the reaction, the reaction mixture was concentrated *in vacuo.* The resulting crude product was subjected to silica gel chromatography (dichloromethane:methanol = 10:1) to give a white solid (19.5 mg, yield: 15%).

LC-MS: $[M+H]^+$: 381.2.

$^1$H NMR (400 MHz, DMSO-*d6*) δ 10.56 (s, 1H), 8.17 (s, 1H), 7.40 (d, *J*=8.8, 1H), 6.87 (d, *J*=8.8, 1H), 4.97 (t, *J*=8.0, 1H), 4.27 (dd, *J*=12.2, 7.7, 1H), 4.05 (dd, *J*=10.1, 7.7, 1H), 3.52 - 3.46 (m, 0H), 3.16 - 3.06 (m, 1H), 2.81 (dd, *J*=17.4, 9.8, 0H), 2.65 (dd, *J*=12.0, 5.8, 1H), 2.24 - 2.17 (m, 1H), 2.04 (dd, *J*=13.1, 7.1, 1H), 1.08 (dd, *J*=6.2, 2.7, 6H).

**Example 81. 1-((*R*)-3-((*S*)-6-(2,3-Dichloro-6-hydroxyphenyl)-6,7-dihydro-5*H*-pyrrolo[2,1-*c*][1,2,4]triazol-3-yl)pyrrolidin-1-yl)ethan-1-one**

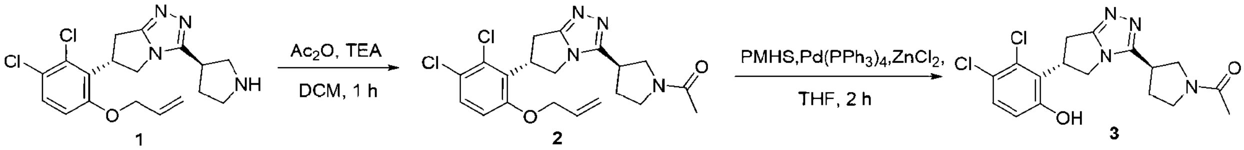


**Synthesis of 1-((*R*)-3-((*S*)-6-(6-(allyloxy)-2,3-dichlorophenyl)-6,7-dihydro-5*H*-pyrrolo[2,1-*c*][1,2,4]triazol-3-yl) pyrrolidin-1-yl)ethan-1-one (2):**

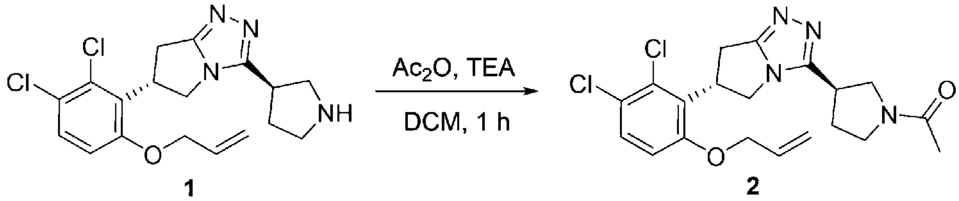


The compound (*S*)-6-(6-(allyloxy)-2,3-dichlorophenyl)-3-((*R*)-pyrrolidin-3-yl)-6,7-dihydro-5*H*-pyrrolo[2,1-*c*][1,2,4]triazole (150.0 mg, 0.39 mmol) and triethylamine (80.0 mg, 0.79 mmol) were dissolved in a dichloromethane solution (2 mL), and the reaction mixture was cooled to 0 °C. Acetic anhydride (60.5 mg, 0.59 mmol) was added dropwise to the reaction mixture, and the resulting mixture was stirred at room temperature for 1 h. After completion of the reaction, the mixture was diluted by adding water (10 mL) and extracted three times with dichloromethane (10 mL). The organic phases were combined, washed with saturated brine (20 mL), dried over anhydrous sodium sulfate, and then concentrated under reduced pressure. The resulting crude product was subjected to silica gel chromatography (dichloromethane:methanol = 10:1) to give a white solid (100.0 mg, yield: 60%).

LC-MS: $[M+H]^+$: 421.2.

**Synthesis of 1-((*R*)-3-((*S*)-6-(2,3-dichloro-6-hydroxyphenyl)-6,7-dihydro-5*H*-pyrrolo[2,1-*c*][1,2,4]triazol-3-yl)pyrrolidin-1-yl)ethan-1-one (3):**

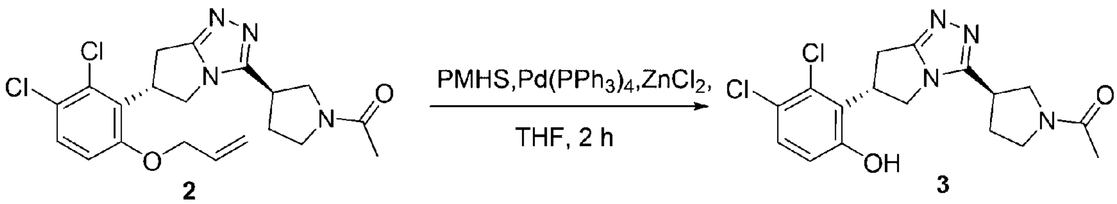


The compound 1-((*R*)-3-((*S*)-6-(6-(allyloxy)-2,3-dichlorophenyl)-6,7-dihydro-5*H*-pyrrolo[2,1-*c*][1,2,4]triazol-3-yl)pyrrolidin-1-yl)ethan-1-one (100.0 mg, 0.23 mmol) was dissolved in a tetrahydrofuran solution (3 mL), and polymethylhydrosiloxane (105.6 mg, 0.47 mmol), zinc chloride (2.9 mg, 0.021 mmol), and tetrakis(triphenylphosphine)palladium(0) (10.9 mg, 0.0094 mmol) were added to the reaction mixture. The resulting mixture was allowed to react at room temperature for 2 h. After completion of the reaction, the reaction mixture was concentrated *in vacuo,* and the crude product was subjected to preparative chromatography to give a white solid (15.0 mg, yield: 16%).

LC-MS: $[M+H]^+$: 381.1.

$^1H$ NMR (400 MHz, DMSO-*d6*) δ 10.75 (s, 1H), 7.42 (d, *J*=8.8, 1H), 6.89 (d, *J*=8.8, 1H), 5.10 - 5.00 (m, 1H), 4.52 - 4.44 (m, 1H), 4.30 - 4.22 (m, 1H), 3.95 - 3.69 (m, 2H), 3.67 - 3.60 (m, 1H), 3.56 - 3.42 (m, 2H), 3.39 - 3.32 (m, 1H), 3.28 - 3.19 (m, 1H), 2.43 - 2.29 (m, 1H), 2.23 - 2.06 (m, 1H), 1.96 (s, 3H).

**Example 82. 3,4-Dichloro-2-((*S*)-3-((*R*)-1-(1-isopropylpiperidin-4-yl)pyrrolidin-3-yl)-6,7-dihydro-5*H*-pyrrolo[2,1-*c*][1,2,4]triazol-6-yl)phenol**

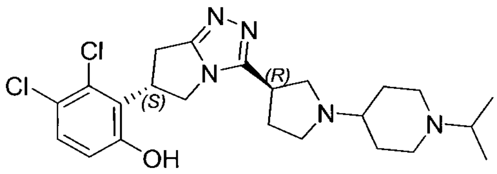

This example was prepared by reference to the preparation method for Example 80, except that the acetone was replaced by 1-isopropylpiperidin-4-one.

LCMS $[M+H]^+$: 463.9.

$^1$H NMR (400 MHz, DMSO-*d6*) δ 10.77 (s, 1H), 7.42 (d, *J* = 8.8 Hz, 1H), 6.91 (d, *J* = 8.8 Hz, 1H), 5.04 - 4.98 (m, 1H), 4.66 (s, 2H), 4.32 (t, *J* = 9.6 Hz, 1H), 4.17 - 4.11 (m, 1H), 3.80 (s, 3H), 3.52 (t, *J* = 12.5 Hz, 4H), 3.31 (s, 1H), 3.18 (d, *J* = 7.8 Hz, 2H), 3.00 (d, *J* = 8.2 Hz, 2H), 2.36 (d, *J* = 12.2 Hz, 2H), 2.21 (s, 1H), 1.93 (d, *J* = 12.4 Hz, 2H), 1.25 (d, *J* = 6.6 Hz, 6H).

**Example 83. 3,4-Dichloro-2-((*S*)-3-((*R*)-1-(tetrahydro-2*H*-pyran-4-yl)pyrrolidin-3-yl)-6,7-dihydro-5*H*-pyrrolo[2,1-*c*][1,2,4]triazol-6-yl)phenol**

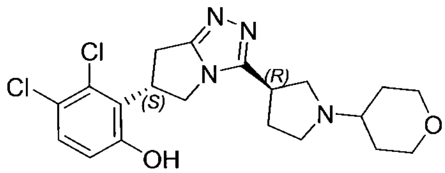

This example was prepared by reference to the preparation method for Example 80, except that the acetone was replaced by tetrahydro-4*H*-pyran-4-one.

LCMS $[M+H]^+$: 423.2.

$^1$H NMR (400 MHz, DMSO-d6) δ 10.57 (s, 1H), 7.39 (d, *J* = 8.8 Hz, 1H), 6.85 (d, *J* = 8.8 Hz, 1H), 5.02 - 4.92 (m, 1H), 4.30 - 4.22 (m, 1H), 4.10 - 4.00 (m, 1H), 3.82 (d, *J* = 11.5 Hz, 2H), 3.46 - 3.40 (m, 1H), 3.27 (d, *J* = 11.5 Hz, 2H), 3.15 - 2.96 (m, 3H), 2.69 - 2.65 (m, 1H), 2.63 - 2.55 (m, 2H), 2.21 (ddd, *J*= 22.8, 11.2, 4.6 Hz, 2H), 2.04 - 1.97 (m, 1H), 1.75 (d, *J* = 11.8 Hz, 2H), 1.37 (d, *J* = 12.7 Hz, 2H).

**Example 84. (*S*)-3,4-Dichloro-2-(3-(2-methylpyridin-4-yl)-6,7-dihydro-5*H*-pyrrolo[2,1-*c*][1,2,4]triazol-6-yl)phenol**

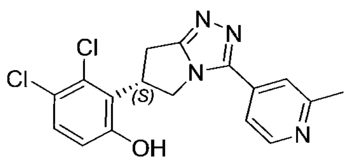

This example was prepared by reference to the preparation method for Example 32, except that the methyl 3-hydroxypropanoate was replaced by methyl 2-methylisonicotinate.

LCMS $[M+H]^+$: 361.1.

$^1$H NMR (400 MHz, DMSO-*d6*) δ 10.84 (s, 1H), 8.55 (d, *J*=5.2, 1H), 7.70 - 7.65 (m, 2H), 7.39 (d, *J*=8.8, 1H), 6.85 (d, *J*=8.8, 1H), 5.09 (s, 1H), 4.69 (t, *J*=10.0, 1H), 4.46 - 4.40 (m, 1H), 3.28 (d, *J*=5.9, 1H), 3.18 (d, *J*=7.5, 1H), 2.53 (s, 3H).

**Example 85. (*S*)-3,4-Dichloro-2-(3-(1,1-difluoroethyl)-6,7-dihydro-5*H*-pyrrolo[2,1-*c*][1,2,4]triazol-6-yl)phenol**

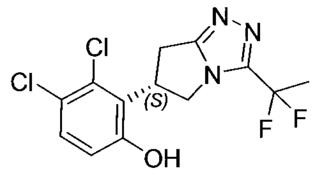

This example was prepared by reference to the preparation method for Example 32, except that the methyl 3-hydroxypropanoate was replaced by methyl 2,2-difluoropropanoate.

LCMS $[M+H]^+$: 333.8.

$^1$H NMR (400 MHz, DMSO-d6) δ 10.67 (s, 1H), 7.39 (d, *J* = 8.8 Hz, 1H), 6.83 (d, *J* = 8.8 Hz, 1H), 5.14 - 4.99 (m, 1H), 4.42 (t, *J* = 10.2 Hz, 1H), 4.16 (dd, *J* = 10.7, 6.9 Hz, 1H), 3.29 - 3.21 (m, 1H), 3.04 (dd, *J* = 16.1, 7.0 Hz, 1H), 2.12 (t, *J*= 19.2 Hz, 3H).

**Example 86. (*S*)-3,4-Dichloro-2-(3-(trifluoromethyl)-6,7-dihydro-5*H*-pyrrolo[2,1-*c*][1,2,4]triazol-6-yl)phenol**

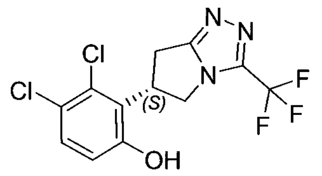

This example was prepared by reference to the preparation method for Example 32, except that the 3-hydroxypropanehydrazide was replaced by 2,2,2-trifluoroacetohydrazide.

LCMS $[M+H]^+$: 338.0.

$^1$H NMR (400 MHz, DMSO-d6) δ 10.62 (s, 1H), 7.41 (d, *J* = 8.8 Hz, 1H), 6.84 (d, *J* = 8.8 Hz, 1H), 5.09 (tt, *J* = 10.0, 6.6 Hz, 1H), 4.51 (t, *J* = 10.3 Hz, 1H), 4.20 (dd, *J* = 10.8, 6.5 Hz, 1H), 3.38 (d, *J* = 10.4 Hz, 1H), 3.08 (dd, *J* = 16.4, 6.6 Hz, 1H).

**Example 87. (*S*)-3,4-Dichloro-2-(3-(2,2,2-trifluoroethyl)-6,7-dihydro-5*H*-pyrrolo[2,1-*c*][1,2,4]triazol-6-yl)phenol**

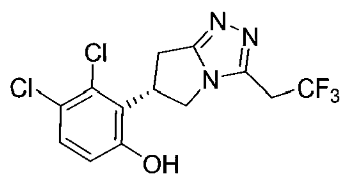

This example was prepared by reference to the preparation method for Example 32, except that the methyl 3-hydroxypropanoate was replaced by ethyl 3,3,3-trifluoropropanoate.

LCMS $[M+H]^+$: 352.1.

$^1$H NMR (400 MHz, DMSO-*d6*) δ 10.59 (s, 1H), 7.41 (d, *J*=8.8, 1H), 6.86 (d, *J*=8.8, 1H), 4.99 (t, *J*=8.7, 1H), 4.30 (t, *J*=10.0, 1H), 4.09 (dd, *J*=10.5, 7.5, 1H), 4.01 (dd, *J*=11.1, 3.5, 2H), 3.25 - 3.18 (m, 1H), 3.09 (dd, *J*=15.9, 7.7, 1H).

**Example 88. 3,4-Dichloro-2-(3-(2,2,6,6-tetramethylpiperidin-4-yl)-6,7-dihydro-5*H*-pyrrolo[2,1-*c*][1,2,4]triazol-6-yl)phenol**

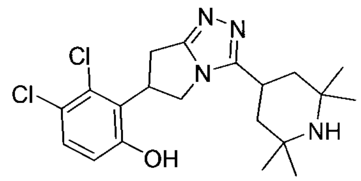

This example was prepared by reference to the preparation method for Example 3, except that the ethyl 3-hydroxy-3-methylbutanoate was replaced by ethyl 2,2,6,6-tetramethylpiperidine-4-carboxylate.

LCMS $[M+H]^+$: 408.9.

$^1$H NMR (400 MHz, DMSO-$d_6$) δ 8.34 (s, 1H), 7.39 (d, *J* = 8.8 Hz, 1H), 6.89 (d, *J* = 8.8 Hz, 1H), 5.02 - 4.90 (m, 1H), 4.31 (t, *J* = 9.8 Hz, 1H), 4.16 - 4.05 (m, 1H), 3.36 (t, *J* = 12.6 Hz, 1H), 3.11 (d, *J* = 9.1 Hz, 2H), 1.89 (dd, *J* = 23.0, 13.1 Hz, 2H), 1.53 (t, *J* = 13.1 Hz, 2H), 1.32 (s, 6H), 1.24 (s, 6H).

**Example 89. 3,4-Dichloro-2-(3-(2,2-dimethylpiperidin-4-yl)-6,7-dihydro-5*H*-pyrrolo[2,1-*c*][1,2,4]triazol-6-yl)phenol**

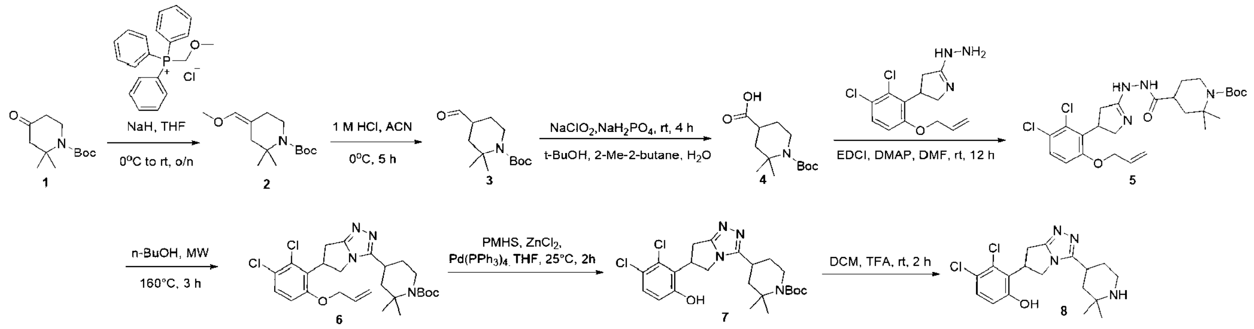


**Synthesis of *tert*-butyl (Z)-4-(methoxymethylene)-2,2-dimethylpiperidine-1-carboxylate (2):**

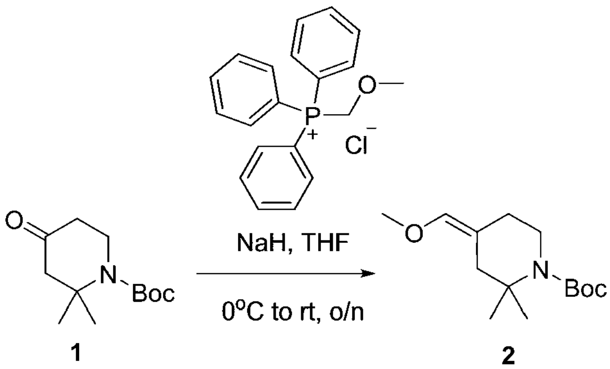


A solution of (methoxymethyl)triphenylphosphonium chloride (5.6 g, 16.50 mmol) in tetrahydrofuran (50 mL) was cooled to 0 °C, and sodium hydride (5.8 g, 145 mmol, 60% in mineral oil) was added in portions. The mixture was stirred at 0 °C for 1 h. A solution of *tert*-butyl 2,2-dimethyl-4-oxopiperidine-1-carboxylate (2.5 g, 11.00 mmol) in tetrahydrofuran (10 mL) was slowly added, and the resulting reaction mixture was stirred at room temperature overnight. After completion of the reaction, the mixture was diluted by adding water (100 mL) and extracted three times with ethyl acetate (100 mL). The organic phases were combined, washed with saturated brine (300 mL), dried over anhydrous sodium sulfate, and then concentrated under reduced pressure. The residue was purified by silica gel column chromatography and eluted (petroleum ether:ethyl acetate = 10:1) to give a colorless oily product (1 g, yield: 35%).

**Synthesis of *tert*-butyl 4-formyl-2,2-dimethylpiperidine-1-carboxylate (3):**

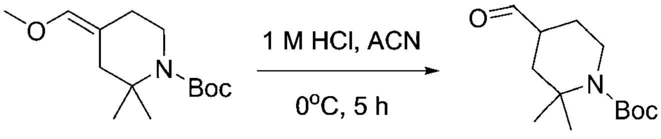


2 3

A solution of *tert*-butyl (*Z*)-4-(methoxymethylene)-2,2-dimethylpiperidine-1-carboxylate (1.2 g, 4.90 mmol) in acetonitrile (115 mL) was cooled to 0 °C, and a 1 M aqueous HCl solution (5.75 mL) was added. The mixture was stirred at 0 °C for 5 h. After completion of the reaction, the reaction mixture was diluted by adding a saturated aqueous sodium bicarbonate solution (20 mL) and extracted three times with ethyl acetate (20 mL). The organic phases were combined, washed with saturated brine (20 mL), dried over anhydrous sodium sulfate, and then concentrated under reduced pressure to give the target product (0.5 g, yield: 42%).

**Synthesis of 1-(*tert*-butoxycarbonyl)-2,2-dimethylpiperidine-4-carboxylic acid (4):**

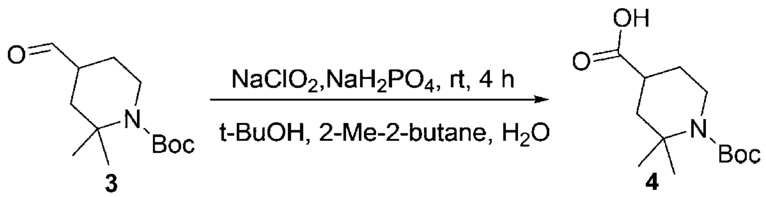


3 4

*tert*-Butyl 4-formyl-2,2-dimethylpiperidine-1-carboxylate (900.0 mg, 3.71 mmol) was dissolved in tert-butanol (5.4 mL) and 2-methyl-2-butene (10.8 mL), and the mixture was cooled to 0 °C. An aqueous solution (6 mL) of sodium chlorite (3.3 g, 37.1 mmol) and sodium dihydrogen phosphate (3.6 g, 30 mmol) was added dropwise, and the resulting mixture was stirred at room temperature for 4 h. After completion of the reaction, the reaction mixture was diluted by adding water (20 mL) and extracted three times with ethyl acetate (20 mL). The organic phases were combined, washed with saturated brine (20 mL), dried over anhydrous sodium sulfate, and then concentrated under reduced pressure to give a white solid product (500.0 mg, yield: 52%).

**Synthesis of *tert*-butyl 4-(2-(3-(6-(allyloxy)-2,3-dichlorophenyl)-3,4-dihydro-2*H*-pyrrol-5-yl)hydrazine-1-carbonyl)-2,2-dimethylpiperidine-1-carboxylate (5):**

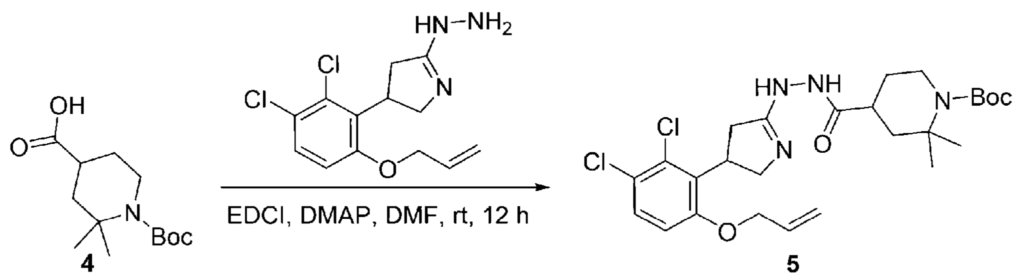


4 5

1-(*tert*-Butoxycarbonyl)-2,2-dimethylpiperidine-4-carboxylic acid (146.2 mg, 0.56 mmol), 3-(6-(allyloxy)-2,3-dichlorophenyl)-5-hydrazinyl-3,4-dihydro-2*H*-pyrrole (170.0 mg, 0.56 mmol), 1-(3-dimethylaminopropyl)-3-ethylcarbodiimide hydrochloride (151.9 mg, 0.79 mmol), and 4-dimethylaminopyridine (6.92 mg, 0.056 mmol) were dissolved in *N,N*-dimethylformamide (3.0 mL). The mixture was stirred at room temperature for 12 h. After completion of the reaction, the reaction mixture was diluted by adding water (10 mL) and extracted three times with ethyl acetate (10 mL). The organic phases were combined, washed with saturated brine (20 mL), dried over anhydrous sodium sulfate, and then concentrated under reduced pressure to give a white solid product (30.0 mg, yield: 9%).

LC-MS: $[M+H]^+$: 539.3.

**Synthesis of *tert*-butyl 4-(6-(6-(allyloxy)-2,3-dichlorophenyl)-6,7-dihydro-5*H*-pyrrolo[2,1-*c*][1,2,4]triazol-3-yl)-2,2-dimethylpiperidine-1-carboxylate (6):**

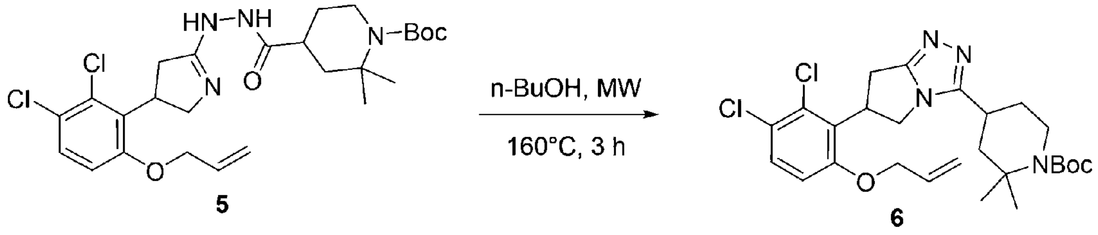


*tert*-Butyl 4-(2-(3-(6-(allyloxy)-2,3-dichlorophenyl)-3,4-dihydro-2*H*-pyrrol-5-yl)hydrazine-1-carbonyl)-2,2-dimethylpiperidine-1-carboxylate (90.0 mg, 0.16 mmol) was transferred to a microwave tube, and *n*-butanol (3.0 mL) was added. The mixture was stirred under microwave at 160 °C for 3 h. After completion of the reaction, the *n*-butanol was removed by distillation under reduced pressure. The resulting crude product was purified by silica gel chromatography (methanol:dichloromethane = 1:20) to give a white solid (30.0 mg, yield: 34%).

LC-MS: $[M+H]^+$: 521.3.

**Synthesis of *tert-butyl* 4-(6-(2,3-dichloro-6-hydroxyphenyl)-6,7-dihydro-5*H*-pyrrolo[2,1-*c*][1,2,4]triazo1-3-yl)-2,2-dimethylpiperidine-1-carboxylate (7):**

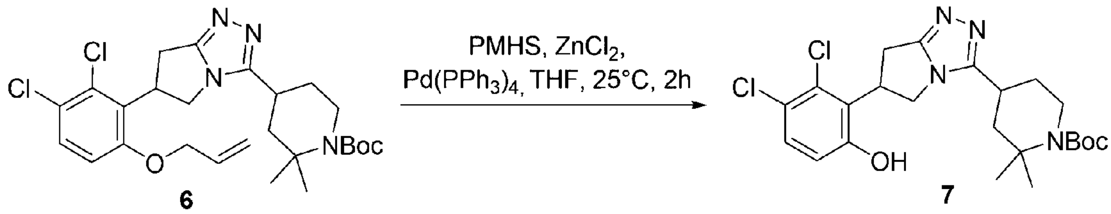


The compound *tert*-butyl 4-(6-(6-(allyloxy)-2,3-dichlorophenyl)-6,7-dihydro-5*H*-pyrrolo[2,1-*c*][1,2,4]triazol-3-yl)-2,2-dimethylpiperidine-1-carboxylate (30.0 mg, 0.057 mmol) was dissolved in a tetrahydrofuran solution (2 mL), and polymethylhydrosiloxane (25.5 mg, 0.11 mmol), zinc chloride (0.7 mg, 0.0051 mmol), and tetrakis(triphenylphosphine)palladium(0) (2.6 mg, 0.0023 mmol) were added to the reaction mixture. The resulting mixture was allowed to react at 25 °C for 2 h. After completion of the reaction, the reaction mixture was concentrated *in vacuo.* The resulting crude product was subjected to silica gel chromatography (dichloromethane:methanol = 10:1) to give a white solid (10.0 mg, yield: 36%).

LC-MS: $[M+H]^+$: 481.3.

**Synthesis of 3,4-dichloro-2-(3-(2,2-dimethylpiperidin-4-yl)-6,7-dihydro-5*H*-pyrrolo[2,1-*c*][1,2,4]triazol-6-yl)phenol (8):**

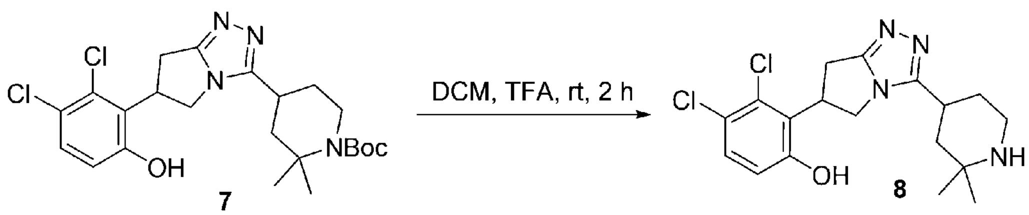


A solution of the compound *tert*-butyl 4-(6-(2,3-dichloro-6-hydroxyphenyl)-6,7-dihydro-5*H*-pyrrolo[2,1-*c*][1,2,4]triazol-3-yl)-2,2-dimethylpiperidine-1-carboxylate (10.0 mg, 0.02 mmol) in dichloromethane/trifluoroacetic acid (3 mL/1 mL) was stirred at room temperature for 2 h. After completion of the reaction, the reaction mixture was concentrated *in*

*vacuo,* and the crude product was subjected to preparative chromatography to give a white solid (3.9 mg, yield: 49%).
LC-MS: $[M+H]^+$: 381.2.
$^1H$ NMR (400 MHz, DMSO-d6) δ 8.27 (s, 1H), 7.40 (d, *J*=8.8, 1H), 6.88 (d, *J*=9.0, 1H), 5.01 - 4.88 (m, 1H), 4.29 (q, *J*=9.6, 1H), 4.07 (dd, *J*=17.9, 8.2, 1H), 3.25 - 3.16 (m, 2H), 3.14 - 3.03 (m, 3H), 2.00 (dd, *J*=30.6, 15.0, 2H), 1.72 (dd, *J*=24.5, 12.5, 2H), 1.31 (d, *J*=8.1, 6H), 1.23 (s, 1H).

**Examples 90-91. (*S*)-3,4-Dichloro-2-(3-cyclopentyl-6,7-dihydro-5*H*-pyrrolo[1,2-a]imidazol-6-yl)phenol and (*R*)-3,4-dichloro-2-(3-cyclopentyl-6,7-dihydro-5*H*-pyrrolo[1,2-*a*]imidazol-6-yl)phenol**

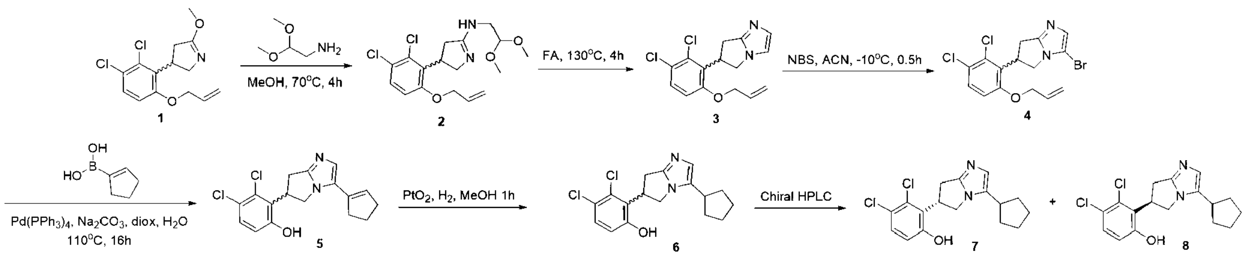


**Synthesis of 3-(6-(allyloxy)-2,3-dichlorophenyl)-*N*-(2,2-dimethoxyethyl)-3,4-dihydro-2*H*-pyrrol-5-amine (2):**

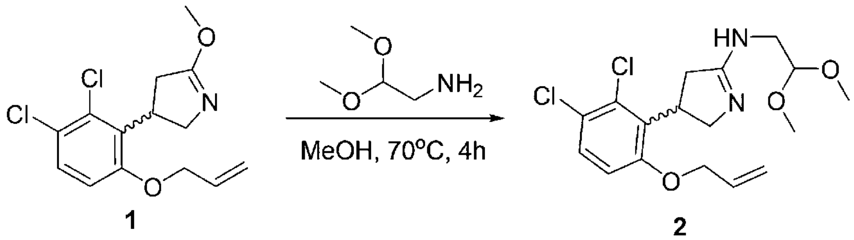


At room temperature, 3-(6-(allyloxy)-2,3-dichlorophenyl)-5-methoxy-3,4-dihydro-2*H*-pyrrole (10.0 g, 33.33 mmol) and 2,2-dimethoxyethan-1-amine (4.2 g, 39.92 mmol) were added to methanol (100.0 mL). The resulting solution was stirred at 70 °C for 4 h. After completion of the reaction, the reaction mixture was concentrated under reduced pressure, and the resulting crude product was directly used in the next step without purification.
LC-MS: $[M+H]^+$: 373.0.

**Synthesis of 6-(6-(allyloxy)-2,3-dichlorophenyl)-6,7-dihydro-5*H*-pyrrolo[1,2-*a*]imidazole (3):**

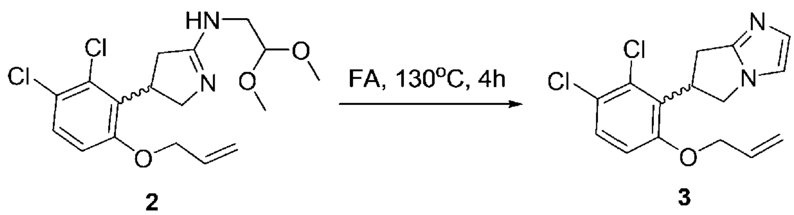


At room temperature, the crude product resulting from the previous step was dissolved in formic acid (100 mL). Then, the resulting mixture was stirred at 130 °C for 4 h. After completion of the reaction, the formic acid was removed by concentration under reduced pressure, and the resulting residue was diluted with a saturated aqueous sodium bicarbonate solution (200.0 mL) and extracted 3 times with ethyl acetate (500.0 mL). The organic phases were combined, washed with saturated sodium chloride solution (200.0 mL), dried over anhydrous sodium sulfate, and then concentrated under reduced pressure. The residue was separated by silica gel column chromatography (dichloromethane:methanol = 20:1) to give the target product (8.1 g, 66%). LC-MS: $[M+H]^+$: 308.9.

**Synthesis of 6-(6-(allyloxy)-2,3-dichlorophenyl)-3-bromo-6,7-dihydro-5*H*-pyrrolo[1,2-*a*]imidazole (4):**

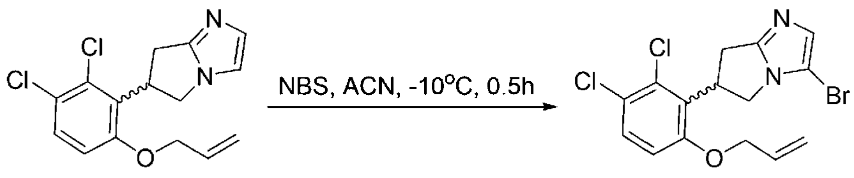


3 4

At -10 °C, 6-(6-(allyloxy)-2,3-dichlorophenyl)-6,7-dihydro-5*H*-pyrrolo[1,2-*a*]imidazole (6.5 g, 0.021 mol) was dissolved in acetonitrile (65 mL), and then *N*-bromosuccinimide (3.4 g, 0.019 mol) was added in portions. Then, the mixture was stirred for 30 min with the temperature maintained. After completion of the reaction, the reaction mixture was quenched with a sodium thiosulfate solution (100 mL) and extracted twice with ethyl acetate (200 mL). The organic phases were combined, washed with saturated brine (100 mL), dried over anhydrous sodium sulfate, and then concentrated under reduced pressure. The residue was purified by silica gel column chromatography and eluted (dichloromethane:methanol = 15:1) to give a pale yellow solid (2.4 g, yield: 25%).

LC-MS: $[M+H]^+$: 386.7.

**Synthesis of 3,4-dichloro-2-(3-(cyclopent-1-en-1-yl)-6,7-dihydro-5*H*-pyrrolo[1,2-*a*]imidazol-6-yl)phenol (5):**

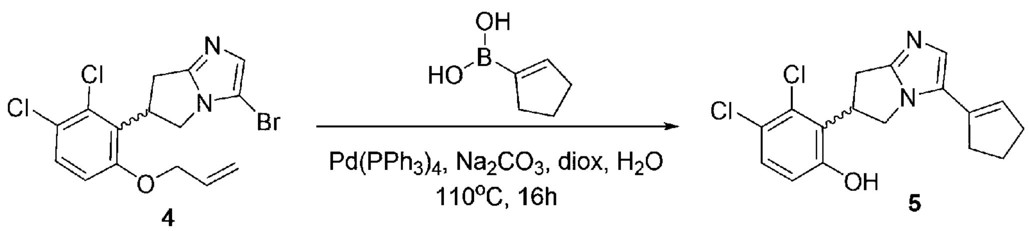


4 5

At room temperature, 6-(6-(allyloxy)-2,3-dichlorophenyl)-3-bromo-6,7-dihydro-5*H*-pyrrolo[1,2-*a*]imidazole (500.0 mg, 1.29 mmol), cyclopent-1-en-1-ylboronic acid (360.1 mg, 3.22 mmol), sodium carbonate (273.1 mg, 2.58 mmol), and tetrakis(triphenylphosphine)palladium(0) (297.7 mg, 0.26 mol) were added to a reaction flask containing dioxane and water (5 mL, 1 mL). The mixture was stirred at 110 °C for 16 h. After completion of the reaction, the reaction mixture was diluted by adding water (50 mL) and extracted twice with ethyl acetate (50 mL). The organic phases were combined, washed with saturated brine (50 mL), dried over anhydrous sodium sulfate, and then concentrated under reduced pressure. The residue was purified by silica gel column chromatography and eluted (dichloromethane:methanol = 10:1) to give an off-white solid (150 mg, yield: 33%).

LC-MS: $[M+H]^+$: 335.0.

**Synthesis of 3,4-dichloro-2-(3-cyclopentyl-6,7-dihydro-5*H*-pyrrolo[1,2-*a*]imidazol-6-yl)phenol (6):**

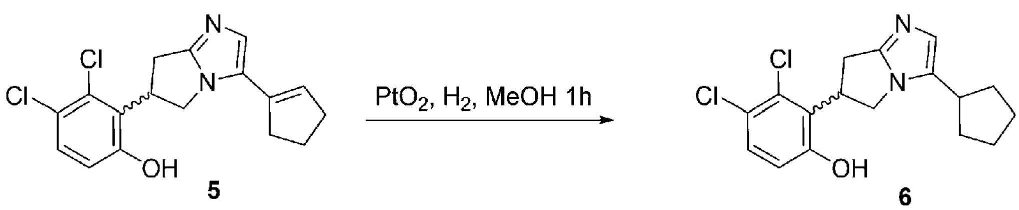


5 6

3,4-Dichloro-2-(3-(cyclopent-1-en-1-yl)-6,7-dihydro-5*H*-pyrrolo[1,2-*a*]imidazol-6-yl)phenol (150.0 mg, 0.45 mol) and platinum dioxide (75.0 mg) were added to methanol (10 mL), and the mixture was stirred at 25 °C for 1 h under a hydrogen atmosphere. After completion of the reaction, the reaction mixture was filtered, and the filtrate was

concentrated under reduced pressure. The residue was subjected to preparative chromatography to give the target product (50 mg, yield: 98%).

LC-MS: $[M+H]^+$: 336.9.

**Preparation of (*S*)-3,4-dichloro-2-(3-cyclopentyl-6,7-dihydro-5*H*-pyrrolo[1,2-*a*]imidazol-6-yl)phenol and (*R*)-3,4-dichloro-2-(3-cyclopentyl-6,7-dihydro-5*H*-pyrrolo[1,2-*a*]imidazol-6-yl)phenol:**

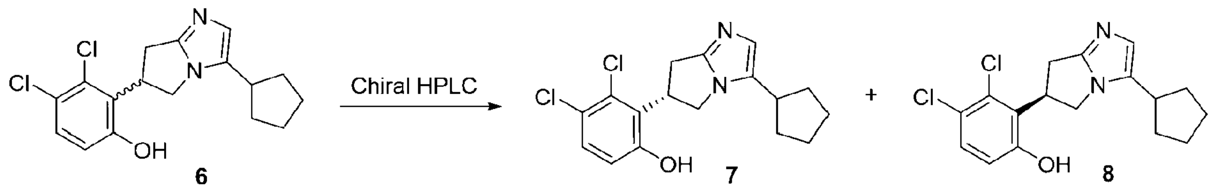


6 7 8

3,4-Dichloro-2-(3-cyclopentyl-6,7-dihydro-5*H*-pyrrolo[1,2-*a*]imidazol-6-yl)phenol (50.0 mg, 0.14 mmol) was subjected to preparative chiral chromatography (chromatographic column: CHIRALPAK IC 250 mm 20 mm, 5 μm; mobile phase: 40% EtOH ($NH_4OH$ 0.2%)) to give a pair of enantiomers.

The first compound obtained from the SFC resolution was designated as Example 90 (10.7 mg, yield: 21.4%), with its RT of 3.26 min analyzed under the analytical chromatographic conditions of SFC.

LC-MS: $[M+H]^+$: 336.9.

$^1H$ NMR (400 MHz, DMSO-d6) δ 10.49 (s, 1H), 7.39 (d, *J* = 8.8 Hz, 1H), 6.86 (d, *J* = 8.9 Hz, 1H), 6.68 (s, 1H), 4.87 - 4.78 (m, 1H), 4.21 (t, *J* = 9.8 Hz, 1H), 4.08 - 4.02 (m, 1H), 3.03 (d, *J* = 8.7 Hz, 2H), 2.99 - 2.94 (m, 1H), 1.95 (s, 2H), 1.69 (s, 2H), 1.58 (dd, *J* = 11.5, 6.8 Hz, 2H), 1.51 (d, *J* = 10.8 Hz, 2H).

The second compound obtained from the SFC resolution was designated as Example 91 (11.6 mg, yield: 23.2%), with its RT of 6.36 min analyzed under the analytical chromatographic conditions of SFC.

LC-MS: $[M+H]^+$: 336.9.

$^1H$ NMR (400 MHz, DMSO-d6) δ 10.48 (s, 1H), 7.38 (d, *J* = 8.8 Hz, 1H), 6.86 (d, *J* = 8.8 Hz, 1H), 6.58 (s, 1H), 4.85 - 4.75 (m, 1H), 4.16 (t, *J* = 9.6 Hz, 1H), 4.08 - 3.99 (m, 1H), 2.96 (dd, *J* = 15.2, 7.6 Hz, 3H), 1.95 (d, *J* = 7.4 Hz, 2H), 1.58 (ddd, *J* = 34.9, 30.8, 17.5 Hz, 6H).

**Examples 92-93. (*S*)-3,4-Dichloro-2-(3-ethyl-6,7-dihydro-5*H*-pyrrolo[1,2-*a*]imidazol-6-yl)phenol and (*R*)-3,4-dichloro-2-(3-ethyl-6,7-dihydro-5*H*-pyrrolo[1,2-*a*]imidazol-6-yl)phenol**

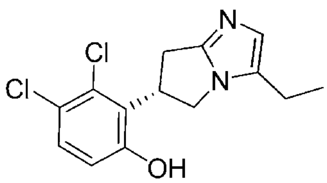

+

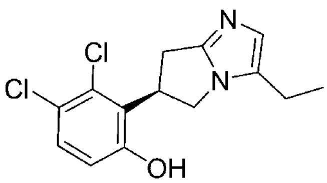

This example was prepared by reference to the preparation method for Examples 90-91, except that the cyclopent-1-en-1-ylboronic acid was replaced by 4,4,5,5-tetramethyl-2-vinyl-1,3,2-dioxaborolane.

The first compound obtained from the SFC resolution was designated as Example 92, with its RT of 2.92 min analyzed under the analytical chromatographic conditions of SFC.

LC-MS: $[M+H]^+$: 296.8.

$^1H$ NMR (400 MHz, DMSO-$d_6$) δ 10.50 (s, 1H), 7.38 (d, *J* = 8.8 Hz, 1H), 6.85 (d, *J* = 8.8 Hz, 1H), 6.57 (s, 1H), 4.79 (p, *J* = 8.8 Hz, 1H), 4.13 (t, *J* = 9.6 Hz, 1H), 4.06 - 3.98 (m, 1H), 3.07 - 2.91 (m, 2H), 2.53 (d, *J* = 7.6 Hz, 2H), 1.14 (t, *J* = 7.5 Hz, 3H).

The second compound obtained from the SFC resolution was designated as Example 93, with its RT of 4.07 min analyzed under the analytical chromatographic conditions of SFC.

LC-MS: $[M+H]^+$: 296.8.

$^1H$ NMR (400 MHz, DMSO-$d_6$) δ 10.61 (dd, *J* = 51.5, 28.6 Hz, 1H), 7.36 (d, *J* = 8.8 Hz, 1H), 6.83 (d, *J* = 8.8 Hz, 1H), 6.57 (s, 1H), 4.79 (t, *J* = 9.0 Hz, 1H), 4.12 (t, *J* = 9.6 Hz, 1H), 4.06 - 4.00 (m, 1H), 3.00 (ddd, *J* = 25.1, 15.3, 9.1 Hz, 2H), 2.52 (s, 2H), 1.14 (t, *J* = 7.5 Hz, 3H).

**Examples 94-95. (*S*)-3,4-Dichloro-2-(3-(1-isopropylpiperidin-4-yl)-6,7-dihydro-5*H*-pyrrolo[1,2-*a*]imidazol-6-yl) phenol and (*R*)-3,4-dichloro-2-(3-(1-isopropylpiperidin-4-yl)-6,7-dihydro-5*H*-pyrrolo[1,2-*a*]imidazol-6-yl)phenol**

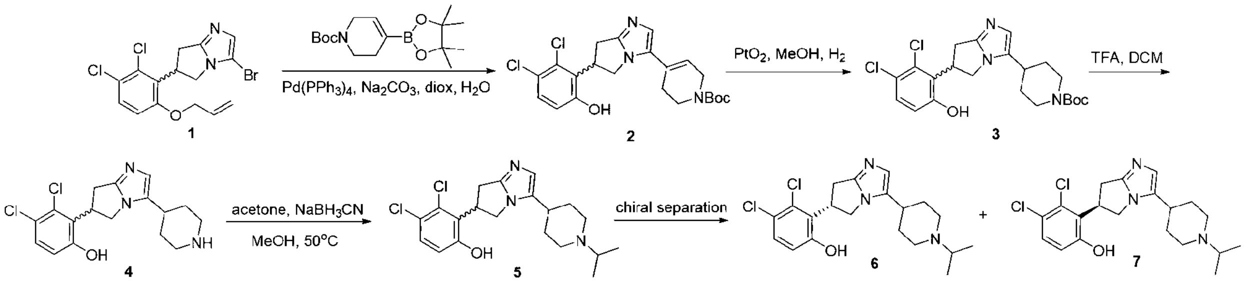


**Synthesis of *tert*-butyl 4-(6-(2,3-dichloro-6-hydroxyphenyl)-6,7-dihydro-5*H*-pyrrolo[1,2-*a*]imidazol-3-yl)-3,6-dihydropyridine-1(2*H*)-carboxylate (2):**

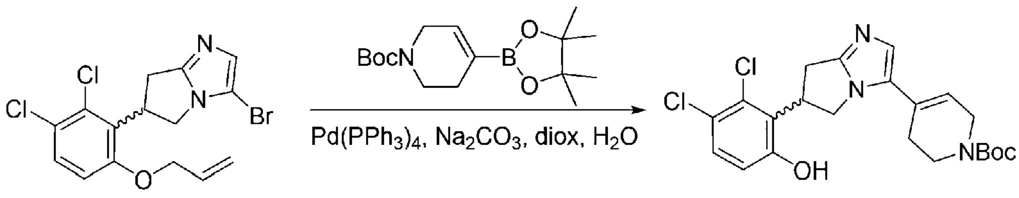


1 2

At room temperature, 6-(6-(allyloxy)-2,3-dichlorophenyl)-3-bromo-6,7-dihydro-5*H*-pyrrolo[1,2-*a*]imidazole (500.0 mg, 1.29 mmol), *tert*-butyl 4-(4,4,5,5-tetramethyl-1,3,2-dioxaborolan-2-yl)-3,6-dihydropyridine-1(2*H*)-carboxylate (799.4 mg, 2.58 mmol), sodium carbonate (409.7 mg, 3.87 mmol), and tetrakis(triphenylphosphine)palladium(0) (297.7 mg, 0.26 mol) were added to a reaction flask containing dioxane and water (5 mL, 1 mL). The mixture was stirred at 110 °C for 16 h. After completion of the reaction, the reaction mixture was diluted by adding water (50 mL) and extracted twice with ethyl acetate (50 mL). The organic phases were combined, washed with saturated brine (100 mL), dried over anhydrous sodium sulfate, and then concentrated under reduced pressure. The residue was purified by silica gel column chromatography (dichloromethane:methanol = 10:1) to give a white solid (210 mg, yield: 28%).

LC-MS: $[M+H]^+$: 449.8.

**Synthesis of *tert*-butyl 4-(6-(2,3-dichloro-6-hydroxyphenyl)-6,7-dihydro-5*H*-pyrrolo[1,2-*a*]imidazol-3-yl)piperidine-1-carboxylate (3):**

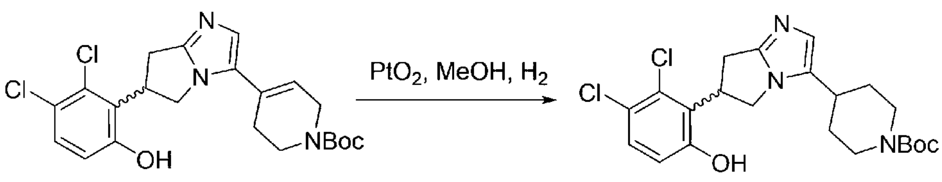


2 3

*tert-Butyl* 4-(6-(2,3-dichloro-6-hydroxyphenyl)-6,7-dihydro-5*H*-pyrrolo[1,2-*a*]imidazol-3-yl)-3,6-dihydropyridine-1(2*H*)-carboxylate (300.0 mg, 0.66 mmol) and platinum dioxide (75.0 mg) were added to methanol (10 mL), and the mixture was stirred at 25 °C for 2 h under a hydrogen atmosphere. After completion of the reaction, the reaction mixture was filtered, and the filtrate was concentrated under reduced pressure to give a white solid (230 mg, yield: 64%).

LC-MS: $[M+H]^+$: 451.9.

**Synthesis of 3,4-dichloro-2-(3-(piperidin-4-yl)-6,7-dihydro-5*H*-pyrrolo[1,2-*a*]imidazol-6-yl)phenol (4):**

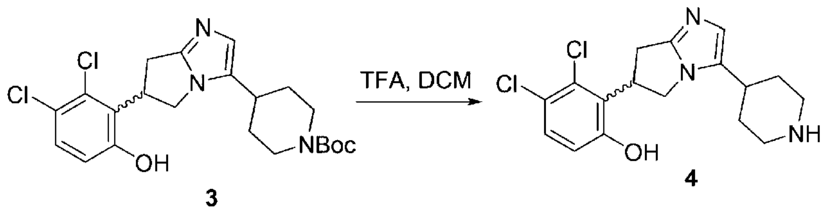


The compound *tert*-butyl 4-(6-(2,3-dichloro-6-hydroxyphenyl)-6,7-dihydro-5*H*-pyrrolo[1,2-*a*]imidazol-3-yl)piperidine-1-carboxylate (260.0 mg, 0.57 mmol) was dissolved in a solution of dichloromethane:trifluoroacetic acid (3:1, 4.0 mL), and the mixture was stirred at room temperature for 1 h. The reaction mixture was evaporated to dryness to give a white solid (230.0 mg, yield: 95%).

LC-MS: $[M+H]^+$: 351.9.

**Synthesis of 3,4-dichloro-2-(3-(1-isopropylpiperidin-4-yl)-6,7-dihydro-5*H*-pyrrolo[1,2-*a*]imidazol-6-yl)phenol (5):**

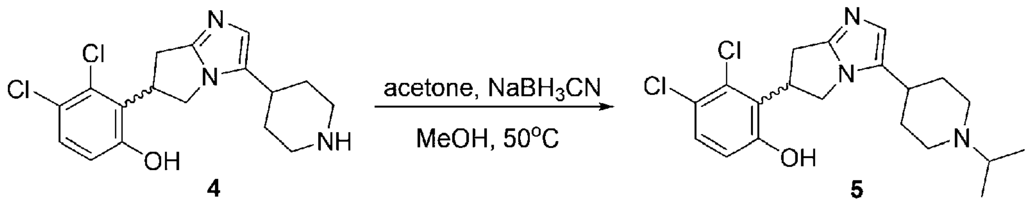


The compound 3,4-dichloro-2-(3-(piperidin-4-yl)-6,7-dihydro-5*H*-pyrrolo[1,2-*a*]imidazol-6-yl)phenol 3 (230.0 mg, 0.65 mmol) and acetone (379.2 mg, 6.53 mmol) were dissolved in methanol (10.0 mL), and the mixture was stirred at 0 °C for 30 min. Then, at 0 °C, sodium triacetoxyborohydride (123.1 mg, 0.58 mmol) was added to the mixture. The resulting mixture was stirred at 50 °C for 2 h. The reaction mixture was extracted three times with dichloromethane and washed twice with brine. The resulting organic phase was dried over anhydrous sodium sulfate and concentrated *in vacuo*. The resulting crude product was purified by silica gel chromatography (dichloromethane:methanol = 3:2) to give a white solid (80.0 mg, yield: 29%).

LC-MS: $[M+H]^+$: 394.0.

**Preparation of (*S*)-3,4-dichloro-2-(3-(1-isopropylpiperidin-4-yl)-6,7-dihydro-5*H*-pyrrolo[1,2-*a*]imidazol-6-yl)phenol and (*R*)-3,4-dichloro-2-(3-(1-isopropylpiperidin-4-yl)-6,7-dihydro-5*H*-pyrrolo[1,2-*a*]imidazol-6-yl)phenol:**

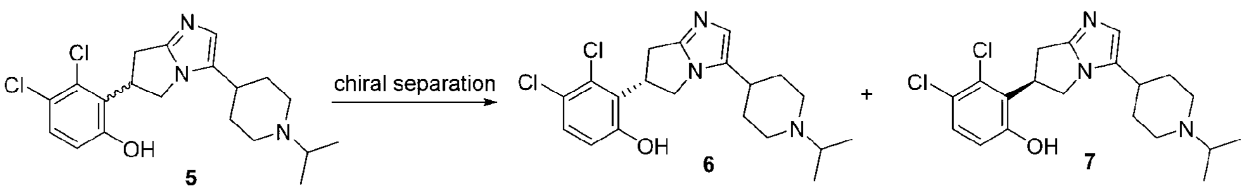


3,4-Dichloro-2-(3-(1-isopropylpiperidin-4-yl)-6,7-dihydro-5*H*-pyrrolo[1,2-*a*]imidazol-6-yl)phenol (80.0 mg, 0.20 mmol) was subjected to preparative chiral chromatography (chromatographic column: CHIRALPAK AD-H 250 mm × 20 mm, 5 μm; mobile phase: 40% EtOH ($NH_4OH$ 0.2%):60% $CO_2$) to give a pair of enantiomers.

The first compound obtained from the SFC resolution was designated as Example 94 (13.1 mg, yield: 16%), with its RT of 2.51 min analyzed under the analytical chromatographic conditions of SFC.

LC-MS: $[M+H]^+$: 394.0.

$^1H$ NMR (400 MHz, DMSO-*d6*) δ 10.95 (s, 1H), 7.32 (d, *J* = 8.8 Hz, 1H), 6.78 (d, *J* = 8.8 Hz, 1H), 6.56 (s, 1H), 4.75

(d, *J* = 8.8 Hz, 1H), 4.11 (dd, *J* = 21.2, 8.8 Hz, 2H), 3.02 (d, *J* = 8.4 Hz, 1H), 2.94 (d, *J* = 9.8 Hz, 1H), 2.79 (dd, *J* = 11.2, 6.8 Hz, 2H), 2.72 - 2.64 (m, 1H), 2.45 (s, 1H), 2.15 (dd, *J* = 11.6, 9.4 Hz, 2H), 1.82 (d, *J* = 14.8 Hz, 2H), 1.47 (td, *J* = 12.1, 3.7 Hz, 2H), 0.95 (d, *J* = 6.6 Hz, 6H).

The second compound obtained from the SFC resolution was designated as Example 95 (11.3 mg, yield: 14%), with its RT of 3.27 min analyzed under the analytical chromatographic conditions of SFC.

LC-MS: $[M+H]^+$: 394.0.

$^1H$ NMR (400 MHz, DMSO-*d6*) δ 10.66 (s, 1H), 7.44 - 7.38 (m, 1H), 7.05 (s, 1H), 6.88 (d, *J* = 8.8 Hz, 1H), 5.00 - 4.83 (m, 1H), 4.39 (t, *J* = 9.9 Hz, 1H), 4.14 (dd, *J* = 10.1, 7.2 Hz, 1H), 3.47 (dd, J = 23.1, 16.7 Hz, 3H), 3.28 - 3.21 (m, 1H), 3.14 (dd, *J* = 16.1, 7.1 Hz, 1H), 3.01 (dd, *J* = 26.8, 14.6 Hz, 3H), 2.13 (t, *J* = 13.3 Hz, 2H), 1.82 (dd, *J* = 25.1, 12.9 Hz, 2H), 1.25 (d, *J* = 6.6 Hz, 6H).

**Example 96. 3,4-Dichloro-2-(3-(4-methoxyphenyl)-6,7-dihydro-5*H*-pyrrolo[1,2-*a*]imidazol-6-yl)phenol**

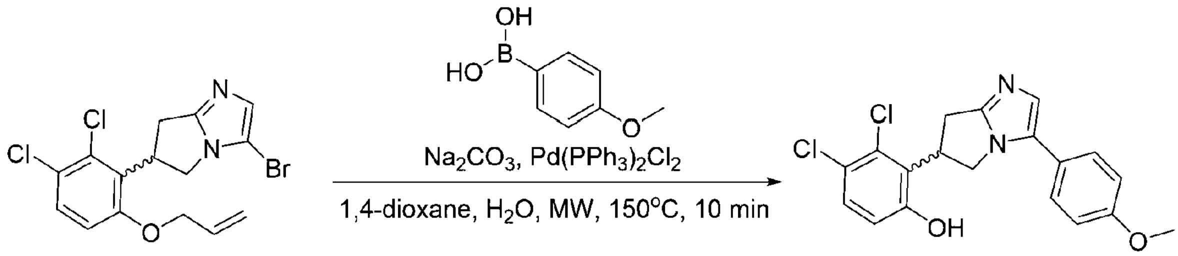


At room temperature, (4-methoxyphenyl)boronic acid (16.0 mg, 0.11 mmol) was added to a mixed solution (3.0 mL) of 6-(6-(allyloxy)-2,3-dichlorophenyl)-3-bromo-6,7-dihydro-5*H*-pyrrolo[1,2-*a*]imidazole (35.0 mg, 0.09 mmol) in dioxane and water (volume ratio: 10:1), and then sodium carbonate (29.0 mg, 0.27 mmol) and bis(triphenylphosphine)palladium(II) dichloride (13 mg, 0.02 mmol) were added. The mixture was stirred in a microwave reactor at 150 °C for 10 min. After completion of the reaction, the reaction mixture was diluted with water (20 mL) and extracted with ethyl acetate (30 mL). The organic phase was washed with saturated brine (30 mL), and the resulting organic phase was dried over anhydrous sodium sulfate and concentrated under reduced pressure. The resulting residue was subjected to preparative chromatography to give a white solid (4.8 mg, yield: 15%).

LC-MS: $[M+H]^+$: 375.0.

$^1H$ NMR (400 MHz, DMSO-d6) δ 7.62 (s, 1H), 7.56 (d, *J* = 8.8 Hz, 2H), 7.32 (d, *J* = 8.8 Hz, 1H), 7.04 (d, *J* = 8.8 Hz, 2H), 6.79 (d, *J* = 8.8 Hz, 1H), 5.23 (tt, *J* = 10.2, 6.4 Hz, 1H), 4.75 (t, *J* = 10.2 Hz, 1H), 4.49 (dd, *J* = 10.9, 6.4 Hz, 1H), 3.82 (s, 3H), 3.60 (dd, *J* = 17.1, 10.2 Hz, 1H), 3.42 *(dd, J* = 17.3, 6.2 Hz, 1H).

**Example 97. 3,4-Dichloro-2-(3-(1-methyl-1*H*-pyrazol-4-yl)-6,7-dihydro-5*H*-pyrrolo[1,2-*a*]imidazol-6-yl)phenol**

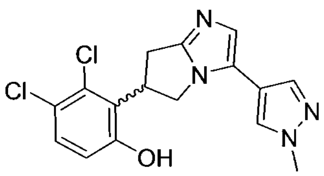

This example was prepared by reference to the preparation method for Example 96, except that the (4-methoxyphenyl)boronic acid was replaced by 1-methyl-4-(4,4,5,5-tetramethyl-1,3,2-dioxaborolan-2-yl)-1H-pyrazole.

LC-MS: $[M+H]^+$: 349.0.

$^1H$ NMR (400 MHz, $CD_3OD$) δ 8.08 (s, 1H), 7.84 (s, 1H), 7.61 (s, 1H), 7.35 (d, *J* = 8.8 Hz, 1H), 6.82 (d, *J* = 8.8 Hz, 1H), 5.32 - 5.21 (m, 1H), 4.72 (t, *J* = 9.9 Hz, 1H), 4.48 (dd, *J* = 10.5, 6.4 Hz, 1H), 3.94 (s, 3H), 3.58 (d, *J* = 10.4 Hz, 1H), 3.46 (d, *J* = 6.7 Hz, 1H).

**Example 98. 2-(3-(1-(*tert*-Butyl)-1*H*-pyrazol-4-yl)-6,7-dihydro-5*H*-pyrrolo[1,2-*a*]imidazol-6-yl)-3,4-dichlorophenol**

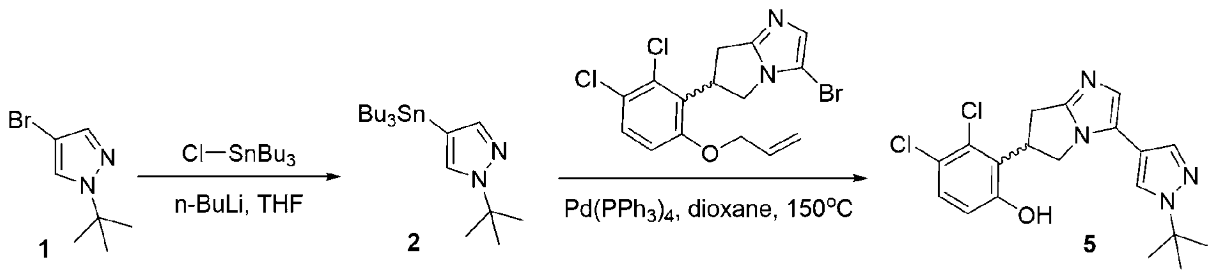


**Synthesis of 1-(*tert*-butyl)-4-(tributylstannyl)-1*H*-pyrazole (2):**

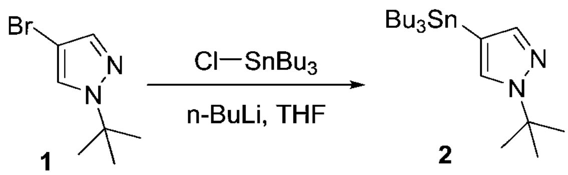


4-Bromo-1-*tert*-butylpyrazole (3.0 g, 0.015 mol) was added to a three-necked reaction flask containing tetrahydrofuran (30 mL). At -78 °C, *n*-butyllithium (6.5 mL, 2.5 M in *n*-hexane) was slowly added dropwise under a nitrogen atmosphere. The mixture was stirred at -78 °C for 30 min, and then tributyltin chloride (5.3 g, 0.016 mol) was added. The reaction mixture was stirred at room temperature for 16 h. After completion of the reaction, the reaction mixture was quenched with an aqueous ammonium chloride solution (20 mL) and extracted twice with ethyl acetate (100 mL). The organic phases were combined, washed with saturated brine (200 mL), and dried over anhydrous sodium sulfate. After filtration, the filtrate was concentrated under reduced pressure, and the resulting residue was purified by silica gel column chromatography and eluted (petroleum ether:ethyl acetate = 20:1) to give a colorless oily subtance (2.8 g, yield: 39%).

**Synthesis of 2-(3-(1-(tert-butyl)-1*H*-pyrazol-4-yl)-6,7-dihydro-5*H*-pyrrolo[1,2-*a*]imidazol-6-yl)-3,4-dichlorophenol (5):**

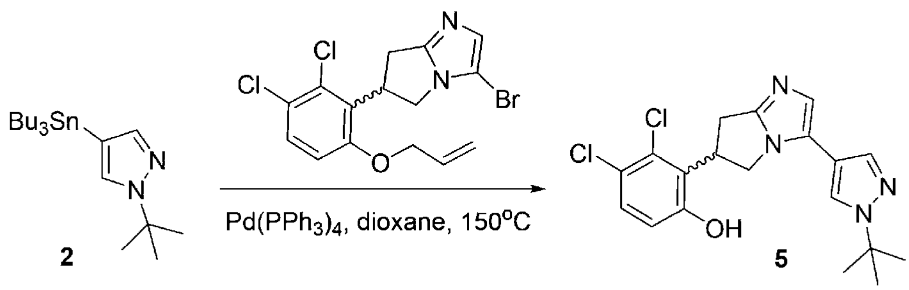


6-(6-(Allyloxy)-2,3-dichlorophenyl)-3-bromo-6,7-dihydro-5*H*-pyrrolo[1,2-*a*]imidazole (120.0 mg, 0.31 mmol), 1-(tert-butyl)-4-(tributylstannyl)-1*H*-pyrazole (255.5 mg, 0.62 mmol), and tetrakis(triphenylphosphine)palladium(0) (71.2 mg, 0.06 mmol) were mixed in 1,4-dioxane and water (5 mL/0.5 mL), and the mixture was allowed to react under microwave at 150 °C for 1.5 h under an argon atmosphere. After completion of the reaction, the reaction mixture was diluted with water (10 mL) and extracted twice with ethyl acetate (20 mL). The organic phases were combined, washed with saturated brine (20 mL), and dried over anhydrous sodium sulfate. After filtration, the filtrate was concentrated under reduced pressure, and the resulting residue was purified by silica gel column chromatography and eluted (dichloromethane:methanol = 5:1) to give a crude product, which was then purified by preparative chromatography to give a white

solid (5.2 mg, yield: 4%).

LC-MS: $[M+H]^+$: 390.8.

$^1$H NMR (400 MHz, DMSO-*d6*) δ 10.50 (s, 1H), 7.69 (s, 1H), 7.40 (d, *J* = 8.8 Hz, 1H), 7.07 (s, 1H), 6.87 (d, *J* = 8.8 Hz, 1H), 4.88 (d, *J* = 8.7 Hz, 1H), 4.44 (t, *J* = 9.8 Hz, 1H), 4.20 (dd, *J* = 9.7, 7.9 Hz, 1H), 3.09 (d, *J* = 8.9 Hz, 2H), 1.52 (s, 9H).

**Example 99. 2-(3-(1*H*-Pyrazol-4-yl)-6,7-dihydro-5*H*-pyrrolo[1,2-*a*]imidazol-6-yl)-3,4-dichlorophenol**

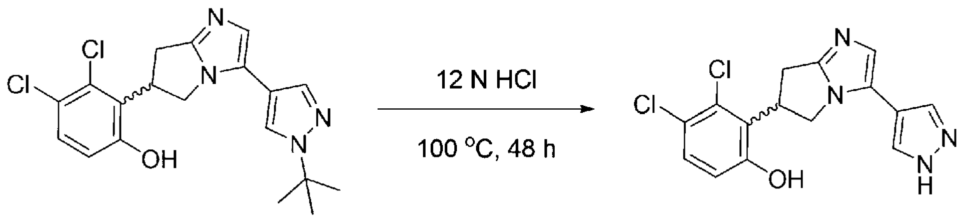


2-(3-(1-(*tert*-Butyl)-1*H*-pyrazol-4-yl)-6,7-dihydro-5*H*-pyrrolo[1,2-*a*]imidazol-6-yl)-3,4-dichlorophenol (10.0 mg) was added to a 100-mL reaction flask, and concentrated hydrochloric acid (10 mL) was added. The resulting solution was stirred at 100 °C for 48 h. After completion of the reaction, the reaction mixture was concentrated under reduced pressure, and the residue was purified by preparative chromatography to give a white solid (3.0 mg, yield: 37%).

LC-MS: $[M+H]^+$: 335.0.

$^1$H NMR (400 MHz, $CD_3OD$) δ 12.97 (s, 1H), 11.17 - 10.52 (m, 1H), 7.86 (s, 2H), 7.38 (d, *J* = 8.8 Hz, 1H), 7.02 (s, 1H), 6.87 (d, *J* = 8.8 Hz, 1H), 4.87 (p, *J* = 8.8 Hz, 1H), 4.38 (t, *J* = 9.7 Hz, 1H), 4.19 (dd, *J* = 9.6, 8.0 Hz, 1H), 3.11 - 3.00 (m, 2H).

**Example 100. 3,4-Dichloro-2-(3-(4-isopropylpiperazin-1-yl)-6,7-dihydro-5*H*-pyrrolo[1,2-*a*]imidazol-6-yl)phenol**

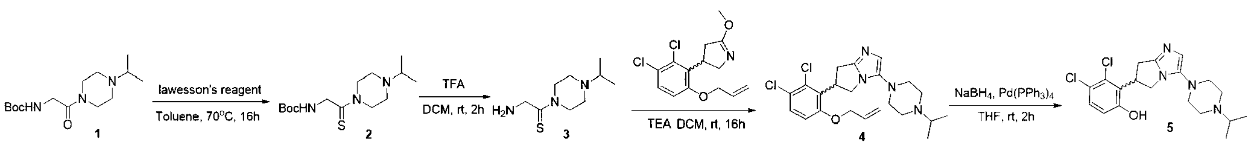


**Synthesis of *tert*-butyl (2-(4-isopropylpiperazin-1-yl)-2-thioxoethyl)carbamate (2):**

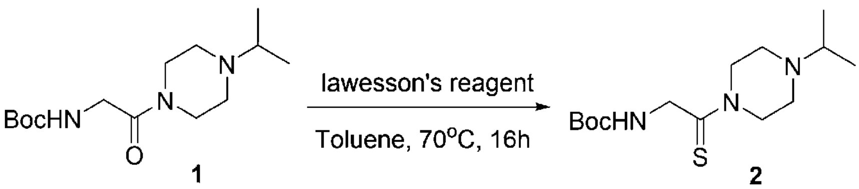


At 0 °C, Lawesson's reagent (1.9 g, 4.68 mmol) was added to a solution of *tert*-butyl (2-(4-isopropylpiperazin-1-yl)-2-oxoethyl)carbamate (1.1 g, 3.90 mmol) in toluene (15.0 mL). The mixture was stirred at 70 °C for 16 h under a nitrogen atmosphere. After completion of the reaction, the reaction mixture was diluted with water (50 mL) and extracted three times with ethyl acetate (50 mL). The organic phases were combined, washed with saturated brine (100 mL), and dried over anhydrous sodium sulfate, and the solvent was removed by distillation under reduced pressure. The resulting crude product was subjected to silica gel chromatography (dichloromethane:methanol = 10:1) to give the target product (1.0 g, yield: 86%).

LC-MS: $[M+H]^+$: 302.0.

**Synthesis of 2-amino-1-(4-isopropylpiperazin-1-yl)ethane-1-thione (3):**

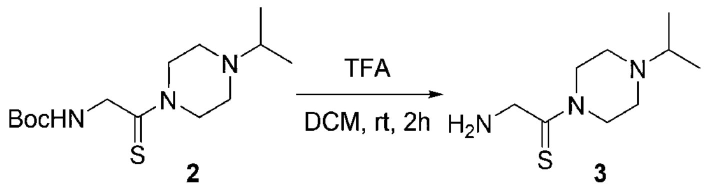


At room temperature, *tert*-butyl (2-(4-isopropylpiperazin-1-yl)-2-thioxoethyl)carbamate (700.0 mg, 2.32 mmol) was added to a solution of trifluoroacetic acid and dichloromethane (1:5, 6.0 mL). The mixture was stirred at room temperature for 2 h. After completion of the reaction, the reaction mixture was directly rotary evaporated to dryness to give a yellow oily substance (700.0 mg, crude product, yield: 80%).
LC-MS: $[M+H]^+$: 202.0.

**Synthesis of 6-(6-(allyloxy)-2,3-dichlorophenyl)-3-(4-isopropylpiperazin-1-yl)-6,7-dihydro-5*H*-pyrrolo[1,2-*a*]imidazole (4):**

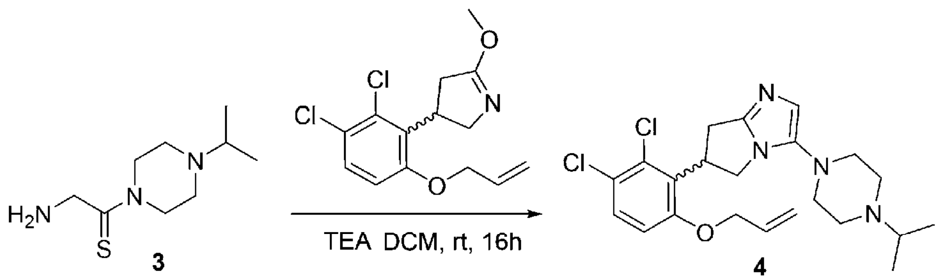


At room temperature, 2-amino-1-(4-isopropylpiperazin-1-yl)ethane-1-thione (450.0 mg, 2.24 mmol) was added to a solution of 3-(6-(allyloxy)-2,3-dichlorophenyl)-5-methoxy-3,4-dihydro-2*H*-pyrrole (200.0 mg, 0.67 mmol) in dichloromethane (5.0 mL). The mixture was stirred at room temperature for 16 h. After completion of the reaction, the reaction mixture was directly rotary evaporated to dryness, and the resulting crude product was subjected to silica gel chromatography (dichloromethane:methanol = 20:1) to give the target product (80.0 mg, yield: 28%).
LC-MS: $[M+H]^+$: 434.9.

Synthesis **of 3,4-dichloro-2-(3-(4-isopropylpiperazin-1-yl)-6,7-dihydro-5*H*-pyrrolo[1,2-*a*]imidazol-6-yl)phenol (5):**

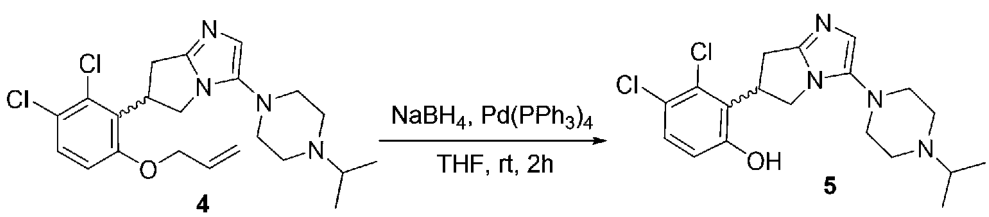


At room temperature, tetrakis(triphenylphosphine)palladium(0) (21.0 mg, 0.02 mmol) was added to a solution of 6-(6-(allyloxy)-2,3-dichlorophenyl)-3-(4-isopropylpiperazin-1-yl)-6,7-dihydro-5*H*-pyrrolo[1,2-*a*]imidazole (80.0 mg, 0.18 mmol) and sodium borohydride (14.0 mg, 0.35 mmol) in tetrahydrofuran (3.0 mL). The flask was purged using an argon balloon, and the mixture was stirred at room temperature for 2 h. After completion of the reaction, the reaction mixture was filtered through an organic-phase filter membrane, and the filtrate was rotary evaporated to dryness. The crude product was subjected to preparative chromatography to give a white solid (15.5 mg, yield: 22%).
LC-MS: $[M+H]^+$: 394.9.

$^1$H NMR (400 MHz, DMSO-$d_6$) δ 10.98 (s, 1H), 7.43 (d, *J* = 8.6 Hz, 1H), 7.20 (s, 1H), 6.91 (d, *J* = 8.3 Hz, 1H), 5.01 (s, 1H), 4.55 (t, *J* = 10.1 Hz, 1H), 4.18 (dd, *J* = 10.2, 6.1 Hz, 1H), 3.78 (d, *J* = 7.0 Hz, 6H), 3.29 - 3.00 (m, 5H), 1.26 (t, *J* = 9.6 Hz, 6H).

**Example 101. 3,4-Dichloro-2-(3-morpholino-6,7-dihydro-5*H*-pyrrolo[2,1-*c*][1,2,4]triazol-6-yl)phenol**

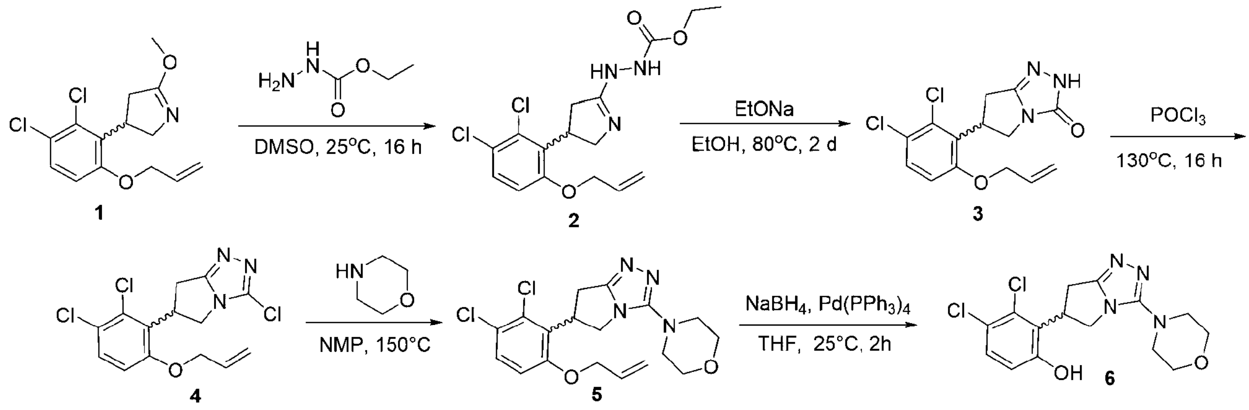

1 2 3 4 5 6

**Synthesis of ethyl 2-(3-(6-(allyloxy)-2,3-dichlorophenyl)-3,4-dihydro-2*H*-pyrrol-5-yl)hydrazine-1-carboxylate (2):**

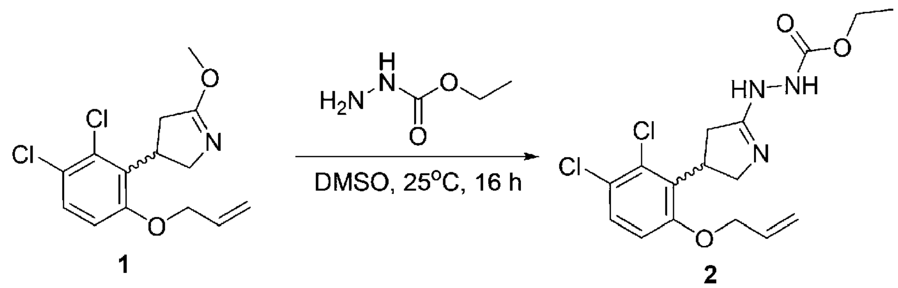

1 2

A solution of the compound 3-(6-(allyloxy)-2,3-dichlorophenyl)-5-methoxy-3,4-dihydro-2*H*-pyrrole (5.0 g, 0.02 mol) and ethyl hydrazinecarboxylate (2.6 g, 0.03 mol) in dimethylsulfoxide (50.0 mL) was stirred at 25 °C for 16 h. After completion of the reaction, the solvent was removed by lyophilization under reduced pressure. The resulting crude product was purified by silica gel chromatography (methanol:dichloromethane = 1:12) to give a yellow oily subtance (6.0 g, yield: 97%).

LC-MS: [M+H]$^+$: 371.8.

**Synthesis of 6-(6-(allyloxy)-2,3-dichlorophenyl)-2,5,6,7-tetrahydro-3*H*-pyrrolo[2,1-*c*][1,2,4]triazol-3-one (3):**

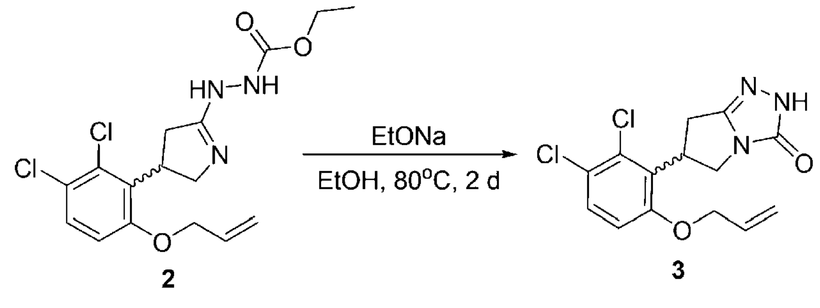

2 3

Sodium ethoxide (11.0 g, 0.16 mmol, 20% wt in ethanol) was added to a solution of ethyl 2-(3-(6-(allyloxy)-2,3-dichlorophenyl)-3,4-dihydro-2*H*-pyrrol-5-yl)hydrazine-1-carboxylate (6.0 g, 0.02 mol) in ethanol (60.0 mL), and the mixture was stirred at 80 °C for 2 days. After completion of the reaction, the reaction mixture was cooled to room temperature, then diluted by adding water (200.0 mL), and extracted three times with ethyl acetate (300.0 mL). The organic phases were combined, washed twice with saturated brine (500.0 mL), concentrated *in vacuo,* and dried. The resulting crude product was dissolved by adding dichloromethane (20.0 mL), and the solution was slowly stirred while petroleum ether (100.0 mL) was added, resulting in the precipitation of a large amount of solids. The solids were filtered out, and the filter cake was washed with petroleum ether and dried to give a brown solid (3.4 g, yield: 65%).
LC-MS: $[M+H]^+$: 325.8.
$^1$H NMR (400 MHz, DMSO-$d_6$) δ 11.22 (s, 1H), 7.54 (d, *J* = 8.9 Hz, 1H), 7.10 (d, *J* = 9.0 Hz, 1H), 5.81 (ddd, *J* = 22.5, 10.6, 5.3 Hz, 1H), 5.26 - 5.14 (m, 2H), 4.85 (tt, *J* = 10.0, 6.9 Hz, 1H), 4.57 (d, *J* = 4.6 Hz, 2H), 3.92 (t, *J* = 9.9 Hz, 1H), 3.61 (dd, *J* = 10.0, 6.6 Hz, 1H), 3.07 (dd, *J* = 16.4, 10.5 Hz, 1H), 2.83 (dd, *J* = 16.4, 7.1 Hz, 1H).

**Synthesis of 6-(6-(allyloxy)-2,3-dichlorophenyl)-3-chloro-6,7-dihydro-5*H*-pyrrolo[2,1-*c*][1,2,4]triazole (4):**

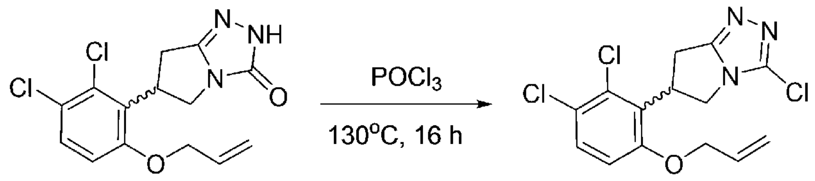


3 4

The compound 6-(6-(allyloxy)-2,3-dichlorophenyl)-2,5,6,7-tetrahydro-3*H*-pyrrolo[2,1-*c*][1,2,4]triazol-3-one (1.0 g, 3.1 mmol) was dissolved in phosphoryl chloride (5 mL), and the mixture was stirred at 130 °C for 16 h. The reaction mixture was quenched after being poured into ice water, and the pH was adjusted to 8-9 with a saturated aqueous sodium bicarbonate solution. The mixture was extracted three times with ethyl acetate and washed twice with saturated brine. The resulting organic phase was dried over anhydrous sodium sulfate and concentrated *in vacuo,* and the resulting crude product was purified by silica gel chromatography (petroleum ether:ethyl acetate = 1:4) to give a brown solid (70.0 mg, yield: 6%).
LC-MS: $[M+H]^+$: 344.0.

**Synthesis of 4-(6-(6-(allyloxy)-2,3-dichlorophenyl)-6,7-dihydro-5*H*-pyrrolo[2,1-*c*][1,2,4]triazol-3-yl)morpholine (5):**

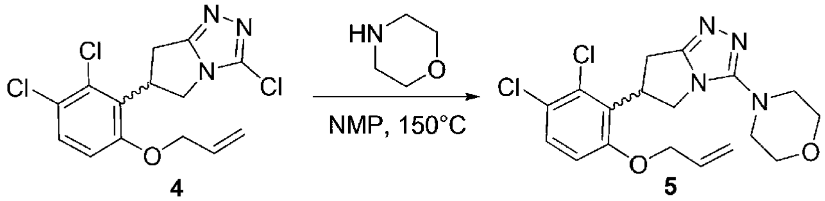


4 5

The compound 6-(6-(allyloxy)-2,3-dichlorophenyl)-3-chloro-6,7-dihydro-5*H*-pyrrolo[2,1-*c*][1,2,4]triazole (70.0 mg, 0.20 mmol) was dissolved in *N*-methylpyrrolidone (2 mL), and morpholine (176.9 mg, 2.03 mmol) was added to the mixture. The resulting mixture was stirred at 150 °C for 16 h. The reaction mixture was diluted by adding water, extracted three times with ethyl acetate, washed twice with saturated brine, concentrated *in vacuo,* and dried. The resulting crude product was purified by silica gel chromatography (dichloromethane:methanol = 10:1) to give a white solid (30.0 mg, yield: 37%).
LC-MS: $[M+H]^+$: 395.0.

**Synthesis of 3,4-dichloro-2-(3-morpholino-6,7-dihydro-5*H*-pyrrolo[2,1-*c*][1,2,4]triazol-6-yl)phenol (6):**

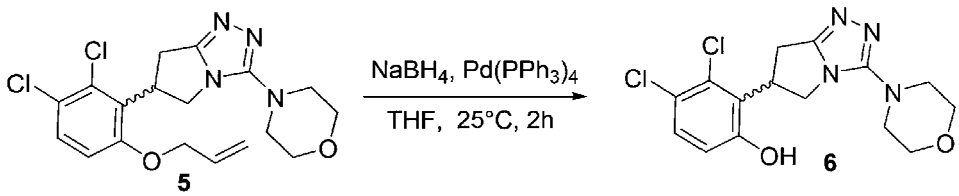


5 6

The compound 4-(6-(6-(allyloxy)-2,3-dichlorophenyl)-6,7-dihydro-5*H*-pyrrolo[2,1-*c*][1,2,4]triazol-3-yl)morpholine (30.0 mg, 0.076 mmol) was dissolved in a tetrahydrofuran solution (5 mL), and sodium borohydride (4.3 mg, 0.11 mmol) and tetrakis(triphenylphosphine)palladium(0) (17.5 mg, 0.015 mmol) were added to the reaction mixture. The resulting mixture was allowed to react at 25 °C for 2 h. The reaction mixture was filtered through celite. The filtrate was evaporated to dryness, and the residue was separated by preparative chromatography to give a white solid (2.0 mg, yield: 7%).

LC-MS: $[M+H]^+$: 355.0.

$^1H$ NMR (400 MHz, DMSO-$d_6$) δ 10.86 (s, 1H), 7.38 (d, *J* = 8.8 Hz, 1H), 6.87 (d, *J* = 8.8 Hz, 1H), 4.91 (dd, *J* = 17.2, 9.1 Hz, 1H), 4.23 (t, *J* = 9.6 Hz, 1H), 4.01 (dd, *J* = 9.6, 7.6 Hz, 1H), 3.73 - 3.62 (m, 4H), 3.20 - 3.13 (m, 4H), 3.00 (dd, *J* = 9.0, 3.6 Hz, 2H).

Example 102. **3,4-Dichloro-2-(3-((1-isopropylpiperidin-4-yl)oxy)-6,7-dihydro-5*H*-pyrrolo[2,1-*c*][1,2,4]triazol-6-yl) phenol**

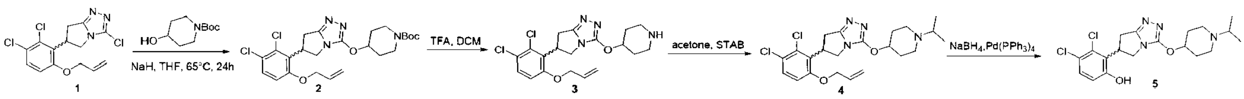


**Synthesis of *tert*-butyl 4-((6-(6-(allyloxy)-2,3-dichlorophenyl)-6,7-dihydro-5*H*-pyrrolo[2,1-*c*][1,2,4]triazol-3-yl) oxy)piperidine-1-carboxylate (2):**

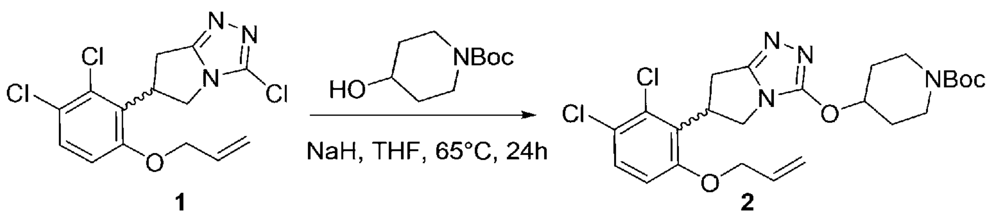


1 2

The compound *tert*-butyl 4-hydroxypiperidine-1-carboxylate (87.5 mg, 0.44 mmol) was dissolved in tetrahydrofuran (20.0 mL). At 0 °C, sodium hydride (17.3 mg, 0.44 mmol, 60% in mineral oil) was added to the mixture. The resulting mixture was stirred at 0 °C for 30 min. Then, at 0 °C, 6-(6-(allyloxy)-2,3-dichlorophenyl)-3-chloro-6,7-dihydro-5*H*-pyrrolo[2,1-*c*][1,2,4]triazole (100 mg, 0.29 mmol) was added to the mixture, and the resulting mixture was stirred at 65 °C for 24 h. The reaction mixture was extracted three times with dichloromethane and washed twice with brine. The resulting organic phase was dried over anhydrous sodium sulfate and concentrated *in vacuo.* The resulting crude product was directly fed for the next step without purification.

LC-MS: $[M+H]^+$: 508.8.

**Synthesis of 6-(6-(allyloxy)-2,3-dichlorophenyl)-3-(piperidin-4-oxy)-6,7-dihydro-5*H*-pyrrolo[2,1-*c*][1,2,4]triazole (3):**

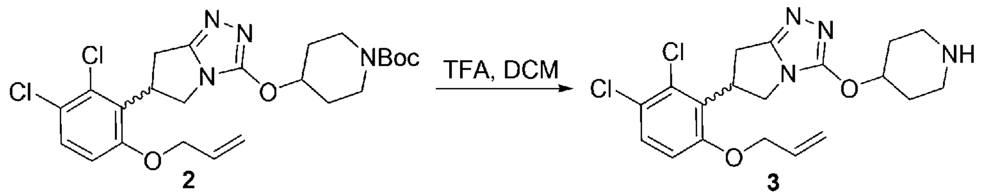


2 3

The compound *tert*-butyl 4-((6-(6-(allyloxy)-2,3-dichlorophenyl)-6,7-dihydro-5*H*-pyrrolo[2,1-*c*][1,2,4]triazol-3-yl)oxy)piperidine-1-carboxylate (200 mg, 0.39 mmol) was dissolved in a solution of dichloromethane:trifluoroacetic acid (3:1, 4.0 mL), and the mixture was stirred at room temperature for 1 h. The reaction mixture was evaporated to dryness, and the residue was separated by preparative chromatography to give a white solid (90.0 mg, yield: 56%).
LC-MS: $[M+H]^+$: 409.1.

**Synthesis of 6-(6-(allyloxy)-2,3-dichlorophenyl)-3-((1-isopropylpiperidin-4-yl)oxy)-6,7-dihydro-5*H*-pyrrolo[2,1-*c*][1,2,4]triazole (4):**

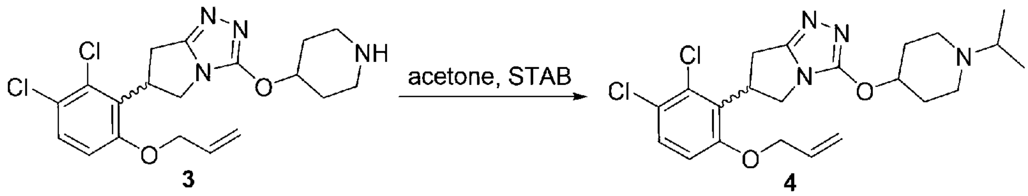


3 4

The compound 6-(6-(allyloxy)-2,3-dichlorophenyl)-3-(piperidin-4-oxy)-6,7-dihydro-5*H*-pyrrolo[2,1-*c*][1,2,4]triazole (90 mg, 0.22 mmol) and acetone (25.5 mg, 0.44 mmol) were dissolved in dichloromethane (9.0 mL). At 0 °C, triethylamine (44.5 mg, 0.44 mmol) was added to the mixture. The resulting mixture was stirred at 0 °C for 30 min. Then, at 0 °C, sodium triacetoxyborohydride (93.2 mg, 0.44 mmol) was added to the mixture. The resulting mixture was stirred at room temperature for 2 h. The reaction mixture was extracted three times with dichloromethane and washed twice with brine. The resulting organic phase was dried over anhydrous sodium sulfate and concentrated *in vacuo.* The resulting crude product was purified by silica gel chromatography (dichloromethane:methanol = 3:2) to give a yellow oily liquid (50.0 mg, yield: 50%).
LC-MS: $[M+H]^+$: 450.9.

**Synthesis of 3,4-dichloro-2-(3-((1-isopropylpiperidin-4-yl)oxy)-6,7-dihydro-5*H*-pyrrolo[2,1-*c*][1,2,4]triazol-6-yl) phenol (5):**

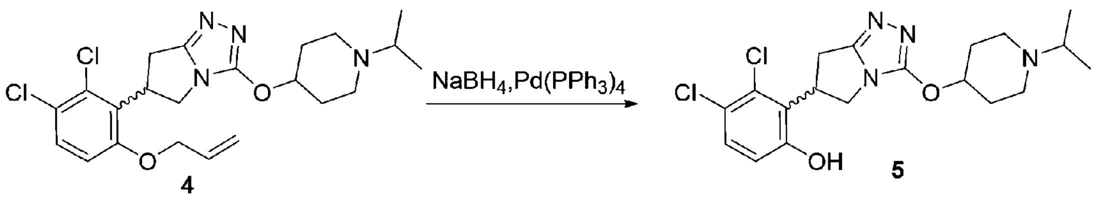


4 5

The compound 6-(6-(allyloxy)-2,3-dichlorophenyl)-3-((1-isopropylpiperidin-4-yl)oxy)-6,7-dihydro-5*H*-pyrrolo [2,1-*c*][1,2,4]triazole (50.0 mg, 0.11 mmol) was dissolved in a tetrahydrofuran solution (9.0 mL), and sodium borohydride (6.3 mg, 0.17 mmol) and tetrakis(triphenylphosphine)palladium(0) (25.6 mg, 0.022 mmol) were added to the reaction mixture. The resulting mixture was allowed to react at room temperature for 2 h. The reaction mixture was filtered through a filter membrane. The resulting filtrate was evaporated to dryness, and the residue was separated by preparative chromatography to give a white solid (18.9 mg, yield: 41%).
LC-MS: $[M+H]^+$: 411.1.
$^1H$ NMR (400 MHz, DMSO-$d_6$) δ 9.34 (d, *J* = 8.5 Hz, 1H), 7.42 (dd, *J* = 8.8, 3.5 Hz, 1H), 6.89 (dd, *J* = 8.8, 5.6 Hz, 1H), 5.12 - 4.93 (m, 2H), 4.19 (t, *J* = 18.6 Hz, 1H), 3.99 (dd, *J* = 16.8, 9.6 Hz, 1H), 3.56 - 3.44 (m, 2H), 3.35 (d, *J* = 6.1 Hz, 1H), 3.29 - 3.05 (m, 4H), 2.43 (d, *J* = 11.4 Hz, 1H), 2.27 (s, 1H), 2.17 - 2.05 (m, 1H), 1.90 (s, 1H), 1.26 (dd, *J* = 6.6, 3.4 Hz, 6H).

**Examples 103-104. (*S*)-6-(2,3-Dichloro-6-hydroxyphenyl)-2-(1-isopropylpiperidin-4-yl)-2,5,6,7-tetrahydro-3*H*-pyrrolo[2,1-*c*][1,2,4]triazol-3-one and (*R*)-6-(2,3-dichloro-6-hydroxyphenyl)-2-(1-isopropylpiperidin-4-yl)-2,5,6,7-tetrahydro-3*H*-pyrrolo[2,1-*c*][1,2,4]triazol-3-one**

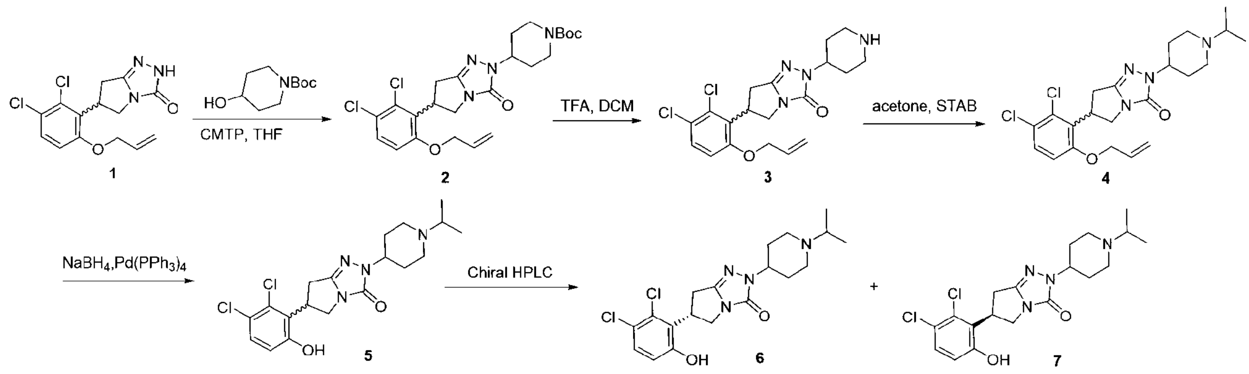

1 2 3 4 5 6 + 7

**Synthesis of *tert*-butyl 4-(6-(6-(allyloxy)-2,3-dichlorophenyl)-3-carbonyl-6,7-dihydro-3*H*-pyrrolo[2,1-*c*][1,2,4]triazol-2(5*H*)-yl)piperidine-1-carboxylate (2):**

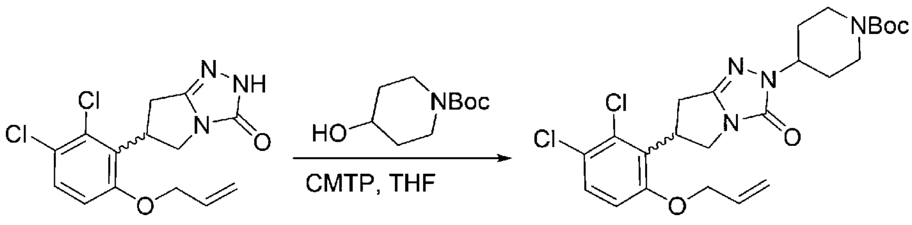


1 2

The compound 6-(6-(allyloxy)-2,3-dichlorophenyl)-2,5,6,7-tetrahydro-3*H*-pyrrolo[2,1-*c*][1,2,4]triazol-3-one (600.0 mg, 1.84 mmol) and *tert*-butyl 4-hydroxypiperidine-1-carboxylate (1.17 g, 5.81 mmol) were dissolved in a tetrahydrofuran solution (10.0 mL). At room temperature, cyanomethylenetributylphosphorane (1.33 g, 5.52 mmol) was added to the mixture. The resulting mixture was stirred under microwave heating at 150 °C for 40 min under a nitrogen atmosphere. The reaction mixture was extracted three times with ethyl acetate and washed twice with brine. The resulting organic phase was dried over anhydrous sodium sulfate and concentrated *in vacuo*. The resulting crude product was purified by silica gel chromatography (petroleum ether:ethyl acetate = 1:1) to give a pale yellow solid (650.0 mg, yield: 69%).

LC-MS: $[M+H]^+$: 508.8.

**Synthesis of 6-(6-(allyloxy)-2,3-dichlorophenyl)-2-(piperidin-4-yl)-2,5,6,7-tetrahydro-3*H*-pyrrolo[2,1-*c*][1,2,4]triazol-3-one (3):**

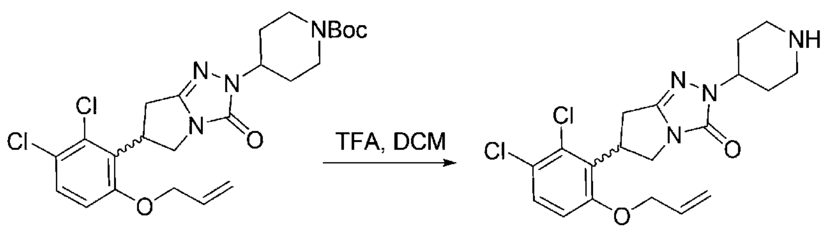


2 3

*tert*-Butyl 4-(6-(6-(allyloxy)-2,3-dichlorophenyl)-3-carbonyl-6,7-dihydro-3*H*-pyrrolo[2,1-*c*][1,2,4]triazol-2(5*H*)-yl)piperidine-1-carboxylate (650.0 mg, 1.28 mmol) was dissolved in a mixed solution of dichloromethane:trifluoroacetic acid (3:1, 12.0 mL), and the mixture was stirred at room temperature for 1 h. The reaction mixture was evaporated to dryness, and the residue was separated by preparative chromatography to give a white solid (300.0 mg, yield: 57%).

LC-MS: $[M+H]^+$: 409.0.

**Synthesis of 6-(6-(allyloxy)-2,3-dichlorophenyl)-2-(1-isopropylpiperidin-4-yl)-2,5,6,7-tetrahydro-3*H*-pyrrolo[2,1-*c*][1,2,4]triazol-3-one (4):**

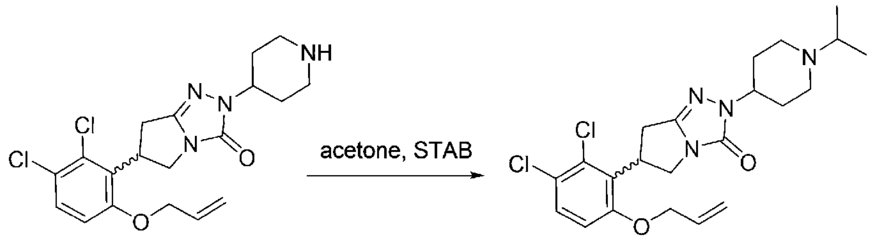


3 4

The compound 6-(6-(allyloxy)-2,3-dichlorophenyl)-2-(piperidin-4-yl)-2,5,6,7-tetrahydro-3*H*-pyrrolo[2,1-*c*][1,2,4]triazol-3-one (300.0 mg, 0.73 mmol) and acetone (127.7 mg, 2.20 mmol) were dissolved in dichloromethane (12.0 mL). At 0 °C, triethylamine (148.3 mg, 1.47 mmol) was added to the mixture. The resulting mixture was stirred at 0 °C for 30 min. Then, at 0 °C, sodium triacetoxyborohydride (466.0 mg, 2.20 mmol) was added to the mixture. The resulting mixture was stirred at room temperature for 2 h. The reaction mixture was extracted three times with dichloromethane and washed twice with brine. The resulting organic phase was dried over anhydrous sodium sulfate and concentrated *in vacuo.* The resulting crude product was purified by silica gel chromatography (dichloromethane:methanol = 5:1) to give a yellow oily liquid (200.0 mg, yield: 60%).

LC-MS: $[M+H]^+$: 450.9.

**Synthesis of 6-(2,3-dichloro-6-hydroxyphenyl)-2-(1-isopropylpiperidin-4-yl)-2,5,6,7-tetrahydro-3*H*-pyrrolo[2,1-*c*][1,2,4]triazol-3-one (5):**

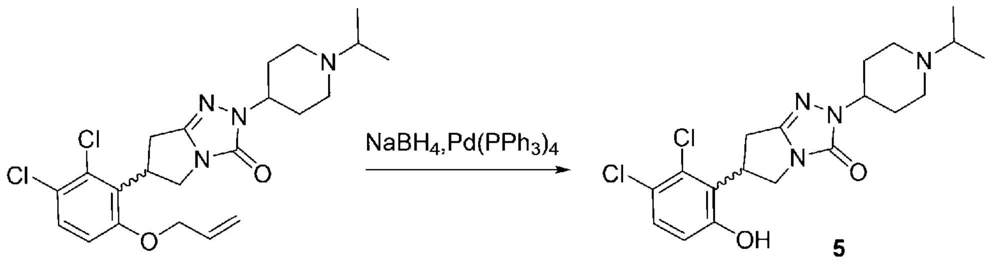


5

4

The compound 6-(6-(allyloxy)-2,3-dichlorophenyl)-2-(1-isopropylpiperidin-4-yl)-2,5,6,7-tetrahydro-3*H*-pyrrolo[2,1-*c*][1,2,4]triazol-3-one (150.0 mg, 0.33 mmol) was dissolved in a tetrahydrofuran solution (15.0 mL), and sodium borohydride (18.9 mg, 0.50 mmol) and tetrakis(triphenylphosphine)palladium(0) (76.8 mg, 0.066 mmol) were added to the reaction mixture. The resulting mixture was allowed to react at room temperature for 2 h. The reaction mixture was filtered through a filter membrane. The resulting filtrate was evaporated to dryness, and the residue was separated by preparative chromatography to give a pale red solid (34.3 mg, yield: 25%).

LC-MS: $[M+H]^+$: 410.9.

$^1H$ NMR (400 MHz, DMSO-$d_6$) δ 11.05 (s, 1H), 8.20 (s, 1H), 7.39 (d, *J* = 8.8 Hz, 1H), 6.86 (d, *J* = 8.8 Hz, 1H), 4.77 (t, *J* = 8.6 Hz, 1H), 3.96 - 3.87 (m, 2H), 3.72 (dd, *J* = 9.8, 7.2 Hz, 1H), 3.10 - 3.03 (m, 1H), 2.95 (dd, *J* = 16.2, 7.7 Hz, 3H), 2.90 - 2.83 (m, 1H), 2.39 (t, *J* = 11.1 Hz, 2H), 1.96 - 1.83 (m, 2H), 1.77 (s, 2H), 1.02 (d, *J* = 6.6 Hz, 6H).

**Preparation of (*S*)-6-(2,3-dichloro-6-hydroxyphenyl)-2-(1-isopropylpiperidin-4-yl)-2,5,6,7-tetrahydro-3*H*-pyrrolo[2,1-*c*][1,2,4]triazol-3-one and (*R*)-6-(2,3-dichloro-6-hydroxyphenyl)-2-(1-isopropylpiperidin-4-yl)-2,5,6,7-tetrahydro-3*H*-pyrrolo[2,1-*c*][1,2,4]triazol-3-one:**

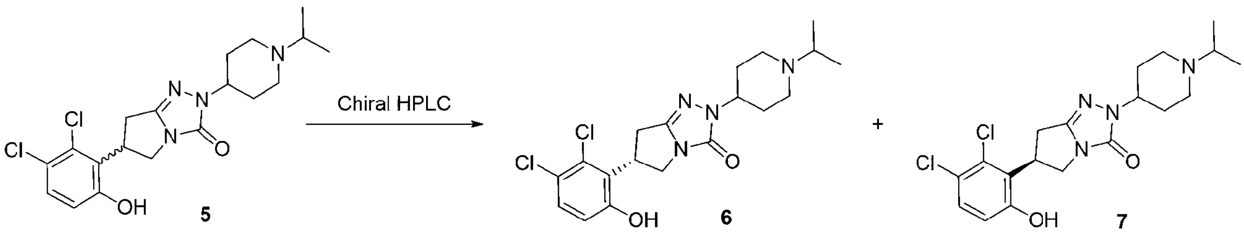


6-(2,3-Dichloro-6-hydroxyphenyl)-2-(1-isopropylpiperidin-4-yl)-2,5,6,7-tetrahydro-3*H*-pyrrolo[2,1-*c*][1,2,4]triazol-3-one (34.3 mg, 0.084 mmol) was subjected to preparative chiral chromatography (chromatographic column: CHIRALPAK AD-H 250 mm × 20 mm, 5 μm; mobile phase: 40% EtOH ($NH_4OH$ 0.2%): 60% $CO_2$) to give a pair of enantiomers.

The first compound obtained from the SFC resolution was designated as Example 103 (9.6 mg, yield: 28%), with its RT of 4.412 min analyzed under the analytical chromatographic conditions of SFC.

LC-MS: $[M+H]^+$: 410.9.

$^1H$ NMR (400 MHz, DMSO-d6) δ 7.38 (d, *J* = 8.8 Hz, 1H), 6.85 (d, *J* = 8.8 Hz, 1H), 4.81 - 4.72 (m, 1H), 3.93 (t, *J* = 9.7 Hz, 1H), 3.87 - 3.78 (m, 1H), 3.72 (dd, *J* = 9.8, 7.1 Hz, 1H), 3.06 (dd, *J* = 16.3, 10.1 Hz, 1H), 2.98 - 2.84 (m, 3H), 2.75 (dt, *J* = 13.2, 6.5 Hz, 1H), 2.25 (t, *J* = 11.5 Hz, 2H), 1.87 - 1.69 (m, 4H), 0.98 (d, *J* = 6.6 Hz, 6H).

The second compound obtained from the SFC resolution was designated as Example 104 (10.6 mg, yield: 31%), with its RT of 4.403 min analyzed under the analytical chromatographic conditions of SFC.

LC-MS: $[M+H]^+$: 410.9.

$^1H$ NMR (400 MHz, DMSO-d6) δ 7.38 (d, *J* = 8.8 Hz, 1H), 6.85 (d, *J* = 8.8 Hz, 1H), 4.82 - 4.71 (m, 1H), 3.93 (t, *J* = 9.7 Hz, 1H), 3.89 - 3.80 (m, 1H), 3.72 (dd, *J* = 9.9, 7.1 Hz, 1H), 3.06 (dd, *J* = 16.3, 10.1 Hz, 1H), 2.98 - 2.86 (m, 3H), 2.77 (dt, *J* = 13.0, 6.5 Hz, 1H), 2.28 (t, *J* = 11.4 Hz, 2H), 1.81 (ddd, *J* = 29.4, 19.0, 3.1 Hz, 4H), 0.99 (d, *J* = 6.6 Hz, 6H).

**Example 105. 6-(2,3-Dichloro-6-hydroxyphenyl)-2-(piperidin-4-yl)-2,5,6,7-tetrahydro-3*H*-pyrrolo[2,1-*c*][1,2,4]triazol-3-one**

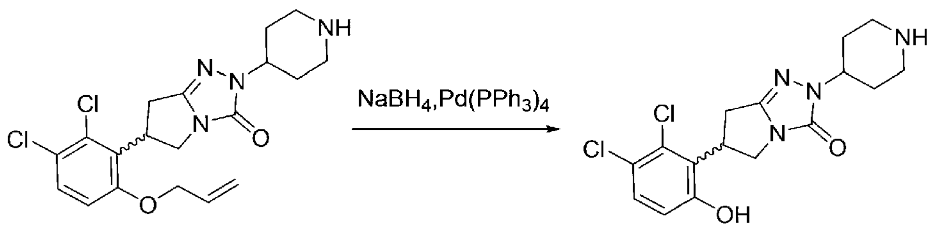


The compound 6-(6-(allyloxy)-2,3-dichlorophenyl)-2-(piperidin-4-yl)-2,5,6,7-tetrahydro-3*H*-pyrrolo[2,1-*c*][1,2,4]triazol-3-one (100.0 mg, 0.24 mmol) was dissolved in a tetrahydrofuran solution (10.0 mL), and sodium borohydride (13.9 mg, 0.37 mmol) and tetrakis(triphenylphosphine)palladium(0) (56.5 mg, 0.049 mmol) were added to the reaction mixture. The resulting mixture was allowed to react at room temperature for 2 h. The reaction mixture was filtered through a filter membrane. The resulting filtrate was evaporated to dryness, and the residue was separated by preparative chromatography to give a white solid (9.3 mg, yield: 10%).

LC-MS: $[M+H]^+$: 369.1.

$^1H$ NMR (400 MHz, DMSO-d6) δ 10.14 - 9.13 (m, 1H), 8.35 (s, 1H), 7.36 (d, *J* = 8.8 Hz, 1H), 6.88 (d, *J* = 8.8 Hz, 1H), 4.81 - 4.70 (m, 1H), 4.20 - 4.09 (m, 1H), 3.93 (t, *J* = 9.7 Hz, 1H), 3.74 (dd, *J* = 9.8, 7.3 Hz, 1H), 3.25 (d, *J* = 11.2 Hz, 2H), 3.10 - 2.96 (m, 2H), 2.91 (dd, *J* = 20.9, 8.7 Hz, 2H), 1.89 (dt, *J* = 23.8, 10.9 Hz, 4H).

**Example 106. (*S*)-6-(2,3-Dichloro-6-hydroxyphenyl)-2-ethyl-2,5,6,7-tetrahydro-3*H*-pyrrolo[2,1-*c*][1,2,4]triazol-3-one**

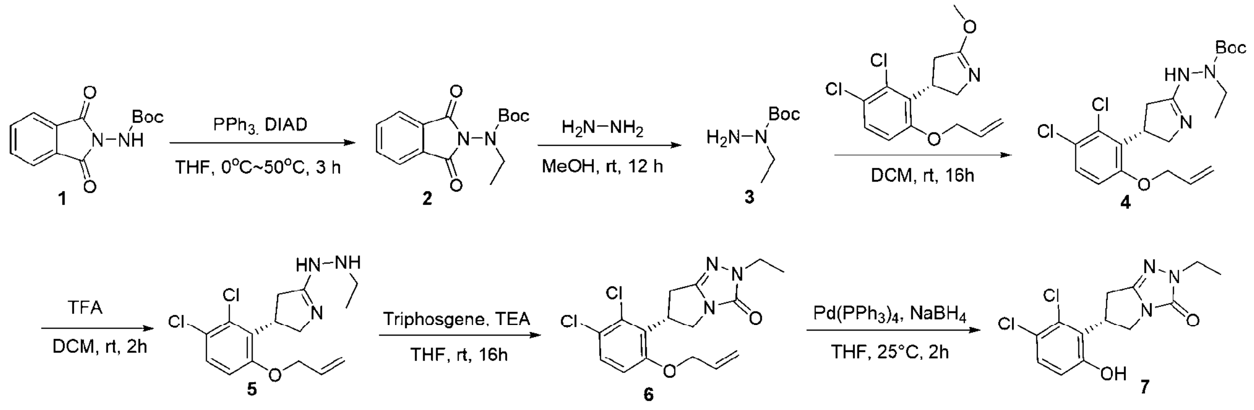


**Synthesis of *tert*-butyl (1,3-dioxoisoindolin-2-yl)(ethyl)carbamate (2):**

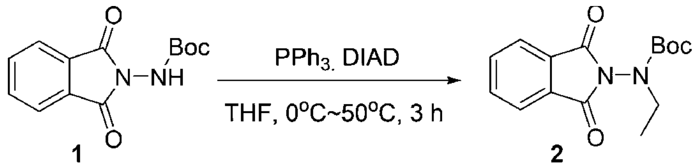


**At room temperature, triphenylphosphine (4.5 g, 17.10 mmol) and ethanol (0.79 g, 17.10 mmol) were added to** a **solution of *tert*-butyl (1,3-dioxoisoindolin-2-yl)carbamate (3.0 g, 11.40 mmol) in tetrahydrofuran (50 mL).** The mixture was cooled to 0 °C, and then diisopropyl azodicarboxylate (3.5 g, 17.10 mmol) was added. The mixture was stirred at 50 °C for 3 h. After completion of the reaction, water (50.0 mL) was added to the reaction mixture, and the resulting mixture was extracted twice with ethyl acetate (50.0 mL). The organic phases were combined and dried over anhydrous sodium sulfate. The solvent was rotary evaporated to dryness by concentration under reduced pressure. The resulting crude product was purified by silica gel chromatography (ethyl acetate:petroleum ether = 1:5) to give a pale yellow solid (4.0 g, yield: 80%).

LC-MS: $[M+H-Boc]^+$: 191.0.

**Synthesis of** *tert*-butyl 1-ethylhydrazine-1-carboxylate (3):

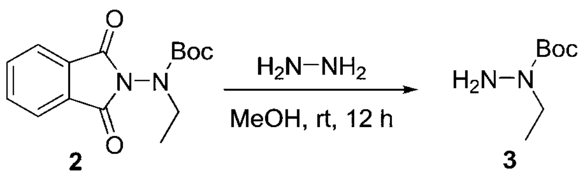


A solution of *tert-butyl* (1,3-dioxoisoindolin-2-yl)(ethyl)carbamate (3.5 g, 12.00 mmol) and hydrazine hydrate (1.15 g, 28.75 mmol, 80% in water) in methanol (40.0 mL) was stirred at room temperature for 12 h. After completion of the reaction, the reaction mixture was filtered, and the filtrate was concentrated under reduced pressure. The resulting crude product was purified by silica gel chromatography (dichloromethane:methanol = 20:1) to give a white solid (1.4 g, yield: 74%).

LC-MS: $[M+H-56]^+$: 105.0.

**Synthesis of *tert*-butyl (*S*)-2-(3-(6-(allyloxy)-2,3-dichlorophenyl)-3,4-dihydro-2*H*-pyrrol-5-yl)-1-ethylhydrazine-1-carboxylate (4):**

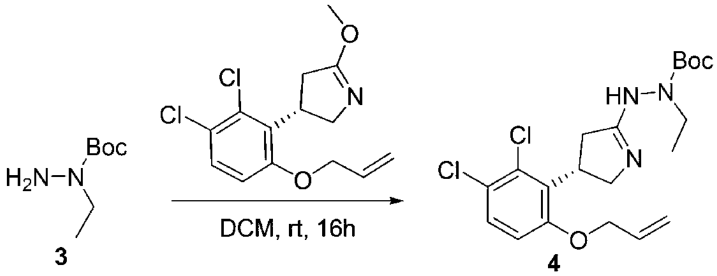


3 4

*tert*-Butyl 1-ethylhydrazine-1-carboxylate (1878.0 mg, 11.70 mmol) was added to a solution of (S)-3-(6-(allyloxy)-2,3-dichlorophenyl)-5-methoxy-3,4-dihydro-2*H*-pyrrole (350.0 mg, 1.17 mmol) in dichloromethane (8.0 mL), and the mixture was stirred at room temperature for 16 h. After completion of the reaction, the solvent was rotary evaporated to dryness by concentration under reduced pressure. The resulting crude product was purified by silica gel chromatography (methanol:dichloromethane = 1:10) to give a white solid (350.0 mg, yield: 82%).

LC-MS: $[M+H]^+$: 428.0.

**Synthesis of (*S*)-3-(6-(allyloxy)-2,3-dichlorophenyl)-5-(2-ethylhydrazino)-3,4-dihydro-2*H*-pyrrole (5):**

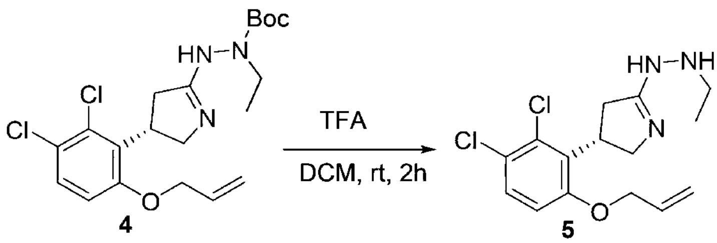


4 5

*tert*-Butyl (*S*)-2-(3-(6-(allyloxy)-2,3-dichlorophenyl)-3,4-dihydro-2*H*-pyrrol-5-yl)-1-ethylhydrazine-1-carboxylate (350.0 mg, 0.81 mmol) was added to a mixed solvent of dichloromethane and trifluoroacetic acid (5:1, 6.0 mL), and the mixture was stirred at room temperature for 2 h. After completion of the reaction, the solvent was rotary evaporated to dryness by concentration under reduced pressure. The resulting crude product (350.0 mg, yield: 80%) was directly used in the next step.

LC-MS: $[M+H]^+$: 328.0.

**Synthesis of (*S*)-6-(6-(allyloxy)-2,3-dichlorophenyl)-2-ethyl-2,5,6,7-tetrahydro-3*H*-pyrrolo[2,1-*c*][1,2,4]triazol-3-one (6):**

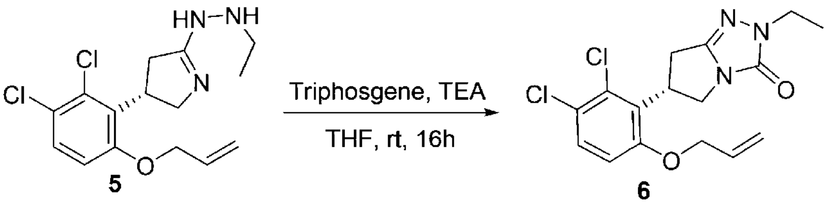


5 6

A solution of (*S*)-3-(6-(allyloxy)-2,3-dichlorophenyl)-5-(2-ethylhydrazino)-3,4-dihydro-2*H*-pyrrole (200.0 mg, 0.61 mmol) and triethylamine (308.0 mg, 3.01 mmol) in tetrahydrofuran (5.0 mL) was cooled to 0 °C in an ice bath. Then, triphosgene (163.0 mg, 0.55 mmol) was slowly added to the above mixture. The mixture was stirred at room temperature for 16 h. After completion of the reaction, water (20.0 mL) was added to the reaction mixture, and the resulting

mixture was extracted twice with ethyl acetate (20.0 mL). The organic phases were combined and dried over anhydrous sodium sulfate. The solvent was rotary evaporated to dryness by concentration under reduced pressure. The resulting crude product was purified by silica gel chromatography (methanol:dichloromethane = 1:10) to give a white solid (80.0 mg, yield: 47%).

LC-MS: $[M+H]^+$: 353.9.

**Synthesis of (*S*)-6-(2,3-dichloro-6-hydroxyphenyl)-2-ethyl-2,5,6,7-tetrahydro-3*H*-pyrrolo[2,1-*c*][1,2,4]triazol-3-one (7):**

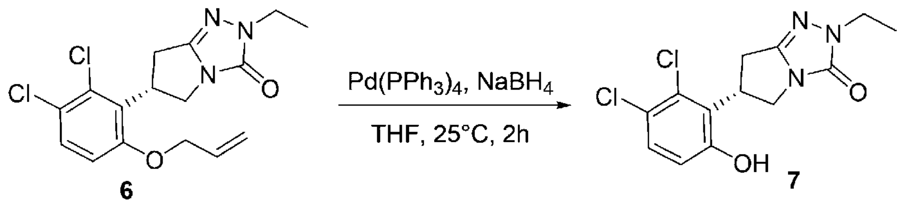


At room temperature, tetrakis(triphenylphosphine)palladium(0) (13 mg, 0.01 mmol) was added to a solution of (*S*)-6-(6-(allyloxy)-2,3-dichlorophenyl)-2-ethyl-2,5,6,7-tetrahydro-3*H*-pyrrolo[2,1-*c*][1,2,4]triazol-3-one (80.0 mg, 0.23 mmol) and sodium borohydride (17.0 mg, 0.45 mmol) in tetrahydrofuran (3.0 mL). The flask was purged using an argon balloon, and the mixture was stirred at 25 °C for 2 h. After completion of the reaction, the reaction mixture was filtered through an organic-phase filter membrane, and the filtrate was rotary evaporated to dryness. The crude product was subjected to preparative chromatography to give a white solid (20.0 mg, yield: 29%).

LC-MS: $[M+H]^+$: 314.0.

$^1H$ NMR (400 MHz, DMSO-d6) δ 10.74 (s, 1H), 7.38 (d, *J* = 8.8 Hz, 1H), 6.84 (d, *J* = 8.8 Hz, 1H), 4.77 (t, *J* = 7.6 Hz, 1H), 3.93 (t, *J* = 9.7 Hz, 1H), 3.74 - 3.69 (m, 1H), 3.67 - 3.62 (m, 2H), 3.09 - 3.02 (m, 1H), 2.94 (dd, *J* = 16.3, 7.6 Hz, 1H), 1.20 (t, *J* = 7.1 Hz, 3H).

**Example 107. (*S*)-6-(2,3-Dichloro-6-hydroxyphenyl)-2-(2,2-difluoroethyl)-2,5,6,7-tetrahydro-3*H*-pyrrolo[2,1-*c*][1,2,4]triazol-3-one**

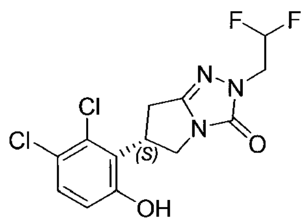

This example was prepared by reference to the preparation method for Example 106, except that the ethanol was replaced by 2,2-difluoroethan-1-ol.

LC-MS: $[M+H]^+$: 349.8.

$^1H$ NMR (400 MHz, DMSO-$d_6$) δ 10.61 (s, 1H), 7.39 (d, *J* = 8.8 Hz, 1H), 6.86 (d, *J* = 8.8 Hz, 1H), 6.25 (tt, *J* = 55.0, 3.7 Hz, 1H), 4.83 - 4.76 (m, 1H), 4.07 (dd, *J* = 15.0, 11.4 Hz, 2H), 3.98 (t, *J* = 9.8 Hz, 1H), 3.73 (dd, *J* = 9.9, 7.0 Hz, 1H), 3.10 (dd, *J* = 16.5, 10.2 Hz, 1H), 2.95 (dd, *J* = 16.5, 7.5 Hz, 1H).

**Example 108. (*S*)-6-(2,3-Dichloro-6-hydroxyphenyl)-2-(tetrahydro-2H-pyran-4-yl)-2,5,6,7-tetrahydro-3*H*-pyrrolo[2,1-*c*][1,2,4]triazol-3-one**

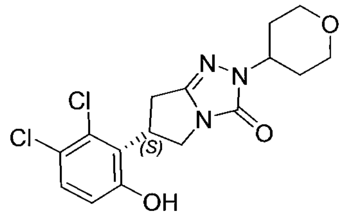

This example was prepared by reference to the preparation method for Example 106, except that the ethanol was replaced by tetrahydro-2*H*-pyran-4-ol.
LC-MS: $[M+H]^+$: 370.1.
$^1$H NMR (400 MHz, DMSO-$d_6$) δ 10.69 (s, 1H), 7.38 (d, *J* = 8.8 Hz, 1H), 6.85 (d, *J* = 8.8 Hz, 1H), 4.77 (dt, *J* = 17.2, 8.7 Hz, 1H), 4.12 (td, *J* = 11.3, 5.6 Hz, 1H), 3.93 (dd, *J* = 17.1, 7.4 Hz, 3H), 3.73 (dd, *J* = 9.7, 7.2 Hz, 1H), 3.42 (t, *J* = 11.9 Hz, 2H), 3.01 (ddd, *J* = 24.1, 16.3, 9.0 Hz, 2H), 1.92 - 1.77 (m, 2H), 1.69 (s, 2H).

Examples 109-110. **(*S*)-6-(2,3-Dichloro-6-hydroxyphenyl)-2-((*S*)-tetrahydrofuran-3-yl)-2,5,6,7-tetrahydro-3*H*-pyrrolo[2,1-*c*][1,2,4]triazol-3-one and (*S*)-6-(2,3-dichloro-6-hydroxyphenyl)-2-((*R*)-tetrahydrofuran-3-yl)-2,5,6,7-tetrahydro-3*H*-pyrrolo[2,1-*c*][1,2,4]triazol-3-one**

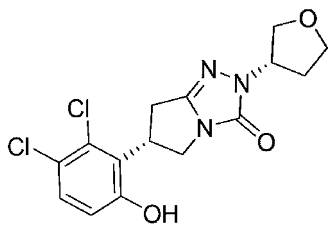
+
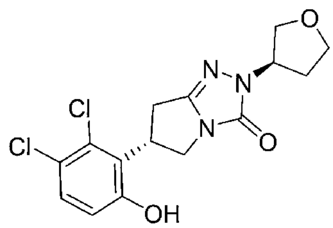

This example was prepared by reference to the preparation method for Example 106, except that the ethanol was replaced by 3-hydroxytetrahydrofuran. The resulting racemate was subjected to preparative chiral chromatography (chromatographic column: CHIRALPAK AD-H 250 mm × 20 mm, 5 μm; mobile phase: 40% EtOH ($NH_4OH$ 0.2%): 60% $CO_2$) to give a pair of enantiomers.
The first compound obtained from the SFC resolution was designated as Example 109, with its RT of 3.26 min analyzed under the analytical chromatographic conditions of SFC.
LC-MS: $[M+H]^+$: 355.8.
$^1$H NMR (400 MHz, DMSO-$d_6$) δ 10.61 (s, 1H), 7.39 (d, *J* = 8.8 Hz, 1H), 6.85 (d, *J* = 8.8 Hz, 1H), 4.83 - 4.70 (m, 2H), 3.98 - 3.85 (m, 3H), 3.82 - 3.68 (m, 3H), 3.01 (dt, *J* = 16.4, 7.5 Hz, 2H), 2.22 - 2.05 (m, 2H).
The second compound obtained from the SFC resolution was designated as Example 110, with its RT of 4.23 min analyzed under the analytical chromatographic conditions of SFC.
LC-MS: $[M+H]^+$: 355.8.
$^1$H NMR (400 MHz, DMSO-$d_6$) δ 10.61 (s, 1H), 7.39 (d, *J* = 8.8 Hz, 1H), 6.85 (d, *J* = 8.8 Hz, 1H), 4.82 - 4.69 (m, 2H), 3.97 - 3.86 (m, 3H), 3.81 - 3.65 (m, 3H), 3.11 - 2.92 (m, 2H), 2.23 - 2.08 (m, 2H).

**Example 111. (*S*)-3,4-Dichloro-2-(3-(2-(trifluoromethyl)pyridin-4-yl)-6,7-dihydro-5*H*-pyrrolo[2,1-*c*][1,2,4]triazol-6-yl)phenol**

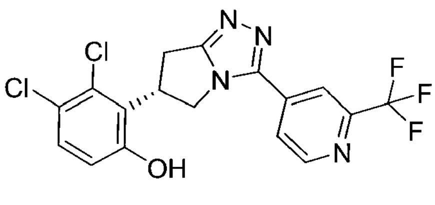

This example was prepared by reference to the preparation method for Example 32, except that the methyl 3-hydroxypropanoate was replaced by methyl 2-(trifluoromethyl)isonicotinate.

LCMS $[M+H]^+$: 414.9.

$^1H$ NMR (400 MHz, DMSO-d6) δ 10.63 (s, 1H), 8.89 (d, *J* = 5.1 Hz, 1H), 8.24 (s, 1H), 8.15 (d, *J* = 5.1 Hz, 1H), 7.41 (d, *J* = 8.8 Hz, 1H), 6.86 (d, *J* = 8.8 Hz, 1H), 5.14 - 5.06 (m, 1H), 4.78 (t, *J* = 10.0 Hz, 1H), 4.48 (dd, *J* = 10.3, 7.2 Hz, 1H), 3.20 (dd, *J* = 16.1, 7.5 Hz, 2H).

**Example 112. 3,4-Dichloro-2-(2-ethyl-2,4,5,6-tetrahydrocyclopenta[c]pyrazol-5-yl)phenol**

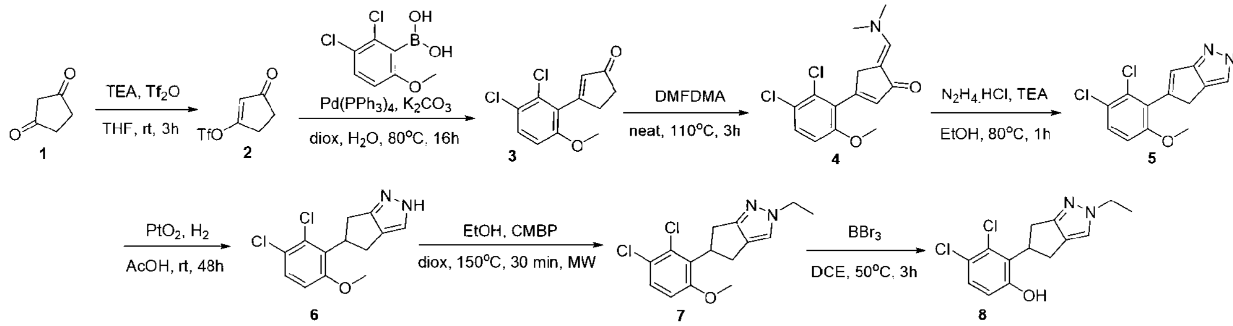


**Synthesis of 3-oxocyclopent-1-en-1-yl trifluoromethanesulfonate (2):**

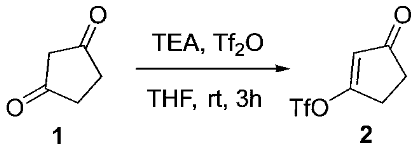


At 0 °C, triethylamine (20.5 g, 0.20 mol) was added to a solution of 1,3-cyclopentanedione (10.0 g, 0.10 mol) in tetrahydrofuran (150.0 mL). After stirring for 10 min, trifluoromethanesulfonic anhydride (34.5 g, 0.12 mol) was slowly added dropwise to the mixture with the temperature maintained at 0 °C. After completion of the dropwise addition, the mixture was warmed to room temperature and stirred for 3 h. After completion of the reaction, as detected by TLC, the mixture was concentrated under reduced pressure at 30 °C to remove the solvent. The residue was wet-loaded and eluted with an eluent (petroleum ether:ethyl acetate = 20:1). The resulting product was concentrated under reduced pressure at 30 °C to remove most of the solvent, and then directly used in the next step.

**Synthesis of 3-(2,3-dichloro-6-methoxyphenyl)cyclopent-2-en-1-one (3):**

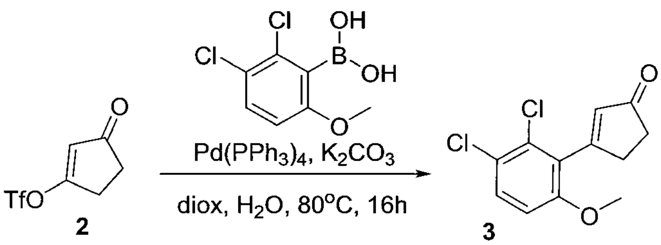


3-Oxocyclopent-1-en-1-yl trifluoromethanesulfonate (20.0 g, 0.09 mol), potassium carbonate (16.2 g, 0.12 mol), and tetrakis(triphenylphosphine)palladium(0) (5.2 g, 0.004 mol) were added to a mixed solution of (2,3-dichloro-6-methoxyphenyl)boronic acid (10.0 g, 0.04 mol) in dioxane:water (5:1, 300 mL). The mixture was stirred at 80 °C for 16 h under a nitrogen atmosphere. After completion of the reaction, the reaction mixture was diluted with water (100.0 mL) and extracted twice with ethyl acetate (100.0 mL). The organic phases were combined, washed with saturated brine (200.0 mL), and dried over anhydrous sodium sulfate. After filtration, the filtrate was concentrated under reduced pressure, and the resulting residue was purified by silica gel column chromatography and eluted (petroleum ether:ethyl acetate = 3:1) to

give the target product (4.0 g, yield: 34%).

LC-MS: $[M+H]^+$: 257.0.

**Synthesis of (*E*)-3-(2,3-dichloro-6-methoxyphenyl)-5-((dimethylamino)methylene)cyclopent-2-en-1-one (4):**

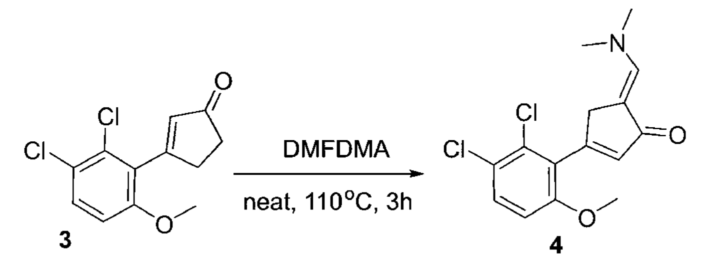


3 4

At room temperature, 3-(2,3-dichloro-6-methoxyphenyl)cyclopent-2-en-1-one (1.2 g, 4.67 mmol) was added to a N,N-dimethylformamide dimethyl acetal solution (16.0 mL). The mixture was stirred at 110 °C for 3 h. After completion of the reaction, the reaction mixture was directly rotary evaporated to dryness to give a crude product, which was directly used in the next step.

LC-MS: $[M+H]^+$: 312.0.

**Synthesis of 5-(2,3-dichloro-6-methoxyphenyl)-2,4-dihydrocyclopenta[c]pyrazole (5):**

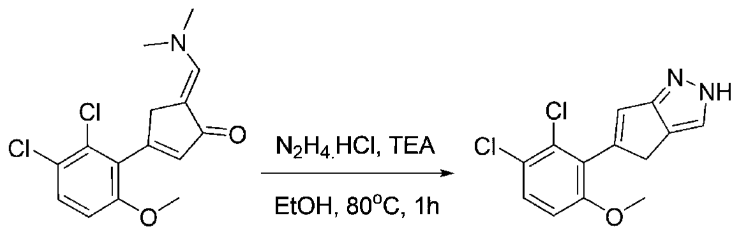


4 5

Triethylamine was added to a solution of (E)-3-(2,3-dichloro-6-methoxyphenyl)-5-((dimethylamino)methylene) cyclopent-2-en-1-one (1.0 g, 3.22 mmol) and hydrazine hydrochloride (0.9 g, 13.13 mmol) in ethanol (20.0 mL) until pH = 8. The mixture was stirred at 80 °C for 1 h. After completion of the reaction, the filtrate was rotary evaporated to dryness by concentration under reduced pressure, and the resulting crude product was purified by silica gel chromatography (dichloromethane:methanol = 20:1) to give a white solid (400.0 mg, yield: 44%).

LC-MS: $[M+H]^+$: 281.0.

**Synthesis of 5-(2,3-dichloro-6-methoxyphenyl)-2,4,5,6-tetrahydrocyclopenta[*c*]pyrazole (6):**

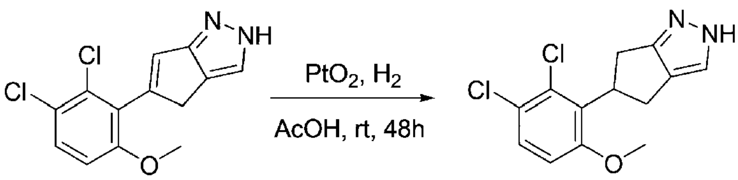


5

Platinum dioxide (100.0 mg, 0.5 w%) was added to a solution of 5-(2,3-dichloro-6-methoxyphenyl)-2,4-dihydrocyclopenta[c]pyrazole (200.0 mg, 0.72 mmol) in acetic acid (5.0 mL), and the mixture was stirred at room temperature for 48 h under a hydrogen atmosphere. After completion of the reaction, the reaction mixture was filtered, and the filtrate was concentrated under reduced pressure. The resulting crude product was a gray solid (130.0 mg, yield: 65%), which was

directly used in the next step.

LC-MS: $[M+H]^+$: 283.0.

**Synthesis of 5-(2,3-dichloro-6-methoxyphenyl)-2-ethyl-2,4,5,6-tetrahydrocyclopenta[c]pyrazole (7):**

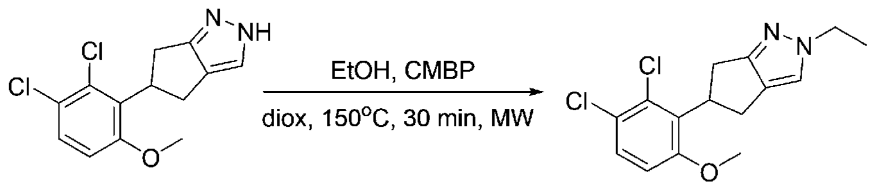


6 7

5-(2,3-Dichloro-6-methoxyphenyl)-2,4,5,6-tetrahydrocyclopenta[c]pyrazole (90.0 mg, 0.32 mmol) was added to dioxane (3.0 mL), and ethanol (146.0 mg, 3.20 mmol) and cyanomethylenetributylphosphorane (384.0 mg, 1.59 mmol) were added to the mixture. The resulting mixture was stirred under microwave at 150 °C for 0.5 h. After completion of the reaction, the solvent was rotary evaporated to dryness by concentration under reduced pressure. The resulting crude product was purified by silica gel chromatography (methanol:dichloromethane = 1:20) to give a white solid (30.0 mg, yield: 61%).

LC-MS: $[M+H]^+$: 311.0.

**Synthesis of 3,4-dichloro-2-(2-ethyl-2,4,5,6-tetrahydrocyclopenta[c]pyrazol-5-yl)phenol (8):**

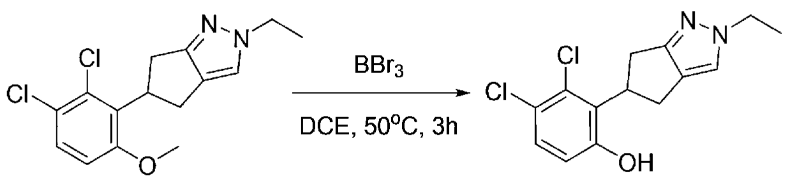


7 8

At room temperature, boron tribromide (121.0 mg, 0.48 mmol) was added to a solution of 5-(2,3-dichloro-6-methoxyphenyl)-2-ethyl-2,4,5,6-tetrahydrocyclopenta[c]pyrazole (30.0 mg, 0.10 mmol) in dichloroethane (2.0 mL). The mixture was stirred at 50 °C for 3 h. After completion of the reaction, the reaction mixture was quenched with ice water (10.0 mL), and the resulting mixture was extracted twice with ethyl acetate (10.0 mL). The organic phases were combined and dried over anhydrous sodium sulfate. The solvent was rotary evaporated to dryness by concentration under reduced pressure, and the crude product was subjected to preparative chromatography to give a white solid (5.4 mg, yield: 19%).

LC-MS: $[M+H]^+$: 296.9.

$^1H$ NMR (400 MHz, DMSO-d6) δ 10.35 (s, 1H), 7.33 (s, 1H), 7.32 (d, *J* = 9.0 Hz, 1H), 6.84 (d, *J* = 8.8 Hz, 1H), 4.54 (t, *J* = 9.1 Hz, 1H), 4.05 (q, *J* = 7.2 Hz, 2H), 3.06 (dd, *J* = 14.6, 9.3 Hz, 1H), 2.97 (dd, *J* = 14.1, 8.9 Hz, 1H), 2.74 (dd, *J* = 14.3, 9.3 Hz, 2H), 1.35 (t, *J* = 7.2 Hz, 3H).

**Examples 113-114. 3,4-Dichloro-2-(3-ethyl-2-methyl-2,4,5,6-tetrahydrocyclopenta[*c*]pyrazol-5-yl)phenol** and **3,4-dichloro-2-(3-ethyl-1-methyl-1,4,5,6-tetrahydrocyclopenta[c]pyrazol-5-yl)phenol**

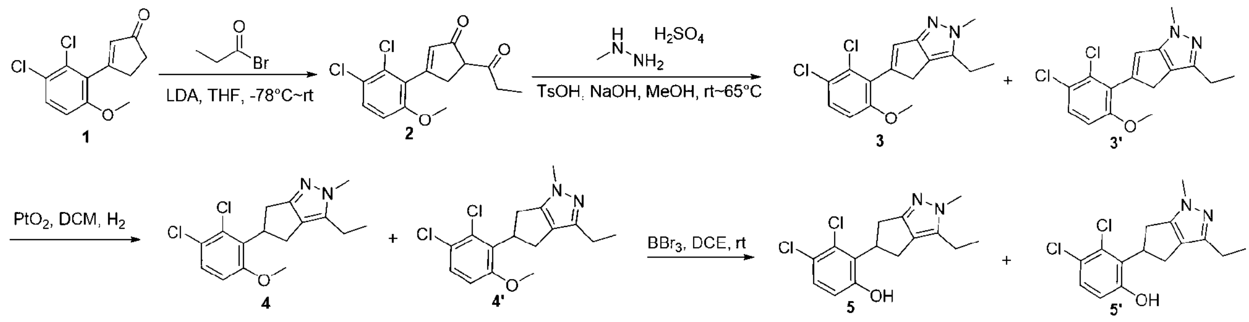

1 2 3 3' 4 4' 5 5'

**Synthesis of 3-(2,3-dichloro-6-methoxyphenyl)-5-propionylcyclopent-2-en-1-one (2):**

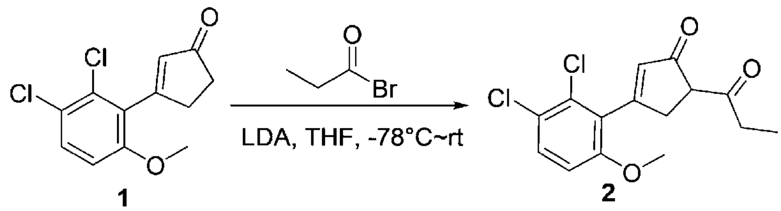

1 2

At -78 °C, *n*-butyllithium (0.45 g, 7.05 mmol) was slowly added dropwise to a solution (40.0 mL) of diisopropylamine (0.71 g, 7.05 mmol) in tetrahydrofuran. The mixture was stirred at -78 °C for 1 h. Then, at -78 °C, the compound 3-(2,3-dichloro-6-methoxyphenyl)cyclopent-2-en-1-one (0.45 g, 1.75 mmol) was added to the above solution, and the mixture was stirred at -78 °C for another 1 h. Finally, at -78 °C, propionyl bromide (0.97 g, 7.05 mmol) was added to the above solution. The mixture was stirred at room temperature for another 16 h. The reaction mixture was extracted three times with ethyl acetate and washed twice with brine. The resulting organic phase was dried over anhydrous sodium sulfate and concentrated *in vacuo.* The resulting crude product was purified by silica gel chromatography (petroleum ether:dichloromethane = 1:1) to give a yellow oily liquid (500.0 mg, yield: 34%).

LC-MS: $[M+H]^+$: 312.8.

**Synthesis of 5-(2,3-dichloro-6-methoxyphenyl)-3-ethyl-2-methyl-2,4-dihydrocyclopenta[c]pyrazole and 5-(2,3-dichloro-6-methoxyphenyl)-3-ethyl-1-methyl-1,4-dihydrocyclopenta[c]pyrazole (3/3'):**

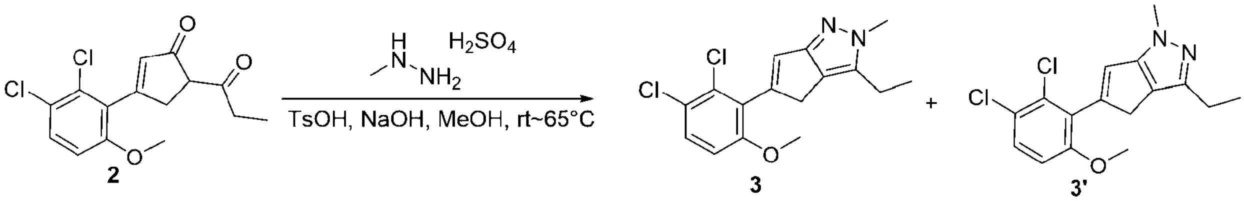

2 3 3'

The compound 3-(2,3-dichloro-6-methoxyphenyl)-5-propionylcyclopent-2-en-1-one (300 mg, 0.96 mmol) and methylhydrazine sulfate (690.4 mg, 4.79 mmol) were dissolved in methanol (12.0 mL). At room temperature, p-toluenesulfonic acid (364.4 mg, 1.92 mmol) and sodium hydroxide (191.6 mg, 4.79 mmol) were added to the mixture. The resulting mixture was stirred at 65 °C for 16 h. The reaction mixture was extracted three times with ethyl acetate and washed twice with brine. The resulting organic phase was dried over anhydrous sodium sulfate and concentrated *in vacuo.* The resulting crude product was purified by silica gel chromatography (petroleum ether:ethyl acetate = 10:1) to give a yellow solid as the mixture of 3 and 3' (220.0 mg, yield: 71%). LC-MS: $[M+H]^+$: 322.9.

**Synthesis of 5-(2,3-dichloro-6-methoxyphenyl)-3-ethyl-2-methyl-2,4,5,6-tetrahydrocyclopenta[c]pyrazole and 5-(2,3-dichloro-6-methoxyphenyl)-3-ethyl-1-methyl-1,4,5,6-tetrahydrocyclopenta[c]pyrazole (4/4'):**

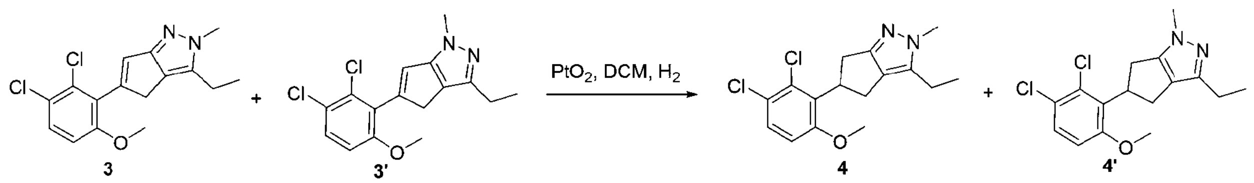


3 3' 4 4'

The compounds 5-(2,3-dichloro-6-methoxyphenyl)-3-ethyl-1-methyl-1,4-dihydrocyclopenta[*c*]pyrazole and 5-(2,3-dichloro-6-methoxyphenyl)-3-ethyl-2-methyl-2,4-dihydrocyclopenta[c]pyrazole (220.0 mg, 0.68 mmol) were dissolved in a dichloromethane solution (22.0 mL), and platinum dioxide (108.2 mg, 0.48 mmol) was added to the reaction mixture. The resulting mixture was allowed to react at room temperature for 16 h under a hydrogen atmosphere. The reaction mixture was filtered through a filter membrane, and the resulting crude product was purified by silica gel chromatography (petroleum ether:ethyl acetate = 1:1) to give a pale yellow solid as the mixture of 4 and 4' (180.0 mg, yield: 81%).

LC-MS: $[M+H]^+$: 324.9.

**Synthesis of 3,4-dichloro-2-(3-ethyl-2-methyl-2,4,5,6-tetrahydrocyclopenta[c]pyrazol-5-yl)phenol and 3,4-dichloro-2-(3-ethyl-1-methyl-1,4,5,6-tetrahydrocyclopenta[*c*]pyrazol-5-yl)phenol (5/5'):**

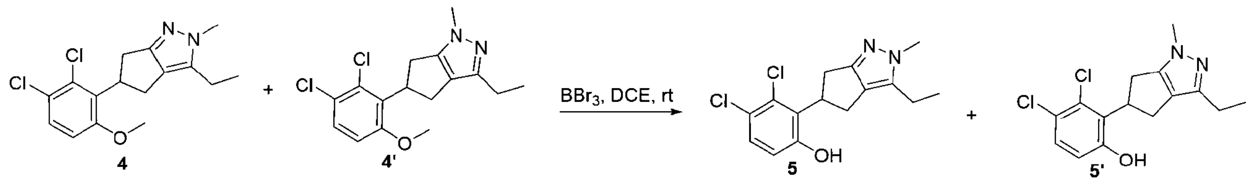


4 4' 5 5'

The compounds 5-(2,3-dichloro-6-methoxyphenyl)-3-ethyl-2-methyl-2,4,5,6-tetrahydrocyclopenta[c]pyrazole and 5-(2,3-dichloro-6-methoxyphenyl)-3-ethyl-1-methyl-1,4,5,6-tetrahydrocyclopenta[*c*]pyrazole (200.0 mg, 0.61 mmol) were dissolved in a dichloroethane solution (8.0 mL), and boron tribromide (770.2 mg, 3.07 mmol) was added to the reaction mixture. The resulting mixture was allowed to react at 50 °C for 6 h. The reaction mixture was quenched with ice water, and the pH was adjusted to 8 with a saturated aqueous sodium bicarbonate solution. The mixture was extracted three times with ethyl acetate and washed twice with brine. The resulting organic phase was dried over anhydrous sodium sulfate and concentrated *in vacuo,* and the resulting crude product was separated by preparative chromatography to give a white solid as Compound 5 (Example 113) (5.3 mg, yield: 3%).

LC-MS: $[M+H]^+$: 311.0.

$^1H$ NMR (400 MHz, DMSO-*d6*) δ 10.23 (s, 1H), 7.31 (d, *J* = 8.8 Hz, 1H), 6.84 (d, *J* = 8.8 Hz, 1H), 4.53 (dd, *J* = 18.1, 9.0 Hz, 1H), 3.65 (s, 3H), 3.05 - 2.93 (m, 2H), 2.76 (dd, *J* = 13.7, 9.2 Hz, 1H), 2.70 - 2.63 (m, 1H), 2.58 (d, *J* = 7.6 Hz, 2H), 1.16 (t, *J* = 7.6 Hz, 3H).

Further, a white solid as Compound 5' (Example 114) (30.0 mg, yield: 16%) was also obtained.

LC-MS: $[M+H]^+$: 311.0.

**Examples 115-116. (*S*)-3,4-Dichloro-2-(3-ethyl-1-methyl-1,4,5,6-tetrahydrocyclopenta[*c*]pyrazol-5-yl)phenol and (*R*)-3,4-dichloro-2-(3-ethyl-1-methyl-1,4,5,6-tetrahydrocyclopenta[c]pyrazol-5-yl)phenol:**

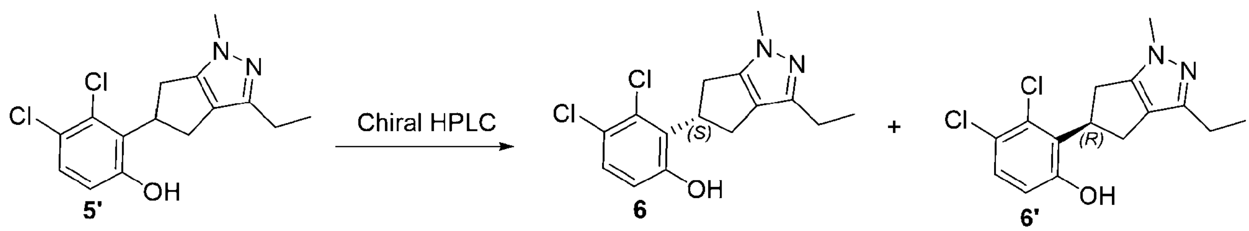


5' 6 6'

3,4-Dichloro-2-(3-ethyl-1-methyl-1,4,5,6-tetrahydrocyclopenta[c]pyrazol-5-yl)phenol (30.0 mg, 0.097 mmol) was subjected to preparative chiral chromatography (chromatographic column: CHIRALPAK AD-H 250 mm × 20

mm, 5 μm; mobile phase: 40% EtOH ($NH_4OH$ 0.2%): 60% $CO_2$) to give a pair of enantiomers.

The first compound obtained from the SFC resolution was designated as Example 115 (11.9 mg, yield: 40%), with its RT of 5.551 min analyzed under the analytical chromatographic conditions of SFC.

LC-MS: $[M+H]^+$: 311.0.

$^1H$ NMR (400 MHz, DMSO-$d_6$) δ 10.24 (s, 1H), 7.32 (d, *J* = 8.8 Hz, 1H), 6.84 (d, *J* = 8.8 Hz, 1H), 4.71 (dd, *J* = 17.7, 8.9 Hz, 1H), 3.60 (s, 3H), 3.06 (dd, *J* = 14.7, 8.8 Hz, 1H), 2.85 (ddd, *J* = 24.1, 14.1, 9.0 Hz, 2H), 2.69 (dd, *J* = 13.3, 9.1 Hz, 1H), 2.47 - 2.41 (m, 2H), 1.11 (t, *J*= 7.6 Hz, 3H).

The second compound obtained from the SFC resolution was designated as Example 116 (12.5 mg, yield: 42%), with its RT of 5.528 min analyzed under the analytical chromatographic conditions of SFC.

LC-MS: $[M+H]^+$: 311.0.

$^1H$ NMR (400 MHz, DMSO-$d_6$) δ 10.22 (s, 1H), 7.32 (d, *J* = 8.8 Hz, 1H), 6.84 (d, *J* = 8.8 Hz, 1H), 4.71 (dd, *J* = 17.8, 8.9 Hz, 1H), 3.60 (s, 3H), 3.06 (dd, *J* = 14.7, 8.8 Hz, 1H), 2.85 (ddd, *J* = 24.0, 14.0, 8.9 Hz, 2H), 2.69 (dd, *J* = 13.2, 9.1 Hz, 1H), 2.44 (q, *J* = 7.6 Hz, 2H), 1.11 (t, *J* = 7.6 Hz, 3H).

**Examples 117-118. (*S*)-3,4-Dichloro-2-(1-ethyl-1,4,5,6-tetrahydrocyclopenta[*c*]pyrazol-5-yl)phenol and (*R*)-3,4-dichloro-2-(1-ethyl-1,4,5,6-tetrahydrocyclopenta[c]pyrazol-5-yl)phenol**

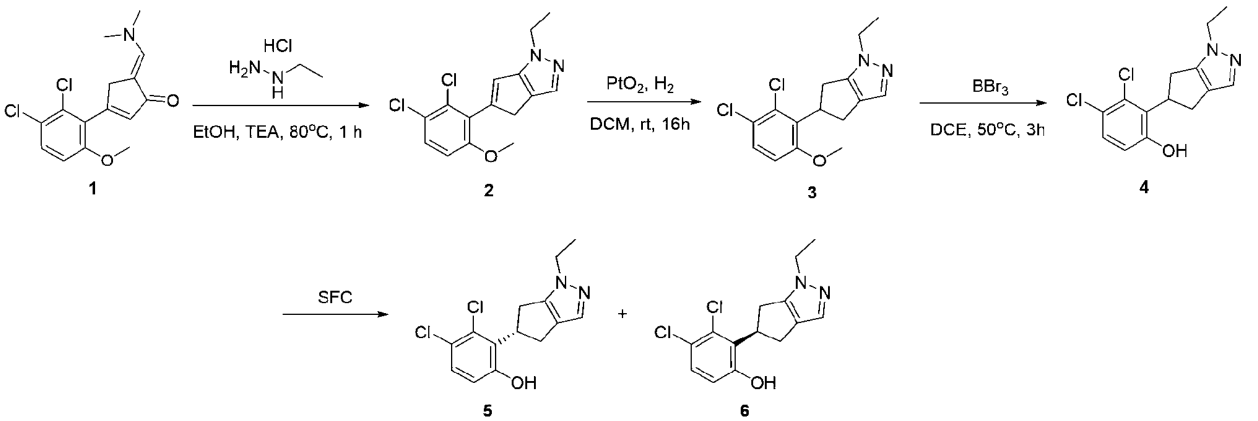


1 2 3 4

5 6

**Synthesis of 5-(2,3-dichloro-6-methoxyphenyl)-1-ethyl-1,4-dihydrocyclopenta[*c*]pyrazole (2):**

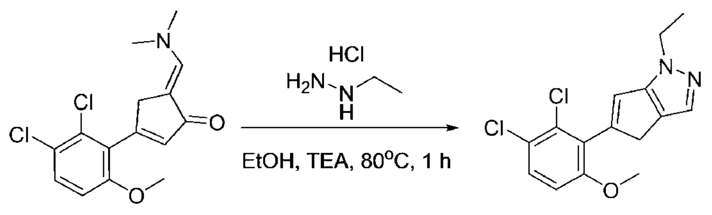


1 2

Triethylamine was added to a solution of (*E*)-3-(2,3-dichloro-6-methoxyphenyl)-5-((dimethylamino)methylene) cyclopent-2-en-1-one (300.0 mg, 0.96 mmol) and ethylhydrazine hydrochloride (463.0 mg, 4.80 mmol) in ethanol (5.0 mL) until pH = 8. The mixture was stirred at 80 °C for 1 h. After completion of the reaction, the filtrate was rotary evaporated to dryness by concentration under reduced pressure, and the resulting crude product was purified by silica gel chromatography (petroleum ether:ethyl acetate = 2:1) to give a white solid (70.0 mg, yield: 14%).

LC-MS: $[M+H]^+$: 309.0.

**Synthesis of 5-(2,3-dichloro-6-methoxyphenyl)-1-ethyl-1,4,5,6-tetrahydrocyclopenta[c]pyrazole (3):**

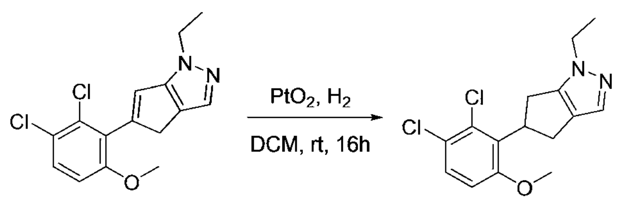


2 3

Platinum dioxide (35.0 mg, 0.5 w%) was added to a solution of 5-(2,3-dichloro-6-methoxyphenyl)-1-ethyl-1,4-dihydrocyclopenta[c]pyrazole (70.0 mg, 0.23 mmol) in dichloromethane (3.0 mL), and the mixture was stirred at room temperature for 16 h. After completion of the reaction, the filtrate obtained by filtration was concentrated under reduced pressure. The resulting crude product was a gray solid (50.0 mg, yield: 71%), which was directly used in the next step.

LC-MS: $[M+H]^+$: 311.0.

**Synthesis of 3,4-dichloro-2-(1-ethyl-1,4,5,6-tetrahydrocyclopenta[c]pyrazol-5-yl)phenol (4):**

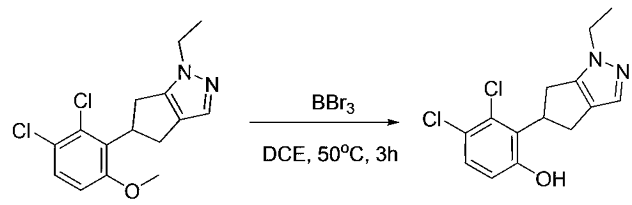


3 4

At room temperature, boron tribromide (644.0 mg, 2.57 mmol) was added to a solution of 5-(2,3-dichloro-6-methoxyphenyl)-1-ethyl-1,4,5,6-tetrahydrocyclopenta[c]pyrazole (160.0 mg, 0.51 mmol) in dichloroethane (5.0 mL). The mixture was stirred at 50 °C for 3 h. After completion of the reaction, the reaction mixture was quenched with ice water (10.0 mL), and the pH was adjusted to about 7 with a saturated aqueous sodium bicarbonate solution. The resulting mixture was extracted twice with ethyl acetate (10.0 mL). The organic phases were combined and dried over anhydrous sodium sulfate. The crude product was purified by preparative chromatography to give a white solid (40.0 mg, yield: 26%).

LC-MS: $[M+H]^+$: 297.0.

**Preparation of (*S*)-3,4-dichloro-2-(1-ethyl-1,4,5,6-tetrahydrocyclopenta[*c*]pyrazol-5-yl)phenol and (*R*)-3,4-dichloro-2-(1-ethyl-1,4,5,6-tetrahydrocyclopenta[c]pyrazol-5-yl)phenol (5/6):**

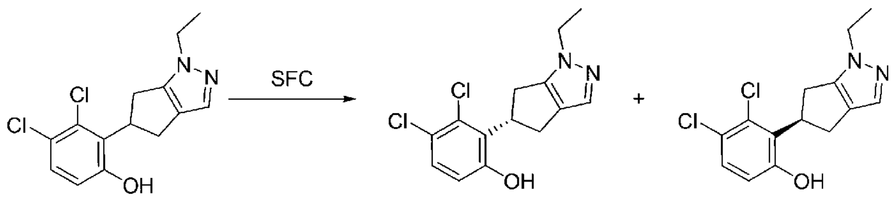


4 5 6

3,4-Dichloro-2-(1-ethyl-1,4,5,6-tetrahydrocyclopenta[*c*]pyrazol-5-yl)phenol (40.0 mg, 0.13 mmol) was subjected to preparative chiral chromatography (chromatographic column: CHIRALPAK AD-H 250 mm × 20 mm, 5 μm; mobile phase: 40% EtOH ($NH_4OH$ 0.2%): 60% $CO_2$) to give a pair of enantiomers.

The first compound obtained from the SFC resolution was designated as Example 117 (14.2 mg, yield: 36%), with its RT of 1.78 min analyzed under the analytical chromatographic conditions of SFC.

LC-MS: $[M+H]^+$: 297.0.

$^1$H NMR (400 MHz, DMSO-$d_6$) δ 10.24 (s, 1H), 7.33 (d, *J* = 8.8 Hz, 1H), 7.08 (s, 1H), 6.84 (d, *J* = 8.8 Hz, 1H), 4.73 (p, *J* = 8.7 Hz, 1H), 3.99 (q, *J* = 7.2 Hz, 2H), 3.09 (dd, *J* = 14.4, 8.5 Hz, 1H), 2.90 (dd, *J* = 12.9, 8.6 Hz, 2H), 2.72 (dd, *J* = 13.3, 9.2 Hz, 1H), 1.31 (t, *J* = 7.2 Hz, 3H).

The second compound obtained from the SFC resolution was designated as Example 118 (16.0 mg, yield: 40%),

with its RT of 2.19 min analyzed under the analytical chromatographic conditions of SFC.

LC-MS: $[M+H]^+$: 297.0.

$^1H$ NMR (400 MHz, DMSO-$d_6$) δ 10.24 (s, 1H), 7.33 (d, *J* = 8.8 Hz, 1H), 7.08 (s, 1H), 6.84 (d, *J* = 8.8 Hz, 1H), 4.73 (p, *J* = 8.7 Hz, 1H), 3.99 (q, *J* = 7.3 Hz, 2H), 3.09 (dd, *J* = 14.2, 8.8 Hz, 1H), 2.90 (dd, *J* = 13.3, 8.5 Hz, 2H), 2.72 (dd, *J* = 13.6, 9.5 Hz, 1H), 1.31 (t, *J* = 7.3 Hz, 3H).

**Example 119. 3,4-Dibromo-2-(3-((*S*)-tetrahydrofuran-3-yl)-6,7-dihydro-5*H*-pyrrolo[2,1-*c*][1,2,4]triazol-6-yl)phenol**

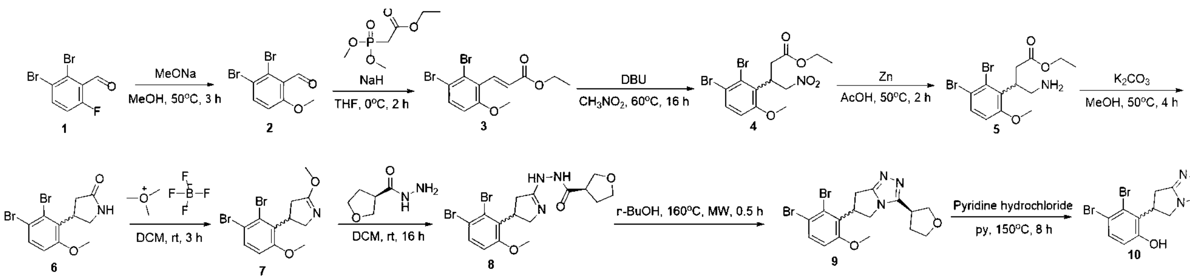


**Synthesis of 2,3-dibromo-6-methoxybenzaldehyde (2):**

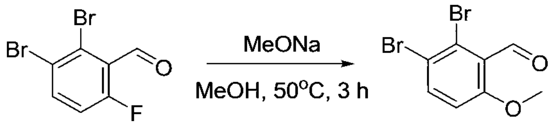


1 2

At room temperature, a solution of sodium methoxide in methanol (7.14 mL, 35.71 mmol, 5 M in methanol) was added to a solution of 2,3-dibromo-6-fluorobenzaldehyde (5.0 g, 17.86 mmol) in methanol (60.0 mL). The mixture was stirred at 50 °C for 3 h. After completion of the reaction, the reaction mixture was cooled to room temperature, diluted with water (100.0 mL), and extracted twice with ethyl acetate (200.0 mL). The organic phases were combined, washed with saturated brine (100.0 mL), and dried over anhydrous sodium sulfate. After filtration, the filtrate was concentrated under reduced pressure. The resulting crude product was purified by silica gel chromatography (ethyl acetate:petroleum ether = 1:1) to give a white solid (3.7 g, yield: 71%). LC-MS: $[M+H]^+$: 292.7.

**Synthesis of ethyl (E)-3-(2,3-dibromo-6-methoxyphenyl)acrylate** (3):

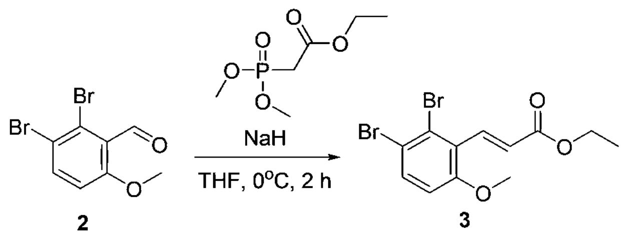


2 3

At 0 °C, sodium hydride (0.6 g, 15.21 mmol, 60% in mineral oil) was added to a solution of ethyl 2-(dimethoxyphosphoryl)acetate (3.0 g, 15.21 mmol) in tetrahydrofuran (80.0 mL). The mixture was stirred for 30 min, and then 2,3-dibromo-6-methoxybenzaldehyde (3.7 g, 12.67 mmol) was added. The resulting mixture was stirred at 0 °C for 2 h. After completion of the reaction, the reaction mixture was diluted with water (100.0 mL) and extracted with ethyl acetate (200.0 mL), and the organic phase was washed with saturated brine (100.0 mL), dried over anhydrous sodium sulfate, and filtered. The filtrate was concentrated under reduced pressure, and the resulting crude product was purified by silica gel

chromatography (ethyl acetate:petroleum ether = 1:8) to give a white solid (4.2 g, yield: 91%).

LC-MS: $[M+H]^+$: 362.7.

$^1H$ NMR (400 MHz, DMSO-$d_6$) δ 7.81 (d, *J* = 16.1 Hz, 1H), 7.76 - 7.71 (m, 1H), 7.13 (d, *J* = 9.1 Hz, 1H), 6.67 *(d, J*= 16.1 Hz, 1H), 4.20 (q, *J* = 7.1 Hz, 2H), 3.88 (s, 3H), 1.26 (t, *J* = 7.1 Hz, 3H).

Synthesis of ethyl **3-(2,3-dibromo-6-methoxyphenyl)-4-nitrobutanoate (4):**

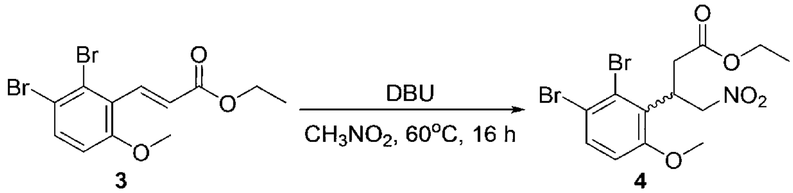


3 4

At room temperature, ethyl (*E*)-3-(2,3-dibromo-6-methoxyphenyl)acrylate (4.2 g, 11.60 mmol) and 1,8-diazabicyclo[5.4.0]undec-7-ene (2.1 g, 13.92 mmol) were added to a reaction flask containing nitromethane (40.0 mL). The reaction mixture was stirred at 60 °C for 16 h. After completion of the reaction, the reaction mixture was diluted with water (50.0 mL) and extracted with ethyl acetate (100 mL). The organic phase was washed with saturated brine (100 mL), dried over anhydrous sodium sulfate, and filtered. The filtrate was concentrated under reduced pressure, and the resulting crude product was purified by silica gel chromatography (ethyl acetate:petroleum ether = 1:8) to give a colorless oily subtance (3.9 g, yield: 79%).

LC-MS: $[M+H+Na]^+$: 445.7.

Synthesis of ethyl 4-amino-3-(2,3-dibromo-6-methoxyphenyl)butanoate (5):

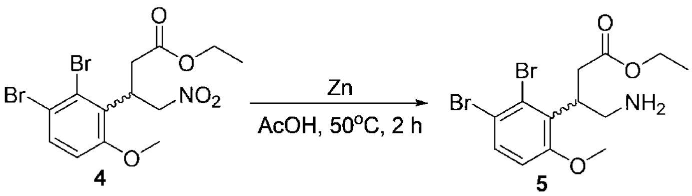


4 5

At room temperature, ethyl 3-(2,3-dibromo-6-methoxyphenyl)-4-nitrobutanoate (3.9 g, 9.22 mmol) and zinc powder (6.0 g, 92.20 mmol) were added to a reaction flask containing glacial acetic acid (41.7 mL). The mixture was stirred at 50 °C for 2 h. After completion of the reaction, the reaction mixture was filtered, and the filter cake was washed with methanol. The filtrate was collected and concentrated under reduced pressure to give a colorless oily subtance (3.6 g, yield: 100%), which was directly used in the next step without purification.

LC-MS: $[M+H]^+$: 393.7.

**Synthesis of 4-(2,3-dibromo-6-methoxyphenyl)pyrrolidin-2-one** (6):

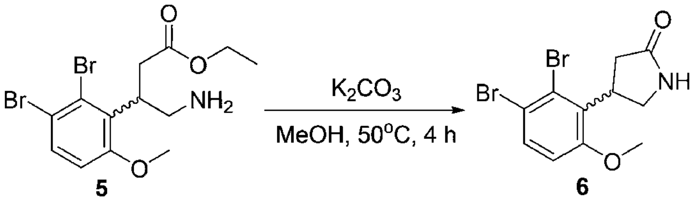


5 6

Ethyl 4-amino-3-(2,3-dibromo-6-methoxyphenyl)butanoate (3.6 g, 9.16 mmol) and potassium carbonate (6.3 g, 45.80 mmol) were added to methanol (100.0 mL), and the mixture was stirred at 50 °C for 4 h. After completion of the reaction, the reaction mixture was diluted with water (100.0 mL) and extracted three times with ethyl acetate (200.0 mL).

The organic phases were combined and then dried over anhydrous sodium sulfate. After filtration, the filtrate was concentrated under reduced pressure, and the resulting crude product was purified by silica gel chromatography (ethyl acetate) to give a colorless oily subtance (0.5 g, yield: 16%).

LC-MS: $[M+H]^+$: 347.7.

$^1H$ NMR (400 MHz, DMSO-$d_6$) δ 7.71 (s, 1H), 7.64 (d, *J* = 8.9 Hz, 1H), 7.06 (d, *J* = 9.0 Hz, 1H), 4.41 - 4.30 (m, 1H), 3.82 (s, 3H), 3.44 (t, *J* = 9.3 Hz, 1H), 3.30 (d, *J* = 8.5 Hz, 1H), 2.49 - 2.42 (m, 1H), 2.34 (dd, *J* = 16.6, 10.7 Hz, 1H).

Synthesis of 3-(2,3-dibromo-6-methoxyphenyl)-5-methoxy-3,4-dihydro-2H-pyrrole (7):

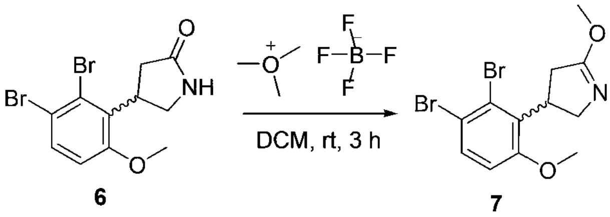


4-(2,3-Dibromo-6-methoxyphenyl)pyrrolidin-2-one (500.0 mg, 1.44 mmol) was dissolved in dichloromethane (5.0 mL), and trimethyloxonium tetrafluoroborate (255.7 mg, 1.73 mmol) was added. The mixture was stirred at room temperature for 3 h under a nitrogen atmosphere. After completion of the reaction, a solution of the target product in dichloromethane (about 520.0 mg, 100%) was obtained and directly used in the next step without purification.

LC-MS: $[M+H]^+$: 361.9.

Synthesis of (*R*)-*N'*-(3-(2,3-dibromo-6-methoxyphenyl)-3,4-dihydro-2*H*-pyrrol-5-yl)tetrahydrofuran-3-carbohydrazide (8):

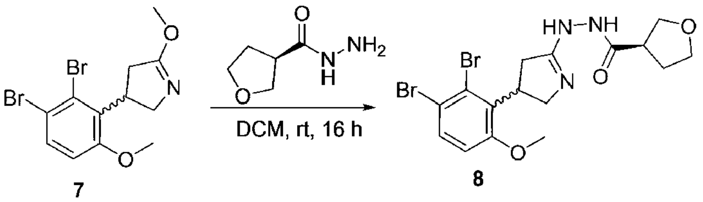


At room temperature, (R)-tetrahydrofuran-3-carbohydrazide (224.7 mg, 1.73 mmol) was added to a solution of 3-(2,3-dibromo-6-methoxyphenyl)-5-methoxy-3,4-dihydro-2H-pyrrole (520.0 mg, 1.44 mmol) in dichloromethane (5.0 mL). The resulting mixture was stirred at room temperature for 16 h. After completion of the reaction, the reaction mixture was concentrated under reduced pressure. The resulting crude product was purified by silica gel chromatography (methanol:dichloromethane = 1:15) to give a white solid (560.0 mg, yield: 85%).

LC-MS: $[M+H]^+$: 459.8.

Synthesis of (S)-6-(2,3-dibromo-6-methoxyphenyl)-3-(tetrahydrofuran-3-yl)-6,7-dihydro-5H-pyrrolo[2,1-***c*][1,2,4]triazole** (9):

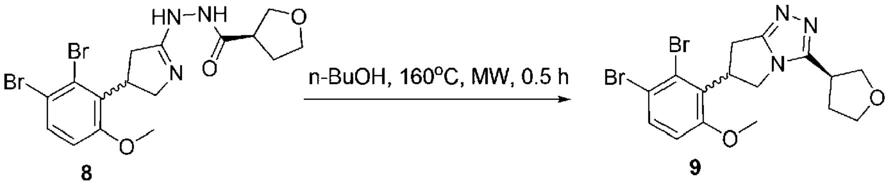


A solution of (*R*)-*N'*-(3-(2,3-dibromo-6-methoxyphenyl)-3,4-dihydro-2*H*-pyrrol-5-yl)tetrahydrofuran-3-carbohydrazide (560.0 mg, 1.22 mmol) in n-butanol (11.2 mL) was stirred under microwave at 160 °C for 0.5 h. After completion of the reaction, the reaction mixture was cooled to room temperature and then the solvent was rotary evaporated to dryness by concentration under reduced pressure. The resulting crude product was purified by silica gel chromatography (methanol:dichloromethane = 1:10) to give a white solid (400.0 mg, yield: 74%).

LC-MS: $[M+H]^+$: 441.9.

Synthesis of **3,4-dibromo-2-(3-((S)-tetrahydrofuran-3-yl)-6,7-dihydro-5H-pyrrolo[2,1-c][1,2,4]triazol-6-**yl)phenol (10):

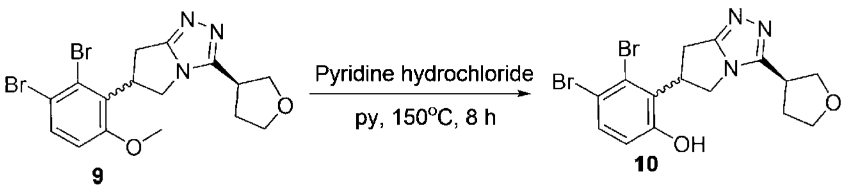


9 10

At room temperature, (*S*)-6-(2,3-dibromo-6-methoxyphenyl)-3-(tetrahydrofuran-3-yl)-6,7-dihydro-5*H*-pyrrolo [2,1-c][1,2,4]triazole (200.0 mg, 0.45 mmol) and pyridine hydrochloride (262.0 mg, 2.27 mmol) were added to a solution in a reaction flask, and pyridine (0.2 mL) was added as a co-solvent. The resulting mixture was stirred at 150 °C for 8 h. After completion of the reaction, the mixture was diluted by adding water (5.0 mL) and extracted three times with ethyl acetate (10.0 mL). The organic phases were combined, then dried over anhydrous sodium sulfate, and concentrated under reduced pressure. The resulting crude product was subjected to preparative chromatography to give a pale yellow solid (7.2 mg, yield: 4%).

LC-MS: $[M+H]^+$: 427.8.

$^1$H NMR (400 MHz, DMSO-$d_6$) δ 10.74 (s, 1H), 7.51 (d, *J* = 8.7 Hz, 1H), 6.82 (d, *J* = 8.7 Hz, 1H), 5.07 - 4.95 (m, 1H), 4.23 (t, *J* = 9.8 Hz, 1H), 4.07 - 3.95 (m, 2H), 3.87 (dt, *J* = 8.2, 5.7 Hz, 1H), 3.81 - 3.72 (m, 2H), 3.58 - 3.48 (m, 1H), 3.07 (ddd, *J* = 23.8, 15.9, 9.0 Hz, 2H), 2.31 - 2.20 (m, 1H), 2.18 - 2.05 (m, 1H).

Example 120. (S)-4-Bromo-3-chloro-2-(3-(tetrahydrofuran-3-yl)-6,7-dihydro-SH-pyrrolo[2,1-c][1,2,4]triazol-6-yl)phenol

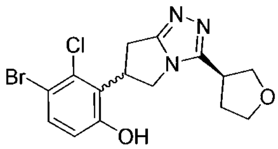

This example was prepared by reference to the preparation method for Example 119, except that the 2,3-dibromo-6-fluorobenzaldehyde was replaced by 3-bromo-2-chloro-6-fluorobenzaldehyde.

LCMS $[M+H]^+$: 384.1.

$^1$H NMR (400 MHz, $CD_3OD$) δ 7.43 (d, *J* = 8.8 Hz, 1H), 6.72 (d, *J* = 8.8 Hz, 1H), 5.18 (dd, *J* = 12.3, 4.7 Hz, 1H), 4.38 - 4.27 (m, 1H), 4.20 (dd, *J* = 10.0, 7.1 Hz, 1H), 4.04 (dtd, *J* = 25.8, 8.2, 4.9 Hz, 2H), 3.93 - 3.81 (m, 2H), 3.68 - 3.58 (m, 1H), 3.20 (t, *J* = 9.0 Hz, 2H), 2.37 (dd, *J* = 8.3, 4.4 Hz, 1H), 2.23 - 2.13 (m, 1H).

Example 121. **(*S*)-(6-(2,3-Dichloro-6-hydroxyphenyl)-6,7-dihydro-5*H*-pyrrolo[1,2-*a*]imidazol-3-yl)(4-**isopropylpiperazin-1-yl)methanone

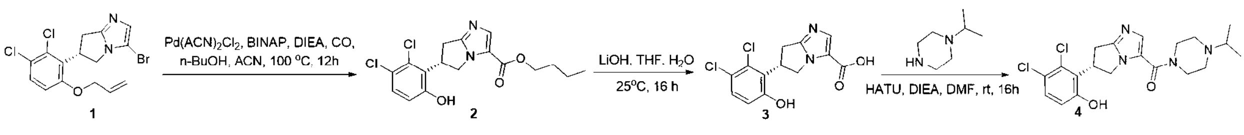


1 2 3 4

Synthesis of butyl **(*S*)-6-(2,3-dichloro-6-hydroxyphenyl)-6,7-dihydro-5*H*-pyrrolo[1,2-*a*]imidazole-3-**carboxylate (2):

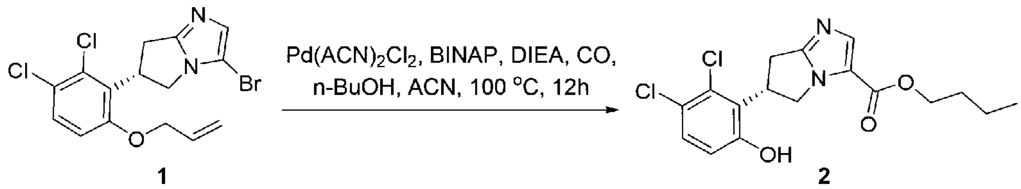


A solution of (*S*)-6-(6-(allyloxy)-2,3-dichlorophenyl)-3-bromo-6,7-dihydro-5*H*-pyrrolo[1,2-*a*]imidazole (150.0 mg, 0.38 mmol), bis(acetonitrile)dichloropalladium(II) (20.0 mg, 0.07 mmol), 2,2'-bis(diphenylphosphino)-1,1'-binaphthyl (96.2 mg, 0.15 mmol), and N,N-diisopropylethylamine (149.8 mg, 1.15 mmol) in n-butanol:acetonitrile (3/3 mL) was stirred at 100 °C for 12 h under a carbon monoxide atmosphere. The solvent was removed by distillation under reduced pressure. The resulting crude product was purified by silica gel chromatography (methanol:dichloromethane = 1:10) to give a white solid (20.0 mg, yield: 14%).
LC-MS: $[M+H]^+$: 369.1.

Synthesis of **(*S*)-6-(2,3-dichloro-6-hydroxyphenyl)-6,7-dihydro-5*H*-pyrrolo[1,2-*a*]imidazole-3-carboxylic** acid (3):

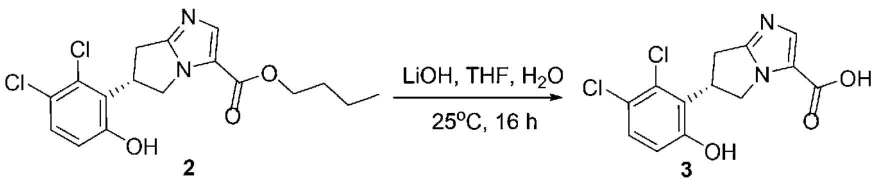


A solution of butyl (*S*)-6-(2,3-dichloro-6-hydroxyphenyl)-6,7-dihydro-5*H*-pyrrolo[1,2-*a*]imidazole-3-carboxylate (20.0 mg, 0.05 mmol) and lithium hydroxide (11.3 mg, 0.27 mmol) in tetrahydrofuran:water (2/0.4 mL) was stirred at 25 °C for 16 h. The reaction mixture was adjusted to pH = 3-4 with a 1 M hydrochloric acid solution, extracted three times with ethyl acetate, and washed twice with brine. The resulting organic phase was dried over anhydrous sodium sulfate and concentrated *in vacuo,* and the resulting crude product was purified by silica gel chromatography (dichloromethane:methanol = 10:1) to give a white solid (15.0 mg, yield: 88%).
LC-MS: $[M+H]^+$: 313.1.

Synthesis of **(*S*)-(6-(2,3-dichloro-6-hydroxyphenyl)-6,7-dihydro-5*H*-pyrrolo[1,2-*a*]imidazol-3-yl)(4-**isopropylpiperazin-1-yl)methanone (4):

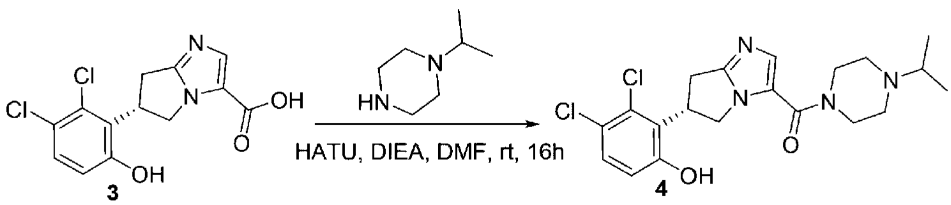


The compound (*S*)-6-(2,3-dichloro-6-hydroxyphenyl)-6,7-dihydro-5*H*-pyrrolo[1,2-*a*]imidazole-3-carboxylic acid (15.0 mg, 0.04 mmol) and 1-isopropylpiperazine (6.1 mg, 0.04 mmol) were dissolved in N,N-dimethylformamide (2.0 mL). At room temperature, N,N-diisopropylethylamine (12.3 mg, 0.09 mmol) and *O*-(7-azabenzotriazol-1-yl)-*N*,*N*,*N'*,*N'*-tetramethyluronium hexafluorophosphate (20.0 mg, 0.05 mmol) were added to the mixture. The resulting mixture was stirred at room temperature for 16 h. The reaction mixture was diluted with ethyl acetate and washed twice with brine. The resulting organic phase was dried over anhydrous sodium sulfate and concentrated *in vacuo.* The crude product was subjected to preparative chromatography to give a white solid (1.0 mg, yield: 5%).

LC-MS: [M+H]+: 423.2.

$^1$H NMR (400 MHz, DMSO) δ 8.34 (s, 1H), 7.37 (d, J=8.7, 1H), 7.32 (s, 1H), 6.84 (d, *J*=8.8, 1H), 4.85 - 4.79 (m, 1H), 4.34 (t, *J*=10.3, 2H), 4.21 - 4.15 (m, 2H), 3.08 - 3.02 (m, 4H), 2.46 (d, *J*=5.5, 5H), 0.97 (d, *J*=6.5, 6H).

**Example 122.** The following compounds were prepared according to the synthetic routes and the preparation methods in the examples.

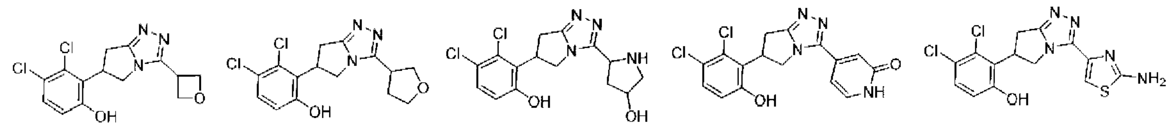

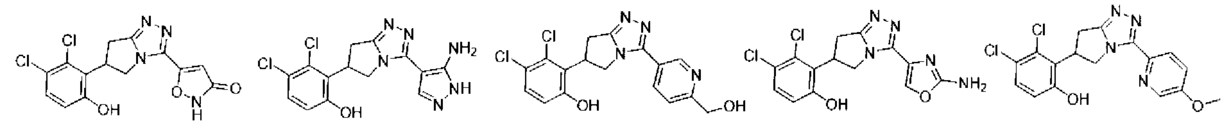

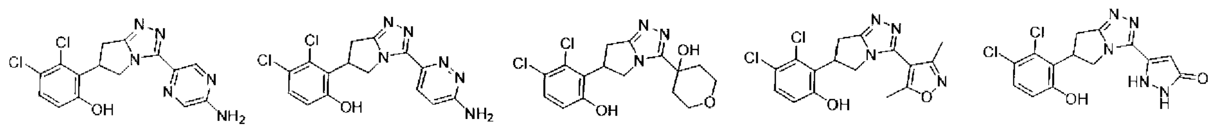

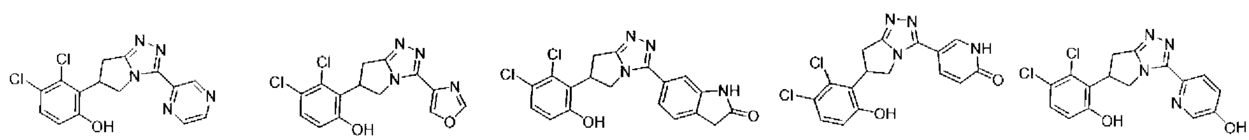

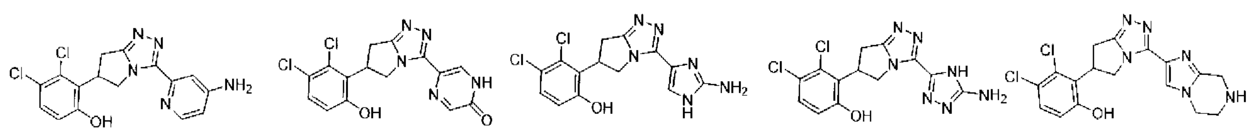

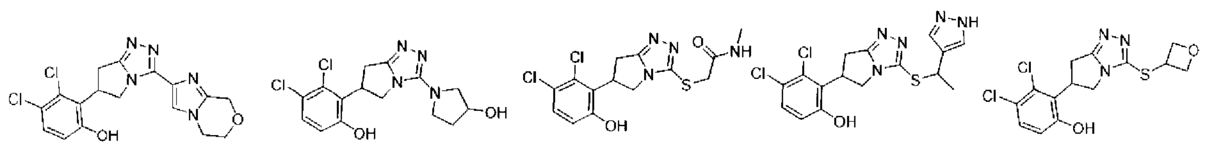

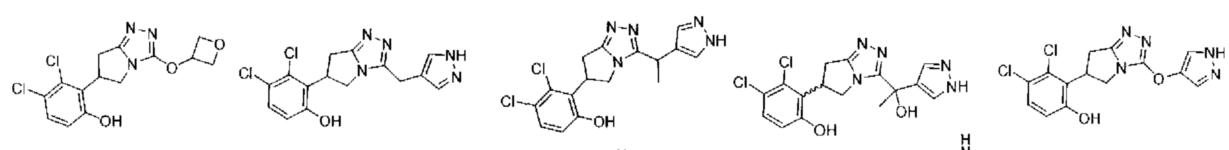

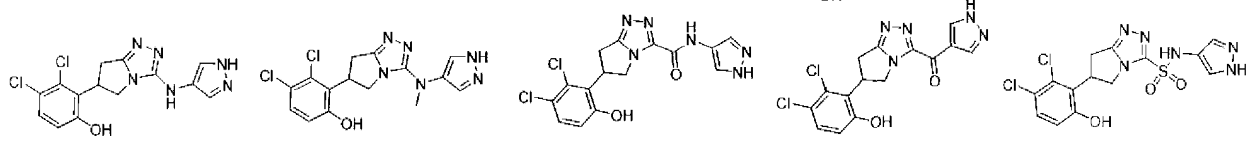

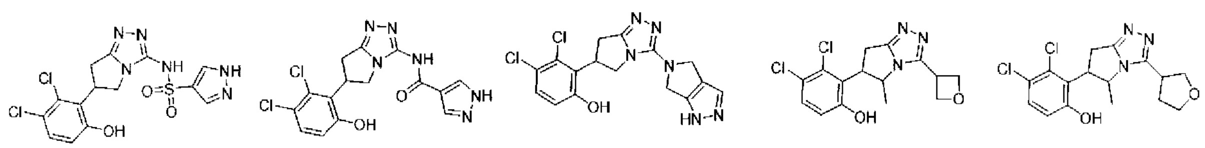

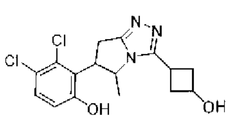 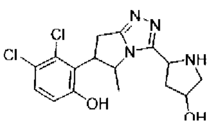 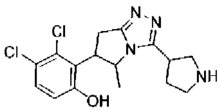 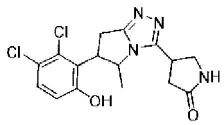 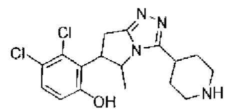

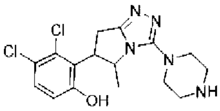 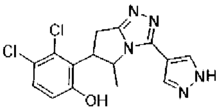 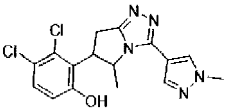 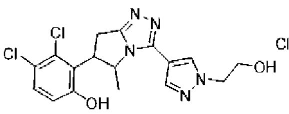 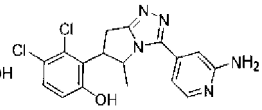

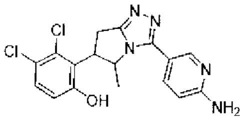 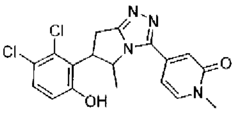 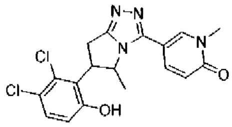 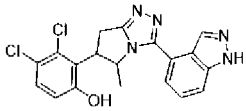 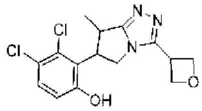

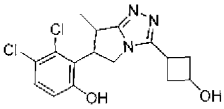 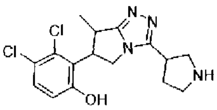 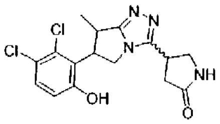 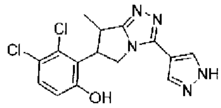 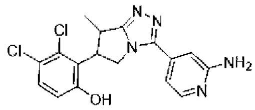

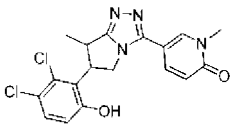 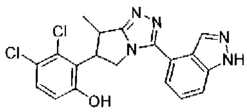

Example 123. Activity assay

1. Assay for inhibitory activity of compounds against Kv1.X

1.1. Cell culturing

HEK293 cells stably expressing Kv1.X (Kv1.1, Kv1.2, Kv1.3, Kv1.4, Kv1.5) potassium channels were cultured in DMEM medium containing 10% fetal bovine serum and 800 μg/mL G418 (geneticin) at a culture temperature of 37 °C and a carbon dioxide concentration of 5%.

1.2. Fluids used for recording

The extracellular fluid contained 40 mM NaCl, 3.5 mM KCl, 1 mM $MgCl_2•6H_2O$, 2 mM $CaCl_2•2H_2O$, 10 mM D-glucose, 10 mM HEPES, 1.25 mM $NaH_2PO_4•2H_2O$, and the pH was adjusted to 7.4 with NaOH. The intracellular fluid contained 20 mM KCl, 115 mM K-Aspartic, 1 mM $MgCl_2•6H_2O$, 5 mM EGTA, 10 mM HEPES, 2 mM $Na_2$-ATP, and the pH was adjusted to 7.2 with KOH.

1.3. Patch-clamp testing

The voltage stimulation protocol for recording whole-cell Kv1.X potassium currents via patch-clamp was as follows: After formation of a whole-cell seal, the cell membrane voltage was clamped at -80 mV, and then stepped to +40 mV for 500 ms. Data were acquired repeatedly every 30 s and the effect of the drug on Kv1.X currents was observed. The test data were acquired by EPC-10 amplifier (HEKA) and stored in the PatchMaster (HEKA) software. Recording electrodes were drawn from capillary glass tubes using a microelectrode puller. Under an inverted microscope, the recording electrode was brought into contact with the cell surface by manipulating a microelectrode manipulator. Negative-pressure suction was applied to form a GΩ seal. After formation of the GΩ seal, a rapid capacitance compensation was given. The application of negative pressure was continued to rupture the cell membrane, forming a whole-cell recording mode. Then, a slow capacitance compensation was given, and the membrane capacitance and series resistance were recorded. No current leakage compensation was provided.

Once the whole-cell recorded Kv1.X current stabilized, drug administration was started. Each drug concentration

was allowed to act for 5 min (or until the current reached a steady state) before the next concentration was tested. Multiple concentrations were tested for each test compound. The cover glass plated with the cells was placed in a recording bath of the inverted microscope. The test compound and the compound-free extracellular fluid were allowed to sequentially flow through the recording bath from low concentrations to high concentrations using a gravity perfusion method, thus acting on the cells, and liquid exchange was performed using a vacuum pump during recording. For each cell, the current detected in the compound-free extracellular liquid was used as its own control group. The test was independently repeated three times using at least three cells per concentration. All electrophysiological experiments were performed at room temperature.

1.4 Data analysis

(1) Kv1.1, Kv1.2, Kv1.5 - Steady state current

First, the current after the action of each drug concentration and the current of the blank control were normalized ( $\frac{\text{Steady state current}_{compound}}{\text{Steady state current}_{control}}$ ), and then the inhibition rate corresponding to each drug concentration was calculated ( $1 - \frac{\text{Steady state current}_{compound}}{\text{Steady state current}_{control}}$ ).

(2) Kv1.3, Kv1.4 - Peak current

First, the peak current after the action of each drug concentration and the peak current of the blank control were standardized ( $\frac{\text{Peak current}_{compound}}{\text{Peak current}_{control}}$ ), and then the inhibition rate corresponding to each drug concentration was calculated ( $1 - \frac{\text{Peak current}_{compound}}{\text{Peak current}_{control}}$ ). Subsequently, the mean, standard deviation (SD), and standard error (SE) were calculated for the inhibition rate corresponding to each concentration.

$$Y = Bottom + (Top - Bottom)/(1 + 10 \wedge ((LogIC_{50} - X) * HillSlope))$$

Using the above equation, the $IC_{50}$ value for each compound was calculated, and the dose-dependent effect was subjected to non-linear fitting, where $IC_{50}$ is the half maximal inhibitory concentration. The $IC_{50}$ calculation and the curve fitting were performed using the GraphPad Prism software.

2. Assay for inhibitory activity of compounds **against hERG**

2.1. Cell culturing

CHO cells stably expressing hERG were cultured in 35 mm diameter cell culture dishes at 37 °C in a 5% $CO_2$ incubator. The cells were passaged at a ratio of 1:5 every 48 h. The culture medium recipe was as follows: 90% F12 (Invitrogen), 10% fetal bovine serum (Gibco), 100 g/mL G418 (Invitrogen), and 100 g/mL Hygromycin B (Invitrogen). On the day of the test, the cell culture fluid was aspirated, and the cells were rinsed once with the extracellular fluid. Then, a 0.25% Trypsin-EDTA solution (Invitrogen) was added, and the cell cultures were digested at room temperature for 3-5 min. The digestion solution was then aspirated, and the cells were resuspended with the extracellular fluid and transferred to an experimental dish for electrophysiological recording for later use.

2.2 Electrophysiological recording process

hERG potassium channel currents were recorded at room temperature by the whole-cell voltage clamp technique in the CHO cells stably expressing hERG potassium channels. Glass microelectrodes were formed by pulling a glass electrode blank (BF150-86-10, Sutter) using a puller. The tip resistance after perfusion with the electrode internal solution was about 2-5 MΩ. The glass microelectrodes could be connected to a patch clamp amplifier after being inserted into amplifier probes. The clamping voltage and data recording were controlled and recorded by a computer with the pClamp software, with a sampling frequency of 10 kHz and a filtering frequency of 2 kHz. After achieving the whole-cell recording, the cells were clamped at -100 mV. The step voltage evoking hERG potassium current ($I_{hERG}$) was changed from -100 mV to +20 mV by applying a 2 s depolarization voltage, followed by repolarization to -50 mV for 1 s, and then returned to -100 mV. This voltage stimulation was applied every 5 s, and after the hERG potassium currents were

determined to be stable (for 1 min), the drug administration process was started. Each test concentration of the compound was applied for a minimum of 1 min (to reach a steady-state effect) or for a maximum of 3 min. Each concentration was tested for at least 2 cells (n ≥ 2).

2.3 Data processing

Data analysis and processing were performed using the pClamp, GraphPad Prism 8, and Excel softwares. The degree of inhibition of the hERG potassium current (the peak hERG tail current evoked at -50 mV) by different compound concentrations was calculated using the following formula:

$$\text{Inhibition } \% = [1 - (I/Io)] \times 100\%$$

where Inhibition % represents the percentage of inhibition of the hERG potassium current by the compound, and *I* and *Io* represent the amplitudes of the hERG potassium current after and before drug administration, respectively.

The $IC_{50}$ of the compound was calculated using the GraphPad Prism 8 software by equation fitting as follows:

$$Y = \text{Bottom} + (\text{Top} - \text{Bottom})/(1 + 10 \text{ ^ } ((\text{LogIC}_{50} - X) * \text{HillSlope}))$$

where X is the Log value of the test concentration of the test sample, Y is the inhibition percentage at the corresponding concentration, and Bottom and Top are the minimum and maximum inhibition percentages, respectively.

3. **Experimental results**

The compounds of the present invention exhibit inhibitory activity against the Kv1.3 potassium channel, with $IC_{50}$ values of less than 100 nM, as specifically shown in Table 1 below.

Table 1. $IC_{50}$ (nM) values of compounds of the present invetion for Kv1.3

| Compound No. | Kv1.3 | Compound No. | Kv1.3 | Compound No. | Kv1.3 |
|---|---|---|---|---|---|
| Example 1 | 94.34 | Example 2 | 28.08 | Example 4 | 45.2 |
| Example 5 | 39.0 | Example 6 | 13.2 | Example 7 | 12.0 |
| Example 8 | 20.6 | Example 9 | 17.1 | Example 10 | 27.7 |
| Example 11 | 9.24 | Example 12 | 30.9 | Example 13 | 5.09 |
| Example 14 | 19.6 | Example 15 | 26.8 | Example 16 | 74.7 |
| Example 17 | 6.68 | Example 18 | 5.76 | Example 19 | 46.3 |
| Example 20 | 35.3 | Example 21 | 48.6 | Example 22 | 13.6 |
| Example 25 | 11.1 | Example 32 | 13.0 | Example 33 | 47.8 |
| Example 34 | 9.75 | Example 35 | 3.15 | Example 36 | 6.8 |
| Example 37 | 12.7 | Example 38 | 43.1 | Example 39 | 52.1 |
| Example 40 | 10.6 | Example 41 | 23.8 | Example 42 | 13.0 |
| Example 43 | 19.7 | Example 44 | 8.11 | Example 45 | 1.5 |
| Example 46 | 5.12 | Example 47 | 1.30 | Example 48 | 8.49 |
| Example 49 | 3.64 | Example 50 | 10.9 | Example 51 | 7.24 |
| Example 52 | 14.0 | Example 53 | 15.0 | Example 54 | 7.7 |
| Example 55 | 12.0 | Example 56 | 20.6 | Example 57 | 11.4 |
| Example 58 | 12.0 | Example 59 | 2.56 | Example 60 | 27.6 |
| Example 61 | 7.12 | Example 62 | 16.4 | Example 63 | 18.2 |
| Example 64 | 42.8 | Example 65 | 51.7 | Example 66 | 33.3 |
| Example 67 | 10.2 | Example 68 | 16.0 | Example 69 | 53.0 |

(continued)

| Compound No. | Kv1.3 | Compound No. | Kv1.3 | Compound No. | Kv1.3 |
|---|---|---|---|---|---|
| Example 70 | 21.6 | Example 71 | 10.3 | Example 72 | 39.1 |
| Example 73 | 24.7 | Example 76 | 27.9 | Example 77 | 45.2 |
| Example 78 | 12.6 | Example 79 | 14.8 | Example 80 | 2.14 |
| Example 81 | 11.3 | Example 82 | 5.01 | Example 83 | 3.99 |
| Example 84 | 8.04 | Example 85 | 24.5 | Example 86 | 25.0 |
| Example 87 | 20.7 | Example 88 | 58.6 | Example 89 | 8.25 |
| Example 90 | 7.08 | Example 91 | 4.92 | Example 92 | *5.51* |
| Example 93 | 3.11 | Example 94 | 6.09 | Example 95 | 6.06 |
| Example 96 | 51.6 | Example 97 | 11.7 | Example 98 | 25.5 |
| Example 100 | 9.89 | Example 101 | 9.8 | Example 102 | 6.99 |
| Example 103 | 7.68 | Example 104 | 6.93 | Example 105 | 5.93 |
| Example 106 | 29.8 | Example 107 | 31.0 | Example 108 | 27.8 |
| Example 109 | 31.7 | Example 110 | 20.1 | Example 111 | 14.4 |
| Example 112 | 43.1 | Example 113 | 57.9 | Example 115 | 58.8 |
| Example 116 | 44.1 | Example 117 | 65.0 | Example 118 | 14.6 |
| Example 119 | 6.54 | Example 120 | 20.2 | Example 121 | 9.68 |

The compounds of the present invention exhibit no inhibitory activity against the Kv1.1, Kv1.2, Kv1.4, Kv1.5, and hERG channels, with $IC_{50}$ values all greater than 10 μM, as specifically shown in Table 2 below.

Table 2. $IC_{50}$ (nM) values of compounds of the present invention for inhibition of Kv1.1, Kv1.2, Kv1.4, Kv1.5, and hERG

| **Compound** No. | Kv1.1 | Kv1.2 | Kv1.4 | Kv1.5 | hERG |
|---|---|---|---|---|---|
| Example 2 | >100000 | >100000 | >30000 | >100000 | >30000 |
| Example 54 | >100000 | >100000 | >30000 | >100000 | >30000 |

Therefore, the compounds of the present invention are selective Kv1.3 inhibitors, demonstrating not only excellent activity but also excellent selectivity.

## Claims

1. A compound represented by Formula (I) or a pharmaceutically acceptable salt or a deuterated compound thereof:

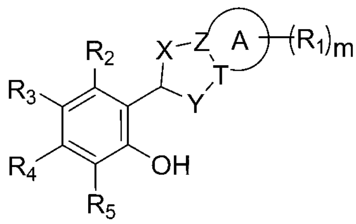

(I)

wherein

A is selected from 5-membered heteroaryl containing 1-3 ring heteroatoms independently selected from N, O,

and S;
Z and T are independently selected from C and N;
X and Y are independently selected from CRaRb, NRc, and O;
Ra and Rb are independently selected from H, OH, $NH_2$, $C_{1-4}$ linear or branched alkyl, $C_{2-4}$ linear or branched alkenyl, $C_{2-4}$ linear or branched alkynyl, and $C_{1-4}$ linear or branched alkyloxy; or Ra and Rb, together with the carbon atom to which they are attached, form $C_{3-7}$ cycloalkyl or 3- to 7-membered heterocycloalkyl containing 1-3 ring heteroatoms independently selected from N, O, and S;
Rc is selected from H, $C_{1-4}$ linear or branched alkyl, $C_{2-4}$ linear or branched alkenyl, and $C_{3-7}$ cycloalkyl;
$R_2$, $R_3$, $R_4$, and $R_5$ are independently selected from H, F, Cl, Br, CN, $C_{1-4}$ linear or branched alkyl, $C_{2-4}$ linear or branched alkenyl, $C_{2-4}$ linear or branched alkynyl, $C_{1-4}$ linear or branched alkyloxy, $C_{2-4}$ linear or branched alkenyloxy, $C_{2-4}$ linear or branched alkynyloxy, and $C_{3-7}$ cycloalkyl;
each $R_1$ is independently selected from F, Cl, Br, CN, =O, $C_{1-4}$ linear or branched alkyl, $C_{2-4}$ linear or branched alkenyl, $C_{2-4}$ linear or branched alkynyl, $C_{3-7}$ cycloalkyl, 3- to 7-membered heterocycloalkyl attached via C or N and containing 1-3 ring heteroatoms independently selected from N, O, and S, aryl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, $-C_{1-4}$ linear or branched alkylene-$R_7$, $-OR_7$, $-O-C_{1-4}$ linear or branched alkylene-$R_7$, $-SR_7$, $-S-C_{1-4}$ linear or branched alkylene-$R_7$, $-N(R_6)R_7$, $-N(R_6)-C_{1-4}$ linear or branched alkylene-$R_7$, $-C(O)R_7$, $-C(O)-C_{1-4}$ linear or branched alkylene-$R_7$, $-C(O)R_7-OR_7$, $-C(O)R_7-N(R_6)R_7$, $-S(O)_2R_7$, $-N(R_6)C(O)R_7$, $-N(R_6)C(O)-C_{1-4}$ linear or branched alkylene-$R_7$, $-C(O)N(R_6)R_7$, $-C(O)N(R_6)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, and $-S(O)_2N(R_6)-C_{1-4}$ linear or branched alkylene-$R_7$, wherein:

the alkylene is optionally substituted with OH;
the alkyl, alkenyl, alkynyl, cycloalkyl, heterocycloalkyl, aryl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $-C(O)OC_{1-4}$ linear or branched alkyl, $C(O)OC_{2-4}$ linear or branched alkenyl, $C(O)OC_{2-4}$ linear or branched alkynyl, $-C(O)R_7$, $-S(O)_2R_7$, $-N(R_6)R_7$, $-N(R_6)-C_{1-4}$ linear or branched alkylene-$R_7$, $-N(R_6)C(O)R_7$, $-N(R_6)C(O)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, $-S(O)_2N(R_6)-C_{1-4}$ linear or branched alkylene-$R_7$, $-C(O)N(R_6)R_7$, $-C(O)N(R_6)-C_{1-4}$ linear or branched alkylene-$R_7$, $-P(O)(R_6)_2$, substituted or unsubstituted $C_{3-7}$ cycloalkyl, substituted or unsubstituted aryl, substituted or unsubstituted 3- to 7-membered heterocycloalkyl attached via C or N and containing 1-3 ring heteroatoms independently selected from N, O, and S, and substituted or unsubstituted 5- to 6-membered heteroaryl attached via C or N and containing 1-3 ring heteroatoms independently selected from N, O, and S, wherein substituent groups for the substituted $C_{3-7}$ cycloalkyl, substituted aryl, substituted 3- to 7-membered heterocycloalkyl, and substituted 5- to 6-membered heteroaryl are selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, and $C_{1-4}$ linear or branched alkyloxy; the two substituents on the same ring carbon atom in the cycloalkyl, heterocycloalkyl, and heteroaryl, together with the carbon atom to which they are attached, may optionally form $C_{3-6}$ cycloalkyl or 3- to 6-membered heterocycloalkyl containing 1 ring

heteroatom selected from N and O, the formed cycloalkyl or heterocycloalkyl being optionally substituted with one or more groups independently selected from OH, CN, $NH_2$, =O, F, Cl, Br, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $C_{3-6}$ cycloalkyl, and 3- to 6-membered heterocycloalkyl containing 1-3 ring heteroatoms independently selected from N, O, and S;

$R_6$ is selected from H, $C_{1-4}$ linear or branched alkyl, $C_{2-4}$ linear or branched alkenyl, and $C_{2-4}$ linear or branched alkynyl;

$R_7$ is independently selected from H, OH, $NH_2$, $C_{1-4}$ linear or branched alkyl, $C_{2-4}$ linear or branched alkenyl, $C_{2-4}$ linear or branched alkynyl, $C_{1-4}$ linear or branched alkyloxy, $C_{1-4}$ linear or branched alkylene-OH, $C_{3-7}$ cycloalkyl, 3- to 7-membered heterocycloalkyl attached via C or N and containing 1-3 ring heteroatoms independently selected from N, O, and S, aryl, and 5- to 11-membered heteroaryl containing 1-3 ring heteroatoms independently selected from N, O, and S, the alkyl, alkenyl, alkynyl, cycloalkyl, heterocycloalkyl, aryl, and heteroaryl being optionally substituted with one or more groups independently selected from F, Cl, Br, OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;

m is selected from 1 and 2.

**2.** The compound represented by Formula (I) or the pharmaceutically acceptable salt or the deuterated compound thereof according to claim 1:

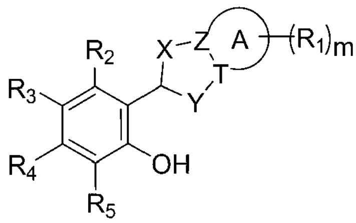

(I)

wherein

A is selected from 5-membered heteroaryl containing 1-3 ring heteroatoms independently selected from N, O, and S;

Z and T are independently selected from C and N;

X and Y are independently selected from CRaRb, NRc, and O;

Ra and Rb are independently selected from H, OH, $NH_2$, $C_{1-4}$ linear or branched alkyl, $C_{2-4}$ linear or branched alkenyl, $C_{2-4}$ linear or branched alkynyl, and $C_{1-4}$ linear or branched alkyloxy, or Ra and Rb, together with the carbon atom to which they are attached, form $C_{3-7}$ cycloalkyl or 3- to 7-membered heterocycloalkyl containing 1-3 ring heteroatoms independently selected from N, O, and S;

Rc is selected from H, $C_{1-4}$ linear or branched alkyl, $C_{2-4}$ linear or branched alkenyl, and $C_{3-7}$ cycloalkyl;

$R_2$, $R_3$, $R_4$, and $R_5$ are independently selected from H, F, Cl, Br, CN, $C_{1-4}$ linear or branched alkyl, $C_{2-4}$ linear or branched alkenyl, $C_{2-4}$ linear or branched alkynyl, and $C_{3-7}$ cycloalkyl;

each $R_1$ is independently selected from F, Cl, Br, CN, $C_{1-4}$ linear or branched alkyl, $C_{2-4}$ linear or branched alkenyl, $C_{2-4}$ linear or branched alkynyl, $C_{3-7}$ cycloalkyl, 3- to 7-membered heterocycloalkyl attached via C or N and containing 1-3 ring heteroatoms independently selected from N, O, and S, aryl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing

1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, $-C_{1-4}$ linear or branched alkylene-$R_7$, $-OR_7$, $-O-C_{1-4}$ linear or branched alkylene-$R_7$, $-SR_7$, $-S-C_{1-4}$ linear or branched alkylene-$R_7$, $-N(R_6)R_7$, $-N(R_6)-C_{1-4}$ linear or branched alkylene-$R_7$, $-C(O)R_7$, $-C(O)-C_{1-4}$ linear or branched alkylene-$R_7$, $-C(O)R_7-OR_7$, $-C(O)R_7-N(R_6)R_7$, $-S(O)_2R_7$, $-N(R_6)C(O)R_7$, $-N(R_6)C(O)-C_{1-4}$ linear or branched alkylene-$R_7$, $-C(O)N(R_6)R_7$, $-C(O)N(R_6)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, and $-S(O)_2N(R_6)-C_{1-4}$ linear or branched alkylene-$R_7$, wherein: the alkylene is optionally substituted with OH; the alkyl, alkenyl, alkynyl, cycloalkyl, heterocycloalkyl, aryl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $-C(O)OC_{1-4}$ linear or branched alkyl, $C(O)OC_{2-4}$ linear or branched alkenyl, $C(O)OC_{2-4}$ linear or branched alkynyl, $-C(O)R_7$, $-S(O)_2R_7$, $-N(R_6)R_7$, $-N(R_6)-C_{1-4}$ linear or branched alkylene-$R_7$, $-N(R_6)C(O)R_7$, $-N(R_6)C(O)-C_{1-4}$ linear or branched alkylene-$R_7$, $-N(R_6)S(O)_2R_7$, $-N(R_6)S(O)_2-C_{1-4}$ linear or branched alkylene-$R_7$, $-S(O)_2N(R_6)R_7$, $-S(O)_2N(R_6)-C_{1-4}$ linear or branched alkylene-$R_7$, $-C(O)N(R_6)R_7$, $-C(O)N(R_6)-C_{1-4}$ linear or branched alkylene-$R_7$, $-P(O)(R_6)_2$, $C_{3-7}$ cycloalkyl, and 3- to 7-membered heterocycloalkyl attached via C or N and containing 1-3 ring heteroatoms independently selected from N, O, and S; the two substituents on the same ring carbon atom in the cycloalkyl, heterocycloalkyl, and heteroaryl, together with the carbon atom to which they are attached, may optionally form $C_{3-6}$ cycloalkyl or 3- to 6-membered heterocycloalkyl containing 1 ring heteroatom selected from N and O, the formed cycloalkyl or heterocycloalkyl being optionally substituted with one or more groups independently selected from OH, CN, $NH_2$, =O, F, Cl, Br, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, $C_{1-4}$ linear or branched alkylene-$NH_2$, $C_{2-4}$ linear or branched alkenylene-$NH_2$, $C_{2-4}$ linear or branched alkynylene-$NH_2$, $C_{1-4}$ linear or branched alkyloxy, $C_{3-6}$ cycloalkyl, and 3- to 6-membered heterocycloalkyl containing 1-3 ring heteroatoms independently selected from N, O, and S;

$R_6$ is selected from H, $C_{1-4}$ linear or branched alkyl, $C_{2-4}$ linear or branched alkenyl, and $C_{2-4}$ linear or branched alkynyl;

$R_7$ is independently selected from H, OH, $NH_2$, $C_{1-4}$ linear or branched alkyl, $C_{2-4}$ linear or branched alkenyl, $C_{2-4}$ linear or branched alkynyl, $C_{1-4}$ linear or branched alkyloxy, $C_{1-4}$ linear or branched alkylene-OH, $C_{3-7}$ cycloalkyl, 3- to 7-membered heterocycloalkyl attached via C or N and containing 1-3 ring heteroatoms independently selected from N, O, and S, aryl, and 5- to 6-membered heteroaryl containing 1-3 ring heteroatoms independently selected from N, O, and S, the alkyl, alkenyl, alkynyl, cycloalkyl, heterocycloalkyl, aryl, and heteroaryl being optionally substituted with one or more groups independently selected from F, Cl, Br, OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, $-C(O)OC_{1-4}$ linear or branched alkyl, $C(O)OC_{2-4}$ linear or branched alkenyl, $C(O)OC_{2-4}$ linear or branched alkynyl, $C(O)NHC_{1-4}$ linear or branched alkyl, $C(O)NHC_{2-4}$ linear or branched alkenyl, and $C(O)NHC_{2-4}$ linear or branched alkynyl;

m is selected from 1 and 2.

3. The compound or the pharmaceutically acceptable salt or the deuterated compound thereof according to claim 1 or 2, **characterized in that**

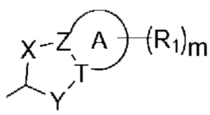

is selected from

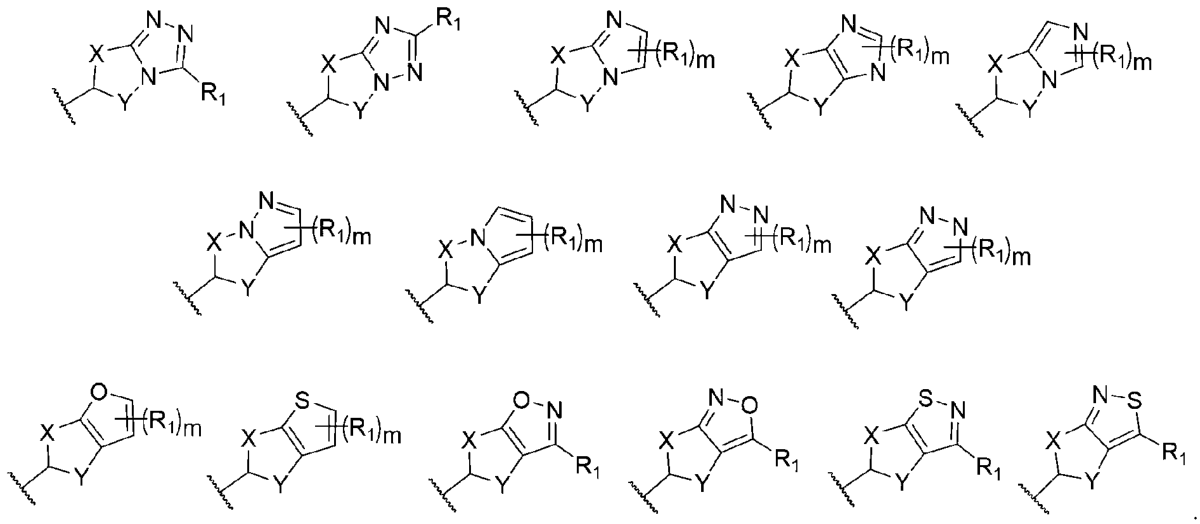

.

4. The compound or the pharmaceutically acceptable salt or the deuterated compound thereof according to claim 3, **characterized in that** X and Y are CRaRb.

5. The compound or the pharmaceutically acceptable salt or the deuterated compound thereof according to claim 1, **characterized in that** the compound has a structure represented by Formula (II):

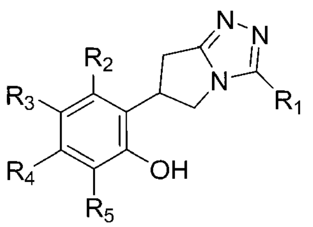

(II)

wherein:

$R_1$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -$OR_7$, -O-$C_{1-4}$ linear or branched alkylene-$R_7$, -$SR_7$, -S-$C_{1-4}$ linear or branched alkylene-$R_7$, -$N(R_6)R_7$, -$N(R_6)$-$C_{1-4}$ linear or branched alkylene-$R_7$, -$C(O)R_7$, -C(O)-$C_{1-4}$ linear or branched alkylene-$R_7$, -$N(R_6)C(O)R_7$, -$N(R_6)C(O)$-$C_{1-4}$ linear or branched alkylene-$R_7$, -$C(O)N(R_6)R_7$, -$C(O)N(R_6)$-$C_{1-4}$ linear or branched alkylene-$R_7$, -$N(R_6)S(O)_2R_7$, -$N(R_6)S(O)_2$-$C_{1-4}$ linear or branched alkylene-$R_7$, -$S(O)_2N(R_6)R_7$, and -$S(O)_2N(R_6)$-$C_{1-4}$ linear or branched alkylene-$R_7$, wherein:

the alkylene is optionally substituted with OH;

the alkyl, alkenyl, alkynyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from F, Cl, and Br, $C_{2-4}$ linear or branched alkenyl optionally substituted with one or more groups independently selected from F, Cl, and Br, $C_{2-4}$ linear or branched alkynyl optionally substituted with one or

more groups independently selected from F, Cl, and Br, $C_{1-4}$ linear or branched alkylene-OH, $C_{2-4}$ linear or branched alkenylene-OH, $C_{2-4}$ linear or branched alkynylene-OH, $C_{1-4}$ linear or branched alkyloxy, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, C(O)NH$C_{2-4}$ linear or branched alkynyl, -C(O)$R_7$, substituted or unsubstituted $C_{3-7}$ cycloalkyl, substituted or unsubstituted aryl, substituted or unsubstituted 3- to 7-membered heterocycloalkyl attached via C or N and containing 1-3 ring heteroatoms independently selected from N, O, and S, and substituted or unsubstituted 5-to 6-membered heteroaryl attached via C or N and containing 1-3 ring heteroatoms independently selected from N, O, and S, wherein substituent groups for the substituted $C_{3-7}$ cycloalkyl, substituted aryl, substituted 3-to 7-membered heterocycloalkyl, and substituted 5- to 6-membered heteroaryl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, $C_{2-4}$ linear or branched alkenylene-$NH_2$, $C_{2-4}$ linear or branched alkynylene-$NH_2$, and $C_{1-4}$ linear or branched alkyloxy;

$R_6$ is selected from H, $C_{1-4}$ linear or branched alkyl, $C_{2-4}$ linear or branched alkenyl, and $C_{2-4}$ linear or branched alkynyl;

$R_7$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, alkenyl, alkynyl, cycloalkyl, heterocycloalkyl, phenyl, and heteroaryl are optionally substituted with one or more groups independently selected from OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;

$R_2$ and $R_3$ are independently selected from F, Cl, and Br;

$R_4$ and $R_5$ are H.

**6.** The compound or the pharmaceutically acceptable salt or the deuterated compound thereof according to claim 5, **characterized in that**, in Formula (II):

$R_1$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, -$C_{1-4}$ linear or branched alkylene-$R_7$, -O$R_7$, -O-$C_{1-4}$ linear or branched alkylene-$R_7$, -S$R_7$, -S-$C_{1-4}$ linear or branched alkylene-$R_7$, -N($R_6$)$R_7$, -N($R_6$)-$C_{1-4}$ linear or branched alkylene-$R_7$, -C(O)$R_7$, -C(O)-$C_{1-4}$ linear or branched alkylene-$R_7$, -N($R_6$)C(O)$R_7$, -N($R_6$)C(O)-$C_{1-4}$ linear or branched alkylene-$R_7$, -C(O)N($R_6$)$R_7$, -C(O)N($R_6$)-$C_{1-4}$ linear or branched alkylene-$R_7$, -N($R_6$)S(O)$_2R_7$, -N($R_6$)S(O)$_2$-$C_{1-4}$ linear or branched alkylene-$R_7$, -S(O)$_2$N($R_6$)$R_7$, and -S(O)$_2$N($R_6$)-$C_{1-4}$ linear or branched alkylene-$R_7$; the alkylene is optionally substituted with OH; the alkyl, alkenyl, alkynyl, cycloalkyl, heterocycloalkyl, phenyl, and heteroaryl are optionally substituted with one or more groups independently selected from OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;

$R_6$ is selected from H, $C_{1-4}$ linear or branched alkyl, $C_{2-4}$ linear or branched alkenyl, and $C_{2-4}$ linear or branched alkynyl;

$R_7$ is selected from $C_{1-4}$ linear or branched alkyl, $C_{2-4}$ linear or branched alkenyl, $C_{2-4}$ linear or branched alkynyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, alkenyl, alkynyl, cycloalkyl, heterocycloalkyl, phenyl, and heteroaryl are optionally substituted with one or more groups independently selected from OH, CN, $NH_2$, =O, $C_{1-4}$ linear or branched alkyl, $C_{2-4}$ linear or branched alkenyl, $C_{2-4}$ linear or branched alkynyl, $C_{1-4}$ linear or branched alkylene-OH, $C_{2-4}$ linear or branched alkenylene-OH, $C_{2-4}$ linear or branched alkynylene-OH, -C(O)O$C_{1-4}$ linear or branched alkyl, C(O)O$C_{2-4}$ linear or branched alkenyl, C(O)O$C_{2-4}$ linear or branched alkynyl, C(O)NH$C_{1-4}$ linear or branched alkyl, C(O)NH$C_{2-4}$ linear or branched alkenyl, and C(O)NH$C_{2-4}$ linear or branched alkynyl;

$R_2$ and $R_3$ are independently selected from F, Cl, and Br;

$R_4$ and $R_5$ are H;
preferably, in Formula (II):

$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S,

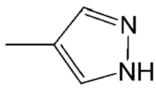,

pyridinyl,

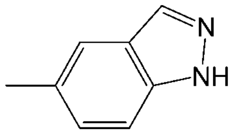,

-$C_{1-4}$ linear or branched alkylene-$R_7$, -$OR_7$, -$SR_7$, -S-$C_{1-4}$ linear or branched alkylene-$R_7$, -$N(R_6)R_7$, -$C(O)R_7$, -$N(R_6)C(O)R_7$, -$C(O)N(R_6)R_7$, -$N(R_6)S(O)_2R_7$, and -$S(O)_2N(R_6)R_7$; the alkylene is optionally substituted with OH; the alkyl, cycloalkyl, heterocycloalkyl,

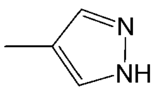,

pyridinyl, and

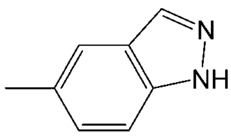

are optionally substituted with one or more groups independently selected from OH, CN, $NH_2$, =O, $C_{1-4}$ linear or branched alkyl, $C_{1-4}$ linear or branched alkylene-OH, -C(O)O$C_{1-4}$ linear or branched alkyl, and C(O)NH$C_{1-4}$ linear or branched alkyl;
$R_6$ is selected from H and $C_{1-4}$ linear or branched alkyl;
$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S,

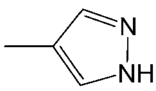,

pyridinyl, and

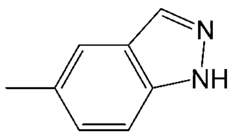;

the alkyl, cycloalkyl, heterocycloalkyl,

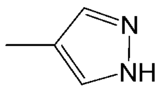

pyridinyl, and

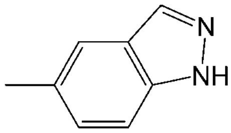

are optionally substituted with one or more groups independently selected from OH, CN, $NH_2$, =O, $C_{1-4}$ linear or branched alkyl, $C_{1-4}$ linear or branched alkylene-OH, -C(O)O$C_{1-4}$ linear or branched alkyl, and C(O)NH$C_{1-4}$ linear or branched alkyl;
$R_2$ and $R_3$ are independently selected from F, Cl, and Br;
$R_4$ and $R_5$ are H;
further preferably, in Formula (II):

$R_1$ is $C_{1-4}$ linear or branched alkyl substituted with one or more OHs;
$R_2$ and $R_3$ are independently selected from F, Cl, and Br;
$R_4$ and $R_5$ are H;
more preferably, in Formula (II):

$R_1$ is $C_{1-4}$ linear or branched alkyl substituted with one OH;
$R_2$ and $R_3$ are Cl;
$R_4$ and $R_5$ are H.

**7.** The compound or the pharmaceutically acceptable salt or the deuterated compound thereof according to claim 1, **characterized in that** the compound has a structure represented by Formula (II):

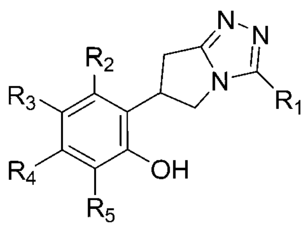

(II),

wherein:
$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -O$R_7$, -O-$C_{1-4}$ linear or branched alkylene-$R_7$, -C(O)$R_7$, and -C(O)-$C_{1-4}$ linear or branched alkylene-$R_7$, wherein:

the alkylene is optionally substituted with OH;
the alkyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl optionally substituted with one or more groups independently selected from

F, Cl, and Br, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, and $C_{1-4}$ linear or branched alkyloxy;
$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, cycloalkyl, heterocycloalkyl, phenyl, and heteroaryl are optionally substituted with one or more groups independently selected from OH, $C_{1-4}$ linear or branched alkyl, and $C_{1-4}$ linear or branched alkylene-OH;
$R_2$ and $R_3$ are independently selected from F, Cl, and Br;
$R_4$ and $R_5$ are H;
preferably, in Formula (II):

$R_1$ is selected from $C_{1-4}$ linear or branched alkyl, $C_{3-6}$ cycloalkyl, 3- to 6-membered heterocycloalkyl attached via C or N and containing 1-2 ring heteroatoms independently selected from N and O, phenyl,

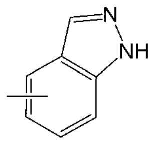
,

pyridinyl,

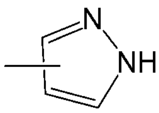
,

, 5- to 7-membered bridged heterocycloalkyl containing 1 ring heteroatom of O, a fused-ring structure consisting of a 3- to 7-membered heteroalkane ring containing 1 ring heteroatom of O and a 3- to 7-membered alkane ring, -$C_{1-4}$ linear or branched alkylene-$R_7$, -O$R_7$, and -C(O)$R_7$, wherein: the alkyl, cycloalkyl, heterocycloalkyl, phenyl,

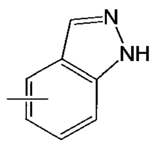
,

pyridinyl,

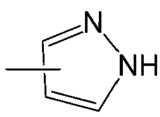
,

bridged heterocycloalkyl, and fused-ring structure are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-2 ring heteroatoms independently selected from N and O, wherein substituent groups for the substituted $C_{3-7}$ cycloalkyl and substituted 3- to 7-membered heterocycloalkyl are selected from $C_{1-4}$ linear or branched alkyl optionally substituted with one or more groups

independently selected from F, Cl, and Br;
$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;
$R_2$ and $R_3$ are independently selected from F, Cl, and Br;
$R_4$ and $R_5$ are H;
more preferably, in Formula (II):

$R_1$ is selected from $C_{3-6}$ cycloalkyl and 3- to 6-membered heterocycloalkyl attached via C or N and containing 1-2 ring heteroatoms independently selected from N and O, wherein: the cycloalkyl and heterocycloalkyl are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-6}$ cycloalkyl, and substituted or unsubstituted 3- to 6-membered heterocycloalkyl attached via C or N and containing 1-2 ring heteroatoms independently selected from N and O, wherein substituent groups for the substituted $C_{3-6}$ cycloalkyl and substituted 3- to 6-membered heterocycloalkyl are selected from $C_{1-4}$ linear or branched alkyl optionally substituted with one or more groups independently selected from F, Cl, and Br;
$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;
$R_2$ and $R_3$ are independently selected from F, Cl, and Br;
$R_4$ and $R_5$ are H.

**8.** The compound or the pharmaceutically acceptable salt or the deuterated compound thereof according to claim 1, **characterized in that** the compound has a structure represented by Formula (III):

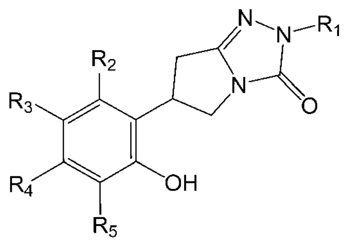

Formula (III),

wherein:

$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, $C_{5-7}$ bridged cycloalkyl, and 5- to 7-membered bridged heterocycloalkyl containing 1-3 ring heteroatoms independently selected from N, O, and S, wherein: the alkyl, cycloalkyl, heterocycloalkyl, bridged cycloalkyl, and bridged heterocycloalkyl are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from $C_{1-4}$ linear or branched alkyl optionally substituted with one or more groups independently selected from F, Cl, and Br;
$R_2$ and $R_3$ are independently selected from F, Cl, and Br;
$R_4$ and $R_5$ are H;
preferably, in Formula (III):

$R_1$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O, wherein: the alkyl, cycloalkyl, and heterocycloalkyl are optionally substituted with one or more groups independently

selected from F, Cl, Br, $C_{1-4}$ linear or branched alkyl, $C_{1-4}$ linear or branched alkylene-OH, and $C_{1-4}$ linear or branched alkyloxy;
$R_2$ and $R_3$ are independently selected from F, Cl, and Br;
$R_4$ and $R_5$ are H.

**9.** The compound or the pharmaceutically acceptable salt or the deuterated compound thereof according to claim 1, **characterized in that** the compound has a structure represented by Formula (IV):

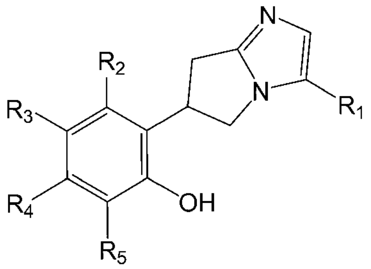

Formula (IV),

wherein:
$R_1$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -$OR_7$, -O-$C_{1-4}$ linear or branched alkylene-$R_7$, -C(O)$R_7$, and -C(O)-$C_{1-4}$ linear or branched alkylene-$R_7$, wherein:

the alkylene is optionally substituted with OH;
the alkyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, and $C_{1-4}$ linear or branched alkyloxy;
$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, cycloalkyl, heterocycloalkyl, phenyl, and heteroaryl are optionally substituted with one or more groups independently selected from OH, $C_{1-4}$ linear or branched alkyl, and $C_{1-4}$ linear or branched alkylene-OH;
$R_2$ and $R_3$ are independently selected from F, Cl, and Br;
$R_4$ and $R_5$ are H;
preferably, in Formula (IV):

$R_1$ is selected from $C_{1-4}$ linear or branched alkyl, $C_{3-6}$ cycloalkyl, 3- to 6-membered heterocycloalkyl attached via C or N and containing 1-2 ring heteroatoms independently selected from N and O, phenyl,

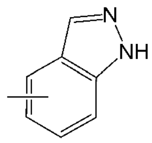,

pyridinyl,

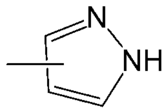,

5- to 7-membered bridged heterocycloalkyl containing 1 ring heteroatom of O, a fused-ring structure consisting of a 3- to 7-membered heteroalkane ring containing 1 ring heteroatom of O and a 3- to 7-membered alkane ring, -$C_{1-4}$ linear or branched alkylene-$R_7$, -C(O)$R_7$, and -O$R_7$, wherein: the alkyl, cycloalkyl, heterocycloalkyl, phenyl,

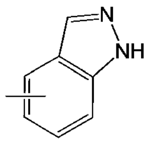,

pyridinyl,

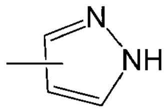,

bridged heterocycloalkyl, and fused-ring structure are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-2 ring heteroatoms independently selected from N and O, wherein substituent groups for the substituted $C_{3-7}$ cycloalkyl and substituted 3- to 7-membered heterocycloalkyl are selected from $C_{1-4}$ linear or branched alkyl optionally substituted with one or more groups independently selected from F, Cl, and Br;
$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;
$R_2$ and $R_3$ are independently selected from F, Cl, and Br;
$R_4$ and $R_5$ are H;
more preferably, in Formula (IV):

$R_1$ is selected from $C_{1-4}$ linear or branched alkyl, $C_{3-6}$ cycloalkyl, 3- to 6-membered heterocycloalkyl attached via C or N and containing 1-2 ring heteroatoms independently selected from N and O, phenyl,

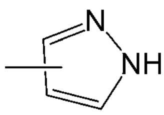,

-$C_{1-4}$ linear or branched alkylene-$R_7$, -C(O)$R_7$, and -O$R_7$, wherein: the alkyl, cycloalkyl, heterocycloalkyl, phenyl, and

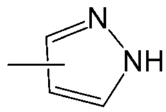

are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, and -C(O)O$C_{1-4}$ linear or branched alkyl;

$R_7$ is selected from $C_{1-4}$ linear or branched alkyl, $C_{3-6}$ cycloalkyl, and 3- to 6-membered heterocycloalkyl attached via C or N and containing 1-2 ring heteroatoms independently selected from N and O; the alkyl, cycloalkyl, and heterocycloalkyl are optionally substituted with one or more groups independently selected from OH and $C_{1-4}$ linear or branched alkyl;

$R_2$ and $R_3$ are independently selected from F, Cl, and Br;

$R_4$ and $R_5$ are H.

**10.** The compound or the pharmaceutically acceptable salt or the deuterated compound thereof according to claim 1, **characterized in that** the compound has a structure represented by Formula (V) or Formula (V'):

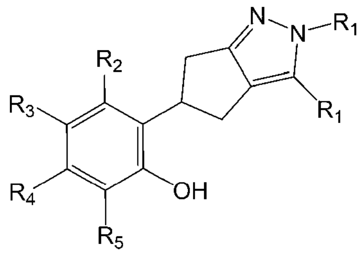

Formula V or

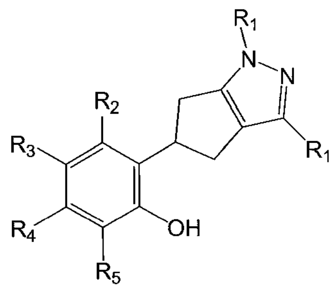

Formula V';

in Formula (V) or Formula (V'), each $R_1$ is identical or different and is independently selected from H, $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, 5- to 11-membered heteroaryl containing 1-3 ring heteroatoms independently selected from N, O, and S, $C_{5-7}$ bridged cycloalkyl, 5- to 7-membered bridged heterocycloalkyl containing 1-3 ring heteroatoms independently selected from N, O, and S, a fused-ring structure consisting of a 3- to 7-membered heteroalkane ring containing 1-3 ring heteroatoms independently selected from N, O, and S and a 3- to 7-membered alkane ring, a fused-ring structure consisting of a 3- to 7-membered heteroalkane ring containing 1-3 ring heteroatoms independently selected from N, O, and S and a 3- to 7-membered heteroalkane ring containing 1-3 ring heteroatoms independently selected from N, O, and S, a fused-ring structure consisting of a benzene ring and a 3- to 7-membered heteroalkane ring containing 1-3 ring heteroatoms independently selected from N, O, and S, a fused-ring structure consisting of a 5- to 7-membered heteroaromatic ring containing 1-3 ring heteroatoms independently selected from N, O, and S and a 3- to 7-membered heteroalkane ring containing 1-3 ring heteroatoms independently selected from N, O, and S, -$C_{1-4}$ linear or branched alkylene-$R_7$, -O$R_7$, -O-$C_{1-4}$ linear or branched alkylene-$R_7$, -C(O)$R_7$, and -C(O)-$C_{1-4}$ linear or branched alkylene-$R_7$, wherein:

the alkylene is optionally substituted with OH;

the alkyl, cycloalkyl, heterocycloalkyl, phenyl, heteroaryl, bridged cycloalkyl, bridged heterocycloalkyl, and fused-ring structures are optionally substituted with one or more groups independently selected from OH, CN, $NH_2$, =O, F, Cl, Br, $C_{1-4}$ linear or branched alkyl, $C_{1-4}$ linear or branched alkylene-OH, $C_{1-4}$ linear or branched alkyloxy, -C(O)O$C_{1-4}$ linear or branched alkyl, -C(O)$R_7$, substituted or unsubstituted $C_{3-7}$ cycloalkyl, and substituted or unsubstituted 3- to 7-membered heterocycloalkyl attached via C or N and containing 1-3 ring heteroatoms independently selected from N, O, and S, wherein substituent groups for the substituted $C_{3-7}$ cycloalkyl and substituted 3- to 7-membered heterocycloalkyl are selected from OH, CN, $NH_2$, =O, F, Cl, Br, $C_{1-4}$ linear or branched alkyl optionally substituted with one or more groups independently selected from F, Cl, and Br, $C_{1-4}$ linear or branched alkylene-OH, $C_{1-4}$ linear or branched alkylene-$NH_2$, and $C_{1-4}$ linear or branched alkyloxy;

$R_7$ is selected from $C_{1-4}$ linear or branched alkyl, $C_{3-6}$ cycloalkyl, 3- to 6-membered heterocycloalkyl attached via C or N and containing 1-3 ring heteroatoms independently selected from N, O, and S, phenyl, and 5- to 11-membered heteroaryl containing 1-3 ring heteroatoms independently selected from N, O, and S; the alkyl, cycloalkyl, heterocycloalkyl, phenyl, and heteroaryl are optionally substituted with one or more groups inde-

pendently selected from OH, $C_{1-4}$ linear or branched alkyl, and $C_{1-4}$ linear or branched alkylene-OH;
$R_2$ and $R_3$ are independently selected from F, Cl, and Br;
$R_4$ and $R_5$ are H;
preferably, in Formula (V) or Formula (V'):

each $R_1$ is identical or different and is independently selected from H, $C_{1-4}$ linear or branched alkyl, $C_{3-6}$ cycloalkyl, 3- to 6-membered heterocycloalkyl attached via C or N and containing 1-2 ring heteroatoms independently selected from N and O, and phenyl, wherein: the alkyl, cycloalkyl, heterocycloalkyl, and phenyl are optionally substituted with one or more groups independently selected from OH, F, Cl, Br, $C_{1-4}$ linear or branched alkyl, $C_{1-4}$ linear or branched alkylene-OH, and $C_{1-4}$ linear or branched alkyloxy;
$R_2$ and $R_3$ are independently selected from F, Cl, and Br;
$R_4$ and $R_5$ are H;
more preferably, in Formula (V) or Formula (V'):

each $R_1$ is identical or different and is independently selected from H and $C_{1-4}$ linear or branched alkyl, wherein: the alkyl is optionally substituted with one or more groups independently selected from OH, F, Cl, Br, $C_{1-4}$ linear or branched alkyl, $C_{1-4}$ linear or branched alkylene-OH, and $C_{1-4}$ linear or branched alkyloxy;
$R_2$ and $R_3$ are independently selected from F, Cl, and Br;
$R_4$ and $R_5$ are H.

**11.** The compound or the pharmaceutically acceptable salt or the deuterated compound thereof according to claim 1, **characterized in that** the compound or the pharmaceutically acceptable salt or the deuterated compound thereof is selected from the following compounds:

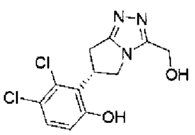 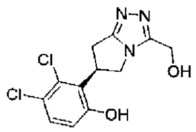 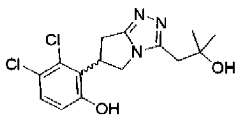 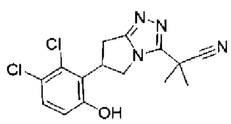 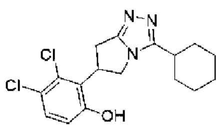

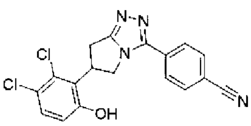 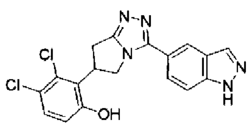 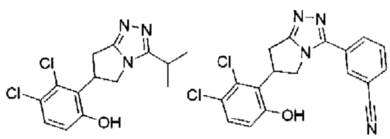 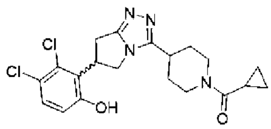

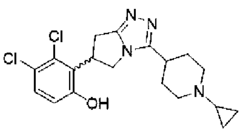 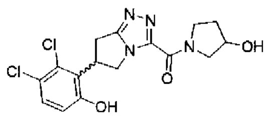 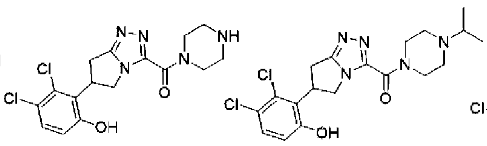 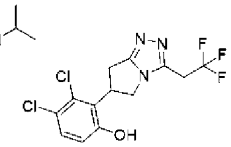

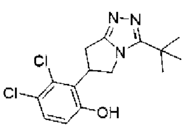 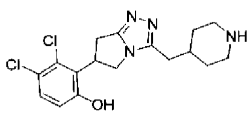 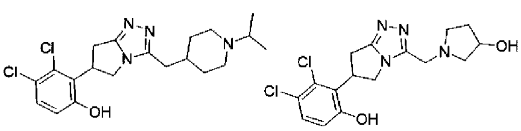 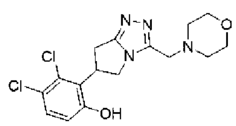

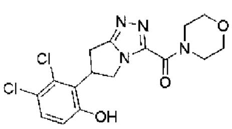 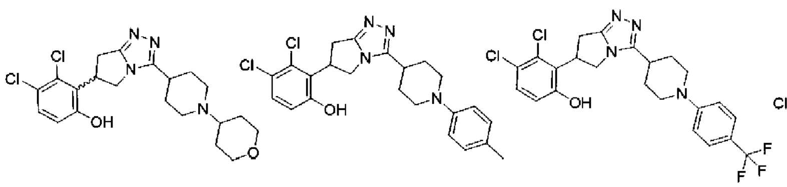 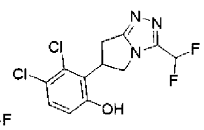

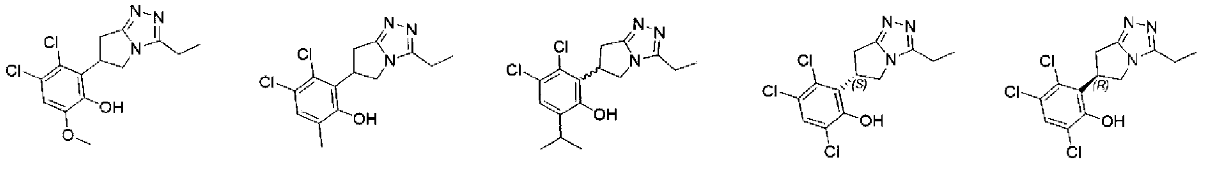

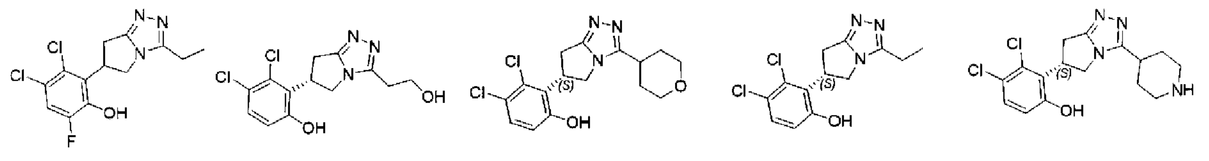

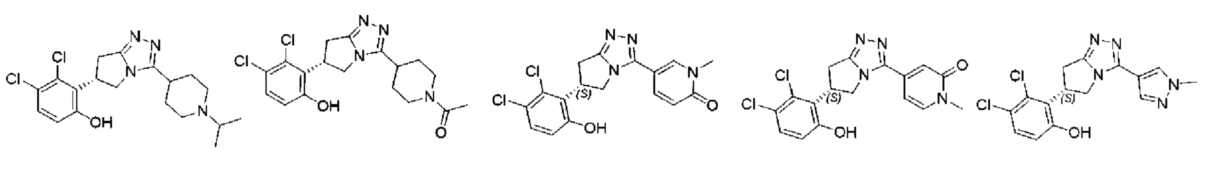

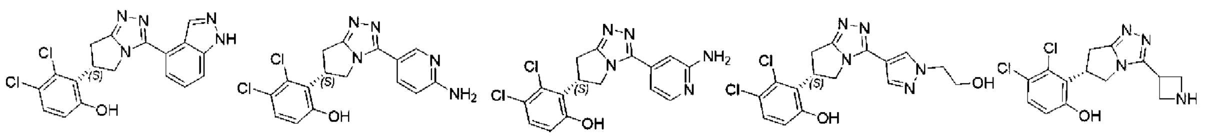

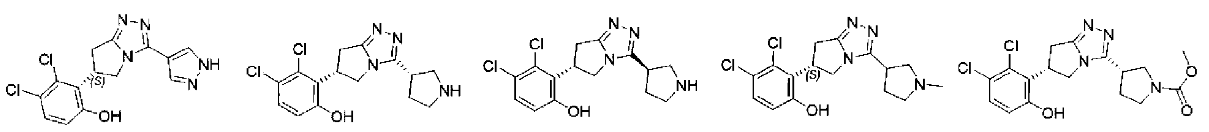

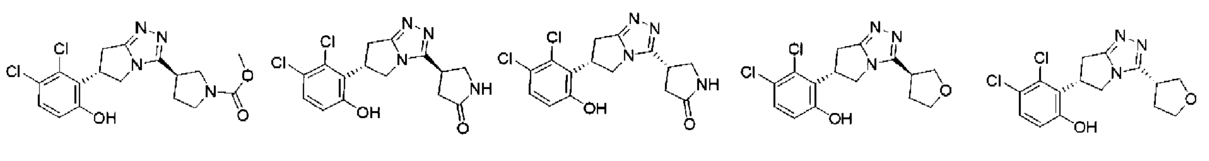

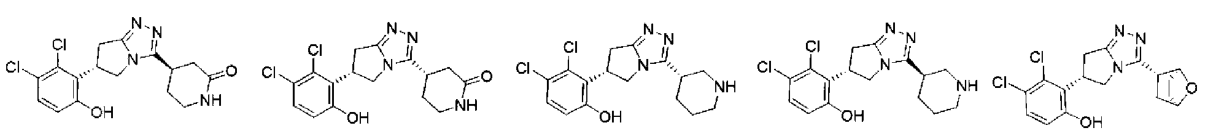

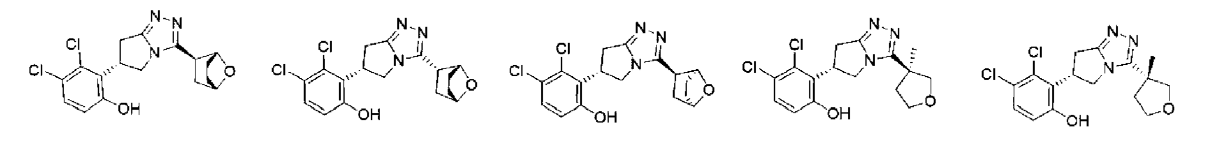

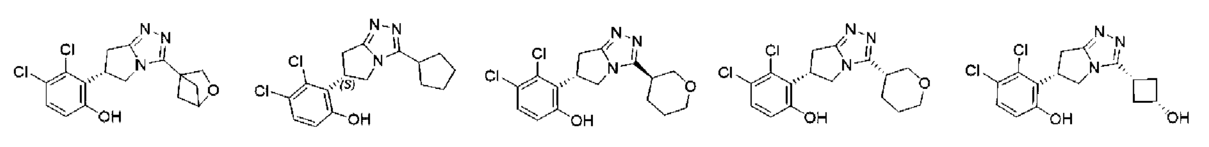

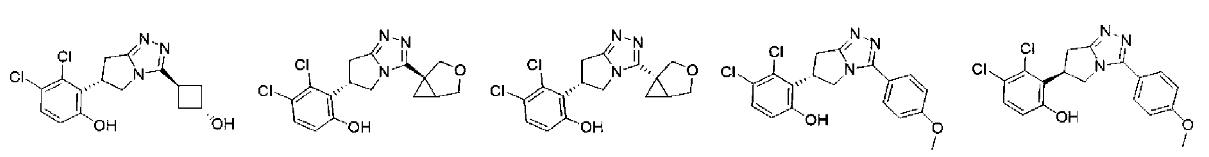

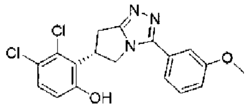
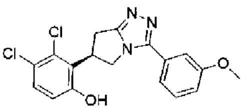
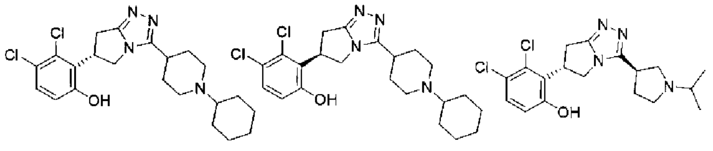

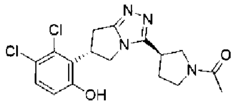
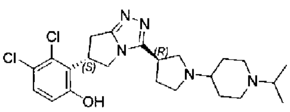
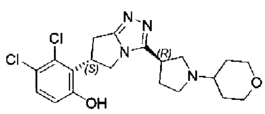
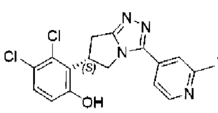
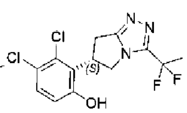

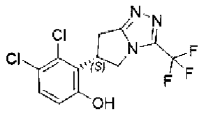
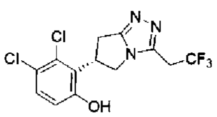
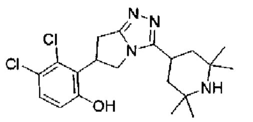
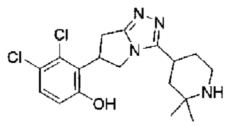
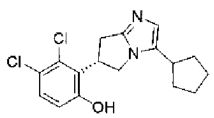

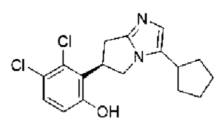
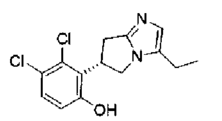
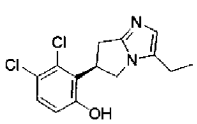
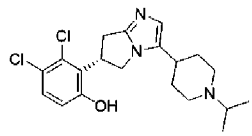
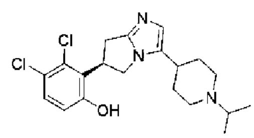

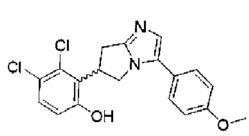
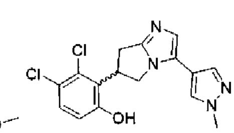
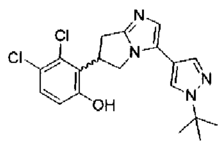
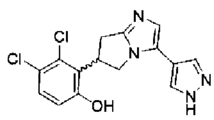
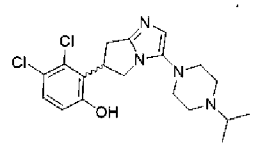

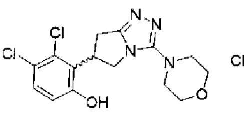
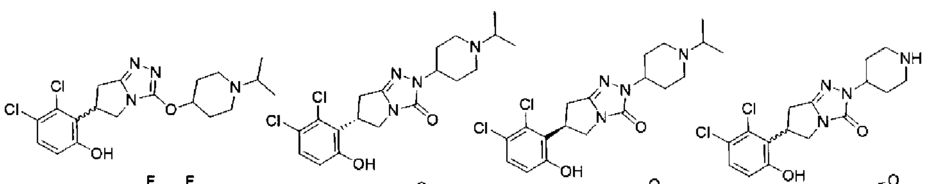

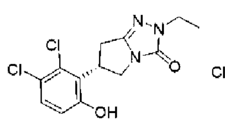
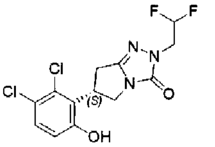
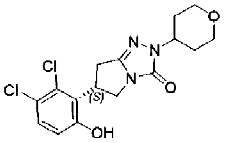
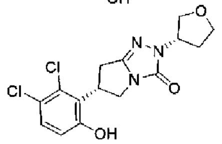
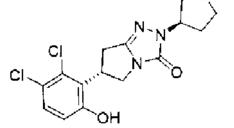

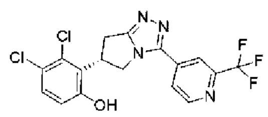
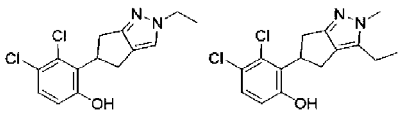
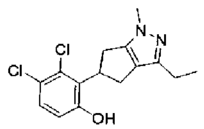
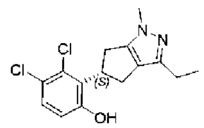

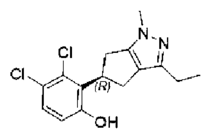
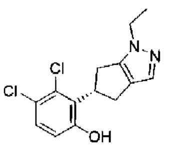
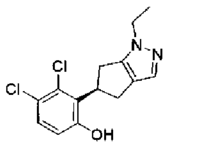
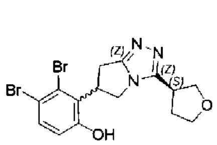
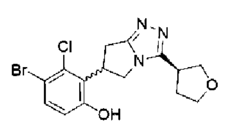

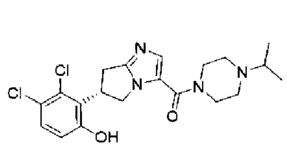

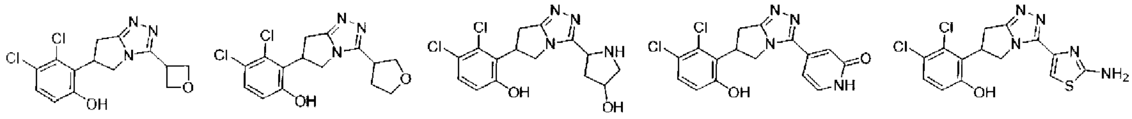

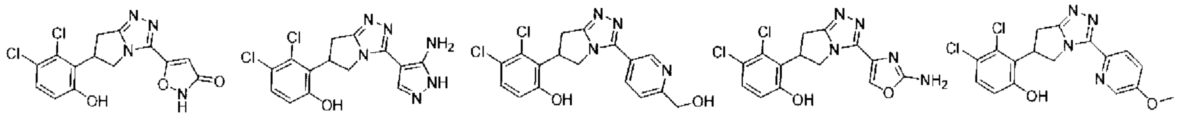

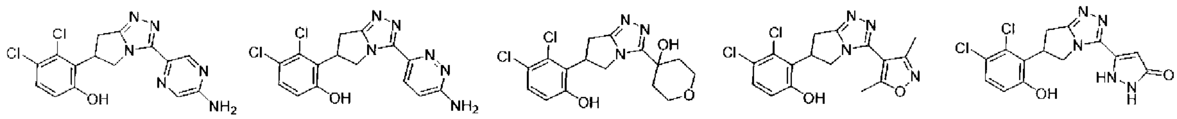

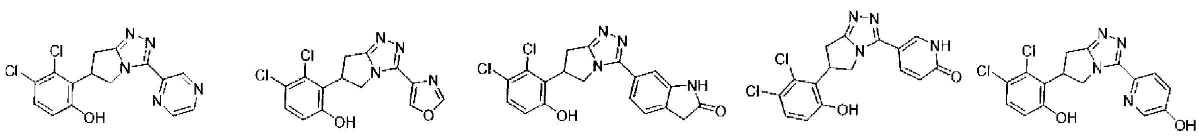

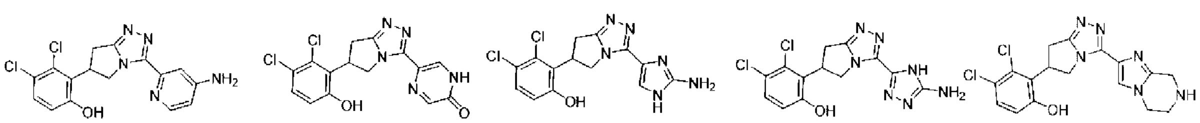

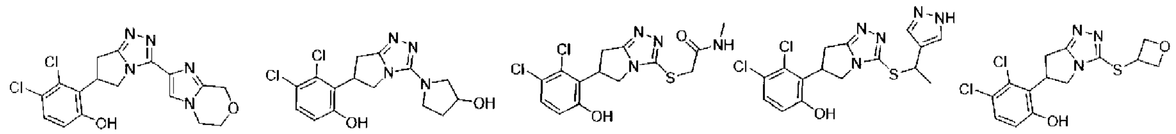

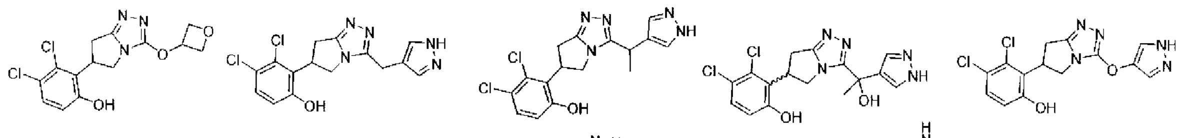

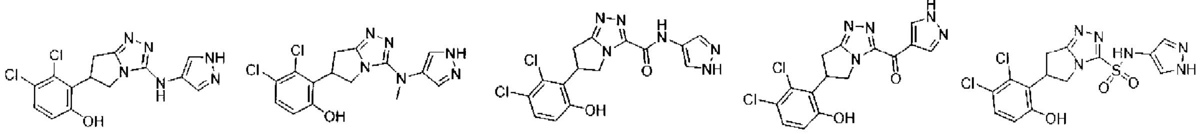

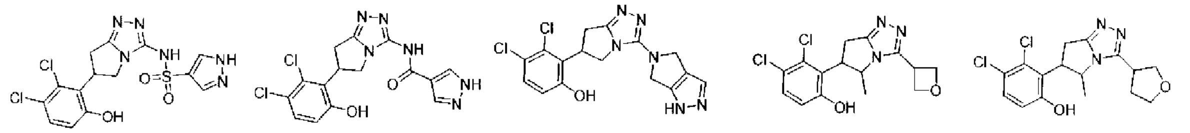

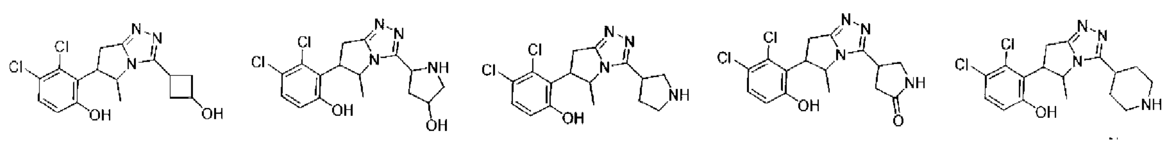

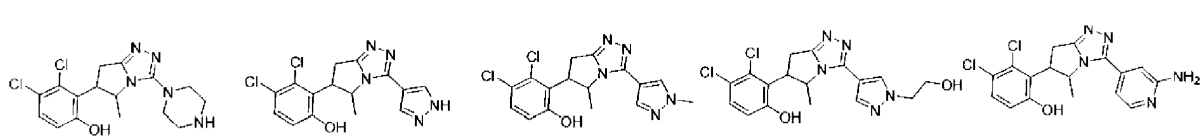

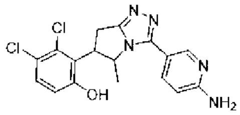
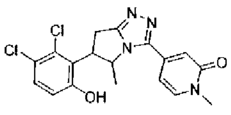
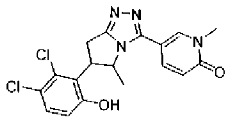
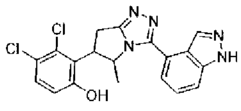
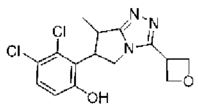
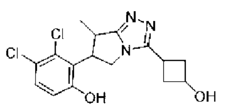
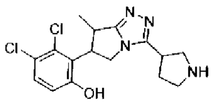
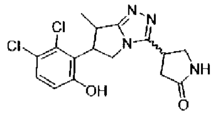
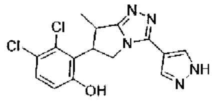
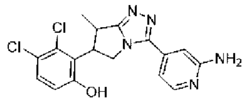
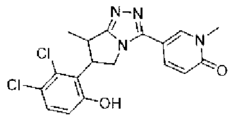
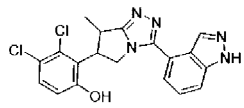

.

**12.** A pharmaceutical composition, comprising the compound or the pharmaceutically acceptable salt or the deuterated compound thereof according to any one of claims 1-11, and a pharmaceutically acceptable carrier.

**13.** The compound or the pharmaceutically acceptable salt or the deuterated compound thereof according to any one of claims 1-11 or the pharmaceutical composition according to claim 12 for use as a medicament.

**14.** The compound or the pharmaceutically acceptable salt or the deuterated compound thereof according to any one of claims 1-11 or the pharmaceutical composition according to claim 12 for use in preventing and/or treating a Kv1.3-associated disease, preferably, the disease includes any of a group of immune and inflammatory diseases, such as multiple sclerosis, inflammatory bowel disease, ulcerative colitis, Crohn's disease, rheumatoid arthritis, type 1 diabetes, psoriasis and asthma, spondylitis, and periodontitis, as well as obesity, type 2 diabetes, renal fibrosis, Alzheimer's disease, ischemic stroke.

**Patentansprüche**

**1.** Verbindung, dargestellt durch Formel (I) oder ein pharmazeutisch verträgliches Salz oder eine deuterierte Verbindung davon:

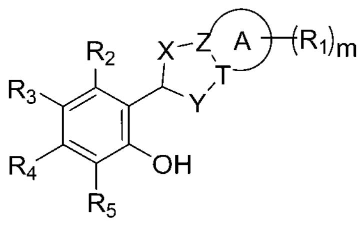

(I)

,

wobei

A ausgewählt ist aus 5-gliedrigem Heteroaryl enthaltend 1-3 Ringheteroatome unabhängig ausgewählt aus N, O und S;
Z und T unabhängig ausgewählt sind aus C und N;
X und Y unabhängig ausgewählt sind aus CRaRb, NRc und O;
Ra und Rb unabhängig ausgewählt sind aus H, OH, $NH_2$, linearem oder verzweigtem $C_{1-4}$-Alkyl, linearem oder verzweigtem $C_{2-4}$-Alkenyl, linearem oder verzweigtem $C_{2-4}$-Alkinyl und linearem oder verzweigtem $C_{1-4}$-Alkyloxy; oder Ra und Rb zusammen mit dem Kohlenstoffatom, an das sie gebunden sind, $C_{3-7}$-Cycloalkyl oder 3- bis 7-gliedriges Heterocycloalkyl enthaltend 1-3 Ringheteroatome unabhängig ausgewählt aus N, O und S bilden;
Rc ausgewählt ist aus H, linearem oder verzweigtem $C_{1-4}$-Alkyl, linearem oder verzweigtem $C_{2-4}$-Alkenyl und $C_{3-7}$-Cycloalkyl;

$R_2$, $R_3$, $R_4$ und $R_5$ unabhängig ausgewählt sind aus H, F, Cl, Br, CN, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkyloxy, linearem oder verzweigtem $C_{2-4}$-Alkenyloxy, linearem oder verzweigtem $C_{2-4}$-Alkynyloxy und $C_{3-7}$-Cycloalkyl;

jedes $R_1$ unabhängig ausgewählt ist aus F, Cl, Br, CN, =O, linearem oder verzweigtem $C_{1-4}$-Alkyl, linearem oder verzweigtem $C_{2-4}$-Alkenyl, linearem oder verzweigtem $C_{2-4}$-Alkinyl, $C_{3-7}$-Cycloalkyl, 3- bis 7-gliedrigem Heterocycloalkyl gebunden über C oder N und enthaltend 1-3 Ringheteroatome unabhängig ausgewählt aus N, O und S, Aryl, 5- bis 11-gliedrigem Heteroaryl enthaltend 1-3 Ringheteroatome unabhängig ausgewählt aus N, O und S, verbrücktem $C_{5-7}$-Cycloalkyl, 5- bis 7-gliedrigem verbrücktem Heterocycloalkyl enthaltend 1-3 Ringheteroatome unabhängig ausgewählt aus N, O und S, einer kondensierten Ringstruktur bestehend aus einem 3-bis 7-gliedrigen Heteroalkanring enthaltend 1-3 Ringheteroatome unabhängig ausgewählt aus N, O und S und einem 3- bis 7-gliedrigen Alkanring, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, einer kondensierten Ringstruktur bestehend aus einem Benzolring und einem 3- bis 7-gliedrigen Heteroalkanring enthaltend 1-3 Ringheteroatome unabhängig ausgewählt aus N, O und S, einer kondensierten Ringstruktur bestehend aus einem 5- bis 7-gliedrigen heteroaromatischen Ring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, linearem oder verzweigtem -$C_{1-4}$-Alkylen-$R_7$, -$OR_7$, linearem oder verzweigtem -O-$C_{1-4}$-Alkylen-$R_7$, -$SR_7$, linearem oder verzweigtem -S-$C_{1-4}$-Alkylen-$R_7$, -N($R_6$)$R_7$, linearem oder verzweigtem -N($R_6$)-$C_{1-4}$-Alkylen-$R_7$, -C(O)$R_7$, linearem oder verzweigtem -C(O)-$C_{1-4}$-Alkylen-$R_7$, -C(O)$R_7$-$OR_7$, -C(O)$R_7$-N($R_6$)$R_7$, - S$(O)_2R_7$, -N($R_6$)C(O)$R_7$, linearem oder verzweigtem -N($R_6$)C(O)-$C_{1-4}$-Alkylen-$R_7$, -C(O)N($R_6$)$R_7$, linearem oder verzweigtem -C(O)N($R_6$)-$C_{1-4}$-Alkylen-$R_7$, -N($R_6$)S$(O)_2R_7$, linearem oder verzweigtem -N($R_6$)S$(O)_2$-$C_{1-4}$-Alkylen-$R_7$, -S$(O)_2$N($R_6$)$R_7$ und linearem oder verzweigtem -S$(O)_2$N($R_6$)-$C_{1-4}$-Alkylen-$R_7$, wobei:

das Alkylen optional substituiert ist mit OH;

das Alkyl, Alkenyl, Alkinyl, Cycloalkyl, Heterocycloalkyl, Aryl, Heteroaryl, verbrückte Cycloalkyl, verbrückte Heterocycloalkyl und die kondensierten Ringstrukturen optional substituiert sind mit einer oder mehreren Gruppen unabhängig ausgewählt aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{2-4}$-Alkenyl optional substituiert mit einer oder mehreren Gruppen unabhängig ausgewählt aus F, Cl und Br, linearem oder verzweigtem $C_{2-4}$-Alkinyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $C_{1-4}$-Alkylen-$NH_2$, linearem oder verzweigtem $C_{2-4}$-Alkenylen-$NH_2$, linearem oder verzweigtem $C_{2-4}$-Alkinylen-$NH_2$, linearem oder verzweigtem $C_{1-4}$-Alkyloxy, linearem oder verzweigtem -C(O)O$C_{1-4}$-Alkyl, linearem oder verzweigtem C(O)O$C_{2-4}$-Alkenyl, linearem oder verzweigtem C(O)O$C_{2-4}$-Alkinyl, -C(O)$R_7$, -S$(O)_2R_7$, -N($R_6$)$R_7$, linearem oder verzweigtem - N($R_6$)-$C_{1-4}$-Alkylen-$R_7$, -N($R_6$)C(O)$R_7$, linearem oder verzweigtem -N($R_6$)C(O)-$C_{1-4}$-Alkylen-$R_7$, -N($R_6$)S$(O)_2R_7$, linearem oder verzweigtem -N($R_6$)S$(O)_2$-$C_{1-4}$-Alkylen-$R_7$, -S$(O)_2$N($R_6$)$R_7$, linearem oder verzweigtem -S$(O)_2$N($R_6$)-$C_{1-4}$-Alkylen-$R_7$, -C(O)N($R_6$)$R_7$, linearem oder verzweigtem -C(O)N($R_6$)-$C_{1-4}$-Alkylen-$R_7$, -P(O)$(R_6)_2$, substituiertem oder unsubstituiertem $C_{3-7}$-Cycloalkyl, substituiertem oder unsubstituiertem Aryl, substituiertem oder unsubstituiertem 3- bis 7-gliedrigen Heterocycloalkyl gebunden über C oder N und enthaltend 1-3 Ringheteroatome unabhängig ausgewählt aus N, O und S und substituiertem oder unsubstituiertem 5- bis 6-gliedrigen Heteroaryl gebunden über C oder N und enthaltend 1-3 Ringheteroatome unabhängig ausgewählt aus N, O und S, wobei Substituentengruppen für das substituierte $C_{3-7}$-Cycloalkyl, substituierte Aryl, substituierte 3- bis 7-gliedrige Heterocycloalkyl und substituierte 5- bis 6-gliedrige Heteroaryl ausgewählt sind aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{2-4}$-Alkenyl optional substituiert mit einer oder mehreren Gruppen unabhängig ausgewählt aus F, Cl und Br, linearem oder verzweigtem $C_{2-4}$-Alkinyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $C_{1-4}$-Alkylen-$NH_2$, linearem oder verzweigtem $C_{2-4}$-Alkenylen-$NH_2$, linearem oder verzweigtem $C_{2-4}$-Alkinylen-$NH_2$ und linearem oder verzweigtem $C_{1-4}$-Alkyloxy; wobei die zwei Substituenten an dem gleichen Ringkohlenstoffatom in dem Cycloalkyl, Heterocycloalkyl und Heteroaryl, zusammen mit dem Kohlenstoffatom, an das sie gebunden sind, optional $C_{3-6}$-Cycloalkyl oder 3- bis 6-gliedriges Heterocycloalkyl enthaltend 1 Ringheteroatom ausgewählt aus N und O bilden können, wobei das gebildete Cycloalkyl oder Heterocycloalkyl optional substituiert ist mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $C_{1-4}$-Alkylen-$NH_2$, linearem oder verzweigtem $C_{2-4}$-Alkenylen-$NH_2$, linearem oder verzweigtem $C_{2-4}$-Alkinylen-$NH_2$, linearem oder verzweigtem $C_{1-4}$-Alkyloxy, $C_{3-6}$-Cycloalkyl und 3- bis 6-gliedrigem Heterocycloalkyl enthaltend 1-3 Ringheteroatome unabhängig ausgewählt aus N, O und S;

$R_6$ ausgewählt ist aus H, linearem oder verzweigtem $C_{1-4}$-Alkyl, linearem oder verzweigtem $C_{2-4}$-Alkenyl und linearem oder verzweigtem $C_{2-4}$-Alkinyl;

$R_7$ unabhängig ausgewählt ist aus H, OH, $NH_2$, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkyloxy, linearem oder verzweigtem $C_{1-4}$-Alkylen-OH, $C_{3-7}$-Cycloalkyl, 3- bis 7-gliedrigem Heterocycloalkyl gebunden über C oder N und enthaltend 1-3 Ringheteroatome unabhängig ausgewählt aus N, O und S, Aryl und 5- bis 11-gliedrigem Heteroaryl enthaltend 1-3 Ringheteroatome unabhängig ausgewählt aus N, O und S, wobei das Alkyl, Alkenyl, Alkinyl, Cycloalkyl, Heterocycloalkyl, Aryl und Heteroaryl optional substituiert sind mit einer oder mehreren Gruppen unabhängig ausgewählt aus F, Cl, Br, OH, CN, $NH_2$, =O, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem - $C(O)OC_{1-4}$-Alkyl, linearem oder verzweigtem $C(O)OC_{2-4}$-Alkenyl, linearem oder verzweigtem $C(O)OC_{2-4}$-Alkinyl, linearem oder verzweigtem $C(O)NHC_{1-4}$-Alkyl, linearem oder verzweigtem $C(O)NHC_{2-4}$-Alkenyl und linearem oder verzweigtem $C(O)NHC_{2-4}$-Alkinyl;

m ausgewählt ist aus 1 und 2.

**2.** Verbindung dargestellt durch Formel (I) oder das pharmazeutisch verträgliche Salz oder die deuterierte Verbindung davon nach Anspruch 1:

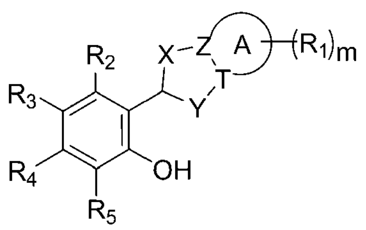

(I)

,

wobei

A ausgewählt ist aus 5-gliedrigem Heteroaryl enthaltend 1-3 Ringheteroatome unabhängig ausgewählt aus N, O und S;

Z und T unabhängig ausgewählt sind aus C und N;

X und Y unabhängig ausgewählt sind aus CRaRb, NRc und O;

Ra und Rb unabhängig ausgewählt sind aus H, OH, $NH_2$, linearem oder verzweigtem $C_{1-4}$-Alkyl, linearem oder verzweigtem $C_{2-4}$-Alkenyl, linearem oder verzweigtem $C_{2-4}$-Alkinyl und linearem oder verzweigtem $C_{1-4}$-Alkyloxy oder Ra und Rb zusammen mit dem Kohlenstoffatom, an das sie gebunden sind, $C_{3-7}$-Cycloalkyl oder 3- bis 7-gliedriges Heterocycloalkyl enthaltend 1-3 Ringheteroatome unabhängig ausgewählt aus N, O und S bilden;

Rc ausgewählt ist aus H, linearem oder verzweigtem $C_{1-4}$-Alkyl, linearem oder verzweigtem $C_{2-4}$-Alkenyl und $C_{3-7}$-Cycloalkyl;

$R_2$, $R_3$, $R_4$ und $R_5$ unabhängig ausgewählt sind aus H, F, Cl, Br, CN, linearem oder verzweigtem $C_{1-4}$-Alkyl, linearem oder verzweigtem $C_{2-4}$-Alkenyl, linearem oder verzweigtem $C_{2-4}$-Alkinyl und $C_{3-7}$-Cycloalkyl;

jedes $R_1$ unabhängig ausgewählt ist aus F, Cl, Br, CN, linearem oder verzweigtem $C_{1-4}$-Alkyl, linearem oder verzweigtem $C_{2-4}$-Alkenyl, linearem oder verzweigtem $C_{2-4}$-Alkinyl, $C_{3-7}$-Cycloalkyl, 3- bis 7-gliedrigem Heterocycloalkyl gebunden über C oder N und enthaltend 1-3 Ringheteroatome unabhängig ausgewählt aus N, O und S, Aryl, 5- bis 11-gliedrigem Heteroaryl enthaltend 1-3 Ringheteroatome unabhängig ausgewählt aus N, O und S, verbrücktem $C_{5-7}$-Cycloalkyl, 5- bis 7-gliedrigem verbrücktem Heterocycloalkyl enthaltend 1-3 Ringheteroatome unabhängig ausgewählt aus N, O und S, einer kondensierten Ringstruktur bestehend aus einem 3-bis 7-

gliedrigen Heteroalkanring enthaltend 1-3 Ringheteroatome unabhängig ausgewählt aus N, O und S und einem 3- bis 7-gliedrigen Alkanring, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, einer kondensierten Ringstruktur bestehend aus einem Benzolring und einem 3- bis 7-gliedrigen Heteroalkanring enthaltend 1-3 Ringheteroatome unabhängig ausgewählt aus N, O und S, einer kondensierten Ringstruktur bestehend aus einem 5- bis 7-gliedrigen heteroaromatischen Ring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, linearem oder verzweigtem -$C_{1-4}$-Alkylen-$R_7$, -$OR_7$, linearem oder verzweigtem -O-$C_{1-4}$-Alkylen-$R_7$, -$SR_7$, linearem oder verzweigtem -S-$C_{1-4}$-Alkylen-$R_7$, -N($R_6$)$R_7$, linearem oder verzweigtem -N($R_6$)-$C_{1-4}$-Alkylen-$R_7$, -C(O)$R_7$, linearem oder verzweigtem -C(O)-$C_{1-4}$-Alkylen-$R_7$, -C(O)$R_7$-$OR_7$, -C(O)$R_7$-N($R_6$)$R_7$, - S(O)$_2R_7$, -N($R_6$)C(O)$R_7$, linearem oder verzweigtem -N($R_6$)C(O)-$C_{1-4}$-Alkylen-$R_7$, -C(O)N($R_6$)$R_7$, linearem oder verzweigtem -C(O)N($R_6$)-$C_{1-4}$-Alkylen-$R_7$, -N($R_6$)S(O)$_2R_7$, linearem oder verzweigtem -N($R_6$)S(O)$_2$-$C_{1-4}$-Alkylen-$R_7$, -S(O)$_2$N($R_6$)$R_7$ und linearem oder verzweigtem -S(O)$_2$N($R_6$)-$C_{1-4}$-Alkylen-$R_7$, wobei: das Alkylen optional substituiert ist mit OH; das Alkyl, Alkenyl, Alkinyl, Cycloalkyl, Heterocycloalkyl, Aryl, Heteroaryl, verbrückte Cycloalkyl, verbrückte Heterocycloalkyl und die kondensierten Ringstrukturen optional substituiert sind mit einer oder mehreren Gruppen unabhängig ausgewählt aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{2-4}$-Alkenyl optional substituiert mit einer oder mehreren Gruppen unabhängig ausgewählt aus F, Cl und Br, linearem oder verzweigtem $C_{2-4}$-Alkinyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $C_{1-4}$-Alkylen-$NH_2$, linearem oder verzweigtem $C_{2-4}$-Alkenylen-$NH_2$, linearem oder verzweigtem $C_{2-4}$-Alkinylen-$NH_2$, linearem oder verzweigtem $C_{1-4}$-Alkyloxy, linearem oder verzweigtem - C(O)O$C_{1-4}$-Alkyl, linearem oder verzweigtem C(O)O$C_{2-4}$-Alkenyl, linearem oder verzweigtem C(O)O$C_{2-4}$-Alkinyl, -C(O)$R_7$, -S(O)$_2R_7$, -N($R_6$)$R_7$, linearem oder verzweigtem -N($R_6$)-$C_{1-4}$-Alkylen-$R_7$, -N($R_6$)C(O)$R_7$, linearem oder verzweigtem -N($R_6$)C(O)-$C_{1-4}$-Alkylen-$R_7$, - N($R_6$)S(O)$_2R_7$, linearem oder verzweigtem -N($R_6$)S(O)$_2$-$C_{1-4}$-Alkylen-$R_7$, -S(O)$_2$N($R_6$)$R_7$, linearem oder verzweigtem -S(O)$_2$N($R_6$)-$C_{1-4}$-Alkylen-$R_7$, -C(O)N($R_6$)$R_7$, linearem oder verzweigtem -C(O)N($R_6$)-$C_{1-4}$-Alkylen-$R_7$, -P(O)($R_6$)$_2$, $C_{3-7}$-Cycloalkyl und 3- bis 7-gliedrigem Heterocycloalkyl gebunden über C oder N und enthaltend 1-3 Ringheteroatome unabhängig ausgewählt aus N, O und S; wobei die zwei Substituenten auf dem gleichen Ringkohlenstoffatom in dem Cycloalkyl, Heterocycloalkyl und Heteroaryl, zusammen mit dem Kohlenstoffatom, an das sie gebunden sind, optional $C_{3-6}$-Cycloalkyl oder 3- bis 6-gliedriges Heterocycloalkyl enthaltend 1 Ringheteroatom ausgewählt aus N und O bilden können, wobei das gebildete Cycloalkyl oder Heterocycloalkyl optional substituiert ist mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $C_{1-4}$-Alkylen-$NH_2$, linearem oder verzweigtem $C_{2-4}$-Alkenylen-$NH_2$, linearem oder verzweigtem $C_{2-4}$-Alkinylen-$NH_2$, linearem oder verzweigtem $C_{1-4}$-Alkyloxy, $C_{3-6}$-Cycloalkyl und 3- bis 6-gliedrigem Heterocycloalkyl enthaltend 1-3 Ringheteroatome unabhängig ausgewählt aus N, O und S;

$R_6$ ausgewählt ist aus H, linearem oder verzweigtem $C_{1-4}$-Alkyl, linearem oder verzweigtem $C_{2-4}$-Alkenyl und linearem oder verzweigtem $C_{2-4}$-Alkinyl;

$R_7$ unabhängig ausgewählt ist aus H, OH, $NH_2$, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkyloxy, linearem oder verzweigtem $C_{1-4}$-Alkylen-OH, $C_{3-7}$-Cycloalkyl, 3- bis 7-gliedrigem Heterocycloalkyl gebunden über C oder N und enthaltend 1-3 Ringheteroatome unabhängig ausgewählt aus N, O und S, Aryl und 5- bis 6-gliedrigem Heteroaryl enthaltend 1-3 Ringheteroatome unabhängig ausgewählt aus N, O und S, wobei das Alkyl, Alkenyl, Alkinyl, Cycloalkyl, Heterocycloalkyl, Aryl und Heteroaryl optional substituiert sind mit einer oder mehreren Gruppen unabhängig ausgewählt aus F, Cl, Br, OH, CN, $NH_2$, =O, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem - C(O)O$C_{1-4}$-Alkyl, linearem oder verzweigtem C(O)O$C_{2-4}$-Alkenyl, linearem oder verzweigtem C(O)O$C_{2-4}$-Alkinyl, linearem oder verzweigtem C(O)NH$C_{1-4}$-Alkyl, linearem oder verzweigtem C(O)NH$C_{2-4}$-Alkenyl und linearem oder verzweigtem C(O)NH$C_{2-4}$-Alkinyl;

m ausgewählt ist aus 1 und 2.

**3.** Verbindung oder das pharmazeutisch verträgliche Salz oder die deuterierte Verbindung davon nach Anspruch 1 oder

2, **dadurch gekennzeichnet, dass**

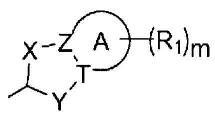

ausgewählt ist aus

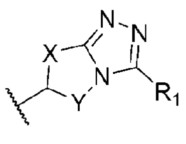 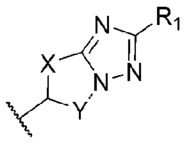 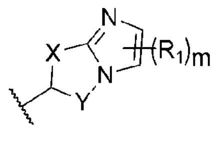 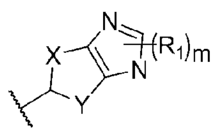 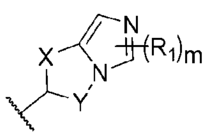

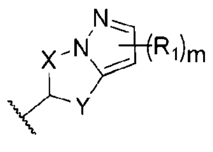 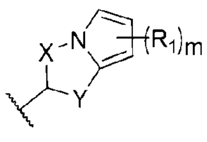 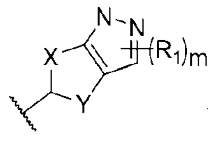 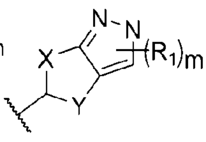

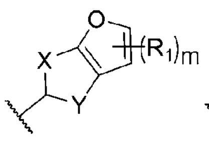 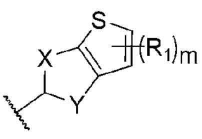 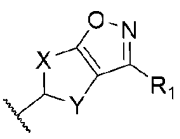 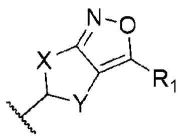 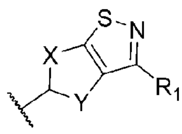 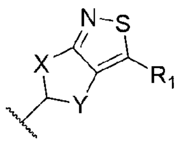 .

**4.** Verbindung oder das pharmazeutisch verträgliche Salz oder die deuterierte Verbindung davon nach Anspruch 3, **dadurch gekennzeichnet, dass** X und Y CRaRb sind.

**5.** Verbindung oder das pharmazeutisch verträgliche Salz oder die deuterierte Verbindung davon nach Anspruch 1, **dadurch gekennzeichnet, dass** die Verbindung eine Struktur dargestellt durch Formel (II) aufweist:

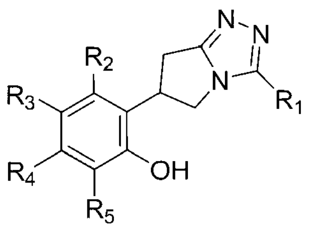

(II) ,

wobei:

$R_1$ ausgewählt ist aus linearem oder verzweigtem $C_{1-4}$-Alkyl, linearem oder verzweigtem $C_{2-4}$-Alkenyl, linearem oder verzweigtem $C_{2-4}$-Alkinyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl, 5- bis 11-gliedrigem Heteroaryl enthaltend 1-3 Ringheteroatome unabhängig ausgewählt aus N, O und S, verbrücktem $C_{5-7}$-Cycloalkyl, 5- bis 7-gliedrigem verbrücktem Heterocycloalkyl enthaltend 1-3 Ringheteroatome unabhängig ausgewählt aus N, O und S, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Alkanring, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, einer kondensierten Ringstruktur bestehend aus einem Benzolring und einem 3- bis 7-gliedrigen Heteroalkanring enthaltend 1-3 Ringheteroatome unabhängig ausgewählt aus N, O und S, einer kondensierten Ringstruktur bestehend aus einem 5-bis 7-gliedrigen heteroaromatischen Ring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, linearem oder verzweigtem $-C_{1-4}$-Alkylen-$R_7$, $-OR_7$, linearem oder verzweigtem $-O-C_{1-4}$-Alkylen-$R_7$, $-SR_7$, linearem oder verzweigtem $-S-C_{1-4}$-Alkylen-$R_7$, $-N(R_6)R_7$,

linearem oder verzweigtem $-N(R_6)-C_{1-4}$-Alkylen-$R_7$, $-C(O)R_7$, linearem oder verzweigtem $-C(O)-C_{1-4}$-Alkylen-$R_7$, $-N(R_6)C(O)R_7$, linearem oder verzweigtem - $N(R_6)C(O)-C_{1-4}$-Alkylen-$R_7$, $-C(O)N(R_6)R_7$, linearem oder verzweigtem $-C(O)N(R_6)-C_{1-4}$-Alkylen-$R_7$, $-N(R_6)S(O)_2R_7$, linearem oder verzweigtem $-N(R_6)S(O)_2-C_{1-4}$-Alkylen-$R_7$, - $S(O)_2N(R_6)R_7$ und linearem oder verzweigtem $-S(O)_2N(R_6)-C_{1-4}$-Alkylen-$R_7$, wobei:

das Alkylen optional mit OH substituiert ist;
das Alkyl, Alkenyl, Alkinyl, Cycloalkyl, Heterocycloalkyl, Phenyl, Heteroaryl, verbrückte Cycloalkyl, verbrückte Heterocycloalkyl und die kondensierten Ringstrukturen optional substituiert sind mit einer oder mehreren Gruppen unabhängig ausgewählt aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{2-4}$-Alkenyl optional substituiert mit einer oder mehreren Gruppen unabhängig ausgewählt aus F, Cl und Br, linearem oder verzweigtem $C_{2-4}$-Alkinyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $C_{1-4}$-Alkyloxy, linearem oder verzweigtem $-C(O)OC_{1-4}$-Alkyl, linearem oder verzweigtem $C(O)OC_{2-4}$-Alkenyl, linearem oder verzweigtem $C(O)OC_{2-4}$-Alkinyl, linearem oder verzweigtem $C(O)NHC_{1-4}$-Alkyl, linearem oder verzweigtem $C(O)NHC_{2-4}$-Alkenyl, linearem oder verzweigtem $C(O)NHC_{2-4}$-Alkinyl, $-C(O)R_7$, substituiertem oder unsubstituiertem $C_{3-7}$-Cycloalkyl, substituiertem oder unsubstituiertem Aryl, substituiertem oder unsubstituiertem 3- bis 7-gliedrigem Heterocycloalkyl gebunden über C oder N und enthaltend 1-3 Ringheteroatome unabhängig ausgewählt aus N, O und S und substituiertem oder unsubstituiertem 5- bis 6-gliedrigem Heteroaryl gebunden über C oder N und enthaltend 1-3 Ringheteroatome unabhängig ausgewählt aus N, O und S, wobei Substituentengruppen für das substituierte $C_{3-7}$-Cycloalkyl, substituierte Aryl, substituierte 3- bis 7-gliedrige Heterocycloalkyl und substituierte 5- bis 6-gliedrige Heteroaryl ausgewählt sind aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{1-4}$-Alkylen-$NH_2$, linearem oder verzweigtem $C_{2-4}$-Alkenylen-$NH_2$, linearem oder verzweigtem $C_{2-4}$-Alkinylen-$NH_2$ und linearem oder verzweigtem $C_{1-4}$-Alkyloxy;
$R_6$ ausgewählt ist aus H, linearem oder verzweigtem $C_{1-4}$-Alkyl, linearem oder verzweigtem $C_{2-4}$-Alkenyl und linearem oder verzweigtem $C_{2-4}$-Alkinyl;
$R_7$ ausgewählt ist aus linearem oder verzweigtem $C_{1-4}$-Alkyl, linearem oder verzweigtem $C_{2-4}$-Alkenyl, linearem oder verzweigtem $C_{2-4}$-Alkinyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl und 5- bis 11-gliedrigem Heteroaryl enthaltend 1-3 Ringheteroatome unabhängig ausgewählt aus N, O und S; wobei das Alkyl, Alkenyl, Alkinyl, Cycloalkyl, Heterocycloalkyl, Phenyl und Heteroaryl optional substituiert sind mit einer oder mehreren Gruppen unabhängig ausgewählt aus OH, CN, $NH_2$, =O, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem $-C(O)OC_{1-4}$-Alkyl, linearem oder verzweigtem $C(O)OC_{2-4}$-Alkenyl, linearem oder verzweigtem $C(O)OC_{2-4}$-Alkinyl, linearem oder verzweigtem $C(O)NHC_{1-4}$-Alkyl, linearem oder verzweigtem $C(O)NHC_{2-4}$-Alkenyl und linearem oder verzweigtem $C(O)NHC_{2-4}$-Alkinyl;
$R_2$ und $R_3$ unabhängig ausgewählt sind aus F, Cl und Br;
$R_4$ und $R_5$ H sind.

6. Verbindung oder das pharmazeutisch verträgliche Salz oder die deuterierte Verbindung davon nach Anspruch 5, **dadurch gekennzeichnet, dass** in Formel (II):

$R_1$ ausgewählt ist aus linearem oder verzweigtem $C_{1-4}$-Alkyl, linearem oder verzweigtem $C_{2-4}$-Alkenyl, linearem oder verzweigtem $C_{2-4}$-Alkinyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl, 5- bis 11-gliedrigem Heteroaryl enthaltend 1-3 Ringheteroatome unabhängig ausgewählt aus N, O und S, linearem oder verzweigtem $-C_{1-4}$-Alkylen-$R_7$, $-OR_7$, linearem oder verzweigtem $-O-C_{1-4}$-Alkylen-$R_7$, $-SR_7$, linearem oder verzweigtem -S-$C_{1-4}$-Alkylen-$R_7$, $-N(R_6)R_7$, linearem oder verzweigtem $-N(R_6)-C_{1-4}$-Alkylen-$R_7$, $-C(O)R_7$, linearem oder verzweigtem $-C(O)-C_{1-4}$-Alkylen-$R_7$, $-N(R_6)C(O)R_7$, linearem oder verzweigtem $-N(R_6)C(O)-C_{1-4}$-Alkylen-$R_7$, $-C(O)N(R_6)R_7$, linearem oder verzweigtem - $C(O)N(R_6)-C_{1-4}$-Alkylen-$R_7$, $-N(R_6)S(O)_2R_7$, linearem oder verzweigtem $-N(R_6)S(O)_2-C_{1-4}$-Alkylen-$R_7$, $-S(O)_2N(R_6)R_7$ und linearem oder verzweigtem $-S(O)_2N(R_6)-C_{1-4}$-Alkylen-$R_7$; wobei das Alkylen optional substituiert ist mit OH; das Alkyl, Alkenyl, Alkinyl, Cycloalkyl, Heterocycloalkyl, Phenyl und Heteroaryl optional substituiert sind mit einer oder mehreren Gruppen unabhängig ausgewählt aus OH, CN, $NH_2$, =O, linearem oder verzweigtem $C_{1-4}$-Alkyl, linearem oder verzweigtem $C_{2-4}$-Alkenyl, linearem oder

verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem -C(O)O$C_{1-4}$-Alkyl, linearem oder verzweigtem C(O)O$C_{2-4}$-Alkenyl, linearem oder verzweigtem C(O)O$C_{2-4}$-Alkinyl, linearem oder verzweigtem C(O)NH$C_{1-4}$-Alkyl, linearem oder verzweigtem C(O)NH$C_{2-4}$-Alkenyl und linearem oder verzweigtem C(O)NH$C_{2-4}$-Alkinyl;

$R_6$ ausgewählt ist aus H, linearem oder verzweigtem $C_{1-4}$-Alkyl, linearem oder verzweigtem $C_{2-4}$-Alkenyl und linearem oder verzweigtem $C_{2-4}$-Alkinyl;

$R_7$ ausgewählt ist aus linearem oder verzweigtem $C_{1-4}$-Alkyl, linearem oder verzweigtem $C_{2-4}$-Alkenyl, linearem oder verzweigtem $C_{2-4}$-Alkinyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl und 5- bis 11-gliedrigem Heteroaryl enthaltend 1-3 Ringheteroatome unabhängig ausgewählt aus N, O und S; wobei das Alkyl, Alkenyl, Alkinyl, Cycloalkyl, Heterocycloalkyl, Phenyl und Heteroaryl optional substituiert sind mit einer oder mehreren Gruppen unabhängig ausgewählt aus OH, CN, $NH_2$, =O, linearem oder verzweigtem $C_{1-4}$-Alkyl, linearem oder verzweigtem $C_{2-4}$-Alkenyl, linearem oder verzweigtem $C_{2-4}$-Alkinyl, linearem oder verzweigtem $C_{1-4}$-Alkylen-OH, linearem oder verzweigtem $C_{2-4}$-Alkenylen-OH, linearem oder verzweigtem $C_{2-4}$-Alkinylen-OH, linearem oder verzweigtem -C(O)O$C_{1-4}$-Alkyl, linearem oder verzweigtem C(O)O$C_{2-4}$-Alkenyl, linearem oder verzweigtem C(O)O$C_{2-4}$-Alkinyl, linearem oder verzweigtem C(O)NH$C_{1-4}$-Alkyl, linearem oder verzweigtem C(O) NH$C_{2-4}$-Alkenyl und linearem oder verzweigtem C(O)NH$C_{2-4}$-Alkinyl;

$R_2$ und $R_3$ unabhängig ausgewählt sind aus F, Cl und Br;

$R_4$ und $R_5$ H sind;

wobei bevorzugt in Formel (II):

$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S,

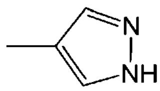
,

Pyridinyl,

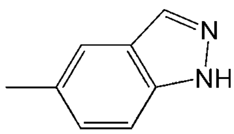
,

linearem oder verzweigtem -$C_{1-4}$-Alkylen-$R_7$, -O$R_7$, -S$R_7$, linearem oder verzweigtem -S-$C_{1-4}$-Alkylen-$R_7$, -N($R_6$)$R_7$, -C(O)$R_7$, -N($R_6$)C(O)$R_7$, -C(O)N($R_6$)$R_7$, -N($R_6$)S(O)$_2R_7$ und -S(O)$_2$N($R_6$)$R_7$; wobei das Alkylen optional substituiert ist mit OH; das Alkyl, Cycloalkyl, Heterocycloalkyl,

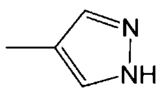
,

Pyridinyl und

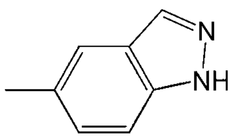

optional substituiert sind mit einer oder mehreren Gruppen unabhängig ausgewählt aus OH, CN, $NH_2$, =O, linearem oder verzweigtem $C_{1-4}$-Alkyl, linearem oder verzweigtem $C_{1-4}$-Alkylen-OH, linearem oder verzweigtem -C(O)O$C_{1-4}$-Alkyl und linearem oder verzweigtem C(O)NH$C_{1-4}$-Alkyl;

$R_6$ ausgewählt ist aus H und linearem oder verzweigtem $C_{1-4}$-Alkyl;
$R_7$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S,

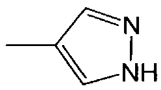,

Pyridinyl und

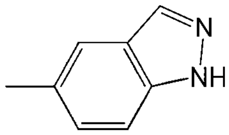;

wobei das Alkyl, Cycloalkyl, Heterocycloalkyl,

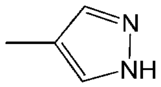,

Pyridinyl und

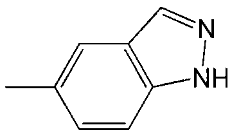

optional substituiert sind mit einer oder mehreren Gruppen unabhängig ausgewählt aus OH, CN, $NH_2$, =O, linearem oder verzweigtem $C_{1-4}$-Alkyl, linearem oder verzweigtem $C_{1-4}$-Alkylen-OH, linearem oder verzweigtem -C(O)O$C_{1-4}$-Alkyl und linearem oder verzweigtem C(O)NH$C_{1-4}$-Alkyl;
$R_2$ und $R_3$ unabhängig ausgewählt sind aus F, Cl und Br;
$R_4$ und $R_5$ H sind;
wobei ferner bevorzugt in Formel (II):

$R_1$ lineares oder verzweigtes$C_{1-4}$-Alkyl substituiert mit einem oder mehreren OHs ist;
$R_2$ und $R_3$ unabhängig ausgewählt sind aus F, Cl und Br;
$R_4$ und $R_5$ H sind;
wobei bevorzugter in Formel (II):

$R_1$ lineares oder verzweigtes $C_{1-4}$-Alkyl substituiert mit einem OH ist;
$R_2$ und $R_3$ Cl sind;
$R_4$ und $R_5$ H sind.

7. Verbindung oder das pharmazeutisch verträgliche Salz oder die deuterierte Verbindung davon nach Anspruch 1, **dadurch gekennzeichnet, dass** die Verbindung eine Struktur dargestellt durch Formel (II) aufweist:

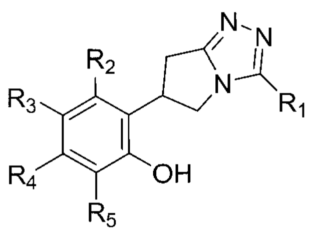

(II),

wobei:
$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl, 5- bis 11-gliedrigem Heteroaryl enthaltend 1-3 Ringheteroatome unabhängig ausgewählt aus N, O und S, verbrücktem $C_{5-7}$-Cycloalkyl, 5- bis 7-gliedrigem verbrücktem Heterocycloalkyl enthaltend 1-3 Ringheteroatome unabhängig ausgewählt aus N, O und S, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Alkanring, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, einer kondensierten Ringstruktur bestehend aus einem Benzolring und einem 3- bis 7-gliedrigen Heteroalkanring enthaltend 1-3 Ringheteroatome unabhängig ausgewählt aus N, O und S, einer kondensierten Ringstruktur bestehend aus einem 5-bis 7-gliedrigen heteroaromatischen Ring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, linearem oder verzweigtem -$C_{1-4}$-Alkylen-$R_7$, -$OR_7$, linearem oder verzweigtem -O-$C_{1-4}$-Alkylen-$R_7$, -C(O)$R_7$ und linearem oder verzweigtem -C(O)-$C_{1-4}$-Alkylen-$R_7$, wobei:

das Alkylen optional substituiert ist mit OH;
das Alkyl, Cycloalkyl, Heterocycloalkyl, Phenyl, Heteroaryl, verbrückte Cycloalkyl, verbrückte Heterocycloalkyl und die kondensierten Ringstrukturen optional substituiert sind mit einer oder mehreren Gruppen unabhängig ausgewählt aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{1-4}$-Alkyloxy, linearem oder verzweigtem -C(O)O$C_{1-4}$-Alkyl, -C(O)$R_7$, substituiertem oder unsubstituiertem $C_{3-7}$-Cycloalkyl und substituiertem oder unsubstituiertem 3-bis 7-gliedrigem Heterocycloalkyl gebunden über C oder N und enthaltend 1-3 Ringheteroatome unabhängig ausgewählt aus N, O und S, wobei Substituentengruppen für das substituierte $C_{3-7}$-Cycloalkyl und substituierte 3- bis 7-gliedrige Heterocycloalkyl ausgewählt sind aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{1-4}$-Alkylen-$NH_2$ und linearem oder verzweigtem $C_{1-4}$-Alkyloxy;
$R_7$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl und 5- bis 11-gliedrigem Heteroaryl enthaltend 1-3 Ringheteroatome unabhängig ausgewählt aus N, O und S; wobei das Alkyl, Cycloalkyl, Heterocycloalkyl, Phenyl und Heteroaryl optional substituiert sind mit einer oder mehreren Gruppen unabhängig ausgewählt aus OH, linearem oder verzweigtem $C_{1-4}$-Alkyl und linearem oder verzweigtem $C_{1-4}$-Alkylen-OH;
$R_2$ und $R_3$ unabhängig ausgewählt sind aus F, Cl und Br;
$R_4$ und $R_5$ H sind;
wobei bevorzugt in Formel (II):

$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-2 Ringheteroatome unabhängig ausgewählt aus N und O, Phenyl,

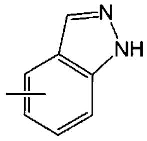

,

Pyridinyl,

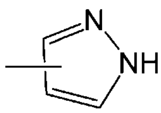

,

5- bis 7-gliedrigem verbrücktem Heterocycloalkyl enthaltend 1 Ringheteroatom von O, einer kondensierten Ringstruktur bestehend aus einem 3- bis 7-gliedrigen Heteroalkanring enthaltend 1 Ringheteroatom von O und einem 3- bis 7-gliedrigen Alkanring, linearem oder verzweigtem -$C_{1-4}$-Alkylen-$R_7$, -$OR_7$ und -C(O)$R_7$, wobei: das Alkyl, Cycloalkyl, Heterocycloalkyl, Phenyl,

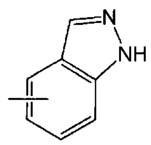

,

Pyridinyl,

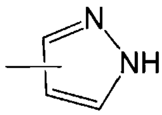

,

verbrückte Heterocycloalkyl und die kondensierte Ringstruktur optional substituiert sind mit einer oder mehreren Gruppen unabhängig ausgewählt aus OH, CN, $NH_2$, =O, F, Cl, Br, linearem oder verzweigtem $C_{1-4}$-Akyl, linearem oder verzweigtem $C_{1-4}$-Alkylen-OH, linearem oder verzweigtem $C_{1-4}$-Alkyloxy, linearem oder verzweigtem -C(O)O$C_{1-4}$-Alkyl, -C(O)$R_7$, substituiertem oder unsubstituiertem $C_{3-7}$-Cycloalkyl und substituiertem oder unsubstituiertem 3- bis 7-gliedrigem Heterocycloalkyl gebunden über C oder N und enthaltend 1-2 Ringheteroatome unabhängig ausgewählt aus N und O, wobei Substituentengruppen für das substituierte $C_{3-7}$-Cycloalkyl und substituierte 3- bis 7-gliedrige Heterocycloalkyl ausgewählt sind aus linearem oder verzweigtem $C_{1-4}$-Alkyl optional substituiert mit einer oder mehreren Gruppen unabhängig ausgewählt aus F, Cl und Br;
$R_7$ ausgewählt ist aus linearem oder verzweigtem $C_{1-4}$-Alkyl, $C_{3-6}$-Cycloalkyl und 3- bis 6-gliedrigem Heterocycloalkyl gebunden über C oder N und enthaltend 1-2 Ringheteroatome unabhängig ausgewählt aus N und O; wobei das Alkyl, Cycloalkyl und Heterocycloalkyl optional substituiert sind mit einer oder mehreren Gruppen unabhängig ausgewählt aus OH und linearem oder verzweigtem $C_{1-4}$-Alkyl;
$R_2$ und $R_3$ unabhängig ausgewählt sind aus F, Cl und Br;
$R_4$ und $R_5$ H sind;
wobei bevorzugter in Formel (II):

$R_1$ ausgewählt ist aus $C_{3-6}$-Cycloalkyl und 3- bis 6-gliedrigem Heterocycloalkyl gebunden über C oder N und enthaltend 1-2 Ringheteroatome unabhängig ausgewählt aus N und O, wobei: das Cycloalkyl und Heterocycloalkyl optional substituiert sind mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{1-4}$-Alkylen-OH, linearem oder verzweigtem $C_{1-4}$-Alkyloxy, linearem oder verzweigtem -C(O)O$C_{1-4}$-Alkyl, -C(O)$R_7$, substituiertem oder unsubstituiertem $C_{3-6}$-Cycloalkyl und substituiertem oder unsubstituiertem 3- bis 6-gliedrigem Heterocycloalkyl gebunden über C oder N und enthaltend 1-2 Ringheteroatome unabhängig ausgewählt aus N und O, wobei Substituentengruppen für das substituierte $C_{3-6}$-Cycloalkyl und substituierte 3- bis 6-gliedrige Heterocycloalkyl ausgewählt sind aus linearem oder verzweigtem $C_{1-4}$-Alkyl optional substituiert mit einer oder mehreren Gruppen unabhängig ausgewählt aus F, Cl und Br;
$R_7$ ausgewählt ist aus linearem oder verzweigtem $C_{1-4}$-Alkyl, $C_{3-6}$-Cycloalkyl und 3- bis 6-gliedrigem Heterocycloalkyl gebunden über C oder N und enthaltend 1-2 Ringheteroatome unabhängig ausgewählt aus N und O; wobei das Alkyl, Cycloalkyl und Heterocycloalkyl optional substituiert sind mit einer oder mehreren Gruppen unabhängig ausgewählt aus OH und linearem oder verzweigtem $C_{1-4}$-Alkyl;
$R_2$ und $R_3$ unabhängig ausgewählt sind aus F, Cl und Br;
$R_4$ und $R_5$ H sind.

8. Verbindung oder das pharmazeutisch verträgliche Salz oder die deuterierte Verbindung davon nach Anspruch 1, **dadurch gekennzeichnet, dass** die Verbindung eine Struktur dargestellt durch Formel (III) aufweist:

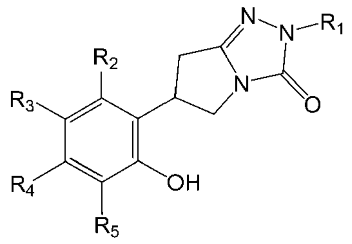

Formel (III),

wobei:

$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, verbrücktem $C_{5-7}$-Cycloalkyl und 5- bis 7-gliedrigem verbrücktem Heterocycloalkyl enthaltend 1-3 Ringheteroatome unabhängig ausgewählt aus N, O und S, wobei: das Alkyl, Cycloalkyl, Heterocycloalkyl, verbrückte Cycloalkyl und verbrückte Heterocycloalkyl optional substituiert sind mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{1-4}$-Alkylen-OH, linearem oder verzweigtem $C_{1-4}$-Alkyloxy, substituiertem oder unsubstituiertem $C_{3-7}$-Cycloalkyl und substituiertem oder unsubstituiertem 3- bis 7-gliedrigem Heterocycloalkyl gebunden über C oder N und enthaltend 1-3 Ringheteroatome unabhängig ausgewählt aus N, O und S, wobei Substituentengruppen für das substituierte $C_{3-7}$-Cycloalkyl und substituierte 3- bis 7-gliedrige Heterocycloalkyl ausgewählt sind aus linearem oder verzweigtem $C_{1-4}$-Alkyl optional substituiert mit einer oder mehreren Gruppen unabhängig ausgewählt aus F, Cl und Br;
$R_2$ und $R_3$ unabhängig ausgewählt sind aus F, Cl und Br;
$R_4$ und $R_5$ H sind;
wobei bevorzugt in Formel (III):

$R_1$ ausgewählt ist aus linearem oder verzweigtem $C_{1-4}$-Alkyl, $C_{3-6}$-Cycloalkyl und 3- bis 6-gliedrigem Heterocycloalkyl gebunden über C oder N und enthaltend 1-2 Ringheteroatome unabhängig ausgewählt aus N und O, wobei: das Alkyl, Cycloalkyl und Heterocycloalkyl optional substituiert sind mit einer oder mehreren Gruppen unabhängig ausgewählt aus F, Cl, Br, linearem oder verzweigtem $C_{1-4}$-Alkyl, linearem oder verzweigtem $C_{1-4}$-Alkylen-OH und linearem oder verzweigtem $C_{1-4}$-Alkyloxy;
$R_2$ und $R_3$ unabhängig ausgewählt sind aus F, Cl und Br;
$R_4$ und $R_5$ H sind.

**9.** Verbindung oder das pharmazeutisch verträgliche Salz oder die deuterierte Verbindung davon nach Anspruch 1, **dadurch gekennzeichnet, dass** die Verbindung eine Struktur dargestellt durch Formel (IV) aufweist:

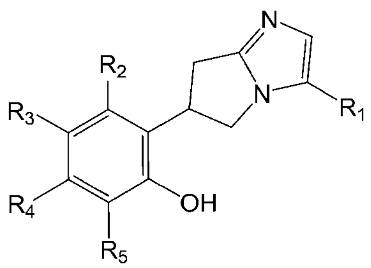

Formel (IV),

wobei:
$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl, 5- bis 11-gliedrigem Heteroaryl enthaltend 1-3 Ringheteroatome unabhängig ausgewählt aus N, O und S, verbrücktem $C_{5-7}$-Cycloalkyl, 5- bis 7-gliedrigem verbrücktem Heterocycloalkyl enthaltend 1-3 Ringheteroatome unabhängig ausgewählt aus N, O und S, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Alkanring, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Heteroalkanring enthaltend 1-3

Ringheteroatome unabhängig ausgewählt aus N, O und S, einer kondensierten Ringstruktur bestehend aus einem Benzolring und einem 3- bis 7-gliedrigen Heteroalkanring enthaltend 1-3 Ringheteroatome unabhängig ausgewählt aus N, O und S, einer kondensierten Ringstruktur bestehend aus einem 5-bis 7-gliedrigen heteroaromatischen Ring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, linearem oder verzweigtem -$C_{1-4}$-Alkylen-$R_7$, -$OR_7$, linearem oder verzweigtem -O-$C_{1-4}$-Alkylen-$R_7$, -C(O)$R_7$ und linearem oder verzweigtem -C(O)-$C_{1-4}$-Alkylen-$R_7$, wobei:

das Alkylen optional substituiert ist mit OH;
das Alkyl, Cycloalkyl, Heterocycloalkyl, Phenyl, Heteroaryl, verbrückte Cycloalkyl, verbrückte Heterocycloalkyl und die kondensierten Ringstrukturen optional substituiert sind mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{1-4}$-Alkylen-OH, linearem oder verzweigtem $C_{1-4}$-Alkyloxy, linearem oder verzweigtem -C(O)O$C_{1-4}$-Alkyl, -C(O)$R_7$, substituiertem oder unsubstituiertem $C_{3-7}$-Cycloalkyl und substituiertem oder unsubstituiertem 3-bis 7-gliedrigem Heterocycloalkyl gebunden über C oder N und enthaltend 1-3 Ringheteroatome unabhängig ausgewählt aus N, O und S, wobei Substituentengruppen für das substituierte $C_{3-7}$-Cycloalkyl und substituierte 3- bis 7-gliedrige Heterocycloalkyl ausgewählt sind aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{1-4}$-Alkylen-$NH_2$ und linearem oder verzweigtem $C_{1-4}$-Alkyloxy;
$R_7$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl und 5- bis 11-gliedrigem Heteroaryl enthaltend 1-3 Ringheteroatome unabhängig ausgewählt aus N, O und S; wobei das Alkyl, Cycloalkyl, Heterocycloalkyl, Phenyl und Heteroaryl optional substituiert sind mit einer oder mehreren Gruppen unabhängig ausgewählt aus OH, linearem oder verzweigtem $C_{1-4}$-Alkyl und linearem oder verzweigtem $C_{1-4}$-Alkylen-OH;
$R_2$ und $R_3$ unabhängig ausgewählt sind aus F, Cl und Br;
$R_4$ und $R_5$ H sind;
wobei bevorzugt in Formel (IV):

$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-2 Ringheteroatome unabhängig ausgewählt aus N und O, Phenyl,

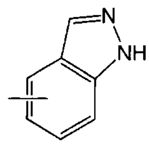

,

Pyridinyl,

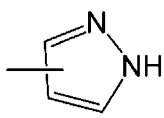

,

5- bis 7-gliedrigem verbrücktem Heterocycloalkyl enthaltend 1 Ringheteroatom von O, einer kondensierten Ringstruktur bestehend aus einem 3- bis 7-gliedrigen Heteroalkanring enthaltend 1 Ringheteroatom von O und einem 3- bis 7-gliedrigen Alkanring, linearem oder verzweigtem -$C_{1-4}$-Alkylen-$R_7$, -C(O)$R_7$ und -$OR_7$, wobei: das Alkyl, Cycloalkyl, Heterocycloalkyl, Phenyl,

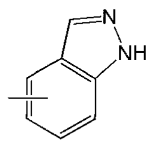

,

Pyridinyl,

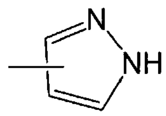

,

verbrückte Heterocycloalkyl und die kondensierte Ringstruktur optional substituiert sind mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{1-4}$-Alkylen-OH, linearem oder verzweigtem $C_{1-4}$-Alkyloxy, linearem oder verzweigtem -C(O)O$C_{1-4}$-Alkyl, -C(O)$R_7$, substituiertem oder unsubstituiertem $C_{3-7}$-Cycloalkyl und substituiertem oder unsubstituiertem 3- bis 7-gliedrigem Heterocycloalkyl gebunden über C oder N und enthaltend 1-2 Ringheteroatome unabhängig ausgewählt aus N und O, wobei Substituentengruppen für das substituierte $C_{3-7}$-Cycloalkyl und substituierte 3- bis 7-gliedrige Heterocycloalkyl ausgewählt sind aus linearem oder verzweigtem $C_{1-4}$-Alkyl optional substituiert mit einer oder mehreren Gruppen unabhängig ausgewählt aus F, Cl und Br;
$R_7$ ausgewählt ist aus linearem oder verzweigtem $C_{1-4}$-Alkyl, $C_{3-6}$-Cycloalkyl und 3- bis 6-gliedrigem Heterocycloalkyl gebunden über C oder N und enthaltend 1-2 Ringheteroatome unabhängig ausgewählt aus N und O; wobei das Alkyl, Cycloalkyl und Heterocycloalkyl optional substituiert sind mit einer oder mehreren Gruppen unabhängig ausgewählt aus OH und linearem oder verzweigtem $C_{1-4}$-Alkyl;
$R_2$ und $R_3$ unabhängig ausgewählt sind aus F, Cl und Br;
$R_4$ und $R_5$ H sind;
wobei bevorzugter in Formel (IV):

$R_1$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-2 Ringheteroatome unabhängig ausgewählt aus N und O, Phenyl,

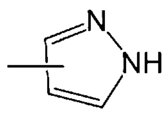

,

linearem oder verzweigtem -$C_{1-4}$-Alkylen-$R_7$, -C(O)$R_7$ und -O$R_7$, wobei: das Alkyl, Cycloalkyl, Heterocycloalkyl, Phenyl und

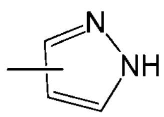

optional substituiert sind mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{1-4}$-Alkylen-OH, linearem oder verzweigtem $C_{1-4}$-Alkyloxy und linearem oder verzweigtem -C(O)O$C_{1-4}$-Alkyl;
$R_7$ ausgewählt ist aus linearem oder verzweigtem $C_{1-4}$-Alkyl, $C_{3-6}$-Cycloalkyl und 3- bis 6-gliedrigem Heterocycloalkyl gebunden über C oder N und enthaltend 1-2 Ringheteroatome unabhängig ausgewählt aus N und O; wobei das Alkyl, Cycloalkyl und Heterocycloalkyl optional substituiert sind mit einer oder mehreren Gruppen unabhängig ausgewählt aus OH und linearem oder verzweigtem $C_{1-4}$-Alkyl;;
$R_2$ und $R_3$ unabhängig ausgewählt sind aus F, Cl und Br;
$R_4$ und $R_5$ H sind.

10. Verbindung oder das pharmazeutisch verträgliche Salz oder die deuterierte Verbindung davon nach Anspruch 1, **dadurch gekennzeichnet, dass** die Verbindung eine Struktur dargestellt durch Formel (V) oder Formel (V') aufweist:

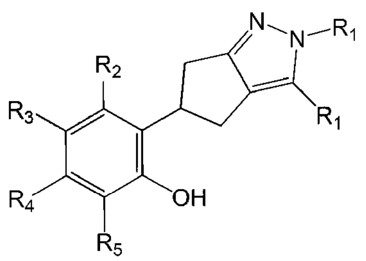

Formel V oder

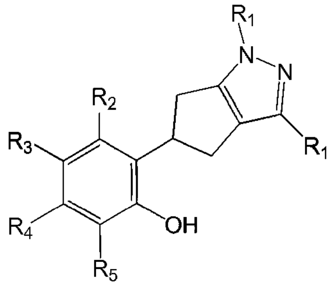

Formel V';

wobei in Formel (V) oder Formel (V') jedes $R_1$ identisch oder verschieden ist und unabhängig ausgewählt ist aus H, linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl, 5- bis 11-gliedrigem Heteroaryl enthaltend 1-3 Ringheteroatome unabhängig ausgewählt aus N, O und S, verbrücktem $C_{5-7}$-Cycloalkyl, 5- bis 7-gliedrigem verbrücktem Heterocycloalkyl enthaltend 1-3 Ringheteroatome unabhängig ausgewählt aus N, O und S, einer kondensierten Ringstruktur bestehend aus einem 3- bis 7-gliedrigen Heteroalkanring enthaltend 1-3 Ringheteroatome unabhängig ausgewählt aus N, O und S und einem 3- bis 7-gliedrigen Alkanring, einer kondensierten Ringstruktur bestehend aus einem 3- bis 7- gliedrigen Heteroalkanring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, einer kondensierten Ringstruktur bestehend aus einem Benzolring und einem 3- bis 7-gliedrigen Heteroalkanring enthaltend 1-3 Ringheteroatome unabhängig ausgewählt aus N, O und S, einer kondensierten Ringstruktur bestehend aus einem 5-bis 7-gliedrigen heteroaromatischen Ring enthaltend 1-3 Ringheteroatome unabhängig ausgewählt aus N, O und S und einem 3- bis 7-gliedrigen Heteroalkanring enthaltend 1-3 Ringheteroatome unabhängig ausgewählt aus N, O und S, linearem oder verzweigtem -$C_{1-4}$-Alkylen-$R_7$, -$OR_7$, linearem oder verzweigtem -O-$C_{1-4}$-Alkylen-$R_7$, -C(O)$R_7$ und linearem oder verzweigtem -C(O)-$C_{1-4}$-Alkylen-$R_7$, wobei:

das Alkylen optional substituiert ist mit OH;
das Alkyl, Cycloalkyl, Heterocycloalkyl, Phenyl, Heteroaryl, verbrückte Cycloalkyl, verbrückte Heterocycloalkyl und die kondensierten Ringstrukturen optional substituiert sind mit einer oder mehreren Gruppen unabhängig ausgewählt aus OH, CN, $NH_2$, =O, F, Cl, Br, linearem oder verzweigtem $C_{1-4}$-Alkyl, linearem oder verzweigtem $C_{1-4}$-Alkylen-OH, linearem oder verzweigtem $C_{1-4}$-Alkyloxy, linearem oder verzweigtem -C(O)O$C_{1-4}$-Alkyl, -C(O)$R_7$, substituiertem oder unsubstituiertem $C_{3-7}$-Cycloalkyl und substituiertem oder unsubstituiertem 3-bis 7-gliedrigem Heterocycloalkyl gebunden über C oder N und enthaltend 1-3 Ringheteroatome unabhängig ausgewählt aus N, O und S, wobei Substituentengruppen für das substituierte $C_{3-7}$-Cycloalkyl und substituierte 3- bis 7-gliedrige Heterocycloalkyl ausgewählt sind aus OH, CN, $NH_2$, =O, F, Cl, Br, linearem oder verzweigtem $C_{1-4}$-Alkyl optional substituiert mit einer oder mehreren Gruppen unabhängig ausgewählt aus F, Cl und Br, linearem oder verzweigtem $C_{1-4}$-Alkylen-OH, linearem oder verzweigtem $C_{1-4}$-Alkylen-$NH_2$ und linearem oder verzweigtem $C_{1-4}$-Alkyloxy;
$R_7$ ausgewählt ist aus linearem oder verzweigtem $C_{1-4}$-Alkyl, $C_{3-6}$-Cycloalkyl, 3- bis 6-gliedrigem Heterocycloalkyl gebunden über C oder N und enthaltend 1-3 Ringheteroatome unabhängig ausgewählt aus N, O und S, Phenyl und 5- bis 11-gliedrigem Heteroaryl enthaltend 1-3 Ringheteroatome unabhängig ausgewählt aus N, O und S; wobei das Alkyl, Cycloalkyl, Heterocycloalkyl, Phenyl und Heteroaryl optional substituiert sind mit einer oder mehreren Gruppen unabhängig ausgewählt aus OH, linearem oder verzweigtem $C_{1-4}$-Alkyl und linearem oder verzweigtem $C_{1-4}$-Alkylen-OH;
$R_2$ und $R_3$ unabhängig ausgewählt sind aus F, Cl und Br;
$R_4$ und $R_5$ H sind;
wobei bevorzugt in Formel (V) oder Formel (V'):

jedes $R_1$ identisch oder verschieden ist und unabhängig ausgewählt ist aus H, linearem oder verzweigtem $C_{1-4}$-Alkyl, $C_{3-6}$-Cycloalkyl, 3- bis 6-gliedrigem Heterocycloalkyl gebunden über C oder N und enthaltend 1-2 Ringheteroatome unabhängig ausgewählt aus N und O und Phenyl, wobei: das Alkyl, Cycloalkyl, Heterocycloalkyl und Phenyl optional substituiert sind mit einer oder mehreren Gruppen unabhängig ausgewählt aus OH, F, Cl, Br, linearem oder verzweigtem $C_{1-4}$-Alkyl, linearem oder verzweigtem $C_{1-4}$-Alkylen-OH und linearem oder verzweigtem $C_{1-4}$-Alkyloxy;
$R_2$ und $R_3$ unabhängig ausgewählt sind aus F, Cl und Br;
$R_4$ und $R_5$ H sind;

wobei bevorzugter in Formel (V) oder Formel (V'):

jedes $R_1$ identische oder verschieden ist und unabhängig ausgewählt ist aus H und linearem oder verzweigtem $C_{1-4}$-Alkyl, wobei: das Alkyl optional substituiert ist mit einer oder mehreren Gruppen unabhängig ausgewählt aus OH, F, Cl, Br, linearem oder verzweigtem $C_{1-4}$-Alkyl, linearem oder verzweigtem $C_{1-4}$-Alkylen-OH und linearem oder verzweigtem $C_{1-4}$-Alkyloxy;
$R_2$ und $R_3$ unabhängig ausgewählt sind aus F, Cl und Br;
$R_4$ und $R_5$ H sind.

**11.** Verbindung oder das pharmazeutisch verträgliche Salz oder die deuterierte Verbindung davon nach Anspruch 1, **dadurch gekennzeichnet, dass** die Verbindung oder das pharmazeutisch verträgliche Salz oder die deuterierte Verbindung davon ausgewählt ist aus den folgenden Verbindungen:

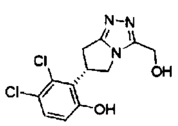 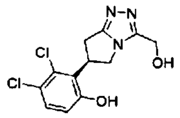 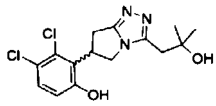 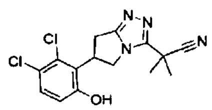 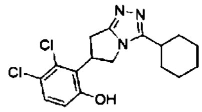

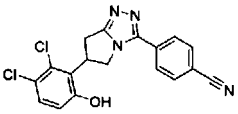 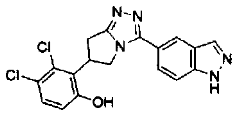 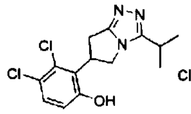 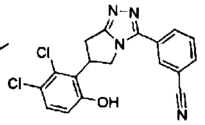 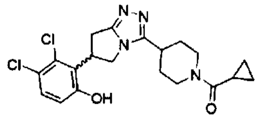

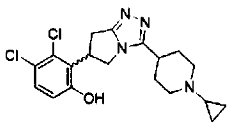 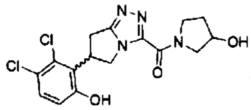 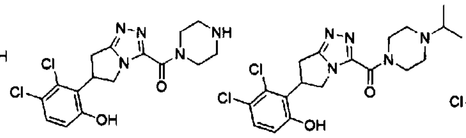 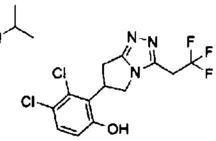

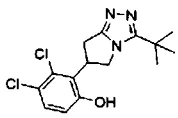 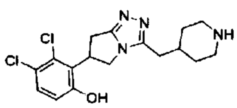 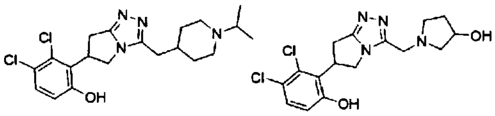 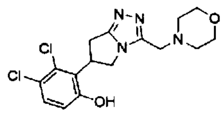

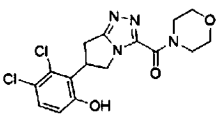 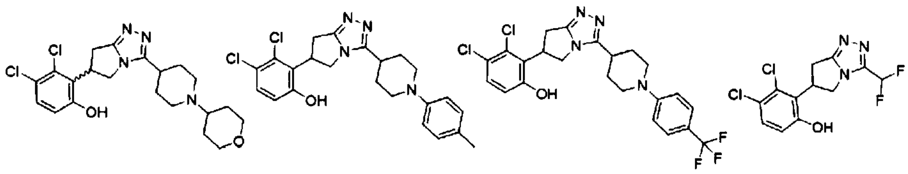

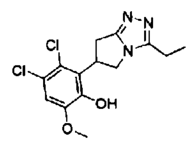 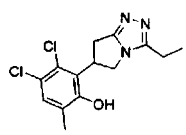 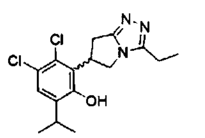 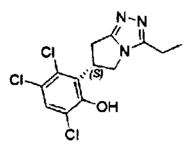 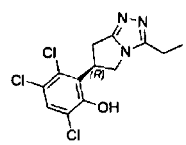

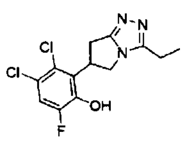 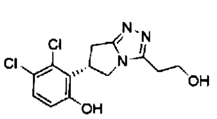 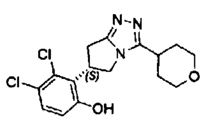 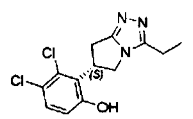 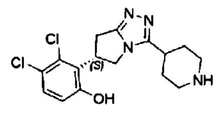

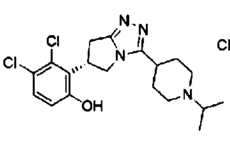 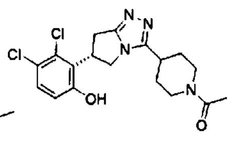 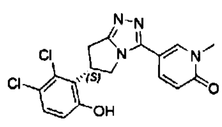 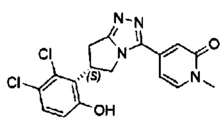 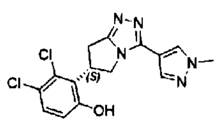

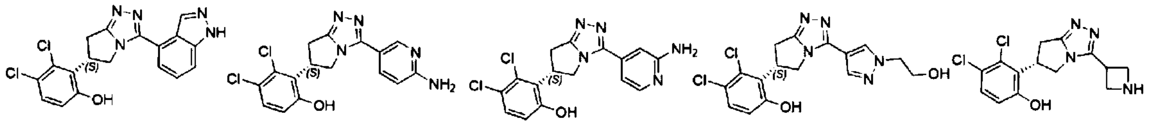

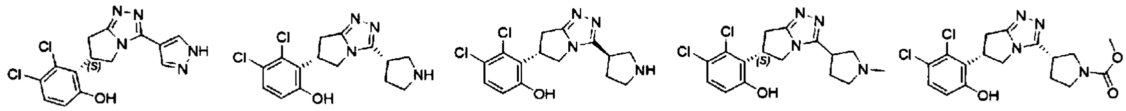

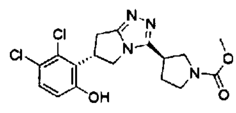 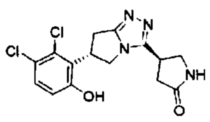 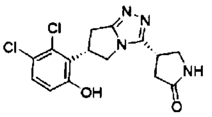 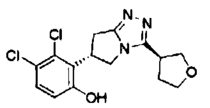 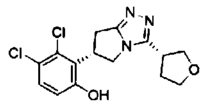

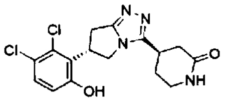 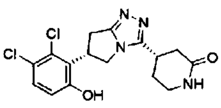 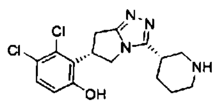 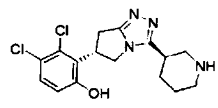 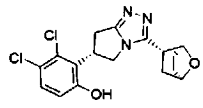

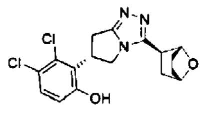 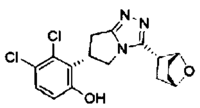 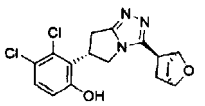 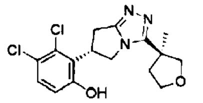 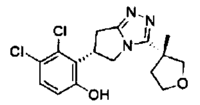

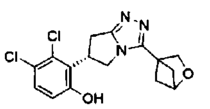 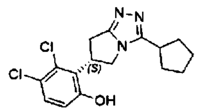 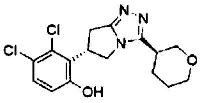 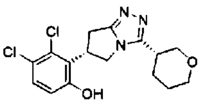 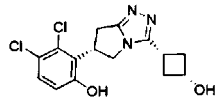

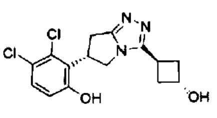 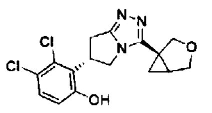 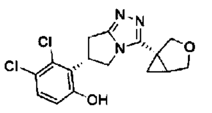 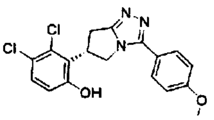 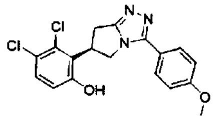

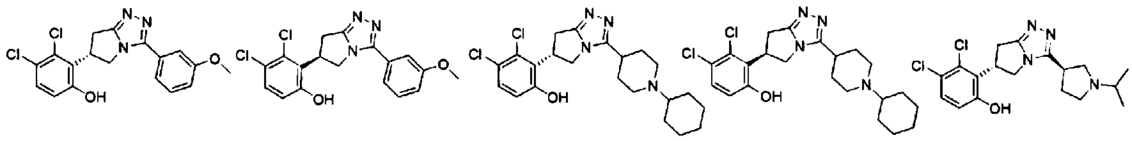

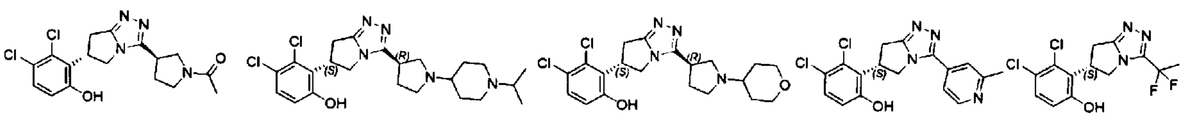

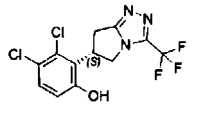 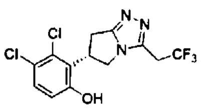 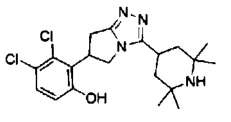 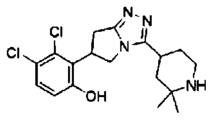 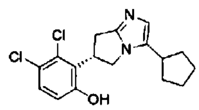

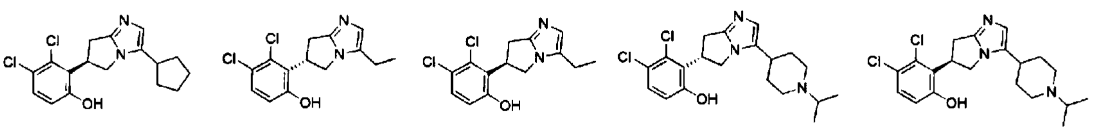

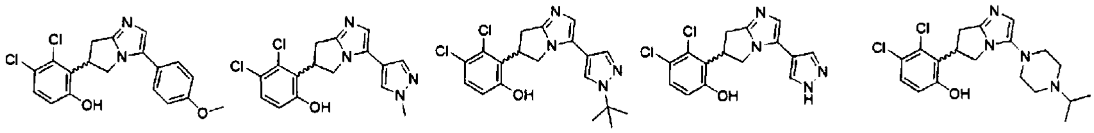

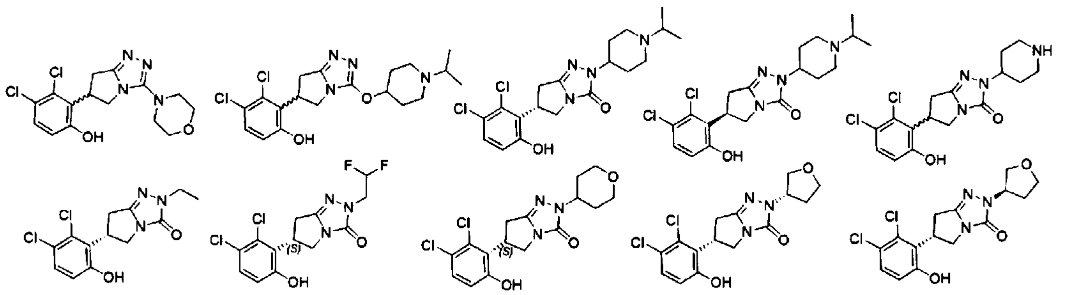

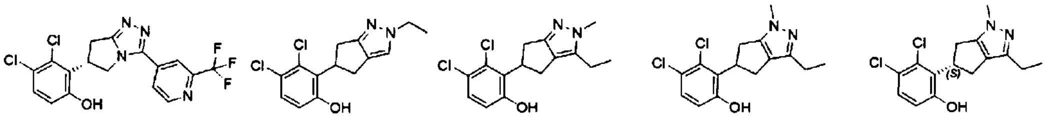

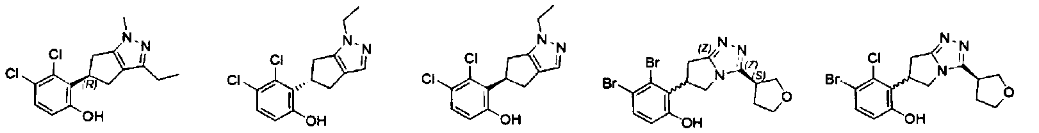

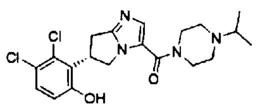

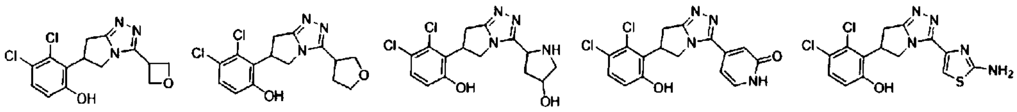

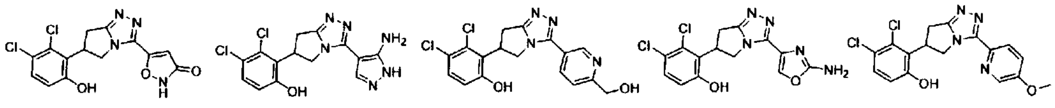

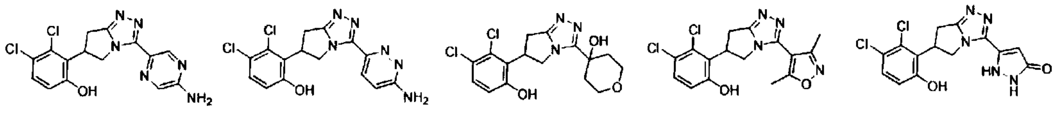

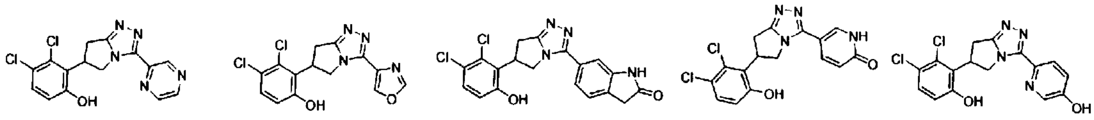

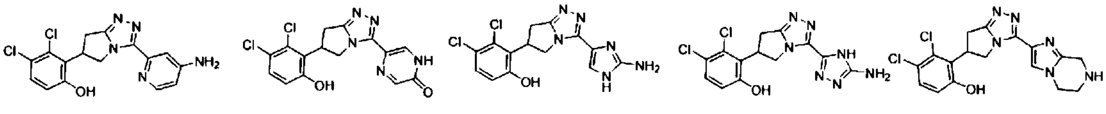

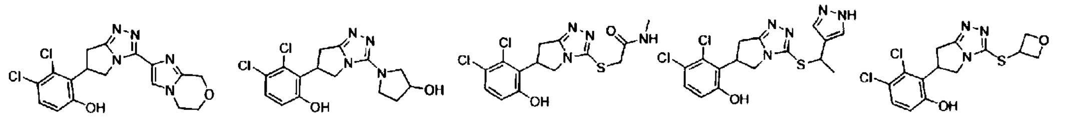

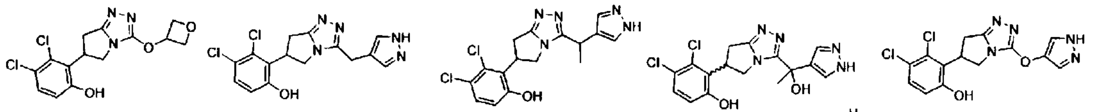

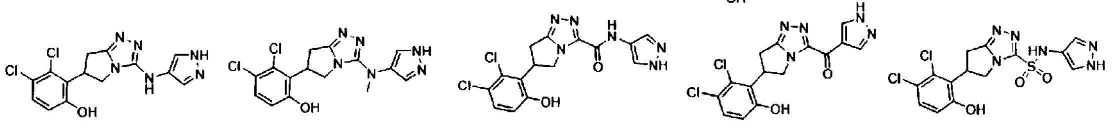

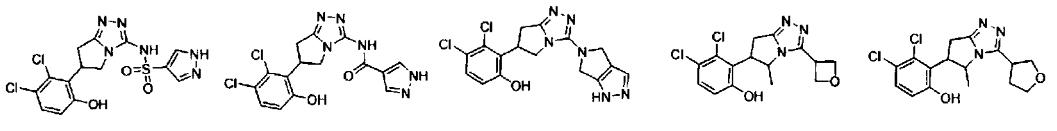

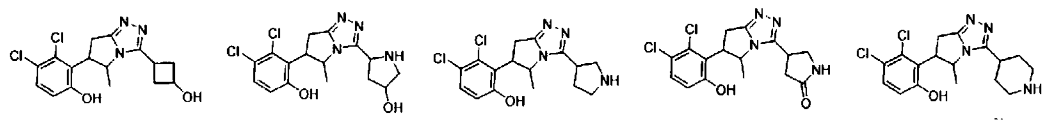

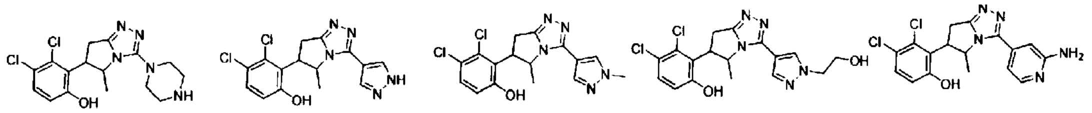

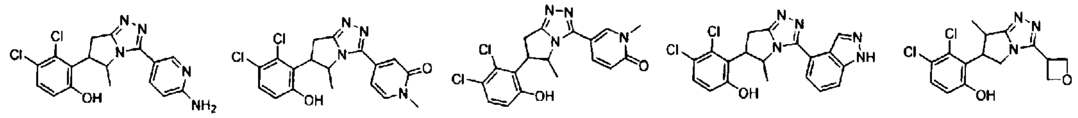

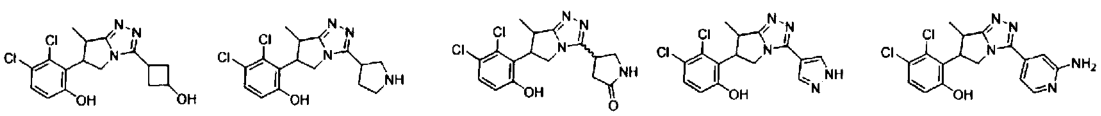

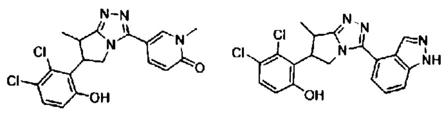

**12.** Pharmazeutische Zusammensetzung, umfassend die Verbindung oder das pharmazeutisch verträgliche Salz oder die deuterierte Verbindung davon nach einem der Ansprüche 1-11 und einen pharmazeutisch verträglichen Träger.

**13.** Verbindung oder das pharmazeutisch verträgliche Salz oder die deuterierte Verbindung davon nach einem der Ansprüche 1-11 oder die pharmazeutische Zusammensetzung nach Anspruch 12 zur Verwendung als ein Medikament.

**14.** Verbindung oder das pharmazeutisch verträgliche Salz oder die deuterierte Verbindung davon nach einem der Ansprüche 1-11 oder die pharmazeutische Zusammensetzung nach Anspruch 12 zur Verwendung beim Verhindern und/oder Behandeln einer Kv1.3-assoziierten Erkrankung, wobei bevorzugt die Erkrankung beliebige aus einer Gruppe von Immun- und Entzündungserkrankungen, wie Multiple Sklerose, entzündliche Darmerkrankung, Colitis ulcerosa, Crohn-Erkrankung, rheumatoide Arthritis, Typ-1-Diabetes, Psoriasis und Asthma, Spondylitis und Parodontitis, sowie Fettleibigkeit, Typ-2-Diabetes, Nierenfibrose, Alzheimer-Erkrankung, ischämischen Schlaganfall beinhaltet.

## Revendications

**1.** Composé représenté par la Formule (I) ou sel acceptable pharmaceutique ou composé deutéré de celui-ci :

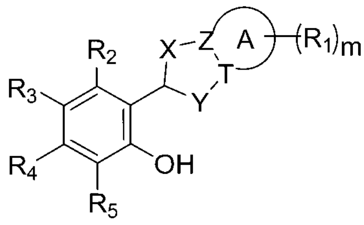

(I)

où

A est choisi parmi un hétéroaryle à 5 chaînons contenant 1 à 3 hétéroatomes de cycle indépendamment choisis parmi N, O et S ;
Z et T sont indépendamment choisis parmi C et N ;
X et Y sont indépendamment choisis parmi CRaRb, NRc et O ;
Ra et Rb sont indépendamment choisis parmi H, OH, $NH_2$, un alkyle en $C_{1-4}$ linéaire ou ramifié, un alcényle en $C_{2-4}$ linéaire ou ramifié, un alcynyle en $C_{2-4}$ linéaire ou ramifié, et un alkyloxy en $C_{1-4}$ linéaire ou ramifié ; ou Ra et Rb, conjointement avec l'atome de carbone auquel ils sont liés, forment un cycloalkyle en $C_{3-7}$ ou un hétérocycloalkyle à 3 à 7 chaînons contenant 1 à 3 hétéroatomes de cycle indépendamment choisis parmi N, O et S ;
Rc est choisi parmi H, un alkyle en $C_{1-4}$ linéaire ou ramifié, un alcényle en $C_{2-4}$ linéaire ou ramifié et un cycloalkyle en $C_{3-7}$ ;
$R_2$, $R_3$, $R_4$ et $R_5$ sont indépendamment choisis parmi H, F, Cl, Br, CN, un alkyle en $C_{1-4}$ linéaire ou ramifié, un alcényle en $C_{2-4}$ linéaire ou ramifié, un alcynyle en $C_{2-4}$ linéaire ou ramifié, un alkyloxy en $C_{1-4}$ linéaire ou ramifié, un alcényloxy en $C_{2-4}$ linéaire ou ramifié, un alcynyloxy en $C_{2-4}$ linéaire ou ramifié et un cycloalkyle en $C_{3-7}$ ;
chaque $R_1$ est indépendamment choisi parmi F, Cl, Br, CN, =O, un alkyle en $C_{1-4}$ linéaire ou ramifié, un alcényle en $C_{2-4}$ linéaire ou ramifié, un alcynyle en $C_{2-4}$ linéaire ou ramifié, un cycloalkyle en $C_{3-7}$, un hétérocycloalkyle à 3 à 7 chaînons lié par C ou N et contenant 1 à 3 hétéroatomes de cycle indépendamment choisis parmi N, O et S, un aryle, un hétéroaryle à 5 à 11 chaînons contenant 1 à 3 hétéroatomes de cycle indépendamment choisis parmi N, O et S, un cycloalkyle ponté en $C_{5-7}$, un hétérocycloalkyle ponté à 5 à 7 chaînons contenant 1 à 3 hétéroatomes de cycle indépendamment choisis parmi N, O et S, une structure à cycles fusionnés constituée d'un cycle hétéroalcanique à 3 à 7 chaînons contenant 1 à 3 hétéroatomes de cycle indépendamment choisis parmi N, O et S et d'un cycle alcanique à 3 à 7 chaînons, une structure à cycles fusionnés constituée d'un cycle hétéroalcanique à 3 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, une structure à cycles fusionnés constituée d'un cycle benzénique et d'un cycle hétéroalcanique à 3 à 7 chaînons contenant 1 à 3 hétéroatomes de cycle indépendamment choisis parmi N, O et S, une structure à cycles fusionnés constituée d'un cycle hétéroaromatique à 5 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, -alkylène en $C_{1-4}$ linéaire ou ramifié-$R_7$, -$OR_7$, -O-alkylène en $C_{1-4}$ linéaire ou ramifié-$R_7$, -$SR_7$, - S-alkylène en $C_{1-4}$ linéaire ou ramifié-$R_7$, -$N(R_6)R_7$, -$N(R_6)$-alkylène en $C_{1-4}$ linéaire ou ramifié-$R_7$, -$C(O)R_7$, -C(O)-alkylène en $C_{1-4}$ linéaire ou ramifié-$R_7$, -$C(O)R_7$-$OR_7$, -$C(O)R_7$-$N(R_6)R_7$, - $S(O)_2R_7$, -$N(R_6)C(O)R_7$, -$N(R_6)C(O)$-alkylène en $C_{1-4}$ linéaire ou ramifié-$R_7$, -$C(O)N(R_6)R_7$, - $C(O)N(R_6)$-alkylène en $C_{1-4}$ linéaire ou ramifié-$R_7$, -$N(R_6)S(O)_2R_7$, -$N(R_6)S(O)_2$-alkylene en $C_{1-4}$ linéaire ou ramifié-$R_7$, -$S(O)_2N(R_6)R_7$ et -$S(O)_2N(R_6)$-alkylène en $C_{1-4}$ linéaire ou ramifié-$R_7$, où :

l'alkylène est éventuellement substitué par OH ;
l'alkyle, l'alcényle, l'alcynyle, le cycloalkyle, l'hétérocycloalkyle, l'aryle, l'hétéroaryle, le cycloalkyle ponté, l'hétérocycloalkyle ponté et les structures à cycles fusionnés sont éventuellement substitués par un ou plusieurs groupes indépendamment choisis parmi OH, CN, $NH_2$, =O, F, Cl, Br, un alkyle en $C_{1-4}$ linéaire ou

ramifié éventuellement substitué par un ou plusieurs groupes indépendamment choisis parmi F, Cl et Br, un alcényle en $C_{2-4}$ linéaire ou ramifié éventuellement substitué par un ou plusieurs groupes indépendamment choisis parmi F, Cl et Br, un alcynyle en $C_{2-4}$ linéaire ou ramifié éventuellement substitué par un ou plusieurs groupes indépendamment choisis parmi F, Cl et Br, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, un alkylène en $C_{1-4}$ linéaire ou ramifié-$NH_2$, un alcénylène en $C_{2-4}$ linéaire ou ramifié-$NH_2$, un alcynylène en $C_{2-4}$ linéaire ou ramifié-$NH_2$, un alkyloxy en $C_{1-4}$ linéaire ou ramifié, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, -C(O)$R_7$, -S(O)$_2R_7$, -N($R_6$)$R_7$, -N($R_6$)-alkylène en $C_{1-4}$ linéaire ou ramifié-$R_7$, -N($R_6$)C(O)$R_7$, -N($R_6$)C(O)-alkylène en $C_{1-4}$ linéaire ou ramifié-$R_7$, -N($R_6$)S(O)$_2R_7$, -N($R_6$)S(O)$_2$-alkylène en $C_{1-4}$ linéaire ou ramifié-$R_7$, -S(O)$_2$N($R_6$)$R_7$, -S(O)$_2$N($R_6$)-alkylène en $C_{1-4}$ linéaire ou ramifié-$R_7$, -C(O)N($R_6$)$R_7$, -C(O)N($R_6$)-alkylène en $C_{1-4}$ linéaire ou ramifié-$R_7$, -P(O)($R_6$)$_2$, un cycloalkyle en $C_{3-7}$ substitué ou non substitué, un aryle substitué ou non substitué, un hétérocycloalkyle à 3 à 7 chaînons substitué ou non substitué lié par C ou N et contenant 1 à 3 hétéroatomes de cycle indépendamment choisis parmi N, O et S, et un hétéroaryle à 5 à 6 chaînons substitué ou non substitué lié par C ou N et contenant 1 à 3 hétéroatomes de cycle indépendamment choisis parmi N, O et S, où des groupes substituants pour le cycloalkyle en $C_{3-7}$ substitué, l'aryle substitué, l'hétérocycloalkyle à 3 à 7 chaînons substitué et l'hétéroaryle à 5 à 6 chaînons substitué sont choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alcényle en $C_{2-4}$ linéaire ou ramifié éventuellement substitué par un ou plusieurs groupes indépendamment choisis parmi F, Cl et Br, un alcynyle en $C_{2-4}$ linéaire ou ramifié éventuellement substitué par un ou plusieurs groupes indépendamment choisis parmi F, Cl et Br, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, un alkylène en $C_{1-4}$ linéaire ou ramifié-$NH_2$, un alcénylène en $C_{2-4}$ linéaire ou ramifié-$NH_2$, un alcynylène en $C_{2-4}$ linéaire ou ramifié-$NH_2$ et un alkyloxy en $C_{1-4}$ linéaire ou ramifié ; les deux substituants sur le même atome de carbone de cycle dans le cycloalkyle, l'hétérocycloalkyle et l'hétéroaryle, ainsi que l'atome de carbone auquel ils sont liés, peuvent éventuellement former un cycloalkyle en $C_{3-6}$ ou un hétérocycloalkyle à 3 à 6 chaînons contenant 1 hétéroatome de cycle choisi parmi N et O, le cycloalkyle ou hétérocycloalkyle formé étant éventuellement substitué par un ou plusieurs groupes indépendamment choisis parmi OH, CN, $NH_2$, =O, F, Cl, Br, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, un alkylène en $C_{1-4}$ linéaire ou ramifié-$NH_2$, un alcénylène en $C_{2-4}$ linéaire ou ramifié-$NH_2$, un alcynylène en $C_{2-4}$ linéaire ou ramifié-$NH_2$, un alkyloxy en $C_{1-4}$ linéaire ou ramifié, un cycloalkyle en $C_{3-6}$ et un hétérocycloalkyle à 3 à 6 chaînons contenant 1 à 3 hétéroatomes de cycle indépendamment choisis parmi N, O et S ;

$R_6$ est choisi parmi H, un alkyle en $C_{1-4}$ linéaire ou ramifié, un alcényle en $C_{2-4}$ linéaire ou ramifié et un alcynyle en $C_{2-4}$ linéaire ou ramifié ;

$R_7$ est indépendamment choisi parmi H, OH, $NH_2$, un alkyle en $C_{1-4}$ linéaire ou ramifié, un alcényle en $C_{2-4}$ linéaire ou ramifié, un alcynyle en $C_{2-4}$ linéaire ou ramifié, un alkyloxy en $C_{1-4}$ linéaire ou ramifié, un alkylène en $C_{1-4}$ linéaire ou ramifié-OH, un cycloalkyle en $C_{3-7}$, un hétérocycloalkyle à 3 à 7 chaînons lié par C ou N et contenant 1 à 3 hétéroatomes de cycle indépendamment choisis parmi N, O et S, un aryle et un hétéroaryle à 5 à 11 chaînons contenant 1 à 3 hétéroatomes de cycle indépendamment choisis parmi N, O et S, l'alkyle, l'alcényle, l'alcynyle, le cycloalkyle, l'hétérocycloalkyle, l'aryle et l'hétéroaryle étant éventuellement substitués par un ou plusieurs groupes indépendamment choisis parmi F, Cl, Br, OH, CN, $NH_2$, =O, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, C(O)NH-alkyle en $C_{1-4}$ linéaire ou ramifié, C(O)NH-alcényle en $C_{2-4}$ linéaire ou ramifié et C(O)NH-alcynyle en $C_{2-4}$ linéaire ou ramifié ;

m est choisi parmi 1 et 2.

**2.** Composé représenté par la Formule (I) ou sel acceptable pharmaceutique ou composé deutéré de celui-ci selon la revendication 1 :

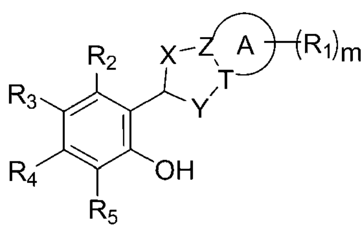

(I)

où

A est choisi parmi un hétéroaryle à 5 chaînons contenant 1 à 3 hétéroatomes de cycle indépendamment choisis parmi N, O et S ;
Z et T sont indépendamment choisis parmi C et N ;
X et Y sont indépendamment choisis parmi CRaRb, NRc et O ;
Ra et Rb sont indépendamment choisis parmi H, OH, $NH_2$, un alkyle en $C_{1-4}$ linéaire ou ramifié, un alcényle en $C_{2-4}$ linéaire ou ramifié, un alcynyle en $C_{2-4}$ linéaire ou ramifié, et un alkyloxy en $C_{1-4}$ linéaire ou ramifié, ou Ra et Rb, conjointement avec l'atome de carbone auquel ils sont liés, forment un cycloalkyle en $C_{3-7}$ ou un hétérocycloalkyle à 3 à 7 chaînons contenant 1 à 3 hétéroatomes de cycle indépendamment choisis parmi N, O et S ;
Rc est choisi parmi H, un alkyle en $C_{1-4}$ linéaire ou ramifié, un alcényle en $C_{2-4}$ linéaire ou ramifié et un cycloalkyle en $C_{3-7}$ ;
$R_2$, $R_3$, $R_4$ et $R_5$ sont indépendamment choisis parmi H, F, Cl, Br, CN, un alkyle en $C_{1-4}$ linéaire ou ramifié, un alcényle en $C_{2-4}$ linéaire ou ramifié, un alcynyle en $C_{2-4}$ linéaire ou ramifié et un cycloalkyle en $C_{3-7}$ ;
chaque $R_1$ est indépendamment choisi parmi F, Cl, Br, CN, un alkyle en $C_{1-4}$ linéaire ou ramifié, un alcényle en $C_{2-4}$ linéaire ou ramifié, un alcynyle en $C_{2-4}$ linéaire ou ramifié, un cycloalkyle en $C_{3-7}$, un hétérocycloalkyle à 3 à 7 chaînons lié par C ou N et contenant 1 à 3 hétéroatomes de cycle indépendamment choisis parmi N, O et S, un aryle, un hétéroaryle à 5 à 11 chaînons contenant 1 à 3 hétéroatomes de cycle indépendamment choisis parmi N, O et S, un cycloalkyle ponté en $C_{5-7}$, un hétérocycloalkyle ponté à 5 à 7 chaînons contenant 1 à 3 hétéroatomes de cycle indépendamment choisis parmi N, O et S, une structure à cycles fusionnés constituée d'un cycle hétéroalcanique à 3 à 7 chaînons contenant 1 à 3 hétéroatomes de cycle indépendamment choisis parmi N, O et S et d'un cycle alcanique à 3 à 7 chaînons, une structure à cycles fusionnés constituée d'un cycle hétéroalcanique à 3 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, une structure à cycles fusionnés constituée d'un cycle benzénique et d'un cycle hétéroalcanique à 3 à 7 chaînons contenant 1 à 3 hétéroatomes de cycle indépendamment choisis parmi N, O et S, une structure à cycles fusionnés constituée d'un cycle hétéroaromatique à 5 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, -alkylène en $C_{1-4}$ linéaire ou ramifié-$R_7$, -$OR_7$, -O-alkylène en $C_{1-4}$ linéaire ou ramifié-$R_7$, -$SR_7$, - S-alkylène en $C_{1-4}$ linéaire ou ramifié-$R_7$, -$N(R_6)R_7$, -$N(R_6)$-alkylène en $C_{1-4}$ linéaire ou ramifié-$R_7$, -$C(O)R_7$, -C(O)-alkylène en $C_{1-4}$ linéaire ou ramifié-$R_7$, -$C(O)R_7$-$OR_7$, -$C(O)R_7$-$N(R_6)R_7$, - $S(O)_2R_7$, -$N(R_6)C(O)R_7$, -$N(R_6)C(O)$-alkylène en $C_{1-4}$ linéaire ou ramifié-$R_7$, -$C(O)N(R_6)R_7$, - $C(O)N(R_6)$-alkylène en $C_{1-4}$ linéaire ou ramifié-$R_7$, -$N(R_6)S(O)_2R_7$, -$N(R_6)S(O)_2$-alkylène en $C_{1-4}$ linéaire ou ramifié-$R_7$, -$S(O)_2N(R_6)R_7$ et -$S(O)_2N(R_6)$-alkylène en $C_{1-4}$ linéaire ou ramifié-$R_7$, où : l'alkylène est éventuellement substitué par OH ; l'alkyle, l'alcényle, l'alcynyle, le cycloalkyle, l'hétérocycloalkyle, l'aryle, l'hétéroaryle, le cycloalkyle ponté, l'hétérocycloalkyle ponté et les structures à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alcényle en $C_{2-4}$ linéaire ou ramifié éventuellement substitué par un ou plusieurs groupes indépendamment choisis parmi F, Cl et Br, un alcynyle en $C_{2-4}$ linéaire ou ramifié éventuellement substitué par un ou plusieurs groupes indépendamment choisis parmi F, Cl et Br, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, un alkylène en $C_{1-4}$ linéaire ou ramifié-$NH_2$, un alcénylène en $C_{2-4}$ linéaire ou ramifié-$NH_2$, un alcynylène en $C_{2-4}$ linéaire ou ramifié-$NH_2$, un alkyloxy en $C_{1-4}$ linéaire ou ramifié, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, -$C(O)R_7$, -$S(O)_2R_7$, -$N(R_6)R_7$, -$N(R_6)$-alkylène en $C_{1-4}$ linéaire ou ramifié-$R_7$, -$N(R_6)C(O)R_7$, -$N(R_6)C(O)$-alkylène en $C_{1-4}$ linéaire ou ramifié-$R_7$, - $N(R_6)S(O)_2R_7$, -$N(R_6)S(O)_2$-alkylène en $C_{1-4}$ linéaire ou ramifié-$R_7$, -$S(O)_2N(R_6)R_7$, -$S(O)_2N(R_6)$-alkylène en $C_{1-4}$ linéaire ou ramifié-$R_7$, -$C(O)N(R_6)R_7$, -$C(O)N(R_6)$-alkylène en $C_{1-4}$ linéaire ou ramifié-$R_7$, -$P(O)(R_6)_2$, un cycloalkyle en $C_{3-7}$

et un hétérocycloalkyle à 3 à 7 chaînons lié par C ou N et contenant 1 à 3 hétéroatomes de cycle indépendamment choisis parmi N, O et S ; les deux substituants sur le même atome de carbone de cycle dans le cycloalkyle, l'hétérocycloalkyle et l'hétéroaryle, conjointement avec l'atome de carbone auquel ils sont liés, peuvent éventuellement former un cycloalkyle en $C_{3-6}$ ou un hétérocycloalkyle à 3 à 6 chaînons contenant 1 hétéroatome de cycle choisi parmi N et O, le cycloalkyle ou l'hétérocycloalkyle formé étant éventuellement substitué par un ou plusieurs groupes indépendamment choisis parmi OH, CN, $NH_2$, =O, F, Cl, Br, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, un alkylène en $C_{1-4}$ linéaire ou ramifié-$NH_2$, un alcénylène en $C_{2-4}$ linéaire ou ramifié-$NH_2$, un alcynylène en $C_{2-4}$ linéaire ou ramifié-$NH_2$, un alkyloxy en $C_{1-4}$ linéaire ou ramifié, un cycloalkyle en $C_{3-6}$ et un hétérocycloalkyle à 3 à 6 chaînons contenant 1 à 3 hétéroatomes de cycle indépendamment choisis parmi N, O et S ;

$R_6$ est choisi parmi H, un alkyle en $C_{1-4}$ linéaire ou ramifié, un alcényle en $C_{2-4}$ linéaire ou ramifié et un alcynyle en $C_{2-4}$ linéaire ou ramifié ;

$R_7$ est indépendamment choisi parmi H, OH, $NH_2$, un alkyle en $C_{1-4}$ linéaire ou ramifié, un alcényle en $C_{2-4}$ linéaire ou ramifié, un alcynyle en $C_{2-4}$ linéaire ou ramifié, un alkyloxy en $C_{1-4}$ linéaire ou ramifié, un alkylène en $C_{1-4}$ linéaire ou ramifié-OH, un cycloalkyle en $C_{3-7}$, un hétérocycloalkyle à 3 à 7 chaînons lié par C ou N et contenant 1 à 3 hétéroatomes de cycle indépendamment choisis parmi N, O et S, un aryle, et un hétéroaryle à 5 à 6 chaînons contenant 1 à 3 hétéroatomes de cycle indépendamment choisis parmi N, O et S, l'alkyle, l'alcényle, l'alcynyle, le cycloalkyle, l'hétérocycloalkyle, l'aryle et l'hétéroaryle étant éventuellement substitués par un ou plusieurs groupes indépendamment choisis parmi F, Cl, Br, OH, CN, $NH_2$, =O, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, C(O)NH-alkyle en $C_{1-4}$ linéaire ou ramifié, C(O)NH-alcényle en $C_{2-4}$ linéaire ou ramifié et C(O)NH-alcynyle en $C_{2-4}$ linéaire ou ramifié;

m est choisi parmi 1 et 2.

3. Composé ou sel acceptable pharmaceutique ou composé deutéré de celui-ci selon l'une des revendications 1 ou 2, **caractérisé en ce que**

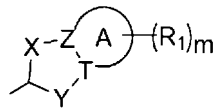

est choisi parmi

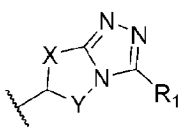
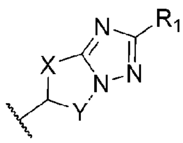
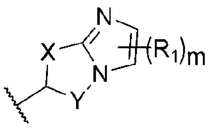
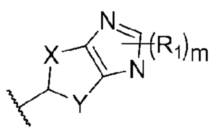
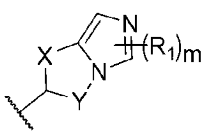

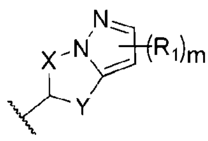
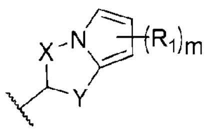
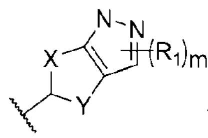
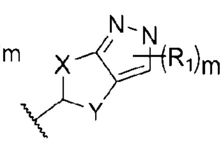

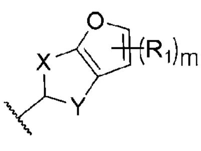
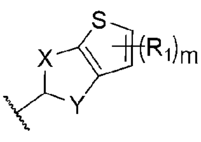
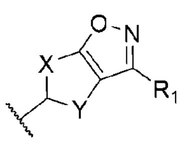
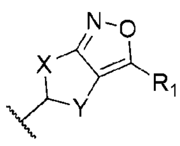
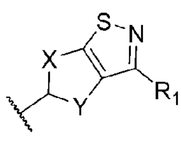
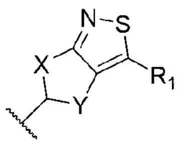
.

4. Composé ou sel acceptable pharmaceutique ou composé deutéré de celui-ci selon la revendication 3, **caractérisé en ce que** X et Y sont CRaRb.

**5.** Composé ou sel acceptable pharmaceutique ou composé deutéré de celui-ci selon la revendication 1, **caractérisé en ce que** le composé présente une structure représentée par la Formule (II) :

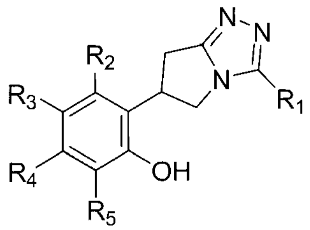

(II)

où :

$R_1$ est choisi parmi un alkyle en $C_{1-4}$ linéaire ou ramifié, un alcényle en $C_{2-4}$ linéaire ou ramifié, un alcynyle en $C_{2-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle, un hétéroaryle à 5 à 11 chaînons contenant 1 à 3 hétéroatomes de cycle indépendamment choisis parmi N, O et S, un cycloalkyle ponté en $C_{5-7}$, un hétérocycloalkyle ponté à 5 à 7 chaînons contenant 1 à 3 hétéroatomes de cycle indépendamment choisis parmi N, O et S, une structure à cycles fusionnés constituée d'un cycle hétéroalcanique à 3 à 7 chaînons contenant 1 à 3 hétéroatomes de cycle indépendamment choisis parmi N, O et S et d'un cycle alcanique à 3 à 7 chaînons, une structure à cycles fusionnés constituée d'un cycle hétéroalcanique à 3 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, une structure à cycles fusionnés constituée d'un cycle benzénique et d'un cycle hétéroalcanique à 3 à 7 chaînons contenant 1 à 3 hétéroatomes de cycle indépendamment choisis parmi N, O et S, une structure à cycles fusionnés constituée d'un cycle hétéroaromatique *à 5* à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, -alkylène en $C_{1-4}$ linéaire ou ramifié-$R_7$, -$OR_7$, -O-alkylène en $C_{1-4}$ linéaire ou ramifié-$R_7$, -$SR_7$, -S-alkylène en $C_{1-4}$ linéaire ou ramifié-$R_7$, -$N(R_6)R_7$, -$N(R_6)$-alkylène en $C_{1-4}$ linéaire ou ramifié-$R_7$, -$C(O)R_7$, -C(O)-alkylène en $C_{1-4}$ linéaire ou ramifié-$R_7$, -$N(R_6)C(O)R_7$, -$N(R_6)C(O)$-alkylène en $C_{1-4}$ linéaire ou ramifié-$R_7$, -$C(O)N(R_6)R_7$, - $C(O)N(R_6)$-alkylène en $C_{1-4}$ linéaire ou ramifié-$R_7$, -$N(R_6)S(O)_2R_7$, -$N(R_6)S(O)_2$-alkylène en $C_{1-4}$ linéaire ou ramifié-$R_7$, -$S(O)_2N(R_6)R_7$ et -$S(O)_2N(R_6)$-alkylène en $C_{1-4}$ linéaire ou ramifié-$R_7$, où :

l'alkylène est éventuellement substitué par OH ;

l'alkyle, l'alcényle, l'alcynyle, le cycloalkyle, l'hétérocycloalkyle, le phényle, l'hétéroaryle, le cycloalkyle ponté, l'hétérocycloalkyle ponté et les structures à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alcényle en $C_{2-4}$ linéaire ou ramifié éventuellement substitué par un ou plusieurs groupes indépendamment choisis parmi F, Cl, et Br, un alcynyle en $C_{2-4}$ linéaire ou ramifié éventuellement substitué par un ou plusieurs groupes indépendamment choisis parmi F, Cl, et Br, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, un alkyloxy en $C_{1-4}$ linéaire ou ramifié, -C(O) O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, C(O)NH-alkyle en $C_{1-4}$ linéaire ou ramifié, C(O)NH-alcényle en $C_{2-4}$ linéaire ou ramifié, C(O)NH-alcynyle en $C_{2-4}$ linéaire ou ramifié, -$C(O)R_7$, un cycloalkyle en $C_{3-7}$ substitué ou non substitué, un aryle substitué ou non substitué, un hétérocycloalkyle à 3 à 7 chaînons substitué ou non substitué lié par C ou N et contenant 1 à 3 hétéroatomes de cycle indépendamment choisis parmi N, O et S, et un hétéroaryle à 5 à 6 chaînons substitué ou non substitué lié par C ou N et contenant 1 à 3 hétéroatomes de cycle indépendamment choisis parmi N, O et S, où des groupes substituants pour le cycloalkyle en $C_{3-7}$ substitué, l'aryle substitué, l'hétérocycloalkyle à 3 à 7 chaînons substitué et l'hétéroaryle à 5 à 6 chaînons substitué sont choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alkylène en $C_{1-4}$ linéaire ou ramifié-OH, un alkylène en $C_{1-4}$ linéaire ou ramifié-$NH_2$, un alcénylène en $C_{2-4}$ linéaire ou ramifié-$NH_2$, un alcynylène en $C_{2-4}$ linéaire ou ramifié-$NH_2$ et un alkyloxy en $C_{1-4}$ linéaire ou ramifié ;

$R_6$ est choisi parmi H, un alkyle en $C_{1-4}$ linéaire ou ramifié, un alcényle en $C_{2-4}$ linéaire ou ramifié et un alcynyle en $C_{2-4}$ linéaire ou ramifié ;

$R_7$ est choisi parmi un alkyle en $C_{1-4}$ linéaire ou ramifié, un alcényle en $C_{2-4}$ linéaire ou ramifié, un alcynyle en $C_{2-4}$ linéaire ou ramifié, un cycloalkyle en $C_{3-6}$, un hétérocycloalkyle à 3 à 6 chaînons lié par C ou N et contenant 1 à 3

hétéroatomes de cycle indépendamment choisis parmi N, O et S, un phényle et un hétéroaryle à 5 à 11 chaînons contenant 1 à 3 hétéroatomes de cycle indépendamment choisis parmi N, O et S ; l'alkyle, l'alcényle, l'alcynyle, le cycloalkyle, l'hétérocycloalkyle, le phényle et l'hétéroaryle sont éventuellement substitués par un ou plusieurs groupes indépendamment choisis parmi OH, CN, $NH_2$, =O, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, C(O) NH-alkyle en $C_{1-4}$ linéaire ou ramifié, C(O)NH-alcényle en $C_{2-4}$ linéaire ou ramifié et C(O)NH-alcynyle en $C_{2-4}$ linéaire ou ramifié ;

$R_2$ et $R_3$ sont indépendamment choisis parmi F, Cl et Br ;

$R_4$ et $R_5$ sont H.

6. Composé ou sel acceptable pharmaceutique ou composé deutéré de celui-ci selon la revendication 5, **caractérisé en ce que**, dans la Formule (II) :

$R_1$ est choisi parmi un alkyle en $C_{1-4}$ linéaire ou ramifié, un alcényle en $C_{2-4}$ linéaire ou ramifié, un alcynyle en $C_{2-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle, un hétéroaryle à 5 à 11 chaînons contenant 1 à 3 hétéroatomes de cycle indépendamment choisis parmi N, O et S, -alkylène en $C_{1-4}$ linéaire ou ramifié-$R_7$, -$OR_7$, -O-alkylène en $C_{1-4}$ linéaire ou ramifié-$R_7$, -$SR_7$, -S-alkylène en $C_{1-4}$ linéaire ou ramifié-$R_7$, -$N(R_6)R_7$, -$N(R_6)$-alkylène en $C_{1-4}$ linéaire ou ramifié-$R_7$, -C(O)$R_7$, -C(O)-alkylène en $C_{1-4}$ linéaire ou ramifié-$R_7$, -$N(R_6)C(O)R_7$, -$N(R_6)$C(O)-alkylène en $C_{1-4}$ linéaire ou ramifié-$R_7$, -C(O)$N(R_6)R_7$, - C(O)$N(R_6)$-alkylène en $C_{1-4}$ linéaire ou ramifié-$R_7$, -$N(R_6)S(O)_2R_7$, -$N(R_6)S(O)_2$-alkylène en $C_{1-4}$ linéaire ou ramifié-$R_7$, -$S(O)_2N(R_6)R_7$ et -$S(O)_2N(R_6)$-alkylène en $C_{1-4}$ linéaire ou ramifié-$R_7$ ; l'alkylène est éventuellement substitué par OH ; l'alkyle, l'alcényle, l'alcynyle, le cycloalkyle, l'hétérocycloalkyle, le phényle et l'hétéroaryle sont éventuellement substitués par un ou plusieurs groupes indépendamment choisis parmi OH, CN, $NH_2$, =O, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, C(O)NH-alkyle en $C_{1-4}$ linéaire ou ramifié, C(O)NH-alcényle en $C_{2-4}$ linéaire ou ramifié, et C(O) NH-alcynyle en $C_{2-4}$ linéaire ou ramifié ;

$R_6$ est choisi parmi H, un alkyle en $C_{1-4}$ linéaire ou ramifié, un alcényle en $C_{2-4}$ linéaire ou ramifié et un alcynyle en $C_{2-4}$ linéaire ou ramifié ;

$R_7$ est choisi parmi un alkyle en $C_{1-4}$ linéaire ou ramifié, un alcényle en $C_{2-4}$ linéaire ou ramifié, un alcynyle en $C_{2-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle et un hétéroaryle à 5 à 11 chaînons contenant 1 à 3 hétéroatomes de cycle indépendamment choisis parmi N, O et S ; l'alkyle, l'alcényle, l'alcynyle, le cycloalkyle, l'hétérocycloalkyle, le phényle et l'hétéroaryle sont éventuellement substitués par un ou plusieurs groupes indépendamment choisis parmi OH, CN, $NH_2$, =O, un alkyle en $C_{1-4}$ linéaire ou ramifié, un alcényle en $C_{2-4}$ linéaire ou ramifié, un alcynyle en $C_{2-4}$ linéaire ou ramifié, un alkylène en $C_{1-4}$ linéaire ou ramifié-OH, un alcénylène en $C_{2-4}$ linéaire ou ramifié-OH, un alcynylène en $C_{2-4}$ linéaire ou ramifié-OH, -C(O)O-alkyle en $C_{1-4}$ linéaire ou ramifié, C(O)O-alcényle en $C_{2-4}$ linéaire ou ramifié, C(O)O-alcynyle en $C_{2-4}$ linéaire ou ramifié, C(O) NH-alkyle en $C_{1-4}$ linéaire ou ramifié, C(O)NH-alcényle en $C_{2-4}$ linéaire ou ramifié et C(O)NH-alcynyle en $C_{2-4}$ linéaire ou ramifié ;

$R_2$ et $R_3$ sont indépendamment choisis parmi F, Cl et Br ;

$R_4$ et $R_5$ sont H ;

de préférence, dans la Formule (II) :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S,

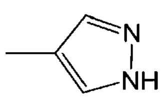

,

un pyridinyle,

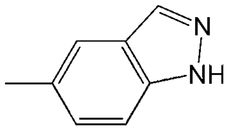

, -

alkylène en $C_{1-4}$ linéaire ou ramifié-$R_7$, -$OR_7$, -$SR_7$, -S-alkylène en $C_{1-4}$ linéaire ou ramifié-$R_7$, - $N(R_6)R_7$, -$C(O)R_7$, -$N(R_6)C(O)R_7$, -$C(O)N(R_6)R_7$, -$N(R_6)S(O)_2R_7$ et -$S(O)_2N(R_6)R_7$ ; l'alkylène est éventuellement substitué par OH ; l'alkyle, le cycloalkyle, l'hétérocycloalkyle,

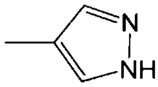

,

le pyridinyle, et

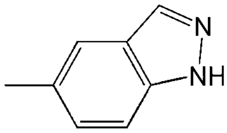

sont éventuellement substitués par un ou plusieurs groupes indépendamment choisis parmi OH, CN, $NH_2$, =O, un alkyle en $C_{1-4}$ linéaire ou ramifié, un alkylène en $C_{1-4}$ linéaire ou ramifié-OH, -C(O)O-alkyle en $C_{1-4}$ linéaire ou ramifié et C(O)NH-alkyle en $C_{1-4}$ linéaire ou ramifié ;

$R_6$ est choisi parmi H et un alkyle en $C_{1-4}$ linéaire ou ramifié ;

$R_7$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S,

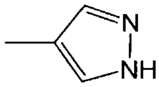

,

un pyridinyle, et

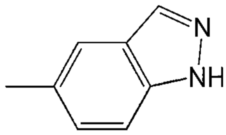

;

l'alkyle, le cycloalkyle, l'hétérocycloalkyle,

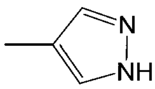

,

le pyridinyle, et

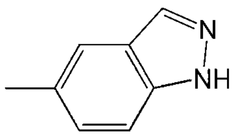

sont éventuellement substitués par un ou plusieurs groupes indépendamment choisis parmi OH, CN, $NH_2$, =O, un alkyle en $C_{1-4}$ linéaire ou ramifié, un alkylène en $C_{1-4}$ linéaire ou ramifié-OH, -C(O)O-alkyle en $C_{1-4}$ linéaire ou ramifié et C(O)NH-alkyle en $C_{1-4}$ linéaire ou ramifié ;

$R_2$ et $R_3$ sont indépendamment choisis parmi F, Cl et Br ;
$R_4$ et $R_5$ sont H ;
en outre de préférence, dans la Formule (II) :

$R_1$ est un alkyle en $C_{1-4}$ linéaire ou ramifié substitué par un ou plusieurs OH ;
$R_2$ et $R_3$ sont indépendamment choisis parmi F, Cl et Br ;
$R_4$ et $R_5$ sont H ;
idéalement, dans la Formule (II) :

$R_1$ est un alkyle en $C_{1-4}$ linéaire ou ramifié substitué par un OH ;
$R_2$ et $R_3$ sont Cl ;
$R_4$ et $R_5$ sont H.

**7.** Composé ou sel acceptable pharmaceutique ou composé deutéré de celui-ci selon la revendication 1, **caractérisé en ce que** le composé présente une structure représentée par la Formule (II) :

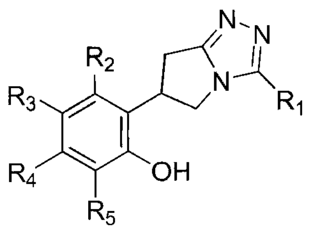

(II),

où :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle, un hétéroaryle à 5 à 11 chaînons contenant 1 à 3 hétéroatomes de cycle indépendamment choisis parmi N, O et S, un cycloalkyle ponté en $C_{5-7}$, un hétérocycloalkyle ponté à 5 à 7 chaînons contenant 1 à 3 hétéroatomes de cycle indépendamment choisis parmi N, O et S, une structure à cycles fusionnés constituée d'un cycle hétéroalcanique à 3 à 7 chaînons contenant 1 à 3 hétéroatomes de cycle indépendamment choisis parmi N, O et S et d'un cycle alcanique à 3 à 7 chaînons, une structure à cycles fusionnés constituée d'un cycle hétéroalcanique à 3 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, une structure à cycles fusionnés constituée d'un cycle benzénique et d'un cycle hétéroalcanique à 3 à 7 chaînons un cycle contenant 1 à 3 hétéroatomes de cycle indépendamment choisis parmi N, O et S, une structure à cycles fusionnés constituée d'un cycle hétéroaromatique à 5 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, - alkylène en $C_{1-4}$ linéaire ou ramifié-$R_7$, -$OR_7$, -O-alkylène en $C_{1-4}$ linéaire ou ramifié-$R_7$, -C(O)$R_7$ et -C(O)-alkylène en $C_{1-4}$ linéaire ou ramifié-$R_7$, où :

l'alkylène est éventuellement substitué par OH ;
l'alkyle, le cycloalkyle, l'hétérocycloalkyle, le phényle, l'hétéroaryle, le cycloalkyle ponté, l'hétérocycloalkyle ponté et les structures à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alkylène en $C_{1-4}$ linéaire ou ramifié-OH, un alkyloxy en $C_{1-4}$ linéaire ou ramifié, -C(O)O-alkyle en $C_{1-4}$ linéaire ou ramifié, -C(O)$R_7$, un cycloalkyle en $C_{3-7}$ substitué ou non substitué et un hétérocycloalkyle à 3 à 7 chaînons substitué ou non substitué lié par C ou N et contenant 1 à 3 hétéroatomes de cycle indépendamment choisis parmi N, O et S, où des groupes substituants pour le cycloalkyle en $C_{3-7}$ substitué et l'hétérocycloalkyle à 3 à 7 chaînons substitué sont choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alkylène en $C_{1-4}$ linéaire ou ramifié-OH, un alkylène en $C_{1-4}$ linéaire ou ramifié-$NH_2$ et un alkyloxy en $C_{1-4}$ linéaire ou ramifié ;
$R_7$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle et un hétéroaryle à 5 à 11 chaînons contenant 1 à 3 hétéroatomes de cycle indépendamment choisis parmi N, O et S ; l'alkyle, le cycloalkyle, l'hétérocycloalkyle, le phényle et l'hétéroaryle sont éventuellement

substitués par un ou plusieurs groupes indépendamment choisis parmi OH, un alkyle en $C_{1-4}$ linéaire ou ramifié et un alkylène en $C_{1-4}$ linéaire ou ramifié-OH ;
$R_2$ et $R_3$ sont indépendamment choisis parmi F, Cl et Br ;
$R_4$ et $R_5$ sont H ;
de préférence, dans la Formule (II) :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 2 hétéroatomes de cycle indépendamment choisis parmi N et O, un phényle,

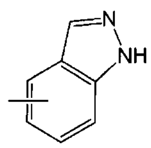

,

un pyridinyle,

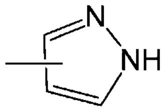

,

un hétérocycloalkyle ponté à 5 à 7 chaînons contenant 1 hétéroatome de cycle O, une structure à cycles fusionnés constituée d'un cycle hétéroalcanique à 3 à 7 chaînons contenant 1 hétéroatome de cycle O et d'un cycle alcanique à 3 à 7 chaînons, -alkylène en $C_{1-4}$ linéaire ou ramifié-$R_7$, -$OR_7$ et -C(O)$R_7$, où : l'alkyle, le cycloalkyle, l'hétérocycloalkyle, le phényle,

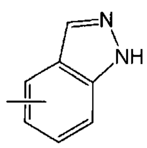

,

le pyridinyle,

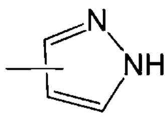

,

l'hétérocycloalkyle ponté, et la structure à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié, -C(O)O-alkyle en $C_{1-4}$ linéaire ou ramifié, -C(O)$R_7$, un cycloalkyle en $C_{3-7}$ substitué ou non substitué et un hétérocycloalkyle à 3 à 7 chaînons substitué ou non substitué lié par C ou N et contenant 1 à 2 hétéroatomes de cycle indépendamment choisis parmi N et O, où des groupes substituants pour le cycloalkyle en $C_{3-7}$ substitué et l'hétérocycloalkyle à 3 à 7 chaînons substitué sont choisis parmi un alkyle en $C_{1-4}$ linéaire ou ramifié éventuellement substitué par un ou plusieurs groupes indépendamment choisis parmi F, Cl et Br ;
$R_7$ est choisi parmi un alkyle en $C_{1-4}$ linéaire ou ramifié, un cycloalkyle en $C_{3-6}$ et un hétérocycloalkyle à 3 à 6 chaînons lié par C ou N et contenant 1 à 2 hétéroatomes de cycle indépendamment choisis parmi N et O ;
l'alkyle, le cycloalkyle et l'hétérocycloalkyle sont éventuellement substitués par un ou plusieurs groupes indépendamment choisis parmi OH et un alkyle en $C_{1-4}$ linéaire ou ramifié ;
$R_2$ et $R_3$ sont indépendamment choisis parmi F, Cl et Br ;
$R_4$ et $R_5$ sont H ;
idéalement, dans la Formule (II) :

$R_1$ est choisi parmi un cycloalkyle en $C_{3-6}$ et un hétérocycloalkyle à 3 à 6 chaînons lié par C ou N et contenant 1 à 2 hétéroatomes de cycle indépendamment choisis parmi N et O, où : le cycloalkyle et l'hétérocycloalkyle sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié, -C(O)O-alkyle en $C_{1-4}$ linéaire ou ramifié, -C(O)$R_7$, un cycloalkyle en $C_{3-6}$ substitué ou non substitué et un hétérocycloalkyle à 3 à 6 chaînons substitué ou non substitué lié par C ou N et contenant 1 à 2 hétéroatomes de cycle indépendamment choisis parmi N et O, où des groupes substituants pour le cycloalkyle en $C_{3-6}$ substitué et un hétérocycloalkyle à 3 à 6 chaînons substitué sont choisis parmi un alkyle en $C_{1-4}$ linéaire ou ramifié éventuellement substitué par un ou plusieurs groupes indépendamment choisis parmi F, Cl et Br ;
$R_7$ est choisi parmi un alkyle en $C_{1-4}$ linéaire ou ramifié, un cycloalkyle en $C_{3-6}$ et un hétérocycloalkyle à 3 à 6 chaînons lié par C ou N et contenant 1 à 2 hétéroatomes de cycle indépendamment choisis parmi N et O ; l'alkyle, le cycloalkyle et l'hétérocycloalkyle sont éventuellement substitués par un ou plusieurs groupes indépendamment choisis parmi OH et un alkyle en $C_{1-4}$ linéaire ou ramifié ;
$R_2$ et $R_3$ sont indépendamment choisis parmi F, Cl et Br ;
$R_4$ et $R_5$ sont H.

8. Composé ou sel acceptable pharmaceutique ou composé deutéré de celui-ci selon la revendication 1, **caractérisé en ce que** le composé présente une structure représentée par la Formule (III) :

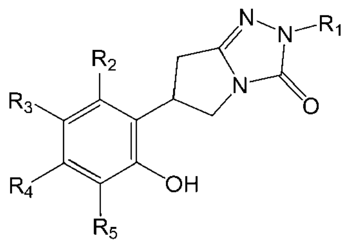

Formule (III)

, où :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un cycloalkyle ponté en $C_{5-7}$ et un hétérocycloalkyle ponté à 5 à 7 chaînons contenant 1 à 3 hétéroatomes de cycle indépendamment choisis parmi N, O et S, où : l'alkyle, le cycloalkyle, l'hétérocycloalkyle, le cycloalkyle ponté et l'hétérocycloalkyle ponté sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié, un cycloalkyle en $C_{3-7}$ substitué ou non substitué et un hétérocycloalkyle à 3 à 7 chaînons substitué ou non substitué lié par C ou N et contenant 1 à 3 hétéroatomes de cycle indépendamment choisis parmi N, O et S, où des groupes substituants pour le cycloalkyle en $C_{3-7}$ substitué et l'hétérocycloalkyle à 3 à 7 chaînons substitué sont choisis parmi un alkyle en $C_{1-4}$ linéaire ou ramifié éventuellement substitué par un ou plusieurs groupes indépendamment choisis parmi F, Cl et Br ;
$R_2$ et $R_3$ sont indépendamment choisis parmi F, Cl et Br ;
$R_4$ et $R_5$ sont H ;
de préférence, dans la Formule (III) :

$R_1$ est choisi parmi un alkyle en $C_{1-4}$ linéaire ou ramifié, un cycloalkyle en $C_{3-6}$ et un hétérocycloalkyle à 3 à 6 chaînons lié par C ou N et contenant 1 à 2 hétéroatomes de cycle indépendamment choisis parmi N et O, où : l'alkyle, le cycloalkyle et l'hétérocycloalkyle sont éventuellement substitués par un ou plusieurs groupes indépendamment choisis parmi F, Cl, Br, un alkyle en $C_{1-4}$ linéaire ou ramifié, un alkylène en $C_{1-4}$ linéaire ou ramifié-OH et un alkyloxy en $C_{1-4}$ linéaire ou ramifié ;
$R_2$ et $R_3$ sont indépendamment choisis parmi F, Cl et Br ;
$R_4$ et $R_5$ sont H.

9. Composé ou sel acceptable pharmaceutique ou composé deutéré de celui-ci selon la revendication 1, **caractérisé en ce que** le composé présente une structure représentée par la Formule (IV) :

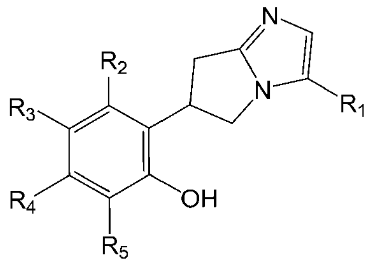

Formule (IV),

où :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle, un hétéroaryle à 5 à 11 chaînons contenant 1 à 3 hétéroatomes de cycle indépendamment choisis parmi N, O et S, un cycloalkyle ponté en $C_{5-7}$, un hétérocycloalkyle ponté à 5 à 7 chaînons contenant 1 à 3 hétéroatomes de cycle indépendamment choisis parmi N, O et S, une structure à cycles fusionnés constituée d'un cycle hétéroalcanique à 3 à 7 chaînons contenant 1 à 3 hétéroatomes de cycle indépendamment choisis parmi N, O et S et d'un cycle alcanique à 3 à 7 chaînons, une structure à cycles fusionnés constituée d'un cycle hétéroalcanique à 3 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, une structure à cycles fusionnés constituée d'un cycle benzénique et d'un cycle hétéroalcanique à 3 à 7 chaînons contenant 1 à 3 hétéroatomes de cycle indépendamment choisis parmi N, O et S, une structure à cycles fusionnés constituée d'un cycle hétéroaromatique à 5 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, -alkylène en $C_{1-4}$ linéaire ou ramifié-$R_7$, -$OR_7$, -O-alkylène en $C_{1-4}$ linéaire ou ramifié-$R_7$, -C(O)$R_7$ et -C(O)-alkylène en $C_{1-4}$ linéaire ou ramifié-$R_7$, où :

l'alkylène est éventuellement substitué par OH ;

l'alkyle, le cycloalkyle, l'hétérocycloalkyle, le phényle, l'hétéroaryle, le cycloalkyle ponté, l'hétérocycloalkyle ponté et les structures à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié, -C(O)O-alkyle en $C_{1-4}$ linéaire ou ramifié, -C(O)$R_7$, un cycloalkyle en $C_{3-7}$ substitué ou non substitué et un hétérocycloalkyle à 3 à 7 chaînons substitué ou non substitué lié par C ou N et contenant 1 à 3 hétéroatomes de cycle indépendamment choisis parmi N, O et S, où des groupes substituants pour le cycloalkyle en $C_{3-7}$ substitué et l'hétérocycloalkyle à 3 à 7 chaînons substitué sont choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alkylène en $C_{1-4}$ linéaire ou ramifié-OH, un alkylène en $C_{1-4}$ linéaire ou ramifié-$NH_2$ et un alkyloxy en $C_{1-4}$ linéaire ou ramifié ;

$R_7$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle et un hétéroaryle à 5 à 11 chaînons contenant 1 à 3 hétéroatomes de cycle indépendamment choisis parmi N, O et S ; l'alkyle, le cycloalkyle, l'hétérocycloalkyle, le phényle et l'hétéroaryle sont éventuellement substitués par un ou plusieurs groupes indépendamment choisis parmi OH, un alkyle en $C_{1-4}$ linéaire ou ramifié et un alkylène en $C_{1-4}$ linéaire ou ramifié-OH ;

$R_2$ et $R_3$ sont indépendamment choisis parmi F, Cl et Br ;

$R_4$ et $R_5$ sont H ;

de préférence, dans la Formule (IV) :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 2 hétéroatomes de cycle indépendamment choisis parmi N et O, un phényle,

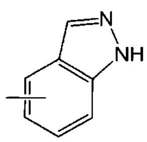

,

un pyridinyle,

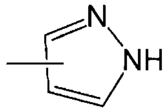

,

un hétérocycloalkyle ponté à 5 à 7 chaînons contenant 1 hétéroatome de cycle O, une structure à cycles fusionnés constituée d'un cycle hétéroalcanique à 3 à 7 chaînons contenant 1 hétéroatome de cycle O et d'un cycle alcanique à 3 à 7 chaînons, -alkylène en $C_{1-4}$ linéaire ou ramifié-$R_7$, - C(O)$R_7$ et -O$R_7$, où : l'alkyle, le cycloalkyle, l'hétérocycloalkyle, le phényle,

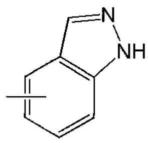

,

le pyridinyle,

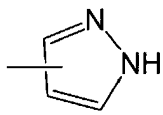

,

l'hétérocycloalkyle ponté, et la structure à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié, -C(O)O-alkyle en $C_{1-4}$ linéaire ou ramifié, -C(O)$R_7$, un cycloalkyle en $C_{3-7}$ substitué ou non substitué et un hétérocycloalkyle à 3 à 7 chaînons substitué ou non substitué lié par C ou N et contenant 1 à 2 hétéroatomes de cycle indépendamment choisis parmi N et O, où des groupes substituants pour le cycloalkyle en $C_{3-7}$ substitué et l'hétérocycloalkyle à 3 à 7 chaînons substitué sont choisis parmi un alkyle en $C_{1-4}$ linéaire ou ramifié éventuellement substitué par un ou plusieurs groupes indépendamment choisis parmi F, Cl et Br ;
$R_7$ est choisi parmi un alkyle en $C_{1-4}$ linéaire ou ramifié, un cycloalkyle en $C_{3-6}$ et un hétérocycloalkyle à 3 à 6 chaînons lié par C ou N et contenant 1 à 2 hétéroatomes de cycle indépendamment choisis parmi N et O ; l'alkyle, le cycloalkyle et l'hétérocycloalkyle sont éventuellement substitués par un ou plusieurs groupes indépendamment choisis parmi OH et un alkyle en $C_{1-4}$ linéaire ou ramifié ;
$R_2$ et $R_3$ sont indépendamment choisis parmi F, Cl et Br ;
$R_4$ et $R_5$ sont H ;
idéalement, dans la Formule (IV) :

$R_1$ est choisi parmi un alkyle en $C_{1-4}$ linéaire ou ramifié, un cycloalkyle en $C_{3-6}$, un hétérocycloalkyle à 3 à 6 chaînons lié par C ou N et contenant 1 à 2 hétéroatomes de cycle indépendamment choisis parmi N et O, un phényle,

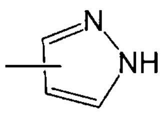

,

-alkylène en $C_{1-4}$ linéaire ou ramifié-$R_7$, -C(O)$R_7$ et -O$R_7$, où : l'alkyle, le cycloalkyle, l'hétérocycloalkyle, le phényle, et

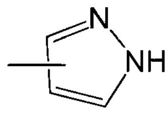

sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié et -C(O)O-alkyle en $C_{1-4}$ linéaire ou ramifié ;
$R_7$ est choisi parmi un alkyle en $C_{1-4}$ linéaire ou ramifié, un cycloalkyle en $C_{3-6}$ et un hétérocycloalkyle à 3 à 6 chaînons lié par C ou N et contenant 1 à 2 hétéroatomes de cycle indépendamment choisis parmi N et O ; l'alkyle, le cycloalkyle et l'hétérocycloalkyle sont éventuellement substitués par un ou plusieurs groupes indépendamment choisis parmi OH et un alkyle en $C_{1-4}$ linéaire ou ramifié ;
$R_2$ et $R_3$ sont indépendamment choisis parmi F, Cl et Br ;
$R_4$ et $R_5$ sont H.

**10.** Composé ou sel acceptable pharmaceutique ou composé deutéré de celui-ci selon la revendication 1, **caractérisé en ce que** le composé présente une structure représentée par la Formule (V) ou la Formule (V') :

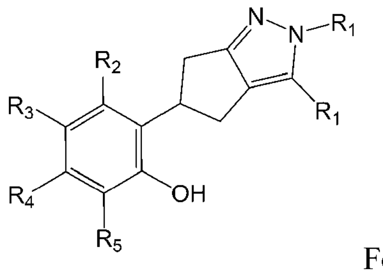

Formule V ou

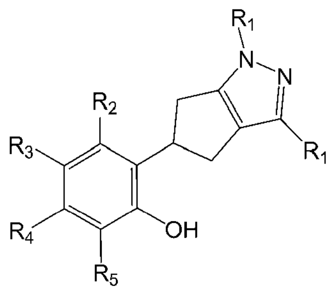

Formule V' ;

dans la Formule (V) ou la Formule (V'), chaque $R_1$ est identique ou différent et est indépendamment choisi parmi H, un alkyle en $C_{1-4}$ linéaire ou ramifié, un cycloalkyle en $C_{3-6}$, un hétérocycloalkyle à 3 à 6 chaînons lié par C ou N et contenant 1 à 3 hétéroatomes de cycle indépendamment choisis parmi N, O et S, un phényle, un hétéroaryle à 5 à 11 chaînons contenant 1 à 3 hétéroatomes de cycle indépendamment choisis parmi N, O, et S, un cycloalkyle ponté en $C_{5-7}$, un hétérocycloalkyle ponté à 5 à 7 chaînons contenant 1 à 3 hétéroatomes de cycle indépendamment choisis parmi N, O et S, une structure à cycles fusionnés constituée d'un cycle hétéroalcanique à 3 à 7 chaînons contenant 1 à 3 hétéroatomes de cycle indépendamment choisis parmi N, O et S et d'un cycle alcanique à 3 à 7 chaînons, une structure à cycles fusionnés constituée d'un cycle hétéroalcanique à 3 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, une structure à cycles fusionnés constituée d'un cycle benzénique et d'un cycle hétéroalcanique à 3 à 7 chaînons contenant 1 à 3 hétéroatomes de cycle indépendamment choisis parmi N, O et S, une structure à cycles fusionnés constituée d'un cycle hétéroaromatique à 5 à 7 chaînons contenant 1 à 3 hétéroatomes de cycle indépendamment choisis parmi N, O et S et d'un cycle hétéroalcanique à 3 à 7 chaînons contenant 1 à 3 hétéroatomes de cycle indépendamment choisis parmi N, O et S, - alkylène en $C_{1-4}$ linéaire ou ramifié-$R_7$, -$OR_7$-, -O-alkylène en $C_{1-4}$ linéaire ou ramifié-$R_7$, -C(O)$R_7$ et -C(O)-alkylène en $C_{1-4}$ linéaire ou ramifié-$R_7$, où :

l'alkylène est éventuellement substitué par OH ;
l'alkyle, le cycloalkyle, l'hétérocycloalkyle, le phényle, l'hétéroaryle, le cycloalkyle ponté, l'hétérocycloalkyle ponté et les structures à cycles fusionnés sont éventuellement substitués par un ou plusieurs groupes indépendamment choisis parmi OH, CN, $NH_2$, =O, F, Cl, Br, un alkyle en $C_{1-4}$ linéaire ou ramifié, un alkylène en $C_{1-4}$ linéaire ou ramifié-OH, un alkyloxy en $C_{1-4}$ linéaire ou ramifié, -C(O)O-alkyl en $C_{1-4}$ linéaire ou ramifié, -C(O)$R_7$, un cycloalkyle en $C_{3-7}$ substitué ou non substitué et un hétérocycloalkyle à 3 à 7 chaînons substitué ou non substitué lié par C ou N et contenant 1 à 3 hétéroatomes de cycle indépendamment choisis parmi N, O et S, où des groupes substituants pour le cycloalkyle en $C_{3-7}$ substitué et l'hétérocycloalkyle à 3 à 7 chaînons substitué sont choisis parmi OH, CN, $NH_2$, =O, F, Cl, Br, un alkyle en $C_{1-4}$ linéaire ou ramifié éventuellement substitué par un ou plusieurs groupes indépendamment choisis parmi F, Cl et Br, un alkylène en $C_{1-4}$ linéaire ou ramifié-OH, un alkylène en $C_{1-4}$ linéaire ou ramifié-$NH_2$ et un alkyloxy en $C_{1-4}$ linéaire ou ramifié ;
$R_7$ est choisi parmi un alkyle en $C_{1-4}$ linéaire ou ramifié, un cycloalkyle en $C_{3-6}$, un hétérocycloalkyle à 3 à 6

chaînons lié par C ou N et contenant 1 à 3 hétéroatomes de cycle indépendamment choisis parmi N, O et S, un phényle et un hétéroaryle à 5 à 11 chaînons contenant 1 à 3 hétéroatomes de cycle indépendamment choisis parmi N, O et S ; l'alkyle, le cycloalkyle, l'hétérocycloalkyle, le phényle et l'hétéroaryle sont éventuellement substitués par un ou plusieurs groupes indépendamment choisis parmi OH, un alkyle en $C_{1-4}$ linéaire ou ramifié et un alkylène en $C_{1-4}$ linéaire ou ramifié-OH ;
$R_2$ et $R_3$ sont indépendamment choisis parmi F, Cl et Br ;
$R_4$ et $R_5$ sont H ;
de préférence, dans la Formule (V) ou la Formule (V') :

chaque $R_1$ est identique ou différent et est indépendamment choisi parmi H, un alkyle en $C_{1-4}$ linéaire ou ramifié, un cycloalkyle en $C_{3-6}$, un hétérocycloalkyle à 3 à 6 chaînons lié par C ou N et contenant 1 à 2 hétéroatomes de cycle indépendamment choisis parmi N et O et un phényle, où : l'alkyle, le cycloalkyle, l'hétérocycloalkyle et le phényle sont éventuellement substitués par un ou plusieurs groupes indépendamment choisis parmi OH, F, Cl, Br, un alkyle en $C_{1-4}$ linéaire ou ramifié, un alkylène en $C_{1-4}$ linéaire ou ramifié-OH et un alkyloxy en $C_{1-4}$ linéaire ou ramifié ;
$R_2$ et $R_3$ sont indépendamment choisis parmi F, Cl et Br ;
$R_4$ et $R_5$ sont H ;
idéalement, dans la Formule (V) ou la Formule (V') :

chaque $R_1$ est identique ou différent et est indépendamment choisi parmi H et un alkyle en $C_{1-4}$ linéaire ou ramifié, où : l'alkyle est éventuellement substitué par un ou plusieurs groupes indépendamment choisis parmi OH, F, Cl, Br, un alkyle en $C_{1-4}$ linéaire ou ramifié, un alkylène en $C_{1-4}$ linéaire ou ramifié-OH et un alkyloxy en $C_{1-4}$ linéaire ou ramifié ;
$R_2$ et $R_3$ sont indépendamment choisis parmi F, Cl et Br ;
$R_4$ et $R_5$ sont H.

**11.** Composé ou sel acceptable pharmaceutique ou composé deutéré de celui-ci selon la revendication 1, **caractérisé en ce que** le composé ou le sel acceptable pharmaceutique ou le composé deutéré de celui-ci est choisi parmi les composés suivants :

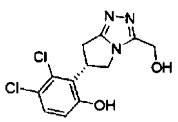 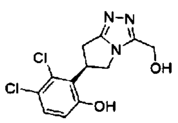 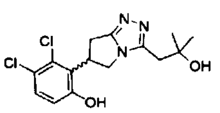 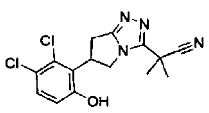 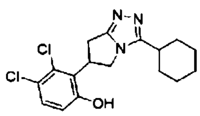

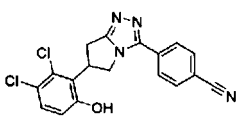 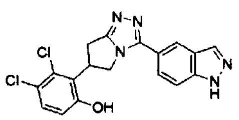 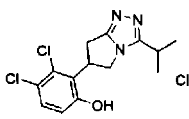 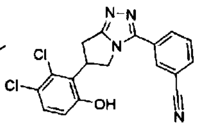 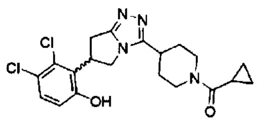

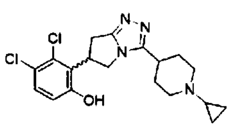 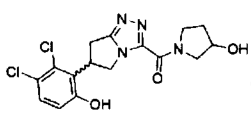 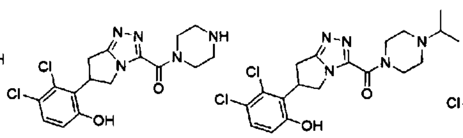 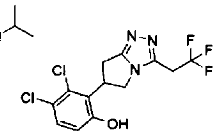

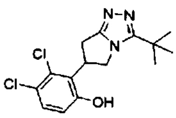 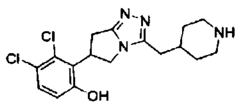 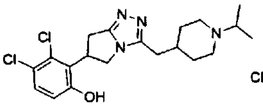 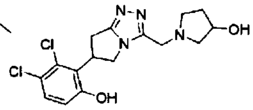 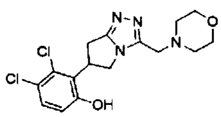

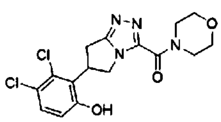 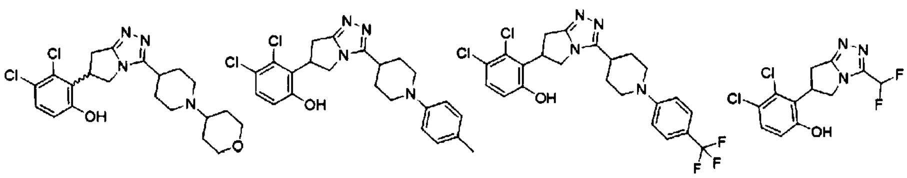

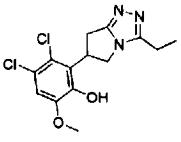 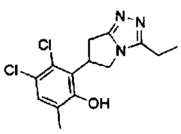 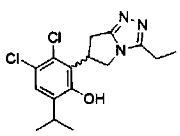 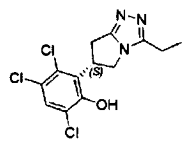 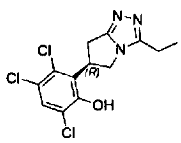

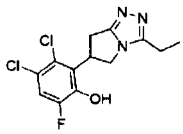 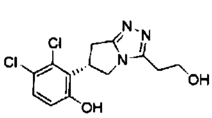 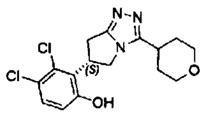 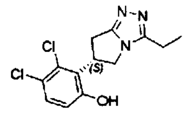 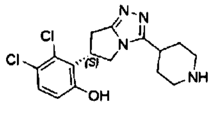

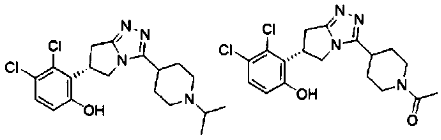 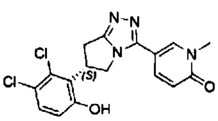 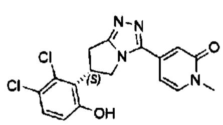 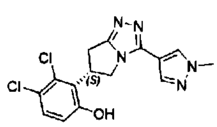

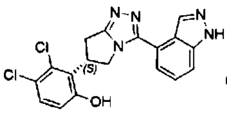 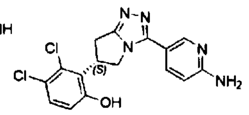 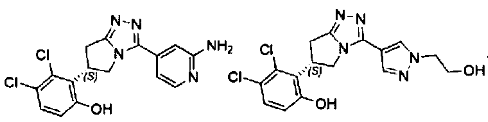 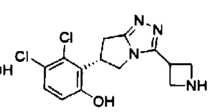

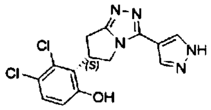 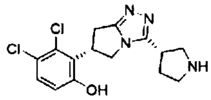 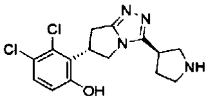 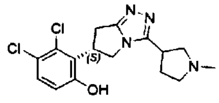 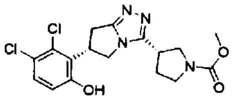

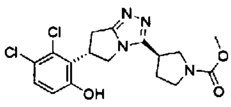 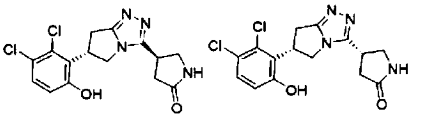 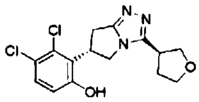 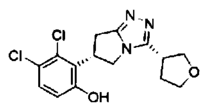

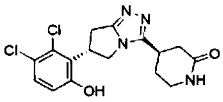 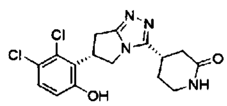 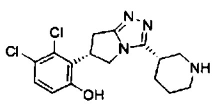 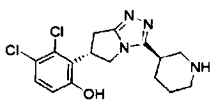 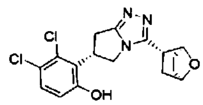

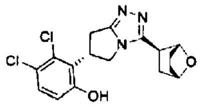 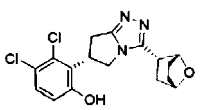 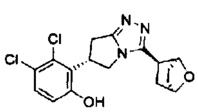 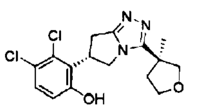 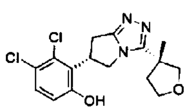

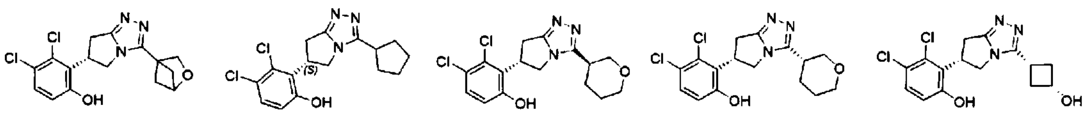

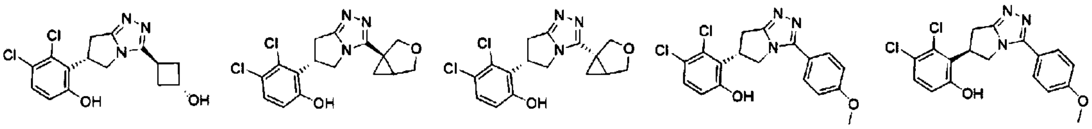

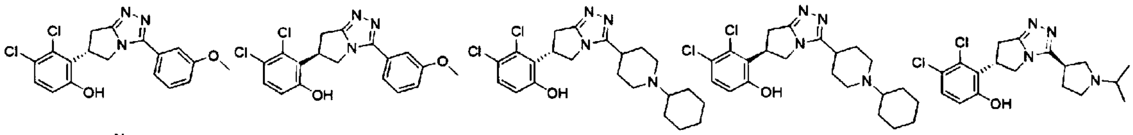

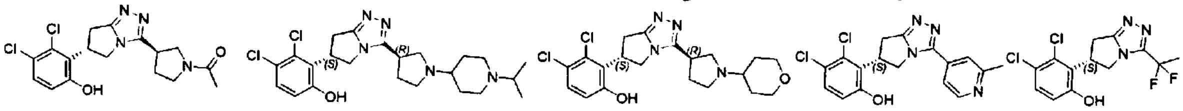

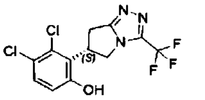 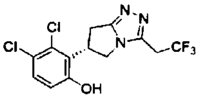 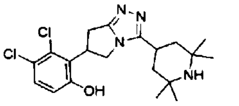 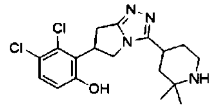 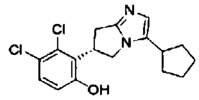

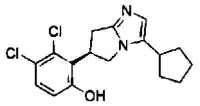 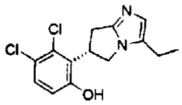 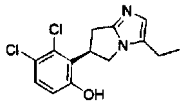 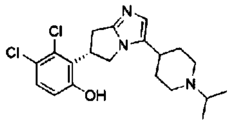 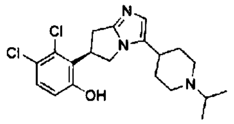

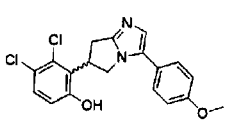 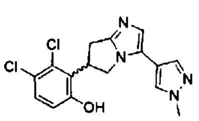 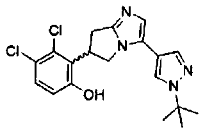 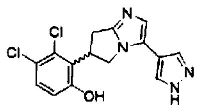 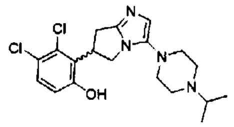

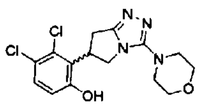 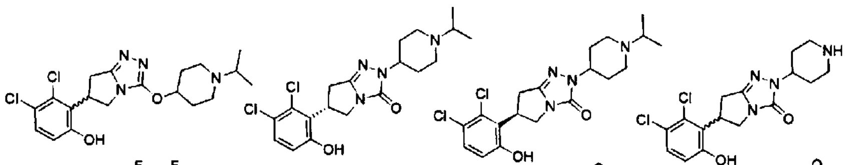

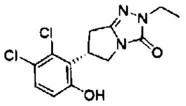 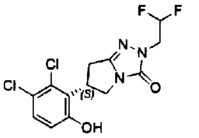 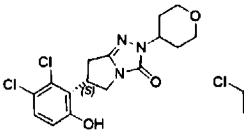 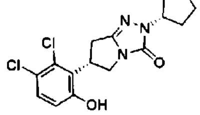 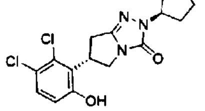

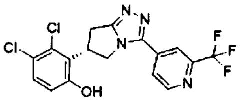 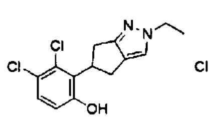 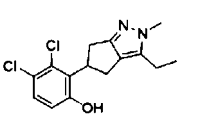 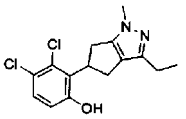 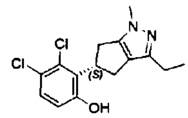

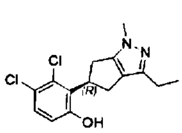 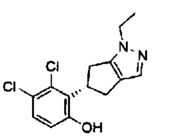 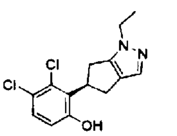 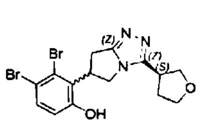 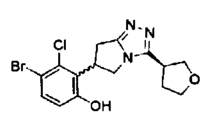

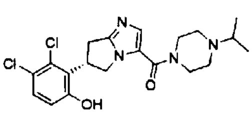

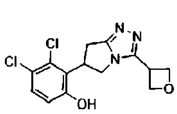 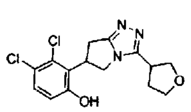 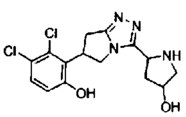 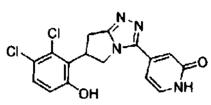 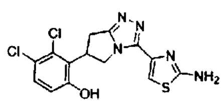

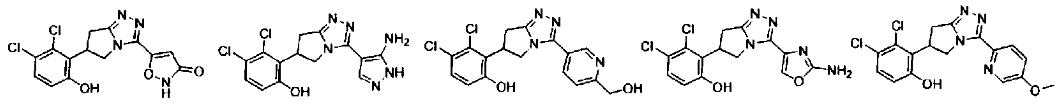

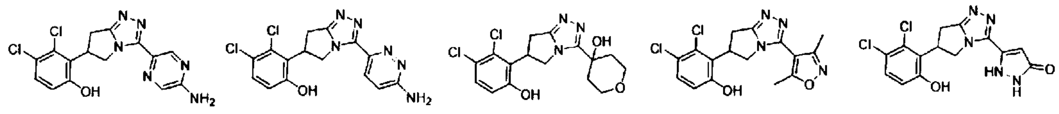

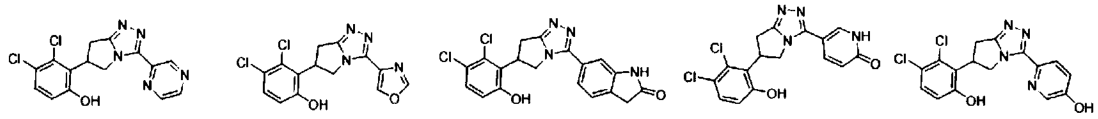

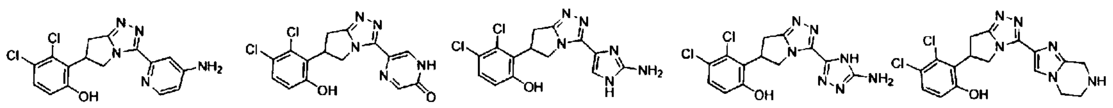

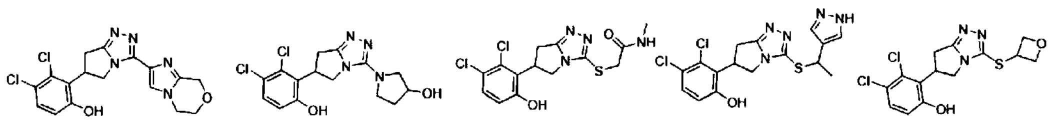

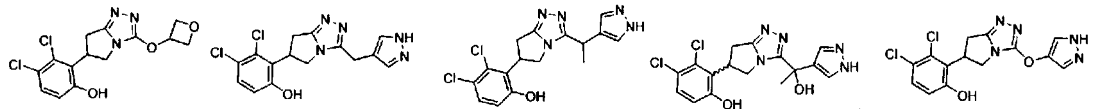

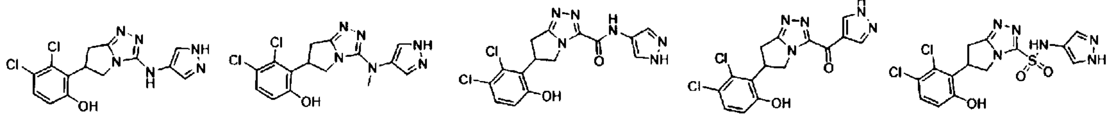

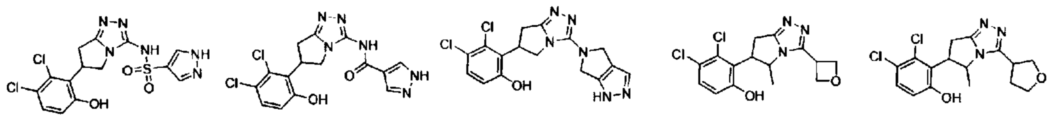

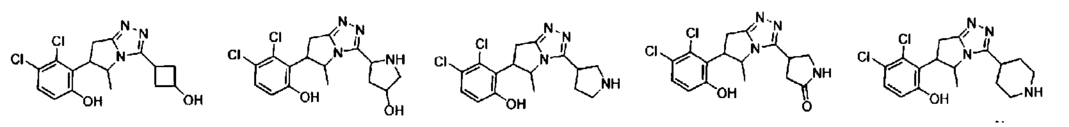

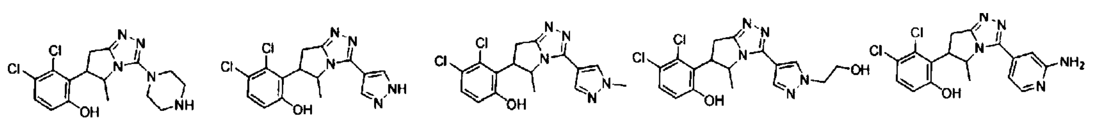

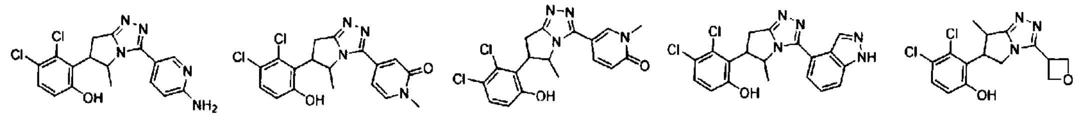

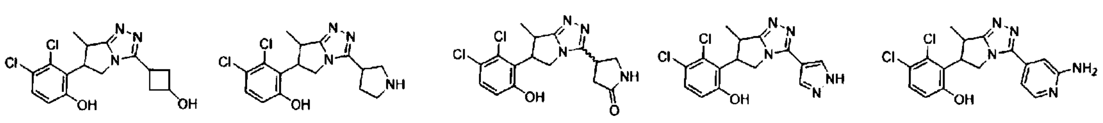

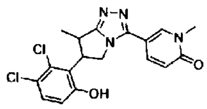 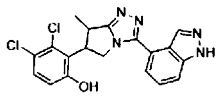

12. Composition pharmaceutique comprenant le composé ou le sel acceptable pharmaceutiquement ou le composé deutéré de celui-ci selon l'une quelconque des revendications 1 à 11 et un support acceptable pharmaceutiquement.

13. Composé ou sel acceptable pharmaceutiquement ou composé deutéré de celui-ci selon l'une quelconque des revendications 1 à 11 ou composition pharmaceutique selon la revendication 12, destiné(e) à être utilisé(e) en tant que médicament.

14. Composé ou sel acceptable pharmaceutiquement ou composé deutéré de celui-ci selon l'une quelconque des revendications 1 à 11 ou composition pharmaceutique selon la revendication 12 destiné(e) à être utilisé(e) dans la prévention et/ou le traitement d'une maladie associée à Kv1.3, de préférence, la maladie comprend l'une quelconque d'un groupe de maladies immunitaires et inflammatoires, telles que la sclérose en plaques, la maladie inflammatoire chronique de l'intestin, la colite ulcéreuse, la maladie de Crohn, la polyarthrite rhumatoïde, le diabète de type 1, le psoriasis et l'asthme, la spondylite et la parodontite, ainsi que l'obésité, le diabète de type 2, la fibrose rénale, la maladie d'Alzheimer, l'accident vasculaire cérébral ischémique.

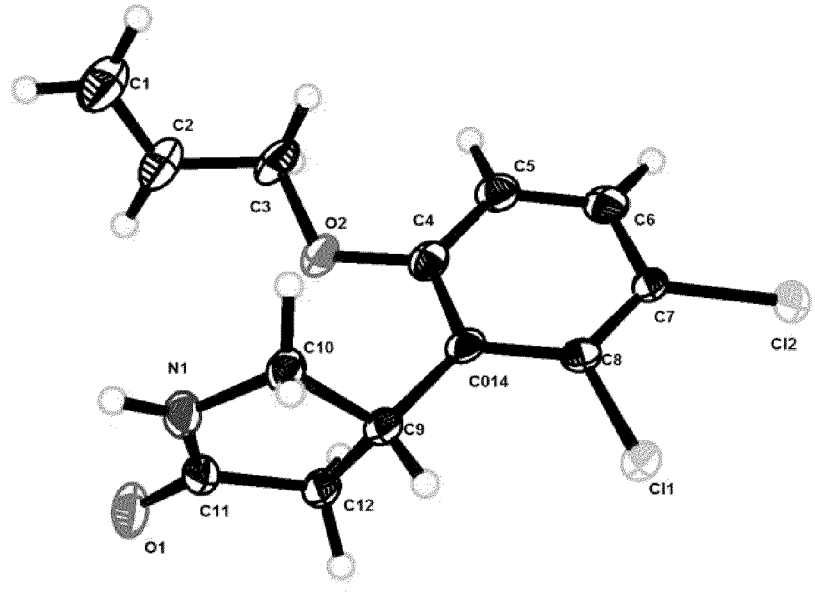

FIG. 1

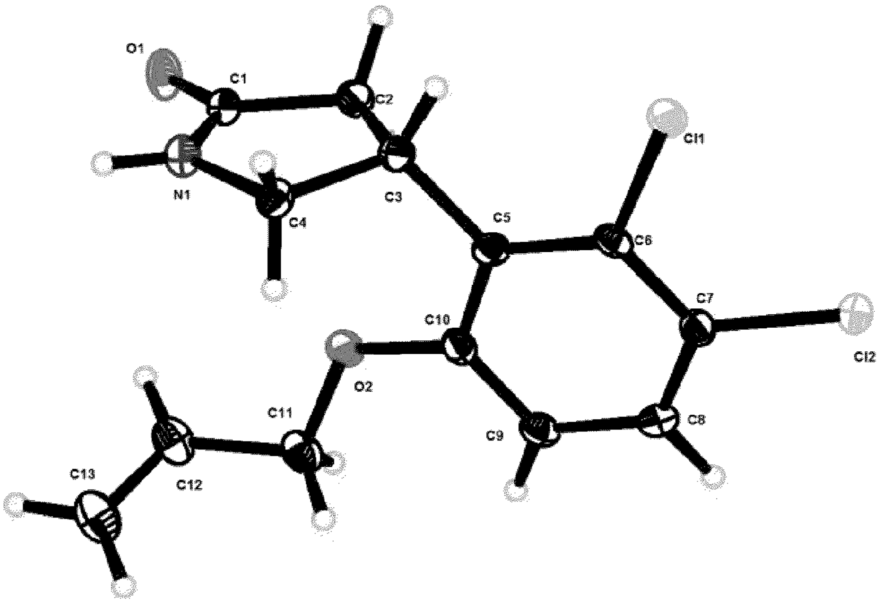

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022076285 A
- WO 2021071806 A
- CN 114746151 A
- CN 114828963 A
- CN 114728177 A


### Non-patent literature cited in the description


- *Proceedings of the National Academy of Sciences*, 2006, vol. 103 (46), 17414-17419